(19) **Europäisches Patentamt**

**European Patent Office**

**Office européen des brevets**

(11) **EP 1 652 858 A1**

(12) **EUROPEAN PATENT APPLICATION**

(43) Date of publication:
03.05.2006 Bulletin 2006/18

(51) Int Cl.:
*C07K 14/18* [(2006.01)]   *A61K 39/295* [(2006.01)]
*G01N 33/576* [(2006.01)]   *A61K 38/16* [(2006.01)]

(21) Application number: **04447239.7**

(22) Date of filing: **28.10.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicants:
- **Innogenetics N.V.**
  **9052 Gent (BE)**
- **Epimmune Inc.**
  **San Diego,**
  **California 92121 (US)**

(72) Inventor: **The designation of the inventor has not yet been filed**

(74) Representative: **Schüller, Cornelis et al**
**Innogenetics N.V.**
**Industriepark Zwijnaarde 7**
**P.O. Box 4**
**9052 Ghent (BE)**

Remarks:
•The sequence listing, which is published as annex to the application documents, was filed after the date of filing. The applicant has declared that it does not include matter which goes beyond the content of the application as filed.
•Claims 11 to 53 are deemed to be abandoned due to non-payment of the claims fees (Rule 31 (2) EPC).

(54) **Peptides for inducing a CTL and/or HTL response to hepatitis C virus**

(57)    The present invention is directed to peptides, and nucleic acids encoding them, derived from the Hepatitis C Virus (HCV). The peptides are those which elicit a CTL and/or HTL response in a host. The invention is also directed to compositions and vaccines for prevention and treatment of HCV infection and diagnostic methods for detection of HCV exposure in patients.

EP 1 652 858 A1

**Description**

**FIELD OF THE INVENTION**

The present invention is directed to peptides or nucleic acids encoding them, derived from the Hepatitis C Virus (HCV). The peptides are those which elicit a cytotoxic and/or helper T lymphocyte response in a host. The invention is also directed to vaccines for prevention and treatment of HCV infection and diagnostic methods for detection of HCV exposure in patients.

**BACKGROUND OF THE INVENTION**

The about 9.6 kb single-stranded RNA genome of the HCV virus comprises a 5'- and 3'-noncoding region (NCRs) and, in between these NCRs a single long open reading frame of about 9 kb encoding an HCV polyprotein of about 3000 amino acids.

HCV polypeptides are produced by translation from the open reading frame and cotranslational proteolytic processing. Structural proteins are derived from the amino-terminal one-fourth of the coding region and include the capsid or Core protein (about 21 kDa), the E1 envelope glycoprotein (about 35 kDa) and the E2 envelope glycoprotein (about 70 kDa, previously called NS1), and p7 (about 7kDa). The E2 protein can occur with or without a C-terminal fusion of the p7 protein (Shimotohno et al. 1995). Recently, an alternative open reading frame in the Core-region was found which is encoding and expressing a protein of about 17 kDa called F (Frameshift) protein (Xu et al. 2001; Ou & Xu in US Patent Application Publication No. US2002/0076415). In the same region, ORFs for other 14-17 kDa ARFPs (Alternative Reading Frame Proteins), A1 to A4, were discovered and antibodies to at least A1, A2 and A3 were detected in sera of chronically infected patients (Walewski et al. 2001). From the remainder of the HCV coding region, the non-structural HCV proteins are derived which include NS2 (about 23 kDa), NS3 (about 70 kDa), NS4A (about 8 kDa), NS4B (about 27 kDa), NS5A (about 58 kDa) and NS5B (about 68 kDa) (Grakoui et al. 1993).

HCV is the major cause of non-A, non-B hepatitis worldwide. Acute infection with HCV (20% of all acute hepatitis infections) frequently leads to chronic hepatitis (70% of all chronic hepatitis cases) and end-stage cirrhosis. It is estimated that up to 20% of HCV chronic carriers may develop cirrhosis over a time period of about 20 years and that of those with cirrhosis between 1 to 4%/year is at risk to develop liver carcinoma (Lauer & Walker 2001, Shiffman 1999). An option to increase the life-span of HCV-caused end-stage liver disease is liver transplantation (30% of all liver transplantations world-wide are due to HCV-infection).

Virus-specific, human leukocyte antigen (HLA) class I-restricted cytotoxic T lymphocytes (CTL) are known to play a major role in the prevention and clearance of virus infections in vivo (Houssaint et al., 2001; Gruters et al., 2002; Tsai et al., 1997; Marray et al., 1992; Lukacher et al, 1984; Tigges et al., 1993).

MHC molecules are classified as either class I or class II. Class I MHC molecules are expressed on virtually all nucleated cells. Peptide fragments presented in the context of Class I MHC molecules are recognized by CD8+ T lymphocytes (cytotoxic T lymphocytes or CTLs). CD8+ T lymphocytes frequently mature into cytotoxic effecters which can lyse cells bearing the stimulating antigen. CTLs are particularly effective in eliminating tumor cells and in fighting viral infections.

Class II MHC molecules are expressed primarily on activated lymphocytes and antigen-presenting cells. CD4+ T lymphocytes (helper T lymphocytes or HTLs) are activated with recognition of a unique peptide fragment presented by a class II MHC molecule, usually found on an antigen presenting cell like a macrophage or dendritic cell. CD4+ T lymphocytes proliferate and secrete cytokines that either support an antibody-mediated response through the production of IL-4 and IL-10 or support a cell-mediated response through the production of IL-2 and IFN-gamma.

T lymphocytes recognize an antigen in the form of a peptide fragment bound to the MHC class I or class II molecule rather than the intact foreign antigen itself. An antigen presented by a MHC class I molecule is typically one that is endogenously synthesized by the cell (e.g., an intracellular pathogen). The resulting cytoplasmic antigens are degraded into small fragments in the cytoplasm, usually by the proteasome (Niedermann et al.,1995). Antigens presented by MHC class II molecules are usually soluble antigens that enter the antigen presenting cell via phagocytosis, pinocytosis, or receptor-mediated endocytosis. Once in the cell, the antigen is partially degraded by acid-dependent proteases in endosomes (Blum et al., 1997; Arndt et al., 1997).

Functional HLAs are characterized by a deep binding groove to which endogenous as well as foreign, potentially antigenic peptides bind. The groove is further characterized by a well-defined shape and physico-chemical properties. HLA class I binding sites are closed, in that the peptide termini are pinned down into the ends of the groove. They are also involved in a network of hydrogen bonds with conserved HLA residues (Madden et al.,1992). In view of these restraints, the length of bound peptides is limited to 8-10 residues. However, it has been demonstrated by Henderson et al (1992) that peptides of up to 12 amino acid residues are also capable of binding HLA class I. Superposition of the structures of different HLA complexes confirmed a general mode of binding wherein peptides adopt a relatively linear, extended conformation.

At the same time, a significant variability in the conformation of different peptides was observed also. This variability ranges from minor structural differences to notably different binding modes. Such variation is not unexpected in view of the fact that class I molecules can bind thousands of different peptides, varying in length (8-12 residues) and in amino acid sequence. The different class I allotypes bind peptides sharing one or two conserved amino acid residues at specific positions. These residues are referred to as anchor residues and are accommodated in complementary pockets (Falk, K. et al., 1991). Besides primary anchors, there are also secondary anchor residues occupied in more shallow pockets (Matsumura et al., 1992). In total, six allele-specific pockets termed A-F have been characterized (Saper et al., 1991; Latron et al., 1992). The constitution of these pockets varies in accordance with the polymorphism of class I molecules, giving rise to both a high degree of specificity (limited cross reactivity) while preserving a broad binding capacity.

In contrast to HLA class I binding sites, class II sites are open at both ends. This allows peptides to extend from the actual region of binding, thereby "hanging out" at both ends (Brown et al., 1993). Class II HLAs can therefore bind peptide ligands of variable length, ranging from 9 to more than 25 amino acid residues. Similar to HLA class I, the affinity of a class II ligand is determined by a "constant" and a "variable" component. The constant part again results from a network of hydrogen bonds formed between conserved residues in the HLA class II groove and the main-chain of a bound peptide. However, this hydrogen bond pattern is not confined to the N- and C-terminal residues of the peptide but distributed over the whole of the chain. The latter is important because it restricts the conformation of complexed peptides to a strictly linear mode of binding. This is common for all class II allotypes. The second component determining the binding affinity of a peptide is variable due to certain positions of polymorphism within class II binding sites. Different allotypes form different complementary pockets within the groove, thereby accounting for subtype-dependent selection of peptides, or specificity. Importantly, the constraints on the amino acid residues held within class II pockets are in general "softer" than for class I. There is much more cross reactivity of peptides among different HLA class II allotypes. Unlike for class I, it has been impossible to identify highly conserved residue patterns in peptide ligands (so-called motifs) that correlate with the class II allotypes.

Peptides that bind some MHC complexes have been identified by acid elusion methods (Buus et al., 1988), chromatography methods (Jardetzky, et al., 1991 and Falk et al., 1991), and by mass spectrometry methods (Hunt, et al., 1992). A review of naturally processed peptides that bind MHC class I molecules is set forth in Rotzschke and Falk, 1991.

Of the many thousand possible peptides that are encoded by a complex foreign pathogen, only a small fraction ends up in a peptide form capable of binding to MHC class I or class II antigens and can thus be recognized by T cells if containing a matching T-cell receptor. This phenomenon is known as immunodominance (Yewdell et al., 1997). More simply, immunodominance describes the phenomenon whereby immunization or exposure to a whole native antigen results in an immune response directed to one or a few "dominant" epitopes of the antigen rather than every epitope that the native antigen contains. Immunodominance is influenced by a variety of factors that include MHC-peptide affinity, antigen processing and T-cell receptor recognition.

In view of the heterogeneous immune response observed with HCV infection, induction of a multi-specific cellular immune response directed simultaneously against multiple HCV epitopes appears to be important for the development of an efficacious vaccine against HCV. There is a need, however, to establish vaccine embodiments that elicit immune responses that correspond to responses seen in patients that clear HCV infection.

The large degree of HLA polymorphism is an important factor to consider with the epitope-based approach to vaccine development. To address this factor, epitope selection can include identification of peptides capable of binding at high or intermediate affinity to multiple HLA molecules or selection of peptides binding the most prevalent HLA types. Another important factor to be considered in HCV vaccine development is the existence of different HCV genotypes and subtypes. Therefore, HCV genotype- or subtype- specific immunogenic epitopes need to be identified for all considered genotypes or subtypes. However, it is preferred to identify epitopes covering more than one HCV genotype or subtype.

The different characteristics of class I and class II MHC molecules are responsible for specific problems associated with the prediction of potential T-cell epitopes. As discussed before, class I molecules bind short peptides that exhibit well-defined residue type patterns.

This has led to various prediction methods that are based on experimentally determined statistical preferences for particular residue types at specific positions in the peptide.

Although these methods work relatively well, uncertainties associated with non-conserved positions limit their accuracy. Methods for MHC/peptide binding prediction can grossly be subdivided into two categories: "statistical methods" that are driven by experimentally obtained affinity data and "structure-related methods" that are based on available 3D structural information of MHC molecules. Alternatively, a molecular dynamics simulation is sometimes performed to model a peptide within an MHC binding groove (Lim et al., 1996). Another approach is to combine loop modeling with simulated annealing (Rognan et al., 1999). Most research groups emphasize the importance of the scoring function used in the affinity prediction step.

Several MHC binding HCV peptides have already been disclosed, e.g. in WO02/34770 (Imperial College Innovations Ltd), WO01/21189 and WO02/20035 (Epimmune), WO04/024182 (Intercell), WO95/25122 (The Scripps Research Institute), WO95/27733 (Government of the USA, Department of Health and Human Services), EP 0935662 (Chiron), WO02/26785 (Immusystems GmbH), WO95/12677 (Innogenetics N.V) and WO97/34621 (Cytel Corp).

There is a need to substantially improve both the structure prediction and the affinity assessment steps of methods which predict the affinity of a peptide for a major histocompatibility (MHC) class I or class II molecule. The main problem encountered in this field is the poor performance of prediction algorithms with respect to MHC alleles for which experimentally determined data (both binding and structural information) are scarce. This is e.g. the case for HLA-C.

Accordingly, while some MHC binding peptides have been identified, there is a need in the art to identify novel MHC binding peptides from HCV that can be utilized to generate an immune response against HCV from which they originate. Also, peptides predicted to bind (and binding) with reasonable affinity need a slow off rate in order to be immunogenic (Micheletti et al., 1999; Brooks et al., 1998; van der Burg et al., 1996).

## SUMMARY OF THE INVENTION

The present invention is directed to peptides or epitopes derived from the Core, E1, E2, P7, NS2, NS3, NS4 (NS4A and NS4B) and NS5 (NS5A and NS5B) protein of the Hepatitis C Virus (HCV). The peptides are those which elicit a HLA class I and/or class II restricted T lymphocyte response in an immunized host. More specific, the HLA class I restricted peptides of the present invention bind to at least one HLA molecule of the following HLA class I groups: HLA-A*01, HLA-A*02, HLA-A*03, HLA-A*11, HLA-A*24, HLA-B*07, HLA-B*08, HLA-B*35, HLA-B*40, HLA-B*44, HLA-Cw3, HLA-Cw4, HLA-Cw6 or HLA-Cw7. Preferred peptides are summarized in Table 13. The HLA class II restricted peptides of the present invention bind to at least one HLA molecule of the following HLA class II groups: HLA-DRB1, -DRB2, -DRB3, -DRB4, -DRB5, -DRB6, -DRB7, -DRB8 or -DRB9. Said HLA class II groups are sometimes summarized as HLA-DRB1-9. Preferred class II restricted peptides are given in Table 14.

The HLA class I and II binding peptides of the invention have been identified by the method as described in WO03/105058 -Algonomics, by the method as described by Epimmune in WO01/21189 and/or by three public database prediction servers, respectively Syfpeithi, BIMAS and nHLAPred. Each of the peptides per se (as set out in the Tables) is part of the present invention. Furthermore, it is also an inventive aspect of this application that each peptide may be used in combination with the same peptide as multiple repeats, or with any other peptide(s) or epitope(s), with or without additional linkers. Accordingly, the present invention also relates to a composition and more specific to a polyepitopic peptide.

In a further embodiment, the present invention relates to a polyepitopic peptide comprising at least three peptides selected from the HLA-B and/or HLA-C binding peptides as disclosed in Table 13.

In a further embodiment, the present invention relates to a polyepitopic peptide comprising at least two peptides derived from a HCV protein and capable of inducing a HLA class I and/or class II restricted T lymphocyte response, wherein at least one peptide is a HLA-C binding peptide.

In a specific embodiment of the invention, the peptides are characterized in that they are present in the HCV consensus sequence of genotype 1a, 1b and/or 3a.

Furthermore, the present invention relates to nucleic acids encoding the peptides described herein. More particular, the present invention relates to a "minigene" or a polynucleotide that encodes a polyepitopic peptide as described herein.

The current invention also relates to a vector, plasmid, recombinant virus and host cell comprising the nucleic acid(s) or minigene(s) as described herein.

The peptides, corresponding nucleic acids and compositions of the present invention are useful for stimulating an immune response to HCV by stimulating the production of CTL and/or HTL responses. The peptide epitopes of the present invention, which are derived from native HCV amino acid sequences, have been selected so as to be able to bind to HLA molecules and induce or stimulate an immune response to HCV. In a specific embodiment, the present invention provides "nested epitopes".

In a further embodiment, the present invention provides polyepitopic peptides, polynucleotides, compositions and combinations thereof that enable epitope-based vaccines that are capable of interacting directly or indirectly with HLA molecules encoded by various genetic alleles to provide broader population coverage than prior vaccines. In a preferred embodiment, the invention relates to a composition comprising HCV-specific CTL epitopes in combination with HCV-specific HTL epitopes. Said composition can be in the form of a minigene comprising one or more CTL epitopes and one or more HTL epitopes.

In a further embodiment, the peptides of the invention, or nucleic acids encoding them, are used in diagnostic methods such as the determination of a treatment regimen, the determination of the outcome of an HCV infection, evaluation of an immune response or evaluation of the efficacy of a vaccine.

## FIGURE LEGENDS

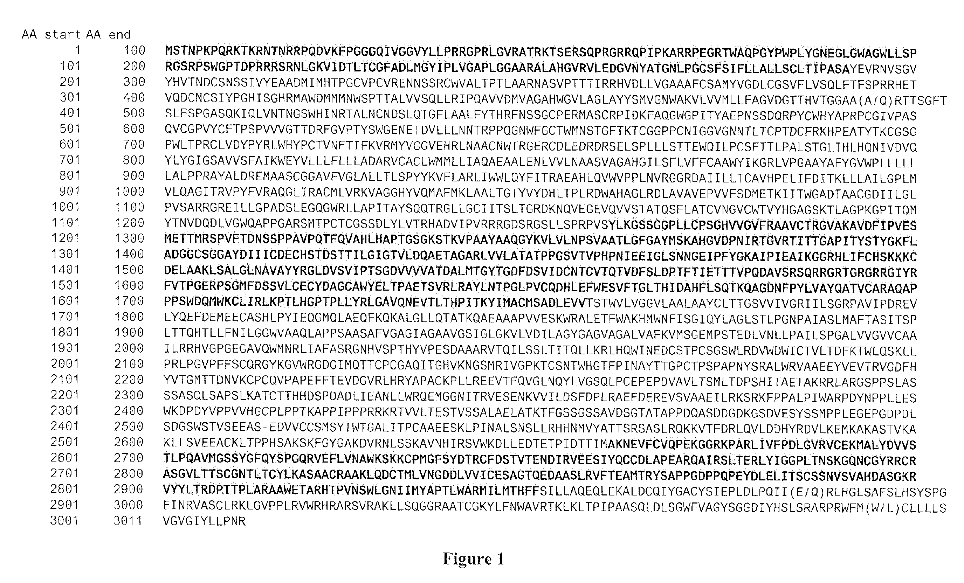

**Figure 1**: HCV 1b consensus sequence (SEQ ID NO 769), based on a selection of available HCV sequences with identification (in bold) of the parts used for the 9-mer peptide design by the method as described by Algonomics N.V.; said parts are Core, NS3 and NS5; the amino acid numbering of the 9-mers present in Tables 1-11 is based on the HCV sequence disclosed in Figure 1.

| | AA start | AA end | part of interest | AA start | AA end | #AA |
|---|---|---|---|---|---|---|
| C | 1 | 191 | C | 1 | 191 | 191 |
| E1 | 192 | 383 | | | | |
| E2 | 384 | 746 | | | | |
| P7 | 747 | 809 | | | | |
| NS2 | 810 | 1026 | | | | |
| NS3 | 1027 | 1657 | NS3 | 1160 | 1657 | 498 |
| NS4A | 1658 | 1711 | | | | |
| NS4B | 1712 | 1972 | | | | |
| NS5A | 1973 | 2420 | | | | |
| NS5B | 2421 | 3011 | NS5B | 2560 | 2850 | 291 |

**Figure 2**: HCV 1b consensus sequence (SEQ ID NO 770) with identification (in bold) of the parts used for the 10-mer peptide design by the method as described by Algonomics N.V., and used for determination of HCV genotype cross-reactivity; said parts are Core, NS3, NS4 and NS5. The amino acid numbering is the same as for Figure 1. The amino acid numbering of the 10-mers present in Tables 1-11 is based on the HCV sequence disclosed in Figure 2.
**Figure 3**: Binding of HLA-A02 reference peptide FLPSDC(F1)FPSV on HLA-A02.
**Figure 4**: Example of a typical HLA-A02 competition experiment.
**Figure 5**: HCV 1a consensus sequence (SEQ ID NO 771) used for determination of HCV genotype cross-reactivity.
**Figure 6**: HCV 3a consensus sequence (SEQ ID NO 772) used for determination of HCV genotype cross-reactivity.

## DETAILED DESCRIPTION OF THE INVENTION

The present invention is directed to peptides derived from the Core, E1, E2, P7, NS2, NS3, NS4 (NS4A and NS4B) or NS5 (NS5A and NS5B) protein of the Hepatitis C Virus (HCV). The peptides are those which elicit a HLA class I and/or class II restricted T lymphocyte response in an immunized host.
More specific, the HLA class I restricted peptides (CTL epitopes) of the present invention bind at least one HLA molecule of the following HLA class I groups: HLA-A*01, HLA-A*02, HLA-A*03, HLA-A*11, HLA-A*24, HLA-B*07, HLA-B*08, HLA-B*35, HLA-B*40, HLA-B*44, HLA-Cw3, HLA-Cw4, HLA-Cw6 or HLA-Cw7. Preferred peptides are summarized in Table 13. The HLA class II restricted peptides (HTL epitopes) of the present invention bind at least one HLA molecule of the following HLA class II groups: HLA-DRB1, -DRB2, -DRB3, -DRB4, -DRB5, -DRB6, -DRB7, -DRB8 or -DRB9. Said HLA class II groups are sometimes summarized as HLA-DRB1-9. Preferred HTL epitopes are given in Table 14.
Each of the HLA class I and class II peptides per se (as set out in the Tables) is part of the present invention. Furthermore, it is an aspect of the invention that each epitope may be used in combination with any other epitope.

### Identification of the peptides

Based on the hundreds of known HCV genotypes and subtypes (at least 3000 amino acids per sequence), thousands of theoretical CTL and/or HTL epitopes are predicted according to the methods as described herein. Starting from said long list, a first selection of epitopes has been made based on the predicted binding affinity.
The HLA class I and II binding peptides of the invention have been identified by the method as described in WO03/105058 -Algonomics, by the method as described by Epimmune in WO01/21189 and/or by three public epitope prediction servers respectively Syfpeithi, BIMAS and nHLAPred.
A first set of CTL peptides is derived by the method as described in WO03/105058 by Algonomics N.V., Zwijnaarde, Belgium, which is incorporated herein by reference. Said method is directed to a structure-based prediction of the affinity of potentially antigenic peptides for major histocompatibility (MHC) receptors.
Initially, a HCV consensus sequence is designed. To do this, a selection of HCV sequences from HCV type 1b present in the "Los Alamos" database are clustered and aligned. The HCV Sequence Database from the Los Alamos National laboratory can be found on: http://hcv.lanl.gov/content/hcv-db/HelpDocs/cluster-help.html.

The generated multiple sequence alignments have been used to identify interesting (i.e. conserved) regions in the HCV proteins for CTL epitope prediction.

Figure 1 discloses the HCV consensus sequence used for the 9-mer CTL epitope prediction in the present invention. Amino acid numbering for the 9-mers present in Tables 1-11 is based on said sequence.

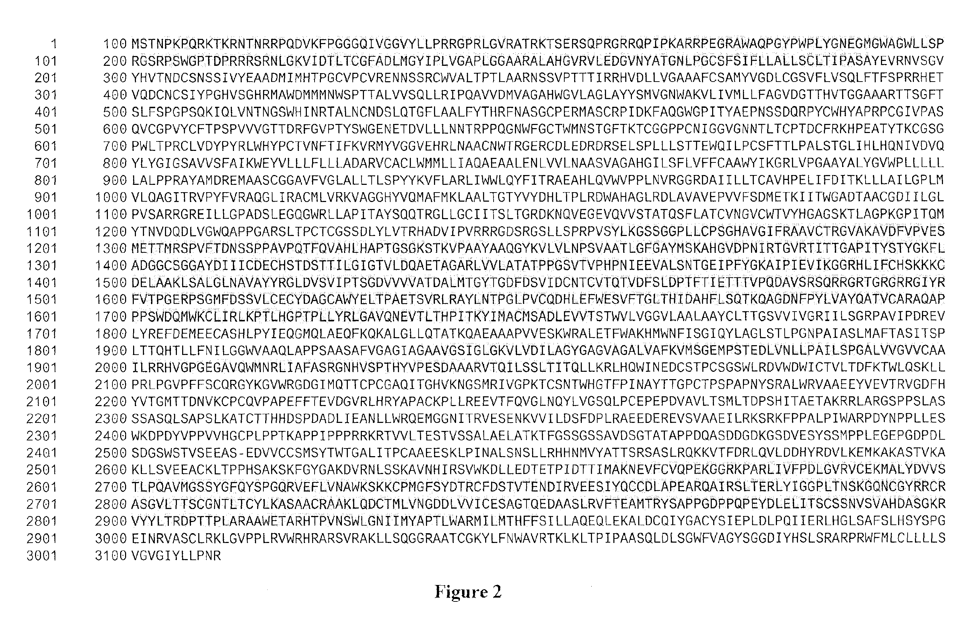

Figure 2 discloses the HCV consensus sequence used for the 10-mer CTL epitope prediction in the present invention. Amino acid numbering for the 10-mers present in Tables 1-11 is based on said sequence.

Predictions were made for HLA-A0101, HLA-A0201, HLA-A0301, HLA-A2402, HLA-B0702, HLA-B0801, HLA-B3501, HLA-B4403, HLA-Cw0401, HLA-Cw0602 and HLA-Cw0702.

Tables 1-11 disclose the HLA-A, HLA-B and HLA-C binding peptides of the current invention derived by the above-described algorithm. Division is made between Strong binders (S) with Kdpred <0.1$\mu$M, Medium binders (M) with Kdpred 0.1-1$\mu$M and Weak binders (W) with Kdpred 1-10$\mu$M. Kdpred is the affinity (dissociation constant) as predicted by the algorithm.

A further selection is made based upon the presence of the epitopes in the most prevalent genotypes. Accordingly, those peptides that are present in

- at least genotype 3 a, or
- at least genotype 1b, or
- at least genotype 1a and 1b, or
- at least genotypes 1a, 1b and 3a, are retained for further testing. These peptides are summarized in Table 13.

Furthermore, other HCV genotypes (e.g. genotype 4a) can be retained in view of prevalence and/or importance.

A second set of peptides is identified by the method as described in WO01/21189 by Epimmune Inc., California, USA, which is incorporated herein by reference. Proprietary computer algorithms are used to rapidly identify potential epitopes from genomic or proteomic sequence data of viruses, bacteria, parasites or tumor-associated antigens. The program can also be used to modify epitopes (analog) in order to enhance or suppress an immune response.

The algorithm is based on the conversion of coefficient-based scores into KD (IC50) predictions (PIC Score) thereby facilitating combined searches involving different peptide sizes or alleles. The combined use of scaling factors and exponential power corrections resulted in best goodness of fit between calculated and actual IC50 values. Because the algorithm predicts epitope binding with any given affinity, a more stringent candidate selection procedure of selecting only top-scoring epitopes, regardless of HLA-type, can be utilized.

Protein sequence data from 57 HCV isolates were evaluated for the presence of the designated supermotif or motif. The 57 strains include COLONEL-ACC-AF290978, H77-ACC-NC, HEC278830-ACC-AJ278830, LTD1-2-XF222-ACC-AF511948, LTD6-2-XF224-ACC-AF511950, JP.HC-J1-ACC-D10749, US.HCV-H-ACC-M67463, US.HCV-PT-ACC-M62321, D89815-ACC-D89815, HC-J4-ACC-AF054250, HCR6-ACC-AY045702, HCV-CG1B-ACC-AF333324, HCV-JS-ACC-D85516, HCV-K1-R1-ACC-D50480, HCV-S1-ACC-AF356827, HCVT050-ACC-AB049087, HPCHCPO-ACC-D45172, M1LE-ACC-AB080299, MD11-ACC-AF207752, Source-ACC-AF313916, TMORF-ACC-D89872, AU.HCV-A-ACC-AJ000009, CN.HC-C2-ACC-D10934, CN.HEBEI-ACC-L02836, DE.HCV-AD78-ACC-AJ132996, DE.HD-1-ACC-U45476, DE.NC1-ACC-AJ238800, JP.HCV-BK-ACC-M58335, JP.HCV-J-ACC-D90208, JP.HCV-N-ACC-AF139594, JP.J33-ACC-D14484, JP.JK1-full-ACC-X61596, JP.JT-ACC-D 11355, JP.MD1-1-ACC-AF165045, KR.HCU16362-ACC-U16362, KR.HCV-L2-ACC-U01214, RU.274933RU-ACC-AF176573, TR.HCV-TR1-ACC-AF483269, TW.HCU89019-ACC-U89019, TW.HPCGENANTI-ACC-M84754, G2AKI-ACC-AF169003, HC-J6CH-ACC-AF177036, MD2A-1-ACC-AF238481, NDM228-ACC-AF169002, JP.JCH-1-ACC-AB047640, JP.JFH-1-ACC-AB047639, JP.Td-6-ACC-D00944, JPUT971017-ACC-AB030907, MD2B-1-ACC-AF238486, JP.HC-J8-ACC-D10988, BEBE1-ACC-D50409, CB-ACC-AF046866, K3A-ACC-D28917, NZL1-ACC-D17763, DE.HCVCENS1-ACC-X76918, JP.HCV-Tr-ACC-D49374 and EG.ED43-ACC-Y11604.

Predictions were made for HLA-A0101, HLA-A0201, HLA-A1101, HLA-A2402, HLA-B0702, HLA-B-0801 and HLA-B4002. For B0801, no PIC algorithm is available but motif-positive sequences were selected.

Tables 1, 2, 3, 4, 5, 6 and 8 disclose the HLA-A and HLA-B peptides of the current invention yielding PIC Scores < 100 derived by the above-described algorithm.

A further selection is made based upon the presence of the epitopes in the most prevalent genotypes. Accordingly, those peptides that are present in

- at least genotype 3a, or
- at least genotype 1b, or
- at least genotype 1a and 1b, or
- at least genotypes 1a, 1b and 3a, are retained for further testing. These peptides are summarized in table 13.
  Furthermore, other HCV genotypes (e.g. genotype 4a) can be retained in view of prevalence and/or importance.

A third set of peptides is identified by three publicly available algorithms.

Initially, a HCV 1b consensus sequence is designed. HCV sequences from 80 HCV type 1b sequences were retrieved from the HCV sequence database http://hcv.lanl.gov/content/hcv-db/index of the Division of Microbiology and Infectious Diseases of the National Institute of Allergies and Infectious Diseases (NIAID).

The generated multiple sequence alignments are used to identify interesting regions in the HCV proteins for CTL epitope prediction. Figure 2 discloses the HCV consensus sequence used for the CTL epitope prediction. Amino acid numbering throughout the specification is based on said sequence.

Based on said consensus sequence, three different prediction algorithms were used for CTL epitope prediction:

A) Syfpeithi:

Hans-Georg Rammensee, Jutta Bachmann, Niels Nikolaus Emmerich, Oskar Alexander Bachor, Stefan Stevanovic: SYFPEITHI: database for MHC ligands and peptide motifs. Immunogenetics (1999) 50: 213-219; www.syfpeithi.de)

The prediction is based on published motifs (pool sequencing, natural ligands) and takes into consideration the amino acids in the anchor and auxiliary anchor positions, as well as other frequent amino acids. The scoring system evaluates every amino acid within a given peptide. Individual amino acids may be given the arbitrary value 1 for amino acids that are only slightly preferred in the respective position, optimal anchor residues are given the value 15; any value between these two is possible. Negative values are also possible for amino acids which are disadvantageous for the peptide's binding capacity at a certain sequence position. The allocation of values is based on the frequency of the respective amino acid in natural ligands, T-cell epitopes, or binding peptides. The maximal scores vary between different MHC alleles. Only those MHC class I alleles for which a large amount of data is available are included in the "epitope prediction" section of SYFPEITHI. SYFPEITHI does not make predictions for HLA-C alleles.

Predictions were made for HLA-A01, A0201, A03, A2402, B0702, B08 and B44. For each class, both 9- and 10-mers were predicted, except for B08, where 8- and 9-mers were predicted, but no 10-mers.

B) BIMAS:

This algorithm allows users to locate and rank 8-mer, 9-mer, or 10-mer peptides that contain peptide-binding motifs for HLA class I molecules. Said rankings employ amino acid/position coefficient tables deduced from the literature by Dr. Kenneth Parker of the National Institute of Allergy and Infectious Diseases (NIAID) at the National Institutes of Health (NIH) in Bethesda, Maryland. The Web site (http://bimas.dcrt.nih.gov/molbio/hla_bind/) was created by Ronald Taylor of the Bioinformatics and Molecular Analysis Section (BIMAS), Computational Bioscience and Engineering Laboratory (CBEL), Division of Computer Research & Technology (CIT), National Institutes of Health, in collaboration with Dr. Parker. The initial (running) score is set to 1.0. For each residue position, the program examines which amino acid is appearing at that position. The running score is then multiplied by the coefficient for that amino acid type, at that position, for the chosen HLA molecule. These coefficients have been pre-calculated and are stored for use by the scoring algorithm in a separate directory as a collection of HLA coefficient files. The idea behind these tables is the assumption that, to the first approximation, each amino acid in the peptide contributes independently to binding to the class I molecule. Dominant anchor residues, which are critical for binding, have coefficients in the tables that are significantly different from 1. Highly favorable amino acids have coefficients substantially greater than 1, and unfavorable amino acids have positive coefficients that are less than one. Auxiliary anchor residues have coefficients that are different from 1 but smaller in magnitude than dominant anchor residues. Using 9-mers, nine multiplications are performed. Using 10-mers, nine multiplications are again performed, because the residue lying at the fifth position in the sequence is skipped.

The resulting running score is multiplied by a final constant to yield an estimate of the half time of disassociation. The final multiplication yields the score reported in an output table. Predictions were made for HLA-A01, A0201, A03, A24, B07, B08, B3501, B4403, Cw0301, Cw0401, Cw0602 and Cw0702. For each class, both 9-and 10-mers were predicted, except for B08, where 8-, 9- and 10-mers were predicted.

C) nHLAPred

nHLAPred is a highly accurate MHC binders' prediction method for the large number of class I MHC alleles. (Dr. GPS Raghava, Coordinator, Bioinformatics Centre, Institute of Microbial Technology, Sector 39A, Chandigarh, India; http://imtech.rs.in/raghava). The algorithm is partitioned in two parts ComPred and ANNpred. In the ComPred part the prediction is based on the hybrid approach of Quantitative matrices and artificial neural network. In ANNPred the prediction is solely based on artificial neural network.

ComPred: This part of the algorithm can predict the MHC binding peptides for 67 MHC alleles. The method is systematically developed as follows:

Firstly, a quantitative matrix (QM) based method has been developed for 47 MHC class I alleles having minimum 15 binders available in the MHCBN database.

Quantitative matrices provide a linear model with easy to implement capabilities. Another advantage of using the matrix approach is that it covers a wider range of peptides with binding potential and it gives a quantitative score to each peptide.

Further, an artificial neural network (ANN) based method has been developed for 30 out of these 47 MHC alleles having 40 or more binders. The ANNs are self-training systems that are able to extract and retain the patterns present in submitted data and subsequently recognize them in previously unseen input. The ANNs are able to classify the data of MHC binders and non-binders accurately as compared to other. The ANNs are able to generalize the data very well. The major constraint of neural based prediction is that it requires large data for training. In addition, the method allows prediction of binders for 20 more MHC alleles using the quantitative matrices reported in the literature.

Predictions were made for HLA-A01, A0201, A0301, A24, B0702, B08, B3501, B4403, Cw0301, Cw0401, Cw0602 and Cw0702. nHLAPred can only predict 9-mers.

For each combination of prediction algorithm, protein and HLA allele, a list of the top ranking peptides (= predicted to have the highest affinity) is retrieved.

A list was created (not shown) with all peptides for all HLA alleles in descending order of affinity. In this list, the peptides were marked according to occurrence in different HCV genotypes (1b, 1a and/or 3a consensus sequences) and to cross-reaction between HLA alleles. For each HLA class, all peptides predicted by the different prediction servers are combined in 1 table (not shown) with the ranknumbers for each of the predictionservers per column. For each peptide the number of predictionservers that assigned a ranknumber up to 60 or 100 are counted.

Those peptides that are predicted by 2 to 4 algorithms and that are within the 60 or 100 best are finally selected. If upon binding analysis (see below) only few high affinity binding peptides are identified, additional selections can be made (e.g. from peptides predicted by the Epimmune algorithm and yielding PIC scores < 1000). All these peptides are given in Table 13.

As an example, the selection of the B07 peptides has been disclosed in Example 2. A comparable procedure was followed for the other HLA-binding peptides predicted by Epimmune and the three public algorithms.

Table 13 discloses the selection of the HLA-A, HLA-B and HLA-C peptides of the current invention that are predicted to bind to a given HLA and that are derived by the above-described procedures. The peptide and corresponding nucleic acid compositions of the present invention are useful for inducing or stimulating an immune response to HCV by stimulating the production of CTL responses.

The HLA class II binding peptides of the present invention have been identified by the method as described in W0 01/21189A1 by Epimmune Inc., California, USA, which is incorporated herein by reference. Protein sequence data from 57 HCV isolates (as for the CTL prediction) were evaluated for the presence of the designated supermotif or motif. Predictions were made using the HLA DR-1-4-7 supermotif for peptides that bind to HLA-DRB 1*0401, DRB1*0101 and DRB1*0701, and using HLA DR3 motifs for peptides that bind to DRB1*0301.

The predicted HTL peptides are given in Table 12.

A further selection is made based upon the presence of the core of the class II epitopes in the most prevalent genotypes. The "core" is defined as the central 9 (uneven amount of total amino acids) or 10 (even amount of total amino acids) amino acids of the total epitope sequence. As an example, the core (9aa) of the following epitope (15aa-uneven) is indicated in bold/underlined: ADL**MGYIPLVGA**PLG.

Accordingly, those peptides that are present in

- at least genotype 3a, or
- at least genotype 1b, or
- at least genotype 1a and 1b, or
- at least genotypes 1a, 1b and 3a, are retained for further testing. These peptides are summarized in table 14.

Furthermore, other HCV genotypes (e.g. genotype 4a) can be retained in view of prevalence and/or importance.

The relationship between binding affinity for HLA class I and II molecules and immunogenicity of discrete peptides or epitopes on bound antigens (HLA molecules) can be analyzed in two different experimental approaches (see, e.g., Sette et al, 1994). E.g. as for HLA-A0201, in the first approach, the immunogenicity of potential epitopes ranging in HLA binding affinity over a 10.000-fold range can be analyzed in HLA-A0201 transgenic mice. In the second approach, the antigenicity of approximately 100 different hepatitis B virus (HBV)-derived potential epitopes, all carrying A0201 binding motifs, was assessed by using PBL from acute hepatitis patients. Pursuant to these approaches, it was determined that an affinity threshold value of approximately 500 nM (preferably 50 nM or less) determines the capacity of a peptide epitope to elicit a CTL response. Said values are not yet available for other HLA Class I alleles.

These data are true for class I binding affinity measurements for naturally processed peptides and for synthesized T cell epitopes.

An affinity threshold associated with immunogenicity in the context of HLA class II DR molecules has also been delineated (see, e.g., Southwood et al.,1998). In order to define a biologically significant threshold of DR binding affinity, a database of the binding affinities of 32 DR- restricted epitopes for their restricting element (i.e., the HLA molecule that binds the motif) was compiled. In this case, 1000 nM can be defined as an affinity threshold associated with immunogenicity in the context of DR molecules.

The predicted binding affinity (Score) of the peptides of the current invention are indicated in Tables 1-11. The experimentally determined binding affinity or inhibition constant (Ki) of peptides for HLA molecules can be determined as described in Example 3. The inhibition constant (Ki) is the affinity of the peptide as determined in a competition experiment with labeled reference peptide. The Ki is calculated from the experimentally determined IC50 value according to the formula:

$$K_i = \frac{IC50}{1 + [Fl\text{-}pep] / Kd}$$

The binding affinities (Ki or IC50) of the peptides of the present invention to the respective HLA class I and II alleles are indicated in Tables 13 and 14.

"IC50" is the concentration of peptide in a binding assay at which 50% inhibition of binding of a reference peptide is observed. Throughout the specification, "binding data" results are often expressed in terms of IC50. Given the conditions in which the assays are run (i.e. limiting HLA proteins and labeled peptide concentrations), these values approximate Ki values. It should also be noted that the calculated Ki values are indicative values and are no absolute values as such, as these values depend on the quality/purity of the peptide/MHC preparations used and the type of non-linear regression used to analyze the binding data.

Binding may be determined using assay systems including those using:

live cells (e.g., Ceppellini et al., 1989; Christnick et al., 1991; Busch et al., 1990; Hill et al., 1991; del Guercio et al., 1995), cell free systems using detergent lysates (e.g., Cerundolo et al., 1991), immobilized purified MHC (e. g., Hill et al., 1994; Marshall et al., 1994), ELISA systems (e.g., Reay et al., 1992), surface plasmon resonance (e.g. Khilko et al., 1993); high flux soluble phase assays (Hammer et al., 1994), and measurement of class I MHC stabilization or assembly (e.g., Ljunggren et al., 1990; Schumacher et al., 1990; Townsend et al., 1990; Parker et al., 1992). The binding assays used in the present invention are demonstrated in Examples 3 and 4.

As used herein, "high affinity" or "strong binder" with respect to HLA class I and II molecules is defined as binding with a Ki or IC50 value of 100 nM or less; "intermediate affinity" or "mediate binder" is binding with a Ki or IC50 value of between about 100 and about 1000 nM.

As used herein, "threshold affinity" is the minimal affinity a peptide needs to display for a given HLA type that assures immunogenicity with high certainty in humans and/or animals. The threshold affinity can - but must not - be different for different HLA types.

Based on the data derived from the binding experiments, a further selection of candidate epitopes is made. Higher HLA binding affinity is typically correlated with higher immunogenicity. Higher immunogenicity can be manifested in several different ways. Immunogenicity corresponds to whether an immune response is elicited at all, and to the vigor of any particular response, as well as to the extent of a population in which a response is elicited. For example, a peptide might elicit an immune response in a diverse array of the population, yet in no instance produce a vigorous response. In accordance with these principles, close to 90% of high affinity binding peptides have been found to be immunogenic, as contrasted with about 50% of the peptides that bind with intermediate affinity. Moreover, higher binding affinity peptides lead to more vigorous immunogenic responses. As a result, less peptide is required to elicit a similar biological effect if a high affinity binding peptide is used. Thus, in preferred embodiments of the invention, high affinity binding peptides (strong binders) and medium affinity peptides (medium binders) are particularly useful.

Various strategies can be utilized to evaluate immunogenicity, including:

1) Evaluation of primary T cell cultures from normal individuals (see, e. g., Wentworth et al., 1995; Celis et al., 1994; Tsai et al., 1997; Kawashima et al., 1998). This procedure involves the stimulation of peripheral blood lymphocytes (PBL) from normal subjects with a test peptide in the presence of antigen presenting cells in vitro over a period of several weeks. T cells specific for the peptide become activated during this time and are detected using, e.g., a $^{51}$Cr-release assay involving peptide sensitized target cells.

2) Immunization of HLA transgenic mice (see, e.g., Wentworth et al., 1996; Wentworth et al., 1996; Alexander et al., 1997). In this method, peptides in incomplete Freund's adjuvant are administered subcutaneously to HLA transgenic mice. Several weeks following immunization, splenocytes are removed and cultured in vitro in the presence of test peptide for approximately one week. Peptide-specific T cells are detected using, e.g., a $^{51}$Cr-release assay involving peptide sensitized target cells and target cells expressing endogenously generated antigen.

3) Demonstration of recall T cell responses from immune individuals who have effectively been vaccinated, recovered from infection, and/or from chronically infected patients (see, e.g., Rehermann et al., 1995; Doolan et al., 1997; Bertoni et al., 1997; Threlkeld et al., 1997; Diepolder et al., 1997). In applying this strategy, recall responses are detected by culturing PBL from subjects that have been naturally exposed to the antigen, for instance through infection, and thus have generated an immune response "naturally", or from patients who were vaccinated with a vaccine comprising the peptide against the infection. PBL from subjects are cultured in vitro for 1-2 weeks in the presence of test peptide plus antigen presenting cells (APC to allow activation of "memory" T cells, as compared to "naive" T cells). At the end of the culture period, T cell activity is detected using assays for T cell activity including $^{51}$Cr release involving peptide-sensitized targets, T cell proliferation, or lymphokine release.

A given epitope is stated to be immunogenic if T cell activity can be shown to targets sensitized with that peptide. Immunogenicity for a given epitope can further be described by the number of individuals in a group of HLA matched infected subjects (e.g. human, transgenic mice) that show T cell reactivity to that particular epitope, or by the number of spots detected in an ELISPOT assay as described in example 6.

Based on the data derived from one of these experiments, a further selection of candidate epitopes is made according to their immunogenicity. Immunogenicity for the peptides of the invention is indicated in Tables 13 and 14. A "+" indicates T cell reactivity in at least one subject.

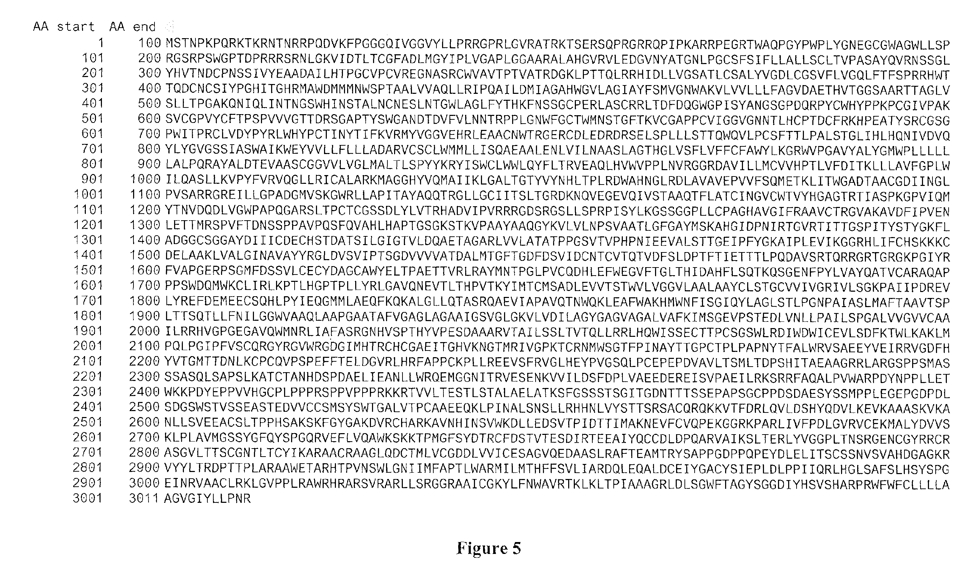

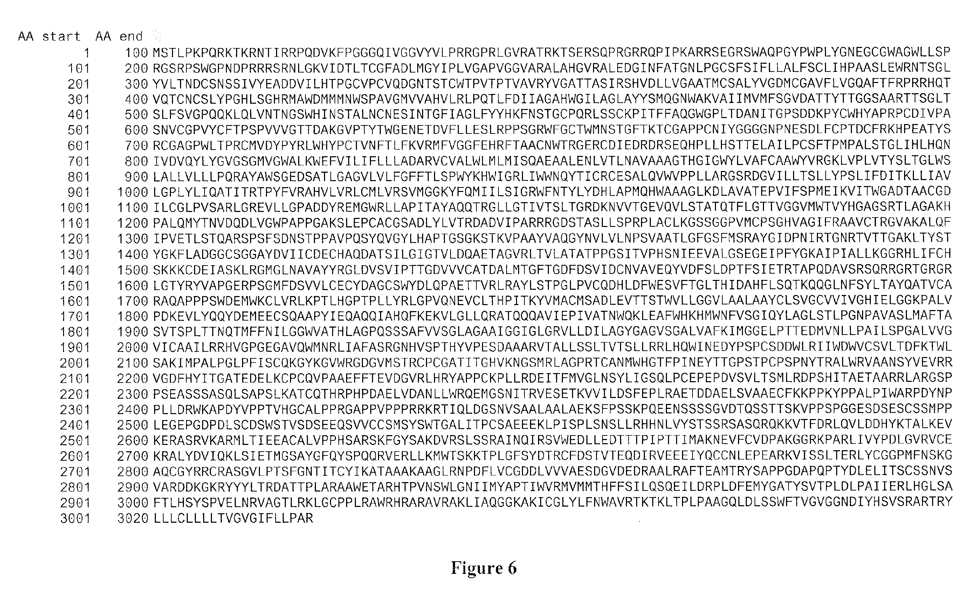

Vaccines having a broad coverage of the existing HCV genotypes or subtypes are preferred. Genotypes 1b, 1a and 3 a are the most prevalent HCV genotypes (among HCV infected individuals) and thus important to be taken into consideration. Other genotypes (e.g. genotype 4a) can be retained in view of their prevalence and/or importance. The present invention contains all selected CTL and HTL epitopes for which immunogenicity has been shown and that are present in the consensus sequence of genotype 1b, 1a and/or genotype 3 a. Said consensus sequences are shown in Figures 2, 5 and 6. Accordingly, the peptides of the present invention are present in the consensus sequence of:

- at least genotype 1a,
- at least genotype 1b,
- at least genotype 3a,
- at least genotype 1a and 1b,
- at least genotype 1a and 3a,
- at least genotype 1b and 3a, or
- at least genotype 1a, 1b and 3a.

The epitopes obtained by the methods as described herein can additionally be evaluated on the basis of their conservancy among and/or within different HCV strains or genotypes.

In a further step of the invention, an array of epitopes is selected for inclusion in a polyepitopic composition for use in a vaccine, or for selecting discrete epitopes to be included in a vaccine and/or to be encoded by nucleic acids such as a minigene. It is preferred that each of the following principles are balanced in order to make the selection:

1) Selection of either HCV native or analoged epitopes.
2) Selection of native HCV epitopes that are present in the most prevalent and/or important HCV genotypes or subtypes.
3) Epitopes are selected that have the requisite binding affinity established to be correlated with immunogenicity: for HLA class I an IC50 or Ki of 1000 nM or less, or for HLA class II an IC50 or Ki of 1000 nM.
4) Epitopes are selected which, upon administration, induce a T cell response (CTL and/or HTL).
5) Sufficient supermotif bearing-peptides and/or a sufficient array of allele-specific peptides are selected to give broad population coverage. It is a serious hurdle to find, for a given pathogen with a specific sequence, enough immunogenic epitopes so as to cover a complete HLA-locus and consequently a complete population. As such, considering immunogenic peptides for two or three HLA class I loci, i.e. HLA-A, -B and/or -C, significantly increases population coverage for a given pathogen.
6) Of relevance are epitopes referred to as "nested epitopes". Nested epitopes occur where at least two epitopes overlap partly or completely in a given peptide sequence. A nested peptide sequence can comprise both HLA class I and HLA class II epitopes, 2 or more HLA class I epitopes or 2 or more HLA class II epitopes.
7) It is important to screen the epitope sequence (e.g. comparing with mammal genome sequence) in order to ensure that it does not have pathological or other deleterious biological properties in the treated subject e.g. by inducing auto-antibodies.

8) When used in a polyepitopic composition, spacer amino acid residues can be introduced to avoid junctional epitopes (an epitope recognized by the immune system, not present in the target antigen, and only created by the man-made juxtaposition of epitopes), or to facilitate cleavage between epitopes and thereby enhance epitope presentation. Junctional epitopes are generally to be avoided because the recipient may generate an immune response to that non-native epitope. Of particular concern is a junctional epitope that is a "dominant epitope." A dominant epitope may lead to such a strong response that immune responses to other epitopes are diminished or suppressed.

The term "peptide" is used interchangeably with "oligopeptide" and "polypeptide" and designates a series of amino acids, connected one to the other, typically by peptide bonds between the amino and carboxyl groups of adjacent amino acids. The preferred CTL-inducing peptides of the invention are 13 residues or less in length and usually consist of 8, 9, 10, 11 or 12 residues, preferably 9 or 10 residues. The preferred HLA class II binding peptides are less than 50 residues in length and usually consist of between 6 and 30 residues, more usually between 12 and 25, and often between 15 and 20 residues. More preferred, an HLA class II binding peptide consists of 12, 13, 14, 15, 16, 17, 18, 19, 20, 21, 22, 23, 24, 25 or more amino acid residues.

The peptides of the invention can be prepared by classical chemical synthesis. "Synthetic peptide" refers to a peptide that is man-made using such methods as chemical synthesis or recombinant DNA technology. The synthesis can be carried out in homogeneous solution or in solid phase. For instance, the synthesis technique in homogeneous solution which can be used is the one described by Houbenweyl in the book entitled "Methode der organischen chemie" (Method of organic chemistry) edited by E. Wunsh, vol. 15-I et II. THIEME, Stuttgart 1974. The polypeptides of the invention can also be prepared in solid phase according to the methods described by Atherton and Shepard in their book entitled "Solid phase peptide synthesis" (IRL Press, Oxford, 1989). The polypeptides according to this invention can also be prepared by means of recombinant DNA techniques as documented below.

Conservative substitutions may be introduced in these HCV polypeptides according to the present invention. The term "conservative substitution" as used herein denotes that one amino acid residue has been replaced by another, biologically similar residue. Peptides having conservative substitutions bind the HLA molecule with a similar affinity as the original peptide and CTL's and/or HTL's generated to or recognizing the original peptide are activated in the presence of cells presenting the altered peptide (and/or vice versa). Examples of conservative substitutions include the substitution of one hydrophobic residue such as isoleucine, valine, leucine or methionine for another, or the substitution of one polar residue for another such as between arginine and lysine, between glutamic and aspartic acids or between glutamine and asparagine and the like. Other substitutions can be introduced as long as the peptide containing said one or more amino acid substitutions is still immunogenic. This can be analysed in ELISPOT assays as described in examples 5 and 6. Accordingly, the current invention also relates to a peptide consisting of an amino acid sequence which is at least 70, 75, 80, 85 or 90% identical to the amino acid sequence of the peptide as disclosed in Tables 13 and 14, and wherein said peptide is still capable of inducing a HLA class I and/or class II restricted T lymphocyte response to cells presenting the original peptides.

A strategy to improve the cross-reactivity of peptides between different HLA types or within a given supermotif or allele is to delete one or more of the deleterious residues present within a peptide and substitute a small "neutral" residue such as Ala, that may not influence T cell recognition of the peptide. Such an improved peptide is sometimes referred to as an analoged peptide.

The peptides can be in their natural (uncharged) forms or in forms which are salts, and either free of modifications such as glycosylation, side chain oxidation, or phosphorylation or containing these modifications. Also included in the definition are peptides modified by additional substituents attached to the amino acids side chains, such as glycosyl units, lipids, or inorganic ions such as phosphates, as well as modifications relating to chemical conversions of the chains, such as oxidation of sulfhydryl groups. Thus, "polypeptide" or its equivalent terms is intended to include the appropriate amino acid sequence referenced, and may be subject to those of the foregoing modifications as long as its functionality is not destroyed.

With regard to a particular amino acid sequence, an "epitope" is a set of amino acid residues which is involved in recognition by a particular immunoglobulin, or in the context of T cells, those residues necessary for recognition by T cell receptor proteins and/or Major Histocompatibility Complex (MHC) molecules. In an immune system setting, in vivo or in vitro, an epitope is the collective features of a molecule, such as primary, secondary and tertiary peptide structure, and charge, that together form a site recognized by an immunoglobulin, T cell receptor or HLA molecule. Throughout this specification "epitope" and "peptide" are used interchangeably.

The phrases "isolated" or "biologically pure" refer to material which is substantially or essentially free from components which normally accompany the material as it is found in its native state. Thus, isolated peptides in accordance with the invention preferably do not contain materials normally associated with the peptides in their in situ environment. An "isolated" epitope refers to an epitope that does not include the whole sequence of the antigen or polypeptide from which the epitope was derived.

It is to be understood that protein or peptide molecules that comprise an epitope of the invention as well as additional amino acid(s) are still within the bounds of the invention.

An "immunogenic peptide" is a peptide that comprises a sequence as disclosed in Tables 13 and 14, or a peptide comprising an allele-specific motif or supermotif, such that the peptide will bind an HLA molecule and induce a CTL and/or HTL response. Thus, immunogenic peptides of the invention comprise a peptide capable of binding to an appropriate HLA molecule and the immunogenic peptide can induce an HLA- restricted cytotoxic and/or helper T cell response to the antigen from which the immunogenic peptide is derived. In a preferred embodiment of the invention, the immunogenic peptide consists of less than 50 amino acid residues. Even more particularly, the immunogenic peptide consists of less than 45, 40, 35, 30, 29, 28, 27, 26, 25, 24, 23, 22, 21, 20, 19, 18, 17, 16, 15, 14, 13, 12, 11, 10 or 9 amino acid residues.

Sette and Sidney (1999) (incorporated herein by reference) describe the epitope approach to vaccine development and identified several HLA supermotifs, each of which corresponds to the ability of peptide ligands to bind several different HLA alleles. The HLA allelic variants that bind peptides possessing a particular HLA supermotif are collectively referred to as an HLA supertype.

A "supermotif" is a peptide binding specificity shared by HLA molecules encoded by two or more HLA alleles. Preferably, a supermotif-bearing peptide is recognized with high or intermediate affinity (as defined herein) by two or more HLA antigens. The term "motif" refers to the pattern of residues in a peptide of defined length, usually a peptide of 8, 9, 10, 11, 12 or 13 amino acids for a class I HLA motif and from about 6 to about 50 amino acids, or more specific a peptide of 13, 14, 15, 16, 17, 18, 19, 20, 21, 22, 22, 24, 25, 30, 35, 40 or 50 amino acids for a class II HLA motif, which is recognized by a particular HLA molecule. According to a preferred embodiment, the immunogenic peptide of the present invention is less than 50, less than 25, less than 20 or less than 15 amino acids. Peptide motifs are typically different for each protein encoded by each human HLA allele and differ in the pattern of the primary and secondary anchor residues. "Cross-reactive binding" indicates that a peptide is bound by more than one HLA molecule derived from more than one HLA allele group or locus; a synonym is degenerate binding.

"Human Leukocyte Antigen" or "HLA" is a human class I or class II Major Histocompatibility Complex (see, e.g., Stites, et al, IMMUNOLOGY, 8 ED, Lange Publishing, Los Altos, CA (1994)). "Major Histocompatibility Complex" or "MHC" is a cluster of genes that plays a role in control of the cellular interactions responsible for physiologic immune responses. In humans, the MHC complex is also known as the HLA complex. For a detailed description of the MHC and HLA complexes, see, Paul, FUNDAMENTAL IMMUNOLOGY, PDED, Raven Press, New York, 1993. The HLA nomenclature used herein is generally known in the art and e.g. as described in "The HLA Factsbook, ed. Marsh et al., Academic Press, 2000".

Also, information on HLA sequences and the currently used nomenclature can be found on http://www.anthonynolan.org.uk/HIG/.

### Polyepitopic peptides

The present invention also relates to the use of the peptides as described herein for the preparation of an HCV immunogenic composition and more specific to a composition comprising at least one of the peptides as provided in Tables 13-14, possibly in combination with one or more of the same or other peptide or epitope. The peptides of the invention can be combined via linkage to form polymers (multimers), or can be formulated in a composition without linkage, as an admixture. In a specific embodiment, the peptides of the invention can be linked as a polyepitopic peptide. Accordingly, the present invention relates to a composition or polyepitopic peptide comprising at least one peptide selected from the peptides disclosed in Tables 13 and 14. Of particular interest are the peptides with Ki or IC50 <1000 nM. More preferably, the peptides of interest are these peptides having a positive immunogenicity after evaluation by the herein described strategies.

More preferably, the composition or polyepitopic peptide comprises at least two, three, four, five or more peptides. Preferably, the peptides are selected from Tables 13 and 14. Any combination of peptides is possible, e.g., the composition can comprise at least one HLA-A binding peptide and at least one HLA-B or HLA-C binding peptide. Furthermore, the composition can also comprise at least one HLA-B binding peptide and at least one HLA-C binding peptide. More specific, the composition comprises at least one HLA-A, at least one HLA-B and at least one HLA-C binding peptide. In a preferred embodiment, the polyepitopic peptide or composition comprises at least two peptides derived from a HCV protein and capable of inducing a HLA class I and/or class II restricted T lymphocyte response, wherein at least one peptide is a HLA-C binding peptide.

A "HLA-A binding peptide" is defined as a peptide capable of binding at least one molecule of the HLA-A locus. Said definition can be extrapolated to the other loci, i.e. HLA-B, HLA-C, HLA-DRB1-9, etc.

In a particular, the epitopes of the invention can be combined in an HLA-group restricted polyepitope. The term "HLA-group restricted polyepitope" refers to a polyepitopic peptide comprising at least two epitopes binding to an allele or molecule of the same HLA group.

The HLA nomenclature used herein is generally known in the art and e.g. as described in "The HLA Factsbook, ed.

Marsh et al., Academic Press, 2000". In a preferred embodiment, the HLA-group restricted polyepitope is a HLA-A01 restricted polyepitope, a HLA-A02 restricted polyepitope, a HLA-A03 restricted polyepitope, a HLA-A11 restricted polyepitope, a HLA-A24 restricted polyepitope, a HLA-B07 restricted polyepitope, a HLA-B08 restricted polyepitope, a HLA-B35 restricted polyepitope, a HLA-B40 restricted polyepitope, a HLA-B44 restricted polyepitope, a HLA-Cw3 restricted polyepitope, a HLA-Cw4 restricted polyepitope, a HLA-Cw6 restricted polyepitope or a HLA-Cw7 restricted polyepitope. The number of epitopes in a HLA-group restricted polyepitope is not limited and can be 2, 3, 4, 5, 6, 7, 8, 9, 10, 11, 12, 13, 14, 15, 20, 25 or more. An HLA-group restricted polyepitope can be used in a first phase of establishing the immunogenicity of a subset of epitopes in a construct. The advantage of using such an HLA-group restricted polyepitope is that a considerable number of HLA restricted epitopes can be evaluated in one and the same construct. Furthermore, a specific selection of more than one HLA-group restricted polyepitope can be administered in order to customize treatment. More specific, the selection can comprise more than one HLA-group restricted polyepitope within a given HLA-locus or covering 2, 3 or more HLA-loci. Optionally, the HLA-group restricted polyepitopes can be combined with one or more HTL epitope.

More particular, the composition as described herein comprises linked peptides that are either contiguous or are separated by a linker or a spacer amino acid or spacer peptide. This is referred to as a polyepitopic or multi-epitopic peptide.

"Link" or "join" refers to any method known in the art for functionally connecting peptides, including, without limitation, recombinant fusion, covalent bonding, non-covalent bonding, disulfide bonding, ionic bonding, hydrogen bonding, polymerization, cyclization, electrostatic bonding and connecting through a central linker or carrier. Polymerization can be accomplished for example by reaction between glutaraldehyde and the -NH2 groups of the lysine residues using routine methodology. The peptides may also be linked as a branched structure through synthesis of the desired peptide directly onto a central carrier, e.g. a polylysyl core resin.

This larger, preferably poly- or multi-epitopic, peptide can be generated synthetically, recombinantly, or via cleavage from the native source. The polyepitopic peptide can exist as a homopolymer comprising multiple copies of the same peptide, or as a heteropolymer of various peptides. Polymers have the advantage of increased immunological reaction and, where different peptide epitopes are used to make up the polymer, the additional ability to induce antibodies and/or CTLs that react with different antigenic determinants of the pathogenic organism targeted for an immune response. Multi-epitope constructs can for example be prepared according to the methods set forth in Ishioka et al., 1999; Velders et al., 2001; or as described in WO04/031210 - Epimmune. The polyepitopic peptide can be expressed as one protein. In order to carry out the expression of the polyepitopic peptide in bacteria, in eukaryotic cells (including yeast) or in cultured vertebrate hosts such as Chinese Hamster Ovary (CHO), Vero cells, RK13, COS1, BHK, and MDCK cells, or invertebrate hosts such as insect cells, the following steps are carried out:

- transformation of an appropriate cellular host with a recombinant vector, or by means of adenoviruses, influenza viruses, BCG, and any other live carrier systems, in which a nucleotide sequence coding for one of the polypeptides of the invention has been inserted under the control of the appropriate regulatory elements, particularly a promoter recognized by the polymerases of the cellular host or of the live carrier system and in the case of a prokaryotic host, an appropriate ribosome binding site (RBS), enabling the expression in said cellular host of said nucleotide sequence,
- culture of said transformed cellular host under conditions enabling the expression of said insert.

The polyepitopic peptide can be purified by methods well known to the person skilled in the art.

Vaccines that have broad population coverage are preferred because they are more commercially viable and generally applicable to most people. Broad population coverage can be obtained through selecting peptides that bind to HLA alleles which, when considered in total, are present in most of the individuals of the population. The A2-, A3-, and B7 supertypes are each present on the average of over 40% in each of the five major ethnic groups, i.e. Caucasian, North American Black, Japanese, Chinese and Hispanic. Coverage in excess of 80% is achieved with a combination of these supermotifs. The B44-, Al-, and A24-supertypes are present, on average, in a range from 25% to 40% in these major ethnic populations. The class HLA types Cw04, Cw03, Cw06 and Cw07 are each present, on average, in a range from 13% to 54% in these major ethnic populations. Thus, by including epitopes from most frequent HLA-A, -B and/or -C types, an average population coverage of 90-99% is obtained for five major ethnic groups. Especially in the field of HLA-C, experimentally determined data (both binding and immunogenic) for HCV epitopes are scarce. Accordingly, the present invention relates to a composition or polyepitopic peptide comprising at least two peptides derived from a HCV protein and capable of inducing a HLA class I and/or class II restricted T lymphocyte response, wherein at least one peptide is a HLA-C binding peptide. More preferred, said composition or polyepitopic peptide comprises at least 2, 3, 4, 5 or more HLA-C binding peptide(s). More particularly, the one or more HLA-C binding peptides are derived from at least one of the following HCV regions: Core, E1, E2/NS1, NS2, NS3, NS4A, NS4B, NS5A and NS5B. Even more preferred is that the HLA-C binding peptides are furthermore characterized in that they are present in the HCV consensus sequence of genotype 1a, 1b and/or 3a. Optionally, the composition or polyepitopic peptide can furthermore comprise at least 1, 2, 3, 4 or more HLA-B binding peptide(s) and/or at least 1, 2, 3, 4 or more HLA-A binding peptide(s) and/or at least 1, 2, 3, 4 or more HLA-DRB1-9 binding peptide(s). More preferred, the composition or the polyepitopic peptide of the present invention comprises at least 1, 2, 3, 4 or more HLA-A binding peptide(s), at least 1, 2, 3, 4 or more HLA-B binding peptide(s) and at least 1, 2, 3, 4 or more HLA-C binding peptide(s), optionally in combination with a HLA class II binding peptide. In a specific embodiment, the peptides are selected from Table 13 or 14.

Furthermore, the present invention relates to a composition comprising at least one peptide selected from Tables 13 and 14 and at least one other HLA class I binding peptide, a HLA class II binding peptide or a HCV derived peptide. Said "other" HLA class I binding peptide and said HLA class II binding peptide to be used in combination with the peptides of the present invention can be derived from HCV or from a foreign antigen or organism (non-HCV). There is no limitation on the length of said other peptides, these can have a length of e.g. 5, 6, 7, 8, 9, 10, 11, 12, 13, 14, 15, 16; 17, 18, 19, 20, 21, 22, 23, 24, 25, 30 or more amino acids. The "at least one" can include, e.g., at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95 or 100 or more peptides. Preferably, said HLA class I binding peptide is a peptide capable of binding one or more HLA class I alleles. More specific, said peptide is selected from the group consisting of peptides binding a molecule of the following HLA groups: HLA-A1, HLA-A2, HLA-A3, HLA-A11, HLA-A24, HLA-B7, HLA-B8, HLA-B27, HLA-B35, HLA-B40, HLA-B44, HLA-B58, HLA-B62, HLA-Cw3, HLA-Cw4, HLA-Cw6 and/or HLA-Cw7.

For HLA class II, the peptides, also called HTL epitopes, are preferably selected from the group consisting of peptides binding a molecule of the HLA-loci HLA-DR, HLA-DQ and/or HLA-DP, or as described in e.g. WO95/27733, WO02/26785, WO01/21189, WO02/23770, WO03/084988, WO04/024182, Hoffmann et al., 1995, Diepolder et al., 1997, Werheimer et al, 2003 and Lamonaca et al, 1999 (incorporated herein by reference). The preferred HLA class II binding peptides are less than about 50 residues in length and usually consist of between about 6 and about 30 residues, more usually between about 12 and 25, and often between about 15 and 20 residues. For example, a HLA class II binding peptide consists of 12, 13, 14, 15, 16, 17, 18, 19, 20, 21, 22, 23, 24, 25 or more amino acid residues. Further and preferred examples of candidate HTL epitopes to include in a polyepitopic construct for use in a vaccine, or for selecting discrete epitopes to be included in a vaccine and/or to be encoded by nucleic acids such as a minigene are enclosed in Table 14. A "CTL inducing peptide" is a HLA Class I binding peptide that is capable of inducing a CTL response. A "HTL inducing peptide" is a HLA Class II binding peptide that is capable of inducing a HTL response.

In a specific embodiment, the present invention relates to a composition or polyepitopic peptide comprising at least two HLA class I binding peptides selected from Table 13. Any combination is possible. More preferred, the at least two peptides are selected to bind HLA molecules derived from the same or a different HLA locus, i.e. HLA-A, -B and/or -C. Alternatively, the at least two peptides are selected to bind HLA molecules derived from the same or a different HLA-group. Preferred HLA-groups are: HLA-A01, A02, A03, A11, A24, B07, B08, B35, B40, B44, Cw3, Cw4, Cw6 and Cw7.

In a more preferred embodiment, the present invention relates to a composition or polyepitopic peptide comprising at least three HLA class I binding peptides selected from Table 13. Any combination is possible, for example:

- at least 3 HLA-A binding peptides,
- at least 3 HLA-B binding peptides,
- at least 3 HLA-C binding peptides,
- at least 2 HLA-A binding peptides and at least 1 HLA-B or HLA-C binding peptide,
- at least 2 HLA-B binding peptides and at least 1 HLA-A or HLA-C binding peptide,
- at least 2 HLA-C binding peptides and at least 1 HLA-A or HLA-B binding peptide, or
- at least one HLA-A, at least one HLA B and at least one HLA-C binding peptide.

More preferred and for each combination, the at least three peptides are selected to bind HLA molecules derived from the same or a different HLA-group. Preferred HLA-groups are:
HLA-A01, A02, A03, A11, A24, B07, B08, B35, B40, B44, Cw3, Cw4, Cw6 and Cw7.
More specifically, the composition or polyepitopic peptide comprises at least three peptides selected from Table 13, said at least three peptides being:

- at least one HLA-A binding peptide selected from a HLA-A01, A02, A3, A11 or A24 binding peptide,
- at least one HLA-B binding peptide selected from a HLA-B07, B08, B35, B40 or B44 binding peptide, and/or
- at least one HLA-C binding peptide selected from a HLA-Cw3, Cw4, Cw6 or Cw7 binding peptide.

An HLA-A01 binding peptide is defined as a peptide capable of binding at least one molecule of the HLA-01 group. Said definition can be extrapolated to the other allele groups, i.e. A02, A03, A11, A24, B07, B08, B35, B40, B44, Cw3, Cw4, Cw6, Cw7 etc.
HLA class I binding peptides of the invention can be admixed with, or linked to, HLA class II binding peptides, to facilitate activation of both cytotoxic T lymphocytes and helper T lymphocytes. Accordingly, the composition or polyepitopic peptide of the present invention further comprises at least one HLA class II binding peptide. More specific, said HLA class II binding peptide is selected from Table 14. The amount of HTL epitopes is not limiting, i.e., 1, 2, 3, 4, 5, 6, 7, 8, 9, 10, 11, 12, 13, 14, 15, 16, 17, 18, 19, 20, 21, 22, 23, 24, 25 or more HTL epitopes can be comprised in the composition or polyepitopic peptide of the present invention. In a specific embodiment, the composition or polyepitopic peptide comprises at least three CTL peptides selected from Table 13 and at least one HTL peptide selected from Table 14.

In a further embodiment, the composition or polyepitopic peptide can also comprise the universal T cell epitope called PADRE® (Epimmune, San Diego; described, for example in US Patent 5736142 or International Application WO95/07707, which are enclosed herein by reference). A 'PanDR binding peptide or PADRE® peptide" is a member of a family of molecules that binds more that one HLA class II DR molecule. The pattern that defines the PADRE® family of molecules can be thought of as an HLA Class II supermotif. PADRE® binds to most HLA-DR molecules and stimulates in vitro and in vivo human helper T lymphocyte (HTL) responses. Alternatively T-help epitopes can be used from universally used vaccines such as tetanos toxoid.

In a further embodiment, the peptides in the composition or polyepitopic peptide are characterized in that they are derived from a HCV protein, and more specific from at least one of the following HCV regions selected from the group consisting of Core, E1, E2/NS1, NS2, NS3, NS4A, NS4B, NS5A and NS5B. Even more preferred is that peptides are characterized in that they are present in the HCV consensus sequence of genotype 1a, 1b and/or 3 a.

In a further embodiment the two or more epitopes in the polyepitopic peptide consist of discrete HCV amino acid sequences or nested HCV amino acid sequences. Particularly preferred are "nested epitopes". Nested epitopes occur where at least two epitopes overlap partly or completely in a given peptide sequence. A nested peptide sequence can comprise both HLA class I and HLA class II epitopes, 2 or more HLA class I epitopes or 2 or more HLA class II epitopes.

The peptides or polypeptides can optionally be modified, such as by lipidation (e.g. a peptide joined to a lipid), addition of targeting or other sequences.

In the HCV peptides as described herein, one cysteine residue, or 2 or more cysteine residues comprised in said peptides may be "reversibly or irreversibly blocked".

An "irreversibly blocked cysteine" is a cysteine of which the cysteine thiol-group is irreversibly protected by chemical means. In particular, "irreversible protection" or "irreversible blocking" by chemical means refers to alkylation, preferably alkylation of a cysteine in a protein by means of alkylating agents, such as, for example, active halogens, ethylenimine or N-(iodoethyl)trifluoro-acetamide. In this respect, it is to be understood that alkylation of cysteine thiol-groups refers to the replacement of the thiol-hydrogen by $(CH_2)_nR$, in which n is 0, 1, 2, 3 or 4 and R= H, COOH, $NH_2$, $CONH_2$, phenyl, or any derivative thereof. Alkylation can be performed by any method known in the art, such as, for example, active halogens $X(CH_2)_nR$ in which X is a halogen such as I, Br, Cl or F. Examples of active halogens are methyliodide, iodoacetic acid, iodoacetamide, and 2-bromoethylamine.

A "reversibly blocked cysteine" is a cysteine of which the cysteine thiol-groups is reversibly protected. In particular, the term "reversible protection" or "reversible blocking" as used herein contemplates covalently binding of modification agents to the cysteine thiol-groups, as well as manipulating the environment of the protein such, that the redox state of the cysteine thiol-groups remains (shielding). Reversible protection of the cysteine thiol-groups can be carried out chemically or enzymatically. The term "reversible protection by enzymatical means" as used herein contemplates reversible protection mediated by enzymes, such as for example acyl-transferases, e.g. acyl-transferases that are involved in catalysing thio-esterification, such as palmitoyl acyltransferase. The term "reversible protection by chemical means" as used herein contemplates reversible protection:

1. by modification agents that reversibly modify cysteinyls such as for example by sulphonation and thio-esterification;
2. by modification agents that reversibly modify the cysteinyls of the present invention such as, for example, by heavy metals, in particular $Zn^{2+}$, $Cd^{2+}$, mono-, dithio- and disulfide-compounds (e.g. aryl- and alkylmethanethiosulfonate, dithiopyridine, dithiomorpholine, dihydrolipoamide, Ellmann reagent, aldrothiol™ (Aldrich) (Rein et al. 1996), dithiocarbamates), or thiolation agents (e.g. gluthathion, N-Acetyl cysteine, cysteineamine). Dithiocarbamate comprise a broad class of molecules possessing an $R_1R_2NC(S)SR_3$ functional group, which gives them the ability to react with sulphydryl groups. Thiol containing compounds are preferentially used in a concentration of 0,1-50 mM, more preferentially in a concentration of 1-50 mM, and even more preferentially in a concentration of 10-50 mM;
3. by the presence of modification agents that preserve the thiol status (stabilise), in particular antioxidantia, such as for example DTT, dihydroascorbate, vitamins and derivates, mannitol, amino acids, peptides and derivates (e.g. histidine, ergothioneine, carnosine, methionine), gallates, hydroxyanisole, hydoxytoluene, hydroquinon, hydroxymethylphenol and their derivates in concentration range of 10 μM-10 mM, more preferentially in a concentration of 1-10 mM;
4. by thiol stabilising conditions such as, for example, (i) cofactors as metal ions ($Zn^{2+}$, $Mg^{2+}$), ATP, (ii) pH control (e.g. for proteins in most cases pH ~5 or pH is preferentially thiol $pK_a$ -2; e.g. for peptides purified by Reversed Phase Chromatography at pH ~2). Combinations of reversible protection as described in (1), (2), (3) and (4) may be applied.

The reversible protection and thiol stabilizing compounds may be presented under a monomeric, polymeric or liposomic form.

The removal of the reversibly protection state of the cysteine residues can chemically or enzymatically accomplished by e.g.:

- a reductant, in particular DTT, DTE, 2-mercaptoethanol, dithionite, $SnCl_2$, sodium borohydride, hydroxylamine, TCEP, in particular in a concentration of 1-200 mM, more preferentially in a concentration of 50-200 mM;
- removal of the thiol stabilising conditions or agents by e.g. pH increase;
- enzymes, in particular thioesterases, glutaredoxine, thioredoxine, in particular in a concentration of 0,01-5 $\mu$M, even more particular in a concentration range of 0,1-5 $\mu$M.;
- combinations of the above described chemical and/or enzymatical conditions.

The removal of the reversibly protection state of the cysteine residues can be carried out *in vitro* or *in vivo,* e.g. in a cell or in an individual.

Alternatively, one cysteine residue, or 2 or more cysteine residues comprised in the HCV peptides as described herein may be mutated to a natural amino acid, preferentially to methionine, glutamic acid, glutamine or lysine.

The peptides of the invention can be combined via linkage or via a spacer amino acid to form polymers (multimers: homopolymers or heteropolymers), or can be formulated in a composition without linkage, as an admixture. The "spacer amino acid" or "spacer peptide" is typically comprised of one or more relatively small, neutral molecules, such as amino acids or amino acid mimetics, which are substantially uncharged under physiological conditions. The spacers are typically selected from, e.g., Ala, Gly, Leu, Ile, or other neutral spacers of nonpolar amino acids or neutral polar amino acids. It will be understood that the optionally present spacer need not be comprised of the same residues and thus may be a hetero- or homo-oligomer. When present, the spacer will be at least 1 or 2 residues, more usually 3, 4, 5 or 6 residues, or even up to 7, 8, 9, 10, 15, 20, 30, or 50 residues. Spacer amino acid residues can be introduced to avoid junctional epitopes (an epitope recognized by the immune system, not present in the target antigen, and only created by the man-made juxtaposition of epitopes), or to facilitate cleavage between epitopes and thereby enhance epitope presentation.

Generally, the spacer sequence will include nonpolar amino acids, though polar residues such as Glu, Gln, Ser, His, and Asn could also be present, particularly for spacer sequences longer than three residues. The only outer limit on the total length and nature of each spacer sequence derives from considerations of ease of synthesis, proteolytic processing, and manipulation of the polypeptide and/or nucleic acid.

Moreover, the present invention also contemplates a polypeptide comprising or consisting of multiple repeats of any of the peptides as defined above or combinations of any of the peptides as defined above.

## Minigene

A further embodiment of the present invention relates to a nucleic acid encoding a peptide selected from Tables 13 and 14. Said nucleic acids are "isolated" or "synthetic". The term "isolated" refers to material that is substantially free from components that normally accompany it as found in its naturally occurring environment. However, it should be clear that the isolated nucleic acid of the present invention might comprise heterologous cell components or a label and the like. The terms "nucleic acid" or "polynucleic acid" are used interchangeable throughout the present application and refer to a deoxyribonucleotide or ribonucleotide polymer in either single- or double stranded form, which may encompass known analogues of natural nucleotides.

More particular, the present invention relates to a "minigene" or a polynucleotide that encodes a polyepitopic peptide as described herein. The term "multi-epitope construct" when referring to nucleic acids can be used interchangeably with the terms "polynucleotides", "minigene" and "multi-epitope nucleic acid vaccine," and other equivalent phrases, and comprises multiple epitope nucleic acids that encode peptide epitopes of any length that can bind to a molecule functioning in the immune system, preferably a HLA class I and a T-cell receptor or a HLA class II and a T-cell receptor. The epitope nucleic acids in a multi-epitope construct can encode HLA class I epitopes and/or HLA class II epitopes. HLA class I-encoding epitope nucleic acids are referred to as CTL epitope nucleic acids, and HLA class II-encoding epitope nucleic acids are referred to as HTL epitope nucleic acids. Some multi-epitope constructs can have a subset of the multi-epitope nucleic acids encoding HLA class I epitopes and another subset of the multi-epitope nucleic acids encoding HLA class II epitopes. A multi-epitope construct may have one or more spacer nucleic acids. A spacer nucleic acid may flank each epitope nucleic acid in a construct. The spacer nucleic acid may encode one or more amino acids (spacer amino acids).

Accordingly, the present invention relates to a polynucleotide or minigene encoding a polyepitopic peptide comprising at least one peptide selected from Tables 13 and 14.

Furthermore, the invention also encompasses a polynucleotide or minigene encoding a polyepitopic peptide comprising at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60 or more peptides. Preferably, the peptides are selected from Tables 13 and 14. Any combination of peptides is possible as described for the polyepitopic peptide.

More particular, the nucleic acids of the invention can be incorporated in an HLA-group restricted construct. Said "HLA-group restricted construct" comprises at least two nucleic acid epitopes encoding peptides binding to an allele or molecule of the same HLA group. The number of epitopes in a HLA-group restricted construct is not limited and can be 2, 3, 4, 5, 6, 7, 8, 9, 10, 11, 12, 13, 14, 15, 20, 25 or more. The same combinations are possible as described for the HLA-group restricted polyepitopic peptide.

In a preferred embodiment, the polyepitopic peptide encoded by the polynucleotide further comprises at least one HLA-class I binding peptide, a HLA class II binding peptide or a HCV derived peptide. Said HLA Class I binding peptide and said HLA Class II binding peptide can be derived from a foreign antigen or organism (non-HCV). There is no limitation on the length of said peptide, this can have a length of e.g. 5, 6, 7, 8, 9, 10, 11, 12, 13, 14, 15, 16, 17, 18, 19, 20, 21, 22, 23, 24, 25, 30 or more amino.

In a further embodiment, the polynucleotide or minigene as described herein can further comprise a spacer nucleic acid. In a particular embodiment, the minigene further comprises one or more regulatory sequences and/or one or more signal sequences and/or one or more promotor sequences.

Polynucleotides or nucleic acids that are not commercially available can be chemically synthesized according to the solid phase phosphoramidite triester method first described by Beaucage & Caruthers, 1981, using an automated synthesizer, as described in Van Devanter et. al., 1984. Purification of polynucleotides is by either native acrylamide gel electrophoresis or by anion-exchange HPLC as described in Pearson & Reanier, 1983. Other purification methods are reversed phase separation and hydroxyapatite and are well known to the skilled person.

The epitopes of the multi-epitope constructs are typically subcloned into an expression vector that contains a promoter to direct transcription, as well as other regulatory sequences such as enhancers and polyadenylation sites. Suitable promoters are well known in the art and described, e.g., in Sambrook et al., Molecular cloning, A Laboratory Manual (2nd ed. 1989) and in Ausubel et al, Current Protocols in Molecular Biology (1994). Eukaryotic expression systems for mammalian cells are well known in the art and are commercially available. Such promoter elements include, for example, cytomegalovirus (CMV), Rous sarcoma virus long terminal repeats (RSV LTR) and Simian Virus 40 (SV40). See, e.g., U.S. Patent Nos. 5,580,859 and 5,589,466 for other suitable promoter sequences.

For therapeutic or prophylactic immunization purposes, the (polyepitopic) peptides of the invention can be expressed by plasmid vectors as well as viral or bacterial vectors as already described herein. The term "vector" may comprise a plasmid, a cosmid, a phage, or a virus or an animal cell. The expression vector typically contains a transcription unit or expression cassette that contains all the additional elements required for the expression of the multi-epitope construct in host cells. A typical expression cassette thus contains a promoter operably linked to the multi-epitope construct and signals required for efficient polyadenylation of the transcript.

Additional elements of the cassette may include enhancers and introns with functional splice donor and acceptor sites. In addition to a promoter sequence, the expression cassette can also contain a transcription termination region downstream of the structural gene to provide for efficient termination. The termination region may be obtained from the same gene as the promoter sequence or may be obtained from different genes.

## Medical use

In a further embodiment, the present invention also relates to the (polyepitopic) peptide, nucleic acid, minigene or composition of the present invention for use as a medicament. Preferably, said medicament is a vaccine. In a specific embodiment the invention also relates to a vector, a plasmid, a recombinant virus or host cell comprising the nucleic acid or minigene as described herein for use a medicament. More specifically, the present invention relates to the use of at least one of the peptides selected from Tables 13 and 14 or the nucleic acid sequence encoding said peptide for the manufacture of a medicament for preventing or treating a HCV infection. In a specific embodiment the invention also relates to a vector, a plasmid, a recombinant virus or host cell comprising the nucleic acid or minigene as described herein for the manufacture of a medicament for preventing or treating a HCV.

## Vaccines and vaccine compositions

The invention furthermore relates to compositions comprising any of the HCV (polyepitopic) peptides as described herein or the corresponding nucleic acids. In a specific embodiment, the composition furthermore comprises at least one of a pharmaceutically acceptable carrier, adjuvant or vehicle. The terms "composition", "immunogenic composition" and "pharmaceutical composition" are used interchangeable with "vaccine composition" or "vaccine". There are numerous embodiments of vaccines in accordance with the invention, such as by a cocktail of one or more peptides, one or more epitopes of the invention comprised in a polyepitopic peptide, and/or nucleic acids that encode such peptides or polypeptides, e.g., a minigene that encodes a polyepitopic peptide. Vaccines can also comprise peptide- pulsed antigen presenting cells, e.g., the epitope can be bound to an HLA molecule on dendritic cells. More particularly, said immunogenic composition is a vaccine composition. Even more particularly, said vaccine composition is a prophylactic vaccine composition. Alternatively, said vaccine composition may also be a therapeutic vaccine composition. The prophylactic vaccine composition refers to a vaccine composition aimed for preventing HCV infection and to be administered to healthy persons who are not yet infected with HCV. The therapeutic vaccine composition refers to a vaccine composition aimed for treatment of HCV infection and to be administered to patients being infected with HCV.

A vaccine or vaccine composition is an immunogenic composition capable of eliciting an immune response sufficiently broad and vigorous to provoke one or both of:

- a stabilizing effect on the multiplication of a pathogen already present in a host and against which the vaccine composition is targeted; and
- an increase of the rate at which a pathogen newly introduced in a host, after immunization with a vaccine composition targeted against said pathogen, is resolved from said host.

A vaccine composition may also provoke an immune response broad and strong enough to exert a negative effect on the survival of a pathogen already present in a host or broad and strong enough to prevent an immunized host from developing disease symptoms caused by a newly introduced pathogen. A vaccine composition may also induce an immune response in a host already infected with the pathogen against which the immune response leading to stabilization, regression or resolving of the disease. In particular the vaccine composition of the invention is a HCV vaccine composition. In particular, the vaccine or vaccine composition comprises an effective amount of the peptides or nucleic acids of the present invention. In a specific embodiment, said vaccine composition comprises a vector, a plasmid, a recombinant virus or host cell comprising the nucleic acid or minigene of the present invention. Said vaccine composition may additionally comprise one or more further active substances and/or at least one of a pharmaceutically acceptable carrier, adjuvant or vehicle.

An "effective amount" of a peptide or nucleic acid in a vaccine or vaccine composition is referred to as an amount required and sufficient to elicit an immune response. It will be clear to the skilled artisan that the immune response sufficiently broad and vigorous to provoke the effects envisaged by the vaccine composition may require successive (in time) immunizations with the vaccine composition as part of a vaccination scheme or vaccination schedule. The "effective amount" may vary depending on the health and physical condition of the individual to be treated, the age of the individual to be treated (e.g. dosing for infants may be lower than for adults) the taxonomic group of the individual to be treated (e.g. human, non-human primate, primate, etc.), the capacity of the individual's immune system to mount an effective immune response, the degree of protection desired, the formulation of the vaccine, the treating doctor's assessment, the strain of the infecting pathogen and other relevant factors. It is expected that the effective amount of the vaccine composition will fall in a relatively broad range that can be determined through routine trials, i.e. 0,01 - 50 mg/dose; more preferably between 0,1 - 5 mg/dose. Usually, the amount will vary from 0,01 to 1000 $\mu$g/dose, more particularly from 0,1 to 100 $\mu$g/dose. Dosage treatment may be a single dose schedule or a multiple dose schedule. The vaccine may be administered in conjunction with other immunoregulatory agents. The dosages, routes of administration, and dose schedules are adjusted in accordance with methodologies known in the art.

A composition or vaccine composition may comprise more than one peptide or nucleic acid, i.e., a plurality thereof, e.g. 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or more, e.g., up to 35, 36, 37, 38, 39, 40, 41, 42, 43, 44, 45, 46, 47, 48, 49, 50 or more distinct peptides or nucleic acids.

**Carriers, adjuvants and vehicles - delivery**

Once appropriately immunogenic peptides, or the nucleic acids encoding them, have been defined, they can be sorted and delivered by various means, herein referred to as "compositions", "vaccine compositions" or "pharmaceutical compositions". The peptides of the present invention and pharmaceutical and vaccine compositions of the invention are usefull for administration to mammals, particularly humans, to treat and/or prevent HCV infection. Vaccine compositions containing the peptides of the invention, or the DNA encoding them, are administered to a patient infected with HCV or to an individual susceptible to, or otherwise at risk for, HCV infection to elicit an immune response against HCV antigens and thus enhance the patient's own immune response capabilities.

Various art-recognized delivery systems may be used to deliver peptides, polyepitopic polypeptides, or polynucleotides encoding peptides or polyepitope polypeptides, into appropriate cells. The peptides and nucleic acids encoding them can be delivered in a pharmaceutically acceptable carrier or as colloidal suspensions, or as powders, with or without diluents. They can be "naked" or associated with delivery vehicles and delivered using delivery systems known in the art.

A "pharmaceutically acceptable carrier" or "pharmaceutically acceptable adjuvant" is any suitable excipient, diluent, carrier and/or adjuvant which, by themselves, do not induce the production of antibodies harmful to the individual receiving the composition nor do they elicit protection. Preferably, a pharmaceutically acceptable carrier or adjuvant enhances the immune response elicited by an antigen. Suitable carriers or adjuvantia typically comprise one or more of the compounds included in the following non-exhaustive list: large slowly metabolized macromolecules such as proteins, polysaccharides, polylactic acids, polyglycolic acids, polymeric amino acids, amino acid copolymers and inactive virus particles; aluminium hydroxide, aluminium phosphate (see International Patent Application Publication No. WO93/24148), alum ($KAI(SO_4)_2.12H_2O$), or one of these in combination with 3-0-deacylated monophosphoryl lipid A (see International Patent Application Publication No. WO93/19780); N-acetyl-muramyl-L-threonyl-D-isoglutamine (see U.S. Patent No. 4,606,918), N-acetyl-normuramyl-L-alanyl-D-isoglutamine, N-acetylmuramyl-L-alanyl-D-isoglutamyl-L-alanine2-(1',2'-dipalmitoyl-sn-glycero-3-hydroxyphosphoryloxy) ethylamine; RIBI (ImmunoChem Research Inc., Hamilton, MT, USA) which contains monophosphoryl lipid A (i.e., a detoxified endotoxin), trehalose-6,6-dimycolate, and cell wall skeleton (MPL + TDM + CWS) in a 2% squalene/Tween 80 emulsion. Any of the three components MPL, TDM or CWS may also be used alone or combined 2 by 2; adjuvants such as Stimulon (Cambridge Bioscience, Worcester, MA, USA), SAF-1 (Syntex); adjuvants such as combinations between QS21 and 3-de-O-acetylated monophosphoryl lipid A (see International Application No. WO94/00153) which may be further supplemented with an oil-in-water emulsion (see, e.g., International Application Nos. WO95/17210, WO97/01640 and WO9856414) in which the oil-in-water emulsion comprises a metabolisable oil and a saponin, or a metabolisable oil, a saponin, and a sterol, or which may be further supplemented with a cytokine (see International Application No. WO98/57659); adjuvants such as MF-59 (Chiron), or poly[di(carboxylatophenoxy) phosphazene] based adjuvants (Virus Research Institute); block copolymer based adjuvants such as Optivax (Vaxcel, Cytrx) or inulin-based adjuvants, such as Algammulin and Gammalnulin (Anutech); Complete or Incomplete Freund's Adjuvant (CFA or IFA, respectively) or Gerbu preparations (Gerbu Biotechnik); a saponin such as QuilA, a purified saponin such as QS21, QS7 or QS17, β-escin or digitonin; immunostimulatory oligonucleotides comprising unmethylated CpG dinucleotides such as [purine-purine-CG-pyrimidine-pyrimidine] oligonucleotides. These immunostimulatory oligonucleotides include CpG class A, B, and C molecules (Coley Pharmaceuticals), ISS (Dynavax), Immunomers (Hybridon). Immunostimulatory oligonucleotides may also be combined with cationic peptides as described, e.g., by Riedl et al. (2002); Immune Stimulating Complexes comprising saponins, for example Quil A (ISCOMS); excipients and diluents, which are inherently non-toxic and non-therapeutic, such as water, saline, glycerol, ethanol, wetting or emulsifying agents, pH buffering substances, preservatives, and the like; a biodegradable and/or biocompatible oil such as squalane, squalene, eicosane, tetratetracontane, glycerol, peanut oil, vegetable oil, in a concentration of, e.g., 1 to 10% or 2,5 to 5%; vitamins such as vitamin C (ascorbic acid or its salts or esters), vitamin E (tocopherol), or vitamin A; carotenoids, or natural or synthetic flavanoids; trace elements, such as selenium; any Toll-like receptor ligand as reviewed in Barton and Medzhitov (2002).

Any of the afore-mentioned adjuvants comprising 3-de-O-acetylated monophosphoryl lipid A, said 3-de-O-acetylated monophosphoryl lipid A may be forming a small particle (see International Application No. WO94/21292).

In any of the aforementioned adjuvants MPL or 3-de-O-acetylated monophosphoryl lipid A can be replaced by a synthetic analogue referred to as RC-529 or by any other amino-alkyl glucosaminide 4-phosphate (Johnson et al. 1999, Persing et al. 2002). Alternatively it can be replaced by other lipid A analogues such as OM-197 (Byl et al. 2003).

A "pharmaceutically acceptable vehicle" includes vehicles such as water, saline, physiological salt solutions, glycerol, ethanol, etc. Auxiliary substances such as wetting or emulsifying agents, pH buffering substances, preservatives may be included in such vehicles. Delivery systems known in the art are e.g. lipopeptides, peptide compositions encapsulated in poly-DL-lactide-co-glycolide ("PLG"), microspheres, peptide compositions contained in immune stimulating complexes (ISCOMS), multiple antigen peptide systems (MAPs), viral delivery vectors, particles of viral or synthetic origin, adjuvants, liposomes, lipids, microparticles or microcapsules, gold particles, nanoparticles, polymers, condensing agents, polysaccharides, polyamino acids, dendrimers, saponins, QS21, adsorption enhancing materials, fatty acids or, naked or particle absorbed cDNA.

Typically, a vaccine or vaccine composition is prepared as an injectable, either as a liquid solution or suspension. Injection may be subcutaneous, intramuscular, intravenous, intraperitoneal, intrathecal, intradermal, intraepidermal, or by "gene gun". Other types of administration comprise electroporation, implantation, suppositories, oral ingestion, enteric application, inhalation, aerosolization or nasal spray or drops. Solid forms, suitable for dissolving in, or suspension in, liquid vehicles prior to injection may also be prepared. The preparation may also be emulsified or encapsulated in liposomes for enhancing adjuvant effect.

A liquid formulation may include oils, polymers, vitamins, carbohydrates, amino acids, salts, buffers, albumin, surfactants, or bulking agents. Preferably carbohydrates include sugar or sugar alcohols such as mono-, di-, or polysaccharides, or water-soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxethyl starch and carboxymethylcellulose, or mixtures thereof. Sucrose is most preferred. "Sugar alcohol" is defined as a C4 to C8 hydrocarbon having an -OH group and includes galactitol, inositol, mannitol, xylitol, sorbitol, glycerol, and arabitol. Mannitol is most preferred. These sugars or sugar alcohols mentioned above may be used individually or in combination. There is no fixed limit to the amount used as long as the sugar or sugar alcohol is soluble in the aqueous preparation. Preferably, the sugar or sugar alcohol concentration is between 1,0 % (w/v) and 7,0 % (w/v), more preferable between 2,0 and 6,0 % (w/v). Preferably amino acids include levorotary (L) forms of carnitine, arginine, and betaine; however, other amino acids may be added. Preferred polymers include polyvinylpyrrolidone (PVP) with an average molecular weight between 2,000 and 3,000, or polyethylene glycol (PEG) with an average molecular weight between 3,000 and 5,000. It is also preferred to use a buffer in the composition to minimize pH changes in the solution before lyophilization or after reconstitution. Any physiological buffer may be used, but citrate, phosphate, succinate, and glutamate buffers or mixtures thereof are preferred. Most preferred is a citrate buffer. Preferably, the concentration is from 0,01 to 0,3 molar. Surfactants that can be added to the formulation are shown in EP patent applications No. EP 0 270 799 and EP 0 268 110.

Additionally, polypeptides can be chemically modified by covalent conjugation to a polymer to increase their circulating half-life, for example. Preferred polymers, and methods to attach them to peptides, are shown in U.S. Patent Nos. 4,766,106; 4,179,337; 4,495,285; and 4,609,546. Preferred polymers are polyoxyethylated polyols and polyethylene glycol (PEG). PEG is soluble in water at room temperature and has the general formula:

$R(O-CH_2-CH_2)_nO-R$ where R can be hydrogen, or a protective group such as an alkyl or alkanol group. Preferably, the protective group has between 1 and 8 carbons, more preferably it is methyl. The symbol n is a positive integer, preferably between 1 and 1.000, more preferably between 2 and 500. The PEG has a preferred average molecular weight between 1000 and 40.000, more preferably between 2000 and 20.000, most preferably between 3.000 and 12.000. Preferably, PEG has at least one hydroxy group, more preferably it is a terminal hydroxy group. It is this hydroxy group which is preferably activated. However, it will be understood that the type and amount of the reactive groups may be varied to achieve a covalently conjugated PEG/polypeptide of the present invention.

Water soluble polyoxyethylated polyols are also useful in the present invention. They include polyoxyethylated sorbitol, polyoxyethylated glucose, polyoxyethylated glycerol (POG), etc. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG. The structure for POG is shown in Knauf et al., 1988, and a discussion of POG/IL-2 conjugates is found in U.S. Patent No. 4,766,106.

Another drug delivery system for increasing circulatory half-life is the liposome. The peptides and nucleic acids of the invention may also be administered via liposomes, which serve to target a particular tissue, such as lymphoid tissue, or to target selectively infected cells, as well as to increase the half-life of the peptide and nucleic acids composition. Liposomes include emulsions, foams, micelles, insoluble monolayers, liquid crystals, phospholipid dispersions, lamellar layers and the like. In these preparations, the peptide or nucleic acids to be delivered is incorporated as part of a liposome or embedded, alone or in conjunction with a molecule which binds to a receptor prevalent among lymphoid cells, such as monoclonal antibodies which bind to the CD45 antigen, or with other therapeutic or immunogenic compositions. Thus, liposomes either filled or decorated with a desired peptide or nucleic acids of the invention can be directed to the site of lymphoid cells, where the liposomes then deliver the peptide and nucleic acids compositions. Liposomes for use in accordance with the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al, 1980, and U.S. Patent Nos. 4,235,871, 4,501,728, 4,837,028, and 5,019,369.

For targeting cells of the immune system, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated. For example, liposomes carrying either immunogenic polypeptides or nucleic acids encoding immunogenic epitopes are known to elicit CTL responses in vivo (Reddy et al., 1992; Collins et al., 1992; Fries et al., 1992; Nabel et al., 1992).

After the liquid pharmaceutical composition is prepared, it is preferably lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is preferably administered to subjects using those methods that are known to those skilled in the art.

The approach known as "naked DNA" is currently being used for intramuscular (IM) administration in clinical trials. To maximize the immunotherapeutic effects of minigene DNA vaccines, an alternative method for formulating purified plasmid DNA may be desirable. A variety of methods have been described, and new techniques may become available. Cationic lipids can also be used in the formulation (see, e.g., as described by WO 93/24640; Mannino & Gould-Fogerite 1988; U.S. Pat No. 5,279,833; WO 91/06309; and Felgner et al., 1987. In addition, glycolipids, fusogenic liposomes, peptides and compounds referred to collectively as protective, interactive, non-condensing compounds could also be complexed to purified plasmid DNA to influence variables such as stability, intramuscular dispersion, or trafficking to specific organs or cell types.

Further examples of DNA-based delivery technologies include facilitated (bupivicaine, polymers, peptide- mediated) delivery, cationic lipid complexes, particle-mediated ("gene gun") or pressure-mediated delivery (see, e.g., U.S. Patent No. 5,922,687), DNA formulated with charged or uncharged lipids, DNA formulated in liposomes, emulsified DNA, DNA included in a viral vector, DNA formulated with a transfection-facilitating protein or polypeptide, DNA formulated with a targeting protein or polypeptide, DNA formulated with calcium precipitating agents, DNA coupled to an inert carrier molecule, and DNA formulated with an adjuvant. In this context it is noted that practically all considerations pertaining to the use of adjuvants in traditional vaccine formulation apply to the formulation of DNA vaccines.

Recombinant virus or live carrier vectors may also be directly used as live vaccines in humans. Accordingly the present invention also relates to a recombinant virus, an expression vector or a plasmid, and a host cell comprising the nucleic acid encoding at least one of the peptides as disclosed in Tables 13 and 14.

In a preferred embodiment of the invention, the nucleic acid or minigene is introduced in the form of a vector wherein expression is under control of a viral promoter. Therefore, further embodiments of the present invention are an expression vector which comprises a polynucleotide encoding at least one of the herein described peptides and which is capable of expressing the respective peptides, a host cell comprising the expression vector and a method of producing and purifying herein described peptides, pharmaceutical compositions comprising the herein described peptides and a pharmaceutically acceptable carrier and/or adjuvants. The "peptides as described herein" refer to the peptides disclosed in Tables 13 and 14.

Detailed disclosures relating to the formulation and use of nucleic acid vaccines are available, e.g. by Donnelly J.J. et al, 1997 and 1997a. Examples of expression vectors include attenuated viral hosts, such as vaccinia or fowlpox. As an example of this approach, vaccinia virus is used as a vector to express nucleotide sequences that encode the peptides of the invention. Upon introduction into a host bearing a tumor, the recombinant vaccinia virus expresses the immunogenic peptide, and thereby elicits a host CTL and/or HTL response. Vaccinia vectors, for example Modified Vaccinia Ankara (MVA), and methods useful in immunization protocols are described in, e.g., U.S. Patent No. 4,722,848. Another vector is BCG (Bacille Calmette Guerin). BCG vectors are described in Stover et al., 1991. Further examples are: Alphaviruses (Semliki Forest Virus, Sindbis Vrius, Venezuelan Equine Encephalitis Virus (VEE)), Transgene Herpes simplex Virus (HSV), replication-deficient strains of Adenovirus (human or simian), SV40 vectors, CMV vectors, papilloma virus vectors, and vectors derived from Epstein Barr virus. A wide variety of other vectors useful for therapeutic administration or immunization of the peptides of the invention, e.g. retroviral vectors, Salmonella typhi vectors, detoxified anthrax toxin vectors, and the like, will be apparent to those skilled in the art from the description herein.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences and sequences for replication in mammalian cells may also be considered for increasing minigene expression.

In addition, immunostimulatory sequences (ISSs or CpGs) appear to play a role in the immunogenicity of nucleic acid vaccines. These sequences may be included in the vector, outside the minigene coding sequence, if desired to enhance immunogenicity.

In some embodiments, a bi-cistronic expression vector which allows production of both the minigene-encoded epitopes and a second protein (included to enhance or decrease immunogenicity) can be used. Examples of proteins or polypeptides that could beneficially enhance the immune response if co-expressed include cytokines (e.g., IL-2, IL- 12, GM-CSF), cytokine-inducing molecules (e.g., LeIF), costimulatory molecules, or for HTL responses, pan-DR binding proteins (PADRE®, Epimmune, San Diego, CA).

Helper (HTL) epitopes can be joined to intracellular targeting signals and expressed separately from expressed CTL epitopes; this allows direction of the HTL epitopes to a cell compartment different than that of the CTL epitopes. If required, this could facilitate more efficient entry of HTL epitopes into the HLA class II pathway, thereby improving HTL induction. In contrast to HTL or CTL induction, specifically decreasing the immune response by co-expression of immunosuppressive molecules (e.g. TGF-P) may be beneficial in certain diseases.

The use of multi-epitope minigenes is described in, e.g., US 6,534,482; An and Whitton, 1997; Thomson et al., 1996; Whitton et al., 1993; Hanke et al., 1998. For example, a multi-epitope DNA plasmid encoding supermotif- and/or motif-bearing HCV epitopes derived from multiple regions of the HCV polyprotein sequence, the PADRE® universal helper T cell epitope (or multiple HTL epitopes from HCV), and an endoplasmic reticulum- translocating signal sequence can be engineered.

The nucleic acids or minigenes encoding the peptides or polyepitopic polypeptides, or the peptides or polyepitopic peptides themselves, can be administered alone or in combination with other therapies known in the art. In addition, the polypeptides and nucleic acids of the invention can be administered in combination with other treatments designed to enhance immune responses, e.g., by co-administration with adjuvants or cytokines (or nucleic acids encoding cytokines), as is well known in the art. Accordingly, the peptides or nucleic acids or vaccine compositions of the invention can also be used in combination with antiviral drugs such as interferon, or other treatments for viral infection.

All disclosures herein which relate to use of adjuvants in the context of protein or (poly)peptide based pharmaceutical compositions apply mutatis mutandis to their use in nucleic acid vaccination technology. The same holds true for other considerations relating to formulation and mode and route of administration and, hence, also these considerations discussed herein in connection with a traditional pharmaceutical composition apply mutatis mutandis to their use in nucleic acid vaccination technology.

In a further embodiment, the present invention relates to the use of the peptide and/or nucleic acid as described herein for inducing immunity against HCV, characterized in that said peptide and/or nucleic acid is used as part of a series of time and compounds. In this regard, it is to be understood that the term "a series of time and compounds" refers to administering with time intervals to an individual the compounds used for eliciting an immune response. The latter compounds may comprise any of the following components: a peptide or polyepitopic peptide, a nucleic acid or minigene or a vector.

In this respect, a series comprises administering, either:

(i) a peptide or polyepitopic peptide, or
(ii) a nucleic acid, minigene or vector, wherein said nucleic acid, minigene or vector can be administered simultaneously, or at different time intervals, including at alternating time intervals, or
(iii) a peptide or polyepitopic peptide in combination with a nucleic acid, minigene or vector, wherein said peptide or polyepitopic peptide and said nucleic acid, minigene or vector can be administered simultaneously, or at different time intervals, including at alternating time intervals, or
(iv) either (i) or (ii), possibly in combination with other peptides or nucleic acids or vectors, with time intervals.

The peptide and nucleic acid compositions of this invention can be provided in kit form together with instructions for vaccine administration. Typically the kit would include desired peptide compositions in a container, preferably in unit dosage form and instructions for administration. An alternative kit would include a minigene construct with desired nucleic acids of the invention in a container, preferably in unit dosage form together with instructions for administration. Lymphokines such as IL-2 or IL- 12 may also be included in the kit. Other kit components that may also be desirable include, for example, a sterile syringe, booster dosages, and other desired excipients.

## Use of the peptides for evaluating immune responses.

The peptides may also find use as diagnostic reagents. For example, a peptide of the invention may be used to determine the susceptibility of a particular individual to a treatment regimen which employs the peptide, related peptides or any other HCV vaccine, and thus may be helpful in modifying an existing treatment protocol or in determining a prognosis for an affected individual. In addition, the peptides may also be used to predict which individuals will be at substantial risk for developing chronic HCV infection.

Accordingly, the present invention relates to a method of determining the outcome for a subject exposed to HCV, comprising the steps of determining whether the subject has an immune response to one or more peptides selected from Tables 13 and 14.

In a preferred embodiment of the invention, the peptides as described herein can be used as reagents to evaluate an immune response. The immune response to be evaluated can be induced by using as an immunogen any agent that may result in the production of antigen-specific CTLs or HTLs that recognize and bind to the peptide(s) to be employed as the reagent. The peptide reagent need not be used as the immunogen. Assay systems that can be used for such an analysis include relatively recent technical developments such as tetramers, staining for intracellular lymphokines and interferon release assays, or ELISPOT assays.

For example, a peptide of the invention may be used in a tetramer staining assay to assess peripheral blood mononuclear cells for the presence of antigen-specific CTLs following exposure to an antigen or an immunogen. The HLA- tetrameric complex is used to directly visualize antigen-specific CTLS (see, e.g., Ogg et al., 1998; and Altman et al., 1996) and determine the frequency of the antigen-specific CTL population in a sample of peripheral blood mononuclear cells. A tetramer reagent using a peptide of the invention may be generated as follows: a peptide that binds to an HLA molecule is refolded in the presence of the corresponding HLA heavy chain and beta2-microglobulin to generate a trimolecular complex. The complex is biotinylated at the carboxyl terminal end of the heavy chain at a site that was previously engineered into the protein. Tetramer formation is then induced by the addition of streptavidin. By means of fluorescently labeled streptavidin, the tetramer can be used to stain antigen-specific cells. The cells may then be identified, for example, by flow cytometry. Such an analysis may be used for diagnostic or prognostic purposes. Cells identified by the procedure can also be used for therapeutic purposes. As an alternative to tetramers also pentamers or dimers can be used (Current Protocols in Immunology (2000) unit 17.2 supplement 35)

Peptides of the invention may also be used as reagents to evaluate immune recall responses. (see, e.g., Bertoni et al.,

1997 and Perma et al., 1991.). For example, patient PBMC samples from individuals with HCV infection may be analyzed for the presence of antigen-specific CTLs or HTLs using specific peptides. A blood sample containing mononuclear cells may be evaluated by cultivating the PBMCs and stimulating the cells with a peptide of the invention. After an appropriate cultivation period, the expanded cell population may be analyzed, for example, for cytotoxic activity (CTL) or for HTL activity.

The peptides may also be used as reagents to evaluate the efficacy of a vaccine.

PBMCs obtained from a patient vaccinated with an immunogen may be analyzed using, for example, either of the methods described above. The patient is HLA typed, and peptide epitope reagents that recognize the allele-specific molecules present in that patient are selected for the analysis. The immunogenicity of the vaccine is indicated by the presence of epitope-specific CTLs and/or HTLs in the PBMC sample.

The peptides of the invention may also be used to make antibodies, using techniques well known in the art (see, e.g. CURRENT PROTOCOLS IN IMMUNOLOGY, Wiley/Greene, NY; and Antibodies A Laboratory Manual, Harlow and Lane, Cold Spring Harbor Laboratory Press, 1989). Such antibodies include those that recognize a peptide in the context of an HLA molecule, i.e., antibodies that bind to a peptide-MHC complex.

**TABLES**

The peptides of current invention are set out in Tables 1-14.

As used herein, "CS_fr" and "CS_to" means Consensus Sequence "from" and "to" residue numbers of the HCV consensus sequence as disclosed in Figure 1 or 2.

S: Strong, Kdpred <0,1μM; M: Medium, Kdpred 0,1-1μM; W: Weak, Kdpred 1-10μM

**Table 1 Predicted HLA-A*0101 binding peptides**

|  | Protein | CS_fr | CS_to | pep_seq | Score | SEQ ID NO |
|---|---|---|---|---|---|---|
| **Algonomics 9-mer** | | | | | | |
| 1 | NS3 | 1436 | 1444 | ATDALMTGY | S | 1 |
| 2 | C | 126 | 134 | LTCGFADLM | S | 2 |
| 3 | NS5B | 2588 | 2596 | RVCEKMALY | S | 3 |
| 4 | C | 130 | 138 | FADLMGYIP | M | 4 |
| 5 | NS3 | 1565 | 1573 | LTHIDAHFL | M | 5 |
| 6 | NS3 | 1285 | 1293 | ITTGAPITY | M | 6 |
| 7 | NS3 | 1210 | 1218 | FTDNSSPPA | M | 7 |
| 8 | NS3 | 1581 | 1589 | DNFPYLVAY | M | 8 |
| 9 | NS5B | 2759 | 2767 | FTEAMTRYS | M | 9 |
| 10 | NS5B | 2795 | 2803 | DASGKRVYY | M | 10 |
| 11 | NS3 | 1288 | 1296 | GAPITYSTY | M | 11 |
| 12 | NS3 | 1241 | 1249 | PAAYAAQGY | M | 12 |
| 13 | NS3 | 1520 | 1528 | CYDAGCAWY | M | 13 |
| 14 | NS5B | 2835 | 2843 | YAPTLWARM | M | 14 |
| 15 | NS3 | 1197 | 1205 | PVESMETTM | M | 15 |
| 16 | NS5B | 2605 | 2613 | AVMGSSYGF | M | 16 |
| 17 | NS3 | 1513 | 1521 | DSSVLCECY | M | 17 |
| 18 | NS3 | 1410 | 1418 | LGLNAVAYY | M | 18 |
| 19 | NS5B | 2770 | 2778 | PGDPPQPEY | M | 19 |
| 20 | NS3 | 1370 | 1378 | NGEIPFYGK | M | 20 |
| 21 | NS3 | 1635 | 1643 | VTLTHPITK | M | 21 |
| 22 | NS5B | 2607 | 2615 | MGSSYGFQY | M | 22 |

Table continued

| | Protein | CS_fr | CS_to | pep_seq | Score | SEQ ID NO |
|---|---|---|---|---|---|---|
| **Algonomics 9-mer** | | | | | | |
| 23 | NS3 | 1637 | 1645 | LTHPITKYI | M | 23 |
| 24 | NS3 | 1579 | 1587 | AGDNFPYLV | M | 24 |
| 25 | NS3 | 1236 | 1244 | KSTKVPAAY | M | 25 |
| 26 | NS3 | 1291 | 1299 | ITYSTYGKF | M | 26 |
| 27 | NS3 | 1532 | 1540 | PAETSVRLR | M | 27 |
| 28 | C | 122 | 130 | VIDTLTCGF | M | 28 |
| 29 | NS3 | 1420 | 1428 | GLDVSVIPT | M | 29 |
| 30 | NS3 | 1466 | 1474 | LDPTFTIET | M | 30 |
| 31 | C | 158 | 166 | LEDGVNYAT | W | 31 |
| 32 | NS3 | 1260 | 1268 | ATLGFGAYM | W | 32 |
| 33 | NS3 | 1602 | 1610 | PSWDQMWKC | W | 33 |
| 34 | NS5B | 2837 | 2845 | PTLWARMIL | W | 34 |
| 35 | NS3 | 1468 | 1476 | PTFTIETTT | W | 35 |
| 36 | NS5B | 2758 | 2766 | VFTEAMTRY | W | 36 |
| 37 | NS5B | 2603 | 2611 | PQAVMGSSY | W | 37 |
| 38 | NS5B | 2792 | 2800 | VAHDASGKR | W | 38 |
| 39 | NS5B | 2757 | 2765 | RVFTEAMTR | W | 39 |
| 40 | NS5B | 2710 | 2718 | GNTLTCYLK | W | 40 |
| 41 | NS5B | 2563 | 2571 | EVFCVQPEK | W | 41 |
| 42 | C | 172 | 180 | CSFSIFLLA | W | 42 |
| 43 | NS5B | 2615 | 2623 | YSPGQRVEF | W | 43 |
| 44 | NS3 | 1434 | 1442 | VVATDALMT | W | 44 |
| 45 | C | 156 | 164 | RVLEDGVNY | W | 45 |
| 46 | NS3 | 1534 | 1542 | ETSVRLRAY | W | 46 |
| 47 | NS3 | 1391 | 1399 | LIFCHSKKK | W | 47 |
| 48 | NS5B | 2662 | 2670 | CCDLAPEAR | W | 48 |
| 49 | NS5B | 2826 | 2834 | NSWLGNIIM | W | 49 |
| 50 | NS3 | 1262 | 1270 | LGFGAYMSK | W | 50 |
| 51 | NS3 | 1409 | 1417 | ALGLNAVAY | W | 51 |
| 52 | NS3 | 1199 | 1207 | ESMETTMRS | W | 52 |
| 53 | NS3 | 1437 | 1445 | TDALMTGYT | W | 53 |
| 54 | NS3 | 1195 | 1203 | FIPVESMET | W | 54 |
| 55 | C | 109 | 117 | PTDPRRRSR | W | 55 |
| 56 | NS3 | 1242 | 1250 | AAYAAQGYK | W | 56 |
| 57 | NS3 | 1203 | 1211 | TTMRSPVFT | W | 57 |
| 58 | NS3 | 1569 | 1577 | DAHFLSQTK | W | 58 |
| 59 | NS5B | 2842 | 2850 | RMILMTHFF | W | 59 |

Table continued

| | Protein | CS_fr | CS_to | pep_seq | Score | SEQ ID NO |
|---|---|---|---|---|---|---|
| **Algonomics 9-mer** | | | | | | |
| 60 | NS3 | 1335 | 1343 | QAETAGARL | W | 60 |
| 61 | NS3 | 1649 | 1657 | MSADLEVVT | W | 61 |
| | | | | | | |
| **Epimmune** | | | | | | |
| | Protein | CS_fr | CS_to | pep_seq | Score PIC | SEQ ID NO |
| | | | | MSATLCSALY | 22 | 1507 |
| | E1 | | | VQDCNCSIY | 16 | 1961 |
| | E1 | | | VQECNCSIY | 24 | 1962 |
| | E1 | | | TQDCNCSIY | 12 | 1864 |
| | | | | DMRPYCWHY | 68 | 970 |
| | | | | ASSVCGPVY | 56 | 874 |
| | | | | TTDRSGAPTY | 88 | 1872 |
| | | | | CTWMNSTGY | 21 | 947 |
| | | | | CGAPPCNIY | 74 | 914 |
| | E2 | | | LTPRCLVDY | 65 | 1474 |
| | E2 | | | LTPRCLIDY | 32 | 1473 |
| | E2 | | | FTI FKVRMY | 34 | 1089 |
| | E2 | | | YTIFKIRMY | 26 | 2070 |
| | E2 | | | FTIFKIRMY | 33 | 1088 |
| | | | | GLSPAITKY | 15 | 1156 |
| | | | | VLALPQQAY | 56 | 1926 |
| | | | | LIAVLGPLY | 31 | 1384 |
| | | | | LLALLGPAY | 30 | 1389 |
| | | | | ISGVLWTVY | 42 | 1304 |
| | NS3 | | | CTCGSSDLY | 18 | 940 |
| | NS3 | | | CTCGAVDLY | 17 | 938 |
| | NS3 | | | CTCGSADLY | 21 | 939 |
| | NS3 | | | LLSPRPISY | 15 | 1408 |
| | NS3 | | | KSTKVPAAY | 71 | 25 |
| | NS3 | | | PAAYAAQGY | 29 | 12 |
| | NS3 | | | PAAYVAQGY | 42 | 1544 |
| | NS3 | | | ITTGAPITY | 13 | 6 |
| | NS3 | | | ITTGSPITY | 15 | 1309 |
| | NS3 | | | STTGEIPFY | 50 | 1791 |
| | NS3 | | | GSEGEIPFY | 42 | 1185 |
| | NS3 | | | GMGLNAVAY | 47 | 1159 |

Table continued

| Epimmune | | | | | | |
|---|---|---|---|---|---|---|
| | Protein | CS_fr | CS_to | pep_seq | Score PIC | SEQ ID NO |
| | NS3 | | | ATDALMTGY | 32 | 1 |
| | NS3 | | | DSSVLCECY | 31 | 17 |
| | NS3 | | | DSVVLCECY | 83 | 987 |
| | | | | ETTVRLRAY | 46 | 1035 |
| | NS5A | | | CTPSPAPNY | 78 | 943 |
| | NS5A | | | EVDGVRLHRY | 17 | 1036 |
| | NS5A | | | ELDGVRLHRY | 23 | 1017 |
| | | | | PLSNSLLRY | 21 | 1560 |
| | NS5B | | | HSAKSKFGY | 24 | 1241 |
| | NS5B | | | HSARSKFGY | 25 | 1242 |
| | NS5B | | | MGSSYGFQY | 91 | 22 |
| | NS5B | | | MGSAYGFQY | 98 | 1495 |
| | NS5B | | | KKDPMGFSY | 77 | 1328 |
| | NS5B | | | TSCGNTLTCY | 41 | 1867 |
| | NS5B | | | TSFGNTITCY | 45 | 1868 |
| | NS5B | | | DASGKRVYY | 55 | 10 |
| | NS5B | | | GLSAFSLHSY | 47 | 1153 |
| | NS5B | | | GLDAFSLHTY | 28 | 1148 |
| | NS5B | | | GLSAFTLHSY | 43 | 1155 |
| | NS5B | | | LSAFSLHSY | 9 | 1456 |
| | NS5B | | | LDAFSLHTY | 32 | 1367 |
| | NS5B | | | LSAFTLHSY | 9 | 1457 |
| | NS5B | | | GRAAICGKY | 95 | 1179 |
| | NS5B | | | LLSVGVGIY | 47 | 1411 |
| | | | | | | |
| **Algonomics 10-mer** | | | | | | |
| | Protein | CS_fr | CS_to | pep_seq | Score | SEQ ID NO |
| | Ns5b | 2759 | 2768 | FTEAMTRYSA | S | 1087 |
| | Ns5b | 2826 | 2835 | NSWLGNIIMY | M | 1534 |
| | Ns4b | 1848 | 1857 | LVDILAGYGA | M | 1478 |
| | Ns3 | 1436 | 1445 | ATDALMTGYT | M | 877 |
| | Ns3 | 1617 | 1626 | TLHGPTPLLY | M | 1833 |
| | Ns3 | 1435 | 1444 | VATDALMTGY | M | 1894 |
| | Ns3 | 1210 | 1219 | FTDNSSPPAV | M | 1086 |
| | Ns5b | 2757 | 2766 | RVFTEAMTRY | M | 1712 |
| | Ns3 | 1635 | 1644 | VTLTHPITKY | M | 1976 |

Table continued

| Algonomics 10-mer | | | | | | |
|---|---|---|---|---|---|---|
| | Protein | CS_fr | CS_to | pep_seq | Score | SEQ ID NO |
| | Ns3 | 1258 | 1267 | VAATLGFGAY | M | 1887 |
| | Ns3 | 1409 | 1418 | ALGLNAVAYY | M | 822 |
| | Ns3 | 1637 | 1646 | LTHPITKYIM | M | 1469 |
| | Ns3 | 1240 | 1249 | VPAAYAAQGY | M | 1943 |
| | Ns3 | 1519 | 1528 | ECYDAGCAWY | M | 1002 |
| | Core | 122 | 131 | VIDTLTCGFA | M | 1914 |
| | Ns3 | 1578 | 1587 | QAGDNFPYLV | W | 1596 |
| | Ns5b | 2794 | 2803 | HDASGKRVYY | W | 1216 |
| | Ns3 | 1408 | 1417 | SALGLNAVAY | W | 1717 |
| | Ns5b | 2606 | 2615 | VMGSSYGFQY | W | 1939 |
| | Ns3 | 1554 | 1563 | HLEFWESVFT | W | 1225 |
| | Ns3 | 1367 | 1376 | LSNTGEIPFY | W | 1459 |
| | Core | 127 | 136 | TCGFADLMGY | W | 1815 |
| | Core | 130 | 139 | FADLMGYIPL | W | 1048 |
| | Ns3 | 1433 | 1442 | VVVATDALMT | W | 1987 |
| | Ns3 | 1465 | 1474 | SLDPTFTIET | W | 1741 |
| | Ns5b | 2832 | 2841 | IIMYAPTLWA | W | 1268 |
| | Ns3 | 1369 | 1378 | NTGEIPFYGK | W | 1535 |
| | Ns5b | 2620 | 2629 | RVEFLVNAWK | W | 1711 |
| | Ns5b | 2602 | 2611 | LPQAVMGSSY | W | 1437 |
| | Core | 157 | 166 | VLEDGVNYAT | W | 1927 |
| | Ns3 | 1197 | 1206 | PVESMETTMR | W | 1588 |
| | Ns3 | 1634 | 1643 | EVTLTHPITK | W | 1041 |
| | Ns5b | 2835 | 2844 | YAPTLWARMI | W | 2028 |
| | Ns3 | 1567 | 1576 | HIDAHFLSQT | W | 1222 |
| | Ns3 | 1490 | 1499 | RTGRGRRGIY | W | 1704 |
| | Ns3 | 1530 | 1539 | LTPAETSVRL | W | 1471 |
| | Ns5b | 2589 | 2598 | VCEKMALYDV | W | 1897 |
| | Ns3 | 1568 | 1577 | IDAHFLSQTK | W | 1256 |
| | Ns3 | 1522 | 1531 | DAGCAWYELT | W | 953 |
| | Ns3 | 1580 | 1589 | GDNFPYLVAY | W | 1112 |
| | Ns3 | 1192 | 1201 | AVDFIPVESM | W | 882 |
| | Ns5b | 2707 | 2716 | TSCGNTLTCY | W | 1867 |
| | Ns3 | 1284 | 1293 | TITTGAPITY | W | 1829 |
| | Ns4b | 1944 | 1953 | VTQILSSLTI | W | 1977 |
| | Ns5b | 2796 | 2805 | ASGKRVYYLT | W | 870 |
| | Ns5b | 2713 | 2722 | LTCYLKASAA | W | 1466 |

Table continued

| Algonomics 10-mer | | | | | | |
|---|---|---|---|---|---|---|
| | Protein | CS_fr | CS_to | pep_seq | Score | SEQ ID NO |
| | Ns3 | 1172 | 1181 | PSGHAVGIFR | W | 1584 |
| | Core | 182 | 191 | LSCLTIPASA | W | 1458 |
| | Ns5b | 2833 | 2842 | IMYAPTLWAR | W | 1279 |
| | Ns3 | 1260 | 1269 | ATLGFGAYMS | W | 878 |
| | Ns5b | 2754 | 2763 | ASLRVFTEAM | W | 871 |

**Table 2 Predicted HLA-A*0201 binding peptides**

| | Protein | CS_fr | CS_to | pep_seq | Score | SEQ ID NO |
|---|---|---|---|---|---|---|
| **Algonomics 9-mer** | | | | | | |
| 1 | NS5B | 2828 | 2836 | WLGNIIMYA | S | 62 |
| 2 | NS3 | 1585 | 1593 | YLVAYQATV | S | 63 |
| 3 | NS3 | 1565 | 1573 | LTHIDAHFL | S | 64 |
| 4 | C | 77 | 85 | AQPGYPWPL | S | 65 |
| 5 | C | 132 | 140 | DLMGYIPLV | S | 66 |
| 6 | NS5B | 2594 | 2602 | ALYDWSTL | S | 67 |
| 7 | NS5B | 2598 | 2606 | VVSTLPQAV | S | 68 |
| 8 | C | 136 | 144 | YIPLVGAPL | S | 69 |
| 9 | C | 181 | 189 | LLSCLTIPA | S | 70 |
| 10 | NS3 | 1510 | 1518 | GMFDSSVLC | M | 71 |
| 11 | C | 150 | 158 | ALAHGVRVL | M | 72 |
| 12 | NS3 | 1250 | 1258 | KVLVLNPSV | M | 73 |
| 13 | NS3 | 1542 | 1550 | YLNTPGLPV | M | 74 |
| 14 | NS5B | 2727 | 2735 | KLQDCTMLV | M | 75 |
| 15 | NS3 | 1560 | 1568 | SVFTGLTHI | M | 76 |
| 16 | NS3 | 1434 | 1442 | VVATDALMT | M | 77 |
| 17 | C | 90 | 98 | GLGWAGWLL | M | 78 |
| 18 | NS5B | 2679 | 2687 | RLYIGGPLT | M | 79 |
| 19 | NS3 | 1195 | 1203 | FIPVESMET | M | 80 |
| 20 | NS3 | 1617 | 1625 | TLHGPTPLL | M | 81 |
| 21 | NS3 | 1252 | 1260 | LVLNPSVAA | M | 82 |
| 22 | NS3 | 1589 | 1597 | YQATVCARA | M | 83 |
| 23 | NS5B | 2833 | 2841 | IMYAPTLWA | M | 84 |
| 24 | NS5B | 2593 | 2601 | MALYDWST | M | 85 |
| 25 | NS3 | 1342 | 1350 | RLVVLATAT | M | 86 |
| 26 | NS5B | 2831 | 2839 | NIIMYAPTL | M | 87 |
| 27 | NS5B | 2748 | 2756 | GTQEDAASL | M | 88 |

Table continued

| | Protein | CS_fr | CS_to | pep_seq | Score | SEQ ID NO |
|---|---|---|---|---|---|---|
| **Algonomics 9-mer** | | | | | | |
| 28 | NS3 | 1325 | 1333 | TILGIGTVL | M | 89 |
| 29 | NS3 | 1645 | 1653 | IMACMSADL | M | 90 |
| 30 | C | 29 | 37 | QIVGGVYLL | M | 91 |
| 31 | NS5B | 2838 | 2846 | TLWARMILM | M | 92 |
| 32 | C | 168 | 176 | NLPGCSFSI | M | 93 |
| 33 | NS5B | 2733 | 2741 | MLVNGDDLV | W | 94 |
| 34 | NS3 | 1244 | 1252 | YAAQGYKVL | W | 95 |
| 35 | NS3 | 1188 | 1196 | GVAKAVDFI | W | 96 |
| 36 | NS5B | 2842 | 2850 | RMILMTHFF | W | 97 |
| 37 | NS3 | 1331 | 1339 | TVLDQAETA | W | 98 |
| 38 | NS3 | 1637 | 1645 | LTHPITKYI | W | 99 |
| 39 | NS3 | 1253 | 1261 | VLNPSVAAT | W | 100 |
| 40 | NS3 | 1210 | 1218 | FTDNSSPPA | W | 101 |
| 41 | NS3 | 1345 | 1353 | VLATATPPG | W | 102 |
| 42 | NS3 | 1251 | 1259 | VLVLNPSVA | W | 103 |
| 43 | NS3 | 1169 | 1177 | LLCPSGHW | W | 104 |
| 44 | NS3 | 1420 | 1428 | GLDVSVIPT | W | 105 |
| 45 | NS3 | 1464 | 1472 | FSLDPTFTI | W | 106 |
| 46 | NS3 | 1260 | 1268 | ATLGFGAYM | W | 107 |
| 47 | NS5B | 2835 | 2843 | YAPTLWARM | W | 108 |
| 48 | NS3 | 1284 | 1292 | TITTGAPIT | W | 109 |
| 49 | NS3 | 1203 | 1211 | TTMRSPVFT | W | 110 |
| 50 | NS5B | 2613 | 2621 | FQYSPGQRV | W | 111 |
| 51 | NS3 | 1224 | 1232 | QVAHLHAPT | W | 112 |
| 52 | NS3 | 1218 | 1226 | AVPQTFQVA | W | 113 |
| 53 | NS3 | 1283 | 1291 | RTITTGAPI | W | 114 |
| 54 | NS3 | 1245 | 1253 | AAQGYKVLV | W | 115 |
| 55 | NS3 | 1586 | 1594 | LVAYQATVC | W | 116 |
| 56 | NS3 | 1178 | 1186 | GVFRAAVCT | W | 117 |
| 57 | C | 133 | 141 | LMGYIPLVG | W | 118 |
| 58 | NS3 | 1630 | 1638 | AVQNEVTLT | W | 119 |
| 59 | NS3 | 1497 | 1505 | GIYRFVTPG | W | 120 |
| 60 | NS5B | 2720 | 2728 | SAACRAAKL | W | 121 |
| 61 | NS3 | 1610 | 1618 | CLIRLKPTL | W | 122 |
| 62 | NS3 | 1572 | 1580 | FLSQTKQAG | W | 123 |
| 63 | NS3 | 1450 | 1458 | SVIDCNTCV | W | 124 |
| 64 | NS3 | 1349 | 1357 | ATPPGSVTV | W | 125 |

Table continued

|  | Protein | CS_fr | CS_to | pep_seq | Score | SEQ ID NO |
|---|---|---|---|---|---|---|
| **Algonomics 9-mer** |  |  |  |  |  |  |
| 65 | NS5B | 2815 | 2823 | AAWETARHT | W | 126 |
| 66 | C | 28 | 36 | GQIVGGVYL | W | 127 |
| 67 | C | 157 | 165 | VLEDGVNYA | W | 128 |
| 68 | NS3 | 1555 | 1563 | LEFWESVFT | W | 129 |
| 69 | NS3 | 1246 | 1254 | AQGYKVLVL | W | 130 |
| 70 | NS5B | 2734 | 2742 | LVNGDDLVV | W | 131 |
| 71 | C | 36 | 44 | LLPRRGPRL | W | 132 |
| 72 | NS5B | 2600 | 2608 | STLPQAVMG | W | 133 |
| 73 | NS3 | 1425 | 1433 | VIPTSGDVV | W | 134 |
| 74 | NS3 | 1509 | 1517 | SGMFDSSVL | W | 135 |
| 75 | NS3 | 1648 | 1656 | CMSADLEVV | W | 136 |
| 76 | NS3 | 1376b | 1384b | YGKAIPIEV | W | 137 |
| 77 | NS3 | 1649 | 1657 | MSADLEVVT | W | 138 |
| 78 | NS5B | 2830 | 2838 | GNIIMYAPT | W | 139 |
| 79 | NS3 | 1328 | 1336 | GIGTVLDQA | W | 140 |
| 80 | NS3 | 1175 | 1183 | HWGVFRAA | W | 141 |
| 81 | NS3 | 1406 | 1414 | KLSALGLNA | W | 142 |
| 82 | NS3 | 1379b | 1387b | AIPIEVIKG | W | 143 |
|  |  |  |  |  |  |  |
| **Epimmune** |  |  |  |  |  |  |
|  | Protein | CS_fr | CS_to | pep_seq | Score PIC | SEQ ID NO |
|  | C |  |  | AQPGYPWPL | 82 | 65 |
|  | C |  |  | GLGWAGWLL | 71 | 78 |
|  | C |  |  | DLMGYIPLV | 21 | 66 |
|  | C |  |  | DLMGYIPVV | 56 | 966 |
|  | C |  |  | NLPGCSFSI | 56 | 93 |
|  | C |  |  | FLLALLSCL | 24 | 361 |
|  | C |  |  | FLLALFSCL | 21 | 1068 |
|  | C |  |  | FLLALLSCI | 24 | 1069 |
|  | C |  |  | FLLALLSCLT | 87 | 1070 |
|  |  |  |  | LLALLSCLTV | 63 | 1390 |
|  |  |  |  | HLPGCVPCV | 75 | 1231 |
|  | E1 |  |  | MMMNWSPTA | 55 | 1498 |
|  | E1 |  |  | MMMNWSPTT | 91 | 1500 |
|  | E1 |  |  | MMMNWSPTAA | 99 | 1499 |
|  | E1 |  |  | MMMNWSPTTA | 90 | 1501 |

Table continued

| Epimmune | | | | | | |
|---|---|---|---|---|---|---|
| | Protein | CS_fr | CS_to | pep_seq | Score PIC | SEQ ID NO |
| | | | | VMFGLAYFSM | 78 | 1937 |
| | | | | SMQGAWAKV | 76 | 1752 |
| | | | | LQTGFLASL | 34 | 1451 |
| | E2 | | | CMVDYPYRL | 40 | 924 |
| | E2 | | | CLVDYPYRL | 41 | 922 |
| | E2 | | | CL1DYPYRL | 37 | 920 |
| | E2 | | | CLVHYPYRL | 81 | 923 |
| | E2 | | | RLWHYPCTI | 25 | 1667 |
| | E2 | | | RLWHYPCTV | 14 | 1669 |
| | E2 | | | RLWHYPCTL | 25 | 1668 |
| | E2 | | | TLFKVRMYV | 89 | 1830 |
| | E2 | | | ALSTGLIHL | 74 | 825 |
| | E2 | | | ALSTGLLHL | 64 | 826 |
| | E2 | | | YLYGVGSAV | 23 | 2056 |
| | E2 | | | YLYGVGSAVV | 43 | 2057 |
| | E2 | | | YVVLLFLLL | 42 | 2075 |
| | E2 | | | YWLLFLLLA | 77 | 2076 |
| | E2 | | | VILLFLLLA | 60 | 1919 |
| | E2 | | | VVLLFLLLA | 77 | 1983 |
| | E2 | | | LLFLLLADA | 61 | 1395 |
| | E2 | | | FLLLADARI | 36 | 1071 |
| | E2 | | | FLLLADARV | 20 | 1072 |
| | | | | LLLADARVCV | 98 | 1399 |
| | | | | MLLISQAEA | 90 | 1497 |
| | P7 | | | GVWPLLLLL | 61 | 1207 |
| | | | | ALQVWVPPL | 72 | 824 |
| | | | | LQVWVPPLL | 45 | 1453 |
| | | | | KLLLAVLGPL | 83 | 1332 |
| | | | | LLLAVLGPL | 50 | 1401 |
| | | | | LLIAVLGPL | 57 | 1398 |
| | | | | LLLAIFGPL | 44 | 1400 |
| | | | | ALLGPAYLL | 46 | 823 |
| | | | | AVLGPLYLI | 53 | 892 |
| | | | | SLLRIPYFV | 18 | 1744 |
| | | | | YIYNHLTPL | 51 | 2040 |
| | | | | YIYDHLTPM | 37 | 2039 |
| | NS2 | | | YVYNHLTPL | 66 | 2079 |

Table continued

| Epimmune | | | | | | |
|---|---|---|---|---|---|---|
| | Protein | CS_fr | CS_to | pep_seq | Score PIC | SEQ ID NO |
| | NS2 | | | YVYDHLTPL | 26 | 2077 |
| | | | | LLAPITAYA | 36 | 1391 |
| | NS3 | | | GLLGCIITSL | 88 | 1151 |
| | NS3 | | | LLGCIITSL | 57 | 1397 |
| | NS3 | | | FLGTTVGGV | 62 | 1067 |
| | NS3 | | | FLGTSISGV | 66 | 1066 |
| | NS3 | | | FLATCINGV | 25 | 1065 |
| | NS3 | | | CINGVCWTV | 84 | 919 |
| | NS3 | | | SISGVLWTV | 61 | 1737 |
| | NS3 | | | GVMWTVYHGA | 100 | 1198 |
| | NS3 | | | VMWTVYHGA | 39 | 1942 |
| | NS3 | | | VLWTVYHGA | 41 | 1935 |
| | NS3 | | | YLVTRHADV | 79 | 2053 |
| | NS3 | | | YLVTRNADV | 38 | 2055 |
| | NS3 | | | ATLGFGAYM | 92 | 32 |
| | NS3 | | | YLNTPGLPV | 45 | 74 |
| | NS3 | | | YLSTPGLPV | 49 | 2049 |
| | NS3 | | | YLVAYQATV | 3 | 63 |
| | NS3 | | | YLTAYQATV | 5 | 2050 |
| | NS3 | | | YQATVCARA | 49 | 83 |
| | NS3 | | | QMWKCLIRL | 71 | 238 |
| | NS3 | | | VMWKCLIRL | 36 | 1940 |
| | NS3 | | | VMWKCLTRL | 61 | 1941 |
| | NS3 | | | RLGAVQNEV | 82 | 265 |
| | NS4A | | | VLVGGVLAA | 74 | 1933 |
| | NS4A | | | VLAGGVLAA | 90 | 1922 |
| | NS4A | | | VLVGGVLAAL | 89 | 1934 |
| | NS4A | | | VLAGGVLAAV | 47 | 1923 |
| | NS4A | | | LAGGVLAAV | 99 | 1360 |
| | NS4A | | | ALAAYCLSV | 7 | 820 |
| | NS4A | | | ALAAYCLTT | 26 | 821 |
| | NS4B | | | HMWNFISGI | 53 | 1233 |
| | NS4B | | | HMWNFVSGI | 49 | 1234 |
| | NS4B | | | FISGIQYLA | 20 | 1060 |
| | NS4B | | | FVSGIQYLA | 25 | 1093 |
| | NS4B | | | SLMAFTASV | 6 | 1746 |
| | NS4B | | | SMMAFSAAL | 19 | 1751 |

Table continued

| Epimmune | Protein | CS_fr | CS_to | pep_seq | Score PIC | SEQ ID NO |
|---|---|---|---|---|---|---|
| | NS4B | | | LLFNILGGWV | 51 | 1396 |
| | NS4B | | | ILLNIMGGWL | 87 | 1272 |
| | NS4B | | | FVVSGLAGA | 77 | 1094 |
| | NS4B | | | ILAGYGAGV | 28 | 1269 |
| | NS4B | | | VLAGYGAGV | 30 | 1925 |
| | NS4B | | | VLAGYGAGI | 54 | 1924 |
| | NS4B | | | WMNRLIAFA | 97 | 2015 |
| | NS5A | | | NMWHGTFPI | 21 | 1525 |
| | NS5A | | | NTWQGTFPI | 99 | 1537 |
| | NS5A | | | NTWHGTFPI | 87 | 1536 |
| | | | | FMGGDVTRI | 46 | 1075 |
| | NS5B | | | RLIVFPDLGV | 83 | 1661 |
| | NS5B | | | ALYDVIQKL | 56 | 829 |
| | NS5B | | | ALYDITQKL | 63 | 828 |
| | NS5B | | | ALYDVVSTL | 29 | 67 |
| | NS5B | | | FLVCGDDLV | 43 | 1073 |
| | NS5B | | | FLVCGDDLVV | 65 | 1074 |
| | NS5B | | | IQYAPTIWV | 39 | 1299 |
| | NS5B | | | IMYAPTLWA | 34 | 84 |
| | NS5B | | | TLWARMILM | 40 | 92 |
| | NS5B | | | ILMTHFFSI | 7 | 1273 |
| | NS5B | | | VLMTHFFSI | 8 | 1928 |
| | NS5B | | | VLMTHFFSIL | 90 | 1929 |
| | NS5B | | | ILMTHFFSIL | 82 | 1274 |
| | NS5B | | | EMYGATYSV | 30 | 1019 |
| | NS5B | | | EMYGAVYSV | 24 | 1020 |
| | NS5B | | | RLHGLSAFT | 74 | 1660 |
| | NS5B | | | RLHGLEAFSL | 89 | 1658 |
| | NS5B | | | RLHGLDAFSL | 73 | 1657 |
| | NS5B | | | GLDAFSLHT | 67 | 1147 |
| | NS5B | | | GLYLFNWAV | 33 | 1157 |
| | NS5B | | | RLLDLSSWFT | 53 | 1663 |
| | NS5B | | | RLLLLGLLLL | 40 | 1664 |
| | NS5B | | | HLLLCLLLL | 42 | 1230 |
| | NS5B | | | LLLLGLLLL | 38 | 1404 |
| | NS5B | | | LLLCLLLLT | 27 | 1402 |
| | NS5B | | | LLLCLLLLTV | 16 | 1403 |

Table continued

| Epimmune | | | | | | |
|---|---|---|---|---|---|---|
| | Protein | CS_fr | CS_to | pep_seq | Score PIC | SEQ ID NO |
| | NS5B | | | LLCLLLLTV | 43 | 1393 |
| | NS5B | | | LLLLTVGVGI | 70 | 1405 |
| | NS5B | | | LTVGVGIFL | 89 | 1477 |

**Table 3 Predicted HLA-A*0301 and HLA-A*1101 binding peptides**

| | Protein | CS_fr | CS_to | pep_seq | Score | SEQ ID NO |
|---|---|---|---|---|---|---|
| **Algonomics 9-mer** | | | | | | |
| 1 | C | 43 | 51 | RLGVRATRK | S | 144 |
| 2 | NS3 | 1411 | 1419 | GLNAVAYYR | S | 145 |
| 3 | NS5B | 2624 | 2632 | LVNAWKSKK | S | 146 |
| 4 | NS3 | 1242 | 1250 | AAYAAQGYK | S | 147 |
| 5 | NS3 | 1390 | 1398 | HLIFCHSKK | S | 148 |
| 6 | C | 35 | 43 | YLLPRRGPR | S | 149 |
| 7 | NS5B | 2623 | 2631 | FLVNAWKSK | S | 150 |
| 8 | NS3 | 1391 | 1399 | LIFCHSKKK | S | 151 |
| 9 | NS3 | 1635 | 1643 | VTLTHPITK | M | 152 |
| 10 | NS3 | 1409 | 1417 | ALGLNAVAY | M | 153 |
| 11 | NS5B | 2584 | 2592 | DLGVRVCEK | M | 154 |
| 12 | NS5B | 2719 | 2727 | ASAACRAAK | M | 155 |
| 13 | NS3 | 1183 | 1191 | AVCTRGVAK | M | 156 |
| 14 | NS5B | 2567 | 2575 | VQPEKGGRK | M | 157 |
| 15 | C | 2 | 10 | STNPKPQRK | M | 158 |
| 16 | C | 62 | 70 | RQPIPKARR | M | 159 |
| 17 | NS5B | 2757 | 2765 | RVFTEAMTR | M | 160 |
| 18 | NS5B | 2716 | 2724 | YLKASAACR | M | 161 |
| 19 | NS5B | 2710 | 2718 | GNTLTCYLK | M | 162 |
| 20 | C | 96 | 104 | WLLSPRGSR | M | 163 |
| 21 | C | 10 | 18 | KTKRNTNRR | M | 164 |
| 22 | NS5B | 2594 | 2602 | ALYDVVSTL | M | 165 |
| 23 | NS3 | 1262 | 1270 | LGFGAYMSK | M | 166 |
| 24 | C | 51 | 59 | KTSERSQPR | M | 167 |
| 25 | NS5B | 2580 | 2588 | IVFPDLGVR | M | 168 |
| 26 | C | 156 | 164 | RVLEDGVNY | M | 169 |
| 27 | NS5B | 2833 | 2841 | IMYAPTLWA | W | 170 |
| 28 | NS3 | 1288 | 1296 | GAPITYSTY | W | 171 |
| 29 | NS5B | 2798 | 2806 | GKRVYYLTR | W | 172 |

Table continued

| | Protein | CS_fr | CS_to | pep_seq | Score | SEQ ID NO |
|---|---|---|---|---|---|---|
| Algonomics 9-mer | | | | | | |
| 30 | NS3 | 1389 | 1397 | RHLIFCHSK | W | 173 |
| 31 | NS3 | 1492 | 1500 | GRGRRGIYR | W | 174 |
| 32 | NS5B | 2679 | 2687 | RLYIGGPLT | W | 175 |
| 33 | NS5B | 2634 | 2642 | PMGFSYDTR | W | 176 |
| 34 | C | 59 | 67 | RGRRQPIPK | W | 177 |
| 35 | NS5B | 2621 | 2629 | VEFLVNAWK | W | 178 |
| 36 | NS3 | 1510 | 1518 | GMFDSSVLC | W | 179 |
| 37 | NS3 | 1605 | 1613 | DQMWKCLIR | W | 180 |
| 38 | NS3 | 1378b | 1386b | KAIPIEVIK | W | 181 |
| 39 | NS3 | 1542 | 1550 | YLNTPGLPV | W | 182 |
| 40 | NS5B | 2588 | 2596 | RVCEKMALY | W | 183 |
| 41 | C | 93 | 101 | WAGWLLSPR | W | 184 |
| 42 | NS3 | 1585 | 1593 | YLVAYQATV | W | 185 |
| 43 | C | 90 | 98 | GLGWAGWLL | W | 186 |
| 44 | NS3 | 1607 | 1615 | MWKCLIRLK | W | 187 |
| 45 | NS5B | 2791 | 2799 | SVAHDASGK | W | 188 |
| 46 | C | 47 | 55 | RATRKTSER | W | 189 |
| 47 | NS5B | 2828 | 2836 | WLGNIIMYA | W | 190 |
| 48 | NS3 | 1378 | 1386 | KAIPIEAIK | W | 191 |
| 49 | NS3 | 1619 | 1627 | HGPTPLLYR | W | 192 |
| 50 | NS5B | 2563 | 2571 | EVFCVQPEK | W | 193 |
| 51 | C | 1 | 9 | MSTNPKPQR | W | 194 |
| 52 | NS3 | 1228 | 1236 | LHAPTGSGK | W | 195 |
| 53 | NS3 | 1482 | 1490 | VSRSQRRGR | W | 196 |
| 54 | C | 31 | 39 | VGGVYLLPR | W | 197 |
| 55 | NS3 | 1178 | 1186 | GVFRAAVCT | W | 198 |
| 56 | C | 15 | 23 | TNRRPQDVK | W | 199 |
| 57 | NS3 | 1624 | 1632 | LLYRLGAVQ | W | 200 |
| 58 | NS3 | 1636 | 1644 | TLTHPITKY | W | 201 |
| 59 | NS3 | 1420 | 1428 | GLDVSVIPT | W | 202 |
| 60 | C | 105 | 113 | PSWGPTDPR | W | 203 |
| 61 | NS3 | 1176 | 1184 | VVGVFRAAV | W | 204 |
| 62 | NS3 | 1611 | 1619 | LIRLKPTLH | W | 205 |
| 63 | C | 45 | 53 | GVRATRKTS | W | 206 |
| 64 | C | 141 | 149 | GAPLGGAAR | W | 207 |
| 65 | C | 132 | 140 | DLMGYIPLV | W | 208 |
| 66 | NS3 | 1221 | 1229 | QTFQVAHLH | W | 209 |

Table continued

| | Protein | CS_fr | CS_to | pep_seq | Score | SEQ ID NO |
|---|---|---|---|---|---|---|
| **Algonomics 9-mer** | | | | | | |
| 67 | NS3 | 1436 | 1444 | ATDALMTGY | W | 210 |
| 68 | NS3 | 1577 | 1585 | KQAGDNFPY | W | 211 |
| 69 | NS3 | 1581 | 1589 | DNFPYLVAY | W | 212 |
| 70 | C | 36 | 44 | LLPRRGPRL | W | 213 |
| 71 | NS3 | 1231 | 1239 | PTGSGKSTK | W | 214 |
| 72 | NS3 | 1291 | 1299 | ITYSTYGKF | W | 215 |
| 73 | C | 78 | 86 | QPGYPWPLY | W | 216 |
| 74 | NS5B | 2762 | 2770 | AMTRYSAPP | W | 217 |
| 75 | NS3 | 1328 | 1336 | GIGTVLDQA | W | 218 |
| 76 | NS3 | 1618 | 1626 | LHGPTPLLY | W | 219 |
| 77 | NS3 | 1530 | 1538 | LTPAETSVR | W | 220 |
| 78 | NS3 | 1485 | 1493 | SQRRGRTGR | W | 221 |
| 79 | NS3 | 1236 | 1244 | KSTKVPAAY | W | 222 |
| 80 | C | 30 | 38 | IVGGVYLLP | W | 223 |
| 81 | NS3 | 1490 | 1498 | RTGRGRRGI | W | 224 |
| 82 | NS3 | 1406 | 1414 | KLSALGLNA | W | 225 |
| 83 | NS5B | 2613 | 2621 | FQYSPGQRV | W | 226 |
| 84 | C | 74 | 82 | RTWAQPGYP | W | 227 |
| 85 | NS5B | 2692 | 2700 | QNCGYRRCR | W | 228 |
| | NS3 | 1513 | 1521 | DSSVLCECY | N | 229 |
| | | | | | | |
| **Epimmune** | | | | | | |
| | Protein | CS_fr | CS_to | pep_seq | Score PIC | SEQ ID NO |
| | C | | | STNPKPQRK | 5,6 | 158 |
| | C | | | STIPKPQRK | 17 | 1789 |
| | C | | | KTSERSQPR | 70 | 167 |
| | C | | | AQPGYPWPLY | 365 | 861 |
| | | | | RVLEDGINY | 51 | 1713 |
| | | | | GQAFTFRPR | 383 | 1177 |
| | E1 | | | QLFTFSPRR | 109 | 1609 |
| | E1 | | | TTQDCNCSIY | 67 | 1877 |
| | E1 | | | ALVVSQLLR | 50 | 827 |
| | E1 | | | GVLAGLAYY | 26 | 1197 |
| | E1 | | | GILAGLAYY | 94 | 1142 |
| | | | | FSMQGAWAK | 3,7 | 1084 |
| | | | | QTGFLASLFY | 59 | 1620 |

Table continued

| Epimmune | | | | | | |
|---|---|---|---|---|---|---|
| | Protein | CS_fr | CS_to | pep_seq | Score PIC | SEQ ID NO |
| | | | | GFIAGLFYY | 57 | 1134 |
| | | | | FIAGLFYYHK | 11 | 1058 |
| | | | | FLASLFYTHK | 50 | 1064 |
| | | | | TLLCPTDCFR | 168 | 1834 |
| | | | | LLCPTDCFRK | 125 | 1394 |
| | E2 | | | CTVNFTIFK | 2,4 | 945 |
| | E2 | | | CTVNFTLFK | 2,0 | 946 |
| | E2 | | | CTVNFSIFK | 4,2 | 944 |
| | | | | GQAEAALEK | 13 | 1176 |
| | P7 | | | VFFCAAWYIK | 6,5 | 1908 |
| | P7 | | | FFCAAWYIK | 30 | 1056 |
| | P7 | | | GFFTLSPWYK | 44 | 1133 |
| | P7 | | | FFTLSPWYK | 31 | 1057 |
| | P7 | | | ILTLSPHYK | 32 | 1277 |
| | | | | SLLRIPYFVR | 112 | 1745 |
| | | | | LTRVPYFVR | 43 | 1476 |
| | | | | LLRIPYFVR | 301 | 1407 |
| | | | | FVRAHALLR | 71 | 1091 |
| | | | | KLGALTGTY | 444 | 1329 |
| | NS2 | | | YVYDHLTPLR | 26 | 2078 |
| | NS2 | | | YVYNHLTPLR | 34 | 2080 |
| | | | | VIFSPMEIK | 5,7 | 1915 |
| | | | | RLLAPITAY | 466 | 1662 |
| | | | | KLLAPITAY | 331 | 1330 |
| | | | | ITAYAQQTR | 58 | 1307 |
| | | | | TVYHGAGNK | 6,7 | 1882 |
| | | | | AVDLYLVTR | 20 | 884 |
| | NS3 | | | GIFRAAVCTR | 27 | 1141 |
| | NS3 | | | GIFRAAVCSR | 47 | 1140 |
| | NS3 | | | AVCTRGVAK | 5,4 | 156 |
| | NS3 | | | AVCSRGVAK | 2,8 | 881 |
| | NS3 | | | TLGFGAYMSK | 18 | 1831 |
| | NS3 | | | TLGFGTYMSK | 22 | 1832 |
| | NS3 | | | PITYSTYGK | 77 | 1556 |
| | NS3 | | | KLTYSTYGK | 15 | 1334 |
| | NS3 | | | SITYSTYGK | 2,1 | 1738 |
| | NS3 | | | AITYSTYGK | 2,0 | 818 |

Table continued

| Epimmune | | | | | | |
|---|---|---|---|---|---|---|
| | Protein | CS_fr | CS_to | pep_seq | Score PIC | SEQ ID NO |
| | NS3 | | | TTGEIPFYGK | 13 | 1873 |
| | NS3 | | | HLIFCHSRK | 95 | 1229 |
| | NS3 | | | LIFCHSKKK | 45 | 47 |
| | NS3 | | | LIFCHSRKK | 80 | 1385 |
| | NS3 | | | SLGLNAVAYY | 327 | 1742 |
| | NS3 | | | GLNAVAYYR | 4,8 | 145 |
| | NS3 | | | GINAVAYYR | 2,0 | 1143 |
| | NS3 | | | GVNAVAYYR | 0,57 | 1199 |
| | NS3 | | | ATDALMTGY | 48 | 1 |
| | NS3 | | | KQSGENFPY | 244 | 1347 |
| | NS3 | | | DVMWKCLTR | 62 | 991 |
| | NS3 | | | DQMWKCLTR | 504 | 983 |
| | NS3 | | | LQGPTPLLYR | 881 | 1448 |
| | NS3 | | | VTLTHPITK | 13 | 21 |
| | NS3 | | | VVLTHPITK | 9,9 | 1984 |
| | NS4B | | | MQLAEQFKQK | 268 | 1506 |
| | NS4B | | | QLAEQFKQK | 34 | 1608 |
| | NS4B | | | RIAEMLKSK | 69 | 1654 |
| | NS4B | | | AVGSIGLGK | 0,9 | 888 |
| | NS4B | | | AVGSVGLGK | 1,2 | 889 |
| | NS4B | | | GVAGALVAFK | 3,0 | 1192 |
| | NS4B | | | GISGALVAFK | 16 | 1145 |
| | NS4B | | | GVSGALVAFK | 5,2 | 1204 |
| | NS4B | | | ISGALVAFK | 29 | 1302 |
| | NS4B | | | VSGALVAFK | 29 | 1971 |
| | NS4B | | | AFKIMSGEK | 326 | 801 |
| | NS4B | | | GWCAAILR | 19 | 1205 |
| | NS4B | | | GVICAAILR | 20 | 1194 |
| | NS4B | | | GWCAAILRR | 17 | 1206 |
| | NS4B | | | GVICAAILRR | 20 | 1195 |
| | NS4B | | | VVCAAILRR | 6,5 | 1978 |
| | NS4B | | | VICAAILRR | 23 | 1913 |
| | | | | SLTITSLLR | 110 | 1747 |
| | | | | SLTVTQLLR | 98 | 1748 |
| | | | | SLTVTSLLR | 103 | 1749 |
| | | | | GLPFISCQK | 68 | 1152 |
| | | | | GIPFISCQK | 28 | 1144 |

Table continued

| Epimmune | | | | | | |
|---|---|---|---|---|---|---|
| | Protein | CS_fr | CS_to | pep_seq | Score PIC | SEQ ID NO |
| | | | | GSMRITGPK | 2,4 | 1188 |
| | | | | QIHRFAPTPK | 34 | 1605 |
| | | | | ITAEAAARR | 70 | 1305 |
| | NS5A | | | ASQLSAPSLK | 25 | 873 |
| | NS5A | | | SQLSAPSLK | 15 | 1781 |
| | NS5A | | | SQLSAPSLR | 151 | 1782 |
| | NS5A | | | NLFMGGDVTR | 273 | 1524 |
| | NS5A | | | RQEMGGNITR | 587 | 1692 |
| | NS5A | | | RQEMGSNITR | 553 | 1693 |
| | NS5A | | | VSVPAEILRK | 23 | 1974 |
| | NS5A | | | SVPAEILRK | 1,5 | 1800 |
| | NS5A | | | SIPSEYLLPK | 18 | 1736 |
| | NS5A | | | SSALAELATK | 28 | 1784 |
| | NS5A | | | STALAELAAK | 13 | 1786 |
| | NS5B | | | SLLRHHNMVY | 215 | 1743 |
| | NS5B | | | TTSRSASQR | 26 | 1879 |
| | NS5B | | | TTSRSASLR | 61 | 1878 |
| | NS5B | | | RQKKVTFDR | 955 | 1694 |
| | NS5B | | | RLQVLDDHYK | 48 | 1665 |
| | NS5B | | | LQVLDDHYK | 139 | 1452 |
| | NS5B | | | VQPEKGGRK | 595 | 157 |
| | NS5B | | | RVCEKMALY | 75 | 3 |
| | NS5B | | | RVFTEAMTR | 13 | 39 |
| | NS5B | | | RVFTEAMTRY | 20 | 1712 |
| | NS5B | | | SVAHDASGK | 5,7 | 188 |
| | NS5B | | | SVAHDASGKR | 54 | 1797 |
| | NS5B | | | SVALDPRGRR | 61 | 1798 |
| | NS5B | | | IQYAPTIWVR | 702 | 1300 |
| | NS5B | | | LLAQEQLEK | 151 | 1392 |
| | NS5B | | | AVRASLISR | 26 | 894 |
| | NS5B | | | SVRAKLLSR | 36 | 1801 |
| | NS5B | | | GLYLFNWAVR | 78 | 1158 |
| | NS5B | | | YLFNWAVRTK | 53 | 2044 |
| | NS5B | | | YLFNWAVKTK | 54 | 2042 |
| | NS5B | | | ILFNWAVRTK | 124 | 1380 |
| | NS5B | | | LFNWAVKTK | 69 | 1379 |

**Table 4 Predicted HLA-A*2402 binding peptides**

| | Protein | CS_fr | CS_to | pep_seq | Score | SEQ ID NO |
|---|---|---|---|---|---|---|
| **Algonomics 9-mer** | | | | | | |
| 1 | NS5B | 2842 | 2850 | RMILMTHFF | S | 230 |
| 2 | NS5B | 2838 | 2846 | TLWARMILM | S | 231 |
| 3 | NS3 | 1610 | 1618 | CLIRLKPTL | S | 232 |
| 4 | NS3 | 1617 | 1625 | TLHGPTPLL | S | 233 |
| 5 | NS3 | 1557 | 1565 | FWESVFTGL | S | 234 |
| 6 | C | 75 | 83 | TWAQPGYPW | S | 235 |
| 7 | C | 129 | 137 | GFADLMGYI | S | 236 |
| 8 | NS5B | 2831 | 2839 | NIIMYAPTL | S | 237 |
| 9 | NS3 | 1606 | 1614 | QMWKCLIRL | S | 238 |
| 10 | NS3 | 1643 | 1651 | KYIMACMSA | S | 239 |
| 11 | NS3 | 1246 | 1254 | AQGYKVLVL | S | 240 |
| 12 | NS3 | 1292 | 1300 | TYSTYGKFL | S | 241 |
| 13 | NS3 | 1270 | 1278 | KAHGVDPNI | S | 242 |
| 14 | C | 85 | 93 | LYGNEGLGW | S | 243 |
| 15 | NS3 | 1266 | 1274 | AYMSKAHGV | S | 244 |
| 16 | C | 90 | 98 | GLGWAGWLL | M | 245 |
| 17 | NS5B | 2832 | 2840 | IIMYAPTLW | M | 246 |
| 18 | C | 28 | 36 | GQIVGGVYL | M | 247 |
| 19 | NS5B | 2828 | 2836 | WLGNIIMYA | M | 248 |
| 20 | NS3 | 1338 | 1346 | TAGARLVVL | M | 249 |
| 21 | C | 173 | 181 | SFSIFLLAL | M | 250 |
| 22 | NS3 | 1464 | 1472 | FSLDPTFTI | M | 251 |
| 23 | NS3 | 1585 | 1593 | YLVAYQATV | M | 252 |
| 24 | NS3 | 1384b | 1392b | VIKGGRHLI | M | 253 |
| 25 | NS3 | 1623 | 1631 | PLLYRLGAV | M | 254 |
| 26 | NS3 | 1325 | 1333 | TILGIGTVL | M | 255 |
| 27 | NS5B | 2824 | 2832 | PVNSWLGNI | M | 256 |
| 28 | NS3 | 1202 | 1210 | ETTMRSPVF | M | 257 |
| 29 | NS3 | 1564 | 1572 | GLTHIDAHF | M | 258 |
| 30 | NS5B | 2605 | 2613 | AVMGSSYGF | M | 259 |
| 31 | NS3 | 1162 | 1170 | KGSSGGPLL | M | 260 |
| 32 | NS5B | 2727 | 2735 | KLQDCTMLV | M | 261 |
| 33 | NS3 | 1244 | 1252 | YAAQGYKVL | M | 262 |
| 34 | NS3 | 1637 | 1645 | LTHPITKYI | M | 263 |
| 35 | NS3 | 1374 | 1382 | PFYGKAIPI | M | 264 |
| 36 | NS3 | 1627 | 1635 | RLGAVQNEV | M | 265 |

Table continued

| | Protein | CS_fr | CS_to | pep_seq | Score | SEQ ID NO |
|---|---|---|---|---|---|---|
| **Algonomics 9-mer** | | | | | | |
| 37 | NS3 | 1384 | 1392 | AIKGGRHLI | M | 266 |
| 38 | NS5B | 2594 | 2602 | ALYDWSTL | M | 267 |
| 39 | C | 149 | 157 | RALAHGVRV | M | 268 |
| 40 | C | 136 | 144 | YIPLVGAPL | M | 269 |
| 41 | C | 36 | 44 | LLPRRGPRL | M | 270 |
| 42 | NS3 | 1417 | 1425 | YYRGLDVSV | M | 271 |
| 43 | NS3 | 1402 | 1410 | ELAAKLSAL | M | 272 |
| 44 | NS3 | 1376b | 1384b | YGKAIPIEV | M | 273 |
| 45 | NS5B | 2607 | 2615 | MGSSYGFQY | M | 274 |
| 46 | NS3 | 1169 | 1177 | LLCPSGHVV | M | 275 |
| 47 | NS5B | 2627 | 2635 | AWKSKKCPM | M | 276 |
| 48 | NS3 | 1243 | 1251 | AYAAQGYKV | M | 277 |
| 49 | NS5B | 2620 | 2628 | RVEFLVNAW | M | 278 |
| 50 | NS3 | 1603 | 1611 | SWDQMWKCL | M | 279 |
| 51 | C | 168 | 176 | NLPGCSFSI | M | 280 |
| 52 | NS5B | 2636 | 2644 | GFSYDTRCF | M | 281 |
| 53 | NS3 | 1217 | 1225 | PAVPQTFQV | M | 282 |
| 54 | C | 118 | 126 | NLGKVIDTL | M | 283 |
| 55 | C | 23 | 31 | KFPGGGQIV | M | 284 |
| 56 | NS3 | 1542 | 1550 | YLNTPGLPV | M | 285 |
| 57 | NS3 | 1604 | 1612 | WDQMWKCLI | W | 286 |
| 58 | NS5B | 2840 | 2848 | WARMILMTH | W | 287 |
| 59 | C | 77 | 85 | AQPGYPWPL | W | 288 |
| 60 | C | 29 | 37 | QIVGGVYLL | W | 289 |
| 61 | NS3 | 1293 | 1301 | YSTYGKFLA | W | 290 |
| 62 | NS3 | 1510 | 1518 | GMFDSSVLC | W | 291 |
| 63 | NS5B | 2834 | 2842 | MYAPTLWAR | W | 292 |
| 64 | C | 172 | 180 | CSFSIFLLA | W | 293 |
| 65 | C | 171 | 179 | GCSFSIFLL | W | 294 |
| 66 | NS3 | 1188 | 1196 | GVAKAVDFI | W | 295 |
| 67 | NS5B | 2613 | 2621 | FQYSPGQRV | W | 296 |
| 68 | C | 150 | 158 | ALAHGVRVL | W | 297 |
| 69 | NS5B | 2821 | 2829 | RHTPVNSWL | W | 298 |
| 70 | NS5B | 2837 | 2845 | PTLWARMIL | W | 299 |
| 71 | NS3 | 1493 | 1501 | RGRRGIYRF | W | 300 |
| 72 | NS5B | 2629 | 2637 | KSKKCPMGF | W | 301 |
| 73 | C | 179 | 187 | LALLSCLTI | W | 302 |

Table continued

| | Protein | CS_fr | CS_to | pep_seq | Score | SEQ ID NO |
|---|---|---|---|---|---|---|
| **Algonomics 9-mer** | | | | | | |
| 74 | NS3 | 1354 | 1362 | SVTVPHPNI | W | 303 |
| 75 | NS5B | 2705 | 2713 | LTTSCGNTL | W | 304 |
| 76 | NS3 | 1641 | 1649 | ITKYIMACM | W | 305 |
| 77 | NS3 | 1375 | 1383 | FYGKAIPIE | W | 306 |
| 78 | NS3 | 1620 | 1628 | GPTPLLYRL | W | 307 |
| 79 | NS3 | 1440 | 1448 | LMTGYTGDF | W | 308 |
| 80 | NS5B | 2588 | 2596 | RVCEKMALY | W | 309 |
| 81 | C | 132 | 140 | DLMGYIPLV | W | 310 |
| 82 | NS3 | 1385 | 1393 | IKGGRHLIF | W | 311 |
| 83 | NS3 | 1220 | 1228 | PQTFQVAHL | W | 312 |
| 84 | NS5B | 2802 | 2810 | YYLTRDPTT | W | 313 |
| 85 | NS5B | 2839 | 2847 | LWARMILMT | W | 314 |
| 86 | NS3 | 1250 | 1258 | KVLVLNPSV | W | 315 |
| 87 | NS3 | 1283 | 1291 | RTITTGAPI | W | 316 |
| 88 | NS3 | 1187 | 1195 | RGVAKAVDF | W | 317 |
| 89 | C | 115 | 123 | RSRNLGKVI | W | 318 |
| 90 | NS5B | 2679 | 2687 | RLYIGGPLT | W | 319 |
| 91 | NS5B | 2715 | 2723 | CYLKASAAC | W | 320 |
| 92 | NS3 | 1527 | 1535 | WYELTPAET | W | 321 |
| 93 | NS3 | 1565 | 1573 | LTHIDAHFL | W | 322 |
| 94 | NS5B | 2617 | 2625 | PGQRVEFLV | W | 323 |
| 95 | NS3 | 1406 | 1414 | KLSALGLNA | W | 324 |
| 96 | NS3 | 1566 | 1574 | THIDAHFLS | W | 325 |
| 97 | NS5B | 2615 | 2623 | YSPGQRVEF | W | 326 |
| 98 | NS3 | 1579 | 1587 | AGDNFPYLV | W | 327 |
| 99 | NS3 | 1645 | 1653 | IMACMSADL | W | 328 |
| 100 | NS3 | 1549 | 1557 | PVCQDHLEF | W | 329 |
| 101 | NS3 | 1245 | 1253 | AAQGYKVLV | W | 330 |
| 102 | NS3 | 1365 | 1373 | IGLSNNGEI | W | 331 |
| 103 | NS5B | 2674 | 2682 | RSLTERLYI | W | 332 |
| 104 | NS3 | 1648 | 1656 | CMSADLEVV | W | 333 |
| 105 | NS5B | 2796 | 2804 | ASGKRVYYL | W | 334 |
| 106 | NS3 | 1376 | 1384 | YGKAIPIEA | W | 335 |
| 107 | NS5B | 2782 | 2790 | LITSCSSNV | W | 336 |
| 108 | NS3 | 1260 | 1268 | ATLGFGAYM | W | 337 |
| 109 | NS5B | 2665 | 2673 | LAPEARQAI | W | 338 |
| 110 | C | 161 | 169 | GVNYATGNL | W | 339 |

Table continued

| | Protein | CS_fr | CS_to | pep_seq | Score | SEQ ID NO |
|---|---|---|---|---|---|---|
| **Algonomics 9-mer** | | | | | | |
| 111 | C | 156 | 164 | RVLEDGVNY | W | 340 |
| 112 | NS3 | 1444 | 1452 | YTGDFDSVI | W | 341 |
| 113 | NS3 | 1640 | 1648 | PITKYIMAC | W | 342 |
| 114 | NS3 | 1433 | 1441 | VVVATDALM | W | 343 |
| 115 | NS3 | 1596 | 1604 | RAQAPPPSW | W | 344 |
| 116 | C | 170 | 178 | PGCSFSIFL | W | 345 |
| 117 | NS3 | 1560 | 1568 | SVFTGLTHI | W | 346 |
| 118 | NS3 | 1629 | 1637 | GAVQNEVTL | W | 347 |
| 119 | NS3 | 1577 | 1585 | KQAGDNFPY | W | 348 |
| 120 | NS5B | 2581 | 2589 | VFPDLGVRV | W | 349 |
| 121 | NS3 | 1462 | 1470 | VDFSLDPTF | W | 350 |
| 122 | NS3 | 1547 | 1555 | GLPVCQDHL | W | 351 |
| 123 | NS5B | 2735 | 2743 | VNGDDLVVI | W | 352 |
| 124 | NS3 | 1443 | 1451 | GYTGDFDSV | W | 353 |
| 125 | NS3 | 1172 | 1180 | PSGHWGVF | W | 354 |
| 126 | NS5B | 2598 | 2606 | WSTLPQAV | W | 355 |
| 127 | NS3 | 1291 | 1299 | ITYSTYGKF | W | 356 |
| 128 | C | 143 | 151 | PLGGAARAL | W | 357 |
| 129 | NS3 | 1584 | 1592 | PYLVAYQAT | W | 358 |
| 130 | NS3 | 1638 | 1646 | THPITKYIM | W | 359 |
| 131 | NS3 | 1264 | 1272 | FGAYMSKAH | W | 360 |
| 132 | C | 177 | 185 | FLLALLSCL | N | 361 |
| 133 | C | 174 | 182 | FSIFLLALL | N | 362 |
| 134 | C | 125 | 133 | TLTCGFADL | N | 363 |
| 135 | C | 133 | 141 | LMGYIPLVG | N | 364 |
| 136 | C | 83 | 91 | WPLYGNEGL | N | 365 |
| 137 | C | 135 | 143 | GYIPLVGAP | N | 366 |
| 138 | C | 89 | 97 | EGLGWAGWL | N | 367 |
| 139 | C | 181 | 189 | LLSCLTIPA | N | 368 |
| 140 | C | 80 | 88 | GYPWPLYGN | N | 369 |
| 141 | C | 111 | 119 | DPRRRSRNL | N | 370 |
| | | | | | | |
| **Epimmune** | | | | | | |
| | Protein | CS_fr | CS_to | pep_seq | Score PIC | SEQ ID NO |
| | C | | | GFADLMGYI | 67 | 236 |
| | | | | SFSIFLLALF | 69 | 1729 |

Table continued

| Epimmune | | | | | | |
|---|---|---|---|---|---|---|
| | Protein | CS_fr | CS_to | pep_seq | Score PIC | SEQ ID NO |
| | E1 | | | CWVALTPTL | 11 | 950 |
| | | | | AYFSMQGAW | 37 | 902 |
| | | | | AWAKVVVIL | 8,8 | 896 |
| | E2 | | | HYAPRPCGI | 9,9 | 1246 |
| | E2 | | | HYPPRPCGI | 11 | 1251 |
| | E2 | | | HYPPKPCGI | 13 | 1250 |
| | E2 | | | HYPYRLWHY | 3,7 | 1252 |
| | E2 | | | LWHYPCTVNF | 77 | 1484 |
| | E2 | | | HYPCTVNFTI | 66 | 1247 |
| | E2 | | | HYPCTVNYTI | 65 | 1249 |
| | E2 | | | HYPCTVNFTL | 64 | 1248 |
| | | | | NYTIFKIRM | 64 | 1543 |
| | | | | EWAILPCSY | 76 | 1043 |
| | | | | KWEYWLLF | 6,5 | 1354 |
| | | | | KWEWWLLF | 6,5 | 1353 |
| | | | | EYWLLFLL | 23 | 1047 |
| | | | | EWWLLFLL | 70 | 1045 |
| | | | | EWVILLFLL | 31 | 1044 |
| | P7 | | | SFLVFFCAAW | 67 | 1728 |
| | P7 | | | WYLVAFCAAW | 58 | 2023 |
| | P7 | | | VFFCAAWYI | 6,9 | 1907 |
| | | | | HWIGRLIWW | 13 | 1245 |
| | | | | LYPSLIFDI | 21 | 1489 |
| | | | | FYPGVVFDI | 2,7 | 1101 |
| | | | | VFDITKWLL | 55 | 1906 |
| | | | | PYFVRAHVL | 21 | 1592 |
| | | | | PYFVRAHALL | 49 | 1591 |
| | | | | YFVRAHALL | 1,4 | 2032 |
| | | | | TYIYNHLTPL | 98 | 1884 |
| | | | | PMEIKVITW | 73 | 1561 |
| | | | | GYTSKGWKL | 52 | 1214 |
| | | | | AYMSKAHGI | 76 | 904 |
| | NS3 | | | TYSTYGKFL | 97 | 241 |
| | NS3 | | | YYRGLDVSI | 80 | 2082 |
| | NS3 | | | TFTIETTTL | 66 | 1823 |
| | NS3 | | | FWESVFTGL | 52 | 234 |
| | NS3 | | | FWEAVFTGL | 56 | 1099 |

Table continued

| Epimmune | | | | | | |
|---|---|---|---|---|---|---|
| | Protein | CS_fr | CS_to | pep_seq | Score PIC | SEQ ID NO |
| | NS3 | | | SWDVMWKCLI | 59 | 1805 |
| | NS3 | | | IMACMSADL | 94 | 90 |
| | NS3 | | | VMACMSADL | 60 | 1936 |
| | | | | PYIEQAQAI | 58 | 1593 |
| | | | | NWQKLEAFW | 20 | 1542 |
| | NS4B | | | TFWAKHMWNF | 50 | 1824 |
| | NS4B | | | AFWAKHMWNF | 87 | 803 |
| | NS4B | | | QFWAKHMWNF | 93 | 1604 |
| | NS4B | | | VFWAKHMWNF | 28 | 1909 |
| | NS4B | | | FWAKHMWNF | 0,23 | 1095 |
| | NS4B | | | FWANDMWNF | 0,19 | 1097 |
| | NS4B | | | FWARHMWNF | 0,16 | 1098 |
| | NS4B | | | NFISGIQYL | 91 | 1521 |
| | | | | SMMAFSAAL | 96 | 1751 |
| | NS5A | | | SWLRDVWDW | 26 | 1807 |
| | NS5A | | | RYAPPCKPL | 32 | 1715 |
| | NS5A | | | RYAPPCKPLL | 20 | 1716 |
| | NS5A | | | KFPPALPIW | 5,1 | 1322 |
| | NS5A | | | KYPPALPIW | 0,75 | 1355 |
| | | | | DYNPPLLETW | 77 | 996 |
| | NS5B | | | SYTWTGALI | 20 | 1810 |
| | NS5B | | | SYSWTGALI | 42 | 1809 |
| | NS5B | | | HYRDVLKEM | 18 | 1253 |
| | NS5B | | | LYDVIQKLSI | 68 | 1486 |
| | NS5B | | | RMILMTHFF | 6,6 | 59 |
| | NS5B | | | RMVLMTHFF | 13 | 1671 |
| | NS5B | | | LMTHFFSILL | 80 | 1415 |

**Table 5 Predicted HLA-B*0702 binding peptides**

| Protein | | CS_fr | CS_to | pep_seq | Score | SEQ ID NO |
|---|---|---|---|---|---|---|
| **Algonomics 9-mer** | | | | | | |
| 1 | NS5B | 2836 | 2844 | APTLWARMI | S | 371 |
| 2 | NS3 | 1503 | 1511 | TPGERPSGM | S | 372 |
| 3 | NS5B | 2616 | 2624 | SPGQRVEFL | S | 373 |
| 4 | C | 111 | 119 | DPRRRSRNL | S | 374 |
| 5 | C | 169 | 177 | LPGCSFSIF | S | 375 |

Table continued

| Protein | | CS_fr | CS_to | pep_seq | Score | SEQ ID NO |
|---|---|---|---|---|---|---|
| **Algonomics 9-mer** | | | | | | |
| 6 | NS3 | 1383b | 1391b | EVIKGGRHL | M | 376 |
| 7 | NS3 | 1383 | 1391 | EAIKGGRHL | M | 377 |
| 8 | C | 83 | 91 | WPLYGNEGL | M | 378 |
| 9 | C | 150 | 158 | ALAHGVRVL | M | 379 |
| 10 | C | 37 | 45 | LPRRGPRLG | M | 380 |
| 11 | NS3 | 1599 | 1607 | APPPSWDQM | M | 381 |
| 12 | NS3 | 1620 | 1628 | GPTPLLYRL | M | 382 |
| 13 | NS3 | 1531 | 1539 | TPAETSVRL | M | 383 |
| 14 | C | 142 | 150 | APLGGAARA | M | 384 |
| 15 | NS3 | 1260 | 1268 | ATLGFGAYM | M | 385 |
| 16 | C | 99 | 107 | SPRGSRPSW | M | 386 |
| 17 | C | 41 | 49 | GPRLGVRAT | M | 387 |
| 18 | NS5B | 2668 | 2676 | EARQAIRSL | M | 388 |
| 19 | NS3 | 1622 | 1630 | TPLLYRLGA | M | 389 |
| 20 | C | 57 | 65 | QPRGRRQPI | M | 390 |
| 21 | NS3 | 1244 | 1252 | YAAQGYKVL | M | 391 |
| 22 | NS3 | 1357 | 1365 | VPHPNIEEI | M | 392 |
| 23 | NS5B | 2605 | 2613 | AVMGSSYGF | M | 393 |
| 24 | NS3 | 1415 | 1423 | VAYYRGLDV | M | 394 |
| 25 | NS3 | 1359 | 1367 | HPNIEEIGL | M | 395 |
| 26 | NS3 | 1639 | 1647 | HPITKYIMA | M | 396 |
| 27 | NS3 | 1230 | 1238 | APTGSGKST | M | 397 |
| 28 | NS3 | 1560 | 1568 | SVFTGLTHI | M | 398 |
| 29 | NS3 | 1171 | 1179 | CPSGHVVGV | M | 399 |
| 30 | NS3 | 1413 | 1421 | NAVAYYRGL | M | 400 |
| 31 | NS5B | 2720 | 2728 | SAACRAAKL | M | 401 |
| 32 | NS3 | 1404 | 1412 | AAKLSALGL | M | 402 |
| 33 | C | 147 | 155 | AARALAHGV | M | 403 |
| 34 | C | 4 | 12 | NPKPQRKTK | W | 404 |
| 35 | NS5B | 2666 | 2674 | APEARQAIR | W | 405 |
| 36 | C | 115 | 123 | RSRNLGKVI | W | 406 |
| 37 | C | 24 | 32 | FPGGGQIVG | W | 407 |
| 38 | NS3 | 1289 | 1297 | APITYSTYG | W | 408 |
| 39 | C | 65 | 73 | IPKARRPEG | W | 409 |
| 40 | NS3 | 1219 | 1227 | VPQTFQVAH | W | 410 |
| 41 | NS5B | 2826 | 2834 | NSWLGNIIM | W | 411 |
| 42 | C | 149 | 157 | RALAHGVRV | W | 412 |

Table continued

| Protein | | CS_fr | CS_to | pep_seq | Score | SEQ ID NO |
|---|---|---|---|---|---|---|
| Algonomics 9-mer | | | | | | |
| 43 | NS3 | 1490 | 1498 | RTGRGRRGI | W | 413 |
| 44 | NS3 | 1641 | 1649 | ITKYIMACM | W | 414 |
| 45 | NS3 | 1426 | 1434 | IPTSGDVVV | W | 415 |
| 46 | NS3 | 1616 | 1624 | PTLHGPTPL | W | 416 |
| 47 | NS3 | 1373 | 1381 | IPFYGKAIP | W | 417 |
| 48 | NS5B | 2835 | 2843 | YAPTLWARM | W | 418 |
| 49 | NS5B | 2796 | 2804 | ASGKRVYYL | W | 419 |
| 50 | NS3 | 1338 | 1346 | TAGARLVVL | W | 420 |
| 51 | NS5B | 2633 | 2641 | CPMGFSYDT | W | 421 |
| 52 | NS3 | 1384 | 1392 | AIKGGRHLI | W | 422 |
| 53 | NS3 | 1255 | 1263 | NPSVAATLG | W | 423 |
| 54 | NS3 | 1325 | 1333 | TILGIGTVL | W | 424 |
| 55 | NS3 | 1380 | 1388 | IPIEAIKGG | W | 425 |
| 56 | NS3 | 1637 | 1645 | LTHPITKYI | W | 426 |
| 57 | NS3 | 1252 | 1260 | LVLNPSVAA | W | 427 |
| 58 | NS5B | 2678 | 2686 | ERLYIGGPL | W | 428 |
| 59 | NS3 | 1188 | 1196 | GVAKAVDFI | W | 429 |
| 60 | NS5B | 2568 | 2576 | QPEKGGRKP | W | 430 |
| 61 | C | 104 | 112 | RPSWGPTDP | W | 431 |
| 62 | C | 161 | 169 | GVNYATGNL | W | 432 |
| 63 | NS3 | 1402 | 1410 | ELAAKLSAL | W | 433 |
| 64 | NS3 | 1540 | 1548 | RAYLNTPGL | W | 434 |
| 65 | NS5B | 2572 | 2580 | GGRKPARLI | W | 435 |
| 66 | NS3 | 1277 | 1285 | NIRTGVRTI | W | 436 |
| 67 | NS3 | 1433 | 1441 | VVVATDALM | W | 437 |
| 68 | NS3 | 1246 | 1254 | AQGYKVLVL | W | 438 |
| 69 | NS3 | 1337 | 1345 | ETAGARLVV | W | 439 |
| 70 | NS5B | 2725 | 2733 | AAKLQDCTM | W | 440 |
| 71 | NS3 | 1162 | 1170 | KGSSGGPLL | W | 441 |
| 72 | C | 137 | 145 | IPLVGAPLG | W | 442 |
| 73 | NS3 | 1583 | 1591 | FPYLVAYQA | N | 443 |
| 74 | C | 93 | 101 | WAGWLLSPR | N | 444 |
| 75 | C | 78 | 86 | QPGYPWPLY | N | 445 |
| 76 | C | 179 | 187 | LALLSCLTI | N | 446 |
| 77 | C | 154 | 162 | GVRVLEDGV | N | 447 |
| 78 | C | 77 | 85 | AQPGYPWPL | N | 448 |
| 79 | C | 38 | 46 | PRRGPRLGV | N | 449 |

Table continued

| Protein | | CS_fr | CS_to | pep_seq | Score | SEQ ID NO |
|---|---|---|---|---|---|---|
| **Algonomics 9-mer** | | | | | | |
| 80 | C | 37 | 46 | LPRRGPRLGV | S | 450 |
| | | | | | | |
| **Epimmune** | | | | | | |
| | **Protein** | **CS_fr** | **CS_to** | **pep_seq** | **Score** PIC | **SEQ ID NO** |
| | C | | | LPRRGPRLGV | 4,9 | 450 |
| | C | | | QPRGRRQPI | 3,0 | 390 |
| | C | | | QPRRRRQPI | 3,9 | 1617 |
| | C | | | QPGYPWPLY | 39918 | 216 |
| | C | | | SPRGSRPSW | 1,6 | 386 |
| | C | | | SPRGSRPNW | 11 | 1772 |
| | C | | | SPRGSRPTW | 2,8 | 1774 |
| | C | | | DPRRSRNL | 12 | 370 |
| | C | | | APLGGAARAL | 3,5 | 836 |
| | C | | | APLGGVARAL | 5,5 | 837 |
| | C | | | APVGGVARAL | 8,1 | 855 |
| | C | | | LPGCSFSIF | 101 | 375 |
| | C | | | LPGCSFSIFL | 32 | 1426 |
| | E1 | | | YPGHVSGHRM | 264 | 2063 |
| | E2 | | | APRPCGIVPA | 38 | 847 |
| | E2 | | | RPCGIVPAL | 1,9 | 1675 |
| | E2 | | | VPARSVCGPV | 48 | 1945 |
| | E2 | | | VPASSVCGPV | 54 | 1947 |
| | E2 | | | GPWLTPRCL | 64 | 1173 |
| | E2 | | | GPWLTPRCM | 105 | 1175 |
| | E2 | | | TPRCLVDYPY | 238 | 1857 |
| | E2 | | | TPRCMVDYPY | 445 | 1858 |
| | E2 | | | YPCTVNFTI | 71 | 2059 |
| | E2 | | | YPCTVNFSI | 42 | 2058 |
| | E2 | | | YPCTVNFTL | 17 | 2061 |
| | E2 | | | YPCTVNFTIF | 307 | 2060 |
| | | | | LPCSFSDLPA | 100 | 1423 |
| | | | | LPALSTGLL | 41 | 1420 |
| | P7 | | | VPGAAYALY | 27777 | 1949 |
| | P7 | | | WPLLLLLAL | 7,5 | 2018 |
| | | | | VPYFVRAHAL | 9,1 | 1959 |
| | | | | TPYFVRAHVL | 32 | 1863 |

Table continued

| Epimmune | | | | | | |
|---|---|---|---|---|---|---|
| | Protein | CS_fr | CS_to | pep_seq | Score PIC | SEQ ID NO |
| | | | | SPMEKKVIV | 31 | 1769 |
| | | | | GPKGPVTQM | 86 | 1166 |
| | | | | CPSGHVVGI | 62 | 933 |
| | | | | CPRGHAVGI | 3,6 | 929 |
| | | | | CPAGHAVGIF | 56 | 925 |
| | | | | CPRGHAVGIF | 6,9 | 930 |
| | NS3 | | | VPAAYAAQGY | 2734 | 1943 |
| | NS3 | | | NPSVAATLGF | 1610 | 1532 |
| | NS3 | | | IPFYGKAIPI | 29 | 1284 |
| | NS3 | | | IPFYGKAIPL | 7,9 | 1285 |
| | NS3 | | | TPGERPSGM | 834 | 372 |
| | NS3 | | | TPGERPSGMF | 699 | 1845 |
| | NS3 | | | RPSGMFDSV | 9,4 | 1688 |
| | NS3 | | | RPSGMFDSSV | 37 | 1687 |
| | NS3 | | | RPSGMFDSVV | 58 | 1689 |
| | NS3 | | | LPVCQDHLEF | 5715 | 1444 |
| | NS3 | | | APPPSWDQM | 933 | 381 |
| | NS3 | | | KPTLHGPTPL | 7,9 | 1343 |
| | NS3 | | | KPTLVGPTPL | 34 | 1345 |
| | NS3 | | | KPTLQGPTPL | 47 | 1344 |
| | NS3 | | | HPITKYIMA | 39 | 396 |
| | NS3 | | | HPVTKYIMA | 48 | 1238 |
| | NS4B | | | LPYIEQGMQL | 43 | 1447 |
| | NS4B | | | APYIEQAQAI | 44 | 857 |
| | NS4B | | | NPAIASLMAF | 7,2 | 1528 |
| | NS4B | | | NPAVASMMAF | 14 | 1530 |
| | NS4B | | | NPAVASLMAF | 11 | 1529 |
| | NS4B | | | SPLTTNQTM | 80 | 1766 |
| | NS4B | | | APPSAASAFV | 76 | 845 |
| | NS4B | | | LPAILSPGAL | 4,4 | 1418 |
| | NS4B | | | GPGEGAVQWM | 976 | 1163 |
| | | | | KPAPNFKTAI | 26 | 1335 |
| | NS5A | | | EPDVAVLTSM | 597 | 1023 |
| | NS5A | | | LPKSRFPPA | 50 | 1432 |
| | NS5A | | | LPKSRFPPAL | 14 | 1433 |
| | NS5A | | | LPIWARPDY | 4290 | 1431 |
| | NS5A | | | RPDYNPPLL | 73 | 1677 |

Table continued

| Epimmune | | | | | | |
|---|---|---|---|---|---|---|
| | Protein | CS_fr | CS_to | pep_seq | Score PIC | SEQ ID NO |
| | NS5A | | | VPPVVHGCPL | 26 | 1953 |
| | | | | PPRKKRTVV | 31 | 1577 |
| | NS5B | | | LPINALSNSL | 46 | 1430 |
| | NS5B | | | TPPHSAKSKF | 699 | 1856 |
| | NS5B | | | PPHSAKSKF | 9170 | 1568 |
| | NS5B | | | PPHSARSKF | 4229 | 1569 |
| | NS5B | | | SPGQRVEFL | 21 | 373 |
| | NS5B | | | SPAQRVEFL | 7,6 | 1757 |
| | NS5B | | | LPTSFGNTI | 61 | 1443 |
| | NS5B | | | PPGDPPQPEY | 633519 | 1566 |
| | NS5B | | | APTLWARMI | 14 | 371 |
| | NS5B | | | APTIWVRMV | 19 | 850 |
| | NS5B | | | APTLWARMIL | 1,2 | 853 |
| | NS5B | | | APTIWVRMVL | 1,9 | 851 |
| | NS5B | | | RPRLLLLGL | 0,17 | 1682 |
| | NS5B | | | RPRLLLLGLL | 0,72 | 1683 |

**Table 6 Predicted HLA-B*0801 binding peptides**

| | Protein | CS_fr | CS_to | pep_seq | Score | SEQ ID NO |
|---|---|---|---|---|---|---|
| **Algonomics 9-mer** | | | | | | |
| 1 | C | 111 | 119 | DPRRRSRNL | S | 451 |
| 2 | C | 57 | 65 | QPRGRRQPI | S | 452 |
| 3 | C | 65 | 73 | IPKARRPEG | S | 453 |
| 4 | NS3 | 1639 | 1647 | HPITKYIMA | S | 454 |
| 5 | NS3 | 1395 | 1403 | HSKKKCDEL | S | 455 |
| 6 | NS3 | 1486 | 1494 | QRRGRTGRG | M | 456 |
| 7 | C | 4 | 12 | NPKPQRKTK | M | 457 |
| 8 | NS5B | 2798 | 2806 | GKRVYYLTR | M | 458 |
| 9 | NS3 | 1536 | 1544 | SVRLRAYLN | M | 459 |
| 10 | C | 132 | 140 | DLMGYIPLV | M | 460 |
| 11 | NS3 | 1413 | 1421 | NAVAYYRGL | M | 461 |
| 12 | C | 72 | 80 | EGRTWAQPG | M | 462 |
| 13 | NS3 | 1641 | 1649 | ITKYIMACM | M | 463 |
| 14 | NS3 | 1494 | 1502 | GRRGIYRFV | M | 464 |
| 15 | NS5B | 2838 | 2846 | TLWARMILM | M | 465 |
| 16 | NS5B | 2640 | 2648 | DTRCFDSTV | M | 466 |

Table continued

| | Protein | CS_fr | CS_to | pep_seq | Score | SEQ ID NO |
|---|---|---|---|---|---|---|
| **Algonomics 9-mer** | | | | | | |
| 17 | NS3 | 1606 | 1614 | QMWKCLIRL | M | 467 |
| 18 | NS3 | 1611 | 1619 | LIRLKPTLH | M | 468 |
| 19 | NS3 | 1415 | 1423 | VAYYRGLDV | M | 469 |
| 20 | NS3 | 1637 | 1645 | LTHPITKYI | M | 470 |
| 21 | NS5B | 2840 | 2848 | WARMILMTH | M | 471 |
| 22 | NS3 | 1583 | 1591 | FPYLVAYQA | M | 472 |
| 23 | NS5B | 2672 | 2680 | AIRSLTERL | M | 473 |
| 24 | NS5B | 2668 | 2676 | EARQAIRSL | M | 474 |
| 25 | NS5B | 2696 | 2704 | YRRCRASGV | M | 475 |
| 26 | C | 8 | 16 | QRKTKRNTN | W | 476 |
| 27 | NS3 | 1393 | 1401 | FCHSKKKCD | W | 477 |
| 28 | NS5B | 2627 | 2635 | AWKSKKCPM | W | 478 |
| 29 | C | 63 | 71 | QPIPKARRP | W | 479 |
| 30 | NS3 | 1553 | 1561 | DHLEFWESV | W | 480 |
| 31 | NS5B | 2797 | 2805 | SGKRVYYLT | W | 481 |
| 32 | NS3 | 1376 | 1384 | YGKAIPIEA | W | 482 |
| 33 | NS3 | 1234 | 1242 | SGKSTKVPA | W | 483 |
| 34 | NS3 | 1622 | 1630 | TPLLYRLGA | W | 484 |
| 35 | NS5B | 2831 | 2839 | NIIMYAPTL | W | 485 |
| 36 | NS3 | 1376b | 1384b | YGKAIPIEV | W | 486 |
| 37 | C | 33 | 41 | GVYLLPRRG | W | 487 |
| 38 | NS5B | 2761 | 2769 | EAMTRYSAP | W | 488 |
| 39 | C | 89 | 97 | EGLGWAGWL | W | 489 |
| 40 | NS3 | 1491 | 1499 | TGRGRRGIY | W | 490 |
| 41 | NS3 | 1384b | 1392b | VIKGGRHLI | W | 491 |
| 42 | NS3 | 1387 | 1395 | GGRHLIFCH | W | 492 |
| 43 | NS3 | 1569 | 1577 | DAHFLSQTK | W | 493 |
| 44 | NS5B | 2714 | 2722 | TCYLKASAA | W | 494 |
| 45 | NS5B | 2755 | 2763 | SLRVFTEAM | W | 495 |
| 46 | NS3 | 1480 | 1488 | DAVSRSQRR | W | 496 |
| 47 | NS3 | 1605 | 1613 | DQMWKCLIR | W | 497 |
| 48 | NS5B | 2586 | 2594 | GVRVCEKMA | W | 498 |
| 49 | NS3 | 1237 | 1245 | STKVPAAYA | W | 499 |
| 50 | NS5B | 2812 | 2820 | LARAAWETA | W | 500 |
| 51 | C | 36 | 44 | LLPRRGPRL | W | 501 |
| 52 | NS3 | 1294 | 1302 | STYGKFLAD | W | 502 |
| 53 | NS5B | 2572 | 2580 | GGRKPARLI | W | 503 |

Table continued

| | Protein | CS_fr | CS_to | pep_seq | Score | SEQ ID NO |
|---|---|---|---|---|---|---|
| **Algonomics 9-mer** | | | | | | |
| 54 | NS3 | 1384 | 1392 | AIKGGRHLI | W | 504 |
| 55 | C | 60 | 68 | GRRQPIPKA | W | 505 |
| 56 | NS3 | 1610 | 1618 | CLIRLKPTL | W | 506 |
| 57 | NS5B | 2573 | 2581 | GRKPARLIV | W | 507 |
| 58 | NS5B | 2833 | 2841 | IMYAPTLWA | W | 508 |
| 59 | C | 115 | 123 | RSRNLGKVI | W | 509 |
| 60 | NS3 | 1372 | 1380 | EIPFYGKAI | W | 510 |
| 61 | NS3 | 1277 | 1285 | NIRTGVRTI | W | 511 |
| 62 | C | 35 | 43 | YLLPRRGPR | W | 512 |
| 63 | C | 147 | 155 | AARALAHGV | W | 513 |
| 64 | C | 37 | 45 | LPRRGPRLG | W | 514 |
| | | | | | | |
| **Epimmune** | | | | | | |
| | Protein | CS_fr | CS_to | pep_seq | Score | SEQ ID NO |
| | C | | | TNRRPQDVKF | | 1838 |
| | C | | | NRRPQDVKF | | 685 |
| | C | | | DVKFPGGGQI | | 990 |
| | C | | | YLLPRRGPRL | | 2047 |
| | C | | | LLPRRGPRL | | 132 |
| | C | | | QPRGRRQPI | | 390 |
| | C | | | TDPRRRSRNL | | 1817 |
| | C | | | DPRRRSRNL | | 370 |
| | C | | | RSRNLGKVI | | 318 |
| | C | | | RNLGKVIDTL | | 1673 |
| | E2 | | | WTRGERCDL | | 2022 |
| | E2 | | | DLEDRDRSEL | | 964 |
| | E2 | | | RDRSELSPL | | 1636 |
| | E2 | | | RDRSELSPLL | | 1637 |
| | E2 | | | LADARVCACL | | 1358 |
| | NS2 | | | NVRGGRDAI | | 1538 |
| | NS2 | | | NVRGGRDAII | | 1539 |
| | NS2 | | | VRGGRDAII | | 1965 |
| | NS2 | | | VRGGRDAIIL | | 1966 |
| | NS2 | | | GGRDAIILL | | 1138 |
| | NS2 | | | PVSARRGREI | | 1589 |
| | NS2 | | | VSARRGREI | | 1969 |

Table continued

| Epimmune | | | | | | |
|---|---|---|---|---|---|---|
| | Protein | CS_fr | CS_to | pep_seq | Score | SEQ ID NO |
| | NS2 | | | VSARRGREIL | | 1970 |
| | NS2 | | | SARRGREIL | | 1718 |
| | NS2 | | | SARRGREILL | | 1719 |
| | NS2 | | | ARRGREILL | | 865 |
| | NS3 | | | QTRGLLGCI | | 1621 |
| | NS3 | | | QTRGLLGCII | | 1622 |
| | NS3 | | | YLVTRHADVI | | 2054 |
| | NS3 | | | RRRGDSRGSL | | 1697 |
| | NS3 | | | RGDSRGSLL | | 1648 |
| | NS3 | | | YLKGSSGGPL | | 2046 |
| | NS3 | | | RGVAKAVDF | | 317 |
| | NS3 | | | RGVAKAVDFI | | 1653 |
| | NS3 | | | ETTMRSPVF | | 257 |
| | NS3 | | | AQGYKVLVL | | 130 |
| | NS3 | | | AYMSKAHGI | | 904 |
| | NS3 | | | NIRTGVRTI | | 436 |
| | NS3 | | | TAGARLVVL | | 249 |
| | NS3 | | | PFYGKAIPI | | 264 |
| | NS3 | | | PFYGKAIPL | | 1554 |
| | NS3 | | | IKGGRHLIF | | 311 |
| | NS3 | | | CHSKKKCDEL | | 918 |
| | NS3 | | | HSKKKCDEL | | 455 |
| | NS3 | | | YYRGLDVSVI | | 2083 |
| | NS3 | | | TPGERPSGMF | | 1845 |
| | NS3 | | | DQMWKCLIRL | | 982 |
| | NS3 | | | KCLIRLKPTL | | 1319 |
| | NS4B | | | AEQFKQKAL | | 794 |
| | NS4B | | | QFKQKALGL | | 1602 |
| | NS4B | | | QFKQKALGLL | | 1603 |
| | NS4B | | | WAKHMWNFI | | 1993 |
| | NS4B | | | IGLGKVLVDI | | 1267 |
| | NS4B | | | LGKVLVDIL | | 1382 |
| | NS4B | | | QWMNRLIAF | | 1623 |
| | NS5A | | | KGVWRGDGI | | 1326 |
| | NS5A | | | LARGSPPSL | | 1363 |
| | NS5A | | | LWRQEMGGNI | | 1485 |
| | NS5A | | | ESENKVVIL | | 1030 |

Table continued

| Epimmune | | | | | | |
|---|---|---|---|---|---|---|
| | **Protein** | **CS_fr** | **CS_to** | **pep_seq** | **Score** | **SEQ ID NO** |
| | NS5A | | | ENKVVILDSF | | 1021 |
| | NS5A | | | WARPDYNPPL | | 1998 |
| | NS5B | | | RQKKVTFDRL | | 1695 |
| | NS5B | | | QKKVTFDRL | | 1607 |
| | NS5B | | | VTFDRLQVL | | 1975 |
| | NS5B | | | TIMAKNEVF | | 1827 |
| | NS5B | | | EKGGRKPARL | | 1015 |
| | NS5B | | | KGGRKPARL | | 1323 |
| | NS5B | | | KGGRKPARLI | | 1324 |
| | NS5B | | | GGRKPARLI | | 435 |
| | NS5B | | | GRKPARLIVF | | 1182 |
| | NS5B | | | RKPARLIVF | | 646 |
| | NS5B | | | PARLIVFPDL | | 1545 |
| | NS5B | | | GVRVCEKMAL | | 1203 |
| | NS5B | | | SPGQRVEFL | | 373 |
| | NS5B | | | YRRCRASGVL | | 2066 |
| | NS5B | | | LTRDPTTPL | | 1475 |
| | NS5B | | | WARMILMTHF | | 1997 |
| | NS5B | | | IERLHGLSAF | | 1264 |
| | NS5B | | | IQRLHGLSAF | | 1298 |
| | NS5B | | | CLRKLGVPPL | | 921 |
| | NS5B | | | LRKLGVPPL | | 1455 |
| | NS5B | | | RARSVRAKL | | 1632 |
| | NS5B | | | RARSVRAKLL | | 1633 |
| | NS5B | | | ARSVRAKLL | | 867 |
| | NS5B | | | NWAVKTKLKL | | 1540 |
| | NS5B | | | NWAVRTKLKL | | 1541 |
| | NS5B | | | RTKLKLTPI | | 1705 |
| **Algonomics 10-mer** | | | | | | |
| | **Protein** | **CS_fr** | CS_to | **pep_seq** | **Score** | **SEQ ID NO** |
| | Core | 65 | 74 | IPKARRPEGR | S | 1287 |
| | Core | 4 | 13 | NPKPQRKTKR | M | 1531 |
| | Ns3 | 1395 | 1403 | HSKKKCDELA | M | 1243 |
| | Core | 111 | 120 | DPRRSRNLG | M | 975 |
| | Core | 37 | 46 | LPRRGPRLGV | M | 450 |
| | Core | 8 | 17 | QRKTKRNTNR | M | 1618 |

Table continued

| Algonomics 10-mer | Protein | CS_fr | CS_to | pep_seq | Score | SEQ ID NO |
|---|---|---|---|---|---|---|
| | Core | 21 | 30 | DVKFPGGGQI | M | 990 |
| | Ns5b | 2696 | 1655 | YRRCRASGVL | M | 2066 |
| | Ns5b | 2626 | 1655 | NAWKSKKCPM | M | 1514 |
| | Core | 57 | 66 | QPRGRRQPIP | M | 1616 |
| | Ns3 | 1491 | 1499 | TGRGRRGIYR | M | 1825 |
| | Ns3 | 1605 | 1613 | DQMWKCLIRL | M | 982 |
| | Ns3 | 1291 | 1299 | ITYSTYGKFL | M | 1310 |
| | Ns3 | 1234 | 1242 | SGKSTKVPAA | M | 1734 |
| | Ns3 | 1376 | 1384 | YGKAIPIEVI | M | 2035 |
| | Core | 35 | 44 | YLLPRRGPRL | M | 2047 |
| | Ns5b | 2797 | 1655 | SGKRVYYLTR | M | 1733 |
| | Ns3 | 1493 | 1501 | RGRRGIYRFV | W | 1650 |
| | Ns4b | 1844 | 1655 | LGKVLVDILA | W | 1383 |
| | Core | 89 | 98 | EGMGWAGWLL | W | 1012 |
| | Ns3 | 1237 | 1245 | STKVPAAYAA | W | 1790 |
| | Ns3 | 1189 | 1197 | VAKAVDFIPV | W | 1893 |
| | Ns3 | 1609 | 1617 | KCLIRLKPTL | W | 1319 |
| | Ns3 | 1494 | 1502 | GRRGIYRFVT | W | 1183 |
| | Ns3 | 1393 | 1401 | FCHSKKKCDE | W | 1051 |
| | Ns3 | 1637 | 1645 | LTHPITKYIM | W | 1469 |
| | Ns3 | 1482 | 1490 | VSRSQRRGRT | W | 1972 |
| | Ns5b | 2677 | 1655 | TERLYIGGPL | W | 1820 |
| | Ns3 | 1340 | 1348 | GARLVVLATA | W | 1106 |
| | Ns5b | 2761 | 1655 | EAMTRYSAPP | W | 999 |
| | Ns5b | 2627 | 1655 | AWKSKKCPMG | W | 899 |
| | Ns5b | 2573 | 1655 | GRKPARLIVF | W | 1182 |
| | Ns5b | 2572 | 1655 | GGRKPARLIV | W | 1139 |
| | Ns3 | 1622 | 1630 | TPLLYRLGAV | W | 1851 |
| | Core | 99 | 108 | SPRGSRPSWG | W | 1773 |
| | Ns3 | 1611 | 1619 | LIRLKPTLHG | W | 1387 |
| | Core | 41 | 50 | GPRLGVRATR | W | 1170 |
| | Ns5b | 2671 | 1655 | QAIRSLTERL | W | 1597 |
| | Core | 132 | 141 | DLMGYIPLVG | W | 965 |
| | Ns5b | 2586 | 1655 | GVRVCEKMAL | W | 1203 |
| | Core | 59 | 68 | RGRRQPIPKA | W | 1651 |
| | Ns3 | 1488 | 1496 | RGRTGRGRRG | W | 1652 |
| | Ns3 | 1294 | 1302 | STYGKFLADG | W | 1795 |

Table continued

| Algonomics 10-mer | | | | | | |
|---|---|---|---|---|---|---|
| | Protein | CS_fr | CS_to | pep_seq | Score | SEQ ID NO |
| | Ns3 | 1486 | 1494 | QRRGRTGRGR | W | 1619 |
| | Ns3 | 1384 | 1392 | VIKGGRHLIF | W | 1917 |
| | Ns3 | 1536 | 1544 | SVRLRAYLNT | W | 1802 |
| | Ns3 | 1373 | 1381 | IPFYGKAIPI | W | 1284 |
| | Ns5b | 2795 | 1655 | DASGKRVYYL | W | 955 |
| | Ns3 | 1485 | 1493 | SQRRGRTGRG | W | 1783 |
| | Ns3 | 1552 | 1560 | QDHLEFWESV | W | 1600 |
| | Core | 45 | 54 | GVRATRKTSE | W | 1200 |
| | Ns5b | 2716 | 1655 | YLKASAACRA | W | 2045 |
| | Ns5b | 2725 | 1655 | AAKLQDCTML | W | 775 |
| | Ns3 | 1160 | 1169 | YLKGSSGGPL | W | 2046 |
| | Core | 113 | 122 | RRRSRNLGKV | W | 1698 |
| | Ns3 | 1242 | 1250 | AAYAAQGYKV | W | 783 |
| | Ns3 | 1606 | 1614 | QMWKCLIRLK | W | 1610 |
| | Core | 68 | 77 | ARRPEGRAWA | W | 866 |
| | Ns5b | 2694 | 1655 | CGYRRCRASG | W | 917 |
| | Ns3 | 1639 | 1647 | HPITKYIMAC | W | 1236 |
| | Ns5b | 2840 | 1655 | WARMILMTHF | W | 1997 |

## Table 7 Predicted HLA-B*3501 binding peptides

| | Protein | CS_fr | CS_to | pep_seq | Score | SEQ ID NO |
|---|---|---|---|---|---|---|
| Algonomics 9-mer | | | | | | |
| 1 | C | 169 | 177 | LPGCSFSIF | S | 515 |
| 2 | NS3 | 1464 | 1472 | FSLDPTFTI | M | 516 |
| 3 | NS5B | 2605 | 2613 | AVMGSSYGF | M | 517 |
| 4 | NS3 | 1583 | 1591 | FPYLVAYQA | M | 518 |
| 5 | C | 24 | 32 | FPGGGQIVG | M | 519 |
| 6 | NS5B | 2607 | 2615 | MGSSYGFQY | M | 520 |
| 7 | NS3 | 1531 | 1539 | TPAETSVRL | M | 521 |
| 8 | C | 156 | 164 | RVLEDGVNY | M | 522 |
| 9 | NS3 | 1359 | 1367 | HPNIEEIGL | M | 523 |
| 10 | NS3 | 1581 | 1589 | DNFPYLVAY | W | 524 |
| 11 | NS5B | 2795 | 2803 | DASGKRVYY | W | 525 |
| 12 | NS3 | 1456 | 1464 | TCVTQTVDF | W | 526 |
| 13 | NS3 | 1175 | 1183 | HVVGVFRAA | W | 527 |
| 14 | NS3 | 1522 | 1530 | DAGCAWYEL | W | 528 |

Table continued

|  | Protein | CS_fr | CS_to | pep_seq | Score | SEQ ID NO |
|---|---|---|---|---|---|---|
| **Algonomics 9-mer** |  |  |  |  |  |  |
| 15 | NS5B | 2826 | 2834 | NSWLGNIIM | W | 529 |
| 16 | NS3 | 1438 | 1446 | DALMTGYTG | W | 530 |
| 17 | NS3 | 1367 | 1375 | LSNNGEIPF | W | 531 |
| 18 | NS5B | 2615 | 2623 | YSPGQRVEF | W | 532 |
| 19 | NS5B | 2840 | 2848 | WARMILMTH | W | 533 |
| 20 | NS3 | 1357 | 1365 | VPHPNIEEI | W | 534 |
| 21 | C | 78 | 86 | QPGYPWPLY | W | 535 |
| 22 | NS5B | 2720 | 2728 | SAACRAAKL | W | 536 |
| 23 | NS3 | 1289 | 1297 | APITYSTYG | W | 537 |
| 24 | C | 83 | 91 | WPLYGNEGL | W | 538 |
| 25 | NS3 | 1620 | 1628 | GPTPLLYRL | W | 539 |
| 26 | C | 174 | 182 | FSIFLLALL | W | 540 |
| 27 | NS3 | 1171 | 1179 | CPSGHVVGV | W | 541 |
| 28 | NS5B | 2633 | 2641 | CPMGFSYDT | W | 542 |
| 29 | NS5B | 2772 | 2780 | DPPQPEYDL | W | 543 |
| 30 | NS3 | 1219 | 1227 | VPQTFQVAH | W | 544 |
| 31 | C | 149 | 157 | RALAHGVRV | W | 545 |
| 32 | NS3 | 1433 | 1441 | VVVATDALM | W | 546 |
| 33 | C | 137 | 145 | IPLVGAPLG | W | 547 |
| 34 | NS3 | 1622 | 1630 | TPLLYRLGA | W | 548 |
| 35 | NS3 | 1240 | 1248 | VPAAYAAQG | W | 549 |
| 36 | NS3 | 1410 | 1418 | LGLNAVAYY | W | 550 |
| 37 | NS3 | 1578 | 1586 | QAGDNFPYL | W | 551 |
| 38 | C | 18 | 26 | RPQDVKFPG | W | 552 |
| 39 | NS5B | 2588 | 2596 | RVCEKMALY | W | 553 |
| 40 | NS5B | 2740 | 2748 | LVVICESAG | W | 554 |
| 41 | C | 142 | 150 | APLGGAARA | W | 555 |
| 42 | C | 172 | 180 | CSFSIFLLA | W | 556 |
| 43 | NS3 | 1259 | 1267 | AATLGFGAY | W | 557 |
| 44 | C | 128 | 136 | CGFADLMGY | W | 558 |
| 45 | NS5B | 2794 | 2802 | HDASGKRVY | W | 559 |
| 46 | NS3 | 1260 | 1268 | ATLGFGAYM | W | 560 |
| 47 | NS3 | 1380b | 1388b | IPIEVIKGG | W | 561 |
| 48 | NS5B | 2751 | 2759 | EDAASLRVF | W | 562 |
|  |  |  |  |  |  |  |

Table continued

| Algonomics 10-mer | | | | | | |
|---|---|---|---|---|---|---|
| | Protein | CS_fr | CS_to | pep_seq | Score | SEQ ID NO |
| 1 | Ns3 | 1548 | 1556 | LPVCQDHLEF | S | 1444 |
| 2 | Core | 169 | 178 | LPGCSFSIFL | M | 1426 |
| 3 | Ns3 | 1196 | 1204 | IPVESMETTM | M | 1296 |
| 4 | Ns3 | 1408 | 1416 | SALGLNAVAY | M | 1717 |
| 5 | Ns5b | 2604 | 1655 | QAVMGSSYGF | M | 1599 |
| 6 | Ns4b | 1873 | 1655 | MPSTEDLVNL | M | 1505 |
| 7 | Core | 24 | 33 | FPGGGQIVGG | M | 1077 |
| 8 | Ns3 | 1373 | 1381 | IPFYGKAIPI | M | 1284 |
| 9 | Ns3 | 1255 | 1263 | NPSVAATLGF | M | 1532 |
| 10 | Ns3 | 1521 | 1529 | YDAGCAWYEL | M | 2030 |
| 11 | Ns3 | 1171 | 1180 | CPSGHAVGIF | W | 932 |
| 12 | Ns5b | 2602 | 1655 | LPQAVMGSSY | W | 1437 |
| 13 | Ns5b | 2840 | 1655 | WARMILMTHF | W | 1997 |
| 14 | Ns5b | 2826 | 1655 | NSWLGNIIMY | W | 1534 |
| 15 | Ns3 | 1240 | 1248 | VPAAYAAQGY | W | 1943 |
| 16 | Core | 142 | 151 | APLGGAARAL | W | 836 |
| 17 | Core | 76 | 85 | WAQPGYPWPL | W | 1996 |
| 18 | Ns5b | 2795 | 1655 | DASGKRVYYL | W | 955 |
| 19 | Core | 74 | 83 | RAWAQPGYPW | W | 1634 |
| 20 | Ns3 | 1637 | 1645 | LTHPITKYIM | W | 1469 |
| 21 | Ns3 | 1175 | 1184 | HAVGIFRAAV | W | 1215 |
| 22 | Core | 81 | 90 | YPWPLYGNEG | W | 2064 |
| 23 | Ns5b | 2794 | 1655 | HDASGKRVYY | W | 1216 |
| 24 | Ns3 | 1622 | 1630 | TPLLYRLGAV | W | 1851 |
| 25 | Ns5b | 2836 | 1655 | APTLWARMIL | W | 853 |
| 26 | Ns4b | 1846 | 1655 | KVLVDILAGY | W | 1350 |
| 27 | Ns5b | 2757 | 1655 | RVFTEAMTRY | W | 1712 |
| 28 | Ns3 | 1258 | 1266 | VAATLGFGAY | W | 1887 |
| 29 | Ns5b | 2651 | 1655 | NDIRVEESIY | W | 1515 |
| 30 | Core | 83 | 92 | WPLYGNEGMG | W | 2020 |
| 31 | Ns5b | 2769 | 1655 | PPGDPPQPEY | W | 1566 |
| 32 | Core | 172 | 181 | CSFSIFLLAL | W | 935 |
| 33 | Core | 89 | 98 | EGMGWAGWLL | W | 1012 |
| 34 | Core | 137 | 146 | IPLVGAPLGG | W | 1290 |
| 35 | Ns3 | 1367 | 1375 | LSNTGEIPFY | W | 1459 |
| 36 | Ns5b | 2823 | 1655 | TPVNSWLGNI | W | 1862 |
| 37 | Ns5b | 2734 | 1655 | LVNGDDLVVI | W | 1480 |

Table continued

| Algonomics 10-mer | | | | | | |
|---|---|---|---|---|---|---|
| | Protein | CS_fr | CS_to | pep_seq | Score | SEQ ID NO |
| 38 | Ns3 | 1639 | 1647 | HPITKYIMAC | W | 1236 |
| 39 | Core | 18 | 27 | RPQDVKFPGG | W | 1681 |
| 40 | Ns5b | 2580 | 1655 | IVFPDLGVRV | W | 1312 |
| 41 | Ns5b | 2674 | 1655 | RSLTERLYIG | W | 1702 |
| 42 | Ns3 | 1219 | 1227 | VPQTFQVAHL | W | 1954 |
| 43 | Ns3 | 1216 | 1224 | PPAVPQTFQV | W | 1564 |
| 44 | Ns3 | 1242 | 1250 | AAYAAQGYKV | W | 783 |
| 45 | Ns5b | 2616 | 1655 | SPGQRVEFLV | W | 1765 |
| 46 | Ns5b | 2810 | 1655 | TPLARAAWET | W | 1850 |
| 47 | Ns3 | 1357 | 1365 | VPHPNIEEVA | W | 1951 |
| 48 | Ns3 | 1426 | 1434 | IPTSGDVVVV | W | 1295 |
| 49 | Core | 37 | 46 | LPRRGPRLGV | W | 450 |
| 50 | Ns5b | 2563 | 1655 | EVFCVQPEKG | W | 1038 |
| 51 | Ns3 | 1337 | 1345 | ETAGARLVVL | W | 1032 |
| 52 | Ns3 | 1245 | 1253 | AAQGYKVLVL | W | 777 |
| 53 | Ns5b | 2754 | 1655 | ASLRVFTEAM | W | 871 |
| 54 | Ns5b | 2593 | 1655 | MALYDVVSTL | W | 1492 |
| 55 | Ns5b | 2773 | 1655 | PPQPEYDLEL | W | 1575 |
| 56 | Ns4b | 1857 | 1655 | AGVAGALVAF | W | 808 |
| | | | | | | |

**Table 8 Predicted HLA-B*4403 and HLA-B*4002 binding peptides**

| | Protein | CS_fr | CS_to | pep_seq | Score | SEQ ID NO |
|---|---|---|---|---|---|---|
| Algonomics 9-mer | | | | | | |
| 1 | C | 77 | 85 | AQPGYPWPL | S | 563 |
| 2 | NS3 | 1555 | 1563 | LEFWESVFT | M | 564 |
| 3 | C | 88 | 96 | NEGLGWAGW | M | 565 |
| 4 | NS5B | 2828 | 2836 | WLGNIIMYA | M | 566 |
| 5 | NS5B | 2842 | 2850 | RMILMTHFF | M | 567 |
| 6 | NS5B | 2838 | 2846 | TLWARMILM | M | 568 |
| 7 | NS3 | 1401 | 1409 | DELAAKLSA | M | 569 |
| 8 | C | 28 | 36 | GQIVGGVYL | M | 570 |
| 9 | NS3 | 1558 | 1566 | WESVFTGLT | W | 571 |
| 10 | NS3 | 1633 | 1641 | NEVTLTHPI | W | 572 |
| 11 | NS3 | 1585 | 1593 | YLVAYQATV | W | 573 |
| 12 | NS3 | 1382 | 1390 | IEAIKGGRH | W | 574 |

Table continued

|  | Protein | CS_fr | CS_to | pep_seq | Score | SEQ ID NO |
|---|---|---|---|---|---|---|
| **Algonomics 9-mer** |  |  |  |  |  |  |
| 13 | NS3 | 1382b | 1390b | IEVIKGGRH | W | 575 |
| 14 | NS5B | 2621 | 2629 | VEFLVNAWK | W | 576 |
| 15 | NS5B | 2817 | 2825 | WETARHTPV | W | 577 |
| 16 | NS3 | 1606 | 1614 | QMWKCLIRL | W | 578 |
| 17 | C | 90 | 98 | GLGWAGWLL | W | 579 |
| 18 | NS5B | 2677 | 2685 | TERLYIGGP | W | 580 |
| 19 | NS5B | 2590 | 2598 | CEKMALYDV | W | 581 |
| 20 | NS3 | 1336 | 1344 | AETAGARLV | W | 582 |
| 21 | NS3 | 1362 | 1370 | IEEIGLSNN | W | 583 |
| 22 | NS5B | 2679 | 2687 | RLYIGGPLT | W | 584 |
| 23 | NS3 | 1409 | 1417 | ALGLNAVAY | W | 585 |
| 24 | NS5B | 2760 | 2768 | TEAMTRYSA | W | 586 |
| 25 | NS5B | 2776 | 2784 | PEYDLELIT | W | 587 |
| 26 | NS3 | 1440 | 1448 | LMTGYTGDF | W | 588 |
| 27 | NS3 | 1518 | 1526 | CECYDAGCA | W | 589 |
| 28 | NS3 | 1201 | 1209 | METTMRSPV | W | 590 |
| 29 | NS5B | 2833 | 2841 | IMYAPTLWA | W | 591 |
| 30 | NS3 | 1260 | 1268 | ATLGFGAYM | W | 592 |
|  |  |  |  |  |  |  |
| Epimmune |  |  |  |  |  |  |
|  | **Protein** | **CS_fr** | **CS_to** | **pep_seq** | **Score** PIC | **SEQ ID NO** |
|  |  |  |  | PEGRSWAQPG | 21 | 1548 |
|  |  |  |  | PEGRTWAQPG | 62 | 1549 |
|  |  |  |  | NEGLGWAGW | 2452 | 565 |
|  |  |  |  | NEGLGWAGWL | 58 | 1516 |
|  | E2 |  |  | SELSPLLLST | 8,1 | 1726 |
|  |  |  |  | TEWAILPCSY | 30 | 1822 |
|  |  |  |  | WEWVILLFL | 1,1 | 2007 |
|  |  |  |  | WEWVVLLFL | 2,1 | 2009 |
|  |  |  |  | WEYVVLLFL | 2,1 | 2011 |
|  |  |  |  | WEWVILLFLL | 1,2 | 2008 |
|  |  |  |  | WEWVVLLFLL | 0,67 | 2010 |
|  |  |  |  | WEYVVLLFLL | 0,75 | 2012 |
|  |  |  |  | AEAALENLV | 47 | 790 |
|  |  |  |  | AEAALEKLV | 83 | 788 |
|  |  |  |  | AEAALENLVI | 55 | 791 |

Table continued

| Epimmune | | | | | | |
|---|---|---|---|---|---|---|
| | **Protein** | **CS_fr** | **CS_to** | **pep_seq** | **Score** PIC | **SEQ ID NO** |
| | | | | AEAALEKLVI | 74 | 789 |
| | | | | LENLVILNA | 72 | 1373 |
| | NS2 | | | REMAASCGG | 53 | 1641 |
| | | | | TEPVIFSPM | 1,8 | 1819 |
| | | | | REILLGPADG | 72 | 1638 |
| | NS3 | | | GEIQVLSTV | 11 | 1120 |
| | NS3 | | | GEVQVLSTA | 35 | 1129 |
| | NS3 | | | GEVQWSTA | 29 | 1131 |
| | NS3 | | | GEIQVLSTVT | 52 | 1121 |
| | NS3 | | | GEVQVLSTAT | 69 | 1130 |
| | NS3 | | | METTMRSPV | 57 | 590 |
| | NS3 | | | LETTMRSPV | 51 | 1375 |
| | NS3 | | | AETAGVRLT | 177 | 797 |
| | NS3 | | | AETAGARLW | 36 | 796 |
| | NS3 | | | AETAGVRLTV | 70 | 798 |
| | NS3 | | | GEIPFYGKA | 11 | 1116 |
| | NS3 | | | GEIPFYGRA | 7,1 | 1118 |
| | NS3 | | | GEIPFYGKAI | 6,5 | 1117 |
| | NS3 | | | GEIPFYGRAI | 2,7 | 1119 |
| | NS3 | | | DELAAALRG | 284 | 956 |
| | NS3 | | | YELTPAETT | 94 | 2031 |
| | NS3 | | | AETTVRLRA | 120 | 800 |
| | NS3 | | | LEFWEGVFT | 72 | 1369 |
| | NS3 | | | LEFWEGVFTG | 91 | 1370 |
| | NS3 | | | WEAVFTGLT | 12 | 2000 |
| | NS3 | | | WESVFTGLT | 11 | 571 |
| | NS3 | | | WEGVFTGLT | 19 | 2001 |
| | NS3 | | | GENFAYLTA | 31 | 1122 |
| | NS3 | | | GENLPYLVA | 1,2 | 1126 |
| | NS3 | | | GENFPYLVA | 2,4 | 1124 |
| | NS3 | | | GENFAYLTAY | 35 | 1123 |
| | NS3 | | | GENLPYLVAY | 37 | 1127 |
| | NS3 | | | GENFPYLVAY | 12 | 1125 |
| | NS3 | | | NEVVLTHPI | 29 | 1520 |
| | NS3 | | | NEITLTHPV | 49 | 1518 |
| | NS3 | | | NEVTLTHPI | 76 | 572 |
| | | | | LEVVTSTWVL | 31 | 1378 |

Table continued

| Epimmune | | | | | | |
|---|---|---|---|---|---|---|
| | **Protein** | **CS_fr** | **CS_to** | **pep_seq** | **Score** PIC | **SEQ ID NO** |
| | | | | IELGGKPAL | 7,7 | 1257 |
| | | | | LEQFWAKHMW | 100 | 1374 |
| | | | | LEVFWAKHMW | 84 | 1376 |
| | | | | VEDVVNLLPA | 87 | 1898 |
| | | | | PEFFSWVDGV | 20 | 1547 |
| | | | | TEVLASMLT | 39 | 1821 |
| | | | | AEAAARRLA | 281 | 787 |
| | | | | SEASSSASQL | 10 | 1723 |
| | NS5A | | | VESENKVVV | 97 | 1903 |
| | NS5A | | | VESENKVVI | 67 | 1901 |
| | NS5A | | | VESETKVVIL | 94 | 1905 |
| | NS5A | | | VESENKVVVL | 95 | 1904 |
| | NS5A | | | VESENKVVIL | 58 | 1902 |
| | NS5A | | | REPSIPSEY | 50 | 1642 |
| | NS5A | | | REVSVAAEI | 55 | 1644 |
| | NS5A | | | REISVPAEI | 17 | 1639 |
| | NS5A | | | REVSVPAEI | 38 | 1646 |
| | NS5A | | | REPSIPSEYL | 38 | 1643 |
| | NS5A | | | REVSVAAEIL | 2,6 | 1645 |
| | NS5A | | | REISVPAEIL | 1,1 | 1640 |
| | NS5A | | | REVSVPAEIL | 1,8 | 1647 |
| | NS5A | | | SEYLLPKSRF | 6,2 | 1727 |
| | NS5A | | | AEILRKSRRF | 18 | 792 |
| | NS5A | | | AELATKTFG | 152 | 793 |
| | | | | EEQSWCCSM | 15 | 1006 |
| | | | | SEDWCCSM | 35 | 1724 |
| | | | | GEDWCCSM | 14 | 1113 |
| | | | | EEKLPISPL | 5,6 | 1005 |
| | | | | EEKLPINPL | 7,9 | 1004 |
| | | | | KEVRSLSRRA | 3,2 | 1320 |
| | | | | CEKMALYDI | 99 | 911 |
| | | | | EESIYQACSL | 63 | 1007 |
| | | | | EEARTAIHSL | 91 | 1003 |
| | NS5B | | | PEYDLELIT | 72 | 587 |
| | NS5B | | | WETVRHSPV | 7,0 | 2005 |
| | NS5B | | | WETVRHTPV | 12 | 2006 |
| | NS5B | | | WETARHTPI | 20 | 2003 |

Table continued

| Epimmune | | | | | | |
|---|---|---|---|---|---|---|
| | Protein | CS_fr | CS_to | pep_seq | Score PIC | SEQ ID NO |
| | NS5B | | | WETARHTPV | 29 | 577 |
| | NS5B | | | FEMYGATYSV | 25 | 1054 |
| | NS5B | | | FEMYGAVYSV | 13 | 1055 |
| | NS5B | | | IEPLDLPQI | 97 | 1258 |
| | NS5B | | | IERLHGLEA | 19 | 1261 |
| | NS5B | | | IERLHGLDA | 20 | 1259 |
| | NS5B | | | IERLHGLSA | 15 | 1263 |
| | NS5B | | | IERLHGLEAF | 33 | 1262 |
| | NS5B | | | IERLHGLDAF | 40 | 1260 |
| | NS5B | | | IERLHGLSAF | 20 | 1264 |
| | NS5B | | | HELTRVAAA | 41 | 1217 |
| | NS5B | | | HELTRVAAAL | 5,8 | 1218 |
| | NS5B | | | GEINRVASCL | 16 | 1115 |
| | | | | | | |
| Algonomics 10-mer | | | | | | |
| | Protein | CS_fr | CS_to | pep_seq | Score | SEQ ID NO |
| | Ns3 | 1382 | 1391 | IEVIKGGRHL | S | 1265 |
| | Ns3 | 1555 | 1564 | LEFWESVFTG | M | 1371 |
| | Ns5b | 2621 | 2630 | VEFLVNAWKS | M | 1900 |
| | Ns5b | 2606 | 2615 | VMGSSYGFQY | M | 1939 |
| | Ns3 | 1558 | 1567 | WESVFTGLTH | M | 2002 |
| | Core | 88 | 97 | NEGMGWAGWL | M | 1517 |
| | Ns5b | 2677 | 2686 | TERLYIGGPL | M | 1820 |
| | Ns3 | 1533 | 1542 | AETSVRLRAY | M | 799 |
| | Ns3 | 1518 | 1527 | CECYDAGCAW | M | 910 |
| | Ns3 | 1201 | 1210 | METTMRSPVF | M | 1493 |
| | Ns3 | 1371 | 1380 | GEIPFYGKAI | M | 1117 |
| | Ns3 | 1409 | 1418 | ALGLNAVAYY | W | 822 |
| | Ns4b | 1913 | 1922 | VQWMNRLIAF | W | 1963 |
| | Ns5b | 2750 | 2759 | QEDAASLRVF | W | 1601 |
| | Ns3 | 1633 | 1642 | NEVTLTHPIT | W | 1519 |
| | Core | 77 | 86 | AQPGYPWPLY | W | 861 |
| | Ns5b | 2656 | 2665 | EESIYQCCDL | W | 1008 |
| | Ns5b | 2679 | 2688 | RLYIGGPLTN | W | 1670 |
| | Ns5b | 2667 | 2676 | PEARQAIRSL | W | 1546 |
| | Ns5b | 2838 | 2847 | TLWARMILMT | W | 1837 |

Table continued

| Algonomics 10-mer | | | | | | |
|---|---|---|---|---|---|---|
| | Protein | CS_fr | CS_to | pep_seq | Score | SEQ ID NO |
| | Ns4b | 1876 | 1885 | TEDLVNLLPA | W | 1818 |
| | Ns3 | 1605 | 1614 | DQMWKCLIRL | W | 982 |
| | Ns3 | 1401 | 1410 | DELAAKLSAL | W | 957 |
| | Ns3 | 1223 | 1232 | FQVAHLHAPT | W | 1080 |
| | Ns5b | 2817 | 2826 | WETARHTPVN | W | 2004 |
| | Ns4b | 1909 | 1918 | GEGAVQWMNR | W | 1114 |
| | Ns5b | 2655 | 2664 | VEESIYQCCD | W | 1899 |
| | Ns3 | 1577 | 1586 | KQAGDNFPYL | W | 1346 |
| | Ns5b | 2776 | 2785 | PEYDLELITS | W | 1552 |
| | Core | 28 | 37 | GQIVGGVYLL | W | 1178 |
| | Ns4b | 1847 | 1856 | VLVDILAGYG | W | 1932 |

**Table 9 Predicted HLA-Cw0401 binding peptides**

| | Protein | CS_fr | CS_to | pep_seq | Score | SEQ ID NO |
|---|---|---|---|---|---|---|
| Algonomics 9-mer | | | | | | |
| 1 | Ns3 | 1603 | 1611 | SWDQMWKCL | S | 593 |
| 2 | Core | 173 | 181 | SFSIFLLAL | M | 594 |
| 3 | Ns3 | 1557 | 1565 | FWESVFTGL | M | 595 |
| 4 | Ns3 | 1292 | 1300 | TYSTYGKFL | M | 596 |
| 5 | Ns5b | 2777 | 2785 | EYDLELITS | M | 597 |
| 6 | Ns3 | 1243 | 1251 | AYAAQGYKV | M | 598 |
| 7 | Ns5b | 2581 | 2589 | VFPDLGVRV | M | 599 |
| 8 | Core | 129 | 137 | GFADLMGYI | M | 600 |
| 9 | Ns3 | 1554 | 1562 | HLEFWESVF | W | 601 |
| 10 | Ns3 | 1520 | 1528 | CYDAGCAWY | W | 602 |
| 11 | Core | 85 | 93 | LYGNEGLGW | W | 603 |
| 12 | Ns5b | 2842 | 2850 | RMILMTHFF | W | 604 |
| 13 | Ns3 | 1266 | 1274 | AYMSKAHGV | W | 605 |
| 14 | Ns3 | 1645 | 1653 | IMACMSADL | W | 606 |
| 15 | Ns3 | 1527 | 1535 | WYELTPAET | W | 607 |
| 16 | Core | 23 | 31 | KFPGGGQIV | W | 608 |
| 17 | Ns5b | 2636 | 2644 | GFSYDTRCF | W | 609 |
| 18 | Ns3 | 1417 | 1425 | YYRGLDVSV | W | 610 |
| 19 | Ns3 | 1469 | 1477 | TFTIETTTV | W | 611 |
| 20 | Ns5b | 2758 | 2766 | VFTEAMTRY | W | 612 |
| 21 | Ns3 | 1359 | 1367 | HPNIEEIGL | W | 613 |

Table continued

| | Protein | CS_fr | CS_to | pep_seq | Score | SEQ ID NO |
|---|---|---|---|---|---|---|
| **Algonomics 9-mer** | | | | | | |
| 22 | Core | 122 | 130 | VIDTLTCGF | W | 614 |
| 23 | Ns5b | 2638 | 2646 | SYDTRCFDS | W | 615 |
| 24 | Core | 29 | 37 | QIVGGVYLL | N | 616 |
| 25 | Core | 90 | 98 | GLGWAGWLL | N | 617 |
| 26 | Core | 125 | 133 | TLTCGFADL | N | 618 |
| 27 | Core | 135 | 143 | GYIPLVGAP | N | 619 |
| 28 | Core | 168 | 176 | NLPGCSFSI | N | 620 |
| | | | | | | |
| **Algonomics 10-mer** | | | | | | |
| | **Protein** | **CS_fr** | **CS_to** | **pep_seq** | **Score** | **SEQ ID NO** |
| | Ns3 | 1603 | 1611 | SWDQMWKCLI | S | 1804 |
| | Ns3 | 1556 | 1564 | EFWESVFTGL | M | 1010 |
| | Core | 173 | 182 | SFSIFLLALL | M | 1730 |
| | Core | 130 | 139 | FADLMGYIPL | M | 1048 |
| | Ns5b | 2834 | 1655 | MYAPTLWARM | M | 1511 |
| | Ns3 | 1463 | 1471 | DFSLDPTFTI | M | 958 |
| | Ns5b | 2614 | 1655 | QYSPGQRVEF | M | 1626 |
| | Core | 82 | 91 | PWPLYGNEGM | M | 1590 |
| | Ns3 | 1243 | 1251 | AYAAQGYKVL | M | 900 |
| | Ns5b | 2816 | 1655 | AWETARHTPV | M | 897 |
| | Ns5b | 2777 | 1655 | EYDLELITSC | W | 1046 |
| | Ns3 | 1584 | 1592 | PYLVAYQATV | W | 1594 |
| | Core | 135 | 144 | GYIPLVGAPL | W | 1211 |
| | Ns3 | 1192 | 1200 | AVDFIPVESM | W | 882 |
| | Ns4b | 1854 | 1655 | GYGAGVAGAL | W | 1210 |
| | Ns3 | 1292 | 1300 | TYSTYGKFLA | W | 1886 |
| | Core | 176 | 185 | IFLLALLSCL | W | 1266 |
| | Ns3 | 1375 | 1383 | FYGKAIPIEV | W | 1100 |
| | Ns5b | 2638 | 1655 | SYDTRCFDST | W | 1808 |
| | Core | 85 | 94 | LYGNEGMGWA | W | 1488 |
| | Ns3 | 1582 | 1590 | NFPYLVAYQA | W | 1522 |
| | Ns3 | 1541 | 1549 | AYLNTPGLPV | W | 903 |
| | Ns4b | 1914 | 1655 | QWMNRLIAFA | W | 1624 |

**Table 10 Predicted HLA-Cw0602 binding peptides**

| # | Protein Protein | CS_fr | CS_to | pep_seq | Score | SEQ ID NO |
|---|---|---|---|---|---|---|
| **Algonomics 9-mer** | | | | | | |
| 1 | Ns3 | 1292 | 1300 | TYSTYGKFL | S | 621 |
| 2 | Ns3 | 1244 | 1252 | YAAQGYKVL | S | 622 |
| 3 | Ns5b | 2696 | 2704 | YRRCRASGV | S | 623 |
| 4 | Ns5b | 2673 | 2681 | IRSLTERLY | S | 624 |
| 5 | Ns3 | 1494 | 1502 | GRRGIYRFV | S | 625 |
| 6 | Ns5b | 2573 | 2581 | GRKPARLIV | S | 626 |
| 7 | Ns5b | 2842 | 2850 | RMILMTHFF | S | 627 |
| 8 | Ns3 | 1417 | 1425 | YYRGLDVSV | S | 628 |
| 9 | Ns3 | 1606 | 1614 | QMWKCLIRL | S | 629 |
| 10 | Core | 173 | 181 | SFSIFLLAL | M | 630 |
| 11 | Ns3 | 1603 | 1611 | SWDQMWKCL | M | 631 |
| 12 | Ns5b | 2587 | 2595 | VRVCEKMAL | M | 632 |
| 13 | Ns3 | 1385 | 1393 | IKGGRHLIF | M | 633 |
| 14 | Ns5b | 2668 | 2676 | EARQAIRSL | M | 634 |
| 15 | Ns3 | 1413 | 1421 | NAVAYYRGL | M | 635 |
| 16 | Ns3 | 1383 | 1391 | EAIKGGRHL | M | 636 |
| 17 | Ns3 | 1415 | 1423 | VAYYRGLDV | M | 637 |
| 18 | Ns5b | 2678 | 2686 | ERLYIGGPL | M | 638 |
| 19 | Ns3 | 1383b | 1391b | EVIKGGRHL | M | 639 |
| 20 | Ns3 | 1266 | 1274 | AYMSKAHGV | M | 640 |
| 21 | Ns5b | 2841 | 2849 | ARMILMTHF | M | 641 |
| 22 | Ns3 | 1645 | 1653 | IMACMSADL | M | 642 |
| 23 | Ns5b | 2577 | 2585 | ARLIVFPDL | M | 643 |
| 24 | Ns3 | 1243 | 1251 | AYAAQGYKV | M | 644 |
| 25 | Ns3 | 1534 | 1542 | ETSVRLRAY | M | 645 |
| 26 | Ns5b | 2574 | 2582 | RKPARLIVF | M | 646 |
| 27 | Ns3 | 1245 | 1253 | AAQGYKVLV | M | 647 |
| 28 | Ns5b | 2613 | 2621 | FQYSPGQRV | M | 648 |
| 29 | Core | 29 | 37 | QIVGGVYLL | W | 649 |
| 30 | Core | 174 | 182 | FSIFLLALL | W | 650 |
| 31 | Ns3 | 1557 | 1565 | FWESVFTGL | W | 651 |
| 32 | Ns3 | 1553 | 1561 | DHLEFWESV | W | 652 |
| 33 | Core | 177 | 185 | FLLALLSCL | W | 653 |
| 34 | Core | 38 | 46 | PRRGPRLGV | W | 654 |
| 35 | Ns5b | 2631 | 2639 | KKCPMGFSY | W | 655 |
| 36 | Ns5b | 2607 | 2615 | MGSSYGFQY | W | 656 |
| 37 | Ns5b | 2835 | 2843 | YAPTLWARM | W | 657 |

Table continued

| # | Protein Protein | CS_fr | CS_to | pep_seq | Score | SEQ ID NO |
|---|---|---|---|---|---|---|
| **Algonomics 9-mer** | | | | | | |
| 38 | Core | 83 | 91 | WPLYGNEGL | W | 658 |
| 39 | Ns3 | 1522 | 1530 | DAGCAWYEL | W | 659 |
| 40 | Ns5b | 2796 | 2804 | ASGKRVYYL | W | 660 |
| 41 | Ns3 | 1338 | 1346 | TAGARLVVL | W | 661 |
| 42 | Ns5b | 2795 | 2803 | DASGKRVYY | W | 662 |
| 43 | Ns3 | 1376b | 1384b | YGKAIPIEV | W | 663 |
| 44 | Ns5b | 2833 | 2841 | IMYAPTLWA | W | 664 |
| 45 | Ns5b | 2827 | 2835 | SWLGNIIMY | W | 665 |
| 46 | Core | 77 | 85 | AQPGYPWPL | W | 666 |
| 47 | Ns5b | 2840 | 2848 | WARMILMTH | W | 667 |
| 48 | Ns5b | 2838 | 2846 | TLWARMILIVI | W | 668 |
| 49 | Ns3 | 1618 | 1626 | LHGPTPLLY | W | 669 |
| 50 | Ns3 | 1637 | 1645 | LTHPITKYI | W | 670 |
| 51 | Ns3 | 1638 | 1646 | THPITKYIM | W | 671 |
| 52 | Ns3 | 1440 | 1448 | LMTGYTGDF | W | 672 |
| 53 | Core | 111 | 119 | DPRRRSRNL | W | 673 |
| 54 | Core | 171 | 179 | GCSFSIFLL | W | 674 |
| 55 | Core | 150 | 158 | ALAHGVRVL | W | 675 |
| 56 | Ns3 | 1554 | 1562 | HLEFWESVF | W | 676 |
| 57 | Ns3 | 1404 | 1412 | AAKLSALGL | W | 677 |
| 58 | Ns3 | 1585 | 1593 | YLVAYQATV | W | 678 |
| 59 | Ns5b | 2636 | 2644 | GFSYDTRCF | W | 679 |
| 60 | Ns3 | 1583 | 1591 | FPYLVAYQA | W | 680 |
| 61 | Ns3 | 1540 | 1548 | RAYLNTPGL | W | 681 |
| 62 | Ns3 | 1418 | 1426 | YRGLDVSVI | W | 682 |
| 63 | Core | 23 | 31 | KFPGGGQIV | W | 683 |
| 64 | Ns3 | 1581 | 1589 | DNFPYLVAY | W | 684 |
| 65 | Core | 16 | 24 | NRRPQDVKF | W | 685 |
| 66 | Ns5b | 2720 | 2728 | SAACRAAKL | W | 686 |
| 67 | Ns3 | 1620 | 1628 | GPTPLLYRL | W | 687 |
| 68 | Core | 136 | 144 | YIPLVGAPL | W | 688 |
| 69 | Core | 28 | 36 | GQIVGGVYL | W | 689 |
| 70 | Ns3 | 1176 | 1184 | VVGVFRAAV | W | 690 |
| 71 | Ns5b | 2758 | 2766 | VFTEAMTRY | W | 691 |
| 72 | Core | 132 | 140 | DLMGYIPLV | W | 692 |
| 73 | Ns3 | 1181 | 1189 | RAAVCTRGV | W | 693 |
| 74 | Ns5b | 2616 | 2624 | SPGQRVEFL | W | 694 |

Table continued

| # | Protein Protein | CS_fr | CS_to | pep_seq | Score | SEQ ID NO |
|---|---|---|---|---|---|---|
| **Algonomics 9-mer** | | | | | | |
| 75 | Ns5b | 2605 | 2613 | AVMGSSYGF | W | 695 |
| 76 | Ns3 | 1402 | 1410 | ELAAKLSAL | W | 696 |
| 77 | Core | 149 | 157 | RALAHGVRV | W | 697 |
| 78 | Ns3 | 1246 | 1254 | AQGYKVLVL | W | 698 |
| 79 | Core | 114 | 122 | RRSRNLGKV | W | 699 |
| 80 | Ns5b | 2821 | 2829 | RHTPVNSWL | W | 700 |
| 81 | Ns5b | 2598 | 2606 | VVSTLPQAV | W | 701 |
| 82 | Ns5b | 2831 | 2839 | NIIMYAPTL | W | 702 |
| 83 | Ns5b | 2834 | 2842 | MYAPTLWAR | W | 703 |
| 84 | Ns5b | 2615 | 2623 | YSPGQRVEF | W | 704 |
| 85 | Ns5b | 2619 | 2627 | QRVEFLVNA | W | 705 |
| 86 | Ns5b | 2594 | 2602 | ALYDWSTL | W | 706 |
| 87 | Core | 73 | 81 | GRTWAQPGY | W | 707 |
| 88 | Ns5b | 2581 | 2589 | VFPDLGVRV | W | 708 |
| 89 | Ns5b | 2579 | 2587 | LIVFPDLGV | W | 709 |
| 90 | Ns5b | 2697 | 2705 | RRCRASGVL | W | 710 |
| 91 | Ns3 | 1571 | 1579 | HFLSQTKQA | W | 711 |
| 92 | Ns3 | 1458 | 1466 | VTQTVDFSL | W | 712 |
| 93 | Ns5b | 2837 | 2845 | PTLWARMIL | W | 713 |
| 94 | Ns5b | 2794 | 2802 | HDASGKRVY | W | 714 |
| 95 | Core | 89 | 97 | EGLGWAGWL | W | 715 |
| | | | | | | |
| **Algonomics 10-mer** | | | | | | |
| | Ns3 | 1180 | 1188 | FRAAVCTRGV | S | 1081 |
| | Ns5b | 2696 | 1655 | YRRCRASGVL | S | 2066 |
| | Ns3 | 1243 | 1251 | AYAAQGYKVL | S | 900 |
| | Ns5b | 2573 | 1655 | GRKPARLIVF | S | 1182 |
| | Ns5b | 2841 | 1655 | ARMILMTHFF | S | 864 |
| | Ns5b | 2820 | 1655 | ARHTPVNSWL | S | 863 |
| | Ns5b | 2628 | 1655 | WKSKKCPMGF | S | 2013 |
| | Ns3 | 1539 | 1547 | LRAYLNTPGL | M | 1454 |
| | Ns4b | 1942 | 1655 | ARVTQILSSL | M | 868 |
| | Core | 76 | 85 | WAQPGYPWPL | M | 1996 |
| | Ns3 | 1492 | 1500 | GRGRRGIYRF | M | 1181 |
| | Ns5b | 2834 | 1655 | MYAPTLWARM | M | 1511 |
| | Ns5b | 2673 | 1655 | IRSLTERLYI | M | 1301 |

Table continued

| Algonomics 10-mer | | | | | | |
|---|---|---|---|---|---|---|
| | Ns3 | 1626 | 1634 | YRLGAVQNEV | M | 2065 |
| | Ns5b | 2614 | 1655 | QYSPGQRVEF | M | 1626 |
| | Core | 149 | 158 | RALAHGVRVL | M | 1629 |
| | Ns5b | 2587 | 1655 | VRVCEKMALY | M | 1967 |
| | Core | 148 | 157 | ARALAHGVRV | M | 862 |
| | Core | 22 | 31 | VKFPGGGQIV | M | 1920 |
| | Ns3 | 1384 | 1392 | VIKGGRHLIF | M | 1917 |
| | Ns5b | 2840 | 1655 | WARMILMTHF | M | 1997 |
| | Ns3 | 1244 | 1252 | YAAQGYKVLV | M | 2024 |
| | Core | 28 | 37 | GQIVGGVYLL | M | 1178 |
| | Core | 35 | 44 | YLLPRRGPRL | M | 2047 |
| | Ns5b | 2630 | 1655 | SKKCPMGFSY | M | 1739 |
| | Ns3 | 1416 | 1424 | AYYRGLDVSV | M | 907 |
| | Ns3 | 1375 | 1383 | FYGKAIPIEV | M | 1100 |
| | Ns5b | 2593 | 1655 | MALYDWSTL | M | 1492 |
| | Core | 130 | 139 | FADLMGYIPL | M | 1048 |
| | Core | 176 | 185 | IFLLALLSCL | M | 1266 |
| | Ns3 | 1417 | 1425 | YYRGLDVSVI | M | 2083 |
| | Ns3 | 1242 | 1250 | AAYAAQGYKV | W | 783 |
| | Ns5b | 2836 | 1655 | APTLWARMIL | W | 853 |
| | Ns5b | 2792 | 1655 | VAHDASGKRV | W | 1892 |
| | Ns3 | 1556 | 1564 | EFWESVFTGL | W | 1010 |
| | Core | 172 | 181 | CSFSIFLLAL | W | 935 |
| | Ns3 | 1291 | 1299 | ITYSTYGKFL | W | 1310 |
| | Ns5b | 2795 | 1655 | DASGKRVYYL | W | 955 |
| | Core | 113 | 122 | RRRSRNLGKV | W | 1698 |
| | Core | 173 | 182 | SFSIFLLALL | W | 1730 |
| | Ns3 | 1249 | 1257 | YKVLVLNPSV | W | 2041 |
| | Core | 155 | 164 | VRVLEDGVNY | W | 1968 |
| | Core | 77 | 86 | AQPGYPWPLY | W | 861 |
| | Ns5b | 2833 | 1655 | IMYAPTLWAR | W | 1279 |
| | Ns4b | 1913 | 1655 | VQWMNRLIAF | W | 1963 |
| | Ns3 | 1403 | 1411 | LAAKLSALGL | W | 1356 |
| | Ns3 | 1382 | 1390 | IEVIKGGRHL | W | 1265 |
| | Ns3 | 1553 | 1561 | DHLEFWESVF | W | 960 |
| | Core | 135 | 144 | GYIPLVGAPL | W | 1211 |
| | Core | 169 | 178 | LPGCSFSIFL | W | 1426 |
| | Ns5b | 2835 | 1655 | YAPTLWARMI | W | 2028 |

Table continued

| Algonomics 10-mer | | | | | | |
|---|---|---|---|---|---|---|
| | Ns3 | 1292 | 1300 | TYSTYGKFLA | W | 1886 |
| | Ns4b | 1839 | 1655 | VGSIGLGKVL | W | 1911 |
| | Ns3 | 1335 | 1343 | QAETAGARLV | W | 1595 |
| | Ns5b | 2726 | 1655 | AKLQDCTMLV | W | 819 |
| | Ns3 | 1605 | 1613 | DQMWKCLIRL | W | 982 |
| | Ns4b | 1897 | 1655 | VCAAILRRHV | W | 1895 |
| | Ns3 | 1189 | 1197 | VAKAVDFIPV | W | 1893 |
| | Ns3 | 1541 | 1549 | AYLNTPGLPV | W | 903 |
| | Ns3 | 1489 | 1497 | GRTGRGRRGI | W | 1184 |
| | Ns3 | 1175 | 1184 | HAVGIFRAAV | W | 1215 |
| | Ns4b | 1939 | 1655 | DAAARVTQIL | W | 952 |
| | Ns5b | 2604 | 1655 | QAVMGSSYGF | W | 1599 |
| | Ns5b | 2615 | 1655 | YSPGQRVEFL | W | 2068 |
| | Ns5b | 2695 | 1655 | GYRRCRASGV | W | 1212 |
| | Ns5b | 2606 | 1655 | VMGSSYGFQY | W | 1939 |
| | Ns3 | 1602 | 1610 | PSWDQMWKCL | W | 1585 |
| | Ns3 | 1644 | 1652 | YIMACMSADL | W | 2037 |
| | Core | 142 | 151 | APLGGAARAL | W | 836 |
| | Ns5b | 2580 | 1655 | IVFPDLGVRV | W | 1312 |
| | Ns5b | 2635 | 1655 | MGFSYDTRCF | W | 1494 |
| | Core | 37 | 46 | LPRRGPRLGV | W | 450 |
| | Ns5b | 2793 | 1655 | AHDASGKRVY | W | 810 |
| | Ns5b | 2586 | 1655 | GVRVCEKMAL | W | 1203 |
| | Ns3 | 1265 | 1273 | GAYMSKAHGV | W | 1110 |
| | Ns3 | 1619 | 1627 | HGPTPLLYRL | W | 1219 |
| | Ns3 | 1200 | 1208 | SMETTMRSPV | W | 1750 |
| | Ns3 | 1609 | 1617 | KCLIRLKPTL | W | 1319 |
| | Ns4b | 1873 | 1655 | MPSTEDLVNL | W | 1505 |
| | Core | 114 | 123 | RRSRNLGKVI | W | 1699 |
| | Ns5b | 2570 | 1655 | EKGGRKPARL | W | 1015 |
| | Ns3 | 1337 | 1345 | ETAGARLVVL | W | 1032 |
| | Ns3 | 1161 | 1170 | LKGSSGGPLL | W | 1388 |
| | Ns3 | 1584 | 1592 | PYLVAYQATV | W | 1594 |
| | Ns3 | 1534 | 1542 | ETSVRLRAYL | W | 1033 |
| | Ns3 | 1412 | 1420 | LNAVAYYRGL | W | 1416 |
| | Ns3 | 1637 | 1645 | LTHPITKYIM | W | 1469 |
| | Ns3 | 1186 | 1194 | TRGVAKAVDF | W | 1866 |
| | Ns5b | 2589 | 1655 | VCEKMALYDV | W | 1897 |

Table continued

| | Algonomics 10-mer | | | | | |
|---|---|---|---|---|---|---|
| | Ns3 | 1578 | 1586 | QAGDNFPYLV | W | 1596 |
| | Ns3 | 1646 | 1654 | MACMSADLEV | W | 1490 |
| | Core | 89 | 98 | EGMGWAGWLL | W | 1012 |
| | Ns5b | 2602 | 1655 | LPQAVMGSSY | W | 1437 |
| | Ns3 | 1219 | 1227 | VPQTFQVAHL | W | 1954 |
| | Ns4b | 1859 | 1655 | VAGALVAFKV | W | 1891 |
| | Ns3 | 1245 | 1253 | AAQGYKVLVL | W | 777 |
| | Ns3 | 1622 | 1630 | TPLLYRLGAV | W | 1851 |
| | Ns4b | 1920 | 1655 | IAFASRGNHV | W | 1255 |
| | Ns5b | 2616 | 1655 | SPGQRVEFLV | W | 1765 |
| | Ns4b | 1864 | 1655 | VAFKVMSGEM | W | 1888 |
| | Ns3 | 1521 | 1529 | YDAGCAWYEL | W | 2030 |
| | Ns3 | 1240 | 1248 | VPAAYAAQGY | W | 1943 |
| | Ns5b | 2672 | 1655 | AIRSLTERLY | W | 817 |
| | Ns4b | 1840 | 1655 | GSIGLGKVLV | W | 1187 |
| | Ns3 | 1617 | 1625 | TLHGPTPLLY | W | 1833 |
| | Ns3 | 1507 | 1515 | RPSGMFDSSV | W | 1687 |
| | Ns3 | 1235 | 1243 | GKSTKVPAAY | W | 1146 |
| | Ns3 | 1373 | 1381 | IPFYGKAIPI | W | 1284 |
| | Ns3 | 1408 | 1416 | SALGLNAVAY | W | 1717 |
| | Ns3 | 1414 | 1422 | AVAYYRGLDV | W | 880 |
| | Core | 46 | 55 | VRATRKTSER | W | 1964 |

**Table 11 Predicted HLA-Cw0702 binding peptides**

| # | Protein | CS_fr | CS_to | pep_seq | Score | SEQ ID NO |
|---|---|---|---|---|---|---|
| **Algonomics 9-mer** | | | | | | |
| 1 | Ns3 | 1292 | 1300 | TYSTYGKFL | S | 716 |
| 2 | Ns3 | 1243 | 1251 | AYAAQGYKV | S | 717 |
| 3 | Ns3 | 1417 | 1425 | YYRGLDVSV | M | 718 |
| 4 | Ns5b | 2577 | 2585 | ARLIVFPDL | M | 719 |
| 5 | Core | 173 | 181 | SFSIFLLAL | M | 720 |
| 6 | Ns5b | 2673 | 2681 | IRSLTERLY | M | 721 |
| 7 | Core | 85 | 93 | LYGNEGLGW | M | 722 |
| 8 | Core | 129 | 137 | GFADLMGYI | M | 723 |
| 9 | Core | 73 | 81 | GRTWAQPGY | M | 724 |
| 10 | Ns5b | 2636 | 2644 | GFSYDTRCF | M | 725 |
| 11 | Ns5b | 2827 | 2835 | SWLGNIIMY | M | 726 |

Table continued

| # | Protein | CS_fr | CS_to | pep_seq | Score | SEQ ID NO |
|---|---------|-------|-------|---------|-------|-----------|
| 12 | Ns3 | 1643 | 1651 | KYIMACMSA | M | 727 |
| 13 | Ns5b | 2841 | 2849 | ARMILMTHF | M | 728 |
| 14 | Ns5b | 2587 | 2595 | VRVCEKMAL | M | 729 |
| 15 | Ns5b | 2835 | 2843 | YAPTLWARM | M | 730 |
| 16 | Core | 75 | 83 | TWAQPGYPW | M | 731 |
| 17 | Ns5b | 2802 | 2810 | YYLTRDPTT | W | 732 |
| 18 | Ns5b | 2834 | 2842 | MYAPTLWAR | W | 733 |
| 19 | Ns5b | 2821 | 2829 | RHTPVNSWL | W | 734 |
| 20 | Ns3 | 1244 | 1252 | YAAQGYKVL | W | 735 |
| 21 | Core | 83 | 91 | WPLYGNEGL | W | 736 |
| 22 | Ns3 | 1571 | 1579 | HFLSQTKQA | W | 737 |
| 23 | Ns3 | 1266 | 1274 | AYMSKAHGV | W | 738 |
| 24 | Ns3 | 1557 | 1565 | FWESVFTGL | W | 739 |
| 25 | Ns5b | 2574 | 2582 | RKPARLIVF | W | 740 |
| 26 | Ns5b | 2697 | 2705 | RRCRASGVL | W | 741 |
| 27 | Ns5b | 2842 | 2850 | RMILMTHFF | W | 742 |
| 28 | Ns5b | 2610 | 2618 | SYGFQYSPG | W | 743 |
| 29 | Ns3 | 1618 | 1626 | LHGPTPLLY | W | 744 |
| 30 | Ns5b | 2678 | 2686 | ERLYIGGPL | W | 745 |
| 31 | Ns3 | 1603 | 1611 | SWDQMWKCL | W | 746 |
| 32 | Ns3 | 1583 | 1591 | FPYLVAYQA | W | 747 |
| 33 | Core | 16 | 24 | NRRPQDVKF | W | 748 |
| 34 | Ns5b | 2581 | 2589 | VFPDLGVRV | W | 749 |
| 35 | Core | 17 | 25 | RRPQDVKFP | W | 750 |
| 36 | Core | 136 | 144 | YIPLVGAPL | W | 751 |
| 37 | Ns3 | 1248 | 1256 | GYKVLVLNP | W | 752 |
| 38 | Ns3 | 1638 | 1646 | THPITKYIM | W | 753 |
| 39 | Ns5b | 2765 | 2773 | RYSAPPGDP | W | 754 |
| 40 | Ns3 | 1494 | 1502 | GRRGIYRFV | W | 755 |
| 41 | Ns3 | 1520 | 1528 | CYDAGCAWY | W | 756 |
| 42 | Ns3 | 1492 | 1500 | GRGRRGIYR | W | 757 |
| 43 | Ns3 | 1499 | 1507 | YRFVTPGER | W | 758 |
| 44 | Ns5b | 2695 | 2703 | GYRRCRASG | W | 759 |
| 45 | Core | 68 | 76 | ARRPEGRTW | W | 760 |
| 46 | Ns5b | 2631 | 2639 | KKCPMGFSY | W | 761 |
| 47 | Core | 69 | 77 | RRPEGRTWA | W | 762 |
| 48 | Core | 34 | 42 | VYLLPRRGP | W | 763 |
| 49 | Ns5b | 2833 | 2841 | IMYAPTLWA | W | 764 |

Table continued

| # | Protein | CS_fr | CS_to | pep_seq | Score | SEQ ID NO |
|---|---------|-------|-------|---------|-------|-----------|
| 50 | Core | 114 | 122 | RRSRNLGKV | W | 765 |
| 51 | Ns3 | 1418 | 1426 | YRGLDVSVI | W | 766 |
| 52 | Ns3 | 1341 | 1349 | ARLWLATA | W | 767 |
| 53 | Ns5b | 2796 | 2804 | ASGKRVYYL | W | 768 |
| | | | | | | |
| **Algonomics 10-mer** | | | | | | |
| | Ns3 | 1243 | 1251 | AYAAQGYKVL | S | 900 |
| | Core | 135 | 144 | GYIPLVGAPL | S | 1211 |
| | Ns5b | 2834 | 1655 | MYAPTLWARM | S | 1511 |
| | Ns5b | 2587 | 1655 | VRVCEKMALY | M | 1967 |
| | Ns5b | 2696 | 1655 | YRRCRASGVL | M | 2066 |
| | Core | 173 | 182 | SFSIFLLALL | M | 1730 |
| | Ns3 | 1398 | 1406 | KKCDELAAKL | M | 1327 |
| | Ns3 | 1417 | 1425 | YYRGLDVSVI | M | 2083 |
| | Core | 85 | 94 | LYGNEGMGWA | M | 1488 |
| | Ns5b | 2841 | 1655 | ARMILMTHFF | M | 864 |
| | Ns3 | 1416 | 1424 | AYYRGLDVSV | M | 907 |
| | Ns3 | 1375 | 1383 | FYGKAIPIEV | M | 1100 |
| | Ns3 | 1539 | 1547 | LRAYLNTPGL | M | 1454 |
| | Ns5b | 2820 | 1655 | ARHTPVNSWL | M | 863 |
| | Ns4b | 1933 | 1655 | HYVPESDAAA | M | 1254 |
| | Ns3 | 1582 | 1590 | NFPYLVAYQA | M | 1522 |
| | Ns3 | 1541 | 1549 | AYLNTPGLPV | W | 903 |
| | Ns5b | 2573 | 1655 | GRKPARLIVF | W | 1182 |
| | Core | 114 | 123 | RRSRNLGKVI | W | 1699 |
| | Ns5b | 2673 | 1655 | IRSLTERLYI | W | 1301 |
| | Core | 176 | 185 | IFLLALLSCL | W | 1266 |
| | Ns3 | 1292 | 1300 | TYSTYGKFLA | W | 1886 |
| | Core | 129 | 138 | GFADLMGYIP | W | 1132 |
| | Core | 155 | 164 | VRVLEDGVNY | W | 1968 |
| | Ns5b | 2827 | 1655 | SWLGNIIMYA | W | 1806 |
| | Core | 73 | 82 | GRAWAQPGYP | W | 1180 |
| | Ns4b | 1854 | 1655 | GYGAGVAGAL | W | 1210 |
| | Core | 76 | 85 | WAQPGYPWPL | W | 1996 |
| | Ns3 | 1492 | 1500 | GRGRRGIYRF | W | 1181 |
| | Ns4b | 1942 | 1655 | ARVTQILSSL | W | 868 |
| | Ns5b | 2628 | 1655 | WKSKKCPMGF | W | 2013 |

Table continued

| | Core | 74 | 83 | RAWAQPGYPW | W | 1634 |
|---|---|---|---|---|---|---|
| | Ns5b | 2593 | 1655 | MALYDWSTL | W | 1492 |
| | Ns3 | 1584 | 1592 | PYLVAYQATV | W | 1594 |
| | Ns5b | 2802 | 1655 | YYLTRDPTTP | W | 2081 |
| | Ns3 | 1235 | 1243 | GKSTKVPAAY | W | 1146 |
| | Ns5b | 2801 | 1655 | VYYLTRDPTT | W | 1991 |
| | Core | 34 | 43 | VYLLPRRGPR | W | 1990 |
| | Ns3 | 1358 | 1366 | PHPNIEEVAL | W | 1555 |
| | Core | 149 | 158 | RALAHGVRVL | W | 1629 |
| | Ns4b | 1873 | 1655 | MPSTEDLVNL | W | 1505 |
| | Ns3 | 1498 | 1506 | IYRFVTPGER | W | 1314 |
| | Ns5b | 2840 | 1655 | WARMILMTHF | W | 1997 |
| | Ns3 | 1443 | 1451 | GYTGDFDSVI | W | 1213 |
| | Ns5b | 2614 | 1655 | QYSPGQRVEF | W | 1626 |
| | Ns5b | 2695 | 1655 | GYRRCRASGV | W | 1212 |
| | Ns5b | 2757 | 1655 | RVFTEAMTRY | W | 1712 |
| | Ns3 | 1626 | 1634 | YRLGAVQNEV | W | 2065 |
| | Core | 23 | 32 | KFPGGGQIVG | W | 1321 |
| | Core | 17 | 26 | RRPQDVKFPG | W | 1696 |
| | Ns5b | 2835 | 1655 | YAPTLWARMI | W | 2028 |
| | Ns3 | 1644 | 1652 | YIMACMSADL | W | 2037 |
| | Ns4b | 1914 | 1655 | QWMNRLIAFA | W | 1624 |
| | Core | 68 | 77 | ARRPEGRAWA | W | 866 |

**Table 12 Predicted HLA-DRB1*0101/0401/0701 and -DRB1*0301 binding peptides**

| Protein | Full Sequence | Score (PIC) | SEQ ID NO |
|---|---|---|---|
| NS4B | AAQLAPPSAASAFVG | 0,074 | 2102 |
| NS5B | ACKLTPPHSAKSKFG | 1,07 | 2103 |
| NS5A | ADLIEANLLWRQEMG | 5,63 | 2104 |
| C | ADLMGYIPLVGAPLG | 0,043 | 2105 |
| NS5B | APTLWARMILMTHFF | 6,67 | 2106 |
| NS3 | AQGYKVLVLNPSVAA | 0,5 | 2107 |
| NS5B | ARAAWETARHTPVNS | | 2108 |
| NS3 | ARLVVLATATPPGSV | 0,12 | 2109 |
| NS5B | ASCLRKLGVPPLRVW | 0,47 | 2110 |
| NS5A | ASQLSAPSLKATCTT | 0,41 | 2111 |
| NS3 | AVGIFRAAVCTRGVA | 5,96 | 2112 |
| NS4B | AVQWMNRLIAFASRG | 9,61 | 2113 |

Table continued

| Protein | Full Sequence | Score (PIC) | SEQ ID NO |
|---|---|---|---|
| E1 | AWDMMMNWSPTTALV | 2,56 | 2114 |
| NS4A | AYCLTTGSVVIVGRI | 0,74 | 2115 |
| NS5B | CQIYGACYSIEPLDL | 2,04 | 2116 |
| NS5A | DADLIEANLLWRQEM | 7,67 | 2117 |
| NS3 | DAHFLSQTKQAGDNF | | 2118 |
| NS3 | DIIICDECHSTDSTT | | 2119 |
| NS2 | DLAVAVEPVVFSDME | 2,42 | 2120 |
| NS2 | DLAVAVEPVVFSDME | | 2120 |
| NS4B | DLVNLLPAILSPGA | 0,17 | 2121 |
| NS3 | DPTFTIETTTVPQDA | | 2122 |
| NS3 | DSSVLCECYDAGCAW | | 2123 |
| | DVVVVATDALMTGFT | 3,12 | 2124 |
| NS3 | DWWATDALMTGYT | 3,12 | 2125 |
| NS4B | EDLVNLLPAILSPG | 0,72 | 2126 |
| NS5A | EPDVAVLTSMLTDPS | 0,027 | 2127 |
| NS4B | FNILGGWVAAQLAPP | 0,16 | 2128 |
| NS3 | FPYLVAYQATVCARA | 4,76 | 2129 |
| C | FSIFLLALLSCLTIP | 5,47 | 2130 |
| | FSIFLLALLSCLTVP | 5,47 | 2131 |
| E2 | FTTLPALSTGLIHLH | 7,05 | 2132 |
| NS5B | GACYSIEPLDLPQII | | 2133 |
| | GAGVAGALVAFKIMS | 0,29 | 2134 |
| NS4B | GAGVAGALVAFKVMS | 0,29 | 2135 |
| NS4B | GALVVGVVCAAILRR | 3,59 | 2136 |
| NS3 | GARLVVLATATPPGS | 0,11 | 2137 |
| | GCGWAGWLLSPRGSR | 6,86 | 2138 |
| C | GCSFSIFLLALLSCL | 4,62 | 2139 |
| NS3 | GDNFPYLVAYQATVC | 0,05 | 2140 |
| NS4A | GGVLAALAAYCLTTG | 0,44 | 2141 |
| E1 | GHRMAWDMMMNWSPT | 6,3 | 2142 |
| E1 | GHRMAWDMMMNWSPT | | 2142 |
| NS4B | GIQYLAGLSTLPGNP | 0,1 | 2143 |
| NS5B | GKYLFNWAVRTKLKL | 9,61 | 2144 |
| | GLGWAGWLLSPRGSR | 6,86 | 2145 |
| NS3 | GLPVCQDHLEFWESV | | 2146 |
| C | GMGWAGWLLSPRGSR | 6,86 | 2147 |
| NS4B | GMQLAEQFKQKALGL | | 2148 |
| C | GPRLGVRATRKTSER | 2,87 | 2149 |

Table continued

| Protein | Full Sequence | Score (PIC) | SEQ ID NO |
|---|---|---|---|
|  | GQGWRLLAPITAYSQ | 1,34 | 2150 |
| C | GQIVGGVYLLPRRGP | 1,3 | 2151 |
| NS5A | GSQLPCEPEPDVAVL |  | 2152 |
| NS5B | GSSYGFQYSPGQRVE | 0,52 | 2153 |
| NS3 | GTVLDQAETAGARLV | 0,32 | 2154 |
| C | GVNYATGNLPGCSFS | 4,02 | 2155 |
| C | GVRVLEDGVNYATGN |  | 2156 |
| NS3 | GYKVLVLNPSVAATL | 6,3 | 2157 |
| NS3 | HLIFCHSKKKCDELA |  | 2158 |
|  | HQWINEDCSTPCSGS |  | 2159 |
| NS5B | IERLHGLSAFSLHSY | 2,56 | 2160 |
|  | IQRLHGLSAFSLHSY | 2,56 | 2161 |
| NS4B | IQYLAGLSTLPGNPA | 0,66 | 2162 |
| NS5A | ITRVESENKWILDS |  | 2163 |
| NS3 | KPTLHGPTPLLYRLG | 0,56 | 2164 |
| NS3 | KVLVLNPSVAATLGF | 0,52 | 2165 |
| NS4B | LAGYGAGVAGALVAF | 1,63 | 2166 |
| E2 | LFLLLADARVCACLW |  | 2167 |
| NS4B | LFNILGGWVAAQLAP | 3,69 | 2168 |
| NS4B | LGKVLVDILAGYGAG |  | 2169 |
| NS5B | LHSYSPGEINRVASC | 2,87 | 2170 |
| NS3 | LIRLKPTLHGPTPLL |  | 2171 |
| NS4B | LPAILSPGALVVGVV | 0,11 | 2172 |
| E2 | LPALSTGLIHLHQNI | 0,68 | 2173 |
| NS4B | LSTLPGNPAIASLMA | 0,24 | 2174 |
| NS3 | LTHIDAHFLSQTKQA | 3,03 | 2175 |
|  | LTHIDAHFLSQTKQS | 3,03 | 2176 |
| NS5A | LTSMLTDPSHITAET | 2,29 | 2177 |
| NS5A | LTSMLTDPSHITAET |  | 2177 |
| NS3 | LVAYQATVCARAQAP | 4,76 | 2178 |
| NS4B | LVNLLPAILSPGALV | 5,63 | 2179 |
| NS3 | LVVLATATPPGSVTV | 0,24 | 2180 |
|  | MACMSADLEVVTSTW |  | 2181 |
| NS4B | MNRLIAFASRGNHVS | 2,79 | 2182 |
| NS5A | MPPLEGEPGDPDL |  | 2183 |
| C | MSTNPKPQRKTK |  | 2184 |
|  | MTGFTGDFDSVIDCN |  | 2185 |
| NS4B | NPAIASLMAFTASIT | 0,29 | 2186 |

Table continued

| Protein | Full Sequence | Score (PIC) | SEQ ID NO |
|---|---|---|---|
| NS5B | NSWLGNIIMYAPTLW | 1,2 | 2187 |
| NS5B | NVSVAHDASGKRVYY | | 2188 |
| E2 | PCSFTTLPALSTGLI | 0,04 | 2189 |
| NS4B | PGALVVGVVCAAILR | 0,55 | 2190 |
| NS5B | PMGFSYDTRCFDSTV | | 2191 |
| NS3 | PQTFQVAHLHAPTGS | 0,28 | 2192 |
| NS4B | PTHYVPESDAAARVT | | 2193 |
| NS5B | PTLWARMILMTHFFS | 0,85 | 2194 |
| NS3 | PYLVAYQATVCARAQ | 0,072 | 2195 |
| NS3 | QDAVSRSQRRGRTGR | | 2196 |
| NS5B | QKKVTFDRLQVLDDH | | 2197 |
| NS5B | QPEYDLELITSCSSN | | 2198 |
| NS3 | RAAVCTRGVAKAVDF | 3,8 | 2199 |
| NS3 | RGLLGCIITSLTGRD | 8,59 | 2200 |
| C | RLGVRATRKTSERSQ | | 2201 |
| NS5B | RLIVFPDLGVRVCEK | | 2202 |
| NS3 | RLVVLATATPPGSVT | 9,34 | 2203 |
| | RPEYDLELITSCSSN | | 2204 |
| NS5A | RQEMGGNITRVESEN | 5,03 | 2205 |
| E2 | RSELSPLLLSTTEWQ | 0,72 | 2206 |
| NS3 | RSPVFTDNSSPPAVP | | 2207 |
| E1 | SAMYVGDLCGSVFLV | | 2208 |
| NS3 | SDLYLVTRHADVIPV | | 2209 |
| C | SFSIFLLALLSCLTI | 4,02 | 2210 |
| | SFSIFLLALLSCLTV | 4,02 | 2211 |
| C | SIFLLALLSCLTIPA | 0,23 | 2212 |
| | SKGWRLLAPITAYAQ | 1,34 | 2213 |
| NS5B | SLRVFTEAMTRYSAP | 2,49 | 2214 |
| NS5B | SLRVFTEAMTRYSAP | | 2214 |
| E1 | SRCWVALTPTLAARN | 0,047 | 2215 |
| NS3 | STKVPAAYAAQGYKV | 0,15 | 2216 |
| NS3 | STTILGIGTVLDQAE | 0,37 | 2217 |
| NS4A | STWVLVGGVLAALAA | 0,28 | 2218 |
| NS5B | SYTWTGALITPCAAE | 5,63 | 2219 |
| NS3 | TFQVAHLHAPTGSGK | 8,35 | 2220 |
| | TMLVCGDDLVVICES | | 2221 |
| NS5B | TPCAAEESKLPINAL | | 2222 |
| | TPCAAEESKLPINPL | | 2223 |

Table continued

| Protein | Full Sequence | Score (PIC) | SEQ ID NO |
|---|---|---|---|
| NS3 | TPLLYRLGAVQNEVT | 0,32 | 2224 |
| NS3 | TRGLLGCIITSLTGR | 7,05 | 2225 |
| NS5B | TTIMAKNEVFCVQPE | 0,55 | 2226 |
| NS3 | TTTVPQDAVSRSQRR | | 2227 |
| NS3 | TVDFSLDPTFTIETT | | 2228 |
| NS4A | TWVLVGGVLAALAAY | 0,15 | 2229 |
| NS4B | VDILAGYGAGVAGAL | 3,69 | 2230 |
| NS3 | VESMETTMRSPVFTD | | 2231 |
| NS5B | VFCVQPEKGGRKPAR | | 2232 |
| NS4A | VGGVLAALAAYCLTT | 1,2 | 2233 |
| NS3 | VGIFRAAVCTRGVAK | 3,8 | 2234 |
| NS3 | VLVLNPSVAATLGFG | 9,61 | 2235 |
| NS4B | VNLLPAILSPGALVV | 0,4 | 2236 |
| NS5B | VNSWLGNIIMYAPTL | 1,42 | 2237 |
| NS4B | VQWMNRLIAFASRGN | 1,59 | 2238 |
| NS4B | VVGVVCAAILRRHVG | 5,03 | 2239 |
| | VVVVATDALMTGFTG | 4,37 | 2240 |
| | VVVVATDALMTGFTG | | 2240 |
| NS3 | VVVVATDALMTGYTG | 4,37 | 2241 |
| NS3 | VVVVATDALMTGYTG | | 2241 |
| P7 | VWPLLLLLLALPPRA | 0,29 | 2242 |
| NS5B | VYYLTRDPTTPLARA | | 2243 |
| NS3 | WDQMWKCLIRLKPTL | 3,69 | 2244 |
| NS3 | WESVFTGLTHIDAHF | 1,73 | 2245 |
| NS3 | WKCLIRLKPTLHGPT | 2,95 | 2246 |
| NS4A | WVLVGGVLAALAAYC | 0,021 | 2247 |
| NS3 | YDIIICDECHSTDST | | 2248 |
| NS3 | YGKFLADGGCSGGAY | | 2249 |
| P7 | YGVWPLLLLLLALPP | 1,2 | 2250 |
| C | YIPLVGAPLGGAARA | 0,072 | 2251 |
| NS3 | YKVLVLNPSVAATLG | 0,18 | 2252 |
| E1 | YYSMVGNWAKVLIVM | 2,56 | 2253 |

**Table 13**

**Selection of predicted CTL epitopes**

Immun = immunogenicity
High = Ki > 20.000 nM
Tg = transgenic mice

| HLA-A0101 | Genotype | Ki (nM) | Immun | SEQ ID NO |
|---|---|---|---|---|
| AATLGFGAY | 1b/1a | 694 | | 557 |
| AGDNFPYLV | 1b | high | | 24 |
| ALGLNAVAYY | 1b | | | 822 |
| ATDALMTGY | 1b | 4 | | 1 |
| ATDALMTGYT | 1b | | | 877 |
| AVMGSSYGF | 1b/1a | high | | 16 |
| CTCGSSDLY | 1b/1a | 14 | | 940 |
| CYDAGCAWY | 1b/1a | High | | 13 |
| DASGKRVYY | 1b | high | | 10 |
| DNFPYLVAY | 1b | 1111 | | 8 |
| DSSVLCECY | 1b/1a | 719 | | 17 |
| ECYDAGCAWY | 1b/1a | | | 1002 |
| EPEPDVAVL | 1b/1a | high | | 1024 |
| EVDGVRLHRY | | 167 | | 1037 |
| FADLMGYIP | 1b/1a/3a | high | | 4 |
| FTDNSSPPA | 1b/1a | 10 | | 7 |
| FTDNSSPPAV | 1b/1a | | | 1086 |
| FTEAMTRYS | 1b/1a/3a | 6611 | | 9 |
| FTEAMTRYSA | 1b/1a/3a | | | 1087 |
| GAPITYSTY | 1b | high | | 11 |
| GLDVSVIPT | 1b/1a/3a | high | | 29 |
| GLSAFSLHSY | | 61 | | 1154 |
| HIDAHFLSQ | 1b/1a/3a | high | | 1221 |
| HSAKSKFGY | 1b/1a | 615 | | 1241 |
| ITTGAPITY | 1b | 910 | | 6 |
| ITYSTYGKF | 1b/1a | high | | 26 |
| IVDVQYLYG | 1b/1a/3a | 6146 | | 1311 |
| KCDELAAKL | 1b/1a | | | 1318 |
| KSTKVPAAY | 1b/1a/3a | 950 | | 25 |
| LADGGCSGGAY | | 60 | | 1359 |
| LCECYDAGC | 1b/1a/3a | high | | 1366 |
| LDPTFTIET | 1b/1a | high | | 30 |
| LGLNAVAYY | 1b | high | | 18 |
| LHGPTPLLY | 1b/1a/3a | | | 219 |
| LSAFSLHSY | 1b/1a | 28 | | 1456 |
| LTCGFADLM | 1b/1a/3a | 3550 | | 2 |
| LTCGFADLMGY | 1b/1a/3a | 11 | | 1465 |
| LTDPSHITA | 1b/1a | 15 | | 1467 |

Table continued

| HLA-A0101 | Genotype | Ki (nM) | Immun | SEQ ID NO |
|---|---|---|---|---|
| LTDPSHITAE | 1b/1a | 237 | | 1468 |
| LTHIDAHFL | 1b/1a/3a | high | | 5 |
| LTHPITKYI | 1b | high | | 23 |
| LTHPITKYIM | 1b | | | 1469 |
| LVDILAGYGA | 1b/1a | 419 | | 1478 |
| MGSSYGFQY | 1b/1a | 1016 | | 22 |
| NSWLGNIIMY | 1b/3a | | | 1534 |
| PAAYAAQGY | 1b/1a | 1613 | | 12 |
| PAETSVRLR | 1b | high | | 27 |
| PGDPPQPEY | 1b/1a | high | | 19 |
| PTDPRRRSR | 1b/1a | high | | 55 |
| PTDPRRRSRN | 1b/1a | 17816 | | 1586 |
| PTLHGPTPLLY | | | | 1587 |
| PVESMETTM | 1b | high | | 15 |
| QAETAGARL | 1b/1a | high | | 60 |
| RSELSPLLL | 1b/1a | 106 | | 1700 |
| RSELSPLLLS | 1b/1a | 1853 | | 1701 |
| RVCEKMALY | 1b/1a | 2384 | | 3 |
| RVFTEAMTRY | 1b | | | 1712 |
| SLDPTFTIET | 1b/1a | high | | 1741 |
| STEDLVNLL | 1b/1a | 8223 | | 1787 |
| STEDLVNLLP | 1b/1a | high | | 1788 |
| TLHGPTPLLY | 1b/1a/3a | 343 | | 1833 |
| TRDPTTPLAR | 1b/1a | high | | 1865 |
| TSCGNTLTCY | 1b/1a | 246 | | 1867 |
| VAATLGFGAY | 1b/1a | | | 1887 |
| VATDALMTGY | 1b | | | 1894 |
| VIDTLTCGF | 1b/1a/3a | 1127 | | 28 |
| VIDTLTCGFA | 1b/1a/3a | | | 1914 |
| VPAAYAAQGY | 1b/1a | | | 1943 |
| VTLTHPITK | 1b | high | | 21 |
| VTLTHPITKY | 1b | 183 | | 1976 |
| YAPTLWARM | 1b | high | | 14 |
| | | | | |
| **HLA-A0201** | **Genotype** | **Ki (nM)** | **Immun** | **SEQ ID NO** |
| ALAHGVRVL | 1b/1a | 674 | | 72 |
| ALSTGLIHL | 1b/1a/3a | 329 | | 825 |

Table continued

| HLA-A0201 | Genotype | Ki (nM) | Immun | SEQ ID NO |
|-----------|----------|---------|-------|-----------|
| ALYDVVSTL | 1b | 19 | | 67 |
| AQPGYPWPL | 1b/1a/3a | 382 | | 65 |
| CLVDYPYRL | 1b/1a | 437 | | 922 |
| DLCGSVFLV | 1b/1a | 789 | | 963 |
| DLMGYIPLV | 1b/1a/3a | 83 | | 66 |
| FIPVESMET | | 934 | | 1059 |
| FLLALLSCL | 1b/1a | 136 | | 361 |
| FLLALLSCLT | 1b/1a | 132 | | 1070 |
| FLLLADARV | 1b/1a/3a | 20 | | 1072 |
| GLGWAGWLL | | 27 | | 1150 |
| GLLGCI ITSL | 1b/1a | 26 | | 1151 |
| GMFDSSVLC | 1b/1a | 71 | | 71 |
| GTQEDAASL | 1b | high | | 88 |
| HLHQNIVDV | 1b/1a/3a | 500 | | 1227 |
| HMWNFISGI | 1b/1a | 12 | | 1233 |
| ILAGYGAGV | 1b/1a/3a | 88 | | 1269 |
| ILSPGALW | 1b/1a/3a | 238 | | 1275 |
| IMACMSADL | 1b | 66 | | 90 |
| IMAKNEVFCV | 1b/1a/3a | 199 | | 1278 |
| IMYAPTLWA | 1b | 46 | | 84 |
| KLQDCTMLV | 1b | 4,6 | | 75 |
| KVLVLNPSV | 1b/1a | 50 | | 73 |
| LLFLLLADA | 1b/1a | 16 | | 1395 |
| LLFNILGGWV | 1b/1a | 4,1 | | 1396 |
| LLGCIITSL | 1b/1a | 56 | | 1397 |
| LLSCLTIPA | 1b | 12 | | 70 |
| LTHIDAHFL | 1b/1a/3a | 181 | | 5 |
| LVLNPSVAA | 1b/1a/3a | 2880 | | 82 |
| MALYDVVST | 1b | 1142 | | 85 |
| NIIMYAPTL | 1b | 70 | | 87 |
| NLPGCSFSI | 1b/1a/3a | 83 | | 93 |
| PLLLLLLAL | 1b/1a | 6554 | | 1557 |
| QIVGGVYLL | 1b/1a | 228 | | 91 |
| QMWKCLIRL | 1b/1a | 153 | | 238 |
| RLGAVQNEV | 1b/1a | 221 | | 265 |
| RLHGLSAFSL | 1b/1a | 179 | | 1659 |
| RLIVFPDLGV | 1b/1a | 89 | | 1661 |
| RLVVLATAT | 1b/1a | 16737 | | 86 |

Table continued

| HLA-A0201 | Genotype | Ki (nM) | Immun | SEQ ID NO |
|---|---|---|---|---|
| RLYIGGPLT | 1b | 845 | | 79 |
| SMVGNWAKV | 1b/1a | 158 | | 1753 |
| SVFTGLTHI | 1b/3a | 101 | | 76 |
| TILGIGTVL | 1b | 207 | | 89 |
| TLHGPTPLL | 1b/1a/3a | 68 | | 81 |
| TLWARMILM | 1b/1a | 11 | | 92 |
| VLVGGVLAA | 1b/1a | 219 | | 1933 |
| VLVGGVLAAL | 1b/1a | 26 | | 1934 |
| WATDALMT | 1b/1a | high | | 44 |
| WSTLPQAV | 1b | 884 | | 68 |
| WLGNIIMYA | 1b/3a | 33 | | 62 |
| WMNRLIAFA | 1b/1a/3a | 122 | | 2015 |
| YIPLVGAPL | 1b/1a | 337 | | 69 |
| YLFNWAVRT | 1b/1a/3a | 29 | | 2043 |
| YLLPRRGPRL | 1b/1a | 140 | | 2047 |
| YLNTPGLPV | 1b | 6,2 | | 74 |
| YLVAYQATV | 1b/1a | 29 | | 63 |
| YLVTRHADV | 1b/1a | 292 | | 2053 |
| YQATVCARA | 1b/1a/3a | 20 | | 83 |
| | | | | |
| AAYAAQGYK | 1b/1a | | | 56 |
| ALGLNAVAY | 1b | | | 51 |
| ALYDVVSTL | 1b | | | 67 |
| ASAACRAAK | 1b | | | 155 |
| AVCTRGVAK | 1b/1a/3a | 423 | | 156 |
| DLGVRVCEK | 1b/1a/3a | | | 154 |
| FLVNAWKSK | 1b | | | 150 |
| GIFRAAVCTR | 1b/1a/3a | | | 1141 |
| GLNAVAYYR | 1b/3a | | | 145 |
| GMFDSSVLC | 1b/1a | | | 71 |
| GNTLTCYLK | 1b | | | 40 |
| GVAGALVAFK | | 28 | | 1193 |
| GVLAALAAY | 1b/1a/3a | | | 1196 |
| GWCAAILR | 1b/1a | | | 1205 |
| GVVCAAILRR | 1b/1a | | | 1206 |
| HLHAPTGSGK | 1b/1a | 19 | | 1226 |
| HLIFCHSKK | 1b/1a/3a | 440 | | 148 |
| HLIFCHSKKK | 1b/1a/3a | 423 | | 1228 |

Table continued

| HLA-A0201 | Genotype | Ki (nM) | Immun | SEQ ID NO |
|---|---|---|---|---|
| HMWNFISGI | 1b/1a | | | 1233 |
| IVFPDLGVR | 1b/1a | | | 168 |
| KTKRNTNRR | 1b/1a | | | 164 |
| KTSERSQPR | 1b/1a/3a | 69 | | 167 |
| KVLVDILAGY | 1b/1a | | | 1350 |
| LFNWAVRTK | 1b/1a/3a | | | 1380 |
| LGFGAYMSK | 1b/1a | 136 | | 50 |
| LIFCHSKKK | 1b/1a/3a | | | 47 |
| LLYRLGAVQ | 1b/1a | | | 200 |
| LVNAWKSKK | 1b | | | 146 |
| QLFTFSPRR | 1b/1a | 15 | | 1609 |
| RLGVRATRK | 1b/1a/3a | 12 | | 144 |
| RLLAPITAY | 1b/1a/3a | | | 1662 |
| RMYVGGVEHR | 1b/1a | 15 | | 1672 |
| RQPIPKARR | 1b/1a/3a | | | 159 |
| RVCEKMALY | 1b/1a | | | 3 |
| RVFTEAMTR | 1b | | | 39 |
| RVLEDGVNY | 1b/1a | | | 45 |
| SQLSAPSLK | 1b/1a/3a | | | 1781 |
| STNPKPQRK | 1b/1a | | | 158 |
| TLGFGAYMSK | 1b/1a | 136 | | 1831 |
| VAGALVAFK | 1b/1a | 46 | | 1890 |
| VQPEKGGRK | 1b/1a | | | 157 |
| VTLTHPITK | 1b | | | 21 |
| WLLSPRGSR | 1b/1a/3a | | | 163 |
| WMNSTGFTK | 1b/1a/3a | 208 | | 2016 |
| YLFNWAVRTK | 1b/1a/3a | | | 2044 |
| YLKASAACR | 1b | | | 161 |
| YLLPRRGPR | 1b/1a | | | 149 |
| | | | | |
| **HLA-A2402** | **Genotype** | **Ki (nM)** | **Immun** | **SEQ ID NO** |
| AIKGGRHLI | | 336 | | 813 |
| ALYDVVSTL | 1b | 360 | | 67 |
| AQGYKVLVL | 1b/1a | 2164 | | 130 |
| AVMGSSYGF | 1b/1a | 10 | | 16 |
| AWKSKKCPM | | 6675 | | 898 |
| AYAAQGYKV | 1b/1a | 30 | | 277 |

Table continued

| HLA-A2402 | Genotype | Ki (nM) | Immun | SEQ ID NO |
|---|---|---|---|---|
| AYAAQGYKVL | 1b/1a | | | 900 |
| AYMSKAHGV | 1b | 30 | | 244 |
| CLIRLKPTL | 1b/1a | 113 | | 122 |
| CYSIEPLDL | 1b/1a | | | 951 |
| ELAAKLSAL | | 932 | | 1016 |
| EPEPDVAVL | 1b/1a | | | 1024 |
| ETTMRSPVF | | 219 | | 1034 |
| FSLDPTFTI | 1b/1a | 74 | | 106 |
| FWAKHMWNF | 1b/1a | 2 | | 1095 |
| FWAKHMWNFI | 1b/1a | 86 | | 1096 |
| FWESVFTGL | 1b/3a | 15 | | 234 |
| GFADLMGYI | 1b/1a/3a | 75 | | 236 |
| GFSYDTRCF | 1b/1a/3a | 45 | | 281 |
| GLGWAGWLL | | 68 | | 1150 |
| GLTHIDAHF | 1b/1a/3a | 10 | | 258 |
| GQIVGGVYL | 1b/1a | 17682 | | 127 |
| GYGAGVAGAL | 1b/1a | | | 1210 |
| GYIPLVGAPL | 1b/1a | | | 1211 |
| IFLLALLSCL | 1b/1a | | | 1266 |
| IIMYAPTLW | 1b | 14 | | 246 |
| KAHGVDPNI | 1b | 17123 | | 242 |
| KCDELAAKL | 1b/1a | | | 1318 |
| KFPGGGQIV | 1b/1a/3a | 168 | | 284 |
| KGSSGGPLL | | 625 | | 1325 |
| KLQDCTMLV | | 2776 | | 1333 |
| KYIMACMSA | 1b | 6475 | | 239 |
| LFNWAVRTKL | 1b/1a | | | 1381 |
| LLPRRGPRL | | 269 | | 1406 |
| LTHPITKYI | 1b | 403 | | 23 |
| LWARMILMTHF | | 230 | | 1483 |
| LYGNEGLGW | | 5 | | 1487 |
| MGSSYG FQY | | 9808 | | 1496 |
| MYTNVDQDL | 1b/1a/3a | | | 1512 |
| MYVGGVEHRL | 1b/1a | 444 | | 1513 |
| NFISGIQYL | 1b/1a | 706 | | 1521 |
| NIIMYAPTL | 1b | 249 | | 87 |
| NLGKVIDTL | 1b/1a/3a | 93 | | 283 |
| NLPGCSFSI | 1b/1a/3a | 8 | | 93 |

Table continued

| HLA-A2402 | Genotype | Ki (nM) | Immun | SEQ ID NO |
|---|---|---|---|---|
| PAVPQTFQV | 1b | 991 | | 282 |
| PFYGKAIPI | | 4 | | 1553 |
| PLLYRLGAV | | high | | 1558 |
| PVNSWLGNI | 1b/1a/3a | 319 | | 256 |
| QFKQKALGL | 1b/1a | | | 1602 |
| QFKQKALGLL | 1b/1a | | | 1603 |
| QMWKCLIRL | 1b/1a | 439 | | 238 |
| QWMNRLIAF | 1b/1a/3a | 177 | | 1623 |
| QYLAGLSTL | 1b/1a/3a | 250 | | 1625 |
| QYSPGQRVEF | 1b/1a | 667 | | 1626 |
| RALAHGVRV | | high | | 1628 |
| RLGAVQNEV | | 3062 | | 1656 |
| RMILMTHFF | 1b/1a | 44 | | 59 |
| RPDYNPPLL | 1b/1a/3a | | | 1677 |
| RSELSPLLL | 1b/1a | | | 1700 |
| RVEFLVNAW | | 261 | | 1710 |
| SFSIFLLAL | 1b/1a/3a | 116 | | 250 |
| SFSIFLLALL | 1b/1a | | | 1730 |
| SWDQMWKCL | 1b/1a | 670 | | 279 |
| TAGARLVVL | 1b/1a | 3194 | | 249 |
| TILGIGTVL | | 2070 | | 1826 |
| TLHGPTPLL | 1b/1a/3a | 217 | | 81 |
| TLWARMILM | 1b/1a | 2641 | | 92 |
| TWAQPGYPW | | 8 | | 1883 |
| TYSTYGKF | | 192 | | 1885 |
| TYSTYGKFL | 1b/1a/3a | 540 | | 241 |
| VIKGGRHLI | | 53 | | 1916 |
| VILDSFDPL | 1b/1a | | | 1918 |
| VMGSSYGF | | 33 | | 1938 |
| WLGNIIMYA | 1b/3a | high | | 62 |
| YAAQGYKVL | 1b/1a | 1895 | | 95 |
| YGKAIPIEV | | high | | 2034 |
| YIPLVGAPL | | 512 | | 2038 |
| YLNTPGLPV | | 380 | | 2048 |
| YLVAYQATV | | 1844 | | 2052 |
| YYRGLDVSV | 1b/1a/3a | 31 | | 271 |
| YYRGLDVSVI | 1b/1a/3a | 437 | | 2083 |
| | | | | |

Table continued

| HLA-B0702 | Genotype | Ki (nM) | Immun in Tg | SEQ ID NO |
|---|---|---|---|---|
| AAKLSALGL | 1b | 277 | x | 402 |
| AAQGYKVLVL | 1b/1a | 5524 | | 777 |
| AARALAHGV | 1b/1a | 209 | | 403 |
| ALAHGVRVL | 1b/1a | 7950 | | 72 |
| APLGGAARA | 1b/1a | 115 | | 384 |
| APLGGAARAL | 1b/1a | 1 | x | 836 |
| APPPSWDQM | 1b/1a | 281 | x | 381 |
| APTGSGKST | 1b/1a/3a | 370 | | 397 |
| APTLWARM | | 13 | | 852 |
| APTLWARMI | 1b/1a | 11 | x | 371 |
| APTLWARMIL | 1b/1a | 1 | x | 853 |
| APTLWARMILM | | 423 | | 854 |
| ATLGFGAYM | 1b/1a | 12973 | | 32 |
| AVMGSSYGF | 1b/1a | 4400 | | 16 |
| DPPQPEYDL | 1b/1a | HIGH | | 543 |
| DPRRRSRNL | 1b/1a/3a | 18 | x | 370 |
| DPTTPLARA | 1b/1a | 13058 | | 980 |
| EARQAIRSL | 1b | 291 | | 388 |
| EPDVAVLTSM | 1b/1a | 454 | | 1023 |
| EPEPDVAVL | 1b/1a | HIGH | | 1024 |
| EVIKGGRHL | 1b/1a | HIGH | | 376 |
| GPGEGAVQWM | 1b/1a/3a | 4747 | | 1163 |
| GPRLGVRAT | 1b/1a/3a | 128 | x | 387 |
| GPTPLLYRL | 1b/1a/3a | 209 | x | 307 |
| HPITKYIMA | 1b | 1106 | | 396 |
| HPNIEEVAL | 1b/1a | 230 | x | 1237 |
| IPFYGKAI | | 458 | | 1283 |
| IPLVGAPL | | 13 | x | 1289 |
| IPTSGDVVV | 1b/1a | 3152 | | 415 |
| KPARLIVF | | 367 | | 1336 |
| KPTLHGPTPL | 1b/1a/3a | 6 | x | 1343 |
| LPAILSPGAL | 1b/1a/3a | 255 | x | 1418 |
| LPALSTGLI | 1b/1a | 233 | x | 1419 |
| LPCEPEPDV | 1b/1a/3a | HIGH | | 1421 |
| LPCSFTTLPA | 1b/1a | 423 | | 1424 |
| LPGCSFSI | | 500 | | 1425 |
| LPGCSFSIF | 1b/1a/3a | 29 | x | 375 |
| LPGCSFSIFL | 1b/1a/3a | 558 | | 1426 |

Table continued

| HLA-B0702 | Genotype | Ki (nM) | Immun in Tg | SEQ ID NO |
|---|---|---|---|---|
| LPGNPAIASL | 1b/1a | 266 | | 1428 |
| LPINALSNSL | 1b/1a | 12 | x | 1430 |
| LPRRGPRL | | 1 | | 1442 |
| LPRRGPRLG | 1b/1a/3a | 124 | x | 380 |
| LPRRGPRLGV | 1b/1a/3a | 3 | x | 450 |
| LPVCQDHLEF | 1b/1a | 1564 | | 1444 |
| LPYIEQGM | | 423 | | 1445 |
| NAVAYYRGL | 1b/1a/3a | HIGH | | 400 |
| NPAIASLMA | 1b/1a | 676 | | 1527 |
| NPAIASLMAF | 1b/1a | 121 | | 1528 |
| NPSVAATLGF | 1b/1a/3a | 1197 | | 1532 |
| PPHSAKSKF | 1b/1a | HIGH | | 1568 |
| PPQPEYDLEL | 1b/1a | HIGH | | 1575 |
| PPVVHGCPL | 1b/1a | 433 | x | 1582 |
| QPRGRRQPI | 1b/1a/3a | 1 | x | 390 |
| RPDYNPPLL | 1b/1a/3a | 143 | x | 1677 |
| RPSGMFDSSV | 1b/1a | 14 | x | 1687 |
| SAACRAAKL | 1b | 106 | x | 121 |
| SPGALVVGV | 1b/1a/3a | 627 | | 1759 |
| SPGQRVEFL | 1b/1a | 38 | | 373 |
| SPRGSRPSW | 1b/1a/3a | 11 | x | 386 |
| SVFTGLTHI | 1b/3a | HIGH | | 76 |
| TPAETSVRL | 1b | 375 | | 383 |
| TPCTCGSSDL | 1b/1a | 168 | | 1843 |
| TPGERPSGM | 1b | 199 | x | 372 |
| TPLLYRLGA | 1b/1a | 74 | | 389 |
| TPLLYRLGAV | 1b/1a | 458 | | 1851 |
| VAYYRGLDV | 1b/1a/3a | 1417 | | 394 |
| WPLLLLLLAL | 1b/1a | 1474 | | 2018 |
| YAAQGYKVL | 1b/1a | 313 | x | 95 |
| | | | | |
| **HLA-B0801** | **Genotype** | **Ki (nM)** | **Immun** | **SEQ ID NO** |
| AIRSLTERL | 1b | 3621 | | 473 |
| AQGYKVLVL | 1b/1a | 1920 | | 130 |
| ARRGREILL | 1b/1a | 3039 | | 865 |
| CLRKLGVPPL | 1b/1a | 549 | | 921 |
| DLCGSVFL | 1b/1a | 11347 | | 962 |

Table continued

| HLA-B0801 | Genotype | Ki (nM) | Immun | SEQ ID NO |
|-----------|----------|---------|-------|-----------|
| DLMGYIPLV | 1b/1a/3a | 1966 | | 66 |
| DPRRRSRN | 1b/1a/3a | 12066 | | 974 |
| DPRRRSRNL | 1b/1a/3a | 111 | | 370 |
| DPRRRSRNLG | 1b/1a/3a | | | 975 |
| DQMWKCLIRL | 1b/1a | | | 982 |
| DTRCFDSTV | 1b/1a/3a | 13145 | | 466 |
| DVKFPGGGQI | 1b/1a/3a | 14129 | | 990 |
| EARQAIRSL | 1b | 9 | | 388 |
| ESENKVVIL | 1b/1a | HIGH | | 1030 |
| FPYLVAYQA | 1b/1a | 12 | | 443 |
| GCSFSIFL | 1b/1a/3a | 6708 | | 1111 |
| GKRVYYLTR | 1b/1a | HIGH | | 172 |
| GRRGIYRFV | 1b | 4984 | | 464 |
| HPITKYIMA | 1b | 59 | | 396 |
| HSKKKCDEL | 1b/1a | 76 | | 455 |
| HSKKKCDELA | 1b/1a | | | 1243 |
| IPKARRPE | 1b/1a | 1214 | | 1286 |
| IPKARRPEG | 1b/1a | 874 | | 409 |
| IPKARRPEGR | 1b/1a | | | 1287 |
| IPLVGAPL | 1b/1a | 20 | | 1288 |
| ITKYIMACM | 1b | 2610 | | 305 |
| ITYSTYGKFL | 1b/1a | | | 1310 |
| LIRLKPTLH | 1b/1a | 62 | | 205 |
| LLPRRGPRL | 1b/1a | 183 | | 132 |
| LPRRGPRL | 1b/1a/3a | 6 | | 1441 |
| LPRRGPRLGV | 1b/1a/3a | | | 450 |
| LTHPITKYI | 1b | HIGH | | 23 |
| LTRDPTTPL | 1b/1a | 4322 | | 1475 |
| NAVAYYRGL | 1b/1a/3a | 11365 | | 400 |
| NAWKSKKCPM | 1b | | | 1514 |
| NIRTGVRTI | 1b/1a | 619 | | 436 |
| NPKPQRKTK | 1b/1a | | | 404 |
| NPKPQRKTKR | 1b/1a | | | 1531 |
| PARLIVFPDL | 1b/1a | 5747 | | 1545 |
| QFKQKALGL | 1b/1a | 65 | | 1602 |
| QMWKCLIRL | 1b/1a | 8712 | | 238 |
| QPRGRRQP | 1b/1a/3a | HIGH | | 1615 |
| QPRGRRQPI | 1b/1a/3a | 3 | | 390 |

Table continued

| HLA-B0801 | Genotype | Ki (nM) | Immun | SEQ ID NO |
|---|---|---|---|---|
| QPRGRRQPIP | 1b/1a/3a | | | 1616 |
| QRKTKRNTNR | 1b/1a | | | 1618 |
| QRRGRTGRG | 1b/1a/3a | 314 | | 456 |
| QTRGLLGCI | 1b/1a | HIGH | | 1621 |
| QTRGLLGCII | 1b/1a | 17793 | | 1622 |
| RSRNLGKVI | 1b/1a/3a | 17415 | | 318 |
| RTKLKLTPI | 1b/1a | 2 | | 1705 |
| SARRGREIL | 1b/1a | 6454 | | 1718 |
| SARRGREILL | 1b/1a | 133 | | 1719 |
| SGKRVYYLTR | 1b | | | 1733 |
| SGKSTKVPAA | 1b/1a/3a | | | 1734 |
| SPGQRVEFL | 1b/1a | 120 | | 373 |
| SVRLRAYLN | 1b | 3314 | | 459 |
| TAGARLVVL | 1b/1a | 16 | | 249 |
| TGRGRRGIYR | 1b | | | 1825 |
| TIMAKNEVF | 1b/1a/3a | 6 | | 1827 |
| TLWARMILM | 1b/1a | 59 | | 92 |
| VAYYRGLDV | 1b/1a/3a | 278 | | 394 |
| VSARRGREI | 1b/1a | 192 | | 1969 |
| VTFDRLQVL | 1b/1a/3a | 4358 | | 1975 |
| WAKHMWNFI | 1b/1a | 104 | | 1993 |
| WARMILMTH | 1b/1a | 166 | | 287 |
| WARPDYNPPL | 1b/1a/3a | 1030 | | 1998 |
| YGKAIPIEVI | 1b | | | 2035 |
| YLKGSSGGPL | 1b/1a | 10 | | 2046 |
| YLLPRRGPRL | 1b/1a | 142 | | 2047 |
| YRRCRASGV | 1b/1a/3a | 11 | | 475 |
| YRRCRASGVL | 1b/1a/3a | | | 2066 |
| | | | | |
| **HLA-B3501** | **Genotype** | **Ki (nM)** | **Immun** | **SEQ ID NO** |
| APPPSWDQM | 1b/1a | 18 | | 381 |
| APTLWARMI | 1b/1a | HIGH | | 371 |
| AVMGSSYGF | 1b/1a | HIGH | | 16 |
| DPPQPEYDL | 1b/1a | HIGH | | 543 |
| EPEPDVAVL | 1b/1a | HIGH | | 1024 |
| FPGGGQIVG | 1b/1a/3a | 2596 | | 407 |
| FPGGGQIVGG | 1b/1a/3a | | | 1077 |

Table continued

| HLA-B3501 | Genotype | Ki (nM) | Immun | SEQ ID NO |
|---|---|---|---|---|
| FPYLVAYQA | 1b/1a | 18 | | 443 |
| FSLDPTFTI | 1b/1a | | | 106 |
| GPGEGAVQW | 1b/1a/3a | HIGH | | 1162 |
| GPTPLLYRL | 1b/1a/3a | 18 | | 307 |
| HPNIEEVAL | 1b/1a | 7 | | 1237 |
| IPFYGKAIPI | 1b/3a | | | 1284 |
| IPLVGAPL | | 295 | | 1289 |
| IPVESMETTM | | | | 1296 |
| LPALSTGLI | 1b/1a | HIGH | | 1419 |
| LPCEPEPDV | 1b/1a/3a | HIGH | | 1421 |
| LPGCSFSIF | 1b/1a/3a | 90 | | 375 |
| LPGCSFSIFL | 1b/1a/3a | | | 1426 |
| LPVCQDHLEF | 1b/1a | 200 | | 1444 |
| MGSSYGFQY | 1b/1a | HIGH | | 22 |
| MPSTEDLVNL | 1b | | | 1505 |
| NPSVAATLGF | 1b/1a/3a | | | 1532 |
| QAVMGSSYGF | 1b | | | 1599 |
| QPRGRRQPI | 1b/1a/3a | HIGH | | 390 |
| RPDYNPPLL | 1b/1a/3a | HIGH | | 1677 |
| RVLEDGVNY | 1b/1a | HIGH | | 45 |
| SALGLNAVAY | 1b | | | 1717 |
| SPGQRVEFL | 1b/1a | HIGH | | 373 |
| TPAETSVRL | 1b | 1643 | | 383 |
| YDAGCAWYEL | 1b/1a | | | 2030 |
| | | | | |
| **HLA-B4403 HLA-B4002** | **Genotype** | **Ki (nM)** | **Immun** | **SEQ ID NO** |
| AATLGFGAY | 1b/1a | 17055 | | 557 |
| ADLEVVTST | 1b/1a | HIGH | | 786 |
| AEAALENLV | 1b/1a/3a | 126 | | 790 |
| AEQFKQKAL | 1b/1a | 67 | | 794 |
| AEQFKQKALG | 1b/1a | | | 795 |
| AETAGARLV | 1b/1a | 176 | | 582 |
| AETAGARLVV | 1b/1a | 2704 | | 796 |
| AETSVRLRAY | 1b | | | 799 |
| AGYSGGDIY | 1b/1a | HIGH | | 809 |
| AQPGYPWPL | 1b/1a/3a | HIGH | | 65 |
| CECYDAGCA | 1b/1a | 13219 | | 589 |

Table continued

| HLA-B4403 HLA-B4002 | Genotype | Ki (nM) | Immun | SEQ ID NO |
|---|---|---|---|---|
| CECYDAGCAW | 1b/1a | 1056 | | 910 |
| CEKMALYDV | 1b/1a | 7759 | | 581 |
| CEPEPDVAV | 1b/1a | | | 912 |
| CEPEPDVAVL | 1b/1a | HIGH | | 913 |
| DELAAKLSA | 1b | 12653 | | 569 |
| DGGCSGGAY | 1b/1a/3a | HIGH | | 959 |
| DQRPYCWHY | 1b/1a | HIGH | | 984 |
| DSSVLCECY | 1b/1a | HIGH | | 17 |
| FDITKLLLA | 1b/1a | HIGH | | 1053 |
| GEIPFYGKA | 1b/1a/3a | HIGH | | 1116 |
| GEIPFYGKAI | 1b/1a/3a | 354 | | 1117 |
| GEPGDPDLS | 1b/1a/3a | HIGH | | 1128 |
| GGQIVGGVY | 1b/1a/3a | HIGH | | 1137 |
| GQIVGGVYL | 1b/1a | HIGH | | 127 |
| HSAKSKFGY | 1b/1a | HIGH | | 1241 |
| IEVIKGGRHL | 1b | | | 1265 |
| LEDGVNYAT | 1b/1a | HIGH | | 31 |
| LEDRDRSEL | 1b/1a | | | 1368 |
| LEFWESVFT | 1b | | | 129 |
| LEFWESVFTG | 1b | | | 1371 |
| LELITSCSS | 1b/1a/3a | HIGH | | 1372 |
| LEVVTSTWV | 1b/1a | HIGH | | 1377 |
| LEVVTSTWVL | 1b/1a | 5346 | | 1378 |
| LSAFSLHSY | 1b/1a | 3145 | | 1456 |
| METTMRSPVF | 1b | | | 1493 |
| NEGMGWAGWL | 1b | | | 1517 |
| PEKGGRKPA | 1b/1a | | | 1550 |
| PESDAAARVT | 1b/1a/3a | | | 1551 |
| PEYDLELIT | 1b/1a | | | 587 |
| RGVAKAVDF | 1b/1a | HIGH | | 317 |
| RMILMTHFF | 1b/1a | 389 | | 59 |
| SCGNTLTCY | 1b/1a | 11574 | | 1722 |
| SELSPLLLS | 1b/1a | 10368 | | 1725 |
| SELSPLLLST | 1b/1a | 2177 | | 1726 |
| TEAMTRYSA | 1b/1a/3a | 7566 | | 586 |
| TEDLVNLLPA | 1b/1a | | | 1818 |
| TERLYIGGPL | 1b | | | 1820 |
| TLWARMILM | 1b/1a | 10410 | | 92 |

Table continued

| HLA-B4403 HLA-B4002 | Genotype | Ki (nM) | Immun | SEQ ID NO |
|---|---|---|---|---|
| VDFSLDPTF | 1b/1a/3a | HIGH | | 350 |
| VEFLVNAWKS | 1b | | | 1900 |
| VESENKVVI | 1b/1a | 1702 | | 1901 |
| VESENKVVIL | 1b/1a | 10100 | | 1902 |
| VMGSSYGFQY | 1b/1a | | | 1939 |
| WESVFTGLTH | 1b/3a | | | 2002 |
| WETARHTPV | 1b/1a/3a | HIGH | | 577 |
| WLGNIIMYA | 1b/3a | 1949 | | 62 |
| | | | | |
| **HLA-Cw0301** | **Genotype** | **Ki (nM)** | **Immun** | **SEQ ID NO** |
| AALENLVVL | 1b | | | 776 |
| ADLMGYIPL | 1b/1a/3a | | | 2085 |
| AILGPLMVL | 1b | | | 816 |
| AKVLIVMLL | 1b | | | 2097 |
| ALYDVVSTL | 1b | | | 67 |
| ARLIVFPDL | 1b/1a | | | 643 |
| AVIPDREVL | 1b | | | 891 |
| CQVPAPEFF | 1b | | | 2094 |
| CWVALTPTL | 1b | | | 950 |
| ERLYIGGPL | 1b | | | 428 |
| ESYSSMPPL | 1b/1a | | | 2089 |
| FLLALLSCL | 1b/1a | | | 361 |
| FQVGLNQYL | 1b | | | 2100 |
| GALVAFKVM | 1b | | | 2086 |
| GAVFVGLAL | 1b | | | 1107 |
| GAVQNEVTL | 1b/1a | | | 347 |
| GAVQWMNRL | 1b/1a/3a | | | 1109 |
| GEIPFYGKA | 1b/1a/3a | | | 1116 |
| GEMPSTEDL | 1b | | | 2084 |
| GSIGLGKVL | 1b | | | 1186 |
| GSVFLVSQL | 1b | | | 1189 |
| HGILSFLVF | 1b | | | 2095 |
| ILMTHFFSI | 1b | | | 1273 |
| ITYSTYGKF | 1b/1a | | | 26 |
| LSVEEACKL | 1b | | | 2092 |
| NAVAYYRGL | 1b/1a/3a | | | 400 |
| NFISGIQYL | 1b/1a | | | 1521 |

Table continued

| HLA-Cw0301 | Genotype | Ki (nM) | Immun | SEQ ID NO |
|---|---|---|---|---|
| NIIMYAPTL | 1b | | | 87 |
| NQYLVGSQL | 1b | | | 2099 |
| QAGDNFPYL | 1b | | | 551 |
| QIIERLHGL | 1b | | | 2091 |
| QNIVDVQYL | 1b/1a/3a | | | 2098 |
| QYLAGLSTL | 1b/1a/3a | | | 1625 |
| RAYLNTPGL | 1b | | | 434 |
| SDVESYSSM | 1b | | | 2088 |
| SGMFDSSVL | 1b/1a | | | 135 |
| SPLTTQHTL | 1b | | | 1767 |
| SSLTITQLL | 1b | | | 1785 |
| STLPGNPAI | 1b/1a | | | 2096 |
| TALNCNDSL | 1b | | | 1812 |
| TALVVSQLL | 1b | | | 1813 |
| TMLVNGDDL | 1b | | | 2101 |
| TPIPAASQL | 1b | | | 1848 |
| TRVPYFVRA | 1b | | | 2087 |
| TTI RRHVDL | 1b | | | 1874 |
| VILDSFDPL | 1b/1a | | | 1918 |
| VLYREFDEM | 1b/1a | | | 2090 |
| VRVCEKMAL | 1b/1a | | | 632 |
| WAVRTKLKL | 1b/1a | | | 1999 |
| WHYPCTVNF | 1b/3a | | | 2093 |
| YAAQGYKVL | 1b/1a | | | 95 |
| YALYGVWPL | 1b | | | 2025 |
| YVLLLFLLL | 1b | | | 2073 |
| | | | | |
| **HLA-Cw0401** | **Genotype** | **Ki (nM)** | **Immun** | **SEQ ID NO** |
| AWETARHTPV | 1b/1a/3a | | | 897 |
| AYAAQGYKV | 1b/1a | high | | 277 |
| AYAAQGYKVL | 1b/1a | | | 900 |
| CYSIEPLDL | 1b/1a | | | 951 |
| DFSLDPTFTI | 1b/1a | | | 958 |
| DPPQPEYDL | 1b/1a | | | 543 |
| EFWESVFTGL | 1b | | | 1010 |
| EPEPDVAVL | 1b/1a | | | 1024 |
| EYDLELITS | 1b/1a | high | | 597 |

Table continued

| HLA-Cw0401 | Genotype | Ki (nM) | Immun | SEQ ID NO |
|---|---|---|---|---|
| FADLMGYIPL | 1b/1a/3a | | | 1048 |
| FWAKHMWNF | 1b/1a | | | 1095 |
| FWESVFTGL | 1b/3a | 2 | | 234 |
| GFADLMGYI | 1b/1a/3a | 598 | | 236 |
| GPTPLLYRL | 1b/1a/3a | | | 307 |
| HPNIEEVAL | 1b/1a | | | 1237 |
| MYAPTLWARM | 1b | | | 1511 |
| NFISGIQYL | 1b/1a | | | 1521 |
| PWPLYGNEGM | 1b | | | 1590 |
| QFKQKALGL | 1b/1a | | | 1602 |
| QYLAGLSTL | 1b/1a/3a | | | 1625 |
| QYSPGQRVEF | 1b/1a | | | 1626 |
| RPDYNPPLL | 1b/1a/3a | | | 1677 |
| SFSIFLLAL | 1b/1a/3a | 396 | | 250 |
| SFSIFLLALL | 1b/1a | | | 1730 |
| SPGQRVEFL | 1b/1a | | | 373 |
| SWDQMWKCL | 1b/1a | 66 | | 279 |
| SWDQMWKCLI | 1b/1a | | | 1804 |
| TYSTYGKFL | 1b/1a/3a | 18449 | | 241 |
| VFPDLGVRV | 1b/1a | 787 | | 349 |
| | | | | |
| HLA-Cw0602 | Genotype | Ki (nM) | Immun | SEQ ID NO |
| AAQGYKVLV | 1b/1a | | | 115 |
| AEQFKQKAL | 1b/1a | | | 794 |
| ALAHGVRVL | 1b/1a | | | 72 |
| AQGYKVLVL | 1b/1a | | | 130 |
| ARALAHGVRV | 1b/1a | | | 862 |
| ARHTPVNSWL | 1b/1a/3a | | | 863 |
| ARLIVFPDL | 1b/1a | | | 643 |
| ARMILMTHF | 1b/1a | | | 641 |
| ARMILMTHFF | 1b/1a | | | 864 |
| ARVTQILSSL | 1b | | | 868 |
| AYAAQGYKV | 1b/1a | | | 277 |
| AYAAQGYKVL | 1b/1a | | | 900 |
| AYMSKAHGV | 1b | | | 244 |
| AYYRGLDVSV | 1b/1a/3a | | | 907 |
| CLI RLKPTL | 1b/1a | | | 122 |

Table continued

| HLA-Cw0602 | Genotype | Ki (nM) | Immun | SEQ ID NO |
|---|---|---|---|---|
| DLVNLLPAI | 1b/1a | | | 968 |
| DRSELSPLL | 1b/1a | | | 986 |
| DVAVLTSML | 1b/1a | | | 989 |
| EARQAIRSL | 1b | | | 388 |
| EPEPDVAVL | 1b/1a | | | 1024 |
| ERLYIGGPL | 1b | | | 428 |
| ESENKVVIL | 1b/1a | | | 1030 |
| ETSVRLRAY | 1b | | | 46 |
| EVIKGGRHL | 1b/1a | | | 376 |
| FADLMGYIPL | 1b/1a/3a | | | 1048 |
| FKQKALGLL | 1b/1a | | | 1062 |
| FLLALLSCL | 1b/1a | | | 361 |
| FQYSPGQRV | 1b/1a | | | 111 |
| FRAAVCTRGV | 1b/1a/3a | | | 1081 |
| FSIFLLALL | 1b/1a | | | 362 |
| FYGKAIPIEV | 1b | | | 1100 |
| GGGQIVGGV | 1b/1a/3a | | | 1136 |
| GPTPLLYRL | 1b/1a/3a | | | 307 |
| GQIVGGVYLL | 1b/1a | | | 1178 |
| GRGRRGIYRF | 1b | | | 1181 |
| GRKPARLIV | 1b/1a/3a | | | 507 |
| GRKPARLIVF | 1b/1a | | | 1182 |
| GRRGIYRFV | 1b | | | 464 |
| HMWNFISGI | 1b/1a | | | 1233 |
| IFLLALLSCL | 1b/1a | | | 1266 |
| IKGGRHLIF | 1b/1a | | | 311 |
| IMACMSADL | 1b | | | 90 |
| IRSLTERLY | 1b | | | 624 |
| IRSLTERLYI | 1b | | | 1301 |
| KCDELAAKL | 1b/1a | | | 1318 |
| LGKVLVDIL | 1b/1a | | | 1382 |
| LLGCI ITSL | 1b/1a | | | 1397 |
| LRAYLNTPGL | 1b | | | 1454 |
| LVNLLPAIL | 1b/1a | | | 1481 |
| MALYDVVSTL | 1b | | | 1492 |
| MYAPTLWARM | 1b | | | 1511 |
| NAVAYYRGL | 1b/1a/3a | | | 400 |
| NFISGIQYL | 1b/1a | | | 1521 |

Table continued

| HLA-Cw0602 | Genotype | Ki (nM) | Immun | SEQ ID NO |
|---|---|---|---|---|
| QMWKCLIRL | 1b/1a | | | 238 |
| QTRGLLGCI | 1b/1a | | | 1621 |
| QYSPGQRVEF | 1b/1a | | | 1626 |
| RALAHGVRVL | 1b/1a | | | 1629 |
| RKPARLIVF | 1b/1a | | | 646 |
| RMILMTHFF | 1b/1a | | | 59 |
| SFSIFLLAL | 1b/1a/3a | | | 250 |
| SKKCPMGFSY | 1b | | | 1739 |
| SPGALVVGV | 1b/1a/3a | | | 1759 |
| STEDLVNLL | 1b/1a | | | 1787 |
| STWVLVGGV | 1b/1a | | | 1793 |
| SWDQMWKCL | 1b/1a | | | 279 |
| TLPALSTGL | 1b/1a | | | 1835 |
| TYSTYGKFL | 1b/1a/3a | | | 241 |
| VAYYRGLDV | 1b/1a/3a | | | 394 |
| VIKGGRHLIF | 1b/1a | | | 1917 |
| VKFPGGGQIV | 1b/1a/3a | | | 1920 |
| VLVDILAGY | 1b/1a | | | 1931 |
| VRVCEKMAL | 1b/1a | | | 632 |
| VRVCEKMALY | 1b/1a | | | 1967 |
| VTFDRLQVL | 1b/1a/3a | | | 1975 |
| WAQPGYPWPL | 1b/1a/3a | | | 1996 |
| WARMILMTHF | 1b/1a | | | 1997 |
| WKSKKCPMGF | 1b | | | 2013 |
| YAAQGYKVL | 1b/1a | | | 95 |
| YAAQGYKVLV | 1b/1a | | | 2024 |
| YLLPRRGPRL | 1b/1a | | | 2047 |
| YRLGAVQNEV | 1b/1a | | | 2065 |
| YRRCRASGV | 1b/1a/3a | | | 475 |
| YRRCRASGVL | 1b/1a/3a | | | 2066 |
| YYRGLDVSV | 1b/1a/3a | | | 271 |
| YYRGLDVSVI | 1b/1a/3a | | | 2083 |
| | | | | |
| **HLA-Cw0702** | **Genotype** | **Ki (nM)** | **Immun** | **SEQ ID NO** |
| AATLGFGAY | 1b/1a | | | 557 |
| ARHTPVNSWL | 1b/1a/3a | | | 863 |
| ARLIVFPDL | 1b/1a | 514 | | 643 |

Table continued

| HLA-Cw0702 | Genotype | Ki (nM) | Immun | SEQ ID NO |
|---|---|---|---|---|
| ARMILMTHF | 1b/1a | | | 641 |
| ARMILMTHFF | 1b/1a | | | 864 |
| AYAAQGYKV | 1b/1a | 15563 | | 277 |
| AYAAQGYKVL | 1b/1a | | | 900 |
| AYYRGLDVSV | 1b/1a/3a | | | 907 |
| CYDAGCAWY | 1b/1a | 1706 | | 13 |
| DGGCSGGAY | 1b/1a/3a | | | 959 |
| DPPQPEYDL | 1b/1a | | | 543 |
| DQRPYCWHY | 1b/1a | | | 984 |
| DREVLYREF | 1b/1a | | | 985 |
| DSSVLCECY | 1b/1a | high | | 17 |
| DYPYRLWHY | 1b/1a/3a | | | 997 |
| FRKHPEATY | 1b/1a/3a | | | 1082 |
| FYGKAIPIEV | 1b | | | 1100 |
| GFADLMGYI | 1b/1a/3a | high | | 236 |
| GFSYDTRCF | 1b/1a/3a | high | | 281 |
| GGQIVGGVY | 1b/1a/3a | | | 1137 |
| GPTPLLYRL | 1b/1a/3a | | | 307 |
| GVAGALVAF | 1b/1a | | | 1191 |
| GVLAALAAY | 1b/1a/3a | | | 1196 |
| GYIPLVGAPL | 1b/1a | | | 1211 |
| HQNIVDVQY | 1b/1a/3a | | | 1239 |
| HSAKSKFGY | 1b/1a | | | 1241 |
| HYVPESDAAA | 1b/1a/3a | | | 1254 |
| IRSLTERLY | 1b | 25 | | 624 |
| KKCDELAAKL | 1b/1a | | | 1327 |
| KSTKVPAAY | 1b/1a/3a | high | | 25 |
| KYIMACMSA | 1b | 7210 | | 239 |
| LHGPTPLLY | 1b/1a/3a | | | 219 |
| LLGCIITSL | 1b/1a | | | 1397 |
| LPGCSFSIF | 1b/1a/3a | | | 375 |
| LRAYLNTPGL | 1b | | | 1454 |
| LSAFSLHSY | 1b/1a | | | 1456 |
| LVGGVLAAL | 1b/1a | | | 1479 |
| LYGNEGMGWA | 1b | | | 1488 |
| MYAPTLWARM | 1b | | | 1511 |
| MYTNVDQDL | 1b/1a/3a | | | 1512 |
| NFPYLVAYQA | 1b/1a | | | 1522 |

Table continued

| HLA-Cw0702 | Genotype | Ki (nM) | Immun | SEQ ID NO |
|---|---|---|---|---|
| NIVDVQYLY | 1b/1a/3a | | | 1523 |
| NLGKVIDTL | 1b/1a/3a | high | | 283 |
| QPGYPWPLY | 1b/1a/3a | | | 216 |
| RLLAPITAY | 1b/1a/3a | | | 1662 |
| SCGNTLTCY | 1b/1a | | | 1722 |
| SFSIFLLAL | 1b/1a/3a | 3047 | | 250 |
| SFSIFLLALL | 1b/1a | | | 1730 |
| SPGALWGV | 1b/1a/3a | | | 1759 |
| SWLGNIIMY | 1b/3a | high | | 665 |
| TYSTYGKFL | 1b/1a/3a | 1017 | | 241 |
| VLVDILAGY | 1b/1a | | | 1931 |
| VRVCEKMAL | 1b/1a | 377 | | 632 |
| VRVCEKMALY | 1b/1a | | | 1967 |
| YAPTLWARM | 1b | high | | 14 |
| YRRCRASGVL | 1b/1a/3a | | | 2066 |
| YYRGLDVSV | 1b/1a/3a | 12 | | 271 |
| YYRGLDVSVI | 1b/1a/3a | | | 2083 |

## Table 14

## Selection of HLA-DRB1*0101 and –DRB1*0301 predicted peptides

Immun = Immunogenicity

Cons. = presence of the "core" in the indicated consensus sequence

| Sequence | Cons 3a | Cons 1b/1a | Cons 1b/1a/3 | 0101 IC$_{50}$ nM | 0301 IC$_{50}$ nM | Immun | SEQ ID NO |
|---|---|---|---|---|---|---|---|
| ADLMGYIPLVGAPLG | | X | X | 8333 | | | 2105 |
| GHRMAWDMMMNWSPT | | X | X | 183 | | | 2142 |
| SKGWRLLAPITAYAQ | | X | X | 0,40 | | | 2213 |
| RAAVCTRGVAKAVDF | | X | X | 619 | | | 2199 |
| GYKVLVLNPSVAATL | | X | X | 8,4 | | | 2157 |
| VLVLNPSVAATLGFG | | X | X | 3,0 | | | 2235 |
| WESVFTGLTHIDAHF | | X | X | 122 | | | 2245 |
| KPTLHGPTPLLYRLG | | X | X | 156 | | | 2164 |
| IQYLAGLSTLPGNPA | | X | X | 3,4 | | | 2162 |
| AVQWMNRLIAFASRG | | X | X | 2,3 | | | 2113 |

| | | | | | | | |
|---|---|---|---|---|---|---|---|
| MNRLIAFASRGNHVS | | X | X | 70 | | | 2182 |
| ASQLSAPSLKATCTT | | X | X | 329 | | | 2111 |
| TTIMAKNEVFCVQPE | | X | X | 3727 | | | 2226 |
| GIQYLAGLSTLPGNP | | X | X | | | | 2143 |
| LPAILSPGALVVGVV | | X | X | | | | 2172 |
| DLVNLLPAILSPGA | | X | X | | | | 2121 |
| YKVLVLNPSVAATLG | | X | X | | | | 2252 |
| LVVLATATPPGSVTV | | X | X | | | | 2180 |
| STTILGIGTVLDQAE | | X | X | | | | 2217 |
| VNLLPAILSPGALVV | | X | X | | | | 2236 |
| GGVLAALAAYCLTTG | | X | X | | | | 2141 |
| KVLVLNPSVAATLGF | | X | X | | | | 2165 |
| LPALSTGLIHLHQNI | | X | X | | | | 2173 |
| VGGVLAALAAYCLTT | | X | X | | | | 2233 |
| GQGWRLLAPITAYSQ | | X | X | | | | 2150 |
| VQWMNRLIAFASRGN | | X | X | | | | 2238 |
| GPRLGVRATRKTSER | | X | X | | | | 2149 |
| LTHIDAHFLSQTKQA | | X | X | | | | 2175 |
| LTHIDAHFLSQTKQS | | X | X | | | | 2176 |
| VGIFRAAVCTRGVAK | | X | X | | | | 2234 |
| LVAYQATVCARAQAP | | X | X | | | | 2178 |
| LVNLLPAILSPGALV | | X | X | | | | 2179 |
| AVGIFRAAVCTRGVA | | X | X | | | | 2112 |
| GLGWAGWLLSPRGSR | | X | X | | | | 2145 |
| GCGWAGWLLSPRGSR | | X | X | | | | 2138 |
| GMGWAGWLLSPRGSR | | X | X | | | | 2147 |
| RLVVLATATPPGSVT | | X | X | | | | 2203 |
| GKYLFNWAVRTKLKL | | X | X | | | | 2144 |
| GHRMAWDMMMNWSPT | | X | X | | 919 | | 2142 |
| DSSVLCECYDAGCAW | | X | X | | | | 2123 |
| PTHYVPESDAAARVT | | X | X | | 4954 | | 2193 |
| GSQLPCEPEPDVAVL | | X | X | | | | 2152 |
| QPEYDLELITSCSSN | | X | X | | | | 2198 |
| RLGVRATRKTSERSQ | | X | X | | | | 2201 |
| YGKFLADGGCSGGAY | | X | X | | | | 2249 |
| HLIFCHSKKKCDELA | | X | X | | | | 2158 |
| LIRLKPTLHGPTPLL | | X | X | | | | 2171 |
| MPPLEGEPGDPDL | | X | X | | | | 2183 |
| QKKVTFDRLQVLDDH | | X | X | | | | 2197 |
| PMGFSYDTRCFDSTV | | X | X | | | | 2191 |
| RPEYDLELITSCSSN | | X | X | | | | 2204 |
| ARAAWETARHTPVNS | | X | X | | | | 2108 |
| GVNYATGNLPGCSFS | | X | | 4564 | | | 2155 |
| GCSFSIFLLALLSCL | | X | | 1634 | | | 2139 |
| FTTLPALSTGLIHLH | | X | | 1,3 | | | 2132 |
| PQTFQVAHLHAPTGS | | X | | 22 | | | 2192 |
| AQGYKVLVLNPSVAA | | X | | 6,0 | | | 2107 |
| GTVLDQAETAGARLV | | X | | | | | 2154 |

| | | | | | | |
|---|---|---|---|---|---|---|
| GARLVVLATATPPGS | | X | | 173 | | | 2137 |
| DVVVVATDALMTGYT | | X | | 456 | | | 2125 |
| VVVVATDALMTGYTG | | X | | 1082 | | | 2241 |
| TWVLVGGVLAALAAY | | X | | 6,9 | | | 2229 |
| LAGYGAGVAGALVAF | | X | | 137 | | | 2166 |
| GALVVGVVCAAILRR | | X | | 314 | | | 2136 |
| LTSMLTDPSHITAET | | X | | 12.500 | | | 2177 |
| DADLIEANLLWRQEM | | X | | 574 | | | 2117 |
| RQEMGGNITRVESEN | | X | | | | | 2205 |
| GSSYGFQYSPGQRVE | | X | | 11 | | | 2153 |
| PTLWARMILMTHFFS | | X | | 788 | | | 2194 |
| ASCLRKLGVPPLRVW | | X | | 4,9 | | | 2110 |
| WVLVGGVLAALAAYC | | X | | | | | 2247 |
| EPDVAVLTSMLTDPS | | X | | | | | 2127 |
| PCSFTTLPALSTGLI | | X | | | | | 2189 |
| GDNFPYLVAYQATVC | | X | | | | | 2140 |
| YIPLVGAPLGGAARA | | X | | | | | 2251 |
| PYLVAYQATVCARAQ | | X | | | | | 2195 |
| ARLVVLATATPPGSV | | X | | | | | 2109 |
| STKVPAAYAAQGYKV | | X | | | | | 2216 |
| FNILGGWVAAQLAPP | | X | | | | | 2128 |
| SIFLLALLSCLTIPA | | X | | | | | 2212 |
| LSTLPGNPAIASLMA | | X | | | | | 2174 |
| STWVLVGGVLAALAA | | X | | | | | 2218 |
| NPAIASLMAFTASIT | | X | | | | | 2186 |
| VWPLLLLLLALPPRA | | X | | | | | 2242 |
| GAGVAGALVAFKVMS | | X | | | | | 2135 |
| GAGVAGALVAFKIMS | | X | | | | | 2134 |
| TPLLYRLGAVQNEVT | | X | | | | | 2224 |
| PGALVVGVVCAAILR | | X | | | | | 2190 |
| RSELSPLLLSTTEWQ | | X | | | | | 2206 |
| EDLVNLLPAILSPG | | X | | | | | 2126 |
| ACKLTPPHSAKSKFG | | X | | | | | 2103 |
| YGVWPLLLLLLALPP | | X | | | | | 2250 |
| GQIVGGVYLLPRRGP | | X | | | | | 2151 |
| CQIYGACYSIEPLDL | | X | | | | | 2116 |
| DLAVAVEPVVFSDME | | X | | | | | 2120 |
| YYSMVGNWAKVLIVM | | X | | | | | 2253 |
| IQRLHGLSAFSLHSY | | X | | | | | 2161 |
| IERLHGLSAFSLHSY | | X | | | | | 2160 |
| LHSYSPGEINRVASC | | X | | | | | 2170 |
| WKCLIRLKPTLHGPT | | X | | | | | 2246 |
| DVVVVATDALMTGFT | | X | | | | | 2124 |
| WDQMWKCLIRLKPTL | | X | | | | | 2244 |
| LFNILGGWVAAQLAP | | X | | | | | 2168 |
| VDILAGYGAGVAGAL | | X | | | | | 2230 |
| SFSIFLLALLSCLTI | | X | | | | | 2210 |
| SFSIFLLALLSCLTV | | X | | | | | 2211 |

| | | | | | | | |
|---|---|---|---|---|---|---|---|
| VVVVATDALMTGFTG | | X | | | | | 2240 |
| FPYLVAYQATVCARA | | X | | | | | 2129 |
| VVGVVCAAILRRHVG | | X | | | | | 2239 |
| FSIFLLALLSCLTIP | | X | | | | | 2130 |
| FSIFLLALLSCLTVP | | X | | | | | 2131 |
| ADLIEANLLWRQEMG | | X | | | | | 2104 |
| APTLWARMILMTHFF | | X | | | | | 2106 |
| TRGLLGCIITSLTGR | | X | | | | | 2225 |
| TFQVAHLHAPTGSGK | | X | | | | | 2220 |
| RGLLGCIITSLTGRD | | X | | | | | 2200 |
| GVRVLEDGVNYATGN | | X | | 392 | | | 2156 |
| SAMYVGDLCGSVFLV | | X | | | | | 2208 |
| SDLYLVTRHADVIPV | | X | | | | | 2209 |
| VVVVATDALMTGYTG | | X | | 93 | | | 2241 |
| TVDFSLDPTFTIETT | | X | | 46 | | | 2228 |
| GLPVCQDHLEFWESV | | X | | 14.286 | | | 2146 |
| GMQLAEQFKQKALGL | | X | | 4992 | | | 2148 |
| LTSMLTDPSHITAET | | X | | 567 | | | 2177 |
| MSTNPKPQRKTK | | X | | | | | 2184 |
| DLAVAVEPVVFSDME | | X | | | | | 2120 |
| RSPVFTDNSSPPAVP | | X | | | | | 2207 |
| YDIIICDECHSTDST | | X | | | | | 2248 |
| DIIICDECHSTDSTT | | X | | | | | 2119 |
| VVVVATDALMTGFTG | | X | | | | | 2240 |
| MACMSADLEVVTSTW | | X | | | | | 2181 |
| LGKVLVDILAGYGAG | | X | | | | | 2169 |
| ITRVESENKVVILDS | | X | | | | | 2163 |
| VFCVQPEKGGRKPAR | | X | | | | | 2232 |
| RLIVFPDLGVRVCEK | | X | | | | | 2202 |
| VYYLTRDPTTPLARA | | X | | | | | 2243 |
| GACYSIEPLDLPQII | | X | | | | | 2133 |
| SYTWTGALITPCAAE | X | | | 5,63 | | | 2219 |
| NSWLGNIIMYAPTLW | X | | | 1,2 | | | 2187 |
| VNSWLGNIIMYAPTL | X | | | 1,42 | | | 2237 |
| QDAVSRSQRRGRTGR | X | | | | | | 2196 |
| MTGFTGDFDSVIDCN | X | | | | | | 2185 |

## EXAMPLES

### Example 1: Identification of CTL specific HCV peptides peptides using the Algonomics algorithm

HLA Class I protein subclasses that should be targeted are defined: HLA-A01, 02, 03 and 24; HLA-B07, 08, 35 and 44; HLA-Cw4, -Cw6 and Cw7.

These HLA-Class I subclasses are modeled based on known homologue structures.

Based on X-ray data, an in depth analysis is performed of the main chain conformational changes in a given HLA-class I subclass for different peptides bound to said HLA-class I.

This analysis results in rules that will be applied when generating backbone variability.

On the average 8 to 10 different HLA-class I peptide complexes for each of the HLA-class I subclasses are built based on a series of epitopes and using Algonomics flexible peptide docking tools (wherein the peptide main and side chains are considered flexible, as well as the side chains of the HLA molecules). This yields in total 88 to 110 different three-dimensional models.

By using the above rules for main chain flexibility and/or by using molecular dynamics techniques or main chain perturbation/relaxation approaches, about five different versions differing in main chain conformation in the neighborhood of the bound peptide of the above models are derived. Hence, about 500 different three-dimensional models of HLA-class I peptide complexes are generated.

For each of the HLA-class I peptide models a prediction of the sequence variability of the peptide moieties in the context with surrounding HLA molecules is made: thread through the peptide backbones all HCV protein sequences of interest for all known HCV genotypes and asses for each "threaded" peptide the likelihood that it can form a stable complex with the underlying HLA-class I.

This is done using Algonomics' advanced inverse folding tools which have been developed within the Extended Dead-End Elimination framework. The end-point of this analysis is a list of binding peptides for each of the 11 HLA-Class I subclasses.

**Example 2: Identification of CTL specific B07-restricted peptides using 4 different algorithms**

For the HLA B07, a selection of the best scoring peptides is retrieved from the 3 on-line prediction servers (BIMAS, Syfpeithi and nHLAPred) using HCV consensus sequence 1b, and from the PIC-algorithm described by Epimmune using 57 HCV sequences. These peptides can either be 8-mers, 9-mers, 10-mers and in some cases 11-mers. Four hundred peptides were retrieved from BIMAS, 250 peptide from Syfpeithi, 100 from nHLAPred and 58 from the PIC algorithm from Epimmune. Said peptides are given in Table 15.

**Table 15. Predicted CTL specific B07-restricted peptides**

Prot: protein
GT = genotype

| BIMAS | | | | | |
|---|---|---|---|---|---|
| **Prot** | **Peptide sequence** | **Score** | **GT** | **rank** | **SEQ ID NO** |
| NS5B | RPRWFMLCL | 800 | 1b | 1 | 1684 |
| C | DPRRRSRNL | 800 | 1b/1a/3a | 2 | 370 |
| NS5B | RPRWFMLCLL | 800 | 1b | 1 | 1685 |
| NS5B | APTLWARMIL | 360 | 1b/1a | 2 | 853 |
| C | APLGGAARAL | 240 | 1b/1a | 3 | 836 |
| NS5B | GVRVCEKMAL | 200 | 1b/1a | 4 | 1203 |
| NS5B | RARSVRAKL | 180 | 1b | 3 | 1632 |
| NS2 | SARRGREIL | 180 | 1b/1a | 4 | 1718 |
| C | QPRGRRQPI | 120 | 1b/1a/3a | 5 | 390 |
| NS5B | RARPRWFML | 120 | 1b | 6 | 1631 |
| NS5B | DPPQPEYDL | 120 | 1b/1a | 7 | 543 |
| NS5A | LARGSPPSL | 120 | 1b | 8 | 1363 |
| NS5B | AIRSLTERL | 120 | 1b | 9 | 473 |
| NS5B | EARQAIRSL | 120 | 1b | 10 | 388 |
| NS2 | SARRGREILL | 120 | 1b/1a | 5 | 1719 |
| NS5A | WARPDYNPPL | 120 | 1b/1a/3a | 6 | 1998 |
| NS5B | RARSVRAKLL | 120 | 1b | 7 | 1633 |
| NS4A | AVIPDREVL | 90 | 1b | 11 | 891 |
| C | LPRRGPRLGV | 90 | 1b/1a/3a | 8 | 450 |

Table continued

| BIMAS | | | | | |
|---|---|---|---|---|---|
| Prot | Peptide sequence | Score | GT | rank | SEQ ID NO |
| NS3 | HPNIEEVAL | 80 | 1b/1a | 12 | 1237 |
| NS3 | TPAETSVRL | 80 | 1b | 13 | 383 |
| NS5B | SPGQRVEFL | 80 | 1b/1a | 14 | 373 |
| NS4B | SPLTTQHTL | 80 | 1b | 15 | 1767 |
| NS3 | GPTPLLYRL | 80 | 1b/1a/3a | 16 | 307 |
| E2 | GPWLTPRCL | 80 | 1b | 17 | 1173 |
| NS5B | TPIPAASQL | 80 | 1b | 18 | 1848 |
| NS5B | IPAASQLDL | 80 | 1b | 19 | 1280 |
| NS4B | MPSTEDLVNL | 80 | 1b | 9 | 1505 |
| NS2 | VPYFVRAQGL | 80 | 1b | 10 | 1960 |
| E2 | SPGPSQKIQL | 80 | 1b | 11 | 1764 |
| NS5A | SPAPNYSRAL | 80 | 1b | 12 | 1756 |
| P7 | WPLLLLLLAL | 80 | 1b/1a | 13 | 2018 |
| NS3 | KPTLHGPTPL | 80 | 1b/1a/3a | 14 | 1343 |
| NS4B | LPAILSPGAL | 80 | 1b/1a/3a | 15 | 1418 |
| NS4B | LPGNPAIASL | 80 | 1b/1a | 16 | 1428 |
| C | LPGCSFSIFL | 80 | 1b/1a/3a | 17 | 1426 |
| NS4B | SPLTTQHTLL | 80 | 1b | 18 | 1768 |
| NS5B | TPCAAEESKL | 80 | 1b | 19 | 1840 |
| NS3 | TPCTCGSSDL | 80 | 1b/1a | 20 | 1843 |
| NS5A | PPRRKRTWL | 80 | 1b | 21 | 1579 |
| NS3 | VPQTFQVAHL | 80 | 1b | 22 | 1954 |
| NS4B | LPYIEQGMQL | 80 | 1b | 23 | 1447 |
| NS5B | LPINALSNSL | 80 | 1b/1a | 24 | 1430 |
| NS5A | VPPVVHGCPL | 80 | 1b | 25 | 1953 |
| E2 | WTRGERCDL | 60 | 1b/1a | 20 | 2022 |
| NS2 | AVFVGLALL | 60 | 1b | 21 | 886 |
| NS3 | APPPSWDQM | 60 | 1b/1a | 22 | 381 |
| NS5B | LTRDPTTPL | 60 | 1b/1a | 23 | 1475 |
| E2 | APRPCGIVPA | 60 | 1b | 26 | 847 |
| E1 | TIRRHVDLL | 40 | 1b | 24 | 1828 |
| NS5B | HIRSVWKDL | 40 | 1b | 25 | 1223 |
| NS5A | KSRKFPPAL | 40 | 1b | 26 | 1348 |
| NS2 | GGRDAIILL | 40 | 1b | 27 | 1138 |
| NS5B | HIRSVWKDLL | 40 | 1b | 27 | 1224 |
| NS5B | CLRKLGVPPL | 40 | 1b/1a | 28 | 921 |
| P7 | AAWYIKGRL | 36 | 1b | 28 | 782 |

Table continued

| BIMAS | | | | | |
|---|---|---|---|---|---|
| Prot | Peptide sequence | Score | GT | rank | SEQ ID NO |
| E2/P7 | AALENLVVL | 36 | 1b | 29 | 776 |
| NS4B | AAARVTQIL | 36 | 1b | 30 | 773 |
| NS3 | AAKLSALGL | 36 | 1b | 31 | 402 |
| NS2 | AACGDIILGL | 36 | 1b | 29 | 774 |
| NS3 | AAQGYKVLVL | 36 | 1b/1a | 30 | 777 |
| NS5B | AAKLQDCTML | 36 | 1b | 31 | 775 |
| NS4A | VVIVGRIIL | 30 | 1b | 32 | 1982 |
| NS2 | NVRGGRDAI | 30 | 1b | 33 | 1538 |
| NS3 | GPKGPITQM | 30 | 1b | 34 | 1165 |
| E2 | DARVCACLWM | 30 | 1b | 32 | 954 |
| NS4A | SVVIVGRIIL | 30 | 1b | 33 | 1803 |
| NS5A | EPEPDVAVL | 24 | 1b/1a | 35 | 1024 |
| NS5A | RPDYNPPLL | 24 | 1b/1a/3a | 36 | 1677 |
| NS5B | APTLWARMI | 24 | 1b/1a | 37 | 371 |
| NS5B | LSRARPRWFM | 22,5 | 1b | 34 | 1462 |
| P7 | LVPGAAYAL | 20 | 1b | 38 | 1482 |
| NS3 | VVVVATDAL | 20 | 1b/1a | 39 | 1988 |
| E2 | YVLLLFLLL | 20 | 1b | 40 | 2073 |
| NS3/N S4A | EWTSTWVL | 20 | 1b/1a | 41 | 1042 |
| NS4A | LVGGVLAAL | 20 | 1b/1a | 42 | 1479 |
| P7 | GVWPLLLLL | 20 | 1b | 43 | 1207 |
| C | GVNYATGNL | 20 | 1b/1a | 44 | 339 |
| NS4B | RVTQILSSL | 20 | 1b | 45 | 1714 |
| E2 | YVGGVEHRL | 20 | 1b/1a | 46 | 2072 |
| NS3 | EVIKGGRHL | 20 | 1b/1a | 47 | 376 |
| NS5A | TVSSALAEL | 20 | 1b | 48 | 1881 |
| NS5B | SVGVGIYLL | 20 | 1b | 49 | 1799 |
| NS5A | EVSVAAEIL | 20 | 1b | 50 | 1040 |
| NS2 | YVYDHLTPL | 20 | 1b | 51 | 2077 |
| NS5A | DVAVLTSML | 20 | 1b/1a | 52 | 989 |
| P7 | SVAGAHGIL | 20 | 1b | 53 | 1796 |
| NS2 | FVGLALLTL | 20 | 1b | 54 | 1090 |
| C | GPRLGVRAT | 20 | 1b/1a/3a | 55 | 387 |
| E1 | MVGNWAKVL | 20 | 1b/1a | 56 | 1510 |
| NS4B | LVNLLPAIL | 20 | 1b/1a | 57 | 1481 |
| NS2 | YVQMAFMKL | 20 | 1b | 58 | 2074 |
| NS3 | TPGERPSGM | 20 | 1b | 59 | 372 |

Table continued

| BIMAS | | | | | |
|---|---|---|---|---|---|
| Prot | Peptide sequence | Score | GT | rank | SEQ ID NO |
| C | WPLYGNEGM | 20 | 1b | 60 | 2019 |
| NS5B | RVASCLRKL | 20 | 1b | 61 | 1707 |
| E2 | RVCACLWMML | 20 | 1b | 35 | 1709 |
| NS4B | GPGEGAVQWM | 20 | 1b/1a/3a | 36 | 1163 |
| NS5B | KVTFDRLQVL | 20 | 1b/1a/3a | 37 | 1352 |
| NS5A | DVWDWICTVL | 20 | 1b | 38 | 995 |
| NS3 | DVVVVATDAL | 20 | 1b/1a | 39 | 994 |
| NS5A | VVILDSFDPL | 20 | 1b/1a | 40 | 1981 |
| E1 | YPGHVSGHRM | 20 | 1b | 41 | 2063 |
| E1 | MVAGAHWGVL | 20 | 1b | 42 | 1509 |
| P7 | GVWPLLLLLL | 20 | 1b | 43 | 1208 |
| NS4B | VVGVVCAAIL | 20 | 1b/1a | 44 | 1980 |
| NS3 | VPVESMETTM | 20 | 1b | 45 | 1958 |
| NS5A | TVLTDFKTWL | 20 | 1b | 46 | 1880 |
| NS2 | NVRGGRDAII | 20 | 1b | 47 | 1539 |
| NS3 | CVTQTVDFSL | 20 | 1b/1a | 48 | 949 |
| NS3 | VVSTATQSFL | 20 | 1b | 49 | 1985 |
| NS2 | AILGPLMVL | 18 | 1b | 62 | 816 |
| C | AARALAHGV | 18 | 1b/1a | 63 | 403 |
| NS2 | LAILGPLMVL | 18 | 1b | 50 | 1361 |
| NS5B | AVRTKLKLT | 15 | 1b/1a | 64 | 895 |
| NS2 | ARRGREILL | 12 | 1b/1a | 65 | 865 |
| NS3 | NAVAYYRGL | 12 | 1b/1a/3a | 66 | 400 |
| NS4B | GAVQWMNRL | 12 | 1b/1a/3a | 67 | 1109 |
| NS3 | QAGDNFPYL | 12 | 1b | 68 | 551 |
| NS5B | ATTSRSASL | 12 | 1b | 69 | 879 |
| NS5B | ALYDWSTL | 12 | 1b | 70 | 67 |
| NS5B | WAVRTKLKL | 12 | 1b/1a | 71 | 1999 |
| NS2 | TAACGDIIL | 12 | 1b | 72 | 1811 |
| NS4A | LAALAAYCL | 12 | 1b/1a/3a | 73 | 1357 |
| E2 | ALSTGLIHL | 12 | 1b/1a/3a | 74 | 825 |
| NS3 | DAGCAWYEL | 12 | 1b/1a | 75 | 528 |
| E2 | CACLWMMLL | 12 | 1b | 76 | 909 |
| NS5A | LASSSASQL | 12 | 1b | 77 | 1365 |
| NS2 | GAVFVGLAL | 12 | 1b | 78 | 1107 |
| NS3 | SGMFDSSVL | 12 | 1b/1a | 79 | 135 |
| NS5B | ASGKRVYYL | 12 | 1b | 80 | 334 |

Table continued

| BIMAS | | | | | |
|-------|------------------|-------|-----------|------|-----------|
| Prot | Peptide sequence | Score | GT | rank | SEQ ID NO |
| NS3 | YAAQGYKVL | 12 | 1b/1a | 81 | 95 |
| NS5B | GACYSIEPL | 12 | 1b/1a | 82 | 1103 |
| NS2 | ACGDIILGL | 12 | 1b | 83 | 784 |
| E2 | FAIKWEYVL | 12 | 1b | 84 | 1049 |
| NS3 | QAPPGARSL | 12 | 1b | 85 | 1598 |
| C | AQPGYPWPL | 12 | 1b/1a/3a | 86 | 65 |
| NS3 | AQGYKVLVL | 12 | 1b/1a | 87 | 130 |
| NS3 | RAYLNTPGL | 12 | 1b | 88 | 434 |
| NS2 | WAHAGLRDL | 12 | 1b | 89 | 1992 |
| NS4B | GAAVGSIGL | 12 | 1b | 90 | 1102 |
| NS5A | ASQLSAPSL | 12 | 1b/1a/3a | 91 | 872 |
| P7 | GAHGILSFL | 12 | 1b | 92 | 1104 |
| NS5B | SAACRAAKL | 12 | 1b | 93 | 121 |
| NS3 | GAVQNEVTL | 12 | 1b/1a | 94 | 347 |
| E2 | AIKWEYVLL | 12 | 1b | 95 | 814 |
| NS3 | TAGARLVVL | 12 | 1b/1a | 96 | 249 |
| E1 | WAKVLIVML | 12 | 1b | 97 | 1994 |
| NS5B | ASTVKAKLL | 12 | 1b | 98 | 875 |
| NS5A | YAPACKPLL | 12 | 1b | 99 | 2027 |
| NS5B | RAATCGKYL | 12 | 1b | 100 | 1627 |
| NS2 | MAFMKLAAL | 12 | 1b | 101 | 1491 |
| C | ALAHGVRVL | 12 | 1b/1a | 102 | 72 |
| P7 | ALYGVWPLL | 12 | 1b | 103 | 830 |
| E1 | TALVVSQLL | 12 | 1b | 104 | 1813 |
| C | EGMGWAGWL | 12 | 1b | 105 | 1011 |
| E2 | TALNCNDSL | 12 | 1b | 106 | 1812 |
| NS5B | KASTVKAKL | 12 | 1b | 107 | 1316 |
| E1 | VAGAHWGVL | 12 | 1b | 108 | 1889 |
| P7 | YALYGVWPL | 12 | 1b | 109 | 2025 |
| NS5B | PARLIVFPDL | 12 | 1b/1a | 51 | 1545 |
| P7 | YALYGVWPLL | 12 | 1b | 52 | 2026 |
| NS5B | ILMTHFFSIL | 12 | 1b | 53 | 1274 |
| NS5B | LAQEQLEKAL | 12 | 1b | 54 | 1362 |
| NS5B | ACYSIEPLDL | 12 | 1b/1a | 55 | 785 |
| NS2 | GAVFVGLALL | 12 | 1b | 56 | 1108 |
| E2 | AIKWEYVLLL | 12 | 1b | 57 | 815 |
| NS5B | YATTSRSASL | 12 | 1b | 58 | 2029 |

Table continued

| BIMAS | | | | | |
|---|---|---|---|---|---|
| Prot | Peptide sequence | Score | GT | rank | SEQ ID NO |
| NS3 | YIMACMSADL | 12 | 1b | 59 | 2037 |
| NS5B | QAIRSLTERL | 12 | 1b | 60 | 1597 |
| P7 | CAAWYIKGRL | 12 | 1b | 61 | 908 |
| E2/P7 | AQAEAALENL | 12 | 1b | 62 | 858 |
| E1 | WAKVLIVMLL | 12 | 1b | 63 | 1995 |
| NS3 | LAAKLSALGL | 12 | 1b | 64 | 1356 |
| P7 | ALYGVWPLLL | 12 | 1b | 65 | 831 |
| NS5A | SASQLSAPSL | 12 | 1b/1a/3a | 66 | 1720 |
| NS3 | TAYSQQTRGL | 12 | 1b | 67 | 1814 |
| E2/P7 | EAALENLVVL | 12 | 1b | 68 | 998 |
| NS5B | MALYDVVSTL | 12 | 1b | 69 | 1492 |
| NS5B | CTMLVNGDDL | 12 | 1b | 70 | 942 |
| C | RALAHGVRVL | 12 | 1b/1a | 71 | 1629 |
| E2 | FAIKWEYVLL | 12 | 1b | 72 | 1050 |
| NS5B | DASGKRVYYL | 12 | 1b | 73 | 955 |
| E1 | GAHWGVLAGL | 12 | 1b | 74 | 1105 |
| NS5B | KASTVKAKLL | 12 | 1b | 75 | 1317 |
| C | EGMGWAGWLL | 12 | 1b | 76 | 1012 |
| NS2 | AQGLIRACML | 12 | 1b | 77 | 860 |
| P7 | AGAHGILSFL | 12 | 1b | 78 | 805 |
| NS4B | AGAAVGSIGL | 12 | 1b | 79 | 804 |
| P7 | ASVAGAHGIL | 12 | 1b | 80 | 876 |
| NS5B | ASAACRAAKL | 12 | 1b | 81 | 869 |
| NS3 | DQMWKCLIRL | 12 | 1b/1a | 82 | 982 |
| NS4B | APPSAASAFV | 12 | 1b | 83 | 845 |
| NS4B | DAAARVTQIL | 12 | 1b | 84 | 952 |
| NS5B | AGTQEDAASL | 12 | 1b | 85 | 807 |
| C | WAQPGYPWPL | 12 | 1b/1a/3a | 86 | 1996 |
| NS5B | SLRVFTEAM | 10 | 1b | 110 | 495 |
| C | GVRVLEDGV | 10 | 1b/1a | 111 | 447 |
| E2 | KVRMYVGGV | 10 | 1b/1a | 112 | 1351 |
| NS5B | DVRNLSSKAV | 10 | 1b | 87 | 993 |
| E1 | AARNSSVPT | 9 | 1b | 113 | 778 |
| NS5B | AAKLQDCTM | 9 | 1b | 114 | 440 |
| E2 | AARTTSGFT | 9 | 1b | 115 | 780 |
| NS3 | APPGARSLT | 9 | 1b | 116 | 839 |
| NS3 | AATLGFGAYM | 9 | 1b/1a | 88 | 781 |

Table continued

| BIMAS | | | | | |
|---|---|---|---|---|---|
| Prot | Peptide sequence | Score | GT | rank | SEQ ID NO |
| E1 | AARNSSVPTT | 9 | 1b | 89 | 779 |
| E2 | GPWLTPRCLV | 9 | 1b | 90 | 1174 |
| NS5A | PPVVHGCPL | 8 | 1b/1a | 117 | 1582 |
| E2 | YPCTVNFTI | 8 | 1b | 118 | 2059 |
| NS3 | CPSGHAVGI | 8 | 1b | 119 | 931 |
| NS5B | EPLDLPQII | 8 | 1b | 120 | 1027 |
| E2 | LPALSTGLI | 8 | 1b/1a | 121 | 1419 |
| E2 | GPSQKIQLI | 8 | 1b | 122 | 1171 |
| NS5A | LPPTKAPPI | 8 | 1b | 123 | 1435 |
| NS2 | GPLMVLQAGI | 8 | 1b | 91 | 1168 |
| NS3 | IPFYGKAIPI | 8 | 1b | 92 | 1284 |
| NS5B | TPVNSWLGNI | 8 | 1b/1a/3a | 93 | 1862 |
| NS5B | PPQPEYDLEL | 8 | 1b/1a | 94 | 1575 |
| NS5B | WSTLPQAVM | 7,5 | 1b | 95 | 1986 |
| NS3 | AVDFVPVESM | 6,75 | 1b | 96 | 883 |
| NS3 | TLHGPTPLL | 6 | 1b/1a/3a | 124 | 81 |
| C | APLGGAARA | 6 | 1b/1a | 125 | 384 |
| NS5B | ESKLPINAL | 6 | 1b | 126 | 1031 |
| C | SPRGSRPSW | 6 | 1b/1a/3a | 127 | 386 |
| NS3 | YSQQTRGLL | 6 | 1b | 128 | 2069 |
| NS3 | LSPRPVSYL | 6 | 1b | 129 | 1460 |
| NS5B | CPMGFSYDT | 6 | 1b | 130 | 421 |
| NS5A | LPCEPEPDV | 6 | 1b/1a/3a | 131 | 1421 |
| NS2 | ILLGPADSL | 6 | 1b | 132 | 1271 |
| NS5A | APSLKATCT | 6 | 1b/1a | 133 | 848 |
| NS5B | FNWAVRTKL | 6 | 1b/1a | 134 | 1076 |
| NS3 | TSVRLRAYL | 6 | 1b | 135 | 1870 |
| NS3 | APTGSGKST | 6 | 1b/1a/3a | 136 | 397 |
| NS5A | LPRLPGVPF | 6 | 1b | 137 | 1439 |
| NS4B | VVESKWRAL | 6 | 1b | 138 | 1979 |
| E2 | APRPCGIVP | 6 | 1b | 139 | 846 |
| P7 | YGVWPLLLL | 6 | 1b | 140 | 2036 |
| NS5B | GGRKPARLI | 6 | 1b/1a/3a | 141 | 435 |
| NS4B | AVQWMNRLI | 6 | 1b/1a/3a | 142 | 893 |
| E1 | MNWSPTTAL | 6 | 1b | 143 | 1503 |
| NS5A | PPRRKRTVV | 6 | 1b | 144 | 1578 |
| NS4B | APVVESKWRA | 6 | 1b | 97 | 856 |

Table continued

| BIMAS | | | | | |
|---|---|---|---|---|---|
| Prot | Peptide sequence | Score | GT | rank | SEQ ID NO |
| NS3 | APITAYSQQT | 6 | 1b | 98 | 835 |
| NS2 | VSARRGREIL | 6 | 1b/1a | 99 | 1970 |
| NS5B | YLTRDPTTPL | 6 | 1b/1a | 100 | 2051 |
| NS2 | EILLGPADSL | 6 | 1b | 101 | 1013 |
| NS2 | AVHPELIFDI | 6 | 1b | 102 | 890 |
| E1 | MMNWSPTTAL | 6 | 1b | 103 | 1502 |
| NS3 | LLSPRPVSYL | 6 | 1b | 104 | 1409 |
| E1 | VPTTTIRRHV | 6 | 1b | 105 | 1956 |
| NS5A | EPDVAVLTSM | 6 | 1b/1a | 106 | 1023 |
| NS3 | RRRGDSRGSL | 6 | 1b/1a | 107 | 1697 |
| E2 | GSWHINRTAL | 6 | 1b | 108 | 1190 |
| NS5A | APSLKATCTT | 6 | 1b | 109 | 849 |
| NS3 | ETSVRLRAYL | 6 | 1b | 110 | 1033 |
| NS4A | RPAVIPDREV | 6 | 1b | 111 | 1674 |
| NS3 | VVVATDALM | 5 | 1b/1a | 145 | 343 |
| NS5B | GVRVCEKMA | 5 | 1b/1a | 146 | 498 |
| NS3 | GVRTITTGA | 5 | 1b | 147 | 1202 |
| NS5B | DVRNLSSKA | 5 | 1b | 148 | 992 |
| NS5A | GVWRGDGIM | 5 | 1b/1a | 149 | 1209 |
| E2 | RVCACLWMM | 5 | 1b | 150 | 1708 |
| NS4A | EVLYREFDEM | 5 | 1b/1a | 112 | 1039 |
| NS3 | VVVVATDALM | 5 | 1b/1a | 113 | 1989 |
| NS2 | KVAGGHYVQM | 5 | 1b | 114 | 1349 |
| NS3 | SVRLRAYLNT | 5 | 1b | 115 | 1802 |
| NS2 | FVRAQGLIRA | 5 | 1b | 116 | 1092 |
| E1 | CVRENNSSRC | 5 | 1b | 117 | 948 |
| E1 | IVYEAADMIM | 5 | 1b | 118 | 1313 |
| NS5A | GVRLHRYAPA | 5 | 1b | 119 | 1201 |
| NS5A | ANLLWRQEM | 4,5 | 1b/1a/3a | 151 | 832 |
| C | KARRPEGRA | 4,5 | 1b | 152 | 1315 |
| NS3 | AVGIFRAAV | 4,5 | 1b/1a | 153 | 887 |
| NS2 | AQGLIRACM | 4,5 | 1b | 154 | 859 |
| NS5A | LARGSPPSLA | 4,5 | 1b | 120 | 1364 |
| NS5B | EARQAIRSLT | 4,5 | 1b | 121 | 1001 |
| NS5A | EANLLWRQEM | 4,5 | 1b/1a | 122 | 1000 |
| NS2 | RAQGLIRACM | 4,5 | 1b | 123 | 1630 |
| NS3 | AGPKGPITQM | 4,5 | 1b | 124 | 806 |

Table continued

| BIMAS | | | | | |
|---|---|---|---|---|---|
| Prot | Peptide sequence | Score | GT | rank | SEQ ID NO |
| NS2 | AVEPVVFSDM | 4,5 | 1b | 125 | 885 |
| NS5A | LQSKLLPRL | 4 | 1b | 155 | 1449 |
| E1 | NSSRCWVAL | 4 | 1b | 156 | 1533 |
| NS3 | NIRTGVRTI | 4 | 1b/1a | 157 | 436 |
| NS5B | SGGDIYHSL | 4 | 1b | 158 | 1731 |
| E1 | TTIRRHVDL | 4 | 1b | 159 | 1874 |
| NS4B | SSLTITQLL | 4 | 1b | 160 | 1785 |
| NS5A | VILDSFDPL | 4 | 1b/1a | 161 | 1918 |
| NS2 | LTCAVHPEL | 4 | 1b | 162 | 1464 |
| NS5B | DLPQIIERL | 4 | 1b | 163 | 967 |
| E2 | CSFTTLPAL | 4 | 1b/1a | 164 | 936 |
| NS5B | EINRVASCL | 4 | 1b | 165 | 1014 |
| E1 | GSVFLVSQL | 4 | 1b | 166 | 1189 |
| C | TLTCGFADL | 4 | 1b/1a/3a | 167 | 363 |
| NS5B | LTTSCGNTL | 4 | 1b/1a | 168 | 304 |
| NS4B | FTASITSPL | 4 | 1b | 169 | 1085 |
| NS2 | CGGAVFVGL | 4 | 1b | 170 | 915 |
| E2 | TLPALSTGL | 4 | 1b/1a | 171 | 1835 |
| NS5B | LSVGVGIYL | 4 | 1b | 172 | 1463 |
| NS3 | VTQTVDFSL | 4 | 1b/1a | 173 | 712 |
| C | FSIFLLALL | 4 | 1b/1a | 174 | 362 |
| C | RSRNLGKVI | 4 | 1b/1a/3a | 175 | 318 |
| C | GQIVGGVYL | 4 | 1b/1a | 176 | 127 |
| NS2 | HLQVWVPPL | 4 | 1b | 177 | 1232 |
| NS3 | TCGSSDLYL | 4 | 1b/1a | 178 | 1816 |
| C | GCSFSIFLL | 4 | 1b/1a/3a | 179 | 294 |
| NS3 | HSKKKCDEL | 4 | 1b/1a | 180 | 455 |
| NS5B | NIIMYAPTL | 4 | 1b | 181 | 87 |
| NS2 | ITKLLLAIL | 4 | 1b | 182 | 1308 |
| E2 | RTTSGFTSL | 4 | 1b | 183 | 1706 |
| NS3 | QMWKCLIRL | 4 | 1b/1a | 184 | 238 |
| NS5B | GTQEDAASL | 4 | 1b | 185 | 88 |
| NS3 | HSTDSTTIL | 4 | 1b | 186 | 1244 |
| NS2 | LSPYYKVFL | 4 | 1b | 187 | 1461 |
| NS3 | QTRGLLGCI | 4 | 1b/1a | 188 | 1621 |
| C | NLGKVIDTL | 4 | 1b/1a/3a | 189 | 283 |
| NS3 | VSTATQSFL | 4 | 1b | 190 | 1973 |

Table continued

| BIMAS | | | | | |
|---|---|---|---|---|---|
| Prot | Peptide sequence | Score | GT | rank | SEQ ID NO |
| NS3 | KGSSGGPLL | 4 | 1b/1a | 191 | 260 |
| NS3 | LLGCIITSL | 4 | 1b/1a | 192 | 1397 |
| C | YIPLVGAPL | 4 | 1b/1a | 193 | 69 |
| NS3 | IPTSGDVVV | 4 | 1b/1a | 194 | 415 |
| E2 | LQTGFLAAL | 4 | 1b | 195 | 1450 |
| NS4B | VGVVCAAIL | 4 | 1b/1a | 196 | 1912 |
| NS2 | LIFDITKLL | 4 | 1b | 197 | 1386 |
| NS2 | FITRAEAHL | 4 | 1b | 198 | 1061 |
| NS4B | SGIQYLAGL | 4 | 1b/1a/3a | 199 | 1732 |
| NS4B | GSIGLGKVL | 4 | 1b | 200 | 1186 |
| P7 | RLVPGAAYAL | 4 | 1b | 126 | 1666 |
| E2 | VCACLWMMLL | 4 | 1b | 127 | 1896 |
| NS5A | WLQSKLLPRL | 4 | 1b | 128 | 2014 |
| NS5B | LLSVEEACKL | 4 | 1b | 129 | 1410 |
| C | RNLGKVIDTL | 4 | 1b/1a/3a | 130 | 1673 |
| E1 | TTALVVSQLL | 4 | 1b | 131 | 1871 |
| NS4A | VLAALAAYCL | 4 | 1b/1a/3a | 132 | 1921 |
| NS3 | YLKGSSGGPL | 4 | 1b/1a | 133 | 2046 |
| NS4B | DLVNLLPAIL | 4 | 1b/1a | 134 | 969 |
| NS3 | CTCGSSDLYL | 4 | 1b/1a | 135 | 941 |
| E1 | TTIRRHVDLL | 4 | 1b | 136 | 1875 |
| NS5B | LMTHFFSILL | 4 | 1b | 137 | 1415 |
| NS5B | YSGGDIYHSL | 4 | 1b | 138 | 2067 |
| NS3 | GLLGCIITSL | 4 | 1b/1a | 139 | 1151 |
| NS5B | RQKKVTFDRL | 4 | 1b/1a/3a | 140 | 1695 |
| NS3 | IPTSGDVVVV | 4 | 1b/1a | 141 | 1295 |
| E2 | VPASQVCGPV | 4 | 1b | 142 | 1946 |
| C | GQIVGGVYLL | 4 | 1b/1a | 143 | 1178 |
| NS2 | ELIFDITKLL | 4 | 1b | 144 | 1018 |
| NS5A | SLASSSASQL | 4 | 1b | 145 | 1740 |
| NS2 | TLSPYYKVFL | 4 | 1b | 146 | 1836 |
| E2 | QILPCSFTTL | 4 | 1b | 147 | 1606 |
| NS4B | GLGKVLVDIL | 4 | 1b/1a | 148 | 1149 |
| NS5B | YGACYSIEPL | 4 | 1b/1a | 149 | 2033 |
| NS4B | EQFKQKALGL | 4 | 1b/1a | 150 | 1029 |
| E1 | CGSVFLVSQL | 4 | 1b | 151 | 916 |
| NS3 | WQAPPGARSL | 4 | 1b | 152 | 2021 |

Table continued

| BIMAS | | | | | |
|---|---|---|---|---|---|
| Prot | Peptide sequence | Score | GT | rank | SEQ ID NO |
| E2 | RCLVDYPYRL | 4 | 1b/1a | 153 | 1635 |
| NS2 | KLLLAILGPL | 4 | 1b | 154 | 1331 |
| NS5B | LTPIPAASQL | 4 | 1b | 155 | 1472 |
| C | YLLPRRGPRL | 4 | 1b/1a | 156 | 2047 |
| NS5A | IPPPRRKRTV | 4 | 1b | 157 | 1292 |
| NS5B | GNIIMYAPTL | 4 | 1b | 158 | 1160 |
| NS2 | DITKLLLAIL | 4 | 1b | 159 | 961 |
| NS2 | PLRDWAHAGL | 4 | 1b | 160 | 1559 |
| NS2 | LLTCAVHPEL | 4 | 1b | 161 | 1413 |
| NS5A | ITAETAKRRL | 4 | 1b | 162 | 1306 |
| E2 | SGPWLTPRCL | 4 | 1b | 163 | 1735 |
| NS3 | QMYTNVDQDL | 4 | 1b/1a/3a | 164 | 1611 |
| NS5B | FSILLAQEQL | 4 | 1b | 165 | 1083 |
| E2 | RDRSELSPLL | 4 | 1b/1a | 166 | 1637 |
| E2 | TTLPALSTGL | 4 | 1b/1a | 167 | 1876 |
| NS3 | GPITQMYTNV | 4 | 1b | 168 | 1164 |
| NS2 | GGAVFVGLAL | 4 | 1b | 169 | 1135 |
| NS3 | QTRGLLGCII | 4 | 1b/1a | 170 | 1622 |
| NS5A | STVSSALAEL | 4 | 1b | 171 | 1792 |
| E2 | RTALNCNDSL | 4 | 1b | 172 | 1703 |
| NS3 | LNAVAYYRGL | 4 | 1b | 173 | 1416 |
| NS5B | VLTTSCGNTL | 4 | 1b/1a | 174 | 1930 |
| E2 | HQNIVDVQYL | 4 | 1b/1a/3a | 175 | 1240 |
| NS4B | LTITQLLKRL | 4 | 1b | 176 | 1470 |
| NS4B | ILSSLTITQL | 4 | 1b | 177 | 1276 |
| NS5B | SPGQRVEFLV | 4 | 1b/1a | 178 | 1765 |
| NS2 | SCGGAVFVGL | 4 | 1b | 179 | 1721 |
| NS3 | LTPAETSVRL | 4 | 1b | 180 | 1471 |
| NS5B | YSPGQRVEFL | 4 | 1b/1a | 181 | 2068 |
| NS3 | RPSGMFDSSV | 4 | 1b/1a | 182 | 1687 |
| NS4A | STWVLVGGVL | 4 | 1b/1a | 183 | 1794 |
| NS2 | RGGRDAIILL | 4 | 1b | 184 | 1649 |
| NS3 | HGPTPLLYRL | 4 | 1b/1a/3a | 185 | 1219 |
| NS5B | LLSVGVGIYL | 4 | 1b | 186 | 1412 |
| C | CSFSIFLLAL | 4 | 1b/1a/3a | 187 | 935 |
| C | DTLTCGFADL | 4 | 1b/1a/3a | 188 | 988 |
| NS5B | YRRCRASGVL | 4 | 1b/1a/3a | 189 | 2066 |

Table continued

| BIMAS | | | | | |
|---|---|---|---|---|---|
| **Prot** | **Peptide sequence** | **Score** | **GT** | **rank** | **SEQ ID NO** |
| NS4B | ISGIQYLAGL | 4 | 1b/1a | 190 | 1303 |
| NS5B | TERLYIGGPL | 4 | 1b | 191 | 1820 |
| NS4B/ NS5A | CSTPCSGSWL | 4 | 1b | 192 | 937 |
| E2 | YTKCGSGPWL | 4 | 1b | 193 | 2071 |
| E2 | TPRCLVDYPY | 4 | 1b/1a | 194 | 1857 |
| NS4B | SPGALVVGVV | 4 | 1b/1a | 195 | 1760 |
| E1 | SMVGNWAKVL | 4 | 1b/1a | 196 | 1754 |
| NS5A | LPRLPGVPFF | 4 | 1b | 197 | 1440 |
| E1 | FCSAMYVGDL | 4 | 1b | 198 | 1052 |
| E1 | NNSSRCWVAL | 4 | 1b | 199 | 1526 |
| NS4B | TSPLTTQHTL | 4 | 1b | 200 | 1869 |
| | | | | | |
| **Syfpeithi** | | | | | |
| **Prot** | **Peptide sequence** | **Score** | **GT** | **rank** | **SEQ ID NO** |
| NS5A | PPRRKRTVVL | 26 | 1b | 1 | 1579 |
| C | APLGGAARAL | 25 | 1b/1a | 2 | 836 |
| NS5A | LPRLPGVPF | 25 | 1b | 3 | 1439 |
| NS5A | EPEPDVAVL | 25 | 1b/1a | 4 | 1024 |
| NS5B | IPAASQLDL | 25 | 1b | 5 | 1280 |
| NS5B | RPRWFMLCL | 25 | 1b | 6 | 1684 |
| E2 | APRPCGIVPA | 24 | 1b | 7 | 847 |
| NS4B | MPSTEDLVNL | 24 | 1b | 8 | 1505 |
| P7 | WPLLLLLLAL | 23 | 1b/1a | 9 | 2018 |
| NS3 | KPTLHGPTPL | 23 | 1b/1a/3a | 10 | 1343 |
| NS5A | SPAPNYSRAL | 23 | 1b | 11 | 1756 |
| NSSB | PPQPEYDLEL | 23 | 1b/1a | 12 | 1575 |
| NS5B | APTLWARMIL | 23 | 1b/1a | 13 | 853 |
| NS5B | RPRWFMLCLL | 23 | 1b | 14 | 1685 |
| NS3 | HPNIEEVAL | 23 | 1b/1a | 15 | 1237 |
| NS3 | TPAETSVRL | 23 | 1b | 16 | 383 |
| NS5A | RPDYNPPLL | 23 | 1b/1a/3a | 17 | 1677 |
| NS5B | SPGQRVEFL | 23 | 1b/1a | 18 | 373 |
| NS5B | DPPQPEYDL | 23 | 1b/1a | 19 | 543 |
| C | LPGCSFSIFL | 22 | 1b/1a/3a | 20 | 1426 |
| E2 | SPGPSQKIQL | 22 | 1b | 21 | 1764 |
| NS3 | VPQTFQVAHL | 22 | 1b | 22 | 1954 |

Table continued

| Syfpeithi | | | | | |
|---|---|---|---|---|---|
| Prot | Peptide sequence | Score | GT | rank | SEQ ID NO |
| NS4B | LPGNPAIASL | 22 | 1b/1a | 23 | 1428 |
| NS4B | LPAILSPGAL | 22 | 1b/1a/3a | 24 | 1418 |
| C | QPRGRRQPI | 22 | 1b/1a/3a | 25 | 390 |
| C | DPRRRSRNL | 22 | 1b/1a/3a | 26 | 370 |
| E2 | TPSPVVVGT | 22 | 1b/1a/3a | 27 | 1860 |
| NS3 | GPKGPITQM | 22 | 1b | 28 | 1165 |
| NS3 | CPSGHAVGI | 22 | 1b | 29 | 931 |
| NS5A | PPWHGCPL | 22 | 1b/1a | 30 | 1582 |
| C | LPRRGPRLGV | 21 | 1b/1a/3a | 31 | 450 |
| NS3 | CPSGHAVGIF | 21 | 1b | 32 | 932 |
| NS3 | NPSVAATLGF | 21 | 1b/1a/3a | 33 | 1532 |
| NS3 | IPTSGDVVVV | 21 | 1b/1a | 34 | 1295 |
| NS3 | RPSGMFDSSV | 21 | 1b/1a | 35 | 1687 |
| NS4B | SPLTTQHTLL | 21 | 1b | 36 | 1768 |
| NS5A | LPRLPGVPFF | 21 | 1b | 37 | 1440 |
| NS5A | APSLKATCTT | 21 | 1b | 38 | 849 |
| NS5A | VPPVVHGCPL | 21 | 1b | 39 | 1953 |
| NS5B | TPCAAEESKL | 21 | 1b | 40 | 1840 |
| C | APLGGAARA | 21 | 1b/1a | 41 | 384 |
| NS3 | APPGARSLT | 21 | 1b | 42 | 839 |
| NS3 | APTGSGKST | 21 | 1b/1a/3a | 43 | 397 |
| NS3 | GPTPLLYRL | 21 | 1b/1a/3a | 44 | 307 |
| NS4B | PPSAASAFV | 21 | 1b | 45 | 1580 |
| NS4B | SPGALVVGV | 21 | 1b/1a/3a | 46 | 1759 |
| NS5A | APSLKATCT | 21 | 1b/1a | 47 | 848 |
| NS5A | PPRRKRTVV | 21 | 1b | 48 | 1578 |
| NS5B | TPIPAASQL | 21 | 1b | 49 | 1848 |
| E2 | TPSPVVVGTT | 20 | 1b/1a/3a | 50 | 1861 |
| E2 | LPCSFTTLPA | 20 | 1b/1a | 51 | 1424 |
| NS2 | VPYFVRAQGL | 20 | 1b | 52 | 1960 |
| NS3 | TPCTCGSSDL | 20 | 1b/1a | 53 | 1843 |
| NS4B | LPYIEQGMQL | 20 | 1b | 54 | 1447 |
| NS4B | APPSAASAFV | 20 | 1b | 55 | 845 |
| NS4B | SPGALVVGVV | 20 | 1b/1a | 56 | 1760 |
| NS5A | VPAPEFFTEV | 20 | 1b | 57 | 1944 |
| NS5A | EPEPDVAVLT | 20 | 1b/1a | 58 | 1025 |
| NS5B | LPINALSNSL | 20 | 1b/1a | 59 | 1430 |

Table continued

| Syfpeithi | | | | | |
|---|---|---|---|---|---|
| Prot | Peptide sequence | Score | GT | rank | SEQ ID NO |
| C | GPRLGVRAT | 20 | 1b/1a/3a | 60 | 387 |
| E2 | GPWLTPRCL | 20 | 1b | 61 | 1173 |
| NS3 | IPTSGDVVV | 20 | 1b/1a | 62 | 415 |
| NS4B | SPLTTQHTL | 20 | 1b | 63 | 1767 |
| NS5A | LPCEPEPDV | 20 | 1b/1a/3a | 64 | 1421 |
| NS5A | DPSHITAET | 20 | 1b | 65 | 976 |
| NS5B | DPTTPLARA | 20 | 1b/1a | 66 | 980 |
| E1 | IPQAWDMVA | 19 | 1b | 67 | 1294 |
| E2 | PPQGNWFGCT | 19 | 1b | 68 | 1574 |
| NS5A | KPLLREEVTF | 19 | 1b | 69 | 1339 |
| NS5A | EPDVAVLTSM | 19 | 1b/1a | 70 | 1023 |
| NS5A | PPPRRKRTVV | 19 | 1b | 71 | 1573 |
| NS5B | QPEKGGRKPA | 19 | 1b/1a | 72 | 1612 |
| E1 | IPQAVVDMV | 19 | 1b | 73 | 1293 |
| NS3 | APPPSWDQM | 19 | 1b/1a | 74 | 381 |
| NS3 | TPLLYRLGA | 19 | 1b/1a | 75 | 389 |
| NS4B | NPAIASLMA | 19 | 1b/1a | 76 | 1527 |
| NS4B | APPSAASAF | 19 | 1b | 77 | 844 |
| NS5A | DPDYVPPVV | 19 | 1b | 78 | 971 |
| NS5A | IPPPRRKRT | 19 | 1b | 79 | 1291 |
| NS5B | CPMGFSYDT | 19 | 1b | 80 | 421 |
| E2 | VPASQVCGPV | 18 | 1b | 81 | 1946 |
| E2 | YPCTVNFTIF | 18 | 1b | 82 | 2060 |
| NS3 | APITAYSQQT | 18 | 1b | 83 | 835 |
| NS3 | PPAVPQTFQV | 18 | 1b | 84 | 1564 |
| NS3 | AAQGYKVLVL | 18 | 1b/1a | 85 | 777 |
| NS3 | DPNIRTGVRT | 18 | 1b/1a | 86 | 973 |
| NS3 | VPHPNIEEVA | 18 | 1b/1a | 87 | 1951 |
| NS3 | IPFYGKAIPI | 18 | 1b | 88 | 1284 |
| NS3 | LPVCQDHLEF | 18 | 1b/1a | 89 | 1444 |
| NS4A | RPAVIPDREV | 18 | 1b | 90 | 1674 |
| NS4B | APWESKWRA | 18 | 1b | 91 | 856 |
| NS4B | NPAIASLMAF | 18 | 1b/1a | 92 | 1528 |
| NS4B | GPGEGAVQWM | 18 | 1b/1a/3a | 93 | 1163 |
| NS5A | DPSHITAETA | 18 | 1b | 94 | 977 |
| NS5A | IPPPRRKRTV | 18 | 1b | 95 | 1292 |
| NS5B | QPEYDLELIT | 18 | 1b/1a | 96 | 1614 |

Table continued

| Syfpeithi | | | | | |
|---|---|---|---|---|---|
| **Prot** | **Peptide sequence** | **Score** | **GT** | **rank** | **SEQ ID NO** |
| C | LPGCSFSIF | 18 | 1b/1a/3a | 97 | 375 |
| E2 | GPSQKIQLI | 18 | 1b | 98 | 1171 |
| E2 | LPALSTGLI | 18 | 1b/1a | 99 | 1419 |
| P7 | WPLLLLLA | 18 | 1b/1a | 100 | 2017 |
| NS2 | AILGPLMVL | 18 | 1b | 101 | 816 |
| NS4B | LPAILSPGA | 18 | 1b/1a/3a | 102 | 1417 |
| NS5A | SPAPNYSRA | 18 | 1b | 103 | 1755 |
| NS5A | KPLLREEVT | 18 | 1b | 104 | 1338 |
| NS5A | PPSLASSSA | 18 | 1b | 105 | 1581 |
| NS5A | SPDADLIEA | 18 | 1b | 106 | 1758 |
| NS5A | LPPTKAPPI | 18 | 1b | 107 | 1435 |
| NS5B | LTRDPTTPL | 18 | 1b/1a | 108 | 1475 |
| NS5B | APTLWARMI | 18 | 1b/1a | 109 | 371 |
| NS5B | SPGEINRVA | 18 | 1b/1a | 110 | 1761 |
| C | KPQRKTKRNT | 17 | 1b/1a/3a | 111 | 1341 |
| E1 | VPTTTIRRHV | 17 | 1b | 112 | 1956 |
| E1 | YPGHVSGHRM | 17 | 1b | 113 | 2063 |
| E2 | GPWLTPRCLV | 17 | 1b | 114 | 1174 |
| NS2 | GPLMVLQAGI | 17 | 1b | 115 | 1168 |
| NS2 | EPVVFSDMET | 17 | 1b | 116 | 1028 |
| NS3 | GPITQMYTNV | 17 | 1b | 117 | 1164 |
| NS3 | VPVESMETTM | 17 | 1b | 118 | 1958 |
| NS3 | ETAGARLVVL | 17 | 1b/1a | 119 | 1032 |
| NS3 | DPTFTIETTT | 17 | 1b/1a | 120 | 979 |
| NS3 | TPGERPSGMF | 17 | 1b | 121 | 1845 |
| NS3 | FPYLVAYQAT | 17 | 1b/1a | 122 | 1079 |
| NS3 | TPLLYRLGAV | 17 | 1b/1a | 123 | 1851 |
| NS4B | VPESDAAARV | 17 | 1b/1a/3a | 124 | 1948 |
| NS5A | CPCQVPAPEF | 17 | 1b | 125 | 927 |
| NS5A | LPCEPEPDVA | 17 | 1b/1a | 126 | 1422 |
| NS5B | SPGQRVEFLV | 17 | 1b/1a | 127 | 1765 |
| NS5B | DPTTPLARAA | 17 | 1b/1a | 128 | 981 |
| NS5B | TPLARAAWET | 17 | 1b/1a/3a | 129 | 1850 |
| NS5B | PPLRVWRHRA | 17 | 1b | 130 | 1571 |
| E2 | APRPCGIVP | 17 | 1b | 131 | 846 |
| NS2 | SPYYKVFLA | 17 | 1b | 132 | 1780 |
| NS2 | HPELIFDIT | 17 | 1b | 133 | 1235 |

Table continued

| Syfpeithi | | | | | |
|---|---|---|---|---|---|
| Prot | Peptide sequence | Score | GT | rank | SEQ ID NO |
| NS2 | TPLRDWAHA | 17 | 1b | 134 | 1852 |
| NS3 | SPPAVPQTF | 17 | 1b | 135 | 1770 |
| NS3 | AQGYKVLVL | 17 | 1b/1a | 136 | 130 |
| NS3 | VPHPNIEEV | 17 | 1b/1a | 137 | 1950 |
| NS3 | TPGERPSGM | 17 | 1b | 138 | 372 |
| NS3 | TLHGPTPLL | 17 | 1b/1a/3a | 139 | 81 |
| NS5A | FPPALPIWA | 17 | 1b | 140 | 1078 |
| NS5A | PRRKRTVVL | 17 | 1b | 141 | 1583 |
| NS5A | EPGDPDLSD | 17 | 1b/1a | 142 | 1026 |
| NS5B | TPIDTTIMA | 17 | 1b/1a | 143 | 1847 |
| NS5B | EPLDLPQII | 17 | 1b | 144 | 1027 |
| P7 | ALYGVWPLLL | 16 | 1b | 145 | 831 |
| NS2 | SCGGAVFVGL | 16 | 1b | 146 | 1721 |
| NS2 | TLSPYYKVFL | 16 | 1b | 147 | 1836 |
| NS2 | AACGDIILGL | 16 | 1b | 148 | 774 |
| NS3 | APPGARSLTP | 16 | 1b | 149 | 840 |
| NS3 | RRRGDSRGSL | 16 | 1b/1a | 150 | 1697 |
| NS3 | GPLLCPSGHA | 16 | 1b | 151 | 1167 |
| NS3 | TPPGSVTVPH | 16 | 1b/1a | 152 | 1854 |
| NS3 | KQAGDNFPYL | 16 | 1b | 153 | 1346 |
| NS5A | SPPSLASSSA | 16 | 1b | 154 | 1771 |
| NS5B | TPPHSAKSKF | 16 | 1b/1a | 155 | 1856 |
| NS5B | TPVNSWLGNI | 16 | 1b/1a/3a | 156 | 1862 |
| NS5B | CLRKLGVPPL | 16 | 1b/1a | 157 | 921 |
| C | PRRGPRLGV | 16 | 1b/1a/3a | 158 | 449 |
| C | WPLYGNEGM | 16 | 1b | 159 | 2019 |
| C | SPRGSRPSW | 16 | 1b/1a/3a | 160 | 386 |
| E1 | MNWSPTTAL | 16 | 1b | 161 | 1503 |
| E2 | RPCGIVPAS | 16 | 1b | 162 | 1676 |
| E2 | GPPCNIGGV | 16 | 1b | 163 | 1169 |
| E2 | YPCTVNFTI | 16 | 1b | 164 | 2059 |
| NS2 | ARRGREILL | 16 | 1b/1a | 165 | 865 |
| NS2 | ILLGPADSL | 16 | 1b | 166 | 1271 |
| NS3 | VPVESMETT | 16 | 1b | 167 | 1957 |
| NS3 | PPAVPQTFQ | 16 | 1b | 168 | 1563 |
| NS3 | DPTFTIETT | 16 | 1b/1a | 169 | 978 |
| NS3 | RPSGMFDSS | 16 | 1b/1a | 170 | 1686 |

Table continued

| Syfpeithi | | | | | |
|---|---|---|---|---|---|
| **Prot** | **Peptide sequence** | **Score** | **GT** | **rank** | **SEQ ID** NO |
| NS3 | FPYLVAYQA | 16 | 1b/1a | 171 | 443 |
| NS3 | HPITKYIMA | 16 | 1b | 172 | 396 |
| NS5A | KSRKFPPAL | 16 | 1b | 173 | 1348 |
| NS5A | CPLPPTKAP | 16 | 1b | 174 | 928 |
| NS5A | APPIPPPRR | 16 | 1b | 175 | 843 |
| NS5A | PPPRRKRTV | 16 | 1b | 176 | 1572 |
| NS5B | PPHSAKSKF | 16 | 1b/1a | 177 | 1568 |
| NS5B | QPEYDLELI | 16 | 1b/1a | 178 | 1613 |
| C | FPGGGQIVGG | 15 | 1b/1a/3a | 179 | 1077 |
| C | QPRGRRQPIP | 15 | 1b/1a/3a | 180 | 1616 |
| C | RPSWGPTDPR | 15 | 1b/1a | 181 | 1690 |
| E1 | NNSSRCWVAL | 15 | 1b | 182 | 1526 |
| E1 | SPRRHETVQD | 15 | 1b | 183 | 1778 |
| E2 | AIKWEYVLLL | 15 | 1b | 184 | 815 |
| E2/P7 | EAALENLVVL | 15 | 1b | 185 | 998 |
| P7 | AYALYGVWPL | 15 | 1b | 186 | 901 |
| NS2 | AHLQVWVPPL | 15 | 1b | 187 | 811 |
| NS3 | AYSQQTRGLL | 15 | 1b | 188 | 906 |
| NS3 | SPRPVSYLKG | 15 | 1b | 189 | 1776 |
| NS3 | AYAAQGYKVL | 15 | 1b/1a | 190 | 900 |
| NS3 | ETSVRLRAYL | 15 | 1b | 191 | 1033 |
| NS4B | AFTASITSPL | 15 | 1b | 192 | 802 |
| NS4B | ILGGWVAAQL | 15 | 1b/1a | 193 | 1270 |
| NS5A | APACKPLLRE | 15 | 1b | 194 | 834 |
| NS5A | VESENKVVIL | 15 | 1b/1a | 195 | 1902 |
| NS5A | RKSRKFPPAL | 15 | 1b | 196 | 1655 |
| NS5A | WARPDYNPPL | 15 | 1b/1a/3a | 197 | 1998 |
| NS5B | EESKLPINAL | 15 | 1b | 198 | 1009 |
| NS5B | EKGGRKPARL | 15 | 1b/1a | 199 | 1015 |
| NS5B | ASAACRAAKL | 15 | 1b | 200 | 869 |
| NS5B | APPGDPPQPE | 15 | 1b/1a | 201 | 842 |
| NS5B | DASGKRVYYL | 15 | 1b | 202 | 955 |
| NS5B | SPGEINRVAS | 15 | 1b | 203 | 1762 |
| C | AQPGYPWPL | 15 | 16/1a/3a | 204 | 65 |
| C | QPGYPWPLY | 15 | 1b/1a/3a | 205 | 216 |
| C | ALAHGVRVL | 15 | 1b/1a | 206 | 72 |
| C | SFSIFLLAL | 15 | 1b/1a/3a | 207 | 250 |

Table continued

| Syfpeithi | | | | | |
|---|---|---|---|---|---|
| Prot | Peptide sequence | Score | GT | rank | SEQ ID NO |
| E1 | NSSRCWVAL | 15 | 1b | 208 | 1533 |
| E1 | AHWGVLAGL | 15 | 1b | 209 | 812 |
| E2 | WTRGERCDL | 15 | 1b/1a | 210 | 2022 |
| E2/P7 | AALENLVVL | 15 | 1b | 211 | 776 |
| P7 | ALYGVWPLL | 15 | 1b | 212 | 830 |
| P7 | YGVWPLLLL | 15 | 1b | 213 | 2036 |
| NS2 | CGGAVFVGL | 15 | 1b | 214 | 915 |
| NS2 | ACGDIILGL | 15 | 1b | 215 | 784 |
| NS3 | AYSQQTRGL | 15 | 1b | 216 | 905 |
| NS3 | KGSSGGPLL | 15 | 1b/1a | 217 | 260 |
| NS3 | TILGIGTVL | 15 | 1b | 218 | 89 |
| NS3 | TAGARLVVL | 15 | 1b/1a | 219 | 249 |
| NS3 | TPPGSVTVP | 15 | 1b/1a | 220 | 1853 |
| NS3 | PPGSVTVPH | 15 | 1b/1a | 221 | 1567 |
| NS3 | TPGLPVCQD | 15 | 1b/1a/3a | 222 | 1846 |
| NS4A | LVGGVLAAL | 15 | 1b/1a | 223 | 1479 |
| NS4A | AVIPDREVL | 15 | 1b | 224 | 891 |
| NS4A | IPDREVLYR | 15 | 1b/1a | 225 | 1282 |
| NS4B | LPGNPAIAS | 15 | 1b/1a | 226 | 1427 |
| NS4B | MPSTEDLVN | 15 | 1b | 227 | 1504 |
| NS5A | LPGVPFFSC | 15 | 1b | 228 | 1429 |
| NS5A | GPCTPSPAP | 15 | 1b | 229 | 1161 |
| NS5A | APACKPLLR | 15 | 1b | 230 | 833 |
| NS5A | EPDVAVLTS | 15 | 1b/1a | 231 | 1022 |
| NS5A | LARGSPPSL | 15 | 1b | 232 | 1363 |
| NS5A | HHDSPDADL | 15 | 1b | 233 | 1220 |
| NS5A | PPALPIWAR | 15 | 1b | 234 | 1562 |
| NS5B | ESKLPINAL | 15 | 1b | 235 | 1031 |
| NS5B | KPARLIVFP | 15 | 1b/1a | 236 | 1337 |
| NS5B | AIRSLTERL | 15 | 1b | 237 | 473 |
| NS5B | APPGDPPQP | 15 | 1b/1a | 238 | 841 |
| NS5B | PPGDPPQPE | 15 | 1b/1a | 239 | 1565 |
| NS5B | ASGKRVYYL | 15 | 1b | 240 | 334 |
| NS5B | RARSVRAKL | 15 | 1b | 241 | 1632 |
| C | GPRLGVRATR | 14 | 1b/1a/3a | 242 | 1170 |
| C | RPEGRAWAQP | 14 | 1b | 243 | 1678 |
| C | EGMGWAGWLL | 14 | 1b | 244 | 1012 |

Table continued

| Syfpeithi | | | | | |
|---|---|---|---|---|---|
| **Prot** | **Peptide sequence** | **Score** | **GT** | **rank** | **SEQ ID** NO |
| C | SPRGSRPSWG | 14 | 1b/1a/3a | 245 | 1773 |
| C | RALAHGVRVL | 14 | 1b/1a | 246 | 1629 |
| C/E1 | IPASAYEVRN | 14 | 1b | 247 | 1281 |
| E1 | FCSAMYVGDL | 14 | 1b | 248 | 1052 |
| E1 | VGDLCGSVFL | 14 | 1b/1a | 249 | 1910 |
| E1 | MVAGAHWGVL | 14 | 1b | 250 | 1509 |
| | | | | | |
| nHLAPred | | | | | |
| **Prot** | **Peptide sequence** | **Score** | **GT** | **rank** | **SEQ ID** NO |
| NS4B | LPAILSPGA | 1.000 | 1b/1a/3a | 1 | 1417 |
| NS4B | PPSAASAFV | 1.000 | 1b | 2 | 1580 |
| NS5B | DPTTPLARA | 1.000 | 1b/1a | 3 | 980 |
| NS3 | PPGSVTVPH | 1.000 | 1b/1a | 4 | 1567 |
| NS5B | DPPQPEYDL | 1.000 | 1b/1a | 5 | 543 |
| NS5B | SPGQRVEFL | 1.000 | 1b/1a | 6 | 373 |
| C | SPRGSRPSW | 1.000 | 1b/1a/3a | 7 | 386 |
| NS3 | IPTSGDVVV | 1.000 | 1b/1a | 8 | 415 |
| NS5A | RPDYNPPLL | 1.000 | 1b/1a/3a | 9 | 1677 |
| NS5B | MTHFFSILL | 1.000 | 1b | 10 | 1508 |
| NS2 | FLARLIWWL | 1.000 | 1b | 11 | 1063 |
| NS5B | TPPHSAKSK | 1.000 | 1b/1a | 12 | 1855 |
| E1 | VPTTTIRRH | 1.000 | 1b | 13 | 1955 |
| NS5A | APACKPLLR | 1.000 | 1b | 14 | 833 |
| NS3 | SPPAVPQTF | 1.000 | 1b | 15 | 1770 |
| C | DPRRRSRNL | 1.000 | 1b/1a/3a | 16 | 370 |
| NS3 | VPQTFQVAH | 1.000 | 1b | 17 | 410 |
| E2 | SPGPSQKIQ | 1.000 | 1b | 18 | 1763 |
| C | RPQDVKFPG | 1.000 | 1b/1a/3a | 19 | 552 |
| NS5B | RHTPVNSWL | 1.000 | 1b/1a/3a | 20 | 298 |
| NS5B | LMTHFFSIL | 1.000 | 1b | 21 | 1414 |
| NS5A | SPAPNYSRA | 1.000 | 1b | 22 | 1755 |
| C | LPGCSFSIF | 1.000 | 1b/1a/3a | 23 | 375 |
| NS3 | TPAETSVRL | 1.000 | 1b | 24 | 383 |
| C | FPGGGQIVG | 1.000 | 1b/1a/3a | 25 | 407 |
| NS3 | IMACMSADL | 1.000 | 1b | 26 | 90 |
| NS4B | SPLTTQHTL | 1.000 | 1b | 27 | 1767 |

Table continued

| nHLAPred | | | | | |
|---|---|---|---|---|---|
| Prot | Peptide sequence | Score | GT | rank | SEQ ID NO |
| NS5A | PPVVHGCPL | 1.000 | 1b/1a | 28 | 1582 |
| NS5A | FPPALPIWA | 1.000 | 1b | 29 | 1078 |
| NS5B | IPAASQLDL | 1.000 | 1b | 30 | 1280 |
| NS3 | HPNIEEVAL | 1.000 | 1b/1a | 31 | 1237 |
| NS5B | TPIPAASQL | 1.000 | 1b | 32 | 1848 |
| NS5B | RPRWFMLCL | 1.000 | 1b | 33 | 1684 |
| NS3 | VPHPNIEEV | 1.000 | 1b/1a | 34 | 1950 |
| NS5A | LPPTKAPPI | 1.000 | 1b | 35 | 1435 |
| NS5A | PPPRRKRTV | 1.000 | 1b | 36 | 1572 |
| NS5A | PPRRKRTVV | 1.000 | 1b | 37 | 1578 |
| E2 | YPCTVNFTI | 1.000 | 1b | 38 | 2059 |
| E2 | GPWLTPRCL | 1.000 | 1b | 39 | 1173 |
| NS3 | GPTPLLYRL | 1.000 | 1b/1a/3a | 40 | 307 |
| NS5A | LPRLPGVPF | 1.000 | 1b | 41 | 1439 |
| C | QPIPKARRP | 0.990 | 1b/1a | 42 | 479 |
| NS5A | PPALPIWAR | 0.990 | 1b | 43 | 1562 |
| E2 | RPIDKFAQG | 0.990 | 1b | 44 | 1679 |
| C | LPRRGPRLG | 0.990 | 1b/1a/3a | 45 | 380 |
| NS5A | PPIPPPRRK | 0.990 | 1b | 46 | 1570 |
| NS5B | LPQIIERLH | 0.990 | 1b | 47 | 1438 |
| E1 | SPRRHETVQ | 0.990 | 1b | 48 | 1777 |
| NS5A | APPIPPPRR | 0.990 | 1b | 49 | 843 |
| NS3 | PPAVPQTFQ | 0.990 | 1b | 50 | 1563 |
| P7/ NS2 | PPRAYAMDR | 0.990 | 1b | 51 | 1576 |
| E2 | CPTDCFRKH | 0.990 | 1b/1a/3a | 52 | 934 |
| E2 | GPPCNIGGV | 0.990 | 1b | 53 | 1169 |
| NS4B | NPAIASLMA | 0.990 | 1b/1a | 54 | 1527 |
| NS3 | HPITKYIMA | 0.990 | 1b | 55 | 396 |
| NS2 | SPYYKVFLA | 0.990 | 1b | 56 | 1780 |
| NS5B | KPARLIVFP | 0.990 | 1b/1a | 57 | 1337 |
| NS5A | LPCEPEPDV | 0.990 | 1b/1a/3a | 58 | 1421 |
| NS3 | IPVRRRGDS | 0.990 | 1b/1a | 59 | 1297 |
| NS4B/ NS5A | TPCSGSWLR | 0.990 | 1b/1a | 60 | 1841 |
| C | GPTDPRRRS | 0.990 | 1b/1a | 61 | 1172 |
| E2 | LPALSTGLI | 0.990 | 1b/1a | 62 | 1419 |
| NS4B | LPYIEQGMQ | 0.990 | 1b | 63 | 1446 |
| E1 | TPGCVPCVR | 0.980 | 1b/1a | 64 | 1844 |

Table continued

| nHLAPred | | | | | |
|---|---|---|---|---|---|
| **Prot** | **Peptide sequence** | **Score** | **GT** | **rank** | **SEQ ID NO** |
| E1 | IPQAVVDMV | 0.980 | 1b | 65 | 1293 |
| C | IPLVGAPLG | 0.980 | 1b/1a | 66 | 442 |
| NS5A | CPCGAQITG | 0.980 | 1b | 67 | 926 |
| E2 | RPYCWHYAP | 0.980 | 1b | 68 | 1691 |
| NS5A | IPPPRRKRT | 0.980 | 1b | 69 | 1291 |
| C | QPRGRRQPI | 0.980 | 1b/1a/3a | 70 | 390 |
| NS4B | LPGNPAIAS | 0.980 | 1b/1a | 71 | 1427 |
| NS5B | TPIDTTIMA | 0.980 | 1b/1a | 72 | 1847 |
| E2 | RPPQGNWFG | 0.980 | 1b | 73 | 1680 |
| P7/ NS2 | LPPRAYAMD | 0.980 | 1b | 74 | 1434 |
| NS5A | DPDYVPPW | 0.970 | 1b | 75 | 971 |
| NS3 | IPIEVIKGG | 0.970 | 1b | 76 | 561 |
| C | KPQRKTKRN | 0.970 | 1b/1a/3a | 77 | 1340 |
| NS5A | VPPVVHGCP | 0.970 | 1b | 78 | 1952 |
| NS5A | APNYSRALW | 0.970 | 1b | 79 | 838 |
| NS5B | TPLARAAWE | 0.970 | 1b/1a/3a | 80 | 1849 |
| NS3 | SPRPVSYLK | 0.970 | 1b | 81 | 1775 |
| NS3 | TPLLYRLGA | 0.970 | 1b/1a | 82 | 389 |
| E2 | GPSQKIQLI | 0.970 | 1b | 83 | 1171 |
| E1 | YPGHVSGHR | 0.970 | 1b | 84 | 2062 |
| NS4B | SPTHYVPES | 0.960 | 1b/1a/3a | 85 | 1779 |
| NS5B | LPQAVMGSS | 0.960 | 1b | 86 | 1436 |
| NS5A | LPGVPFFSC | 0.960 | 1b | 87 | 1429 |
| C | IPKARRPEG | 0.960 | 1b/1a | 88 | 409 |
| NS4B | SPGALVVGV | 0.960 | 1b/1a/3a | 89 | 1759 |
| NS3 | KPTLHGPTP | 0.960 | 1b/1a/3a | 90 | 1342 |
| NS5A | EPGDPDLSD | 0.960 | 1b/1a | 91 | 1026 |
| NS5A | LPIWARPDY | 0.960 | 1b | 92 | 1431 |
| NS5B | PPHSAKSKF | 0.960 | 1b/1a | 93 | 1568 |
| NS3 | TPCTCGSSD | 0.960 | 1b/1a | 94 | 1842 |
| NS4A | IPDREVLYR | 0.960 | 1b/1a | 95 | 1282 |
| NS5B | TPCAAEESK | 0.960 | 1b | 96 | 1839 |
| NS3 | CPSGHAVGI | 0.950 | 1b | 97 | 931 |
| NS3 | DPNIRTGVR | 0.950 | 1b/1a | 98 | 972 |
| NS5B | CPMGFSYDT | 0.950 | 1b | 99 | 421 |
| NS5A | TPSPAPNYS | 0.950 | 1b | 100 | 1859 |
| | | | | | |

Table continued

| Epimmune | | | | | |
|----------|------------------|-------|----------|------|-----------|
| **Prot** | **Peptide sequence** | **Score** | **GT** | **rank** | **SEQ ID** NO |
| NS5B | APTLWARMIL | 1,24 | 1b/1a | 1 | 853 |
| C | SPRGSRPSW | 1,64 | 1b/1a/3a | 2 | 386 |
| E2 | RPCGIVPAL | 1,89 | - | 3 | 1675 |
| C | QPRGRRQPI | 2,95 | 1b/1a/3a | 4 | 390 |
| C | APLGGAARAL | 3,46 | 1b/1a | 5 | 836 |
| NS4B | LPAILSPGAL | 4,39 | 1b/1a/3a | 6 | 1418 |
| C | LPRRGPRLGV | 4,88 | 1b/1a/3a | 7 | 450 |
| C | APLGGVARAL | 5,53 | - | 8 | 837 |
| NS4B | NPAIASLMAF | 7,2 | 1b/1a | 9 | 1528 |
| P7 | WPLLLLLLAL | 7,45 | 1b/1a | 10 | 2018 |
| NS5B | SPAQRVEFL | 7,57 | - | 11 | 1757 |
| NS3 | KPTLHGPTPL | 7,91 | 1b/1a/3a | 12 | 1343 |
| NS3 | IPFYGKAIPL | 7,94 | 1a | 13 | 1285 |
| C | SPRGSRPNW | 10,81 | - | 14 | 1772 |
| C | DPRRRSRNL | 12,09 | 1b/1a/3a | 15 | 370 |
| NS5B | APTLWARMI | 13,88 | 1b/1a | 16 | 371 |
| E2 | YPCTVNFTL | 16,54 | 3a | 17 | 2061 |
| NS5B | SPGQRVEFL | 21,27 | 1b/1a | 18 | 373 |
| NS5A | VPPVVHGCPL | 26,04 | 1b | 19 | 1953 |
| NS3 | IPFYGKAIPI | 29,35 | 1b/3a | 20 | 1284 |
| | PPRKKRTVV | 30,59 | 1a | 21 | 1577 |
| C | LPGCSFSIFL | 31,72 | 1b/1a/3a | 22 | 1426 |
| NS3 | RPSGMFDSSV | 37,43 | 1b/1a | 23 | 1687 |
| E2 | APRPCGIVPA | 38,12 | 1b | 24 | 847 |
| NS3 | HPITKYIMA | 38,64 | 1b | 25 | 396 |
| E2 | YPCTVNFSI | 41,7 | - | 26 | 2058 |
| NS4B | LPYIEQGMQL | 43,26 | 1b | 27 | 1447 |
| NS5B | LPINALSNSL | 45,73 | 1b/1a | 28 | 1430 |
| NS3 | KPTLQGPTPL | 47,48 | - | 29 | 1344 |
| NS3 | HPVTKYIMA | 47,89 | - | 30 | 1238 |
| | CPAGHAVGIF | 56,36 | 1a | 31 | 925 |
| | CPSGHVVGI | 61,68 | - | 32 | 933 |
| E2 | GPWLTPRCL | 64,48 | 1b | 33 | 1173 |
| E2 | YPCTVNFTI | 70,75 | 1b | 34 | 2059 |
| NS5A | RPDYNPPLL | 72,65 | 1b/1a/3a | 35 | 1677 |
| NS4B | APPSAASAFV | 75,67 | 1b | 36 | 845 |
| | GPKGPVTQM | 86,45 | - | 37 | 1166 |

Table continued

| Epimmune | | | | | |
|---|---|---|---|---|---|
| **Prot** | **Peptide sequence** | **Score** | **GT** | **rank** | **SEQ ID** NO |
| C | LPGCSFSIF | 101,3 | 1b/1a/3a | 38 | 375 |
| E2 | GPWLTPRCM | 104,59 | 3a | 39 | 1175 |
| E2 | TPRCLVDYPY | 237,55 | 1b/1a | 40 | 1857 |
| E1 | YPGHVSGHRM | 264,06 | 1b | 41 | 2063 |
| E2 | YPCTVNFTIF | 307,31 | 1b | 42 | 2060 |
| E2 | TPRCMVDYPY | 445,13 | 3a | 43 | 1858 |
| NS5A | EPDVAVLTSM | 597,05 | 1b/1a | 44 | 1023 |
| NS5B | TPPHSAKSKF | 699,16 | 1b/1a | 45 | 1856 |
| NS3 | TPGERPSGMF | 699,46 | 1b | 46 | 1845 |
| NS3 | TPGERPSGM | 833,63 | 1b | 47 | 372 |
| NS3 | APPPSWDQM | 933,01 | 1b/1a | 48 | 381 |
| NS4B | GPGEGAVQWM | 976,39 | 1b/1a/3a | 49 | 1163 |
| NS3 | NPSVAATLGF | 1610,36 | 1b/1a/3a | 50 | 1532 |
| NS3 | VPAAYAAQGY | 2733,82 | 1b/1a | 51 | 1943 |
| NS5B | PPHSARSKF | 4228,63 | 3a | 52 | 1569 |
| NS5A | LPIWARPDY | 4289,5 | 1b/3a | 53 | 1431 |
| NS3 | LPVCQDHLEF | 5715,31 | 1b/1a | 54 | 1444 |
| NS5B | PPHSAKSKF | 9169,56 | 1b/1a | 55 | 1568 |
| P7 | VPGAAYALY | 27777,1 | 1b | 56 | 1949 |
| C | QPGYPWPLY | 39918,4 | 1b/1a/3a | 57 | 216 |
| NS5B | PPGDPPQPEY | 633519,2 | 1b/1a | 58 | 1566 |

Those peptides that are present in at least the consensus sequence of genotype 1a and 1b, are selected. Table 15 contains all these peptides, with their score, and designated ranknumber, of each of the prediction servers in separate columns, and their occurrence in the different genotypes.

A selection according to genotype and ranknumber results in 232 different peptide sequences, i.e. 150+113+45+28=336. The table 16 contains the selection of peptides for which min. 2 out of 4 prediction servers give a rank =< 100. This renders 40 different sequences. Said peptides are finally incorporated in Table 13.

The selection of potential HLA B07 peptide binders is summarized as follows:

BIMAS (B7):

| output prediction server: | 200 9-mers |
|---|---|
| | 200 10-mers |
| BIMAS results: | paste 9-mers + 10-mers, sort on BIMAS score |
| | → 400 peptides, ranknumber for 9- and 10-mers separately (2x 1-200) |
| | → BIMAS ranking for peptides with same score unknown |
| BIMAS selection: | selection on genotype (at least in 1b+1a consensus): |
| | → 150 peptides |

Syfpeithi (B0702):

| output prediction server: | 3002 9-mers |
|---|---|

Table continued

Syfpeithi (B0702):

| | |
|---|---|
| | 3001 10-mers |
| Syfpeithi results: | paste 9-mers + 10-mers, sort on Syfpeithi score |
| | → select 250 peptides, 1 ranking 1-250 (126 9-mers + 124 10-mers) |
| | → Syfpeithi ranking for peptides with same score unknown |
| Syfpeithi selection: | selection on genotype (at least in 1b+1a consensus): |
| | → 113 peptides |

nHLAPred (B0702):

| | |
|---|---|
| output prediction server: | 200 9-mers |
| | no 10-mers |
| nHLAPred results: | → select 100 peptides, ranking 1-100 |
| | → nHLAPred ranking for peptides with same score unknown |
| nHLAPred selection: | selection on genotype (at least in 1b+1a consensus): |
| | → 45 peptides |

EPMN (B07):

| | |
|---|---|
| EPMN results: | 85 peptides (38 9-mers + 47 10-mers) with motif OK |
| | PIC between 0,17 and 633519; 64 with PIC =<100 |
| EPMN selection: | → selection on genotype:select 58 peptides, that are present in at least 1/32 |
| | 1b sequences EPMN used for predictions |
| EPMN 2nd selection: | selection on genotype (at least in 1b+1a consensus): |
| | → 28 peptides (16 with PIC =<100) |

**Table 16. Selected B07 predicted peptides**

| Protein | Peptide sequence | Number of pred. | Ki | Genotype | SEQ ID NO |
|---------|------------------|-----------------|------|----------|-----------|
| C | DPRRRSRNL | 4 | 18 | 1b/1a/3a | 370 |
| C | QPRGRRQPI | 4 | 1 | 1b/1a/3a | 390 |
| NS5A | RPDYNPPLL | 4 | 143 | 1b/1a/3a | 1677 |
| NS5B | SPGQRVEFL | 4 | 38 | 1b/1a | 373 |
| C | LPRRGPRLGV | 3 | 3 | 1b/1a/3a | 450 |
| NS3 | GPTPLLYRL | 3 | 209 | 1b/1a/3a | 307 |
| NS3 | KPTLHGPTPL | 3 | 6 | 1b/1a/3a | 1343 |
| NS4B | LPAILSPGAL | 3 | 255 | 1b/1a/3a | 1418 |
| C | LPGCSFSIFL | 3 | 558 | 1b/1a/3a | 1426 |
| NS4B | GPGEGAVQWM | 3 | 4747 | 1b/1a/3a | 1163 |
| NS5B | APTLWARMIL | 3 | 1 | 1b/1a | 853 |
| C | APLGGAARAL | 3 | 1 | 1b/1a | 836 |
| NS5B | DPPQPEYDL | 3 | high | 1b/1a | 543 |
| NS3 | HPNIEEVAL | 3 | 230 | 1b/1a | 1237 |
| P7 | WPLLLLLLAL | 3 | 1474 | 1b/1a | 2018 |
| NS5B | LPINALSNSL | 3 | 12 | 1b/1a | 1430 |

Table continued

| Protein | Peptide sequence | Number of pred. | Ki | Genotype | SEQ ID NO |
|---------|------------------|-----------------|------|-----------|-----------|
| NS3 | APPPSWDQM | 3 | 281 | 1b/1a | 381 |
| C | LPGCSFSIF | 3 | high | 1b/1a/3a | 375 |
| C | GPRLGVRAT | 2 | 128 | 1b/1a/3a | 387 |
| C | SPRGSRPSW | 2 | 11 | 1b/1a/3a | 386 |
| NS5A | LPCEPEPDV | 2 | high | 1b/1a/3a | 1421 |
| NS4B | LPGNPAIASL | 2 | 266 | 1b/1a | 1428 |
| NS3 | TPCTCGSSDL | 2 | 168 | 1b/1a | 1843 |
| NS3 | AAQGYKVLVL | 2 | 5524 | 1b/1a | 777 |
| NS5A | EPEPDVAVL | 2 | high | 1b/1a | 1024 |
| NS5B | APTLWARMI | 2 | 11 | 1b/1a | 371 |
| NS5A | PPVVHGCPL | 2 | 433 | 1b/1a | 1582 |
| E2 | LPALSTGLI | 2 | 233 | 1b/1a | 1419 |
| NS5B | PPQPEYDLEL | 2 | high | 1b/1a | 1575 |
| NS5A | EPDVAVLTSM | 2 | 454 | 1b/1a | 1023 |
| NS3 | IPTSGDVW | 2 | 3152 | 1b/1a | 415 |
| NS3 | RPSGMFDSSV | 2 | 14 | 1b/1a | 1687 |
| NS4B | SPGALVVGV | 2 | 627 | 1b/1a/3a | 1759 |
| NS5B | DPTTPLARA | 2 | 13058 | 1b/1a | 980 |
| NS4B | NPAIASLMA | 2 | 676 | 1b/1a | 1527 |
| NS3 | TPLLYRLGA | 2 | 74 | 1b/1a | 389 |
| NS5B | PPHSAKSKF | 2 | high | 1b/1a | 1568 |
| NS3 | NPSVAATLGF | 2 | 1197 | 1b/1a/3a | 1532 |
| NS4B | NPAIASLMAF | 2 | 121 | 1b/1a | 1528 |
| NS3 | LPVCQDHLEF | 2 | 1564 | 1b/1a | 1444 |

**Example 3: HLA Class I competition cell-based binding assays**

The interaction of the peptides with the binding groove of the HLA molecules is studied using competition-based cellular peptide binding assays as described by Kessler et al. (2003). Briefly, Epstein-Barr virus (EBV)-transformed B cell lines (B-LCLs) expressing the class I allele of interest are used. EBV transformation is done according to standard procedures (Current Protocols in Immunology, 1991, Wiley Interscience). Naturally bound class I peptide are eluted from the B-LCLs by acid-treatment to obtain free class I molecules. Subsequently, B-LCLs are incubated with a mixture of fluorescein (Fl)-labelled reference peptide, and titrating amounts of the competing test peptide. The reference peptide should have a known, high affinity for the HLA-molecule. Cell-bound fluorescence is determined by flow cytometry. The inhibition of binding of the Fl-labelled reference peptide is determined and IC50-values are calculated (IC50 = concentration of competing peptide that is able to occupy 50% of the HLA molecules). The affinity (Kd) of the reference peptide is determined in a separate experiment in which the direct binding of different concentrations of reference peptide is monitored and data are analysed using a model for one-site binding interactions. The inhibition constant ($K_i$) of the competing peptides (reflecting their affinity) is calculated as:

EP 1 652 858 A1

$$K_i = \frac{IC50}{1 + [Fl\text{-}pep]/Kd}$$

[Fl-pep]: concentration of the Fl-labeled peptide used in the competition experiment.

The predicted peptides were synthesized using standard technology and tested for binding to B-LCLs with the corresponding HLA-allele. Fl-labelled reference peptides are synthesized as Cys-derivatives and labelling is performed with 5-(iodoacetamido) fluorescein at pH 8,3 (50 mM Bicarbonate/ 1 mM EDTA buffer). The labelled peptides were desalted and purified by C18 RP-HPLC. Labelled peptides were analysed by mass spectrometry.

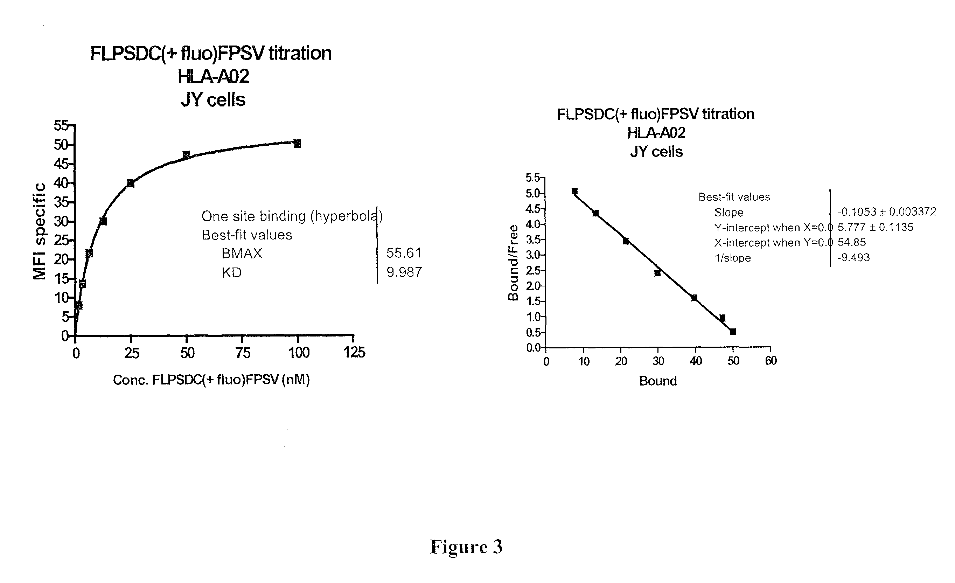

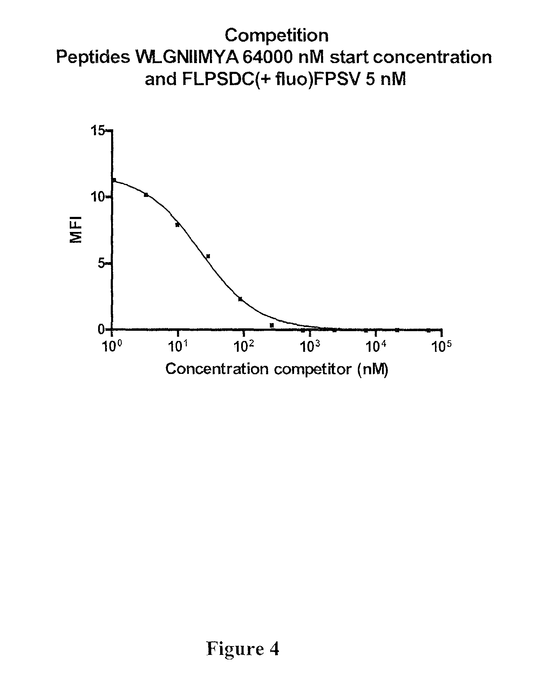

As an example, the interaction of a predicted strong binding peptide with HLA-A02 is shown. An HLA-A02 positive B-LCL (JY, Kessler et al., 2003) is used for analysing the competition of the Fl-labelled reference peptide FLPSDC (Fl)FPSV and the predicted peptides (SEQ ID NO 62 to SEQ ID NO 93). The binding of the reference peptide to HLA A02 is shown in figure 3. Analysing the data according to a one-site binding model reveals an affinity of the reference peptide of about 10 nM. A typical competition experiment is shown in Figure 4. Table 2 contains the calculated inhibition constants (Ki).

**Example 4: HLA Class I and II competition binding assays using soluble HLA**

The following example of peptide binding to soluble HLA molecules demonstrates quantification of binding affinities of HLA class I and class II peptides.

Epstein-Barr virus (EBV)-transformed homozygous cell lines, fibroblasts or transfectants were used as sources of HLA class I molecules. Cell lysates were prepared and HLA molecules purified in accordance with disclosed protocols (Sidney et al., 1998; Sidney et al., 1995; Sette, et al., 1994).

HLA molecules were purified from lysates by affinity chromatography. The lysate was passed over a column of Sepharose CL-4B beads coupled to an appropriate antibody.

The antibodies used for the extraction of HLA from cell lysates are W6/32 (for HLA-A, -B and -C), B123.2 (for HLA-B and -C) and LB3.1 (for HLA-DR).

The anti-HLA column was then washed with 10mM Tris-HCL, pH8, in 1% NP-40, PBS, and PBS containing 0,4% n-octylglucoside and HLA molecules were eluted with 50mM diethylamine in 0,15M NaCl containing 0,4% n-octylglucoside, pH 11,5. A 1/25 volume of 2M Tris, pH6,8, was added to the eluate to reduce the pH to +/- pH8. Eluates were then concentrated by centrifugation in Centriprep 30 concentrators (Amicon, Beverly, MA). Protein content was evaluated by a BCA protein assay (Pierce Chemical Co., Rockford, IL) and confirmed by SDS-PAGE.

A detailed description of the protocol utilized to measure the binding of peptides to Class I and Class II MHC has been published (Sette et al., 1994; Sidney et al., 1998). Briefly, purified MHC molecules (5 to 500nM) were incubated with various unlabeled peptide inhibitors and 1-10nM $^{125}$I-radiolabeled probe peptides for 48h in PBS containing 0,05% Nonidet P-40 (NP40) in the presence of a protease inhibitor cocktail. All assays were at pH7 with the exception of DRB1*0301, which was performed at pH 4,5, and DRB1*1601 (DR2w21 1) and DRB4*0101 (DRw53), which were performed at pH5.

Following incubation, MHC-peptide complexes were separated from free peptide by gel filtration on 7,8 mm x 15 cm TSK200 columns (TosoHaas 16215, Montgomeryville, PA). The eluate from the TSK columns was passed through a Beckman 170 radioisotope detector, and radioactivity was plotted and integrated using a Hewlett-Packard 3396A integrator, and the fraction of peptide bound was determined. Alternatively, MHC-peptide complexes were separated from free peptide by capturing onto ELISA plates coated with anti-HLA antibodies. After free peptide has been washed away, remaining reactivities were measured using the same method as above.

Radiolabeled peptides were iodinated using the chloramine-T method.

Typically, in preliminary experiments, each MHC preparation was titered in the presence of fixed amounts of radiolabeled peptides to determine the concentration of HLA molecules necessary to bind 10-20% of the total radioactivity. All subsequent inhibition and direct binding assays were performed using these HLA concentrations.

Since under these conditions [label] < [HLA] and IC50 $\geq$ [HLA], the measured IC50 values are reasonable approximations of the true KD values. Peptide inhibitors are typically tested at concentrations ranging from 120 $\mu$g/ml to 1,2 ng/ml, and are tested in two to four completely independent experiments. To allow comparison of the data obtained in different experiments, a relative binding figure is calculated for each peptide by dividing the IC5O of a positive control for inhibition by the IC50 for each tested peptide (typically unlabeled versions of the radiolabeled probe peptide). For database purposes, and inter-experiment comparisons, relative binding values are compiled. These values can subse-

127

quently be converted back into IC50 nM values by dividing the IC50 nM of the positive controls for inhibition by the relative binding of the peptide of interest. This method of data compilation has proven to be the most accurate and consistent for comparing peptides that have been tested on different days, or with different lots of purified MHC.

Because the antibody used for HLA-DR purification (LB3.1) is alpha-chain specific, beta-1 molecules are not separated from beta-3 (and/or beta-4 and beta-5) molecules. The beta-1 specificity of the binding assay is obvious in the cases ofDRB1*0101 (DR1), DRB1*0802 (DR8w2), and DRB 1*0803 (DR8w3), where no beta-3 is expressed. It has also been demonstrated for DRB1*0301 (DR3) and DRB3*0101 (DR52a), DRB1*0401 (DR4w4), DRB1*0404 (DR4w14), DRB1*0405 (DR4w15), DRB1*1101 (DR5), DRB1*1201 (DR5w12), DRB1*1302 (DR6w19) and DRB1*0701 (DR7). The problem of beta chain specificity for DRB1*1501 (DR2w2beta-1), DRB5*0101 (DR2w2beta-2), DRB1*1601 (DR2w21beta-1), DRB5*0201 (DR51Dw21), and DRB4*0101 (DRw53) assays is circumvented by the use of fibroblasts. Development and validation of assays with regard to DRbeta molecule specificity have been described previously (see, e. g., Southwood et al., 1998).

### Example 5: Use of peptides to evaluate human recall responses for CD8 epitopes

The peptide epitopes of the invention are used as reagents to evaluate T cell responses, such as acute or recall responses, in patients. Such an analysis may be performed on patients who have recovered from infection, who are chronically infected with HCV, or who have been vaccinated with an HCV vaccine.

For example, PBMC are collected from patients recovered from infection and HLA typed. Appropriate peptide epitopes of the invention that are preferably binding with strong or intermediate affinity (more preferably below the threshold affinity) are then used for analysis of samples derived from patients who bear that HLA type.

PBMC from these patients are separated on density gradients and plated. PBMC are stimulated with peptide on different time points. Subsequently, the cultures are tested for cytotoxic activity.

Cytotoxicity assays are performed in the following manner. Target cells (either autologous or allogeneic EBV-transformed B-LCL that are established from human volunteers or patients; Current Protocols in Immunology, 1991) are incubated overnight with the synthetic peptide epitope, and labelled with $^{51}$Cr (Amersham Corp., Arlington Heights, IL) after which they are washed and radioactivity is counted. Percent cytotoxicity is determined from the formula:

$$100 \times [(\text{experimental release-spontaneous release})/\text{maximum release-spontaneous release})].$$

Maximum release is determined by lysis of targets.

The results of such an analysis indicate the extent to which HLA-restricted CTL populations have been stimulated by previous exposure to HCV or an HCV vaccine.

The class II restricted HTL responses may also be analysed in a comparable way.

### Example 6: Activity of CTL and/or HTL epitopes in Transgenic Mice

This example illustrates the induction of CTLs and/or HTLs in transgenic mice by use of one ore more HCV CTL and/or HTL eitopes. The epitope composition can comprise any combination of CTL and/or HTL epitopes as described in the current invention, and more specific as given in Tables 13 and 14.

For this specific example, the experiment is performed to evaluate the immunogenicity of the peptides with Ki < 1000 nM disclosed in Table 13, section B07 and B35.

The HLA-B7 restricted CTL response induced by peptides which bind to B7 or B35 emulsified in IFA in HLA-B7 Tg mice (F1, crossed with Balb/c) is evaluated. As a comparison, a group of naïve mice were included. The magnitude of CTL responses to the HLA-B7 and -B35 restricted epitopes in immunized HLA-B7/K$^{b}$ transgenic mice are compared to the response in naive animals.

Experimental set-up

HLA-B7/K$^{b}$ transgenic mice (BALB/c x HLA-B7/K$^{b}$.C57BL/6 F1 mice; H2$^{bxd}$), both male and female, were utilized. Mice were used between 8 and 14 weeks of age. Each group consisted of 3 mice and the naive group consisted of 4 mice. Each group was repeated in two independent experiments.

The immunization and testing scheme is shown in Table 17. In general, HLA-B7/K$^{b}$ mice were immunized with a pool of B7-restricted CTL peptides emulsified in Incomplete Freund's Adjuvant (IFA). Nine peptide pools, each consisting of 4 to 6 CTL peptides, of similar binding affinity at a dose of 25 μg/peptide and 120 μg of the HTL epitope, HBV Core 128 (TPPAYRPPNAPIL) (known HTL epitope in these animals), were tested. Each experiment tested three of the pools, and each pool was tested in two independent experiments. Naïve animals (non-immunized HLA-B7/K^b transgenic mice) were included in each experiment as a control group. The mice were immunized with 100 μl of the emulsion subcutaneously at the base of the tail. Eleven to 14 days after immunization, the mice were euthanized, and the spleens were removed.

## Table 17. Immunization and testing schedule for peptide immunogenicity experiments using experiment 6 as an example.

|  | In vivo | | In vitro |
| --- | --- | --- | --- |
|  | 11-14 days | | |
| Group | Week -2 | Week -1 | ELISPOT Assay |
| 1 | Peptide Pool | | |
| 2 | Peptide Pool | | |
| 3 | Peptide Pool | | |
| 4 | Naïve | | |

Spleens were disrupted with a 15-ml tissue grinder and the resulting single cell suspension was treated with DNAse solution (10 μl/spleen of 30 mg/ml DNAse in PBS), washed in RPMI-1640 with 2% FCS, and counted. Splenocytes were then incubated at 4° C for 15-20 minutes in 300μl MACS buffer (PBS with 0.5% BSA and 2mM EDTA) with 35μl of MACS CD8a(Ly-2) Microbeads/10^8 cells according to the manufacturer's specifications. The cells were then applied to a MACS column (Milltenyi) and washed four times. The cells were removed from the column in culture medium consisting of RFMI 1640 medium with HEPES (Gibco Life Technologies) supplemented with 10% FBS, 4 mM L-glutamine, 50 μ M 2-ME, 0,5 mM sodium pyruvate, 100 μg/ml streptomycin and 100 U/ml penicillin. (RPMI-10), washed, and counted again.

The responses to CTL epitopes were evaluated using an IFN-γ ELISPOT assay. Briefly, IP membrane-based 96-well plates (Millipore, Bedford MA) were coated overnight at 4°C with α-mouse IFN-γ monoclonal antibody (Mabtech MabAN18) at a concentration of 10μg/ml in PBS. After washing 3 times with PBS, RPMI-10 was added to each well, and the plates were incubated at 37°C for 1 hour to block the plates. The purified CD8+ cells were applied to the blocked membrane plates at a cell concentration of 4 x 10^5 cells/well.

The peptides were dissolved in RPMI-10 (final peptide concentration 10μg/ml), and mixed with target cells (10^5 HLA-B7/K^b transfected Jurkat cells/well). Controls of media only and Con A (10μg/ml) were also utilized. The target cell/peptide mixture was layered over the effector cells in the membrane plates, which were incubated for 20 hours at 37°C with 5% CO_2.

Media and cells were then washed off the ELISPOT plates with PBS + 0,05% Tween-20, and the plates were incubated with α-mouse biotinylated α-IFN-γ antibody (Mabtech MabR4-6A2-Biotin) at a final concentration of 1 μg/ml for 4 hours at 37°C. After washing, the plates were incubated with Avidin-Peroxidase Complex (Vectastain), prepared according to the manufacturer's instructions, and incubated at room temperature for 1 hour. Finally, the plates were developed with AEC (1 tablet 3-Amino-9-ethylcarbazole dissolved in 2,5 ml dimethylformamid, and adjusted to 50 ml with acetate buffer; 25μl of 30% H_2O_2 was added to the AEC solution), washed, and dried. Spots were counted using AID plate reader.

Data-analysis

Each peptide was tested for recognition in both the immunized group and the naive group. Data was collected in triplicate for each experimental condition.

The raw data for the media control were averaged for each group (both naive and immunized). Net spots were calculated by subtracting the average media control for each group from the raw data points within the group. The average and standard error were then calculated for each peptide, and the average and standard error were normalized to 10^6 cells (by multiplying by a factor of 2,5). Finally, a type 1, one-tailed T test was performed to compare the data from immunized groups to that from naive controls. Data was considered significantly different the naive controls if p≤0,1. The data are reported as the number of peptide-specific IFNγ producing cells/10^6 CD8+ cells.

Data from two replicate experiments are compared. Peptides with discordant data (i.e. positive in one experiment and negative in the other) are repeated in a third experiment. The data from two or more experiments may be averaged as described above.

Peptide immunogenicity results for B7 and B35-restricted peptides.

The data are shown in Tables 18 (B7) and 19 (B35), and represent responses in 2-4 independent experiments. Twenty-six peptides showed a positive response when compared with the response in naïve mice (p≤0,1).

Ten of the peptides that were tested bound both B7 and B35 (6 peptides) or B35 only (4 peptides). Of the 6 peptides that bound both B7 and B35, four were immunogenic in the HLA-B7/K$^b$ transgenic mice (Table 2). The 4 peptides that bound B35 only were all negative in the B7 transgenic mice.

**Table 18. Immunogenicity data for HCV-derived peptides restricted to HLA-B7.** The peptides are sorted by peptide position, and the data are reported in IFN-γ SFC/$10^6$ CD8$^+$ splenocytes. Responses that are significant (p≤0,1) are bolded.

| SEQ ID NO I | Sequence | | Length | nM IC$_{50}$ B*0702 | B*3501 | # alleles bound | Immunized SFC/$10^6$ ± St Error | Naïve SFC/$10^6$ ± St Error | Ttest | # of Exp. |
|---|---|---|---|---|---|---|---|---|---|---|
| 1629.02 | LPRRGPRLG | HCV.037 | 9 | 124 | | 1 | 138.1 ± 25.1 | 8.1 ± 5.9 | 0.00 | 4 |
| 1508.23 | LPRRGPRLGV | HCV.0037 | 10 | 2.6 | -- | 2 | 499.2 ± 28.5 | 11.3 ± 1.6 | 0.00 | 2 |
| 1259.01 | GPRLGVRAT | HCV.0041 | 9 | 128 | - | 1 | 161.3 ± 71.9 | 8.8 ± 7.5 | 0.03 | 2 |
| 1508.19 | QPRGRRQPI | HCV.0057 | 9 | 1.2 | -- | 1 | 96.7 ± 20.3 | 2.1 ± 1.2 | 0.00 | 2 |
| 1508.21 | SPRGSRPSW | HCV.0099 | 9 | 11 | -- | 1 | 266.3 ± 9.5 | 2.9 ± 1.3 | 0.00 | 2 |
| 1508.22 | DPRRRSRNL | HCV.0111 | 9 | 18 | -- | 1 | 16.5 ± 8.1 | 3.5 ± 7.9 | 0.10 | 4 |
| 1468.03 | IPLVGAPL | HCV.0137 | 8 | 25 | 295 | 3 | 206.7 ± 39.1 | 2.1 ± 2.6 | 0.00 | 2 |
| 1629.03 | APLGGAARA | HCV.0142 | 9 | 115 | -- | 2 | 10.4 ± 4.5 | 2.9 ± 2.8 | 0.11 | 2 |
| 1508.24 | APLGGAARAL | HCV.0142 | 10 | 0.80 | 1048 | 1 | 116.3 ± 26.4 | 4.2 ± 2.8 | 0.00 | 2 |
| 1145.12 | LPGCSFSIF | HCV.0169 | 9 | 29 | 90 | 3 | 15.4 ± 5.0 | 2.1 ± 0.8 | 0.02 | 2 |
| 1468.01 | LPALSTGLI | HCV.0682 | 9 | 233 - | -- | 1 | 82.9 ± 21.3 | 3.3 ± 3.5 | 0.00 | 2 |
| 1629.06 | TPCTCGSSDL | HCV.1121 | 10 | 168 | 7976 | 1 | 7.5 ± 7.4 | 7.9 ± 5.4 | 0.46 | 4 |
| 1629.07 | APTGSGKST | HCV.1230 | 9 | 370 | -- | 1 | 4.6 ± 2.6 | 9.2 ± 6.0 | 0.20 | 2 |
| 1629.08 | YAAOGYKVL | HCV.1244 | 9 | 313 | 5836 | 1 | 68.3 ± 29.1 | 1.3 ± 5.7 | 0.03 | 4 |
| 1629.11 | HPNIEEVAL | HCV.1359 | 9 | 230 | 7.4 | 2 | 17.5 ± 5.2 | 3.3 ± 4.2 | 0.01 | 2 |
| 1629.12 | AAKLSALGL | HCV.1404 | 9 | 277 | -- | 1 | 15.0 ± 4.0 | 0.4 ± 3.1 | 0.00 | 2 |
| 1629.16 | TPGERPSGM | HCV.1503 | 9 | 199 | -- | 1 | 45.0 ± 22.0 | 7.5 ± 5.7 | 0.06 | 4 |
| 1629.17 | RPSGMFDSSV | HCV.1507 | 10 | 14 | -- | 1 | 104.2 ± 27.7 | 1.7 ± 7.5 | 0.00 | 4 |
| 1629.18 | TPAETSVRL | HCV.1531 | 9 | 375 | 1643 | 1 | 10.8 ± 6.7 | 7.5 ± 4.4 | 0.17 | 2 |
| 1629.20 | APPPSWDOM | HCV.1599 | 9 | 281 | 17,771 | 1 | 489.6 ± 15.8 | 4.6 ± 3.3 | 0.00 | 2 |
| 1629.21 | KPTLHGPTPL | HCV.1615 | 10 | 5.8 | 14,102 | 1 | 291.3 ± 67.4 | 7.1 ± 3.4 | 0.00 | 2 |
| 1629.22 | GPTPLLYRL | HCV.1620 | 9 | 209 | 17.916 | 1 | 59.6 ± 6.3 | 7.1 ± 5.9 | 0.00 | 2 |
| 1629.23 | TPLLYRLGA | HCV.1622 | 9 | 74 | -- | 2 | 1.5 ± 6.9 | 6.9 ± 7.8 | 0.19 | 4 |
| 1629.25 | LPGNPAIASL | HCV.1781 | 10 | 266 | 3539 | 1 | 17.1 ± 4.4 | 9.2 ± 6.1 | 0.22 | 2 |
| 1629.27 | NPAIASLMAF | HCV.1784 | 10 | 121 | 312 | 2 | 5.4 ± 1.7 | 4.6 ± 3.0 | 0.34 | 2 |
| 1629.28 | LPAILSPGAL | HCV.1883 | 10 | 255 | 550 | 1 | 14.6 ± 3.9 | 3.3 ± 4.1 | 0.04 | 2 |
| 1629.34 | EPDVAVLTSM | HCV.2165 | 10 | 454 | 150 | 2 | 8.8 ± 3.6 | 6.3 ± 5.7 | 0.32 | 2 |
| 1629.35 | RPDYNPPLL | HCV.2290 | 9 | 143 | -- | 1 | 163.3 ± 47.2 | 6.7 ± 7.3 | 0.01 | 2 |
| 1292.17 | PPWHGCPL | HCV.2308 | 9 | 433 | -- | 1 | 30.8 ± 9.6 | 7.9 ± 5.9 | 0.07 | 2 |
| 1629.36 | LPINALSNSL | HCV.2440 | 10 | 12 | 137 | 3 | 223.8 ± 50.3 | 1.7 ± 1.8 | 0.00 | 2 |

| SEQ ID NO I | Sequence | | Length | nM IC$_{50}$ | | # alleles bound | Immunized | Naïve | Ttest | # of Exp. |
|---|---|---|---|---|---|---|---|---|---|---|
| | | | | B*0702 | B*3501 | | SFC/10$^6$ ± St Error | SFC/10$^6$ ± St Error | | |
| 1629.38 | SPGQRVEFL | HCV.2615 | 9 | 38 | -- | 1 | 12.7 ± 8.3 | 11.5 ± 6.8 | 0.43 | 4 |
| 1629.40 | SAACRAAKL | HCV.2719 | 9 | 106 - | -- | 1 | 286.3 ± 32.4 | 8.8 ± 4.4 | 0.00 | 2 |
| 1629.44 | APTLWARMI | HCV.2835 | 9 | 11 | -- | 1 | 302.1 ± 48.8 | 25.0 ± 5.4 | 0.00 | 4 |
| 1629.45 | APTLWARMIL | HCV.2835 | 10 | 1.2 | -- | 1 | 859.2 ± 25.5 | 5.0 ± 3.8 | 0.00 | 2 |

Table continued

**Table 19. Immunogenicity data for HCV-derived peptides restricted to HLA-B35.** The peptides are sorted by peptide position, and the data are reported in IFN-$\gamma$ SFC/$10^6$ CD8$^+$ splenocytes. Responses that are significant (p$\leq$0,1) are bolded.

| Epimmune ID | Sequence | Position | Length | nM IC$_{50}$ B*0702 | nM IC$_{50}$ B*3501 | # alleles bound | Immunized St SFC/$10^6$ $\pm$ St Error | Naïve St SFC/$10^6$ $\pm$ St Erro | Ttest | # of # of Exp. |
|---|---|---|---|---|---|---|---|---|---|---|
| 1468.03 | IPLVGAPL | HCV.0137 | 8 | 25 | 295 | 3 | 206.7 $\pm$ 39.1 | 2.1 $\pm$ 2.6 | 0.00 | 2 |
| 1145.12 | LPGCSFSIF | HCV.0169 | 9 | 29 | 90 | 3 | 15.4 $\pm$ 5.0 | 2.1 $\pm$ 0.8 | 0.02 | 2 |
| 1629.11 | HPNIEEVAL | HCV.1359 | 9 | 230 | 7.4 | 2 | 17.5 $\pm$ 5.2 | 3.3 $\pm$ 4.2 | 0.01 | 2 |
| 1629.15 | IPTSGDVVV | HCV.1426 | 9 | 3152 | 380 | 2 | 7.5 $\pm$ 3.8 | 14.2 $\pm$ 3.6 | 0.18 | 2 |
| 1629.19 | LPVCQDHLEF | HCV.1548 | 10 | 1564 | 104 | 2 | 13.3 $\pm$ 5.2 | 2.5 $\pm$ 3.1 | 0.08 | 2 |
| 1359.04 | FPYLVAYQA | HCV.1583 | 9 | 1336 | 18 | 4 | -0.8 $\pm$ 4.9 | 4.6 $\pm$ 3.9 | 0.20 | 2 |
| 1629.27 | NPAIASLMAF | HCV.1784 | 10 | 121 | 312 | 2 | 5.4 $\pm$ 1.7 | 4.6 $\pm$ 3.0 | 0.34 | 2 |
| 1629.33 | EPEPDVAVL | HCV.2163 | 9 | - | 194 | 1 | 8.3 $\pm$ 4.9 | 8.8 $\pm$ 6.3 | 0.47 | 2 |
| 1629.34 | EPDVAVLTSM | HCV.2165 | 10 | 454 | 150 | 2 | 8.8 $\pm$ 3.6 | 6.3 $\pm$ 5.7 | 0.32 | 2 |
| 1629.36 | LPINALSNSL | HCV.2440 | 10 | 12 | 137 | 3 | 223.8 $\pm$ 50.3 | 1.7 $\pm$ 1.8 | 0.00 | 2 |

**REFERENCES**

Alexander, J. et al., J Immunol. 159:4753, 1997

Altman et al., Science 174:94-96, 1996

An, L. and Whitton, J. L., J Virol. 71:2292,1997

Arndt et al., Immuno.1 Res., 16: 261-72, 1997

Barton,G.M. & Medzhitov,R. Toll-like receptors and their ligands. Curr. Top. Microbiol. Immunol. 270, 81-92, 2002

Bertoni, R. et al., J Clin. Invest. 100:503, 1997

Beaucage & Caruthers, Tetrahedron Letts. 22:1859- 1862, 1981

Blum et al., Grit. Rev. Imnunol., 17: 411-17, 1997

Brooks et al., J Immunol, 161: 5252-5259, 1998

Brown. J. et al., (1993) Nature 364, 33-39

Busch et al., Int. Immunol. 2:443, 1990

Buus et al., Science 242: 1065, 1988

Byl,B. *et al.* OM197-MP-AC induces the maturation of human dendritic cells and promotes a primary T cell response. Int Immunopharmacol. 3, 417-425, 2003

Celis, E. et al., Proc. Natl. Acad Sci. USA 91:2105, 1994

Ceppellini et al., Nature 339:392, 1989

Cerundolo et al., J 1mmunol. 21:2069, 1991

Christnick et al., Nature 352:67, 1991

Collins et al., J. Immunol. 148:3336-3341, 1992

del Guercio et al., J Immunol. 154:685, 1995

Diepolder, H. M. et al., J Virol. 71:6011, 1997

Donnelly JJ, Ulmer JB, Shiver JW, Liu MA. DNA vaccines. Annu Rev Immunol. 1997;15:617-48.

Donnelly JJ, Ulmer JB, Liu MA. DNA vaccines. Life Sci. 1997a;60(3):163-72.

Doolan, D. L. et al., Immunity 7:97, 1997

Falk, K. et al.,(1991) Nature 351, 290-296)

Felgner, et al., Proc. Nat'l Acad. Sci. USA 84:7413, 1987

Fries et al., Proc. Natl. Acad. Sci. (USA) 89:358, 1992

Grakoui,A., Wychowski,C., Lin,C., Feinstone,S.M. & Rice,C.M. Expression and identification of hepatitis C virus polyprotein cleavage products. J. Virol. 67, 1385-1395, 1993

Gruters RA, van Baalen CA, Osterhaus AD.Vaccine. 2002 May 6;20(15):2011-5. The advantage of early recognition of HIV-infected cells by cytotoxic T-lymphocytes.

Hammer et al., J Exp. Med. 180:2353, 1994

Hanke, R. et al., Vaccine 16:426, 1998

Henderson et al, Science 255: 1264, 1992

Hill et al., J 1mmunol. 147:189, 1991

Hill et al., J Immunol. 152, 2890, 1994

Hoffmann et al., Hepatology, 21(3):632-8, 1995

Hunt, et al., Science 225: 1261, 1992

Houssaint E, Saulquin X, Scotet E, Bonneville M Biomed Pharmacother. 2001 Sep; 55(7): 373-80. Immunodominant CD8 T cell response to Epstein -Barr virus.

Ishioka, et al., J. Immunol. (1999) 162(7):3915-3925

Jardetzky, et al., Nature 353: 326, 1991

Johnson,D.A. *et al.* Synthesis and biological evaluation of a new class of vaccine adjuvants: aminoalkyl glucosaminide 4-phosphates (AGPs). Bioorg. Med. Chem Lett 9, 2273-2278, 1999

Kessler et al. Human Immunol, 64: 245-255, 2003

Khilko et al., J Biol. Chem. 268:15425, 1993

Kawashima, 1. et al., Human Immunol. 59:1, 1998

Knauf MJ. et al, J Biol Chem. Oct 15;263(29):15064-70, 1988

Lamonaca et al., Hepatology, 30:1088-1098

Latron, F. et al., (1992) Science 257, 964-967

Lim, J.S. et al. (1996) Mol. Immunol. 33, 221-230

Lukacher AE, Braciale VL, Braciale TJ. J Exp Med. 1984 Sep 1;160(3):814-26. In vivo effector function of influenza virus-specific cytotoxic T lymphocyte clones is highly specific. Ljunggren et al., Nature 346:476, 1990

Lauer,G.M. & Walker,B.D. Hepatitis C virus infection. N. Engl J. Med. 345, 41-52, 2001 Madden, D.R. et al., (1992) Cell 70, 1035-1048

Mannino & Gould- Fogerite, BioTechniques 6(7): 682, 1988

Marshall et al., J 1mmunol. 152:4946, 1994

Matsumura, M. et al., (1992) Science 257, 927-934

Michelletti et al., Immunology, 96: 411-415, 1999

Murray N, McMichael A. Curr Opin Immunol. 1992 Aug;4(4):401-7. Antigen presentation in virus infection.

Nabel et al., Proc. Natl. Acad. Sci. (USA) 89:5157, 1992

Niedermann et al., Immunity, 2: 289-99, 1995

Ogg et al., Science 279:2103-2106, 1998

Parker et al., J Immunol. 149:1896, 1992

Pearson & Reanier, J. Chrom. 255:137-149, 1983

Perma et al., J Exp. Med. 174:1565-1570, 1991

Persing,D. et al. Taking toll: lipid A mimetics as adjuvants and immunomodulators. Trends Microbiol. 10, S32, 2002

Reay et al., EMBO J 11:2829, 1992

Rehermann, B. et al., J Exp. Med. 181:1047, 1995

Reddy et al., J. Immunol. 148:1585, 1992

Riedl,P., Buschle,M., Reimann,J. & Schirmbeck,R. Binding immune-stimulating oligonucleotides to cationic peptides from viral core antigen enhances their potency as adjuvants. Eur. J. Immunol. 32, 1709-1716, 2002

Rognan, D. et al., (1999) J. Med. Chem. 42, 30 4650-4658

Rosenberg et al (1997), Science 278:1447-1450

Rotzschke and Falk, Immunol. Today 12: 447, 1991

Saper, M.A. et al., (1991) J. Mol. Biol. 219, 277-312

Schaeffer et al. Proc. Natl. Acad. Sci. USA 86:4649-4653, 1989

Schumacher et al., Cell 62:563, 1990

Sercarz, et al., Annu. Rev. Immunol 11: 729-766, 1993

Sette, et al J Immunol 153:5586-5592, 1994

Sette, et al., Mol. Immunol. 31: 813 (1994).

Sette and Sidney (1990), Immunogenetics 50: 201-212

Sidney et al., Current Protocols in Immunology, Ed., John Wiley & Sons, NY, Section 18.3 (1998)

Sidney, et al., J Immunol. 154: 247 (1995)

Shiffman,M.L. Improvement in liver histopathology associated with interferon therapy in patients with chronic hepatitis C. Viral Hepatitis Reviews 5, 27-43, 1999

Shimotohno,K. et al. Processing of the hepatitis C virus precursor protein. J. Hepatol. 22, 87-92, 1995

Southwood et al. J Immunology 160:3363-3373,1998

Stover et al., Nature 351:456- 460, 1991

Szoka, et aL, Ann. Rev. Biophys. Bioeng. 9:467,1980

Threlkeld, S. C. et al., J Immunol. 159:1648, 1997

Tigges MA, Koelle D, Hartog K, Sekulovich RE, Corey L, Burke RL J Virol. 1992 Mar;66(3):1622-34. Human CD8+ herpes simplex virus-specific cytotoxic T-lymphocyte clones recognize diverse virion protein antigens.

Thomson, S. A. et al., J Immunol. 157:822, 1996

Townsend et al., Cell 62:285, 1990

Tsai SL, Huang SN. J Gastroenterol Hepatol. 1997 Oct;12(9-10):S227-35. T cell mechanisms in the immunopathogenesis of viral hepatitis B and C.

Tsai, V. et al., J Immunol. 158:1796, 1997

van der Burg et al., J Immunol, 156: 3308-3314, 1996

Van Devanter et. al., Nucleic Acids Res. 12:6159-616S, 1984

Velders MP et al. J Immunol, 166(9): 5366-73, 2001

Yewdell et al., Aurz. Rev. Immunol., 17: 51-88, 1997

Walewski,J.L., Keller,T.R., Stump,D.D. & Branch,A.D. Evidence for a new hepatitis C virus antigen encoded in an overlapping reading frame. RNA. 7, 710-721, 2001

Wentworth, P. A. et al., Mol. Immunol. 32:603, 1995

Wentworth, P. A. et al. , J Immunol. 26:97, 1996

Wentworth, P. A. et al., Int. Immunol. 8:651, 1996

Wertheimer AM et al., Hepatology, 37: 577-589

Whitton, J. L. et al., J Prol. 67:348, 1993

Xu,Z. et al. Synthesis of a novel hepatitis C virus protein by ribosomal frameshift. EMBO J. 20, 3840-3848, 2001

**Claims**

1. An isolated polyepitopic peptide comprising at least three peptides selected from the HLA-B and/or HLA-C binding peptides as disclosed in Table 13.

2. The polyepitopic peptide according to claim 1 further comprising at least one HLA-A or HLA-DRB binding peptide selected from Tables 13 and 14.

3. The polyepitopic peptide according to claims 1 or 2 further **characterized in that** said at least three peptides are present in the HCV consensus sequence of genotype 1a, 1b and/or 3a.

4. The polyepitopic peptide according to any of claims 1 to 3 wherein at least one of said peptides is **characterized in that** it has cross-binding activity for HLA molecules derived from different HLA groups or loci or that it is a nested peptide sequence.

5. The polyepitopic peptide according any of claims 1 to 4 wherein the HLA-B binding peptide is **characterized in that** it binds a HLA molecule, said molecule being selected from the HLA-B group HLA-B07, -B08, -B35, -B40 or -B44.

6. The polyepitopic peptide according to any of claims 1 to 5 wherein the HLA-C binding peptide is **characterized in that** it binds a HLA molecule, said molecule being selected from the HLA-C group HLA-Cw3, Cw4, Cw6 or Cw7.

7. The polyepitopic peptide according to any of claims 2 to 6 wherein the HLA-A binding peptide is **characterized in that** it binds a HLA molecule, said molecule being selected from the HLA-A group HLA-A01, -A02, -A03, -A11 or -A24.

8. An isolated polyepitopic peptide comprising at least two peptides derived from a HCV protein and capable of inducing a HLA class I and/or class II restricted T lymphocyte response, wherein at least one peptide is a HLA-C binding peptide.

9. The polyepitopic peptide according to claim 8 wherein the at least two peptides are selected from different HLA-loci.

10. The polyepitopic peptide according to claim 9 wherein the HLA-loci are HLA-A, HLA-B, HLA-C or HLA-DRB1-9.

11. The polyepitopic peptide according to any of claims 8 to 10 wherein the peptides are selected from Tables 13 and/or 14.

12. The polyepitopic peptide according to any of claims 8 to 11 wherein the at least two peptides are selected from a different HLA-group within HLA-B and/or HLA-C.

13. The polyepitopic peptide according to any of claims 8 to 12 further **characterized in that** said at least two peptides are present in the HCV consensus sequence of genotype 1a, 1b and/or 3 a.

14. An isolated peptide consisting of an amino acid sequence selected from the group consisting of the sequences listed in Tables 13 and 14.

15. The peptide according to claim 14 comprised in an immunogenic peptide of less than 50 amino acid residues.

16. The peptide according to claims 14 or 15, wherein said peptide is capable of inducing a HLA class I and/or class II restricted T lymphocyte response.

17. An isolated peptide consisting of an amino acid sequence which is at least 70% identical to the amino acid sequence of the peptide according to claim 14, said peptide being capable of inducing a HLA class I and/or class II restricted T lymphocyte response.

18. A polyepitopic peptide comprising at least one peptide according to any of claims 14 to 17.

19. A polyepitopic peptide comprising at least two peptides according to any of claims 14 to 17.

20. A polyepitopic peptide comprising at least three peptides according to any of claims 14 to 17.

21. The polyepitopic peptide according to claim 20 wherein the at least three peptides are at least two HLA-B binding peptides in combination with at least one HLA-A binding peptide or at least one HLA-C binding peptide.

22. The polyepitopic peptide according to claim 21 wherein the at least two HLA-B binding peptides are selected from a different HLA-group within the HLA-B locus.

23. The polyepitopic peptide according to claim 20 comprising at least one HLA-A binding peptide, at least one HLA-B binding peptide and at least one HLA-C binding peptide.

24. A polyepitopic peptide according to any of claims 19 to 23, wherein said at least two or three peptides are **characterized in that** they are present in the HCV consensus sequence of genotype 1a, 1b and/or 3 a.

25. The polyepitopic peptide according to any of claims 1 to 13 and 18 to 24 further comprising a HTL epitope.

26. The polyepitopic peptide according to claim 25 wherein the HTL epitope is selected from Table 14.

27. The polyepitopic peptide according to claim 25, wherein the HTL epitope is a PanDR binding peptide.

28. The polyepitopic peptide according to any of claims 1 to 13 and 18 to 27 further comprising at least one HLA class I binding peptide, at least one HLA class II binding peptide or at least one HCV derived peptide.

29. The polyepitopic peptide according to any of claims 1 to 13 and 18 to 28 wherein the peptides are either contiguous or are separated by a linker or a spacer amino acid or spacer peptide.

30. The polyepitopic peptide according to any of claims 1 to 13 and 18 to 29 wherein the peptides are present as homopolymers and/or heteropolymers.

31. An isolated nucleic acid or polynucleotide encoding the peptide or polyepitopic peptide of any of claims 1 to 30.

32. The isolated nucleic or polynucleotide according to claim 31 further comprising at least one spacer nucleic acid.

33. The isolated nucleic or polynucleotide according to claims 31 or 32 further comprising a signal sequence and/or promotor sequence.

34. A vector comprising the nucleic acid or polynucleotide according to any of claims 31 to 33.

35. The vector according to claim 34 wherein said vector is a plasmid.

36. The vector according to claim 34 wherein said vector is viral vector.

37. A host cell comprising the vector according to any of claims 34 to 36.

38. A method for producing the vector according to any of claims 34 to 36 comprising introducing the nucleic acid or polynucleotide according to any of claims 31 to 33 into a vector.

39. A composition comprising the peptide or polyepitopic peptide according to any of claims 1 to 30, or the nucleic acid or polynucleotide according to any of claims 31 to 33, or the vector according to any of claims 34 to 36, or any combination thereof.

40. The composition according to claim 39 wherein the peptides or nucleic acids are present in an admixture.

41. The composition according to claims 39 or 40 wherein said composition is a pharmaceutical composition.

42. The composition according to claim 41 further comprising at least one of a pharmaceutically acceptable carrier, adjuvant or vehicle.

43. The composition according to claims 41 or 42 wherein said composition is a vaccine composition.

44. The peptide or polyepitopic peptide according to any of claims 1 to 30, or the nucleic acid or polynucleotide according to any of claims 31 to 33, or the vector according to any of claims 34 to 36, or the composition according to any of claims of 39 to 43, or any combination thereof, for use as a medicament.

45. A method for inducing an immune response in a subject against HCV which comprises administration of the peptide or polyepitopic peptide according to any of claims 1 to 30, or the nucleic acid or polynucleotide according to any of claims 31 to 33, or the vector according to any of claims 34 to 36, or the composition according to any of claims of 39 to 43, or any combination thereof.

46. Use of the peptide or polyepitopic peptide according to any of claims 1 to 30, or the nucleic acid or polynucleotide according to any of claims 31 to 33, or the vector according to any of claims 34 to 36, or the composition according to any of claims of 39 to 43, or any combination thereof, for the manufacture of a medicament for the treatment of a subject with or at risk of HCV.

47. A method for producing the peptide or polyepitopic peptide according to any of claims 1 to 13 or 18 to 30 comprising the step of synthetic or recombinant production.

48. A method for producing the nucleic acid or polynucleotide according to any of claims 31 to 33 comprising the step of synthetic production.

49. A method of determining the outcome of infection for a subject exposed to HCV, comprising the steps of determining whether the subject has an immune response to one or more peptides, or the nucleic acids encoding them, according to any of claims 1 to 33.

50. Use of the peptide, or the nucleic acids encoding them, according to any of claims 1 to 33 for evaluating an immune response.

51. Use of the peptide, or the nucleic acids encoding them, according to any of claims 1 to 33 for evaluating the immune recall response.

52. Use of the peptide, or the nucleic acids encoding them, according to any of claims 1 to 33 for evaluating the efficacy of a vaccine.

53. Use of the peptide, or the nucleic acids encoding them, according to any of claims 1 to 33 for evaluating or predicting the outcome of the therapeutic effect of a HCV vaccine.

AA start AA end

| | | |
|---|---|---|
| 1 | 100 | MSTNPKPQRKTKRNTNRRPQDVKFPGGGQIVGGVYLLPRRGPRLGVRATRKTSERSQPRGRRQPIPKARRPEGRTWAQPGYPWPLYGNEGLGWAGWLLSP |
| 101 | 200 | RGSRPSWGPTDPRRRSRNLGKVIDTLTCGFADLMGYIPLVGAPLGGAARALAHGVRVLEDGVNYATGNLPGCSFSIFLLALLSCLTIPASAYEVRNVSGV |
| 201 | 300 | YHVTNDCSNSSIVYEAADMIMHTPGCVPCVRENNSSRCWVALTPTLAARNASVPTTTIRRHVDLLVGAAAFCSAMYVGDLCGSVFLVSQLFTFSPRRHET |
| 301 | 400 | VQDCNCSIYPGHISGHRMAWDMMMNWSPTTALVVSQLLRIPQAVVDMVAGAHWGVLAGLAYYSMVGNWAKVLVVMLLFAGVDGTTHVTGGAA(A/Q)RTTSGFT |
| 401 | 500 | SLFSPGASQKIQLVNTNGSWHINRTALNCNDSLQTGFLAALFYTHRFNSSGCPERMASCRPIDKFAQGWGPITYAEPNSSDQRPYCWHYAPRPCGIVPAS |
| 501 | 600 | QVCGPVYCFTPSPVVVGTTDRFGVPTYSWGENETDVLLLNNTRPPQGNWFGCTWMNSTGFTKTCGGPPCNIGGVGNNTLTCPTDCFRKHPEATYTKCGSG |
| 601 | 700 | PWLTPRCLVDYPYRLWHYPCTVNFTIFKVRMYVGGVEHRLNAACNWTRGERCDLEDRDRSELSPLLLSTTEWQILPCSFTTLPALSTGLIHLHQNIVDVQ |
| 701 | 800 | YLYGIGSAVVSFAIKWEYVLLLFLLLADARVCACLWMMLLIAQAEAALENLVVLNAASVAGAHGILSFLVFFCAAWYIKGRLVPGAAYAFYGVWPLLLLL |
| 801 | 900 | LALPPRAYALDREMAASCGGAVFVGLALLTLSPYYKVFLARLIWWLQYFITRAEAHLQVWVPPLNVRGGRDAIILLTCAVHPELIFDITKLLLAILGPLM |
| 901 | 1000 | VLQAGITRVPYFVRAQGLIRACMLVRKVAGGHYVQMAFMKLAALTGTYVYDHLTPLRDWAHAGLRDLAVAVEPVVFSDMETKIITWGADTAACGDIILGL |
| 1001 | 1100 | PVSARRGREILLGPADSLEGQGWRLLAPITAYSQQTRGLLGCIITSLTGRDKNQVEGEVQVVSTATQSFLATCVNGVCWTVYHGAGSKTLAGPKGPITQM |
| 1101 | 1200 | YTNVDQDLVGWQAPPGARSMTPCTCGSSDLYLVTRHADVIPVRRRGDSRGSLLSPRPVSYLKGSSGGPLLCPSGHVVGVFRAAVCTRGVAKAVDFIPVES |
| 1201 | 1300 | METTMRSPVFTDNSSPPAVPQTFQVAHLHAPTGSGKSTKVPAAYAAQGYKVLVLNPSVAATLGFGAYMSKAHGVDPNIRTGVRTITTGAPITYSTYGKFL |
| 1301 | 1400 | ADGGCSGGAYDIIICDECHSTDSTTILGIGTVLDQAETAGARLVVLATATPPGSVTVPHPNIEEIGLSNNGEIPFYGKAIPIEAIKGGRHLIFCHSKKKC |
| 1401 | 1500 | DELAAKLSALGLNAVAYYRGLDVSVIPTSGDVVVVATDALMTGYTGDFDSVIDCNTCVTQTVDFSLDPTFTIETTTVPQDAVSRSQRRGRTGRGRRGIYR |
| 1501 | 1600 | FVTPGERPSGMFDSSVLCECYDAGCAWYELTPAETSVRLRAYLNTPGLPVCQDHLEFWESVFTGLTHIDAHFLSQTKQAGDNFPYLVAYQATVCARAQAP |
| 1601 | 1700 | PPSWDQMWKCLIRLKPTLHGPTPLLYRLGAVQNEVTLTHPITKYIMACMSADLEVVTSTWVLVGGVLAALAAYCLTTGSVVIVGRIILSGRPAVIPDREV |
| 1701 | 1800 | LYQEFDEMEECASHLPYIEQGMQLAEQFKQKALGLLQTATKQAEAAAPVVESKWRALETFWAKHMWNFISGIQYLAGLSTLPGNPAIASLMAFTASITSP |
| 1801 | 1900 | LTTQHTLLFNILGGWVAAQLAPPSAASAFVGAGIAGAAVGSIGLGKVLVDILAGYGAGVAGALVAFKVMSGEMPSTEDLVNLLPAILSPGALVVGVVCAA |
| 1901 | 2000 | ILRRHVGPGEGAVQWMNRLIAFASRGNHVSPTHYVPESDAAARVTQILSSLTITQLLKRLHQWINEDCSTPCSGSWLRDVWDWICTVLTDFKTWLQSKLL |
| 2001 | 2100 | PRLPGVPFFSCQRGYKGVWRGDGIMQTTCPCGAQITGHVKNGSMRIVGPKTCSNTWHGTFPINAYTTGPCTPSPAPNYSRALWRVAAEEYVEVTRVGDFH |
| 2101 | 2200 | YVTGMTTDNVKCPCQVPAPEFFTEVDGVRLHRYAPACKPLLREEVTFQVGLNQYLVGSQLPCEPEPDVAVLTSMLTDPSHITAETAKRRLARGSPPSLAS |
| 2201 | 2300 | SSASQLSAPSLKATCTTHHDSPDADLIEANLLWRQEMGGNITRVESENKVVILDSFDPLRAEEDEREVSVAAEILRKSRKFPPALPIWARPDYNPPLLES |
| 2301 | 2400 | WKDPDYVPPVVHGCPLPPTKAPPIPPPRRKRTVVLTESTVSSALAELATKTFGSSGSSAVDSGTATAPPDQASDDGDKGSDVESYSSMPPLEGEPGDPDL |
| 2401 | 2500 | SDGSWSTVSEEAS-EDVVCCSMSYTWTGALITPCAAEESKLPINALSNSLLRHHNMVYATTSRSASLRQKKVTFDRLQVLDDHYRDVLKEMKAKASTVKA |
| 2501 | 2600 | KLLSVEEACKLTPPHSAKSKFGYGAKDVRNLSSKAVNHIRSVWKDLLEDTETPIDTTIMAKNEVFCVQPEKGGRKPARLIVFPDLGVRVCEKMALYDVVS |
| 2601 | 2700 | TLPQAVMGSSYGFQYSPGQRVEFLVNAWKSKKCPMGFSYDTRCFDSTVTENDIRVEESIYQCCDLAPEARQAIRSLTERLYIGGPLTNSKGQNCGYRRCR |
| 2701 | 2800 | ASGVLTTSCGNTLTCYLKASAACRAAKLQDCTMLVNGDDLVVICESAGTQEDAASLRVFTEAMTRYSAPPGDPPQPEYDLELITSCSSNVSVAHDASGKR |
| 2801 | 2900 | VYYLTRDPTTPLARAAWETARHTPVNSWLGNIIMYAPTLWARMILMTHFFSILLAQEQLEKALDCQIYGACYSIEPLDLPQII(E/Q)RLHGLSAFSLHSYSPG |
| 2901 | 3000 | EINRVASCLRKLGVPPLRVWRHRARSVRAKLLSQGGRAATCGKYLFNWAVRTKLKLTPIPAASQLDLSGWFVAGYSGGDIYHSLSRARPRWFM(W/L)CLLLLS |
| 3001 | 3011 | VGVGIYLLPNR |

Figure 1

```
   1    100 MSTNPKPQRKTKRNTNRRPQDVKFPGGGQIVGGVYLLPRRGPRLGVRATRKTSERSQPRGRRQPIPKARRPEGRAWAQPGYPWPLYGNEGMGWAGWLLSP
 101    200 RGSRPSWGPTDPRRRSRNLGKVIDTLTCGFADLMGYIPLVGAPLGGAARALAHGVRVLEDGVNYATGNLPGCSFSIFLLALLSCLTIPASAYEVRNVSGV
 201    300 YHVTNDCSNSSIVYEAADMIMHTPGCVPCVRENNSSRCWVALTPTLAARNSSVPTTTIRRHVDLLVGAAAFCSAMYVGDLCGSVFLVSQLFTFSPRRHET
 301    400 VQDCNCSIYPGHVSGHRMAWDMMMNWSPTTALVVSQLLRIPQAVVDMVAGAHWGVLAGLAYYSMVGNWAKVLIVMLLFAGVDGTTHVTGGAAARTTSGFT
 401    500 SLFSPGPSQKIQLVNTNGSWHINRTALNCNDSLQTGFLAALFYTHRFNASGCPERMASCRPIDKFAQGWGPITYAEPNSSDQRPYCWHYAPRPCGIVPAS
 501    600 QVCGPVYCFTPSPVVVGTTDRFGVPTYSWGENETDVLLLNNTRPPQGNWFGCTWMNSTGFTKTCGGPPCNIGGVGNNTLTCPTDCFRKHPEATYTKCGSG
 601    700 PWLTPRCLVDYPYRLWHYPCTVNFTIFKVRMYVGGVEHRLNAACNWTRGERCDLEDRDRSELSPLLLSTTEWQILPCSFTTLPALSTGLIHLHQNIVDVQ
 701    800 YLYGIGSAVVSFAIKWEYVLLLFLLLADARVCACLWMMLLIAQAEAALENLVVLNAASVAGAHGILSFLVFFCAAWYIKGRLVPGAAYALYGVWPLLLLL
 801    900 LALPPRAYAMDREMAASCGGAVFVGLALLTLSPYYKVFLARLIWWLQYFITRAEAHLQVWVPPLNVRGGRDAIILLTCAVHPELIFDITKLLLAILGPLM
 901   1000 VLQAGITRVPYFVRAQGLIRACMLVRKVAGGHYVQMAFMKLAALTGTYVYDHLTPLRDWAHAGLRDLAVAVEPVVFSDMETKIITWGADTAACGDIILGL
1001   1100 PVSARRGREILLGPADSLEGQGWRLLAPITAYSQQTRGLLGCIITSLTGRDKNQVEGEVQVVSTATQSFLATCVNGVCWTVYHGAGSKTLAGPKGPITQM
1101   1200 YTNVDQDLVGWQAPPGARSLTPCTCGSSDLYLVTRHADVIPVRRRGDSRGSLLSPRPVSYLKGSSGGPLLCPSGHAVGIFRAAVCTRGVAKAVDFVPVES
1201   1300 METTMRSPVFTDNSSPPAVPQTFQVAHLHAPTGSGKSTKVPAAYAAQGYKVLVLNPSVAATLGFGAYMSKAHGVDPNIRTGVRTITTGAPITYSTYGKFL
1301   1400 ADGGCSGGAYDIIICDECHSTDSTTILGIGTVLDQAETAGARLVVLATATPPGSVTVPHPNIEEVALSNTGEIPFYGKAIPIEVIKGGRHLIFCHSKKKC
1401   1500 DELAAKLSALGLNAVAYYRGLDVSVIPTSGDVVVVATDALMTGYTGDFDSVIDCNTCVTQTVDFSLDPTFTIETTTVPQDAVSRSQRRGRTGRGRRGIYR
1501   1600 FVTPGERPSGMFDSSVLCECYDAGCAWYELTPAETSVRLRAYLNTPGLPVCQDHLEFWESVFTGLTHIDAHFLSQTKQAGDNFPYLVAYQATVCARAQAP
1601   1700 PPSWDQMWKCLIRLKPTLHGPTPLLYRLGAVQNEVTLTHPITKYIMACMSADLEVVTSTWVLVGGVLAALAAYCLTTGSVVIVGRIILSGRPAVIPDREV
1701   1800 LYREFDEMEECASHLPYIEQGMQLAEQFKQKALGLLQTATKQAEAAAPVVESKWRALETFWAKHMWNFISGIQYLAGLSTLPGNPAIASLMAFTASITSP
1801   1900 LTTQHTLLFNILGGWVAAQLAPPSAASAFVGAGIAGAAVGSIGLGKVLVDILAGYGAGVAGALVAFKVMSGEMPSTEDLVNLLPAILSPGALVVGVVCAA
1901   2000 ILRRHVGPGEGAVQWMNRLIAFASRGNHVSPTHYVPESDAAARVTQILSSLTITQLLKRLHQWINEDCSTPCSGSWLRDVWDWICTVLTDFKTWLQSKLL
2001   2100 PRLPGVPFFSCQRGYKGVWRGDGIMQTTCPCGAQITGHVKNGSMRIVGPKTCSNTWHGTFPINAYTTGPCTPSPAPNYSRALWRVAAEEYVEVTRVGDFH
2101   2200 YVTGMTTDNVKCPCQVPAPEFFTEVDGVRLHRYAPACKPLLREEVTFQVGLNQYLVGSQLPCEPEPDVAVLTSMLTDPSHITAETAKRRLARGSPPSLAS
2201   2300 SSASQLSAPSLKATCTTHHDSPDADLIEANLLWRQEMGGNITRVESENKVVILDSFDPLRAEEDEREVSVAAEILRKSRKFPPALPIWARPDYNPPLLES
2301   2400 WKDPDYVPPVVHGCPLPPTKAPPIPPPRRKRTVVLTESTVSSALAELATKTFGSSGSSAVDSGTATAPPDQASDDGDKGSDVESYSSMPPLEGEPGDPDL
2401   2500 SDGSWSTVSEEAS-EDVVCCSMSYTWTGALITPCAAEESKLPINALSNSLLRHHNMVYATTSRSASLRQKKVTFDRLQVLDDHYRDVLKEMKAKASTVKA
2501   2600 KLLSVEEACKLTPPHSAKSKFGYGAKDVRNLSSKAVNHIRSVWKDLLEDTETPIDTTIMAKNEVFCVQPEKGGRKPARLIVFPDLGVRVCEKMALYDVVS
2601   2700 TLPQAVMGSSYGFQYSPGQRVEFLVNAWKSKKCPMGFSYDTRCFDSTVTENDIRVEESIYQCCDLAPEARQAIRSLTERLYIGGPLTNSKGQNCGYRRCR
2701   2800 ASGVLTTSCGNTLTCYLKASAACRAAKLQDCTMLVNGDDLVVICESAGTQEDAASLRVFTEAMTRYSAPPGDPPQPEYDLELITSCSSNVSVAHDASGKR
2801   2900 VYYLTRDPTTPLARAAWETARHTPVNSWLGNIIMYAPTLWARMILMTHFFSILLAQEQLEKALDCQIYGACYSIEPLDLPQIIERLHGLSAFSLHSYSPG
2901   3000 EINRVASCLRKLGVPPLRVWRHRARSVRAKLLSQGGRAATCGKYLFNWAVRTKLKLTPIPAASQLDLSGWFVAGYSGGDIYHSLSRARPRWFMLCLLLLS
3001   3100 VGVGIYLLPNR
```

Figure 2

**FLPSDC(+ fluo)FPSV titration**
**HLA-A02**
**JY cells**

One site binding (hyperbola)
Best-fit values
    BMAX        55.61
    KD           9.987

**FLPSDC(+ fluo)FPSV titration**
**HLA-A02**
**JY cells**

Best-fit values
    Slope                        -0.1053 ± 0.003372
    Y-intercept when X=0.0  5.777 ± 0.1135
    X-intercept when Y=0.0  54.85
    1/slope                     -9.493

**Figure 3**

EP 1 652 858 A1

Competition
Peptides WLGNIIMYA 64000 nM start concentration
and FLPSDC(+ fluo)FPSV 5 nM

Figure 4

```
AA start  AA end
       1    100 MSTNPKPQRKTKRNTNRRPQDVKFPGGGQIVGGVYLLPRRGPRLGVRATRKTSERSQPRGRRQPIPKARRPEGRTWAQPGYPWPLYGNEGCGWAGWLLSP
     101    200 RGSRPSWGPTDPRRRSRNLGKVIDTLTCGFADLMGYIPLVGAPLGGAARALAHGVRVLEDGVNYATGNLPGCSFSIFLLALLSCLTVPASAYQVRNSSGL
     201    300 YHVTNDCPNSSIVYEAADAILHTPGCVPCVREGNASRCWVAVTPTVATRDGKLPTTQLRRHIDLLVGSATLCSALYVGDLCGSVFLVGQLFTFSPRRHWT
     301    400 TQDCNCSIYPGHITGHRMAWDMMMNWSPTAALVVAQLLRIPQAILDMIAGAHWGVLAGIAYFSMVGNWAKVLVVLLLLFAGVDAETHVTGGSAARTTAGLV
     401    500 SLLTPGAKQNIQLINTNGSWHINSTALNCNESLNTGWLAGLFYTHKFNSSGCPERLASCRRLTDFDQGWGPISYANGSGPDQRPYCWHYPPKPCGIVPAK
     501    600 SVCGPVYCFTPSPVVVGTTDRSGAPTYSWGANDTDVFVLNNTRPPLGNWFGCTWMNSTGFTKVCGAPPCVIGGVGNNTLHCPTDCFRKHPEATYSRCGSG
     601    700 PWITPRCLVDYPYRLWHYPCTINYTIFKVRMYVGGVEHRLEAACNWTRGERCDLEDRDRSELSPLLLSTTQWQVLPCSFTTLPALSTGLIHLHQNIVDVQ
     701    800 YLYGVGSSIASWAIKWEYVVLLFLLLADARVCSCLWMMLLISQAEAALENLVILNAASLAGTHGLVSFLVFFCFAWYLKGRWVPGAVYALYGMWPLLLLLL
     801    900 LALPQRAYALDTEVAASCGGVVLVGLMALTLSPYYKRYISWCLWWLQYFLTRVEAQLHVWVPPLNVRGGRDAVILLMCVVHPTLVFDITKLLLAVFGPLW
     901   1000 ILQASLLKVPYFVRVQGLLRICALARKMAGGHYVQMAIIKLGALTGTYVVYNHLTPLRDWAHNGLRDLAVAVEPVVFSQMETKLITWGADTAACGDIINGL
    1001   1100 PVSARRGREILLGPADGMVSKGWRLLAPITAYAQQTRGLLGCIITSLTGRDKNQVEGEVQIVSTAAQTFLATCINGVCWTVYHGAGTRTIASPKGPVIQM
    1101   1200 YTNVDQDLVGWPAPQGARSLTPCTCGSSDLYLVTRHADVIPVRRRGDSRGSLLSPRPISYLKGSSGGPLLCPAGHAVGIFRAAVCTRGVAKAVDFIPVEN
    1201   1300 LETTMRSPVFTDNSSPPAVPQSFQVAHLHAPTGSGKSTKVPAAYAAQGYKVLVLNPSVAATLGFGAYMSKAHGIDPNIRTGVRTITTGSPITYSTYGKFL
    1301   1400 ADGGCSGGAYDIIICDECHSTDATSILGIGTVLDQAETAGARLVVLATATPPGSVTVPHPNIEEVALSTTGEIPFYGKAIPLEVIKGGRHLIFCHSKKKC
    1401   1500 DELAAKLVALGINAVAYYRGLDVSVIPTSGDVVVVATDALMTGFTGDFDSVIDCNTCVTQTVDFSLDPTFTIETTTLPQDAVSRTQRRGRTGRGKPGIYR
    1501   1600 FVAPGERPSGMFDSSVLCECYDAGCAWYELTPAETTVRLRAYMNTPGLPVCQDHLEFWEGVFTGLTHIDAHFLSQTKQSGENFPYLVAYQATVCARAQAP
    1601   1700 PPSWDQMWKCLIRLKPTLHGPTPLLYRLGAVQNEVTLTHPVTKYIMTCMSADLEVVTSTWVLVGGVLAALAAYCLSTGCVVIVGRIVLSGKPAIIPDREV
    1701   1800 LYREFDEMEECSQHLPYIEQGMMLAEQFKQKALGLLQTASRQAEVIAPAVQTNWQKLEAFWAKHMWNFISGIQYLAGLSTLPGNPAIASLMAFTAAVTSP
    1801   1900 LTTSQTLLFNILGGWVAAQLAAPGAATAFVGAGLAGAAIGSVGLGKVLVDILAGYGAGVAGALVAFKIMSGEVPSTEDLVNLLPAILSPGALVVGVVCAA
    1901   2000 ILRRHVGPGEGAVQWMNRLIAFASRGNHVSPTHYVPESDAAARVTAILSSLTVTQLLRRLHQWISSECTTPCSGSWLRDIWDWICEVLSDFKTWLKAKLM
    2001   2100 PQLPGIPFVSCQRGYRGVWRGDGIMHTRCHCGAEITGHVKNGTMRIVGPKTCRNMWSGTFPINAYTTGPCTPLPAPNYTFALWRVSAEEYVEIRRVGDFH
    2101   2200 YVTGMTTDNLKCPCQVPSPEFFTELDGVRLHRFAPPCKPLLREEVSFRVGLHEYPVGSQLPCEPEPDVAVLTSMLTDPSHITAEAAGRRLARGSPPSMAS
    2201   2300 SSASQLSAPSLKATCTANHDSPDAELIEANLLWRQEMGGNITRVESENKVVILDSFDPLVAEEDEREISVPAEILRKSRRFAQALPVWARPDYNPPLLET
    2301   2400 WKKPDYEPPVVHGCPLPPPRSPPVPPPRKKRTVVLTESTLSTALAELATKSFGSSSTSGITGDNTTTSSEPAPSGCPPDSDAESYSSMPPLEGEPGDPDL
    2401   2500 SDGSWSTVSSEASTEDVVCCSMSYSWTGALVTPCAAEEQKLPINALSNSLLRHHNLVYSTTSRSACQRQKKVTFDRLQVLDSHYQDVLKEVKAAASKVKA
    2501   2600 NLLSVEEACSLTPPHSAKSKFGYGAKDVRCHARKAVNHINSVWKDLLEDSVTPIDTTIMAKNEVFCVQPEKGGRKPARLIVFPDLGVRVCEKMALYDVVS
    2601   2700 KLPLAVMGSSYGFQYSPGQRVEFLVQAWKSKKTPMGFSYDTRCFDSTVTESDIRTEEAIYQCCDLDPQARVAIKSLTERLYVGGPLTNSRGENCGYRRCR
    2701   2800 ASGVLTTSCGNTLTCYIKARAACRAAGLQDCTMLVCGDDLVVICESAGVQEDAASLRAFTEAMTRYSAPPGDPPQPEYDLELITSCSSNVSVAHDGAGKR
    2801   2900 VYYLTRDPTTPLARAAWETARHTPVNSWLGNIIMFAPTLWARMILMTHFFSVLIARDQLEQALDCEIYGACYSIEPLDLPPIIQRLHGLSAFSLHSYSPG
    2901   3000 EINRVAACLRKLGVPPLRAWRHRARSVRARLLSRGGRAAICGKYLFNWAVRTKLKLTPIAAAGRLDLSGWFTAGYSGGDIYHSVSHARPRWFWFCLLLLA
    3001   3011 AGVGIYLLPNR
```

Figure 5

```
AA start   AA end
        1      100 MSTLPKPQRKTKRNTIRRPQDVKFPGGGQIVGGVYVLPRRGPRLGVRATRKTSERSQPRGRRQPIPKARRSEGRSWAQPGYPWPLYGNEGCGWAGWLLSP
      101      200 RGSRPSWGPNDPRRRSRNLGKVIDTLTCGFADLMGYIPLVGAPVGGVARALAHGVRALEDGINFATGNLPGCSFSIFLLALFSCLIHPAASLEWRNTSGL
      201      300 YVLTNDCSNSSIVYEADDVILHTPGCVPCVQDGNTSTCWTPVTPTVAVRYVGATTASIRSHVDLLVGAATMCSALYVGDMCGAVFLVGQAFTFRPRRHQT
      301      400 VQTCNCSLYPGHLSGHRMAWDMMMNWSPAVGMVVAHVLRLPQTLFDIIAGAHWGILAGLAYYSMQGNWAKVAIIMVMFSGVDATTYTTGGSAARTTSGLT
      401      500 SLFSVGPQQKLQLVNTNGSWHINSTALNCNESINTGFIAGLFYYHKFNSTGCPQRLSSCKPITFFAQGWGPLTDANITGPSDDKPYCWHYAPRPCDIVPA
      501      600 SNVCGPVYCFTPSPVVVGTTDAKGVPTYTWGENETDVFLLESLRPPSGRWFGCTWMNSTGFTKTCGAPPCNIYGGGGNPNESDLFCPTDCFRKHPEATYS
      601      700 RCGAGPWLTPRCMVDYPYRLWHYPCTVNFTLFKVRMFVGGFEHRFTAACNWTRGERCDIEDRDRSEQHPLLHSTTELAILPCSFTPMPALSTGLIHLHQN
      701      800 IVDVQYLYGVGSGMVGWALKWEFVILIFLLLADARVCVALWLMLMISQAEAALENLVTLNAVAAAGTHGIGWYLVAFCAAWYVRGKLVPLVTYSLTGLWS
      801      900 LALLVLLLPQRAYAWSGEDSATLGAGVLVLFGFFTLSPWYKHWIGRLIWWNQYTICRCESALQVWVPPLLARGSRDGVILLTSLLYPSLIFDITKLLIAV
      901     1000 LGPLYLIQATITRTPYFVRAHVLVRLCMLVRSVMGGKYFQMIILSIGRWFNTYLYDHLAPMQHWAAAGLKDLAVATEPVIFSPMEIKVITWGADTAACGD
     1001     1100 ILCGLPVSARLGREVLLGPADDYREMGWRLLAPITAYAQQTRGLLGTIVTSLTGRDKNVVTGEVQVLSTATQTFLGTTVGGVMWTVYHGAGSRTLAGAKH
     1101     1200 PALQMYTNVDQDLVGWPAPPGAKSLEPCACGSADLYLVTRDADVIPARRRGDSTASLLSPRPLACLKGSSGGPVMCPSGHVAGIFRAAVCTRGVAKALQF
     1201     1300 IPVETLSTQARSPSFSDNSTPPAVPQSYQVGYLHAPTGSGKSTKVPAAYVAQGYNVLVLNPSVAATLGFGSFMSRAYGIDPNIRTGNRTVTTGAKLTYST
     1301     1400 YGKFLADGGCSGGAYDVIICDECHAQDATSILGIGTVLDQAETAGVRLTVLATATPPGSITVPHSNIEEVALGSEGEIPFYGKAIPIALLKGGRHLIFCH
     1401     1500 SKKKCDEIASKLRGMGLNAVAYYRGLDVSVIPTTGDVVVCATDALMTGFTGDFDSVIDCNVAVEQYVDFSLDPTFSIETRTAPQDAVSRSQRRGRTGRGR
     1501     1600 LGTYRYVAPGERPSGMFDSVVLCECYDAGCSWYDLQPAETTVRLRAYLSTPGLPVCQDHLDFWESVFTGLTHIDAHFLSQTKQQGLNFSYLTAYQATVCA
     1601     1700 RAQAPPPSWDEMWKCLVRLKPTLHGPTPLLYRLGPVQNEVCLTHPITKYVMACMSADLEVTTSTWVLLGGVLAALAAYCLSVGCVVIVGHIELGGKPALV
     1701     1800 PDKEVLYQQYDEMEECSQAAPYIEQAQQIAHQFKEKVLGLLQRATQQQAVIEPIVATNWQKLEAFWHKHMWNFVSGIQYLAGLSTLPGNPAVASLMAFTA
     1801     1900 SVTSPLTTNQTMFFNILGGWVATHLAGPQSSSAFVVSGLAGAAIGGIGLGRVLLDILAGYGAGVSGALVAFKIMGGELPTTEDMVNLLPAILSPGALVVG
     1901     2000 VICAAILRRHVGPGEGAVQWMNRLIAFASRGNHVSPTHYVPESDAAARVTALLSSLTVTSLLRRLHQWINEDYPSPCSDDWLRIIWDWVCSVLTDFKTWL
     2001     2100 SAKIMPALPGLPFISCQKGYKGVWRGDGVMSTRCPCGATITGHVKNGSMRLAGPRTCANMWHGTFPINEYTTGPSTPCPSPNYTRALWRVAANSYVEVRR
     2101     2200 VGDFHYITGATEDELKCPCQVPAAEFFTEVDGVRLHRYAPPCKPLLRDEITFMVGLNSYLIGSQLPCEPEPDVSVLTSMLRDPSHITAETAARRLARGSP
     2201     2300 PSEASSSASQLSAPSLKATCQTHRPHPDAELVDANLLWRQEMGSNITRVESETKVVILDSFEPLRAETDDAELSVAAECFKKPPKYPPALPIWARPDYNP
     2301     2400 PLLDRWKAPDYVPPTVHGCALPPRGAPPVPPPRRKRTIQLDGSNVSAALAALAEKSFPSSKPQEENSSSSGVDTQSSTTSKVPPSPGGESDSESCSSMPP
     2401     2500 LEGEPGDPDLSCDSWSTVSDSEEQSVVCCSMSYSWTGALITPCSAEEEKLPISPLSNSLLRHHNLVYSTSSRSASQRQKKVTFDRLQVLDDHYKTALKEV
     2501     2600 KERASRVKARMLTIEEACALVPPHSARSKFGYSAKDVRSLSSRAINQIRSVWEDLLEDTTTPIPTTIMAKNEVFCVDPAKGGRKPARLIVYPDLGVRVCE
     2601     2700 KRALYDVIQKLSIETMGSAYGFQYSPQQRVERLLKMWTSKKTPLGFSYDTRCFDSTVTEQDIRVEEEIYQCCNLEPEARKVISSLTERLYCGGPMFNSKG
     2701     2800 AQCGYRRCRASGVLPTSFGNTITCYIKATAAAKAAGLRNPDFLVCGDDLVVVAESDGVDEDRAALRAFTEAMTRYSAPPGDAPQPTYDLELITSCSSNVS
     2801     2900 VARDDKGKRYYYLTRDATTPLARAAWETARHTPVNSWLGNIIMYAPTIWVRMVMMTHFFSILQSQEILDRPLDFEMYGATYSVTPLDLPAIIERLHGLSA
     2901     3000 FTLHSYSPVELNRVAGTLRKLGCPPLRAWRHRARAVRAKLIAQGGKAKICGLYLFNWAVRTKTKLTPLPAAGQLDLSSWFTVGVGGNDIYHSVSRARTRY
     3001     3020 LLLCLLLLTVGVGIFLLPAR
```

Figure 6

**European Patent Office**

## EUROPEAN SEARCH REPORT

Application Number

EP 04 44 7239

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | CHANG S C ET AL: "Roles of the AX(4)GKS and arginine-rich motifs of hepatitis C virus RNA helicase in ATP- and viral RNA-binding activity." JOURNAL OF VIROLOGY. OCT 2000, vol. 74, no. 20, October 2000 (2000-10), pages 9732-9737, XP002323419 ISSN: 0022-538X The whole document; see particularly page 9732, column 2; fig.1 | 1-7 | C07K14/18 A61K39/295 G01N33/576 A61K38/16 |
| A | WO 01/21189 A (EPIMMUNE INC; SETTE, ALESSANDRO; SIDNEY, JOHN; SOUTHWOOD, SCOTT; LIVIN) 29 March 2001 (2001-03-29) See particularly pages 46-49 | 1-7 | |
| A | US 6 576 240 B1 (JOLIVET MICHEL ET AL) 10 June 2003 (2003-06-10) See particularly column 1, lines 10-18 | 1-7 | |
| A | HUANG J ET AL: "Specific immune response induced by a multi-epitope antigen of hepatitis C virus in mice and rabbits" CHINESE SCIENCE BULLETIN, SCIENCE PRES, BEIJING, CN, vol. 45, no. 10, May 2000 (2000-05), pages 896-901, XP002277922 * the whole document * | 1-7 | TECHNICAL FIELDS SEARCHED (Int.Cl.7) C07K A61K G01N |
| A | HE S ET AL: "IMMUNE RESPONSES IN RHESUS MONKEYS VACCINATED WITH MULTI-EPITOPE ANTIGEN OF HCV AND CHALLENGED BY HCV VIRUS" BINGDUXUE ZAZHI - VIROLOGICA SINICA, KEXUE CHUBANSHE, BEIJING,, CN, vol. 17, no. 1, February 2002 (2002-02), pages 30-33, XP008029886 ISSN: 1000-3223 * the whole document * | 1-7 | |

~~The present search report has been drawn up for all claims~~

| Place of search | Date of completion of the search | Examiner |
|---|---|---|
| The Hague | 6 April 2005 | Groenendijk, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

Application Number

EP 04 44 7239

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☒ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7 (all partially)

**European Patent Office**

**LACK OF UNITY OF INVENTION**
**SHEET B**

Application Number

EP 04 44 7239

---

The Search Division considers that the present European patent application does not comply with the requirements of unity of invention and relates to several inventions or groups of inventions, namely:

Invention 1: claims 1-7(all partially)

An isolated polyepitopic peptide comprising at least three peptides selected from the HLA-B and/or HLA-C binding peptides as disclosed in Table 13, in casu AAKLSALGL.

---

Inventions 2-452:claims 1-7(all partially)

An isolated polyepitopic peptide comprising at least three peptides selected from the HLA-B and/or HLA-C binding peptides as disclosed in Table 13.

---

Invention 452: claims 8-10

Peptides as defined in claims 8-10

---

## ANNEX TO THE EUROPEAN SEARCH REPORT
## ON EUROPEAN PATENT APPLICATION NO.          EP 04 44 7239

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2005

| Patent document cited in search report | | Publication date | Patent family member(s) | | Publication date |
|---|---|---|---|---|---|
| WO 0121189 | A | 29-03-2001 | AU | 6226100 A | 24-04-2001 |
| | | | CA | 2377525 A1 | 29-03-2001 |
| | | | EP | 1200109 A1 | 02-05-2002 |
| | | | JP | 2003509465 T | 11-03-2003 |
| | | | WO | 0121189 A1 | 29-03-2001 |
| US 6576240 | B1 | 10-06-2003 | FR | 2760458 A1 | 11-09-1998 |
| | | | AU | 6839598 A | 22-09-1998 |
| | | | CA | 2284083 A1 | 11-09-1998 |
| | | | EP | 1015481 A1 | 05-07-2000 |
| | | | WO | 9839360 A1 | 11-09-1998 |
| | | | JP | 2001522352 T | 13-11-2001 |
| | | | US | 2003118604 A1 | 26-06-2003 |

EPO FORM P0459

For more details about this annex : see Official Journal of the European Patent Office, No. 12/82

SEQUENCE LISTING

<110>  Innogenetics N.V.

<120>  Peptides for inducing a CTL and/or HTL response to Hepatitis C virus

<130>  EP II 166

<140>  04447239.7

<141>  2004-10-28

<160>  2253

<170>  PatentIn version 3.1

<210>  1
<211>  9
<212>  PRT
<213>  hepatitis C virus

<400>  1
Ala Thr Asp Ala Leu Met Thr Gly Tyr
1               5

<210>  2
<211>  9
<212>  PRT
<213>  hepatitis C virus

<400>  2
Leu Thr Cys Gly Phe Ala Asp Leu Met
1               5

<210>  3
<211>  9
<212>  PRT
<213>  hepatitis C virus

<400> 3

Arg Val Cys Glu Lys Met Ala Leu Tyr
1               5

<210> 4

<211> 9

<212> PRT

<213> hepatitis C virus

<400> 4

Phe Ala Asp Leu Met Gly Tyr Ile Pro
1               5

<210> 5

<211> 9

<212> PRT

<213> hepatitis C virus

<400> 5

Leu Thr His Ile Asp Ala His Phe Leu
1               5

<210> 6

<211> 9

<212> PRT

<213> hepatitis C virus

<400> 6

Ile Thr Thr Gly Ala Pro Ile Thr Tyr
1               5

<210> 7

<211> 9

<212> PRT

<213> hepatitis C virus

<400> 7

Phe Thr Asp Asn Ser Ser Pro Pro Ala
1               5

<210> 8

<211> 9

<212> PRT

<213> hepatitis C virus


<400> 8

Asp Asn Phe Pro Tyr Leu Val Ala Tyr
1               5


<210> 9

<211> 9

<212> PRT

<213> hepatitis C virus


<400> 9

Phe Thr Glu Ala Met Thr Arg Tyr Ser
1               5


<210> 10

<211> 9

<212> PRT

<213> hepatitis C virus


<400> 10

Asp Ala Ser Gly Lys Arg Val Tyr Tyr
1               5


<210> 11

<211> 9

<212> PRT

<213> hepatitis C virus


<400> 11

Gly Ala Pro Ile Thr Tyr Ser Thr Tyr
1               5


<210> 12

<211> 9

<212> PRT

<213> hepatitis C virus


<400> 12

Pro Ala Ala Tyr Ala Ala Gln Gly Tyr
1               5


<210> 13

<211> 9

<212> PRT

<213> hepatitis C virus


<400> 13

Cys Tyr Asp Ala Gly Cys Ala Trp Tyr
1               5


<210> 14

<211> 9

<212> PRT

<213> hepatitis C virus


<400> 14

Tyr Ala Pro Thr Leu Trp Ala Arg Met
1               5


<210> 15

<211> 9

<212> PRT

<213> hepatitis C virus


<400> 15

Pro Val Glu Ser Met Glu Thr Thr Met
1               5


<210> 16

<211> 9

<212> PRT

<213> hepatitis C virus

<400> 16

Ala Val Met Gly Ser Ser Tyr Gly Phe
1                   5

<210> 17

<211> 9

<212> PRT

<213> hepatitis C virus


<400> 17

Asp Ser Ser Val Leu Cys Glu Cys Tyr
1                   5

<210> 18

<211> 9

<212> PRT

<213> hepatitis C virus


<400> 18

Leu Gly Leu Asn Ala Val Ala Tyr Tyr
1               5

<210> 19

<211> 9

<212> PRT

<213> hepatitis C virus


<400> 19

Pro Gly Asp Pro Pro Gln Pro Glu Tyr
1               5

<210> 20

<211> 9

<212> PRT

<213> hepatitis C virus


<400> 20

Asn Gly Glu Ile Pro Phe Tyr Gly Lys
1               5

<210> 21

<211> 9

<212> PRT

<213> hepatitis C virus


<400> 21

Val Thr Leu Thr His Pro Ile Thr Lys
1               5

<210> 22

<211> 9

<212> PRT

<213> hepatitis C virus


<400> 22

Met Gly Ser Ser Tyr Gly Phe Gln Tyr
1               5

<210> 23

<211> 9

<212> PRT

<213> hepatitis C virus


<400> 23

Leu Thr His Pro Ile Thr Lys Tyr Ile
1               5

<210> 24

<211> 9

<212> PRT

<213> hepatitis C virus


<400> 24

Ala Gly Asp Asn Phe Pro Tyr Leu Val
1               5

<210> 25

<211> 9

<212> PRT

<213> hepatitis C virus

<400> 25

Lys Ser Thr Lys Val Pro Ala Ala Tyr
1               5

<210> 26

<211> 9

<212> PRT

<213> hepatitis C virus

<400> 26

Ile Thr Tyr Ser Thr Tyr Gly Lys Phe
1               5

<210> 27

<211> 9

<212> PRT

<213> hepatitis C virus

<400> 27

Pro Ala Glu Thr Ser Val Arg Leu Arg
1               5

<210> 28

<211> 9

<212> PRT

<213> hepatitis C virus

<400> 28

Val Ile Asp Thr Leu Thr Cys Gly Phe
1               5

<210> 29

<211> 9

<212> PRT

<213> hepatitis C virus

<400> 29

Gly Leu Asp Val Ser Val Ile Pro Thr
1               5

<210> 30

<211> 9

<212> PRT

<213> hepatitis C virus


<400> 30

Leu Asp Pro Thr Phe Thr Ile Glu Thr
1               5

<210> 31

<211> 9

<212> PRT

<213> hepatitis C virus


<400> 31

Leu Glu Asp Gly Val Asn Tyr Ala Thr
1               5

<210> 32

<211> 9

<212> PRT

<213> hepatitis C virus


<400> 32

Ala Thr Leu Gly Phe Gly Ala Tyr Met
1               5

<210> 33

<211> 9

<212> PRT

<213> hepatitis C virus


<400> 33

Pro Ser Trp Asp Gln Met Trp Lys Cys
1               5

<210> 34

<211> 9

<212> PRT

<213> hepatitis C virus


<400> 34

Pro Thr Leu Trp Ala Arg Met Ile Leu
1               5


<210> 35

<211> 9

<212> PRT

<213> hepatitis C virus


<400> 35

Pro Thr Phe Thr Ile Glu Thr Thr Thr
1               5


<210> 36

<211> 9

<212> PRT

<213> hepatitis C virus


<400> 36

Val Phe Thr Glu Ala Met Thr Arg Tyr
1               5


<210> 37

<211> 9

<212> PRT

<213> hepatitis C virus


<400> 37

Pro Gln Ala Val Met Gly Ser Ser Tyr
1               5


<210> 38

<211> 9

<212> PRT

<213> hepatitis C virus

<400> 38

Val Ala His Asp Ala Ser Gly Lys Arg
1               5


<210> 39

<211> 9

<212> PRT

<213> hepatitis C virus


<400> 39

Arg Val Phe Thr Glu Ala Met Thr Arg
1               5


<210> 40

<211> 9

<212> PRT

<213> hepatitis C virus


<400> 40

Gly Asn Thr Leu Thr Cys Tyr Leu Lys
1               5


<210> 41

<211> 9

<212> PRT

<213> hepatitis C virus


<400> 41

Glu Val Phe Cys Val Gln Pro Glu Lys
1               5


<210> 42

<211> 9

<212> PRT

<213> hepatitis C virus


<400> 42

Cys Ser Phe Ser Ile Phe Leu Leu Ala
1               5

```
<210>  43
<211>  9
<212>  PRT
<213>  hepatitis C virus


<400>  43

Tyr Ser Pro Gly Gln Arg Val Glu Phe
1               5


<210>  44
<211>  9
<212>  PRT
<213>  hepatitis C virus


<400>  44

Val Val Ala Thr Asp Ala Leu Met Thr
1               5


<210>  45
<211>  9
<212>  PRT
<213>  hepatitis C virus


<400>  45

Arg Val Leu Glu Asp Gly Val Asn Tyr
1               5


<210>  46
<211>  9
<212>  PRT
<213>  hepatitis C virus


<400>  46

Glu Thr Ser Val Arg Leu Arg Ala Tyr
1               5


<210>  47
<211>  9
```

<212> PRT

<213> hepatitis C virus


<400> 47

Leu Ile Phe Cys His Ser Lys Lys Lys
1               5


<210> 48

<211> 9

<212> PRT

<213> hepatitis C virus


<400> 48

Cys Cys Asp Leu Ala Pro Glu Ala Arg
1               5


<210> 49

<211> 9

<212> PRT

<213> hepatitis C virus


<400> 49

Asn Ser Trp Leu Gly Asn Ile Ile Met
1               5


<210> 50

<211> 9

<212> PRT

<213> hepatitis C virus


<400> 50

Leu Gly Phe Gly Ala Tyr Met Ser Lys
1               5


<210> 51

<211> 9

<212> PRT

<213> hepatitis C virus

<400> 51

Ala Leu Gly Leu Asn Ala Val Ala Tyr
1               5

<210> 52

<211> 9

<212> PRT

<213> hepatitis C virus

<400> 52

Glu Ser Met Glu Thr Thr Met Arg Ser
1               5

<210> 53

<211> 9

<212> PRT

<213> hepatitis C virus

<400> 53

Thr Asp Ala Leu Met Thr Gly Tyr Thr
1               5

<210> 54

<211> 9

<212> PRT

<213> hepatitis C virus

<400> 54

Phe Ile Pro Val Glu Ser Met Glu Thr
1               5

<210> 55

<211> 9

<212> PRT

<213> hepatitis C virus

<400> 55

Pro Thr Asp Pro Arg Arg Arg Ser Arg
1               5

<210> 56

<211> 9

<212> PRT

<213> hepatitis C virus


<400> 56

Ala Ala Tyr Ala Ala Gln Gly Tyr Lys
1               5


<210> 57

<211> 9

<212> PRT

<213> hepatitis C virus


<400> 57

Thr Thr Met Arg Ser Pro Val Phe Thr
1               5


<210> 58

<211> 9

<212> PRT

<213> hepatitis C virus


<400> 58

Asp Ala His Phe Leu Ser Gln Thr Lys
1               5


<210> 59

<211> 9

<212> PRT

<213> hepatitis C virus


<400> 59

Arg Met Ile Leu Met Thr His Phe Phe
1               5


<210> 60

<211> 9

<212> PRT

```
<213>   hepatitis C virus


<400>   60

Gln Ala Glu Thr Ala Gly Ala Arg Leu
1               5


<210>   61

<211>   9

<212>   PRT

<213>   hepatitis C virus


<400>   61

Met Ser Ala Asp Leu Glu Val Val Thr
1               5


<210>   62

<211>   9

<212>   PRT

<213>   hepatitis C virus


<400>   62

Trp Leu Gly Asn Ile Ile Met Tyr Ala
1               5


<210>   63

<211>   9

<212>   PRT

<213>   hepatitis C virus


<400>   63

Tyr Leu Val Ala Tyr Gln Ala Thr Val
1               5


<210>   64

<211>   9

<212>   PRT

<213>   hepatitis C virus


<400>   64
```

Leu Thr His Ile Asp Ala His Phe Leu
1               5

<210> 65

<211> 9

<212> PRT

<213> hepatitis C virus


<400> 65

Ala Gln Pro Gly Tyr Pro Trp Pro Leu
1               5

<210> 66

<211> 9

<212> PRT

<213> hepatitis C virus


<400> 66

Asp Leu Met Gly Tyr Ile Pro Leu Val
1               5

<210> 67

<211> 9

<212> PRT

<213> hepatitis C virus


<400> 67

Ala Leu Tyr Asp Val Val Ser Thr Leu
1               5

<210> 68

<211> 9

<212> PRT

<213> hepatitis C virus


<400> 68

Val Val Ser Thr Leu Pro Gln Ala Val
1               5

<210> 69

<211> 9

<212> PRT

<213> hepatitis C virus


<400> 69

Tyr Ile Pro Leu Val Gly Ala Pro Leu
1               5


<210> 70

<211> 9

<212> PRT

<213> hepatitis C virus


<400> 70

Leu Leu Ser Cys Leu Thr Ile Pro Ala
1               5


<210> 71

<211> 9

<212> PRT

<213> hepatitis C virus


<400> 71

Gly Met Phe Asp Ser Ser Val Leu Cys
1               5


<210> 72

<211> 9

<212> PRT

<213> hepatitis C virus


<400> 72

Ala Leu Ala His Gly Val Arg Val Leu
1               5


<210> 73

<211> 9

<212> PRT

<213> hepatitis C virus

<400> 73

Lys Val Leu Val Leu Asn Pro Ser Val
1               5

<210> 74

<211> 9

<212> PRT

<213> hepatitis C virus

<400> 74

Tyr Leu Asn Thr Pro Gly Leu Pro Val
1               5

<210> 75

<211> 9

<212> PRT

<213> hepatitis C virus

<400> 75

Lys Leu Gln Asp Cys Thr Met Leu Val
1               5

<210> 76

<211> 9

<212> PRT

<213> hepatitis C virus

<400> 76

Ser Val Phe Thr Gly Leu Thr His Ile
1               5

<210> 77

<211> 9

<212> PRT

<213> hepatitis C virus

<400> 77

Val Val Ala Thr Asp Ala Leu Met Thr
1               5

EP 1 652 858 A1

<210> 78

<211> 9

<212> PRT

<213> hepatitis C virus


<400> 78

Gly Leu Gly Trp Ala Gly Trp Leu Leu
1               5


<210> 79

<211> 9

<212> PRT

<213> hepatitis C virus


<400> 79

Arg Leu Tyr Ile Gly Gly Pro Leu Thr
1               5


<210> 80

<211> 9

<212> PRT

<213> hepatitis C virus


<400> 80

Phe Ile Pro Val Glu Ser Met Glu Thr
1               5


<210> 81

<211> 9

<212> PRT

<213> hepatitis C virus


<400> 81

Thr Leu His Gly Pro Thr Pro Leu Leu
1               5


<210> 82

<211> 9

154

```
<212>   PRT

<213>   hepatitis C virus


<400>   82

Leu Val Leu Asn Pro Ser Val Ala Ala
1               5


<210>   83

<211>   9

<212>   PRT

<213>   hepatitis C virus


<400>   83

Tyr Gln Ala Thr Val Cys Ala Arg Ala
1               5


<210>   84

<211>   9

<212>   PRT

<213>   hepatitis C virus


<400>   84

Ile Met Tyr Ala Pro Thr Leu Trp Ala
1               5


<210>   85

<211>   9

<212>   PRT

<213>   hepatitis C virus


<400>   85

Met Ala Leu Tyr Asp Val Val Ser Thr
1               5


<210>   86

<211>   9

<212>   PRT

<213>   hepatitis C virus
```

<400> 86

Arg Leu Val Val Leu Ala Thr Ala Thr
1               5

<210> 87

<211> 9

<212> PRT

<213> hepatitis C virus

<400> 87

Asn Ile Ile Met Tyr Ala Pro Thr Leu
1               5

<210> 88

<211> 9

<212> PRT

<213> hepatitis C virus

<400> 88

Gly Thr Gln Glu Asp Ala Ala Ser Leu
1               5

<210> 89

<211> 9

<212> PRT

<213> hepatitis C virus

<400> 89

Thr Ile Leu Gly Ile Gly Thr Val Leu
1               5

<210> 90

<211> 9

<212> PRT

<213> hepatitis C virus

<400> 90

Ile Met Ala Cys Met Ser Ala Asp Leu
1               5

```
<210>   91
<211>   9
<212>   PRT
<213>   hepatitis C virus


<400>   91
Gln Ile Val Gly Gly Val Tyr Leu Leu
1               5


<210>   92
<211>   9
<212>   PRT
<213>   hepatitis C virus


<400>   92
Thr Leu Trp Ala Arg Met Ile Leu Met
1               5


<210>   93
<211>   9
<212>   PRT
<213>   hepatitis C virus


<400>   93
Asn Leu Pro Gly Cys Ser Phe Ser Ile
1               5


<210>   94
<211>   9
<212>   PRT
<213>   hepatitis C virus


<400>   94
Met Leu Val Asn Gly Asp Asp Leu Val
1               5


<210>   95
<211>   9
<212>   PRT
```

<213> hepatitis C virus

<400> 95

```
Tyr Ala Ala Gln Gly Tyr Lys Val Leu
1               5
```

<210> 96

<211> 9

<212> PRT

<213> hepatitis C virus

<400> 96

```
Gly Val Ala Lys Ala Val Asp Phe Ile
1               5
```

<210> 97

<211> 9

<212> PRT

<213> hepatitis C virus

<400> 97

```
Arg Met Ile Leu Met Thr His Phe Phe
1               5
```

<210> 98

<211> 9

<212> PRT

<213> hepatitis C virus

<400> 98

```
Thr Val Leu Asp Gln Ala Glu Thr Ala
1               5
```

<210> 99

<211> 9

<212> PRT

<213> hepatitis C virus

<400> 99

```
Leu Thr His Pro Ile Thr Lys Tyr Ile
1               5
```

<210> 100

<211> 9

<212> PRT

<213> hepatitis C virus


<400> 100

```
Val Leu Asn Pro Ser Val Ala Ala Thr
1               5
```

<210> 101

<211> 9

<212> PRT

<213> hepatitis C virus


<400> 101

```
Phe Thr Asp Asn Ser Ser Pro Pro Ala
1               5
```

<210> 102

<211> 9

<212> PRT

<213> hepatitis C virus


<400> 102

```
Val Leu Ala Thr Ala Thr Pro Pro Gly
1               5
```

<210> 103

<211> 9

<212> PRT

<213> hepatitis C virus


<400> 103

```
Val Leu Val Leu Asn Pro Ser Val Ala
1               5
```

<210> 104

<211> 9

<212> PRT

<213> hepatitis C virus

<400> 104

Leu Leu Cys Pro Ser Gly His Val Val
1               5

<210> 105

<211> 9

<212> PRT

<213> hepatitis C virus

<400> 105

Gly Leu Asp Val Ser Val Ile Pro Thr
1               5

<210> 106

<211> 9

<212> PRT

<213> hepatitis C virus

<400> 106

Phe Ser Leu Asp Pro Thr Phe Thr Ile
1               5

<210> 107

<211> 9

<212> PRT

<213> hepatitis C virus

<400> 107

Ala Thr Leu Gly Phe Gly Ala Tyr Met
1               5

<210> 108

<211> 9

<212> PRT

<213> hepatitis C virus

<400> 108

Tyr Ala Pro Thr Leu Trp Ala Arg Met
1               5

<210> 109

<211> 9

<212> PRT

<213> hepatitis C virus


<400> 109

Thr Ile Thr Thr Gly Ala Pro Ile Thr
1               5

<210> 110

<211> 9

<212> PRT

<213> hepatitis C virus


<400> 110

Thr Thr Met Arg Ser Pro Val Phe Thr
1               5

<210> 111

<211> 9

<212> PRT

<213> hepatitis C virus


<400> 111

Phe Gln Tyr Ser Pro Gly Gln Arg Val
1               5

<210> 112

<211> 9

<212> PRT

<213> hepatitis C virus


<400> 112

Gln Val Ala His Leu His Ala Pro Thr
1               5

```
<210>  113
<211>  9
<212>  PRT
<213>  hepatitis C virus


<400>  113
Ala Val Pro Gln Thr Phe Gln Val Ala
1               5


<210>  114
<211>  9
<212>  PRT
<213>  hepatitis C virus


<400>  114
Arg Thr Ile Thr Thr Gly Ala Pro Ile
1               5


<210>  115
<211>  9
<212>  PRT
<213>  hepatitis C virus


<400>  115
Ala Ala Gln Gly Tyr Lys Val Leu Val
1               5


<210>  116
<211>  9
<212>  PRT
<213>  hepatitis C virus


<400>  116
Leu Val Ala Tyr Gln Ala Thr Val Cys
1               5


<210>  117
<211>  9
```

<212> PRT

<213> hepatitis C virus


<400> 117

Gly Val Phe Arg Ala Ala Val Cys Thr
1               5


<210> 118

<211> 9

<212> PRT

<213> hepatitis C virus


<400> 118

Leu Met Gly Tyr Ile Pro Leu Val Gly
1               5


<210> 119

<211> 9

<212> PRT

<213> hepatitis C virus


<400> 119

Ala Val Gln Asn Glu Val Thr Leu Thr
1               5


<210> 120

<211> 9

<212> PRT

<213> hepatitis C virus


<400> 120

Gly Ile Tyr Arg Phe Val Thr Pro Gly
1               5


<210> 121

<211> 9

<212> PRT

<213> hepatitis C virus

<400> 121

Ser Ala Ala Cys Arg Ala Ala Lys Leu
1               5

<210> 122

<211> 9

<212> PRT

<213> hepatitis C virus


<400> 122

Cys Leu Ile Arg Leu Lys Pro Thr Leu
1               5

<210> 123

<211> 9

<212> PRT

<213> hepatitis C virus


<400> 123

Phe Leu Ser Gln Thr Lys Gln Ala Gly
1               5

<210> 124

<211> 9

<212> PRT

<213> hepatitis C virus


<400> 124

Ser Val Ile Asp Cys Asn Thr Cys Val
1               5

<210> 125

<211> 9

<212> PRT

<213> hepatitis C virus


<400> 125

Ala Thr Pro Pro Gly Ser Val Thr Val
1               5

```
<210>  126
<211>  9
<212>  PRT
<213>  hepatitis C virus


<400>  126
Ala Ala Trp Glu Thr Ala Arg His Thr
1               5


<210>  127
<211>  9
<212>  PRT
<213>  hepatitis C virus


<400>  127
Gly Gln Ile Val Gly Gly Val Tyr Leu
1               5


<210>  128
<211>  9
<212>  PRT
<213>  hepatitis C virus


<400>  128
Val Leu Glu Asp Gly Val Asn Tyr Ala
1               5


<210>  129
<211>  9
<212>  PRT
<213>  hepatitis C virus


<400>  129
Leu Glu Phe Trp Glu Ser Val Phe Thr
1               5


<210>  130
<211>  9
<212>  PRT
```

```
<213>  hepatitis C virus


<400>  130

Ala Gln Gly Tyr Lys Val Leu Val Leu
1               5


<210>  131

<211>  9

<212>  PRT

<213>  hepatitis C virus


<400>  131

Leu Val Asn Gly Asp Asp Leu Val Val
1               5


<210>  132

<211>  9

<212>  PRT

<213>  hepatitis C virus


<400>  132

Leu Leu Pro Arg Arg Gly Pro Arg Leu
1               5


<210>  133

<211>  9

<212>  PRT

<213>  hepatitis C virus


<400>  133

Ser Thr Leu Pro Gln Ala Val Met Gly
1               5


<210>  134

<211>  9

<212>  PRT

<213>  hepatitis C virus


<400>  134
```

Val Ile Pro Thr Ser Gly Asp Val Val
1               5

<210> 135

<211> 9

<212> PRT

<213> hepatitis C virus


<400> 135

Ser Gly Met Phe Asp Ser Ser Val Leu
1               5

<210> 136

<211> 9

<212> PRT

<213> hepatitis C virus


<400> 136

Cys Met Ser Ala Asp Leu Glu Val Val
1               5

<210> 137

<211> 9

<212> PRT

<213> hepatitis C virus


<400> 137

Tyr Gly Lys Ala Ile Pro Ile Glu Val
1               5

<210> 138

<211> 9

<212> PRT

<213> hepatitis C virus


<400> 138

Met Ser Ala Asp Leu Glu Val Val Thr
1               5

<210> 139

<211> 9

<212> PRT

<213> hepatitis C virus

<400> 139

Gly Asn Ile Ile Met Tyr Ala Pro Thr
1               5

<210> 140

<211> 9

<212> PRT

<213> hepatitis C virus

<400> 140

Gly Ile Gly Thr Val Leu Asp Gln Ala
1               5

<210> 141

<211> 9

<212> PRT

<213> hepatitis C virus

<400> 141

His Val Val Gly Val Phe Arg Ala Ala
1               5

<210> 142

<211> 9

<212> PRT

<213> hepatitis C virus

<400> 142

Lys Leu Ser Ala Leu Gly Leu Asn Ala
1               5

<210> 143

<211> 9

<212> PRT

<213> hepatitis C virus

<400> 143

Ala Ile Pro Ile Glu Val Ile Lys Gly
1               5

<210> 144

<211> 9

<212> PRT

<213> hepatitis C virus

<400> 144

Arg Leu Gly Val Arg Ala Thr Arg Lys
1               5

<210> 145

<211> 9

<212> PRT

<213> hepatitis C virus

<400> 145

Gly Leu Asn Ala Val Ala Tyr Tyr Arg
1               5

<210> 146

<211> 9

<212> PRT

<213> hepatitis C virus

<400> 146

Leu Val Asn Ala Trp Lys Ser Lys Lys
1               5

<210> 147

<211> 9

<212> PRT

<213> hepatitis C virus

<400> 147

Ala Ala Tyr Ala Ala Gln Gly Tyr Lys
1               5

<210> 148

<211> 9

<212> PRT

<213> hepatitis C virus


<400> 148

His Leu Ile Phe Cys His Ser Lys Lys
1               5


<210> 149

<211> 9

<212> PRT

<213> hepatitis C virus


<400> 149

Tyr Leu Leu Pro Arg Arg Gly Pro Arg
1               5


<210> 150

<211> 9

<212> PRT

<213> hepatitis C virus


<400> 150

Phe Leu Val Asn Ala Trp Lys Ser Lys
1               5


<210> 151

<211> 9

<212> PRT

<213> hepatitis C virus


<400> 151

Leu Ile Phe Cys His Ser Lys Lys Lys
1               5


<210> 152

<211> 9

<212> PRT

<213> hepatitis C virus


<400> 152

Val Thr Leu Thr His Pro Ile Thr Lys
1               5


<210> 153

<211> 9

<212> PRT

<213> hepatitis C virus


<400> 153

Ala Leu Gly Leu Asn Ala Val Ala Tyr
1               5


<210> 154

<211> 9

<212> PRT

<213> hepatitis C virus


<400> 154

Asp Leu Gly Val Arg Val Cys Glu Lys
1               5


<210> 155

<211> 9

<212> PRT

<213> hepatitis C virus


<400> 155

Ala Ser Ala Ala Cys Arg Ala Ala Lys
1               5


<210> 156

<211> 9

<212> PRT

<213> hepatitis C virus

<400> 156

Ala Val Cys Thr Arg Gly Val Ala Lys
1               5

<210> 157

<211> 9

<212> PRT

<213> hepatitis C virus

<400> 157

Val Gln Pro Glu Lys Gly Gly Arg Lys
1               5

<210> 158

<211> 9

<212> PRT

<213> hepatitis C virus

<400> 158

Ser Thr Asn Pro Lys Pro Gln Arg Lys
1               5

<210> 159

<211> 9

<212> PRT

<213> hepatitis C virus

<400> 159

Arg Gln Pro Ile Pro Lys Ala Arg Arg
1               5

<210> 160

<211> 9

<212> PRT

<213> hepatitis C virus

<400> 160

Arg Val Phe Thr Glu Ala Met Thr Arg
1               5

```
<210>  161
<211>  9
<212>  PRT
<213>  hepatitis C virus


<400>  161
Tyr Leu Lys Ala Ser Ala Ala Cys Arg
1               5


<210>  162
<211>  9
<212>  PRT
<213>  hepatitis C virus


<400>  162
Gly Asn Thr Leu Thr Cys Tyr Leu Lys
1               5


<210>  163
<211>  9
<212>  PRT
<213>  hepatitis C virus


<400>  163
Trp Leu Leu Ser Pro Arg Gly Ser Arg
1               5


<210>  164
<211>  9
<212>  PRT
<213>  hepatitis C virus


<400>  164
Lys Thr Lys Arg Asn Thr Asn Arg Arg
1               5


<210>  165
<211>  9
<212>  PRT
```

<213>  hepatitis C virus


<400>  165

Ala Leu Tyr Asp Val Val Ser Thr Leu
1               5


<210>  166

<211>  9

<212>  PRT

<213>  hepatitis C virus


<400>  166

Leu Gly Phe Gly Ala Tyr Met Ser Lys
1               5


<210>  167

<211>  9

<212>  PRT

<213>  hepatitis C virus


<400>  167

Lys Thr Ser Glu Arg Ser Gln Pro Arg
1               5


<210>  168

<211>  9

<212>  PRT

<213>  hepatitis C virus


<400>  168

Ile Val Phe Pro Asp Leu Gly Val Arg
1               5


<210>  169

<211>  9

<212>  PRT

<213>  hepatitis C virus


<400>  169

Arg Val Leu Glu Asp Gly Val Asn Tyr
1               5

<210>  170

<211>  9

<212>  PRT

<213>  hepatitis C virus


<400>  170

Ile Met Tyr Ala Pro Thr Leu Trp Ala
1               5

<210>  171

<211>  9

<212>  PRT

<213>  hepatitis C virus


<400>  171

Gly Ala Pro Ile Thr Tyr Ser Thr Tyr
1               5

<210>  172

<211>  9

<212>  PRT

<213>  hepatitis C virus


<400>  172

Gly Lys Arg Val Tyr Tyr Leu Thr Arg
1               5

<210>  173

<211>  9

<212>  PRT

<213>  hepatitis C virus


<400>  173

Arg His Leu Ile Phe Cys His Ser Lys
1               5

<210>  174

<211> 9

<212> PRT

<213> hepatitis C virus

<400> 174

Gly Arg Gly Arg Arg Gly Ile Tyr Arg
1               5

<210> 175

<211> 9

<212> PRT

<213> hepatitis C virus

<400> 175

Arg Leu Tyr Ile Gly Gly Pro Leu Thr
1               5

<210> 176

<211> 9

<212> PRT

<213> hepatitis C virus

<400> 176

Pro Met Gly Phe Ser Tyr Asp Thr Arg
1               5

<210> 177

<211> 9

<212> PRT

<213> hepatitis C virus

<400> 177

Arg Gly Arg Arg Gln Pro Ile Pro Lys
1               5

<210> 178

<211> 9

<212> PRT

<213> hepatitis C virus

<400> 178

Val Glu Phe Leu Val Asn Ala Trp Lys
1                   5


<210> 179

<211> 9

<212> PRT

<213> hepatitis C virus


<400> 179

Gly Met Phe Asp Ser Ser Val Leu Cys
1                   5


<210> 180

<211> 9

<212> PRT

<213> hepatitis C virus


<400> 180

Asp Gln Met Trp Lys Cys Leu Ile Arg
1                   5


<210> 181

<211> 9

<212> PRT

<213> hepatitis C virus


<400> 181

Lys Ala Ile Pro Ile Glu Val Ile Lys
1                   5


<210> 182

<211> 9

<212> PRT

<213> hepatitis C virus


<400> 182

Tyr Leu Asn Thr Pro Gly Leu Pro Val
1                   5

<210> 183

<211> 9

<212> PRT

<213> hepatitis C virus


<400> 183

Arg Val Cys Glu Lys Met Ala Leu Tyr
1               5


<210> 184

<211> 9

<212> PRT

<213> hepatitis C virus


<400> 184

Trp Ala Gly Trp Leu Leu Ser Pro Arg
1               5


<210> 185

<211> 9

<212> PRT

<213> hepatitis C virus


<400> 185

Tyr Leu Val Ala Tyr Gln Ala Thr Val
1               5


<210> 186

<211> 9

<212> PRT

<213> hepatitis C virus


<400> 186

Gly Leu Gly Trp Ala Gly Trp Leu Leu
1               5


<210> 187

<211> 9

<212> PRT

<213> hepatitis C virus

<400> 187

Met Trp Lys Cys Leu Ile Arg Leu Lys
1              5

<210> 188

<211> 9

<212> PRT

<213> hepatitis C virus

<400> 188

Ser Val Ala His Asp Ala Ser Gly Lys
1              5

<210> 189

<211> 9

<212> PRT

<213> hepatitis C virus

<400> 189

Arg Ala Thr Arg Lys Thr Ser Glu Arg
1              5

<210> 190

<211> 9

<212> PRT

<213> hepatitis C virus

<400> 190

Trp Leu Gly Asn Ile Ile Met Tyr Ala
1              5

<210> 191

<211> 9

<212> PRT

<213> hepatitis C virus

<400> 191

Lys Ala Ile Pro Ile Glu Ala Ile Lys
1               5

<210> 192

<211> 9

<212> PRT

<213> hepatitis C virus

<400> 192

His Gly Pro Thr Pro Leu Leu Tyr Arg
1               5

<210> 193

<211> 9

<212> PRT

<213> hepatitis C virus

<400> 193

Glu Val Phe Cys Val Gln Pro Glu Lys
1               5

<210> 194

<211> 9

<212> PRT

<213> hepatitis C virus

<400> 194

Met Ser Thr Asn Pro Lys Pro Gln Arg
1               5

<210> 195

<211> 9

<212> PRT

<213> hepatitis C virus

<400> 195

Leu His Ala Pro Thr Gly Ser Gly Lys
1               5

<210> 196

<211> 9

<212> PRT

<213> hepatitis C virus


<400> 196

Val Ser Arg Ser Gln Arg Arg Gly Arg
1               5


<210> 197

<211> 9

<212> PRT

<213> hepatitis C virus


<400> 197

Val Gly Gly Val Tyr Leu Leu Pro Arg
1               5


<210> 198

<211> 9

<212> PRT

<213> hepatitis C virus


<400> 198

Gly Val Phe Arg Ala Ala Val Cys Thr
1               5


<210> 199

<211> 9

<212> PRT

<213> hepatitis C virus


<400> 199

Thr Asn Arg Arg Pro Gln Asp Val Lys
1               5


<210> 200

<211> 9

<212> PRT

<213> hepatitis C virus

<400> 200

Leu Leu Tyr Arg Leu Gly Ala Val Gln
1               5

<210> 201
<211> 9
<212> PRT
<213> hepatitis C virus

<400> 201

Thr Leu Thr His Pro Ile Thr Lys Tyr
1               5

<210> 202
<211> 9
<212> PRT
<213> hepatitis C virus

<400> 202

Gly Leu Asp Val Ser Val Ile Pro Thr
1               5

<210> 203
<211> 9
<212> PRT
<213> hepatitis C virus

<400> 203

Pro Ser Trp Gly Pro Thr Asp Pro Arg
1               5

<210> 204
<211> 9
<212> PRT
<213> hepatitis C virus

<400> 204

Val Val Gly Val Phe Arg Ala Ala Val
1             5

<210> 205

<211> 9

<212> PRT

<213> hepatitis C virus


<400> 205

Leu Ile Arg Leu Lys Pro Thr Leu His
1             5

<210> 206

<211> 9

<212> PRT

<213> hepatitis C virus


<400> 206

Gly Val Arg Ala Thr Arg Lys Thr Ser
1             5

<210> 207

<211> 9

<212> PRT

<213> hepatitis C virus


<400> 207

Gly Ala Pro Leu Gly Gly Ala Ala Arg
1             5

<210> 208

<211> 9

<212> PRT

<213> hepatitis C virus


<400> 208

Asp Leu Met Gly Tyr Ile Pro Leu Val
1             5

<210> 209

<211> 9

<212> PRT

<213> hepatitis C virus

<400> 209

Gln Thr Phe Gln Val Ala His Leu His
1               5

<210> 210

<211> 9

<212> PRT

<213> hepatitis C virus

<400> 210

Ala Thr Asp Ala Leu Met Thr Gly Tyr
1               5

<210> 211

<211> 9

<212> PRT

<213> hepatitis C virus

<400> 211

Lys Gln Ala Gly Asp Asn Phe Pro Tyr
1               5

<210> 212

<211> 9

<212> PRT

<213> hepatitis C virus

<400> 212

Asp Asn Phe Pro Tyr Leu Val Ala Tyr
1               5

<210> 213

<211> 9

<212> PRT

<213> hepatitis C virus

<400> 213

Leu Leu Pro Arg Arg Gly Pro Arg Leu
1                5

<210> 214

<211> 9

<212> PRT

<213> hepatitis C virus


<400> 214

Pro Thr Gly Ser Gly Lys Ser Thr Lys
1                5

<210> 215

<211> 9

<212> PRT

<213> hepatitis C virus


<400> 215

Ile Thr Tyr Ser Thr Tyr Gly Lys Phe
1                5

<210> 216

<211> 9

<212> PRT

<213> hepatitis C virus


<400> 216

Gln Pro Gly Tyr Pro Trp Pro Leu Tyr
1                5

<210> 217

<211> 9

<212> PRT

<213> hepatitis C virus


<400> 217

Ala Met Thr Arg Tyr Ser Ala Pro Pro
1                5

<210> 218

<211> 9

<212> PRT

<213> hepatitis C virus


<400> 218

Gly Ile Gly Thr Val Leu Asp Gln Ala
1               5


<210> 219

<211> 9

<212> PRT

<213> hepatitis C virus


<400> 219

Leu His Gly Pro Thr Pro Leu Leu Tyr
1               5


<210> 220

<211> 9

<212> PRT

<213> hepatitis C virus


<400> 220

Leu Thr Pro Ala Glu Thr Ser Val Arg
1               5


<210> 221

<211> 9

<212> PRT

<213> hepatitis C virus


<400> 221

Ser Gln Arg Arg Gly Arg Thr Gly Arg
1               5


<210> 222

<211> 9

```
<212>  PRT
<213>  hepatitis C virus


<400>  222

Lys Ser Thr Lys Val Pro Ala Ala Tyr
1               5


<210>  223
<211>  9
<212>  PRT
<213>  hepatitis C virus


<400>  223

Ile Val Gly Gly Val Tyr Leu Leu Pro
1               5


<210>  224
<211>  9
<212>  PRT
<213>  hepatitis C virus


<400>  224

Arg Thr Gly Arg Gly Arg Arg Gly Ile
1               5


<210>  225
<211>  9
<212>  PRT
<213>  hepatitis C virus


<400>  225

Lys Leu Ser Ala Leu Gly Leu Asn Ala
1               5


<210>  226
<211>  9
<212>  PRT
<213>  hepatitis C virus
```

<400> 226

Phe Gln Tyr Ser Pro Gly Gln Arg Val
1               5

<210> 227

<211> 9

<212> PRT

<213> hepatitis C virus


<400> 227

Arg Thr Trp Ala Gln Pro Gly Tyr Pro
1               5

<210> 228

<211> 9

<212> PRT

<213> hepatitis C virus


<400> 228

Gln Asn Cys Gly Tyr Arg Arg Cys Arg
1               5

<210> 229

<211> 9

<212> PRT

<213> hepatitis C virus


<400> 229

Asp Ser Ser Val Leu Cys Glu Cys Tyr
1               5

<210> 230

<211> 9

<212> PRT

<213> hepatitis C virus


<400> 230

Arg Met Ile Leu Met Thr His Phe Phe
1               5

<210> 231

<211> 9

<212> PRT

<213> hepatitis C virus


<400> 231

Thr Leu Trp Ala Arg Met Ile Leu Met
1               5


<210> 232

<211> 9

<212> PRT

<213> hepatitis C virus


<400> 232

Cys Leu Ile Arg Leu Lys Pro Thr Leu
1               5


<210> 233

<211> 9

<212> PRT

<213> hepatitis C virus


<400> 233

Thr Leu His Gly Pro Thr Pro Leu Leu
1               5


<210> 234

<211> 9

<212> PRT

<213> hepatitis C virus


<400> 234

Phe Trp Glu Ser Val Phe Thr Gly Leu
1               5


<210> 235

<211> 9

<212> PRT

<213> hepatitis C virus

<400> 235

Thr Trp Ala Gln Pro Gly Tyr Pro Trp
1               5

<210> 236

<211> 9

<212> PRT

<213> hepatitis C virus

<400> 236

Gly Phe Ala Asp Leu Met Gly Tyr Ile
1               5

<210> 237

<211> 9

<212> PRT

<213> hepatitis C virus

<400> 237

Asn Ile Ile Met Tyr Ala Pro Thr Leu
1               5

<210> 238

<211> 9

<212> PRT

<213> hepatitis C virus

<400> 238

Gln Met Trp Lys Cys Leu Ile Arg Leu
1               5

<210> 239

<211> 9

<212> PRT

<213> hepatitis C virus

<400> 239

Lys Tyr Ile Met Ala Cys Met Ser Ala
1               5

<210> 240

<211> 9

<212> PRT

<213> hepatitis C virus


<400> 240

Ala Gln Gly Tyr Lys Val Leu Val Leu
1               5

<210> 241

<211> 9

<212> PRT

<213> hepatitis C virus


<400> 241

Thr Tyr Ser Thr Tyr Gly Lys Phe Leu
1               5

<210> 242

<211> 9

<212> PRT

<213> hepatitis C virus


<400> 242

Lys Ala His Gly Val Asp Pro Asn Ile
1               5

<210> 243

<211> 9

<212> PRT

<213> hepatitis C virus


<400> 243

Leu Tyr Gly Asn Glu Gly Leu Gly Trp
1               5

<210> 244

<211> 9

<212> PRT

<213> hepatitis C virus

<400> 244

Ala Tyr Met Ser Lys Ala His Gly Val
1               5

<210> 245

<211> 9

<212> PRT

<213> hepatitis C virus

<400> 245

Gly Leu Gly Trp Ala Gly Trp Leu Leu
1               5

<210> 246

<211> 9

<212> PRT

<213> hepatitis C virus

<400> 246

Ile Ile Met Tyr Ala Pro Thr Leu Trp
1               5

<210> 247

<211> 9

<212> PRT

<213> hepatitis C virus

<400> 247

Gly Gln Ile Val Gly Gly Val Tyr Leu
1               5

<210> 248

<211> 9

<212> PRT

<213> hepatitis C virus

```
<400>  248

Trp Leu Gly Asn Ile Ile Met Tyr Ala
1               5


<210>  249

<211>  9

<212>  PRT

<213>  hepatitis C virus



<400>  249

Thr Ala Gly Ala Arg Leu Val Val Leu
1               5


<210>  250

<211>  9

<212>  PRT

<213>  hepatitis C virus



<400>  250

Ser Phe Ser Ile Phe Leu Leu Ala Leu
1               5


<210>  251

<211>  9

<212>  PRT

<213>  hepatitis C virus



<400>  251

Phe Ser Leu Asp Pro Thr Phe Thr Ile
1               5


<210>  252

<211>  9

<212>  PRT

<213>  hepatitis C virus



<400>  252

Tyr Leu Val Ala Tyr Gln Ala Thr Val
1               5
```

<210> 253

<211> 9

<212> PRT

<213> hepatitis C virus


<400> 253

Val Ile Lys Gly Gly Arg His Leu Ile
1               5


<210> 254

<211> 9

<212> PRT

<213> hepatitis C virus


<400> 254

Pro Leu Leu Tyr Arg Leu Gly Ala Val
1               5


<210> 255

<211> 9

<212> PRT

<213> hepatitis C virus


<400> 255

Thr Ile Leu Gly Ile Gly Thr Val Leu
1               5


<210> 256

<211> 9

<212> PRT

<213> hepatitis C virus


<400> 256

Pro Val Asn Ser Trp Leu Gly Asn Ile
1               5


<210> 257

<211> 9

<212> PRT

<213> hepatitis C virus

<400> 257

Glu Thr Thr Met Arg Ser Pro Val Phe
1               5

<210> 258

<211> 9

<212> PRT

<213> hepatitis C virus

<400> 258

Gly Leu Thr His Ile Asp Ala His Phe
1               5

<210> 259

<211> 9

<212> PRT

<213> hepatitis C virus

<400> 259

Ala Val Met Gly Ser Ser Tyr Gly Phe
1               5

<210> 260

<211> 9

<212> PRT

<213> hepatitis C virus

<400> 260

Lys Gly Ser Ser Gly Gly Pro Leu Leu
1               5

<210> 261

<211> 9

<212> PRT

<213> hepatitis C virus

<400> 261

Lys Leu Gln Asp Cys Thr Met Leu Val
1               5

<210> 262

<211> 9

<212> PRT

<213> hepatitis C virus


<400> 262

Tyr Ala Ala Gln Gly Tyr Lys Val Leu
1               5

<210> 263

<211> 9

<212> PRT

<213> hepatitis C virus


<400> 263

Leu Thr His Pro Ile Thr Lys Tyr Ile
1               5

<210> 264

<211> 9

<212> PRT

<213> hepatitis C virus


<400> 264

Pro Phe Tyr Gly Lys Ala Ile Pro Ile
1               5

<210> 265

<211> 9

<212> PRT

<213> hepatitis C virus


<400> 265

Arg Leu Gly Ala Val Gln Asn Glu Val
1               5

<210> 266

<211> 9

<212> PRT

<213> hepatitis C virus

<400> 266

Ala Ile Lys Gly Gly Arg His Leu Ile
1               5

<210> 267

<211> 9

<212> PRT

<213> hepatitis C virus

<400> 267

Ala Leu Tyr Asp Val Val Ser Thr Leu
1               5

<210> 268

<211> 9

<212> PRT

<213> hepatitis C virus

<400> 268

Arg Ala Leu Ala His Gly Val Arg Val
1               5

<210> 269

<211> 9

<212> PRT

<213> hepatitis C virus

<400> 269

Tyr Ile Pro Leu Val Gly Ala Pro Leu
1               5

<210> 270

<211> 9

<212> PRT

<213> hepatitis C virus

<400> 270

Leu Leu Pro Arg Arg Gly Pro Arg Leu
1               5

<210> 271

<211> 9

<212> PRT

<213> hepatitis C virus

<400> 271

Tyr Tyr Arg Gly Leu Asp Val Ser Val
1               5

<210> 272

<211> 9

<212> PRT

<213> hepatitis C virus

<400> 272

Glu Leu Ala Ala Lys Leu Ser Ala Leu
1               5

<210> 273

<211> 9

<212> PRT

<213> hepatitis C virus

<400> 273

Tyr Gly Lys Ala Ile Pro Ile Glu Val
1               5

<210> 274

<211> 9

<212> PRT

<213> hepatitis C virus

<400> 274

Met Gly Ser Ser Tyr Gly Phe Gln Tyr
1               5

<210>  275

<211>  9

<212>  PRT

<213>  hepatitis C virus


<400>  275

Leu Leu Cys Pro Ser Gly His Val Val
1               5

<210>  276

<211>  9

<212>  PRT

<213>  hepatitis C virus


<400>  276

Ala Trp Lys Ser Lys Lys Cys Pro Met
1               5

<210>  277

<211>  9

<212>  PRT

<213>  hepatitis C virus


<400>  277

Ala Tyr Ala Ala Gln Gly Tyr Lys Val
1               5

<210>  278

<211>  9

<212>  PRT

<213>  hepatitis C virus


<400>  278

Arg Val Glu Phe Leu Val Asn Ala Trp
1               5

<210>  279

```
<211>  9
<212>  PRT
<213>  hepatitis C virus


<400>  279
Ser Trp Asp Gln Met Trp Lys Cys Leu
1               5


<210>  280
<211>  9
<212>  PRT
<213>  hepatitis C virus


<400>  280
Asn Leu Pro Gly Cys Ser Phe Ser Ile
1               5


<210>  281
<211>  9
<212>  PRT
<213>  hepatitis C virus


<400>  281
Gly Phe Ser Tyr Asp Thr Arg Cys Phe
1               5


<210>  282
<211>  9
<212>  PRT
<213>  hepatitis C virus


<400>  282
Pro Ala Val Pro Gln Thr Phe Gln Val
1               5


<210>  283
<211>  9
<212>  PRT
<213>  hepatitis C virus
```

<400> 283

Asn Leu Gly Lys Val Ile Asp Thr Leu
1     5


<210> 284

<211> 9

<212> PRT

<213> hepatitis C virus


<400> 284

Lys Phe Pro Gly Gly Gly Gln Ile Val
1     5


<210> 285

<211> 9

<212> PRT

<213> hepatitis C virus


<400> 285

Tyr Leu Asn Thr Pro Gly Leu Pro Val
1     5


<210> 286

<211> 9

<212> PRT

<213> hepatitis C virus


<400> 286

Trp Asp Gln Met Trp Lys Cys Leu Ile
1     5


<210> 287

<211> 9

<212> PRT

<213> hepatitis C virus


<400> 287

Trp Ala Arg Met Ile Leu Met Thr His
1     5

<210> 288

<211> 9

<212> PRT

<213> hepatitis C virus


<400> 288

Ala Gln Pro Gly Tyr Pro Trp Pro Leu
1               5


<210> 289

<211> 9

<212> PRT

<213> hepatitis C virus


<400> 289

Gln Ile Val Gly Gly Val Tyr Leu Leu
1               5


<210> 290

<211> 9

<212> PRT

<213> hepatitis C virus


<400> 290

Tyr Ser Thr Tyr Gly Lys Phe Leu Ala
1               5


<210> 291

<211> 9

<212> PRT

<213> hepatitis C virus


<400> 291

Gly Met Phe Asp Ser Ser Val Leu Cys
1               5


<210> 292

<211> 9

```
<212>  PRT
<213>  hepatitis C virus


<400>  292
Met Tyr Ala Pro Thr Leu Trp Ala Arg
1               5


<210>  293
<211>  9
<212>  PRT
<213>  hepatitis C virus


<400>  293
Cys Ser Phe Ser Ile Phe Leu Leu Ala
1               5


<210>  294
<211>  9
<212>  PRT
<213>  hepatitis C virus


<400>  294
Gly Cys Ser Phe Ser Ile Phe Leu Leu
1               5


<210>  295
<211>  9
<212>  PRT
<213>  hepatitis C virus


<400>  295
Gly Val Ala Lys Ala Val Asp Phe Ile
1               5


<210>  296
<211>  9
<212>  PRT
<213>  hepatitis C virus
```

<400> 296

Phe Gln Tyr Ser Pro Gly Gln Arg Val
1               5

<210> 297

<211> 9

<212> PRT

<213> hepatitis C virus

<400> 297

Ala Leu Ala His Gly Val Arg Val Leu
1               5

<210> 298

<211> 9

<212> PRT

<213> hepatitis C virus

<400> 298

Arg His Thr Pro Val Asn Ser Trp Leu
1               5

<210> 299

<211> 9

<212> PRT

<213> hepatitis C virus

<400> 299

Pro Thr Leu Trp Ala Arg Met Ile Leu
1               5

<210> 300

<211> 9

<212> PRT

<213> hepatitis C virus

<400> 300

Arg Gly Arg Arg Gly Ile Tyr Arg Phe
1               5

<210> 301

<211> 9

<212> PRT

<213> hepatitis C virus


<400> 301

Lys Ser Lys Lys Cys Pro Met Gly Phe
1               5


<210> 302

<211> 9

<212> PRT

<213> hepatitis C virus


<400> 302

Leu Ala Leu Leu Ser Cys Leu Thr Ile
1               5


<210> 303

<211> 9

<212> PRT

<213> hepatitis C virus


<400> 303

Ser Val Thr Val Pro His Pro Asn Ile
1               5


<210> 304

<211> 9

<212> PRT

<213> hepatitis C virus


<400> 304

Leu Thr Thr Ser Cys Gly Asn Thr Leu
1               5


<210> 305

<211> 9

<212> PRT

<213> hepatitis C virus

<400> 305

Ile Thr Lys Tyr Ile Met Ala Cys Met
1               5

<210> 306

<211> 9

<212> PRT

<213> hepatitis C virus

<400> 306

Phe Tyr Gly Lys Ala Ile Pro Ile Glu
1               5

<210> 307

<211> 9

<212> PRT

<213> hepatitis C virus

<400> 307

Gly Pro Thr Pro Leu Leu Tyr Arg Leu
1               5

<210> 308

<211> 9

<212> PRT

<213> hepatitis C virus

<400> 308

Leu Met Thr Gly Tyr Thr Gly Asp Phe
1               5

<210> 309

<211> 9

<212> PRT

<213> hepatitis C virus

<400> 309

Arg Val Cys Glu Lys Met Ala Leu Tyr
1               5

<210> 310

<211> 9

<212> PRT

<213> hepatitis C virus


<400> 310

Asp Leu Met Gly Tyr Ile Pro Leu Val
1               5

<210> 311

<211> 9

<212> PRT

<213> hepatitis C virus


<400> 311

Ile Lys Gly Gly Arg His Leu Ile Phe
1               5

<210> 312

<211> 9

<212> PRT

<213> hepatitis C virus


<400> 312

Pro Gln Thr Phe Gln Val Ala His Leu
1               5

<210> 313

<211> 9

<212> PRT

<213> hepatitis C virus


<400> 313

Tyr Tyr Leu Thr Arg Asp Pro Thr Thr
1               5

<210> 314

<211> 9

<212> PRT

<213> hepatitis C virus


<400> 314

Leu Trp Ala Arg Met Ile Leu Met Thr
1               5


<210> 315

<211> 9

<212> PRT

<213> hepatitis C virus


<400> 315

Lys Val Leu Val Leu Asn Pro Ser Val
1               5


<210> 316

<211> 9

<212> PRT

<213> hepatitis C virus


<400> 316

Arg Thr Ile Thr Thr Gly Ala Pro Ile
1               5


<210> 317

<211> 9

<212> PRT

<213> hepatitis C virus


<400> 317

Arg Gly Val Ala Lys Ala Val Asp Phe
1               5


<210> 318

<211> 9

<212> PRT

<213> hepatitis C virus

<400> 318

Arg Ser Arg Asn Leu Gly Lys Val Ile
1               5

<210> 319

<211> 9

<212> PRT

<213> hepatitis C virus


<400> 319

Arg Leu Tyr Ile Gly Gly Pro Leu Thr
1               5

<210> 320

<211> 9

<212> PRT

<213> hepatitis C virus


<400> 320

Cys Tyr Leu Lys Ala Ser Ala Ala Cys
1               5

<210> 321

<211> 9

<212> PRT

<213> hepatitis C virus


<400> 321

Trp Tyr Glu Leu Thr Pro Ala Glu Thr
1               5

<210> 322

<211> 9

<212> PRT

<213> hepatitis C virus


<400> 322

Leu Thr His Ile Asp Ala His Phe Leu
1               5

<210> 323

<211> 9

<212> PRT

<213> hepatitis C virus

<400> 323

Pro Gly Gln Arg Val Glu Phe Leu Val
1               5

<210> 324

<211> 9

<212> PRT

<213> hepatitis C virus

<400> 324

Lys Leu Ser Ala Leu Gly Leu Asn Ala
1               5

<210> 325

<211> 9

<212> PRT

<213> hepatitis C virus

<400> 325

Thr His Ile Asp Ala His Phe Leu Ser
1               5

<210> 326

<211> 9

<212> PRT

<213> hepatitis C virus

<400> 326

Tyr Ser Pro Gly Gln Arg Val Glu Phe
1               5

<210> 327

<211> 9

<212> PRT

<213> hepatitis C virus

<400> 327

Ala Gly Asp Asn Phe Pro Tyr Leu Val

<210> 328

<211> 9

<212> PRT

<213> hepatitis C virus

<400> 328

Ile Met Ala Cys Met Ser Ala Asp Leu

<210> 329

<211> 9

<212> PRT

<213> hepatitis C virus

<400> 329

Pro Val Cys Gln Asp His Leu Glu Phe

<210> 330

<211> 9

<212> PRT

<213> hepatitis C virus

<400> 330

Ala Ala Gln Gly Tyr Lys Val Leu Val

<210> 331

<211> 9

<212> PRT

<213> hepatitis C virus

```
<400>  331

Ile Gly Leu Ser Asn Asn Gly Glu Ile
1               5


<210>  332

<211>  9

<212>  PRT

<213>  hepatitis C virus



<400>  332

Arg Ser Leu Thr Glu Arg Leu Tyr Ile
1               5


<210>  333

<211>  9

<212>  PRT

<213>  hepatitis C virus



<400>  333

Cys Met Ser Ala Asp Leu Glu Val Val
1               5


<210>  334

<211>  9

<212>  PRT

<213>  hepatitis C virus



<400>  334

Ala Ser Gly Lys Arg Val Tyr Tyr Leu
1               5


<210>  335

<211>  9

<212>  PRT

<213>  hepatitis C virus



<400>  335

Tyr Gly Lys Ala Ile Pro Ile Glu Ala
1               5
```

```
<210> 336
<211> 9
<212> PRT
<213> hepatitis C virus


<400> 336
Leu Ile Thr Ser Cys Ser Ser Asn Val
1               5


<210> 337
<211> 9
<212> PRT
<213> hepatitis C virus


<400> 337
Ala Thr Leu Gly Phe Gly Ala Tyr Met
1               5


<210> 338
<211> 9
<212> PRT
<213> hepatitis C virus


<400> 338
Leu Ala Pro Glu Ala Arg Gln Ala Ile
1               5


<210> 339
<211> 9
<212> PRT
<213> hepatitis C virus


<400> 339
Gly Val Asn Tyr Ala Thr Gly Asn Leu
1               5


<210> 340
<211> 9
<212> PRT
```

<213> hepatitis C virus


<400> 340

Arg Val Leu Glu Asp Gly Val Asn Tyr
1               5


<210> 341

<211> 9

<212> PRT

<213> hepatitis C virus


<400> 341

Tyr Thr Gly Asp Phe Asp Ser Val Ile
1               5


<210> 342

<211> 9

<212> PRT

<213> hepatitis C virus


<400> 342

Pro Ile Thr Lys Tyr Ile Met Ala Cys
1               5


<210> 343

<211> 9

<212> PRT

<213> hepatitis C virus


<400> 343

Val Val Val Ala Thr Asp Ala Leu Met
1               5


<210> 344

<211> 9

<212> PRT

<213> hepatitis C virus


<400> 344

Arg Ala Gln Ala Pro Pro Pro Ser Trp
1               5

<210>  345

<211>  9

<212>  PRT

<213>  hepatitis C virus


<400>  345

Pro Gly Cys Ser Phe Ser Ile Phe Leu
1               5

<210>  346

<211>  9

<212>  PRT

<213>  hepatitis C virus


<400>  346

Ser Val Phe Thr Gly Leu Thr His Ile
1               5

<210>  347

<211>  9

<212>  PRT

<213>  hepatitis C virus


<400>  347

Gly Ala Val Gln Asn Glu Val Thr Leu
1               5

<210>  348

<211>  9

<212>  PRT

<213>  hepatitis C virus


<400>  348

Lys Gln Ala Gly Asp Asn Phe Pro Tyr
1               5

<210>  349

<211> 9

<212> PRT

<213> hepatitis C virus


<400> 349

Val Phe Pro Asp Leu Gly Val Arg Val
1               5


<210> 350

<211> 9

<212> PRT

<213> hepatitis C virus


<400> 350

Val Asp Phe Ser Leu Asp Pro Thr Phe
1               5


<210> 351

<211> 9

<212> PRT

<213> hepatitis C virus


<400> 351

Gly Leu Pro Val Cys Gln Asp His Leu
1               5


<210> 352

<211> 9

<212> PRT

<213> hepatitis C virus


<400> 352

Val Asn Gly Asp Asp Leu Val Val Ile
1               5


<210> 353

<211> 9

<212> PRT

<213> hepatitis C virus

<400> 353

Gly Tyr Thr Gly Asp Phe Asp Ser Val
1               5


<210> 354

<211> 9

<212> PRT

<213> hepatitis C virus


<400> 354

Pro Ser Gly His Val Val Gly Val Phe
1               5


<210> 355

<211> 9

<212> PRT

<213> hepatitis C virus


<400> 355

Val Val Ser Thr Leu Pro Gln Ala Val
1               5


<210> 356

<211> 9

<212> PRT

<213> hepatitis C virus


<400> 356

Ile Thr Tyr Ser Thr Tyr Gly Lys Phe
1               5


<210> 357

<211> 9

<212> PRT

<213> hepatitis C virus


<400> 357

Pro Leu Gly Gly Ala Ala Arg Ala Leu
1               5

<210> 358

<211> 9

<212> PRT

<213> hepatitis C virus


<400> 358

Pro Tyr Leu Val Ala Tyr Gln Ala Thr
1               5


<210> 359

<211> 9

<212> PRT

<213> hepatitis C virus


<400> 359

Thr His Pro Ile Thr Lys Tyr Ile Met
1               5


<210> 360

<211> 9

<212> PRT

<213> hepatitis C virus


<400> 360

Phe Gly Ala Tyr Met Ser Lys Ala His
1               5


<210> 361

<211> 9

<212> PRT

<213> hepatitis C virus


<400> 361

Phe Leu Leu Ala Leu Leu Ser Cys Leu
1               5


<210> 362

<211> 9

```
<212>  PRT
<213>  hepatitis C virus


<400>  362
Phe Ser Ile Phe Leu Leu Ala Leu Leu
1               5


<210>  363
<211>  9
<212>  PRT
<213>  hepatitis C virus


<400>  363
Thr Leu Thr Cys Gly Phe Ala Asp Leu
1               5


<210>  364
<211>  9
<212>  PRT
<213>  hepatitis C virus


<400>  364
Leu Met Gly Tyr Ile Pro Leu Val Gly
1               5


<210>  365
<211>  9
<212>  PRT
<213>  hepatitis C virus


<400>  365
Trp Pro Leu Tyr Gly Asn Glu Gly Leu
1               5


<210>  366
<211>  9
<212>  PRT
<213>  hepatitis C virus
```

```
<400>  366

Gly Tyr Ile Pro Leu Val Gly Ala Pro
1               5


<210>  367

<211>  9

<212>  PRT

<213>  hepatitis C virus


<400>  367

Glu Gly Leu Gly Trp Ala Gly Trp Leu
1               5


<210>  368

<211>  9

<212>  PRT

<213>  hepatitis C virus


<400>  368

Leu Leu Ser Cys Leu Thr Ile Pro Ala
1               5


<210>  369

<211>  9

<212>  PRT

<213>  hepatitis C virus


<400>  369

Gly Tyr Pro Trp Pro Leu Tyr Gly Asn
1               5


<210>  370

<211>  9

<212>  PRT

<213>  hepatitis C virus


<400>  370

Asp Pro Arg Arg Arg Ser Arg Asn Leu
1               5
```

<210> 371

<211> 9

<212> PRT

<213> hepatitis C virus


<400> 371

```
Ala Pro Thr Leu Trp Ala Arg Met Ile
1               5
```

<210> 372

<211> 9

<212> PRT

<213> hepatitis C virus


<400> 372

```
Thr Pro Gly Glu Arg Pro Ser Gly Met
1               5
```

<210> 373

<211> 9

<212> PRT

<213> hepatitis C virus


<400> 373

```
Ser Pro Gly Gln Arg Val Glu Phe Leu
1               5
```

<210> 374

<211> 9

<212> PRT

<213> hepatitis C virus


<400> 374

```
Asp Pro Arg Arg Arg Ser Arg Asn Leu
1               5
```

<210> 375

<211> 9

<212> PRT

<213> hepatitis C virus

<400> 375

Leu Pro Gly Cys Ser Phe Ser Ile Phe
1               5

<210> 376
<211> 9
<212> PRT
<213> hepatitis C virus

<400> 376

Glu Val Ile Lys Gly Gly Arg His Leu
1               5

<210> 377
<211> 9
<212> PRT
<213> hepatitis C virus

<400> 377

Glu Ala Ile Lys Gly Gly Arg His Leu
1               5

<210> 378
<211> 9
<212> PRT
<213> hepatitis C virus

<400> 378

Trp Pro Leu Tyr Gly Asn Glu Gly Leu
1               5

<210> 379
<211> 9
<212> PRT
<213> hepatitis C virus

<400> 379

Ala Leu Ala His Gly Val Arg Val Leu
1               5

<210> 380

<211> 9

<212> PRT

<213> hepatitis C virus


<400> 380

Leu Pro Arg Arg Gly Pro Arg Leu Gly
1               5

<210> 381

<211> 9

<212> PRT

<213> hepatitis C virus


<400> 381

Ala Pro Pro Pro Ser Trp Asp Gln Met
1               5

<210> 382

<211> 9

<212> PRT

<213> hepatitis C virus


<400> 382

Gly Pro Thr Pro Leu Leu Tyr Arg Leu
1               5

<210> 383

<211> 9

<212> PRT

<213> hepatitis C virus


<400> 383

Thr Pro Ala Glu Thr Ser Val Arg Leu
1               5

<210> 384

```
<211>  9
<212>  PRT
<213>  hepatitis C virus


<400>  384
Ala Pro Leu Gly Gly Ala Ala Arg Ala
1               5


<210>  385
<211>  9
<212>  PRT
<213>  hepatitis C virus


<400>  385
Ala Thr Leu Gly Phe Gly Ala Tyr Met
1               5


<210>  386
<211>  9
<212>  PRT
<213>  hepatitis C virus


<400>  386
Ser Pro Arg Gly Ser Arg Pro Ser Trp
1               5


<210>  387
<211>  9
<212>  PRT
<213>  hepatitis C virus


<400>  387
Gly Pro Arg Leu Gly Val Arg Ala Thr
1               5


<210>  388
<211>  9
<212>  PRT
<213>  hepatitis C virus
```

```
<400>  388

Glu Ala Arg Gln Ala Ile Arg Ser Leu
1               5


<210>  389

<211>  9

<212>  PRT

<213>  hepatitis C virus


<400>  389

Thr Pro Leu Leu Tyr Arg Leu Gly Ala
1               5


<210>  390

<211>  9

<212>  PRT

<213>  hepatitis C virus


<400>  390

Gln Pro Arg Gly Arg Arg Gln Pro Ile
1               5


<210>  391

<211>  9

<212>  PRT

<213>  hepatitis C virus


<400>  391

Tyr Ala Ala Gln Gly Tyr Lys Val Leu
1               5


<210>  392

<211>  9

<212>  PRT

<213>  hepatitis C virus


<400>  392

Val Pro His Pro Asn Ile Glu Glu Ile
1               5
```

<210> 393

<211> 9

<212> PRT

<213> hepatitis C virus


<400> 393

Ala Val Met Gly Ser Ser Tyr Gly Phe
1               5


<210> 394

<211> 9

<212> PRT

<213> hepatitis C virus


<400> 394

Val Ala Tyr Tyr Arg Gly Leu Asp Val
1               5


<210> 395

<211> 9

<212> PRT

<213> hepatitis C virus


<400> 395

His Pro Asn Ile Glu Glu Ile Gly Leu
1               5


<210> 396

<211> 9

<212> PRT

<213> hepatitis C virus


<400> 396

His Pro Ile Thr Lys Tyr Ile Met Ala
1               5


<210> 397

<211> 9

```
<212>  PRT
<213>  hepatitis C virus


<400>  397
Ala Pro Thr Gly Ser Gly Lys Ser Thr
1               5

<210>  398
<211>  9
<212>  PRT
<213>  hepatitis C virus


<400>  398
Ser Val Phe Thr Gly Leu Thr His Ile
1               5

<210>  399
<211>  9
<212>  PRT
<213>  hepatitis C virus


<400>  399
Cys Pro Ser Gly His Val Val Gly Val
1               5

<210>  400
<211>  9    .
<212>  PRT
<213>  hepatitis C virus


<400>  400
Asn Ala Val Ala Tyr Tyr Arg Gly Leu
1               5

<210>  401
<211>  9
<212>  PRT
<213>  hepatitis C virus
```

<400> 401

Ser Ala Ala Cys Arg Ala Ala Lys Leu
1               5

<210> 402

<211> 9

<212> PRT

<213> hepatitis C virus

<400> 402

Ala Ala Lys Leu Ser Ala Leu Gly Leu
1               5

<210> 403

<211> 9

<212> PRT

<213> hepatitis C virus

<400> 403

Ala Ala Arg Ala Leu Ala His Gly Val
1               5

<210> 404

<211> 9

<212> PRT

<213> hepatitis C virus

<400> 404

Asn Pro Lys Pro Gln Arg Lys Thr Lys
1               5

<210> 405

<211> 9

<212> PRT

<213> hepatitis C virus

<400> 405

Ala Pro Glu Ala Arg Gln Ala Ile Arg
1               5

```
<210>  406
<211>  9
<212>  PRT
<213>  hepatitis C virus


<400>  406
Arg Ser Arg Asn Leu Gly Lys Val Ile
1               5


<210>  407
<211>  9
<212>  PRT
<213>  hepatitis C virus


<400>  407
Phe Pro Gly Gly Gly Gln Ile Val Gly
1               5


<210>  408
<211>  9
<212>  PRT
<213>  hepatitis C virus


<400>  408
Ala Pro Ile Thr Tyr Ser Thr Tyr Gly
1               5


<210>  409
<211>  9
<212>  PRT
<213>  hepatitis C virus


<400>  409
Ile Pro Lys Ala Arg Arg Pro Glu Gly
1               5


<210>  410
<211>  9
<212>  PRT
```

<213> hepatitis C virus


<400> 410

Val Pro Gln Thr Phe Gln Val Ala His
1               5


<210> 411

<211> 9

<212> PRT

<213> hepatitis C virus


<400> 411

Asn Ser Trp Leu Gly Asn Ile Ile Met
1               5


<210> 412

<211> 9

<212> PRT

<213> hepatitis C virus


<400> 412

Arg Ala Leu Ala His Gly Val Arg Val
1               5


<210> 413

<211> 9

<212> PRT

<213> hepatitis C virus


<400> 413

Arg Thr Gly Arg Gly Arg Arg Gly Ile
1               5


<210> 414

<211> 9

<212> PRT

<213> hepatitis C virus


<400> 414

Ile Thr Lys Tyr Ile Met Ala Cys Met
1                   5

<210>  415

<211>  9

<212>  PRT

<213>  hepatitis C virus


<400>  415

Ile Pro Thr Ser Gly Asp Val Val Val
1                   5

<210>  416

<211>  9

<212>  PRT

<213>  hepatitis C virus


<400>  416

Pro Thr Leu His Gly Pro Thr Pro Leu
1                   5

<210>  417

<211>  9

<212>  PRT

<213>  hepatitis C virus


<400>  417

Ile Pro Phe Tyr Gly Lys Ala Ile Pro
1                   5

<210>  418

<211>  9

<212>  PRT

<213>  hepatitis C virus


<400>  418

Tyr Ala Pro Thr Leu Trp Ala Arg Met
1                   5

<210>  419

<211> 9

<212> PRT

<213> hepatitis C virus


<400> 419

Ala Ser Gly Lys Arg Val Tyr Tyr Leu
1               5


<210> 420

<211> 9

<212> PRT

<213> hepatitis C virus


<400> 420

Thr Ala Gly Ala Arg Leu Val Val Leu
1               5


<210> 421

<211> 9

<212> PRT

<213> hepatitis C virus


<400> 421

Cys Pro Met Gly Phe Ser Tyr Asp Thr
1               5


<210> 422

<211> 9

<212> PRT

<213> hepatitis C virus


<400> 422

Ala Ile Lys Gly Gly Arg His Leu Ile
1               5


<210> 423

<211> 9

<212> PRT

<213> hepatitis C virus

```
<400>  423

Asn Pro Ser Val Ala Ala Thr Leu Gly
1               5


<210>  424

<211>  9

<212>  PRT

<213>  hepatitis C virus



<400>  424

Thr Ile Leu Gly Ile Gly Thr Val Leu
1               5


<210>  425

<211>  9

<212>  PRT

<213>  hepatitis C virus



<400>  425

Ile Pro Ile Glu Ala Ile Lys Gly Gly
1               5


<210>  426

<211>  9

<212>  PRT

<213>  hepatitis C virus



<400>  426

Leu Thr His Pro Ile Thr Lys Tyr Ile
1               5


<210>  427

<211>  9

<212>  PRT

<213>  hepatitis C virus



<400>  427

Leu Val Leu Asn Pro Ser Val Ala Ala
1               5
```

<210> 428

<211> 9

<212> PRT

<213> hepatitis C virus


<400> 428

Glu Arg Leu Tyr Ile Gly Gly Pro Leu
1               5


<210> 429

<211> 9

<212> PRT

<213> hepatitis C virus


<400> 429

Gly Val Ala Lys Ala Val Asp Phe Ile
1               5


<210> 430

<211> 9

<212> PRT

<213> hepatitis C virus


<400> 430

Gln Pro Glu Lys Gly Gly Arg Lys Pro
1                5


<210> 431

<211> 9

<212> PRT

<213> hepatitis C virus


<400> 431

Arg Pro Ser Trp Gly Pro Thr Asp Pro
1               5


<210> 432

<211> 9

```
<212>   PRT
<213>   hepatitis C virus


<400>   432
Gly Val Asn Tyr Ala Thr Gly Asn Leu
1                   5


<210>   433
<211>   9
<212>   PRT
<213>   hepatitis C virus


<400>   433
Glu Leu Ala Ala Lys Leu Ser Ala Leu
1                   5


<210>   434
<211>   9
<212>   PRT
<213>   hepatitis C virus


<400>   434
Arg Ala Tyr Leu Asn Thr Pro Gly Leu
1                   5


<210>   435
<211>   9
<212>   PRT
<213>   hepatitis C virus


<400>   435
Gly Gly Arg Lys Pro Ala Arg Leu Ile
1                   5


<210>   436
<211>   9
<212>   PRT
<213>   hepatitis C virus
```

<400> 436

Asn Ile Arg Thr Gly Val Arg Thr Ile
1               5

<210> 437

<211> 9

<212> PRT

<213> hepatitis C virus

<400> 437

Val Val Val Ala Thr Asp Ala Leu Met
1               5

<210> 438

<211> 9

<212> PRT

<213> hepatitis C virus

<400> 438

Ala Gln Gly Tyr Lys Val Leu Val Leu
1               5

<210> 439

<211> 9

<212> PRT

<213> hepatitis C virus

<400> 439

Glu Thr Ala Gly Ala Arg Leu Val Val
1               5

<210> 440

<211> 9

<212> PRT

<213> hepatitis C virus

<400> 440

Ala Ala Lys Leu Gln Asp Cys Thr Met
1               5

<210> 441

<211> 9

<212> PRT

<213> hepatitis C virus


<400> 441

Lys Gly Ser Ser Gly Gly Pro Leu Leu
1               5


<210> 442

<211> 9

<212> PRT

<213> hepatitis C virus


<400> 442

Ile Pro Leu Val Gly Ala Pro Leu Gly
1               5


<210> 443

<211> 9

<212> PRT

<213> hepatitis C virus


<400> 443

Phe Pro Tyr Leu Val Ala Tyr Gln Ala
1               5


<210> 444

<211> 9

<212> PRT

<213> hepatitis C virus


<400> 444

Trp Ala Gly Trp Leu Leu Ser Pro Arg
1               5


<210> 445

<211> 9

<212> PRT

EP 1 652 858 A1

<210> hepatitis C virus

<400> 445

Gln Pro Gly Tyr Pro Trp Pro Leu Tyr
1                   5

<210> 446
<211> 9
<212> PRT
<213> hepatitis C virus

<400> 446

Leu Ala Leu Leu Ser Cys Leu Thr Ile
1                   5

<210> 447
<211> 9
<212> PRT
<213> hepatitis C virus

<400> 447

Gly Val Arg Val Leu Glu Asp Gly Val
1                   5

<210> 448
<211> 9
<212> PRT
<213> hepatitis C virus

<400> 448

Ala Gln Pro Gly Tyr Pro Trp Pro Leu
1                   5

<210> 449
<211> 9
<212> PRT
<213> hepatitis C virus

<400> 449

238

Pro Arg Arg Gly Pro Arg Leu Gly Val
1               5

<210>   450

<211>   10

<212>   PRT

<213>   hepatitis C virus


<400>   450

Leu Pro Arg Arg Gly Pro Arg Leu Gly Val
1               5                       10

<210>   451

<211>   9

<212>   PRT

<213>   hepatitis C virus


<400>   451

Asp Pro Arg Arg Arg Ser Arg Asn Leu
1               5

<210>   452

<211>   9

<212>   PRT

<213>   hepatitis C virus


<400>   452

Gln Pro Arg Gly Arg Arg Gln Pro Ile
1               5

<210>   453

<211>   9

<212>   PRT

<213>   hepatitis C virus


<400>   453

Ile Pro Lys Ala Arg Arg Pro Glu Gly
1               5

<210>   454

```
<211>   9
<212>   PRT
<213>   hepatitis C virus


<400>   454
His Pro Ile Thr Lys Tyr Ile Met Ala
1               5


<210>   455
<211>   9
<212>   PRT
<213>   hepatitis C virus


<400>   455
His Ser Lys Lys Lys Cys Asp Glu Leu
1               5


<210>   456
<211>   9
<212>   PRT
<213>   hepatitis C virus


<400>   456
Gln Arg Arg Gly Arg Thr Gly Arg Gly
1               5


<210>   457
<211>   9
<212>   PRT
<213>   hepatitis C virus


<400>   457
Asn Pro Lys Pro Gln Arg Lys Thr Lys
1               5


<210>   458
<211>   9
<212>   PRT
<213>   hepatitis C virus
```

```
<400>  458

Gly Lys Arg Val Tyr Tyr Leu Thr Arg
1                5


<210>  459

<211>  9

<212>  PRT

<213>  hepatitis C virus


<400>  459

Ser Val Arg Leu Arg Ala Tyr Leu Asn
1                5


<210>  460

<211>  9

<212>  PRT

<213>  hepatitis C virus


<400>  460

Asp Leu Met Gly Tyr Ile Pro Leu Val
1                5


<210>  461

<211>  9

<212>  PRT

<213>  hepatitis C virus


<400>  461

Asn Ala Val Ala Tyr Tyr Arg Gly Leu
1                5


<210>  462

<211>  9

<212>  PRT

<213>  hepatitis C virus


<400>  462

Glu Gly Arg Thr Trp Ala Gln Pro Gly
1                5
```

<210> 463

<211> 9

<212> PRT

<213> hepatitis C virus


<400> 463

Ile Thr Lys Tyr Ile Met Ala Cys Met
1               5

<210> 464

<211> 9

<212> PRT

<213> hepatitis C virus


<400> 464

Gly Arg Arg Gly Ile Tyr Arg Phe Val
1               5

<210> 465

<211> 9

<212> PRT

<213> hepatitis C virus


<400> 465

Thr Leu Trp Ala Arg Met Ile Leu Met
1               5

<210> 466

<211> 9

<212> PRT

<213> hepatitis C virus


<400> 466

Asp Thr Arg Cys Phe Asp Ser Thr Val
1               5

<210> 467

<211> 9

<212>   PRT

<213>   hepatitis C virus


<400>   467

Gln Met Trp Lys Cys Leu Ile Arg Leu
1               5


<210>   468

<211>   9

<212>   PRT

<213>   hepatitis C virus


<400>   468

Leu Ile Arg Leu Lys Pro Thr Leu His
1               5


<210>   469

<211>   9

<212>   PRT

<213>   hepatitis C virus


<400>   469

Val Ala Tyr Tyr Arg Gly Leu Asp Val
1               5


<210>   470

<211>   9

<212>   PRT

<213>   hepatitis C virus


<400>   470

Leu Thr His Pro Ile Thr Lys Tyr Ile
1               5


<210>   471

<211>   9

<212>   PRT

<213>   hepatitis C virus

<400> 471

Trp Ala Arg Met Ile Leu Met Thr His
1               5

<210> 472

<211> 9

<212> PRT

<213> hepatitis C virus

<400> 472

Phe Pro Tyr Leu Val Ala Tyr Gln Ala
1               5

<210> 473

<211> 9

<212> PRT

<213> hepatitis C virus

<400> 473

Ala Ile Arg Ser Leu Thr Glu Arg Leu
1               5

<210> 474

<211> 9

<212> PRT

<213> hepatitis C virus

<400> 474

Glu Ala Arg Gln Ala Ile Arg Ser Leu
1               5

<210> 475

<211> 9

<212> PRT

<213> hepatitis C virus

<400> 475

Tyr Arg Arg Cys Arg Ala Ser Gly Val
1               5

```
<210>    476
<211>    9
<212>    PRT
<213>    hepatitis C virus


<400>    476

Gln Arg Lys Thr Lys Arg Asn Thr Asn
1               5


<210>    477
<211>    9
<212>    PRT
<213>    hepatitis C virus


<400>    477

Phe Cys His Ser Lys Lys Lys Cys Asp
1               5


<210>    478
<211>    9
<212>    PRT
<213>    hepatitis C virus


<400>    478

Ala Trp Lys Ser Lys Lys Cys Pro Met
1               5


<210>    479
<211>    9
<212>    PRT
<213>    hepatitis C virus


<400>    479

Gln Pro Ile Pro Lys Ala Arg Arg Pro
1               5


<210>    480
<211>    9
<212>    PRT
```

<213> hepatitis C virus

<400> 480

Asp His Leu Glu Phe Trp Glu Ser Val
1               5

<210> 481

<211> 9

<212> PRT

<213> hepatitis C virus

<400> 481

Ser Gly Lys Arg Val Tyr Tyr Leu Thr
1               5

<210> 482

<211> 9

<212> PRT

<213> hepatitis C virus

<400> 482

Tyr Gly Lys Ala Ile Pro Ile Glu Ala
1               5

<210> 483

<211> 9

<212> PRT

<213> hepatitis C virus

<400> 483

Ser Gly Lys Ser Thr Lys Val Pro Ala
1               5

<210> 484

<211> 9

<212> PRT

<213> hepatitis C virus

<400> 484

Thr Pro Leu Leu Tyr Arg Leu Gly Ala
1               5

<210>   485

<211>   9

<212>   PRT

<213>   hepatitis C virus


<400>   485

Asn Ile Ile Met Tyr Ala Pro Thr Leu
1               5

<210>   486

<211>   9

<212>   PRT

<213>   hepatitis C virus


<400>   486

Tyr Gly Lys Ala Ile Pro Ile Glu Val
1               5

<210>   487

<211>   9

<212>   PRT

<213>   hepatitis C virus


<400>   487

Gly Val Tyr Leu Leu Pro Arg Arg Gly
1               5

<210>   488

<211>   9

<212>   PRT

<213>   hepatitis C virus


<400>   488

Glu Ala Met Thr Arg Tyr Ser Ala Pro
1               5

<210>   489

<211> 9

<212> PRT

<213> hepatitis C virus


<400> 489

Glu Gly Leu Gly Trp Ala Gly Trp Leu
1               5


<210> 490

<211> 9

<212> PRT

<213> hepatitis C virus


<400> 490

Thr Gly Arg Gly Arg Arg Gly Ile Tyr
1               5


<210> 491

<211> 9

<212> PRT

<213> hepatitis C virus


<400> 491

Val Ile Lys Gly Gly Arg His Leu Ile
1               5


<210> 492

<211> 9

<212> PRT

<213> hepatitis C virus


<400> 492

Gly Gly Arg His Leu Ile Phe Cys His
1               5


<210> 493

<211> 9

<212> PRT

<213> hepatitis C virus

```
<400>  493

Asp Ala His Phe Leu Ser Gln Thr Lys
1               5


<210>  494

<211>  9

<212>  PRT

<213>  hepatitis C virus


<400>  494

Thr Cys Tyr Leu Lys Ala Ser Ala Ala
1               5


<210>  495

<211>  9

<212>  PRT

<213>  hepatitis C virus


<400>  495

Ser Leu Arg Val Phe Thr Glu Ala Met
1               5


<210>  496

<211>  9

<212>  PRT

<213>  hepatitis C virus


<400>  496

Asp Ala Val Ser Arg Ser Gln Arg Arg
1               5


<210>  497

<211>  9

<212>  PRT

<213>  hepatitis C virus


<400>  497

Asp Gln Met Trp Lys Cys Leu Ile Arg
1               5
```

```
<210>  498
<211>  9
<212>  PRT
<213>  hepatitis C virus


<400>  498
Gly Val Arg Val Cys Glu Lys Met Ala
1               5


<210>  499
<211>  9
<212>  PRT
<213>  hepatitis C virus


<400>  499
Ser Thr Lys Val Pro Ala Ala Tyr Ala
1               5


<210>  500
<211>  9
<212>  PRT
<213>  hepatitis C virus


<400>  500
Leu Ala Arg Ala Ala Trp Glu Thr Ala
1               5


<210>  501
<211>  9
<212>  PRT
<213>  hepatitis C virus


<400>  501
Leu Leu Pro Arg Arg Gly Pro Arg Leu
1               5


<210>  502
<211>  9
```

<212> PRT

<213> hepatitis C virus

<400> 502

Ser Thr Tyr Gly Lys Phe Leu Ala Asp
1               5

<210> 503

<211> 9

<212> PRT

<213> hepatitis C virus

<400> 503

Gly Gly Arg Lys Pro Ala Arg Leu Ile
1               5

<210> 504

<211> 9

<212> PRT

<213> hepatitis C virus

<400> 504

Ala Ile Lys Gly Gly Arg His Leu Ile
1               5

<210> 505

<211> 9

<212> PRT

<213> hepatitis C virus

<400> 505

Gly Arg Arg Gln Pro Ile Pro Lys Ala
1               5

<210> 506

<211> 9

<212> PRT

<213> hepatitis C virus

<400> 506

Cys Leu Ile Arg Leu Lys Pro Thr Leu
1               5

<210> 507

<211> 9

<212> PRT

<213> hepatitis C virus

<400> 507

Gly Arg Lys Pro Ala Arg Leu Ile Val
1               5

<210> 508

<211> 9

<212> PRT

<213> hepatitis C virus

<400> 508

Ile Met Tyr Ala Pro Thr Leu Trp Ala
1               5

<210> 509

<211> 9

<212> PRT

<213> hepatitis C virus

<400> 509

Arg Ser Arg Asn Leu Gly Lys Val Ile
1               5

<210> 510

<211> 9

<212> PRT

<213> hepatitis C virus

<400> 510

Glu Ile Pro Phe Tyr Gly Lys Ala Ile
1               5

```
<210>  511
<211>  9
<212>  PRT
<213>  hepatitis C virus


<400>  511

Asn Ile Arg Thr Gly Val Arg Thr Ile
1               5


<210>  512
<211>  9
<212>  PRT
<213>  hepatitis C virus


<400>  512

Tyr Leu Leu Pro Arg Arg Gly Pro Arg
1               5


<210>  513
<211>  9
<212>  PRT
<213>  hepatitis C virus


<400>  513

Ala Ala Arg Ala Leu Ala His Gly Val
1               5


<210>  514
<211>  9
<212>  PRT
<213>  hepatitis C virus


<400>  514

Leu Pro Arg Arg Gly Pro Arg Leu Gly
1               5


<210>  515
<211>  9
<212>  PRT
```

<213> hepatitis C virus

<400> 515

Leu Pro Gly Cys Ser Phe Ser Ile Phe
1               5

<210> 516

<211> 9

<212> PRT

<213> hepatitis C virus

<400> 516

Phe Ser Leu Asp Pro Thr Phe Thr Ile
1               5

<210> 517

<211> 9

<212> PRT

<213> hepatitis C virus

<400> 517

Ala Val Met Gly Ser Ser Tyr Gly Phe
1               5

<210> 518

<211> 9

<212> PRT

<213> hepatitis C virus

<400> 518

Phe Pro Tyr Leu Val Ala Tyr Gln Ala
1               5

<210> 519

<211> 9

<212> PRT

<213> hepatitis C virus

<400> 519

Phe Pro Gly Gly Gly Gln Ile Val Gly
1               5

<210>  520

<211>  9

<212>  PRT

<213>  hepatitis C virus


<400>  520

Met Gly Ser Ser Tyr Gly Phe Gln Tyr
1               5

<210>  521

<211>  9

<212>  PRT

<213>  hepatitis C virus


<400>  521

Thr Pro Ala Glu Thr Ser Val Arg Leu
1               5

<210>  522

<211>  9

<212>  PRT

<213>  hepatitis C virus


<400>  522

Arg Val Leu Glu Asp Gly Val Asn Tyr
1               5

<210>  523

<211>  9

<212>  PRT

<213>  hepatitis C virus


<400>  523

His Pro Asn Ile Glu Glu Ile Gly Leu
1               5

<210>  524

```
<211>    9
<212>    PRT
<213>    hepatitis C virus


<400>    524
Asp Asn Phe Pro Tyr Leu Val Ala Tyr
1                   5


<210>    525
<211>    9
<212>    PRT
<213>    hepatitis C virus


<400>    525
Asp Ala Ser Gly Lys Arg Val Tyr Tyr
1                   5


<210>    526
<211>    9
<212>    PRT
<213>    hepatitis C virus


<400>    526
Thr Cys Val Thr Gln Thr Val Asp Phe
1                   5


<210>    527
<211>    9
<212>    PRT
<213>    hepatitis C virus


<400>    527
His Val Val Gly Val Phe Arg Ala Ala
1                   5


<210>    528
<211>    9
<212>    PRT
<213>    hepatitis C virus
```

<400> 528

Asp Ala Gly Cys Ala Trp Tyr Glu Leu
1               5


<210> 529

<211> 9

<212> PRT

<213> hepatitis C virus


<400> 529

Asn Ser Trp Leu Gly Asn Ile Ile Met
1               5


<210> 530

<211> 9

<212> PRT

<213> hepatitis C virus


<400> 530

Asp Ala Leu Met Thr Gly Tyr Thr Gly
1               5


<210> 531

<211> 9

<212> PRT

<213> hepatitis C virus


<400> 531

Leu Ser Asn Asn Gly Glu Ile Pro Phe
1               5


<210> 532

<211> 9

<212> PRT

<213> hepatitis C virus


<400> 532

Tyr Ser Pro Gly Gln Arg Val Glu Phe
1               5

<210> 533

<211> 9

<212> PRT

<213> hepatitis C virus


<400> 533

Trp Ala Arg Met Ile Leu Met Thr His
1               5


<210> 534

<211> 9

<212> PRT

<213> hepatitis C virus


<400> 534

Val Pro His Pro Asn Ile Glu Glu Ile
1               5


<210> 535

<211> 9

<212> PRT

<213> hepatitis C virus


<400> 535

Gln Pro Gly Tyr Pro Trp Pro Leu Tyr
1               5


<210> 536

<211> 9

<212> PRT

<213> hepatitis C virus


<400> 536

Ser Ala Ala Cys Arg Ala Ala Lys Leu
1               5


<210> 537

<211> 9

```
<212>  PRT

<213>  hepatitis C virus


<400>  537

Ala Pro Ile Thr Tyr Ser Thr Tyr Gly
1               5


<210>  538

<211>  9

<212>  PRT

<213>  hepatitis C virus


<400>  538

Trp Pro Leu Tyr Gly Asn Glu Gly Leu
1               5


<210>  539

<211>  9

<212>  PRT

<213>  hepatitis C virus


<400>  539

Gly Pro Thr Pro Leu Leu Tyr Arg Leu
1               5


<210>  540

<211>  9

<212>  PRT

<213>  hepatitis C virus


<400>  540

Phe Ser Ile Phe Leu Leu Ala Leu Leu
1               5


<210>  541

<211>  9

<212>  PRT

<213>  hepatitis C virus
```

<400> 541

Cys Pro Ser Gly His Val Val Gly Val
1               5

<210> 542

<211> 9

<212> PRT

<213> hepatitis C virus

<400> 542

Cys Pro Met Gly Phe Ser Tyr Asp Thr
1               5

<210> 543

<211> 9

<212> PRT

<213> hepatitis C virus

<400> 543

Asp Pro Pro Gln Pro Glu Tyr Asp Leu
1               5

<210> 544

<211> 9

<212> PRT

<213> hepatitis C virus

<400> 544

Val Pro Gln Thr Phe Gln Val Ala His
1               5

<210> 545

<211> 9

<212> PRT

<213> hepatitis C virus

<400> 545

Arg Ala Leu Ala His Gly Val Arg Val
1               5

<210> 546

<211> 9

<212> PRT

<213> hepatitis C virus


<400> 546

Val Val Val Ala Thr Asp Ala Leu Met
1               5


<210> 547

<211> 9

<212> PRT

<213> hepatitis C virus


<400> 547

Ile Pro Leu Val Gly Ala Pro Leu Gly
1               5


<210> 548

<211> 9

<212> PRT

<213> hepatitis C virus


<400> 548

Thr Pro Leu Leu Tyr Arg Leu Gly Ala
1               5


<210> 549

<211> 9

<212> PRT

<213> hepatitis C virus


<400> 549

Val Pro Ala Ala Tyr Ala Ala Gln Gly
1               5


<210> 550

<211> 9

<212> PRT

<213> hepatitis C virus

<400> 550

Leu Gly Leu Asn Ala Val Ala Tyr Tyr
1               5

<210> 551
<211> 9
<212> PRT
<213> hepatitis C virus

<400> 551

Gln Ala Gly Asp Asn Phe Pro Tyr Leu
1               5

<210> 552
<211> 9
<212> PRT
<213> hepatitis C virus

<400> 552

Arg Pro Gln Asp Val Lys Phe Pro Gly
1               5

<210> 553
<211> 9
<212> PRT
<213> hepatitis C virus

<400> 553

Arg Val Cys Glu Lys Met Ala Leu Tyr
1               5

<210> 554
<211> 9
<212> PRT
<213> hepatitis C virus

<400> 554

```
Leu Val Val Ile Cys Glu Ser Ala Gly
1               5
```

<210> 555

<211> 9

<212> PRT

<213> hepatitis C virus

<400> 555

```
Ala Pro Leu Gly Gly Ala Ala Arg Ala
1               5
```

<210> 556

<211> 9

<212> PRT

<213> hepatitis C virus

<400> 556

```
Cys Ser Phe Ser Ile Phe Leu Leu Ala
1               5
```

<210> 557

<211> 9

<212> PRT

<213> hepatitis C virus

<400> 557

```
Ala Ala Thr Leu Gly Phe Gly Ala Tyr
1               5
```

<210> 558

<211> 9

<212> PRT

<213> hepatitis C virus

<400> 558

```
Cys Gly Phe Ala Asp Leu Met Gly Tyr
1               5
```

<210> 559

```
<211>  9
<212>  PRT
<213>  hepatitis C virus


<400>  559
His Asp Ala Ser Gly Lys Arg Val Tyr
1                   5


<210>  560
<211>  9
<212>  PRT
<213>  hepatitis C virus


<400>  560
Ala Thr Leu Gly Phe Gly Ala Tyr Met
1                   5


<210>  561
<211>  9
<212>  PRT
<213>  hepatitis C virus


<400>  561
Ile Pro Ile Glu Val Ile Lys Gly Gly
1                   5


<210>  562
<211>  9
<212>  PRT
<213>  hepatitis C virus


<400>  562
Glu Asp Ala Ala Ser Leu Arg Val Phe
1                   5


<210>  563
<211>  9
<212>  PRT
<213>  hepatitis C virus
```

<400> 563

Ala Gln Pro Gly Tyr Pro Trp Pro Leu
1               5


<210> 564

<211> 9

<212> PRT

<213> hepatitis C virus


<400> 564

Leu Glu Phe Trp Glu Ser Val Phe Thr
1               5


<210> 565

<211> 9

<212> PRT

<213> hepatitis C virus


<400> 565

Asn Glu Gly Leu Gly Trp Ala Gly Trp
1               5


<210> 566

<211> 9

<212> PRT

<213> hepatitis C virus


<400> 566

Trp Leu Gly Asn Ile Ile Met Tyr Ala
1               5


<210> 567

<211> 9

<212> PRT

<213> hepatitis C virus


<400> 567

Arg Met Ile Leu Met Thr His Phe Phe
1               5

<210> 568

<211> 9

<212> PRT

<213> hepatitis C virus


<400> 568

Thr Leu Trp Ala Arg Met Ile Leu Met
1               5

<210> 569

<211> 9

<212> PRT

<213> hepatitis C virus


<400> 569

Asp Glu Leu Ala Ala Lys Leu Ser Ala
1               5

<210> 570

<211> 9

<212> PRT

<213> hepatitis C virus


<400> 570

Gly Gln Ile Val Gly Gly Val Tyr Leu
1               5

<210> 571

<211> 9

<212> PRT

<213> hepatitis C virus


<400> 571

Trp Glu Ser Val Phe Thr Gly Leu Thr
1               5

<210> 572

<211> 9

```
<212>   PRT
<213>   hepatitis C virus


<400>   572
Asn Glu Val Thr Leu Thr His Pro Ile
1               5


<210>   573
<211>   9
<212>   PRT
<213>   hepatitis C virus


<400>   573
Tyr Leu Val Ala Tyr Gln Ala Thr Val
1               5


<210>   574
<211>   9
<212>   PRT
<213>   hepatitis C virus


<400>   574
Ile Glu Ala Ile Lys Gly Gly Arg His
1               5


<210>   575
<211>   9
<212>   PRT
<213>   hepatitis C virus


<400>   575
Ile Glu Val Ile Lys Gly Gly Arg His
1               5


<210>   576
<211>   9
<212>   PRT
<213>   hepatitis C virus
```

<400> 576

Val Glu Phe Leu Val Asn Ala Trp Lys
1               5

<210> 577

<211> 9

<212> PRT

<213> hepatitis C virus

<400> 577

Trp Glu Thr Ala Arg His Thr Pro Val
1               5

<210> 578

<211> 9

<212> PRT

<213> hepatitis C virus

<400> 578

Gln Met Trp Lys Cys Leu Ile Arg Leu
1               5

<210> 579

<211> 9

<212> PRT

<213> hepatitis C virus

<400> 579

Gly Leu Gly Trp Ala Gly Trp Leu Leu
1               5

<210> 580

<211> 9

<212> PRT

<213> hepatitis C virus

<400> 580

Thr Glu Arg Leu Tyr Ile Gly Gly Pro
1               5

<210> 581

<211> 9

<212> PRT

<213> hepatitis C virus


<400> 581

Cys Glu Lys Met Ala Leu Tyr Asp Val
1               5


<210> 582

<211> 9

<212> PRT

<213> hepatitis C virus


<400> 582

Ala Glu Thr Ala Gly Ala Arg Leu Val
1               5


<210> 583

<211> 9

<212> PRT

<213> hepatitis C virus


<400> 583

Ile Glu Glu Ile Gly Leu Ser Asn Asn
1               5


<210> 584

<211> 9

<212> PRT

<213> hepatitis C virus


<400> 584

Arg Leu Tyr Ile Gly Gly Pro Leu Thr
1               5


<210> 585

<211> 9

<212> PRT

<213> hepatitis C virus

<400> 585

Ala Leu Gly Leu Asn Ala Val Ala Tyr
1               5

<210> 586

<211> 9

<212> PRT

<213> hepatitis C virus

<400> 586

Thr Glu Ala Met Thr Arg Tyr Ser Ala
1               5

<210> 587

<211> 9

<212> PRT

<213> hepatitis C virus

<400> 587

Pro Glu Tyr Asp Leu Glu Leu Ile Thr
1               5

<210> 588

<211> 9

<212> PRT

<213> hepatitis C virus

<400> 588

Leu Met Thr Gly Tyr Thr Gly Asp Phe
1               5

<210> 589

<211> 9

<212> PRT

<213> hepatitis C virus

<400> 589

Cys Glu Cys Tyr Asp Ala Gly Cys Ala
1               5

<210>  590

<211>  9

<212>  PRT

<213>  hepatitis C virus


<400>  590

Met Glu Thr Thr Met Arg Ser Pro Val
1               5


<210>  591

<211>  9

<212>  PRT

<213>  hepatitis C virus


<400>  591

Ile Met Tyr Ala Pro Thr Leu Trp Ala
1               5


<210>  592

<211>  9

<212>  PRT

<213>  hepatitis C virus


<400>  592

Ala Thr Leu Gly Phe Gly Ala Tyr Met
1               5


<210>  593

<211>  9

<212>  PRT

<213>  hepatitis C virus


<400>  593

Ser Trp Asp Gln Met Trp Lys Cys Leu
1               5


<210>  594

<211> 9

<212> PRT

<213> hepatitis C virus


<400> 594

Ser Phe Ser Ile Phe Leu Leu Ala Leu
1               5


<210> 595

<211> 9

<212> PRT

<213> hepatitis C virus


<400> 595

Phe Trp Glu Ser Val Phe Thr Gly Leu
1               5


<210> 596

<211> 9

<212> PRT

<213> hepatitis C virus


<400> 596

Thr Tyr Ser Thr Tyr Gly Lys Phe Leu
1               5


<210> 597

<211> 9

<212> PRT

<213> hepatitis C virus


<400> 597

Glu Tyr Asp Leu Glu Leu Ile Thr Ser
1               5


<210> 598

<211> 9

<212> PRT

<213> hepatitis C virus

<400> 598

Ala Tyr Ala Ala Gln Gly Tyr Lys Val
1               5

<210> 599

<211> 9

<212> PRT

<213> hepatitis C virus

<400> 599

Val Phe Pro Asp Leu Gly Val Arg Val
1               5

<210> 600

<211> 9

<212> PRT

<213> hepatitis C virus

<400> 600

Gly Phe Ala Asp Leu Met Gly Tyr Ile
1               5

<210> 601

<211> 9

<212> PRT

<213> hepatitis C virus

<400> 601

His Leu Glu Phe Trp Glu Ser Val Phe
1               5

<210> 602

<211> 9

<212> PRT

<213> hepatitis C virus

<400> 602

Cys Tyr Asp Ala Gly Cys Ala Trp Tyr
1               5

<210> 603

<211> 9

<212> PRT

<213> hepatitis C virus


<400> 603

Leu Tyr Gly Asn Glu Gly Leu Gly Trp
1               5


<210> 604

<211> 9

<212> PRT

<213> hepatitis C virus


<400> 604

Arg Met Ile Leu Met Thr His Phe Phe
1               5


<210> 605

<211> 9

<212> PRT

<213> hepatitis C virus


<400> 605

Ala Tyr Met Ser Lys Ala His Gly Val
1               5


<210> 606

<211> 9

<212> PRT

<213> hepatitis C virus


<400> 606

Ile Met Ala Cys Met Ser Ala Asp Leu
1               5


<210> 607

<211> 9

<212> PRT

<213> hepatitis C virus

<400> 607

Trp Tyr Glu Leu Thr Pro Ala Glu Thr
1               5

<210> 608

<211> 9

<212> PRT

<213> hepatitis C virus

<400> 608

Lys Phe Pro Gly Gly Gly Gln Ile Val
1               5

<210> 609

<211> 9

<212> PRT

<213> hepatitis C virus

<400> 609

Gly Phe Ser Tyr Asp Thr Arg Cys Phe
1               5

<210> 610

<211> 9

<212> PRT

<213> hepatitis C virus

<400> 610

Tyr Tyr Arg Gly Leu Asp Val Ser Val
1               5

<210> 611

<211> 9

<212> PRT

<213> hepatitis C virus

<400> 611

Thr Phe Thr Ile Glu Thr Thr Thr Val
1               5


<210> 612

<211> 9

<212> PRT

<213> hepatitis C virus


<400> 612

Val Phe Thr Glu Ala Met Thr Arg Tyr
1               5


<210> 613

<211> 9

<212> PRT

<213> hepatitis C virus


<400> 613

His Pro Asn Ile Glu Glu Ile Gly Leu
1               5


<210> 614

<211> 9

<212> PRT

<213> hepatitis C virus


<400> 614

Val Ile Asp Thr Leu Thr Cys Gly Phe
1               5


<210> 615

<211> 9

<212> PRT

<213> hepatitis C virus


<400> 615

Ser Tyr Asp Thr Arg Cys Phe Asp Ser
1               5

```
<210>  616
<211>  9
<212>  PRT
<213>  hepatitis C virus


<400>  616
Gln Ile Val Gly Gly Val Tyr Leu Leu
1               5


<210>  617
<211>  9
<212>  PRT
<213>  hepatitis C virus


<400>  617
Gly Leu Gly Trp Ala Gly Trp Leu Leu
1               5


<210>  618
<211>  9
<212>  PRT
<213>  hepatitis C virus


<400>  618
Thr Leu Thr Cys Gly Phe Ala Asp Leu
1               5


<210>  619
<211>  9
<212>  PRT
<213>  hepatitis C virus


<400>  619
Gly Tyr Ile Pro Leu Val Gly Ala Pro
1               5


<210>  620
<211>  9
<212>  PRT
```

<213> hepatitis C virus

<400> 620

Asn Leu Pro Gly Cys Ser Phe Ser Ile
1               5

<210> 621

<211> 9

<212> PRT

<213> hepatitis C virus

<400> 621

Thr Tyr Ser Thr Tyr Gly Lys Phe Leu
1               5

<210> 622

<211> 9

<212> PRT

<213> hepatitis C virus

<400> 622

Tyr Ala Ala Gln Gly Tyr Lys Val Leu
1               5

<210> 623

<211> 9

<212> PRT

<213> hepatitis C virus

<400> 623

Tyr Arg Arg Cys Arg Ala Ser Gly Val
1               5

<210> 624

<211> 9

<212> PRT

<213> hepatitis C virus

<400> 624

```
Ile Arg Ser Leu Thr Glu Arg Leu Tyr
1               5

<210>  625
<211>  9
<212>  PRT
<213>  hepatitis C virus


<400>  625
Gly Arg Arg Gly Ile Tyr Arg Phe Val
1               5

<210>  626
<211>  9
<212>  PRT
<213>  hepatitis C virus


<400>  626
Gly Arg Lys Pro Ala Arg Leu Ile Val
1               5

<210>  627
<211>  9
<212>  PRT
<213>  hepatitis C virus


<400>  627
Arg Met Ile Leu Met Thr His Phe Phe
1               5

<210>  628
<211>  9
<212>  PRT
<213>  hepatitis C virus


<400>  628
Tyr Tyr Arg Gly Leu Asp Val Ser Val
1               5

<210>  629
```

<211>  9

<212>  PRT

<213>  hepatitis C virus


<400>  629

Gln Met Trp Lys Cys Leu Ile Arg Leu
1                   5


<210>  630

<211>  9

<212>  PRT

<213>  hepatitis C virus


<400>  630

Ser Phe Ser Ile Phe Leu Leu Ala Leu
1                   5


<210>  631

<211>  9

<212>  PRT

<213>  hepatitis C virus


<400>  631

Ser Trp Asp Gln Met Trp Lys Cys Leu
1                   5


<210>  632

<211>  9

<212>  PRT

<213>  hepatitis C virus


<400>  632

Val Arg Val Cys Glu Lys Met Ala Leu
1                   5


<210>  633

<211>  9

<212>  PRT

<213>  hepatitis C virus

<400> 633

Ile Lys Gly Gly Arg His Leu Ile Phe
1               5

<210> 634

<211> 9

<212> PRT

<213> hepatitis C virus

<400> 634

Glu Ala Arg Gln Ala Ile Arg Ser Leu
1               5

<210> 635

<211> 9

<212> PRT

<213> hepatitis C virus

<400> 635

Asn Ala Val Ala Tyr Tyr Arg Gly Leu
1               5

<210> 636

<211> 9

<212> PRT

<213> hepatitis C virus

<400> 636

Glu Ala Ile Lys Gly Gly Arg His Leu
1               5

<210> 637

<211> 9

<212> PRT

<213> hepatitis C virus

<400> 637

Val Ala Tyr Tyr Arg Gly Leu Asp Val
1               5

<210> 638

<211> 9

<212> PRT

<213> hepatitis C virus

<400> 638

Glu Arg Leu Tyr Ile Gly Gly Pro Leu
1               5

<210> 639

<211> 9

<212> PRT

<213> hepatitis C virus

<400> 639

Glu Val Ile Lys Gly Gly Arg His Leu
1               5

<210> 640

<211> 9

<212> PRT

<213> hepatitis C virus

<400> 640

Ala Tyr Met Ser Lys Ala His Gly Val
1               5

<210> 641

<211> 9

<212> PRT

<213> hepatitis C virus

<400> 641

Ala Arg Met Ile Leu Met Thr His Phe
1               5

<210> 642

<211> 9

<212> PRT

<213> hepatitis C virus

<400> 642

Ile Met Ala Cys Met Ser Ala Asp Leu
1               5

<210> 643

<211> 9

<212> PRT

<213> hepatitis C virus

<400> 643

Ala Arg Leu Ile Val Phe Pro Asp Leu
1               5

<210> 644

<211> 9

<212> PRT

<213> hepatitis C virus

<400> 644

Ala Tyr Ala Ala Gln Gly Tyr Lys Val
1               5

<210> 645

<211> 9

<212> PRT

<213> hepatitis C virus

<400> 645

Glu Thr Ser Val Arg Leu Arg Ala Tyr
1               5

<210> 646

<211> 9

<212> PRT

<213> hepatitis C virus

<400> 646

Arg Lys Pro Ala Arg Leu Ile Val Phe
1               5

<210> 647

<211> 9

<212> PRT

<213> hepatitis C virus

<400> 647

Ala Ala Gln Gly Tyr Lys Val Leu Val
1               5

<210> 648

<211> 9

<212> PRT

<213> hepatitis C virus

<400> 648

Phe Gln Tyr Ser Pro Gly Gln Arg Val
1               5

<210> 649

<211> 9

<212> PRT

<213> hepatitis C virus

<400> 649

Gln Ile Val Gly Gly Val Tyr Leu Leu
1               5

<210> 650

<211> 9

<212> PRT

<213> hepatitis C virus

<400> 650

Phe Ser Ile Phe Leu Leu Ala Leu Leu
1               5

<210> 651

<211> 9

<212> PRT

<213> hepatitis C virus


<400> 651

Phe Trp Glu Ser Val Phe Thr Gly Leu
1               5


<210> 652

<211> 9

<212> PRT

<213> hepatitis C virus


<400> 652

Asp His Leu Glu Phe Trp Glu Ser Val
1               5


<210> 653

<211> 9

<212> PRT

<213> hepatitis C virus


<400> 653

Phe Leu Leu Ala Leu Leu Ser Cys Leu
1               5


<210> 654

<211> 9

<212> PRT

<213> hepatitis C virus


<400> 654

Pro Arg Arg Gly Pro Arg Leu Gly Val
1               5


<210> 655

<211> 9

<212> PRT

<213> hepatitis C virus

<400> 655

Lys Lys Cys Pro Met Gly Phe Ser Tyr
1                   5

<210> 656

<211> 9

<212> PRT

<213> hepatitis C virus

<400> 656

Met Gly Ser Ser Tyr Gly Phe Gln Tyr
1                   5

<210> 657

<211> 9

<212> PRT

<213> hepatitis C virus

<400> 657

Tyr Ala Pro Thr Leu Trp Ala Arg Met
1                   5

<210> 658

<211> 9

<212> PRT

<213> hepatitis C virus

<400> 658

Trp Pro Leu Tyr Gly Asn Glu Gly Leu
1                   5

<210> 659

<211> 9

<212> PRT

<213> hepatitis C virus

<400> 659

Asp Ala Gly Cys Ala Trp Tyr Glu Leu
1               5

<210>   660

<211>   9

<212>   PRT

<213>   hepatitis C virus


<400>   660

Ala Ser Gly Lys Arg Val Tyr Tyr Leu
1               5

<210>   661

<211>   9

<212>   PRT

<213>   hepatitis C virus


<400>   661

Thr Ala Gly Ala Arg Leu Val Val Leu
1               5

<210>   662

<211>   9

<212>   PRT

<213>   hepatitis C virus


<400>   662

Asp Ala Ser Gly Lys Arg Val Tyr Tyr
1               5

<210>   663

<211>   9

<212>   PRT

<213>   hepatitis C virus


<400>   663

Tyr Gly Lys Ala Ile Pro Ile Glu Val
1               5

<210>   664

<211> 9

<212> PRT

<213> hepatitis C virus

<400> 664

Ile Met Tyr Ala Pro Thr Leu Trp Ala
1               5

<210> 665

<211> 9

<212> PRT

<213> hepatitis C virus

<400> 665

Ser Trp Leu Gly Asn Ile Ile Met Tyr
1               5

<210> 666

<211> 9

<212> PRT

<213> hepatitis C virus

<400> 666

Ala Gln Pro Gly Tyr Pro Trp Pro Leu
1               5

<210> 667

<211> 9

<212> PRT

<213> hepatitis C virus

<400> 667

Trp Ala Arg Met Ile Leu Met Thr His
1               5

<210> 668

<211> 9

<212> PRT

<213> hepatitis C virus

```
<400>    668

Thr Leu Trp Ala Arg Met Ile Leu Met
1               5


<210>    669

<211>    9

<212>    PRT

<213>    hepatitis C virus


<400>    669

Leu His Gly Pro Thr Pro Leu Leu Tyr
1               5


<210>    670

<211>    9

<212>    PRT

<213>    hepatitis C virus


<400>    670

Leu Thr His Pro Ile Thr Lys Tyr Ile
1               5


<210>    671

<211>    9

<212>    PRT

<213>    hepatitis C virus


<400>    671

Thr His Pro Ile Thr Lys Tyr Ile Met
1               5


<210>    672

<211>    9

<212>    PRT

<213>    hepatitis C virus


<400>    672

Leu Met Thr Gly Tyr Thr Gly Asp Phe
1               5
```

<210> 673

<211> 9

<212> PRT

<213> hepatitis C virus

<400> 673

Asp Pro Arg Arg Arg Ser Arg Asn Leu
1               5

<210> 674

<211> 9

<212> PRT

<213> hepatitis C virus

<400> 674

Gly Cys Ser Phe Ser Ile Phe Leu Leu
1               5

<210> 675

<211> 9

<212> PRT

<213> hepatitis C virus

<400> 675

Ala Leu Ala His Gly Val Arg Val Leu
1               5

<210> 676

<211> 9

<212> PRT

<213> hepatitis C virus

<400> 676

His Leu Glu Phe Trp Glu Ser Val Phe
1               5

<210> 677

<211> 9

```
<212>   PRT

<213>   hepatitis C virus


<400>   677

Ala Ala Lys Leu Ser Ala Leu Gly Leu
1               5


<210>   678

<211>   9

<212>   PRT

<213>   hepatitis C virus


<400>   678

Tyr Leu Val Ala Tyr Gln Ala Thr Val
1               5


<210>   679

<211>   9

<212>   PRT

<213>   hepatitis C virus


<400>   679

Gly Phe Ser Tyr Asp Thr Arg Cys Phe
1               5


<210>   680

<211>   9

<212>   PRT

<213>   hepatitis C virus


<400>   680

Phe Pro Tyr Leu Val Ala Tyr Gln Ala
1               5


<210>   681

<211>   9

<212>   PRT

<213>   hepatitis C virus
```

<400> 681

Arg Ala Tyr Leu Asn Thr Pro Gly Leu
1               5

<210> 682

<211> 9

<212> PRT

<213> hepatitis C virus

<400> 682

Tyr Arg Gly Leu Asp Val Ser Val Ile
1               5

<210> 683

<211> 9

<212> PRT

<213> hepatitis C virus

<400> 683

Lys Phe Pro Gly Gly Gly Gln Ile Val
1               5

<210> 684

<211> 9

<212> PRT

<213> hepatitis C virus

<400> 684

Asp Asn Phe Pro Tyr Leu Val Ala Tyr
1               5

<210> 685

<211> 9

<212> PRT

<213> hepatitis C virus

<400> 685

Asn Arg Arg Pro Gln Asp Val Lys Phe
1               5

<210> 686

<211> 9

<212> PRT

<213> hepatitis C virus


<400> 686

Ser Ala Ala Cys Arg Ala Ala Lys Leu
1               5

<210> 687

<211> 9

<212> PRT

<213> hepatitis C virus


<400> 687

Gly Pro Thr Pro Leu Leu Tyr Arg Leu
1               5

<210> 688

<211> 9

<212> PRT

<213> hepatitis C virus


<400> 688

Tyr Ile Pro Leu Val Gly Ala Pro Leu
1               5

<210> 689

<211> 9

<212> PRT

<213> hepatitis C virus


<400> 689

Gly Gln Ile Val Gly Gly Val Tyr Leu
1               5

<210> 690

<211> 9

<212> PRT

<213> hepatitis C virus

<400> 690

Val Val Gly Val Phe Arg Ala Ala Val
1               5

<210> 691

<211> 9

<212> PRT

<213> hepatitis C virus

<400> 691

Val Phe Thr Glu Ala Met Thr Arg Tyr
1               5

<210> 692

<211> 9

<212> PRT

<213> hepatitis C virus

<400> 692

Asp Leu Met Gly Tyr Ile Pro Leu Val
1               5

<210> 693

<211> 9

<212> PRT

<213> hepatitis C virus

<400> 693

Arg Ala Ala Val Cys Thr Arg Gly Val
1               5

<210> 694

<211> 9

<212> PRT

<213> hepatitis C virus

<400> 694

Ser Pro Gly Gln Arg Val Glu Phe Leu
1               5

<210>  695

<211>  9

<212>  PRT

<213>  hepatitis C virus


<400>  695

Ala Val Met Gly Ser Ser Tyr Gly Phe
1               5

<210>  696

<211>  9

<212>  PRT

<213>  hepatitis C virus


<400>  696

Glu Leu Ala Ala Lys Leu Ser Ala Leu
1               5

<210>  697

<211>  9

<212>  PRT

<213>  hepatitis C virus


<400>  697

Arg Ala Leu Ala His Gly Val Arg Val
1               5

<210>  698

<211>  9

<212>  PRT

<213>  hepatitis C virus


<400>  698

Ala Gln Gly Tyr Lys Val Leu Val Leu
1               5

<210>  699

```
<211>  9
<212>  PRT
<213>  hepatitis C virus


<400>  699
Arg Arg Ser Arg Asn Leu Gly Lys Val
1               5


<210>  700
<211>  9
<212>  PRT
<213>  hepatitis C virus


<400>  700
Arg His Thr Pro Val Asn Ser Trp Leu
1               5


<210>  701
<211>  9
<212>  PRT
<213>  hepatitis C virus


<400>  701
Val Val Ser Thr Leu Pro Gln Ala Val
1               5


<210>  702
<211>  9
<212>  PRT
<213>  hepatitis C virus


<400>  702
Asn Ile Ile Met Tyr Ala Pro Thr Leu
1               5


<210>  703
<211>  9
<212>  PRT
<213>  hepatitis C virus
```

```
<400>  703

Met Tyr Ala Pro Thr Leu Trp Ala Arg
1               5


<210>  704

<211>  9

<212>  PRT

<213>  hepatitis C virus


<400>  704

Tyr Ser Pro Gly Gln Arg Val Glu Phe
1               5


<210>  705

<211>  9

<212>  PRT

<213>  hepatitis C virus


<400>  705

Gln Arg Val Glu Phe Leu Val Asn Ala
1               5


<210>  706

<211>  9

<212>  PRT

<213>  hepatitis C virus


<400>  706

Ala Leu Tyr Asp Val Val Ser Thr Leu
1               5


<210>  707

<211>  9

<212>  PRT

<213>  hepatitis C virus


<400>  707

Gly Arg Thr Trp Ala Gln Pro Gly Tyr
1               5
```

<210> 708

<211> 9

<212> PRT

<213> hepatitis C virus


<400> 708

Val Phe Pro Asp Leu Gly Val Arg Val
1               5


<210> 709

<211> 9

<212> PRT

<213> hepatitis C virus


<400> 709

Leu Ile Val Phe Pro Asp Leu Gly Val
1               5


<210> 710

<211> 9

<212> PRT

<213> hepatitis C virus


<400> 710

Arg Arg Cys Arg Ala Ser Gly Val Leu
1               5


<210> 711

<211> 9

<212> PRT

<213> hepatitis C virus


<400> 711

His Phe Leu Ser Gln Thr Lys Gln Ala
1               5


<210> 712

<211> 9

EP 1 652 858 A1

```
<212>   PRT

<213>   hepatitis C virus


<400>   712

Val Thr Gln Thr Val Asp Phe Ser Leu
1               5


<210>   713

<211>   9

<212>   PRT

<213>   hepatitis C virus


<400>   713

Pro Thr Leu Trp Ala Arg Met Ile Leu
1               5


<210>   714

<211>   9

<212>   PRT

<213>   hepatitis C virus


<400>   714

His Asp Ala Ser Gly Lys Arg Val Tyr
1               5


<210>   715

<211>   9

<212>   PRT

<213>   hepatitis C virus


<400>   715

Glu Gly Leu Gly Trp Ala Gly Trp Leu
1               5


<210>   716

<211>   9

<212>   PRT

<213>   hepatitis C virus
```

<400> 716

Thr Tyr Ser Thr Tyr Gly Lys Phe Leu
1                   5

<210> 717

<211> 9

<212> PRT

<213> hepatitis C virus

<400> 717

Ala Tyr Ala Ala Gln Gly Tyr Lys Val
1                   5

<210> 718

<211> 9

<212> PRT

<213> hepatitis C virus

<400> 718

Tyr Tyr Arg Gly Leu Asp Val Ser Val
1                   5

<210> 719

<211> 9

<212> PRT

<213> hepatitis C virus

<400> 719

Ala Arg Leu Ile Val Phe Pro Asp Leu
1                   5

<210> 720

<211> 9

<212> PRT

<213> hepatitis C virus

<400> 720

Ser Phe Ser Ile Phe Leu Leu Ala Leu
1                   5

<210> 721

<211> 9

<212> PRT

<213> hepatitis C virus


<400> 721

Ile Arg Ser Leu Thr Glu Arg Leu Tyr
1               5

<210> 722

<211> 9

<212> PRT

<213> hepatitis C virus


<400> 722

Leu Tyr Gly Asn Glu Gly Leu Gly Trp
1               5

<210> 723

<211> 9

<212> PRT

<213> hepatitis C virus


<400> 723

Gly Phe Ala Asp Leu Met Gly Tyr Ile
1               5

<210> 724

<211> 9

<212> PRT

<213> hepatitis C virus


<400> 724

Gly Arg Thr Trp Ala Gln Pro Gly Tyr
1               5

<210> 725

<211> 9

<212> PRT

<213> hepatitis C virus

<400> 725

Gly Phe Ser Tyr Asp Thr Arg Cys Phe
1               5

<210> 726

<211> 9

<212> PRT

<213> hepatitis C virus

<400> 726

Ser Trp Leu Gly Asn Ile Ile Met Tyr
1               5

<210> 727

<211> 9

<212> PRT

<213> hepatitis C virus

<400> 727

Lys Tyr Ile Met Ala Cys Met Ser Ala
1               5

<210> 728

<211> 9

<212> PRT

<213> hepatitis C virus

<400> 728

Ala Arg Met Ile Leu Met Thr His Phe
1               5

<210> 729

<211> 9

<212> PRT

<213> hepatitis C virus

<400> 729

```
Val Arg Val Cys Glu Lys Met Ala Leu
1               5

<210>  730
<211>  9
<212>  PRT
<213>  hepatitis C virus


<400>  730
Tyr Ala Pro Thr Leu Trp Ala Arg Met
1               5

<210>  731
<211>  9
<212>  PRT
<213>  hepatitis C virus


<400>  731
Thr Trp Ala Gln Pro Gly Tyr Pro Trp
1               5

<210>  732
<211>  9
<212>  PRT
<213>  hepatitis C virus


<400>  732
Tyr Tyr Leu Thr Arg Asp Pro Thr Thr
1               5

<210>  733
<211>  9
<212>  PRT
<213>  hepatitis C virus


<400>  733
Met Tyr Ala Pro Thr Leu Trp Ala Arg
1               5

<210>  734
```

```
<211>   9
<212>   PRT
<213>   hepatitis C virus


<400>   734
Arg His Thr Pro Val Asn Ser Trp Leu
1               5


<210>   735
<211>   9
<212>   PRT
<213>   hepatitis C virus


<400>   735
Tyr Ala Ala Gln Gly Tyr Lys Val Leu
1               5


<210>   736
<211>   9
<212>   PRT
<213>   hepatitis C virus


<400>   736
Trp Pro Leu Tyr Gly Asn Glu Gly Leu
1               5


<210>   737
<211>   9
<212>   PRT
<213>   hepatitis C virus


<400>   737
His Phe Leu Ser Gln Thr Lys Gln Ala
1               5


<210>   738
<211>   9
<212>   PRT
<213>   hepatitis C virus
```

<400> 738

Ala Tyr Met Ser Lys Ala His Gly Val
1               5


<210> 739

<211> 9

<212> PRT

<213> hepatitis C virus


<400> 739

Phe Trp Glu Ser Val Phe Thr Gly Leu
1               5


<210> 740

<211> 9

<212> PRT

<213> hepatitis C virus


<400> 740

Arg Lys Pro Ala Arg Leu Ile Val Phe
1               5


<210> 741

<211> 9

<212> PRT

<213> hepatitis C virus


<400> 741

Arg Arg Cys Arg Ala Ser Gly Val Leu
1               5


<210> 742

<211> 9

<212> PRT

<213> hepatitis C virus


<400> 742

Arg Met Ile Leu Met Thr His Phe Phe
1               5

<210> 743

<211> 9

<212> PRT

<213> hepatitis C virus


<400> 743

Ser Tyr Gly Phe Gln Tyr Ser Pro Gly
1               5


<210> 744

<211> 9

<212> PRT

<213> hepatitis C virus


<400> 744

Leu His Gly Pro Thr Pro Leu Leu Tyr
1               5


<210> 745

<211> 9

<212> PRT

<213> hepatitis C virus


<400> 745

Glu Arg Leu Tyr Ile Gly Gly Pro Leu
1               5


<210> 746

<211> 9

<212> PRT

<213> hepatitis C virus


<400> 746

Ser Trp Asp Gln Met Trp Lys Cys Leu
1               5


<210> 747

<211> 9

EP 1 652 858 A1

<212> PRT

<213> hepatitis C virus

<400> 747

Phe Pro Tyr Leu Val Ala Tyr Gln Ala
1               5

<210> 748

<211> 9

<212> PRT

<213> hepatitis C virus

<400> 748

Asn Arg Arg Pro Gln Asp Val Lys Phe
1               5

<210> 749

<211> 9

<212> PRT

<213> hepatitis C virus

<400> 749

Val Phe Pro Asp Leu Gly Val Arg Val
1               5

<210> 750

<211> 9

<212> PRT

<213> hepatitis C virus

<400> 750

Arg Arg Pro Gln Asp Val Lys Phe Pro
1               5

<210> 751

<211> 9

<212> PRT

<213> hepatitis C virus

<400> 751

Tyr Ile Pro Leu Val Gly Ala Pro Leu
1               5

<210> 752

<211> 9

<212> PRT

<213> hepatitis C virus

<400> 752

Gly Tyr Lys Val Leu Val Leu Asn Pro
1               5

<210> 753

<211> 9

<212> PRT

<213> hepatitis C virus

<400> 753

Thr His Pro Ile Thr Lys Tyr Ile Met
1               5

<210> 754

<211> 9

<212> PRT

<213> hepatitis C virus

<400> 754

Arg Tyr Ser Ala Pro Pro Gly Asp Pro
1               5

<210> 755

<211> 9

<212> PRT

<213> hepatitis C virus

<400> 755

Gly Arg Arg Gly Ile Tyr Arg Phe Val
1               5

<210> 756

<211> 9

<212> PRT

<213> hepatitis C virus


<400> 756

Cys Tyr Asp Ala Gly Cys Ala Trp Tyr
1               5


<210> 757

<211> 9

<212> PRT

<213> hepatitis C virus


<400> 757

Gly Arg Gly Arg Arg Gly Ile Tyr Arg
1               5


<210> 758

<211> 9

<212> PRT

<213> hepatitis C virus


<400> 758

Tyr Arg Phe Val Thr Pro Gly Glu Arg
1               5


<210> 759

<211> 9

<212> PRT

<213> hepatitis C virus


<400> 759

Gly Tyr Arg Arg Cys Arg Ala Ser Gly
1               5


<210> 760

<211> 9

<212> PRT

<213> hepatitis C virus

<400> 760

Ala Arg Arg Pro Glu Gly Arg Thr Trp
1               5

<210> 761

<211> 9

<212> PRT

<213> hepatitis C virus

<400> 761

Lys Lys Cys Pro Met Gly Phe Ser Tyr
1               5

<210> 762

<211> 9

<212> PRT

<213> hepatitis C virus

<400> 762

Arg Arg Pro Glu Gly Arg Thr Trp Ala
1               5

<210> 763

<211> 9

<212> PRT

<213> hepatitis C virus

<400> 763

Val Tyr Leu Leu Pro Arg Arg Gly Pro
1               5

<210> 764

<211> 9

<212> PRT

<213> hepatitis C virus

<400> 764

Ile Met Tyr Ala Pro Thr Leu Trp Ala
1               5

<210> 765

<211> 9

<212> PRT

<213> hepatitis C virus


<400> 765

Arg Arg Ser Arg Asn Leu Gly Lys Val
1               5

<210> 766

<211> 9

<212> PRT

<213> hepatitis C virus


<400> 766

Tyr Arg Gly Leu Asp Val Ser Val Ile
1               5

<210> 767

<211> 9

<212> PRT

<213> hepatitis C virus


<400> 767

Ala Arg Leu Val Val Leu Ala Thr Ala
1               5

<210> 768

<211> 9

<212> PRT

<213> hepatitis C virus


<400> 768

Ala Ser Gly Lys Arg Val Tyr Tyr Leu
1               5

<210> 769

<211> 3011

<212> PRT

<213> hepatitis C virus


<220>

<221> MISC_FEATURE

<222> (393)..(393)

<223> X is Ala or Gln


<220>

<221> MISC_FEATURE

<222> (2414)..(2414)

<223> X is a blanc that is inserted to keep numbering of HCV genotype 1
b consensus in line with numbering of HCV-1


<220>

<221> MISC_FEATURE

<222> (2884)..(2884)

<223> X is Glu or Gln


<220>

<221> MISC_FEATURE

<222> (2994)..(2994)

<223> X is Trp or Leu


<400> 769

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
            35                  40                  45

Thr Arg Lys Thr Ser Glu Arg Ser Gln Pro Arg Gly Arg Arg Gln Pro
        50                  55                  60

Ile Pro Lys Ala Arg Arg Pro Glu Gly Arg Thr Trp Ala Gln Pro Gly
65                  70                  75                  80

Tyr Pro Trp Pro Leu Tyr Gly Asn Glu Gly Leu Gly Trp Ala Gly Trp
                85                  90              95

Leu Leu Ser Pro Arg Gly Ser Arg Pro Ser Trp Gly Pro Thr Asp Pro
            100             105             110

Arg Arg Arg Ser Arg Asn Leu Gly Lys Val Ile Asp Thr Leu Thr Cys
        115             120             125

Gly Phe Ala Asp Leu Met Gly Tyr Ile Pro Leu Val Gly Ala Pro Leu
    130             135             140

Gly Gly Ala Ala Arg Ala Leu Ala His Gly Val Arg Val Leu Glu Asp
145             150             155             160

Gly Val Asn Tyr Ala Thr Gly Asn Leu Pro Gly Cys Ser Phe Ser Ile
            165             170             175

Phe Leu Leu Ala Leu Leu Ser Cys Leu Thr Ile Pro Ala Ser Ala Tyr
        180             185             190

Glu Val Arg Asn Val Ser Gly Val Tyr His Val Thr Asn Asp Cys Ser
        195             200             205

Asn Ser Ser Ile Val Tyr Glu Ala Ala Asp Met Ile Met His Thr Pro
210             215             220

Gly Cys Val Pro Cys Val Arg Glu Asn Asn Ser Ser Arg Cys Trp Val
225             230             235             240

Ala Leu Thr Pro Thr Leu Ala Ala Arg Asn Ala Ser Val Pro Thr Thr
            245             250             255

Thr Ile Arg Arg His Val Asp Leu Leu Val Gly Ala Ala Ala Phe Cys
        260             265             270

Ser Ala Met Tyr Val Gly Asp Leu Cys Gly Ser Val Phe Leu Val Ser
        275             280             285

Gln Leu Phe Thr Phe Ser Pro Arg Arg His Glu Thr Val Gln Asp Cys
290             295             300

Asn Cys Ser Ile Tyr Pro Gly His Ile Ser Gly His Arg Met Ala Trp
305             310             315             320

Asp Met Met Met Asn Trp Ser Pro Thr Thr Ala Leu Val Val Ser Gln
            325             330             335

Leu Leu Arg Ile Pro Gln Ala Val Val Asp Met Val Ala Gly Ala His
        340             345             350

Trp Gly Val Leu Ala Gly Leu Ala Tyr Tyr Ser Met Val Gly Asn Trp

313

```
              355                    360                    365

Ala Lys Val Leu Val Val Met Leu Leu Phe Ala Gly Val Asp Gly Thr
    370             375             380

Thr His Val Thr Gly Gly Ala Ala Xaa Arg Thr Thr Ser Gly Phe Thr
385             390             395             400

Ser Leu Phe Ser Pro Gly Ala Ser Gln Lys Ile Gln Leu Val Asn Thr
            405             410             415

Asn Gly Ser Trp His Ile Asn Arg Thr Ala Leu Asn Cys Asn Asp Ser
            420             425             430

Leu Gln Thr Gly Phe Leu Ala Ala Leu Phe Tyr Thr His Arg Phe Asn
            435             440             445

Ser Ser Gly Cys Pro Glu Arg Met Ala Ser Cys Arg Pro Ile Asp Lys
    450             455             460

Phe Ala Gln Gly Trp Gly Pro Ile Thr Tyr Ala Glu Pro Asn Ser Ser
465             470             475             480

Asp Gln Arg Pro Tyr Cys Trp His Tyr Ala Pro Arg Pro Cys Gly Ile
            485             490             495

Val Pro Ala Ser Gln Val Cys Gly Pro Val Tyr Cys Phe Thr Pro Ser
            500             505             510

Pro Val Val Val Gly Thr Thr Asp Arg Phe Gly Val Pro Thr Tyr Ser
            515             520             525

Trp Gly Glu Asn Glu Thr Asp Val Leu Leu Leu Asn Asn Thr Arg Pro
    530             535             540

Pro Gln Gly Asn Trp Phe Gly Cys Thr Trp Met Asn Ser Thr Gly Phe
545             550             555             560

Thr Lys Thr Cys Gly Gly Pro Pro Cys Asn Ile Gly Gly Val Gly Asn
            565             570             575

Asn Thr Leu Thr Cys Pro Thr Asp Cys Phe Arg Lys His Pro Glu Ala
            580             585             590

Thr Tyr Thr Lys Cys Gly Ser Gly Pro Trp Leu Thr Pro Arg Cys Leu
            595             600             605

Val Asp Tyr Pro Tyr Arg Leu Trp His Tyr Pro Cys Thr Val Asn Phe
    610             615             620

Thr Ile Phe Lys Val Arg Met Tyr Val Gly Gly Val Glu His Arg Leu
625             630             635             640
```

Asn Ala Ala Cys Asn Trp Thr Arg Gly Glu Arg Cys Asp Leu Glu Asp
645                     650             655

Arg Asp Arg Ser Glu Leu Ser Pro Leu Leu Leu Ser Thr Thr Glu Trp
660             665                 670

Gln Ile Leu Pro Cys Ser Phe Thr Thr Leu Pro Ala Leu Ser Thr Gly
675             680                 685

Leu Ile His Leu His Gln Asn Ile Val Asp Val Gln Tyr Leu Tyr Gly
690             695                 700

Ile Gly Ser Ala Val Val Ser Phe Ala Ile Lys Trp Glu Tyr Val Leu
705             710             715                 720

Leu Leu Phe Leu Leu Leu Ala Asp Ala Arg Val Cys Ala Cys Leu Trp
725                 730             735

Met Met Leu Leu Ile Ala Gln Ala Glu Ala Ala Leu Glu Asn Leu Val
740                 745             750

Val Leu Asn Ala Ala Ser Val Ala Gly Ala His Gly Ile Leu Ser Phe
755             760             765

Leu Val Phe Phe Cys Ala Ala Trp Tyr Ile Lys Gly Arg Leu Val Pro
770             775             780

Gly Ala Ala Tyr Ala Phe Tyr Gly Val Trp Pro Leu Leu Leu Leu Leu
785             790             795                 800

Leu Ala Leu Pro Pro Arg Ala Tyr Ala Leu Asp Arg Glu Met Ala Ala
805             810             815

Ser Cys Gly Gly Ala Val Phe Val Gly Leu Ala Leu Leu Thr Leu Ser
820             825             830

Pro Tyr Tyr Lys Val Phe Leu Ala Arg Leu Ile Trp Trp Leu Gln Tyr
835             840             845

Phe Ile Thr Arg Ala Glu Ala His Leu Gln Val Trp Val Pro Pro Leu
850             855             860

Asn Val Arg Gly Gly Arg Asp Ala Ile Ile Leu Leu Thr Cys Ala Val
865             870             875             880

His Pro Glu Leu Ile Phe Asp Ile Thr Lys Leu Leu Leu Ala Ile Leu
885             890                 895

Gly Pro Leu Met Val Leu Gln Ala Gly Ile Thr Arg Val Pro Tyr Phe
900             905             910

Val Arg Ala Gln Gly Leu Ile Arg Ala Cys Met Leu Val Arg Lys Val

315

915                          920                          925

Ala Gly Gly His Tyr Val Gln Met Ala Phe Met Lys Leu Ala Ala Leu
    930               935               940

Thr Gly Thr Tyr Val Tyr Asp His Leu Thr Pro Leu Arg Asp Trp Ala
945               950               955               960

His Ala Gly Leu Arg Asp Leu Ala Val Ala Val Glu Pro Val Val Phe
            965               970               975

Ser Asp Met Glu Thr Lys Ile Ile Thr Trp Gly Ala Asp Thr Ala Ala
            980               985               990

Cys Gly Asp Ile Ile Leu Gly Leu  Pro Val Ser Ala Arg  Arg Gly Arg
        995               1000              1005

Glu Ile  Leu Leu Gly Pro Ala  Asp Ser Leu Glu Gly  Gln Gly Trp
    1010              1015              1020

Arg Leu  Leu Ala Pro Ile Thr  Ala Tyr Ser Gln Gln  Thr Arg Gly
    1025              1030              1035

Leu Leu  Gly Cys Ile Ile Thr  Ser Leu Thr Gly Arg  Asp Lys Asn
    1040              1045              1050

Gln Val  Glu Gly Glu Val Gln  Val Val Ser Thr Ala  Thr Gln Ser
    1055              1060              1065

Phe Leu  Ala Thr Cys Val Asn  Gly Val Cys Trp Thr  Val Tyr His
    1070              1075              1080

Gly Ala  Gly Ser Lys Thr Leu  Ala Gly Pro Lys Gly  Pro Ile Thr
    1085              1090              1095

Gln Met  Tyr Thr Asn Val Asp  Gln Asp Leu Val Gly  Trp Gln Ala
    1100              1105              1110

Pro Pro  Gly Ala Arg Ser Met  Thr Pro Cys Thr Cys  Gly Ser Ser
    1115              1120              1125

Asp Leu  Tyr Leu Val Thr Arg  His Ala Asp Val Ile  Pro Val Arg
    1130              1135              1140

Arg Arg  Gly Asp Ser Arg Gly  Ser Leu Leu Ser Pro  Arg Pro Val
    1145              1150              1155

Ser Tyr  Leu Lys Gly Ser Ser  Gly Gly Pro Leu Leu  Cys Pro Ser
    1160              1165              1170

Gly His  Val Val Gly Val Phe  Arg Ala Ala Val Cys  Thr Arg Gly
    1175              1180              1185

316

```
Val Ala  Lys Ala Val Asp Phe  Ile Pro Val Glu Ser  Met Glu Thr
    1190              1195          1200

Thr Met  Arg Ser Pro Val Phe  Thr Asp Asn Ser Ser  Pro Pro Ala
    1205              1210          1215

Val Pro  Gln Thr Phe Gln Val  Ala His Leu His Ala  Pro Thr Gly
    1220              1225          1230

Ser Gly  Lys Ser Thr Lys Val  Pro Ala Ala Tyr Ala  Ala Gln Gly
    1235              1240          1245

Tyr Lys  Val Leu Val Leu Asn  Pro Ser Val Ala Ala  Thr Leu Gly
    1250              1255          1260

Phe Gly  Ala Tyr Met Ser Lys  Ala His Gly Val Asp  Pro Asn Ile
    1265              1270          1275

Arg Thr  Gly Val Arg Thr Ile  Thr Thr Gly Ala Pro  Ile Thr Tyr
    1280              1285          1290

Ser Thr  Tyr Gly Lys Phe Leu  Ala Asp Gly Gly Cys  Ser Gly Gly
    1295              1300          1305

Ala Tyr  Asp Ile Ile Ile Cys  Asp Glu Cys His Ser  Thr Asp Ser
    1310              1315          1320

Thr Thr  Ile Leu Gly Ile Gly  Thr Val Leu Asp Gln  Ala Glu Thr
    1325              1330          1335

Ala Gly  Ala Arg Leu Val Val  Leu Ala Thr Ala Thr  Pro Pro Gly
    1340              1345          1350

Ser Val  Thr Val Pro His Pro  Asn Ile Glu Glu Ile  Gly Leu Ser
    1355              1360          1365

Asn Asn  Gly Glu Ile Pro Phe  Tyr Gly Lys Ala Ile  Pro Ile Glu
    1370              1375          1380

Ala Ile  Lys Gly Gly Arg His  Leu Ile Phe Cys His  Ser Lys Lys
    1385              1390          1395

Lys Cys  Asp Glu Leu Ala Ala  Lys Leu Ser Ala Leu  Gly Leu Asn
    1400              1405          1410

Ala Val  Ala Tyr Tyr Arg Gly  Leu Asp Val Ser Val  Ile Pro Thr
    1415              1420          1425

Ser Gly  Asp Val Val Val Val  Ala Thr Asp Ala Leu  Met Thr Gly
    1430              1435          1440

Tyr Thr  Gly Asp Phe Asp Ser  Val Ile Asp Cys Asn  Thr Cys Val
```

317

```
              1445                    1450                    1455

    Thr Gln Thr Val Asp Phe Ser Leu Asp Pro Thr Phe Thr Ile Glu
        1460                    1465                    1470

    Thr Thr Thr Val Pro Gln Asp Ala Val Ser Arg Ser Gln Arg Arg
        1475                    1480                    1485

    Gly Arg Thr Gly Arg Gly Arg Arg Gly Ile Tyr Arg Phe Val Thr
        1490                    1495                    1500

    Pro Gly Glu Arg Pro Ser Gly Met Phe Asp Ser Ser Val Leu Cys
        1505                    1510                    1515

    Glu Cys Tyr Asp Ala Gly Cys Ala Trp Tyr Glu Leu Thr Pro Ala
        1520                    1525                    1530

    Glu Thr Ser Val Arg Leu Arg Ala Tyr Leu Asn Thr Pro Gly Leu
        1535                    1540                    1545

    Pro Val Cys Gln Asp His Leu Glu Phe Trp Glu Ser Val Phe Thr
        1550                    1555                    1560

    Gly Leu Thr His Ile Asp Ala His Phe Leu Ser Gln Thr Lys Gln
        1565                    1570                    1575

    Ala Gly Asp Asn Phe Pro Tyr Leu Val Ala Tyr Gln Ala Thr Val
        1580                    1585                    1590

    Cys Ala Arg Ala Gln Ala Pro Pro Pro Ser Trp Asp Gln Met Trp
        1595                    1600                    1605

    Lys Cys Leu Ile Arg Leu Lys Pro Thr Leu His Gly Pro Thr Pro
        1610                    1615                    1620

    Leu Leu Tyr Arg Leu Gly Ala Val Gln Asn Glu Val Thr Leu Thr
        1625                    1630                    1635

    His Pro Ile Thr Lys Tyr Ile Met Ala Cys Met Ser Ala Asp Leu
        1640                    1645                    1650

    Glu Val Val Thr Ser Thr Trp Val Leu Val Gly Gly Val Leu Ala
        1655                    1660                    1665

    Ala Leu Ala Ala Tyr Cys Leu Thr Thr Gly Ser Val Val Ile Val
        1670                    1675                    1680

    Gly Arg Ile Ile Leu Ser Gly Arg Pro Ala Val Ile Pro Asp Arg
        1685                    1690                    1695

    Glu Val Leu Tyr Gln Glu Phe Asp Glu Met Glu Glu Cys Ala Ser
        1700                    1705                    1710
```

318

His Leu Pro Tyr Ile Glu Gln Gly Met Gln Leu Ala Glu Gln Phe
1715                1720            1725

Lys Gln Lys Ala Leu Gly Leu Leu Gln Thr Ala Thr Lys Gln Ala
1730                1735            1740

Glu Ala Ala Ala Pro Val Val Glu Ser Lys Trp Arg Ala Leu Glu
1745                1750            1755

Thr Phe Trp Ala Lys His Met Trp Asn Phe Ile Ser Gly Ile Gln
1760                1765            1770

Tyr Leu Ala Gly Leu Ser Thr Leu Pro Gly Asn Pro Ala Ile Ala
1775                1780            1785

Ser Leu Met Ala Phe Thr Ala Ser Ile Thr Ser Pro Leu Thr Thr
1790                1795            1800

Gln His Thr Leu Leu Phe Asn Ile Leu Gly Gly Trp Val Ala Ala
1805                1810            1815

Gln Leu Ala Pro Pro Ser Ala Ala Ser Ala Phe Val Gly Ala Gly
1820                1825            1830

Ile Ala Gly Ala Ala Val Gly Ser Ile Gly Leu Gly Lys Val Leu
1835                1840            1845

Val Asp Ile Leu Ala Gly Tyr Gly Ala Gly Val Ala Gly Ala Leu
1850                1855            1860

Val Ala Phe Lys Val Met Ser Gly Glu Met Pro Ser Thr Glu Asp
1865                1870            1875

Leu Val Asn Leu Leu Pro Ala Ile Leu Ser Pro Gly Ala Leu Val
1880                1885            1890

Val Gly Val Val Cys Ala Ala Ile Leu Arg Arg His Val Gly Pro
1895                1900            1905

Gly Glu Gly Ala Val Gln Trp Met Asn Arg Leu Ile Ala Phe Ala
1910                1915            1920

Ser Arg Gly Asn His Val Ser Pro Thr His Tyr Val Pro Glu Ser
1925                1930            1935

Asp Ala Ala Ala Arg Val Thr Gln Ile Leu Ser Ser Leu Thr Ile
1940                1945            1950

Thr Gln Leu Leu Lys Arg Leu His Gln Trp Ile Asn Glu Asp Cys
1955                1960            1965

Ser Thr Pro Cys Ser Gly Ser Trp Leu Arg Asp Val Trp Asp Trp

1970       1975       1980

Ile Cys Thr Val Leu Thr Asp Phe Lys Thr Trp Leu Gln Ser Lys
1985      1990      1995

Leu Leu Pro Arg Leu Pro Gly Val Pro Phe Phe Ser Cys Gln Arg
2000      2005      2010

Gly Tyr Lys Gly Val Trp Arg Gly Asp Gly Ile Met Gln Thr Thr
2015      2020      2025

Cys Pro Cys Gly Ala Gln Ile Thr Gly His Val Lys Asn Gly Ser
2030      2035      2040

Met Arg Ile Val Gly Pro Lys Thr Cys Ser Asn Thr Trp His Gly
2045      2050      2055

Thr Phe Pro Ile Asn Ala Tyr Thr Thr Gly Pro Cys Thr Pro Ser
2060      2065      2070

Pro Ala Pro Asn Tyr Ser Arg Ala Leu Trp Arg Val Ala Ala Glu
2075      2080      2085

Glu Tyr Val Glu Val Thr Arg Val Gly Asp Phe His Tyr Val Thr
2090      2095      2100

Gly Met Thr Thr Asp Asn Val Lys Cys Pro Cys Gln Val Pro Ala
2105      2110      2115

Pro Glu Phe Phe Thr Glu Val Asp Gly Val Arg Leu His Arg Tyr
2120      2125      2130

Ala Pro Ala Cys Lys Pro Leu Leu Arg Glu Glu Val Thr Phe Gln
2135      2140      2145

Val Gly Leu Asn Gln Tyr Leu Val Gly Ser Gln Leu Pro Cys Glu
2150      2155      2160

Pro Glu Pro Asp Val Ala Val Leu Thr Ser Met Leu Thr Asp Pro
2165      2170      2175

Ser His Ile Thr Ala Glu Thr Ala Lys Arg Arg Leu Ala Arg Gly
2180      2185      2190

Ser Pro Pro Ser Leu Ala Ser Ser Ser Ala Ser Gln Leu Ser Ala
2195      2200      2205

Pro Ser Leu Lys Ala Thr Cys Thr Thr His His Asp Ser Pro Asp
2210      2215      2220

Ala Asp Leu Ile Glu Ala Asn Leu Leu Trp Arg Gln Glu Met Gly
2225      2230      2235

```
Gly Asn Ile Thr Arg Val Glu Ser Glu Asn Lys Val Val Ile Leu
    2240            2245            2250

Asp Ser Phe Asp Pro Leu Arg Ala Glu Glu Asp Glu Arg Glu Val
    2255            2260            2265

Ser Val Ala Ala Glu Ile Leu Arg Lys Ser Arg Lys Phe Pro Pro
    2270            2275            2280

Ala Leu Pro Ile Trp Ala Arg Pro Asp Tyr Asn Pro Pro Leu Leu
    2285            2290            2295

Glu Ser Trp Lys Asp Pro Asp Tyr Val Pro Pro Val Val His Gly
    2300            2305            2310

Cys Pro Leu Pro Pro Thr Lys Ala Pro Pro Ile Pro Pro Pro Arg
    2315            2320            2325

Arg Lys Arg Thr Val Val Leu Thr Glu Ser Thr Val Ser Ser Ala
    2330            2335            2340

Leu Ala Glu Leu Ala Thr Lys Thr Phe Gly Ser Ser Gly Ser Ser
    2345            2350            2355

Ala Val Asp Ser Gly Thr Ala Thr Ala Pro Pro Asp Gln Ala Ser
    2360            2365            2370

Asp Asp Gly Asp Lys Gly Ser Asp Val Glu Ser Tyr Ser Ser Met
    2375            2380            2385

Pro Pro Leu Glu Gly Glu Pro Gly Asp Pro Asp Leu Ser Asp Gly
    2390            2395            2400

Ser Trp Ser Thr Val Ser Glu Glu Ala Ser Xaa Glu Asp Val Val
    2405            2410            2415

Cys Cys Ser Met Ser Tyr Thr Trp Thr Gly Ala Leu Ile Thr Pro
    2420            2425            2430

Cys Ala Ala Glu Glu Ser Lys Leu Pro Ile Asn Ala Leu Ser Asn
    2435            2440            2445

Ser Leu Leu Arg His His Asn Met Val Tyr Ala Thr Thr Ser Arg
    2450            2455            2460

Ser Ala Ser Leu Arg Gln Lys Lys Val Thr Phe Asp Arg Leu Gln
    2465            2470            2475

Val Leu Asp Asp His Tyr Arg Asp Val Leu Lys Glu Met Lys Ala
    2480            2485            2490

Lys Ala Ser Thr Val Lys Ala Lys Leu Leu Ser Val Glu Glu Ala
```

```
        2495                    2500                    2505

Cys Lys Leu Thr Pro Pro His Ser Ala Lys Ser Lys Phe Gly Tyr
    2510                2515                2520

Gly Ala Lys Asp Val Arg Asn Leu Ser Ser Lys Ala Val Asn His
    2525                2530                2535

Ile Arg Ser Val Trp Lys Asp Leu Leu Glu Asp Thr Glu Thr Pro
    2540                2545                2550

Ile Asp Thr Thr Ile Met Ala Lys Asn Glu Val Phe Cys Val Gln
    2555                2560                2565

Pro Glu Lys Gly Gly Arg Lys Pro Ala Arg Leu Ile Val Phe Pro
    2570                2575                2580

Asp Leu Gly Val Arg Val Cys Glu Lys Met Ala Leu Tyr Asp Val
    2585                2590                2595

Val Ser Thr Leu Pro Gln Ala Val Met Gly Ser Ser Tyr Gly Phe
    2600                2605                2610

Gln Tyr Ser Pro Gly Gln Arg Val Glu Phe Leu Val Asn Ala Trp
    2615                2620                2625

Lys Ser Lys Lys Cys Pro Met Gly Phe Ser Tyr Asp Thr Arg Cys
    2630                2635                2640

Phe Asp Ser Thr Val Thr Glu Asn Asp Ile Arg Val Glu Glu Ser
    2645                2650                2655

Ile Tyr Gln Cys Cys Asp Leu Ala Pro Glu Ala Arg Gln Ala Ile
    2660                2665                2670

Arg Ser Leu Thr Glu Arg Leu Tyr Ile Gly Gly Pro Leu Thr Asn
    2675                2680                2685

Ser Lys Gly Gln Asn Cys Gly Tyr Arg Arg Cys Arg Ala Ser Gly
    2690                2695                2700

Val Leu Thr Thr Ser Cys Gly Asn Thr Leu Thr Cys Tyr Leu Lys
    2705                2710                2715

Ala Ser Ala Ala Cys Arg Ala Ala Lys Leu Gln Asp Cys Thr Met
    2720                2725                2730

Leu Val Asn Gly Asp Asp Leu Val Val Ile Cys Glu Ser Ala Gly
    2735                2740                2745

Thr Gln Glu Asp Ala Ala Ser Leu Arg Val Phe Thr Glu Ala Met
    2750                2755                2760
```

Thr Arg Tyr Ser Ala Pro Pro Gly Asp Pro Pro Gln Pro Glu Tyr
    2765                2770              2775

Asp Leu Glu Leu Ile Thr Ser Cys Ser Ser Asn Val Ser Val Ala
    2780                2785              2790

His Asp Ala Ser Gly Lys Arg Val Tyr Tyr Leu Thr Arg Asp Pro
    2795                2800              2805

Thr Thr Pro Leu Ala Arg Ala Ala Trp Glu Thr Ala Arg His Thr
    2810                2815              2820

Pro Val Asn Ser Trp Leu Gly Asn Ile Ile Met Tyr Ala Pro Thr
    2825                2830              2835

Leu Trp Ala Arg Met Ile Leu Met Thr His Phe Phe Ser Ile Leu
    2840                2845              2850

Leu Ala Gln Glu Gln Leu Glu Lys Ala Leu Asp Cys Gln Ile Tyr
    2855                2860              2865

Gly Ala Cys Tyr Ser Ile Glu Pro Leu Asp Leu Pro Gln Ile Ile
    2870                2875              2880

Xaa Arg Leu His Gly Leu Ser Ala Phe Ser Leu His Ser Tyr Ser
    2885                2890              2895

Pro Gly Glu Ile Asn Arg Val Ala Ser Cys Leu Arg Lys Leu Gly
    2900                2905              2910

Val Pro Pro Leu Arg Val Trp Arg His Arg Ala Arg Ser Val Arg
    2915                2920              2925

Ala Lys Leu Leu Ser Gln Gly Gly Arg Ala Ala Thr Cys Gly Lys
    2930                2935              2940

Tyr Leu Phe Asn Trp Ala Val Arg Thr Lys Leu Lys Leu Thr Pro
    2945                2950              2955

Ile Pro Ala Ala Ser Gln Leu Asp Leu Ser Gly Trp Phe Val Ala
    2960                2965              2970

Gly Tyr Ser Gly Gly Asp Ile Tyr His Ser Leu Ser Arg Ala Arg
    2975                2980              2985

Pro Arg Trp Phe Met Xaa Cys Leu Leu Leu Leu Ser Val Gly Val
    2990                2995              3000

Gly Ile Tyr Leu Leu Pro Asn Arg
    3005                3010

<210> 770

```
<211>  3011

<212>  PRT

<213>  hepatitis C virus


<220>

<221>  MISC_FEATURE

<222>  (2414)..(2414)

<223>  X is a blanc that is inserted to keep numbering of HCV genotype 1
       b consensus in line with numbering of HCV-1


<400>  770

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
            35                  40                  45

Thr Arg Lys Thr Ser Glu Arg Ser Gln Pro Arg Gly Arg Arg Gln Pro
        50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Met Gly Trp Ala Gly Trp
                85                  90                  95

Leu Leu Ser Pro Arg Gly Ser Arg Pro Ser Trp Gly Pro Thr Asp Pro
                100                 105                 110

Arg Arg Arg Ser Arg Asn Leu Gly Lys Val Ile Asp Thr Leu Thr Cys
            115                 120                 125

Gly Phe Ala Asp Leu Met Gly Tyr Ile Pro Leu Val Gly Ala Pro Leu
            130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala Tyr
                180                 185                 190
```

```
Glu Val Arg Asn Val Ser Gly Val Tyr His Val Thr Asn Asp Cys Ser
        195             200             205

Asn Ser Ser Ile Val Tyr Glu Ala Ala Asp Met Ile Met His Thr Pro
    210             215             220

Gly Cys Val Pro Cys Val Arg Glu Asn Asn Ser Ser Arg Cys Trp Val
225             230             235             240

Ala Leu Thr Pro Thr Leu Ala Ala Arg Asn Ser Ser Val Pro Thr Thr
            245             250             255

Thr Ile Arg Arg His Val Asp Leu Leu Val Gly Ala Ala Ala Phe Cys
        260             265             270

Ser Ala Met Tyr Val Gly Asp Leu Cys Gly Ser Val Phe Leu Val Ser
        275             280             285

Gln Leu Phe Thr Phe Ser Pro Arg Arg His Glu Thr Val Gln Asp Cys
    290             295             300

Asn Cys Ser Ile Tyr Pro Gly His Val Ser Gly His Arg Met Ala Trp
305             310             315             320

Asp Met Met Met Asn Trp Ser Pro Thr Thr Ala Leu Val Val Ser Gln
            325             330             335

Leu Leu Arg Ile Pro Gln Ala Val Val Asp Met Val Ala Gly Ala His
        340             345             350

Trp Gly Val Leu Ala Gly Leu Ala Tyr Tyr Ser Met Val Gly Asn Trp
        355             360             365

Ala Lys Val Leu Ile Val Met Leu Leu Phe Ala Gly Val Asp Gly Thr
    370             375             380

Thr His Val Thr Gly Gly Ala Ala Ala Arg Thr Thr Ser Gly Phe Thr
385             390             395             400

Ser Leu Phe Ser Pro Gly Pro Ser Gln Lys Ile Gln Leu Val Asn Thr
            405             410             415

Asn Gly Ser Trp His Ile Asn Arg Thr Ala Leu Asn Cys Asn Asp Ser
            420             425             430

Leu Gln Thr Gly Phe Leu Ala Ala Leu Phe Tyr Thr His Arg Phe Asn
        435             440             445

Ala Ser Gly Cys Pro Glu Arg Met Ala Ser Cys Arg Pro Ile Asp Lys
    450             455             460

Phe Ala Gln Gly Trp Gly Pro Ile Thr Tyr Ala Glu Pro Asn Ser Ser
465             470             475             480
```

325

EP 1 652 858 A1

Asp Gln Arg Pro Tyr Cys Trp His Tyr Ala Pro Arg Pro Cys Gly Ile
                    485              490              495

Val Pro Ala Ser Gln Val Cys Gly Pro Val Tyr Cys Phe Thr Pro Ser
                500              505              510

Pro Val Val Val Gly Thr Thr Asp Arg Phe Gly Val Pro Thr Tyr Ser
            515              520              525

Trp Gly Glu Asn Glu Thr Asp Val Leu Leu Leu Asn Asn Thr Arg Pro
    530              535              540

Pro Gln Gly Asn Trp Phe Gly Cys Thr Trp Met Asn Ser Thr Gly Phe
545              550              555              560

Thr Lys Thr Cys Gly Gly Pro Pro Cys Asn Ile Gly Gly Val Gly Asn
                565              570              575

Asn Thr Leu Thr Cys Pro Thr Asp Cys Phe Arg Lys His Pro Glu Ala
            580              585              590

Thr Tyr Thr Lys Cys Gly Ser Gly Pro Trp Leu Thr Pro Arg Cys Leu
        595              600              605

Val Asp Tyr Pro Tyr Arg Leu Trp His Tyr Pro Cys Thr Val Asn Phe
    610              615              620

Thr Ile Phe Lys Val Arg Met Tyr Val Gly Gly Val Glu His Arg Leu
625              630              635              640

Asn Ala Ala Cys Asn Trp Thr Arg Gly Glu Arg Cys Asp Leu Glu Asp
            645              650              655

Arg Asp Arg Ser Glu Leu Ser Pro Leu Leu Leu Ser Thr Thr Glu Trp
            660              665              670

Gln Ile Leu Pro Cys Ser Phe Thr Thr Leu Pro Ala Leu Ser Thr Gly
        675              680              685

Leu Ile His Leu His Gln Asn Ile Val Asp Val Gln Tyr Leu Tyr Gly
    690              695              700

Ile Gly Ser Ala Val Val Ser Phe Ala Ile Lys Trp Glu Tyr Val Leu
705              710              715              720

Leu Leu Phe Leu Leu Leu Ala Asp Ala Arg Val Cys Ala Cys Leu Trp
            725              730              735

Met Met Leu Leu Ile Ala Gln Ala Glu Ala Ala Leu Glu Asn Leu Val
            740              745              750

326

```
Val Leu Asn Ala Ala Ser Val Ala Gly Ala His Gly Ile Leu Ser Phe
        755         760             765

Leu Val Phe Phe Cys Ala Ala Trp Tyr Ile Lys Gly Arg Leu Val Pro
    770             775             780

Gly Ala Ala Tyr Ala Leu Tyr Gly Val Trp Pro Leu Leu Leu Leu Leu
785             790             795                 800

Leu Ala Leu Pro Pro Arg Ala Tyr Ala Met Asp Arg Glu Met Ala Ala
            805             810             815

Ser Cys Gly Gly Ala Val Phe Val Gly Leu Ala Leu Leu Thr Leu Ser
            820             825             830

Pro Tyr Tyr Lys Val Phe Leu Ala Arg Leu Ile Trp Trp Leu Gln Tyr
        835             840             845

Phe Ile Thr Arg Ala Glu Ala His Leu Gln Val Trp Val Pro Pro Leu
    850             855             860

Asn Val Arg Gly Gly Arg Asp Ala Ile Ile Leu Leu Thr Cys Ala Val
865             870             875             880

His Pro Glu Leu Ile Phe Asp Ile Thr Lys Leu Leu Leu Ala Ile Leu
            885             890             895

Gly Pro Leu Met Val Leu Gln Ala Gly Ile Thr Arg Val Pro Tyr Phe
            900             905             910

Val Arg Ala Gln Gly Leu Ile Arg Ala Cys Met Leu Val Arg Lys Val
    915             920             925

Ala Gly Gly His Tyr Val Gln Met Ala Phe Met Lys Leu Ala Ala Leu
    930             935             940

Thr Gly Thr Tyr Val Tyr Asp His Leu Thr Pro Leu Arg Asp Trp Ala
945             950             955             960

His Ala Gly Leu Arg Asp Leu Ala Val Ala Val Glu Pro Val Val Phe
            965             970             975

Ser Asp Met Glu Thr Lys Ile Ile Thr Trp Gly Ala Asp Thr Ala Ala
            980             985             990

Cys Gly Asp Ile Ile Leu Gly Leu Pro Val Ser Ala Arg Arg Gly Arg
            995             1000            1005

Glu Ile Leu Leu Gly Pro Ala Asp Ser Leu Glu Gly Gln Gly Trp
    1010            1015            1020

Arg Leu Leu Ala Pro Ile Thr Ala Tyr Ser Gln Gln Thr Arg Gly
    1025            1030            1035
```

```
Leu Leu Gly Cys Ile Ile Thr  Ser Leu Thr Gly Arg  Asp Lys Asn
    1040                1045              1050

Gln Val Glu Gly Glu Val Gln  Val Val Ser Thr Ala  Thr Gln Ser
    1055                1060              1065

Phe Leu Ala Thr Cys Val Asn  Gly Val Cys Trp Thr  Val Tyr His
    1070                1075              1080

Gly Ala Gly Ser Lys Thr Leu  Ala Gly Pro Lys Gly  Pro Ile Thr
    1085                1090              1095

Gln Met Tyr Thr Asn Val Asp  Gln Asp Leu Val Gly  Trp Gln Ala
    1100                1105              1110

Pro Pro Gly Ala Arg Ser Leu  Thr Pro Cys Thr Cys  Gly Ser Ser
    1115                1120              1125

Asp Leu Tyr Leu Val Thr Arg  His Ala Asp Val Ile  Pro Val Arg
    1130                1135              1140

Arg Arg Gly Asp Ser Arg Gly  Ser Leu Leu Ser Pro  Arg Pro Val
    1145                1150              1155

Ser Tyr Leu Lys Gly Ser Ser  Gly Gly Pro Leu Leu  Cys Pro Ser
    1160                1165              1170

Gly His Ala Val Gly Ile Phe  Arg Ala Ala Val Cys  Thr Arg Gly
    1175                1180              1185

Val Ala Lys Ala Val Asp Phe  Val Pro Val Glu Ser  Met Glu Thr
    1190                1195              1200

Thr Met Arg Ser Pro Val Phe  Thr Asp Asn Ser Ser  Pro Pro Ala
    1205                1210              1215

Val Pro Gln Thr Phe Gln Val  Ala His Leu His Ala  Pro Thr Gly
    1220                1225              1230

Ser Gly Lys Ser Thr Lys Val  Pro Ala Ala Tyr Ala  Ala Gln Gly
    1235                1240              1245

Tyr Lys Val Leu Val Leu Asn  Pro Ser Val Ala Ala  Thr Leu Gly
    1250                1255              1260

Phe Gly Ala Tyr Met Ser Lys  Ala His Gly Val Asp  Pro Asn Ile
    1265                1270              1275

Arg Thr Gly Val Arg Thr Ile  Thr Thr Gly Ala Pro  Ile Thr Tyr
    1280                1285              1290
```

Ser Thr Tyr Gly Lys Phe Leu Ala Asp Gly Gly Cys Ser Gly Gly
    1295              1300              1305

Ala Tyr Asp Ile Ile Ile Cys Asp Glu Cys His Ser Thr Asp Ser
    1310              1315              1320

Thr Thr Ile Leu Gly Ile Gly Thr Val Leu Asp Gln Ala Glu Thr
    1325              1330              1335

Ala Gly Ala Arg Leu Val Val Leu Ala Thr Ala Thr Pro Pro Gly
    1340              1345              1350

Ser Val Thr Val Pro His Pro Asn Ile Glu Glu Val Ala Leu Ser
    1355              1360              1365

Asn Thr Gly Glu Ile Pro Phe Tyr Gly Lys Ala Ile Pro Ile Glu
    1370              1375              1380

Val Ile Lys Gly Gly Arg His Leu Ile Phe Cys His Ser Lys Lys
    1385              1390              1395

Lys Cys Asp Glu Leu Ala Ala Lys Leu Ser Ala Leu Gly Leu Asn
    1400              1405              1410

Ala Val Ala Tyr Tyr Arg Gly Leu Asp Val Ser Val Ile Pro Thr
    1415              1420              1425

Ser Gly Asp Val Val Val Val Ala Thr Asp Ala Leu Met Thr Gly
    1430              1435              1440

Tyr Thr Gly Asp Phe Asp Ser Val Ile Asp Cys Asn Thr Cys Val
    1445              1450              1455

Thr Gln Thr Val Asp Phe Ser Leu Asp Pro Thr Phe Thr Ile Glu
    1460              1465              1470

Thr Thr Thr Val Pro Gln Asp Ala Val Ser Arg Ser Gln Arg Arg
    1475              1480              1485

Gly Arg Thr Gly Arg Gly Arg Arg Gly Ile Tyr Arg Phe Val Thr
    1490              1495              1500

Pro Gly Glu Arg Pro Ser Gly Met Phe Asp Ser Ser Val Leu Cys
    1505              1510              1515

Glu Cys Tyr Asp Ala Gly Cys Ala Trp Tyr Glu Leu Thr Pro Ala
    1520              1525              1530

Glu Thr Ser Val Arg Leu Arg Ala Tyr Leu Asn Thr Pro Gly Leu
    1535              1540              1545

Pro Val Cys Gln Asp His Leu Glu Phe Trp Glu Ser Val Phe Thr
    1550              1555              1560

```
Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Thr Leu Thr
    1625                1630                1635

His Pro Ile Thr Lys Tyr Ile Met Ala Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Val
    1670                1675                1680

Gly Arg Ile Ile Leu Ser Gly Arg Pro Ala Val Ile Pro Asp Arg
    1685                1690                1695

Glu Val Leu Tyr Arg Glu Phe Asp Glu Met Glu Glu Cys Ala Ser
    1700                1705                1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Arg Ala Leu Glu
    1745                1750                1755

Thr Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr
    1790                1795                1800

Gln His Thr Leu Leu Phe Asn Ile Leu Gly Gly Trp Val Ala Ala
    1805                1810                1815
```

```
Gln Leu  Ala Pro Pro Ser Ala  Ala Ser Ala Phe Val  Gly Ala Gly
    1820                 1825                 1830

Ile Ala  Gly Ala Ala Val Gly  Ser Ile Gly Leu Gly  Lys Val Leu
    1835                 1840                 1845

Val Asp  Ile Leu Ala Gly Tyr  Gly Ala Gly Val Ala  Gly Ala Leu
    1850                 1855                 1860

Val Ala  Phe Lys Val Met Ser  Gly Glu Met Pro Ser  Thr Glu Asp
    1865                 1870                 1875

Leu Val  Asn Leu Leu Pro Ala  Ile Leu Ser Pro Gly  Ala Leu Val
    1880                 1885                 1890

Val Gly  Val Val Cys Ala Ala  Ile Leu Arg Arg His  Val Gly Pro
    1895                 1900                 1905

Gly Glu  Gly Ala Val Gln Trp  Met Asn Arg Leu Ile  Ala Phe Ala
    1910                 1915                 1920

Ser Arg  Gly Asn His Val Ser  Pro Thr His Tyr Val  Pro Glu Ser
    1925                 1930                 1935

Asp Ala  Ala Ala Arg Val Thr  Gln Ile Leu Ser Ser  Leu Thr Ile
    1940                 1945                 1950

Thr Gln  Leu Leu Lys Arg Leu  His Gln Trp Ile Asn  Glu Asp Cys
    1955                 1960                 1965

Ser Thr  Pro Cys Ser Gly Ser  Trp Leu Arg Asp Val  Trp Asp Trp
    1970                 1975                 1980

Ile Cys  Thr Val Leu Thr Asp  Phe Lys Thr Trp Leu  Gln Ser Lys
    1985                 1990                 1995

Leu Leu  Pro Arg Leu Pro Gly  Val Pro Phe Phe Ser  Cys Gln Arg
    2000                 2005                 2010

Gly Tyr  Lys Gly Val Trp Arg  Gly Asp Gly Ile Met  Gln Thr Thr
    2015                 2020                 2025

Cys Pro  Cys Gly Ala Gln Ile  Thr Gly His Val Lys  Asn Gly Ser
    2030                 2035                 2040

Met Arg  Ile Val Gly Pro Lys  Thr Cys Ser Asn Thr  Trp His Gly
    2045                 2050                 2055

Thr Phe  Pro Ile Asn Ala Tyr  Thr Thr Gly Pro Cys  Thr Pro Ser
    2060                 2065                 2070

Pro Ala  Pro Asn Tyr Ser Arg  Ala Leu Trp Arg Val  Ala Ala Glu
    2075                 2080                 2085
```

```
Glu Tyr   Val Glu Val Thr Arg   Val Gly Asp Phe His   Tyr Val Thr
    2090                  2095               2100

Gly Met   Thr Thr Asp Asn Val   Lys Cys Pro Cys Gln   Val Pro Ala
    2105                  2110               2115

Pro Glu   Phe Phe Thr Glu Val   Asp Gly Val Arg Leu   His Arg Tyr
    2120                  2125               2130

Ala Pro   Ala Cys Lys Pro Leu   Leu Arg Glu Glu Val   Thr Phe Gln
    2135                  2140               2145

Val Gly   Leu Asn Gln Tyr Leu   Val Gly Ser Gln Leu   Pro Cys Glu
    2150                  2155               2160

Pro Glu   Pro Asp Val Ala Val   Leu Thr Ser Met Leu   Thr Asp Pro
    2165                  2170               2175

Ser His   Ile Thr Ala Glu Thr   Ala Lys Arg Arg Leu   Ala Arg Gly
    2180                  2185               2190

Ser Pro   Pro Ser Leu Ala Ser   Ser Ser Ala Ser Gln   Leu Ser Ala
    2195                  2200               2205

Pro Ser   Leu Lys Ala Thr Cys   Thr Thr His His Asp   Ser Pro Asp
    2210                  2215               2220

Ala Asp   Leu Ile Glu Ala Asn   Leu Leu Trp Arg Gln   Glu Met Gly
    2225                  2230               2235

Gly Asn   Ile Thr Arg Val Glu   Ser Glu Asn Lys Val   Val Ile Leu
    2240                  2245               2250

Asp Ser   Phe Asp Pro Leu Arg   Ala Glu Glu Asp Glu   Arg Glu Val
    2255                  2260               2265

Ser Val   Ala Ala Glu Ile Leu   Arg Lys Ser Arg Lys   Phe Pro Pro
    2270                  2275               2280

Ala Leu   Pro Ile Trp Ala Arg   Pro Asp Tyr Asn Pro   Pro Leu Leu
    2285                  2290               2295

Glu Ser   Trp Lys Asp Pro Asp   Tyr Val Pro Pro Val   Val His Gly
    2300                  2305               2310

Cys Pro   Leu Pro Pro Thr Lys   Ala Pro Pro Ile Pro   Pro Pro Arg
    2315                  2320               2325

Arg Lys   Arg Thr Val Val Leu   Thr Glu Ser Thr Val   Ser Ser Ala
    2330                  2335               2340
```

Leu Ala Glu Leu Ala Thr Lys Thr Phe Gly Ser Ser Gly Ser Ser
2345 2350 2355

Ala Val Asp Ser Gly Thr Ala Thr Ala Pro Pro Asp Gln Ala Ser
2360 2365 2370

Asp Asp Gly Asp Lys Gly Ser Asp Val Glu Ser Tyr Ser Ser Met
2375 2380 2385

Pro Pro Leu Glu Gly Glu Pro Gly Asp Pro Asp Leu Ser Asp Gly
2390 2395 2400

Ser Trp Ser Thr Val Ser Glu Glu Ala Ser Xaa Glu Asp Val Val
2405 2410 2415

Cys Cys Ser Met Ser Tyr Thr Trp Thr Gly Ala Leu Ile Thr Pro
2420 2425 2430

Cys Ala Ala Glu Glu Ser Lys Leu Pro Ile Asn Ala Leu Ser Asn
2435 2440 2445

Ser Leu Leu Arg His His Asn Met Val Tyr Ala Thr Thr Ser Arg
2450 2455 2460

Ser Ala Ser Leu Arg Gln Lys Lys Val Thr Phe Asp Arg Leu Gln
2465 2470 2475

Val Leu Asp Asp His Tyr Arg Asp Val Leu Lys Glu Met Lys Ala
2480 2485 2490

Lys Ala Ser Thr Val Lys Ala Lys Leu Leu Ser Val Glu Glu Ala
2495 2500 2505

Cys Lys Leu Thr Pro Pro His Ser Ala Lys Ser Lys Phe Gly Tyr
2510 2515 2520

Gly Ala Lys Asp Val Arg Asn Leu Ser Ser Lys Ala Val Asn His
2525 2530 2535

Ile Arg Ser Val Trp Lys Asp Leu Leu Glu Asp Thr Glu Thr Pro
2540 2545 2550

Ile Asp Thr Thr Ile Met Ala Lys Asn Glu Val Phe Cys Val Gln
2555 2560 2565

Pro Glu Lys Gly Gly Arg Lys Pro Ala Arg Leu Ile Val Phe Pro
2570 2575 2580

Asp Leu Gly Val Arg Val Cys Glu Lys Met Ala Leu Tyr Asp Val
2585 2590 2595

Val Ser Thr Leu Pro Gln Ala Val Met Gly Ser Ser Tyr Gly Phe
2600 2605 2610

Gln Tyr Ser Pro Gly Gln Arg Val Glu Phe Leu Val Asn Ala Trp
    2615                2620            2625

Lys Ser Lys Lys Cys Pro Met Gly Phe Ser Tyr Asp Thr Arg Cys
    2630            2635            2640

Phe Asp Ser Thr Val Thr Glu Asn Asp Ile Arg Val Glu Glu Ser
    2645            2650            2655

Ile Tyr Gln Cys Cys Asp Leu Ala Pro Glu Ala Arg Gln Ala Ile
    2660            2665            2670

Arg Ser Leu Thr Glu Arg Leu Tyr Ile Gly Gly Pro Leu Thr Asn
    2675            2680            2685

Ser Lys Gly Gln Asn Cys Gly Tyr Arg Arg Cys Arg Ala Ser Gly
    2690            2695            2700

Val Leu Thr Thr Ser Cys Gly Asn Thr Leu Thr Cys Tyr Leu Lys
    2705            2710            2715

Ala Ser Ala Ala Cys Arg Ala Ala Lys Leu Gln Asp Cys Thr Met
    2720            2725            2730

Leu Val Asn Gly Asp Asp Leu Val Val Ile Cys Glu Ser Ala Gly
    2735            2740            2745

Thr Gln Glu Asp Ala Ala Ser Leu Arg Val Phe Thr Glu Ala Met
    2750            2755            2760

Thr Arg Tyr Ser Ala Pro Pro Gly Asp Pro Pro Gln Pro Glu Tyr
    2765            2770            2775

Asp Leu Glu Leu Ile Thr Ser Cys Ser Ser Asn Val Ser Val Ala
    2780            2785            2790

His Asp Ala Ser Gly Lys Arg Val Tyr Tyr Leu Thr Arg Asp Pro
    2795            2800            2805

Thr Thr Pro Leu Ala Arg Ala Ala Trp Glu Thr Ala Arg His Thr
    2810            2815            2820

Pro Val Asn Ser Trp Leu Gly Asn Ile Ile Met Tyr Ala Pro Thr
    2825            2830            2835

Leu Trp Ala Arg Met Ile Leu Met Thr His Phe Phe Ser Ile Leu
    2840            2845            2850

Leu Ala Gln Glu Gln Leu Glu Lys Ala Leu Asp Cys Gln Ile Tyr
    2855            2860            2865

334

Gly Ala Cys Tyr Ser Ile Glu Pro Leu Asp Leu Pro Gln Ile Ile
    2870            2875            2880

Glu Arg Leu His Gly Leu Ser Ala Phe Ser Leu His Ser Tyr Ser
    2885            2890            2895

Pro Gly Glu Ile Asn Arg Val Ala Ser Cys Leu Arg Lys Leu Gly
    2900            2905            2910

Val Pro Pro Leu Arg Val Trp Arg His Arg Ala Arg Ser Val Arg
    2915            2920            2925

Ala Lys Leu Leu Ser Gln Gly Gly Arg Ala Ala Thr Cys Gly Lys
    2930            2935            2940

Tyr Leu Phe Asn Trp Ala Val Arg Thr Lys Leu Lys Leu Thr Pro
    2945            2950            2955

Ile Pro Ala Ala Ser Gln Leu Asp Leu Ser Gly Trp Phe Val Ala
    2960            2965            2970

Gly Tyr Ser Gly Gly Asp Ile Tyr His Ser Leu Ser Arg Ala Arg
    2975            2980            2985

Pro Arg Trp Phe Met Leu Cys Leu Leu Leu Leu Ser Val Gly Val
    2990            2995            3000

Gly Ile Tyr Leu Leu Pro Asn Arg
    3005            3010

<210> 771

<211> 3011

<212> PRT

<213> hepatitis C virus


<400> 771

Met Ser Thr Asn Pro Lys Pro Gln Arg Lys Thr Lys Arg Asn Thr Asn
1               5               10              15

Arg Arg Pro Gln Asp Val Lys Phe Pro Gly Gly Gly Gln Ile Val Gly
            20              25              30

Gly Val Tyr Leu Leu Pro Arg Arg Gly Pro Arg Leu Gly Val Arg Ala
            35              40              45

Thr Arg Lys Thr Ser Glu Arg Ser Gln Pro Arg Gly Arg Arg Gln Pro
        50              55              60

Ile Pro Lys Ala Arg Arg Pro Glu Gly Arg Thr Trp Ala Gln Pro Gly
65              70              75              80

```
Tyr Pro Trp Pro Leu Tyr Gly Asn Glu Gly Cys Gly Trp Ala Gly Trp
                     85                  90                  95

Leu Leu Ser Pro Arg Gly Ser Arg Pro Ser Trp Gly Pro Thr Asp Pro
                100                 105             110

Arg Arg Arg Ser Arg Asn Leu Gly Lys Val Ile Asp Thr Leu Thr Cys
            115                 120                 125

Gly Phe Ala Asp Leu Met Gly Tyr Ile Pro Leu Val Gly Ala Pro Leu
        130             135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Gln Val Arg Asn Ser Ser Gly Leu Tyr His Val Thr Asn Asp Cys Pro
        195                 200                 205

Asn Ser Ser Ile Val Tyr Glu Ala Ala Asp Ala Ile Leu His Thr Pro
    210                 215                 220

Gly Cys Val Pro Cys Val Arg Glu Gly Asn Ala Ser Arg Cys Trp Val
225                 230                 235                 240

Ala Val Thr Pro Thr Val Ala Thr Arg Asp Gly Lys Leu Pro Thr Thr
                245                 250                 255

Gln Leu Arg Arg His Ile Asp Leu Leu Val Gly Ser Ala Thr Leu Cys
            260                 265                 270

Ser Ala Leu Tyr Val Gly Asp Leu Cys Gly Ser Val Phe Leu Val Gly
        275                 280                 285

Gln Leu Phe Thr Phe Ser Pro Arg Arg His Trp Thr Thr Gln Asp Cys
    290                 295                 300

Asn Cys Ser Ile Tyr Pro Gly His Ile Thr Gly His Arg Met Ala Trp
305                 310                 315                 320

Asp Met Met Met Asn Trp Ser Pro Thr Ala Ala Leu Val Val Ala Gln
                325                 330                 335

Leu Leu Arg Ile Pro Gln Ala Ile Leu Asp Met Ile Ala Gly Ala His
            340                 345                 350
```

Trp Gly Val Leu Ala Gly Ile Ala Tyr Phe Ser Met Val Gly Asn Trp
        355                 360                 365

Ala Lys Val Leu Val Val Leu Leu Leu Phe Ala Gly Val Asp Ala Glu
    370                 375                 380

Thr His Val Thr Gly Gly Ser Ala Ala Arg Thr Thr Ala Gly Leu Val
385                 390                 395                 400

Ser Leu Leu Thr Pro Gly Ala Lys Gln Asn Ile Gln Leu Ile Asn Thr
            405                 410                 415

Asn Gly Ser Trp His Ile Asn Ser Thr Ala Leu Asn Cys Asn Glu Ser
            420                 425                 430

Leu Asn Thr Gly Trp Leu Ala Gly Leu Phe Tyr Thr His Lys Phe Asn
        435                 440                 445

Ser Ser Gly Cys Pro Glu Arg Leu Ala Ser Cys Arg Arg Leu Thr Asp
    450                 455                 460

Phe Asp Gln Gly Trp Gly Pro Ile Ser Tyr Ala Asn Gly Ser Gly Pro
465                 470                 475                 480

Asp Gln Arg Pro Tyr Cys Trp His Tyr Pro Pro Lys Pro Cys Gly Ile
            485                 490                 495

Val Pro Ala Lys Ser Val Cys Gly Pro Val Tyr Cys Phe Thr Pro Ser
            500                 505                 510

Pro Val Val Val Gly Thr Thr Asp Arg Ser Gly Ala Pro Thr Tyr Ser
        515                 520                 525

Trp Gly Ala Asn Asp Thr Asp Val Phe Val Leu Asn Asn Thr Arg Pro
    530                 535                 540

Pro Leu Gly Asn Trp Phe Gly Cys Thr Trp Met Asn Ser Thr Gly Phe
545                 550                 555                 560

Thr Lys Val Cys Gly Ala Pro Pro Cys Val Ile Gly Gly Val Gly Asn
            565                 570                 575

Asn Thr Leu His Cys Pro Thr Asp Cys Phe Arg Lys His Pro Glu Ala
            580                 585                 590

Thr Tyr Ser Arg Cys Gly Ser Gly Pro Trp Ile Thr Pro Arg Cys Leu
        595                 600                 605

Val Asp Tyr Pro Tyr Arg Leu Trp His Tyr Pro Cys Thr Ile Asn Tyr
    610                 615                 620

Thr Ile Phe Lys Val Arg Met Tyr Val Gly Gly Val Glu His Arg Leu
625                 630                 635                 640

Glu Ala Ala Cys Asn Trp Thr Arg Gly Glu Arg Cys Asp Leu Glu Asp
                    645                 650                 655

Arg Asp Arg Ser Glu Leu Ser Pro Leu Leu Leu Ser Thr Thr Gln Trp
                660                 665                 670

Gln Val Leu Pro Cys Ser Phe Thr Thr Leu Pro Ala Leu Ser Thr Gly
                675                 680                 685

Leu Ile His Leu His Gln Asn Ile Val Asp Val Gln Tyr Leu Tyr Gly
        690                 695                 700

Val Gly Ser Ser Ile Ala Ser Trp Ala Ile Lys Trp Glu Tyr Val Val
705                 710                 715                 720

Leu Leu Phe Leu Leu Leu Ala Asp Ala Arg Val Cys Ser Cys Leu Trp
                725                 730                 735

Met Met Leu Leu Ile Ser Gln Ala Glu Ala Ala Leu Glu Asn Leu Val
                740                 745                 750

Ile Leu Asn Ala Ala Ser Leu Ala Gly Thr His Gly Leu Val Ser Phe
            755                 760                 765

Leu Val Phe Phe Cys Phe Ala Trp Tyr Leu Lys Gly Arg Trp Val Pro
        770                 775                 780

Gly Ala Val Tyr Ala Leu Tyr Gly Met Trp Pro Leu Leu Leu Leu Leu
785                 790                 795                 800

Leu Ala Leu Pro Gln Arg Ala Tyr Ala Leu Asp Thr Glu Val Ala Ala
                805                 810                 815

Ser Cys Gly Gly Val Val Leu Val Gly Leu Met Ala Leu Thr Leu Ser
            820                 825                 830

Pro Tyr Tyr Lys Arg Tyr Ile Ser Trp Cys Leu Trp Trp Leu Gln Tyr
        835                 840                 845

Phe Leu Thr Arg Val Glu Ala Gln Leu His Val Trp Val Pro Pro Leu
    850                 855                 860

Asn Val Arg Gly Gly Arg Asp Ala Val Ile Leu Leu Met Cys Val Val
865                 870                 875                 880

His Pro Thr Leu Val Phe Asp Ile Thr Lys Leu Leu Leu Ala Val Phe
                885                 890                 895

Gly Pro Leu Trp Ile Leu Gln Ala Ser Leu Leu Lys Val Pro Tyr Phe
                900                 905                 910

338

```
Val Arg Val Gln Gly Leu Leu Arg Ile Cys Ala Leu Ala Arg Lys Met
        915                 920                 925

Ala Gly Gly His Tyr Val Gln Met Ala Ile Ile Lys Leu Gly Ala Leu
    930                 935                 940

Thr Gly Thr Tyr Val Tyr Asn His Leu Thr Pro Leu Arg Asp Trp Ala
945                 950                 955                 960

His Asn Gly Leu Arg Asp Leu Ala Val Ala Val Glu Pro Val Val Phe
                965                 970                 975

Ser Gln Met Glu Thr Lys Leu Ile Thr Trp Gly Ala Asp Thr Ala Ala
                980                 985                 990

Cys Gly Asp Ile Ile Asn Gly Leu Pro Val Ser Ala Arg Arg Gly Arg
            995                 1000                1005

Glu Ile Leu Leu Gly Pro Ala Asp Gly Met Val Ser Lys Gly Trp
    1010                1015                1020

Arg Leu Leu Ala Pro Ile Thr Ala Tyr Ala Gln Gln Thr Arg Gly
    1025                1030                1035

Leu Leu Gly Cys Ile Ile Thr Ser Leu Thr Gly Arg Asp Lys Asn
    1040                1045                1050

Gln Val Glu Gly Glu Val Gln Ile Val Ser Thr Ala Ala Gln Thr
    1055                1060                1065

Phe Leu Ala Thr Cys Ile Asn Gly Val Cys Trp Thr Val Tyr His
    1070                1075                1080

Gly Ala Gly Thr Arg Thr Ile Ala Ser Pro Lys Gly Pro Val Ile
    1085                1090                1095

Gln Met Tyr Thr Asn Val Asp Gln Asp Leu Val Gly Trp Pro Ala
    1100                1105                1110

Pro Gln Gly Ala Arg Ser Leu Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Ile
    1145                1150                1155

Ser Tyr Leu Lys Gly Ser Ser Gly Gly Pro Leu Leu Cys Pro Ala
    1160                1165                1170

Gly His Ala Val Gly Ile Phe Arg Ala Ala Val Cys Thr Arg Gly
    1175                1180                1185
```

```
Val Ala Lys Ala Val Asp Phe Ile Pro Val Glu Asn Leu Glu Thr
    1190                1195            1200

Thr Met Arg Ser Pro Val Phe Thr Asp Asn Ser Ser Pro Pro Ala
    1205                1210            1215

Val Pro Gln Ser Phe Gln Val Ala His Leu His Ala Pro Thr Gly
    1220                1225            1230

Ser Gly Lys Ser Thr Lys Val Pro Ala Ala Tyr Ala Ala Gln Gly
    1235                1240            1245

Tyr Lys Val Leu Val Leu Asn Pro Ser Val Ala Ala Thr Leu Gly
    1250                1255            1260

Phe Gly Ala Tyr Met Ser Lys Ala His Gly Ile Asp Pro Asn Ile
    1265                1270            1275

Arg Thr Gly Val Arg Thr Ile Thr Thr Gly Ser Pro Ile Thr Tyr
    1280                1285            1290

Ser Thr Tyr Gly Lys Phe Leu Ala Asp Gly Gly Cys Ser Gly Gly
    1295                1300            1305

Ala Tyr Asp Ile Ile Ile Cys Asp Glu Cys His Ser Thr Asp Ala
    1310                1315            1320

Thr Ser Ile Leu Gly Ile Gly Thr Val Leu Asp Gln Ala Glu Thr
    1325                1330            1335

Ala Gly Ala Arg Leu Val Val Leu Ala Thr Ala Thr Pro Pro Gly
    1340                1345            1350

Ser Val Thr Val Pro His Pro Asn Ile Glu Glu Val Ala Leu Ser
    1355                1360            1365

Thr Thr Gly Glu Ile Pro Phe Tyr Gly Lys Ala Ile Pro Leu Glu
    1370                1375            1380

Val Ile Lys Gly Gly Arg His Leu Ile Phe Cys His Ser Lys Lys
    1385                1390            1395

Lys Cys Asp Glu Leu Ala Ala Lys Leu Val Ala Leu Gly Ile Asn
    1400                1405            1410

Ala Val Ala Tyr Tyr Arg Gly Leu Asp Val Ser Val Ile Pro Thr
    1415                1420            1425

Ser Gly Asp Val Val Val Val Ala Thr Asp Ala Leu Met Thr Gly
    1430                1435            1440
```

```
Phe Thr  Gly Asp Phe Asp Ser  Val Ile Asp Cys Asn  Thr Cys Val
    1445                 1450             1455

Thr Gln  Thr Val Asp Phe Ser  Leu Asp Pro Thr Phe  Thr Ile Glu
    1460                 1465             1470

Thr Thr  Thr Leu Pro Gln Asp  Ala Val Ser Arg Thr  Gln Arg Arg
    1475                 1480             1485

Gly Arg  Thr Gly Arg Gly Lys  Pro Gly Ile Tyr Arg  Phe Val Ala
    1490                 1495             1500

Pro Gly  Glu Arg Pro Ser Gly  Met Phe Asp Ser Ser  Val Leu Cys
    1505                 1510             1515

Glu Cys  Tyr Asp Ala Gly Cys  Ala Trp Tyr Glu Leu  Thr Pro Ala
    1520                 1525             1530

Glu Thr  Thr Val Arg Leu Arg  Ala Tyr Met Asn Thr  Pro Gly Leu
    1535                 1540             1545

Pro Val  Cys Gln Asp His Leu  Glu Phe Trp Glu Gly  Val Phe Thr
    1550                 1555             1560

Gly Leu  Thr His Ile Asp Ala  His Phe Leu Ser Gln  Thr Lys Gln
    1565                 1570             1575

Ser Gly  Glu Asn Phe Pro Tyr  Leu Val Ala Tyr Gln  Ala Thr Val
    1580                 1585             1590

Cys Ala  Arg Ala Gln Ala Pro  Pro Pro Ser Trp Asp  Gln Met Trp
    1595                 1600             1605

Lys Cys  Leu Ile Arg Leu Lys  Pro Thr Leu His Gly  Pro Thr Pro
    1610                 1615             1620

Leu Leu  Tyr Arg Leu Gly Ala  Val Gln Asn Glu Val  Thr Leu Thr
    1625                 1630             1635

His Pro  Val Thr Lys Tyr Ile  Met Thr Cys Met Ser  Ala Asp Leu
    1640                 1645             1650

Glu Val  Val Thr Ser Thr Trp  Val Leu Val Gly Gly  Val Leu Ala
    1655                 1660             1665

Ala Leu  Ala Ala Tyr Cys Leu  Ser Thr Gly Cys Val  Val Ile Val
    1670                 1675             1680

Gly Arg  Ile Val Leu Ser Gly  Lys Pro Ala Ile Ile  Pro Asp Arg
    1685                 1690             1695

Glu Val  Leu Tyr Arg Glu Phe  Asp Glu Met Glu Glu  Cys Ser Gln
    1700                 1705             1710
```

His Leu Pro Tyr Ile Glu Gln Gly Met Met Leu Ala Glu Gln Phe
1715 1720 1725

Lys Gln Lys Ala Leu Gly Leu Leu Gln Thr Ala Ser Arg Gln Ala
1730 1735 1740

Glu Val Ile Ala Pro Ala Val Gln Thr Asn Trp Gln Lys Leu Glu
1745 1750 1755

Ala Phe Trp Ala Lys His Met Trp Asn Phe Ile Ser Gly Ile Gln
1760 1765 1770

Tyr Leu Ala Gly Leu Ser Thr Leu Pro Gly Asn Pro Ala Ile Ala
1775 1780 1785

Ser Leu Met Ala Phe Thr Ala Ala Val Thr Ser Pro Leu Thr Thr
1790 1795 1800

Ser Gln Thr Leu Leu Phe Asn Ile Leu Gly Gly Trp Val Ala Ala
1805 1810 1815

Gln Leu Ala Ala Pro Gly Ala Ala Thr Ala Phe Val Gly Ala Gly
1820 1825 1830

Leu Ala Gly Ala Ala Ile Gly Ser Val Gly Leu Gly Lys Val Leu
1835 1840 1845

Val Asp Ile Leu Ala Gly Tyr Gly Ala Gly Val Ala Gly Ala Leu
1850 1855 1860

Val Ala Phe Lys Ile Met Ser Gly Glu Val Pro Ser Thr Glu Asp
1865 1870 1875

Leu Val Asn Leu Leu Pro Ala Ile Leu Ser Pro Gly Ala Leu Val
1880 1885 1890

Val Gly Val Val Cys Ala Ala Ile Leu Arg Arg His Val Gly Pro
1895 1900 1905

Gly Glu Gly Ala Val Gln Trp Met Asn Arg Leu Ile Ala Phe Ala
1910 1915 1920

Ser Arg Gly Asn His Val Ser Pro Thr His Tyr Val Pro Glu Ser
1925 1930 1935

Asp Ala Ala Ala Arg Val Thr Ala Ile Leu Ser Ser Leu Thr Val
1940 1945 1950

Thr Gln Leu Leu Arg Arg Leu His Gln Trp Ile Ser Ser Glu Cys
1955 1960 1965

```
Thr Thr Pro Cys Ser Gly Ser  Trp Leu Arg Asp Ile  Trp Asp Trp
    1970            1975              1980

Ile Cys Glu Val Leu Ser Asp  Phe Lys Thr Trp Leu  Lys Ala Lys
    1985            1990              1995

Leu Met Pro Gln Leu Pro Gly  Ile Pro Phe Val Ser  Cys Gln Arg
    2000            2005              2010

Gly Tyr Arg Gly Val Trp Arg  Gly Asp Gly Ile Met  His Thr Arg
    2015            2020              2025

Cys His Cys Gly Ala Glu Ile  Thr Gly His Val Lys  Asn Gly Thr
    2030            2035              2040

Met Arg Ile Val Gly Pro Lys  Thr Cys Arg Asn Met  Trp Ser Gly
    2045            2050              2055

Thr Phe Pro Ile Asn Ala Tyr  Thr Thr Gly Pro Cys  Thr Pro Leu
    2060            2065              2070

Pro Ala Pro Asn Tyr Thr Phe  Ala Leu Trp Arg Val  Ser Ala Glu
    2075            2080              2085

Glu Tyr Val Glu Ile Arg Arg  Val Gly Asp Phe His  Tyr Val Thr
    2090            2095              2100

Gly Met Thr Thr Asp Asn Leu  Lys Cys Pro Cys Gln  Val Pro Ser
    2105            2110              2115

Pro Glu Phe Phe Thr Glu Leu  Asp Gly Val Arg Leu  His Arg Phe
    2120            2125              2130

Ala Pro Pro Cys Lys Pro Leu  Leu Arg Glu Glu Val  Ser Phe Arg
    2135            2140              2145

Val Gly Leu His Glu Tyr Pro  Val Gly Ser Gln Leu  Pro Cys Glu
    2150            2155              2160

Pro Glu Pro Asp Val Ala Val  Leu Thr Ser Met Leu  Thr Asp Pro
    2165            2170              2175

Ser His Ile Thr Ala Glu Ala  Ala Gly Arg Arg Leu  Ala Arg Gly
    2180            2185              2190

Ser Pro Pro Ser Met Ala Ser  Ser Ser Ala Ser Gln  Leu Ser Ala
    2195            2200              2205

Pro Ser Leu Lys Ala Thr Cys  Thr Ala Asn His Asp  Ser Pro Asp
    2210            2215              2220

Ala Glu Leu Ile Glu Ala Asn  Leu Leu Trp Arg Gln  Glu Met Gly
    2225            2230              2235
```

Gly Asn Ile Thr Arg Val Glu Ser Glu Asn Lys Val Val Ile Leu
2240 2245 2250

Asp Ser Phe Asp Pro Leu Val Ala Glu Glu Asp Glu Arg Glu Ile
2255 2260 2265

Ser Val Pro Ala Glu Ile Leu Arg Lys Ser Arg Arg Phe Ala Gln
2270 2275 2280

Ala Leu Pro Val Trp Ala Arg Pro Asp Tyr Asn Pro Pro Leu Leu
2285 2290 2295

Glu Thr Trp Lys Lys Pro Asp Tyr Glu Pro Pro Val Val His Gly
2300 2305 2310

Cys Pro Leu Pro Pro Pro Arg Ser Pro Pro Val Pro Pro Pro Arg
2315 2320 2325

Lys Lys Arg Thr Val Val Leu Thr Glu Ser Thr Leu Ser Thr Ala
2330 2335 2340

Leu Ala Glu Leu Ala Thr Lys Ser Phe Gly Ser Ser Ser Thr Ser
2345 2350 2355

Gly Ile Thr Gly Asp Asn Thr Thr Thr Ser Ser Glu Pro Ala Pro
2360 2365 2370

Ser Gly Cys Pro Pro Asp Ser Asp Ala Glu Ser Tyr Ser Ser Met
2375 2380 2385

Pro Pro Leu Glu Gly Glu Pro Gly Asp Pro Asp Leu Ser Asp Gly
2390 2395 2400

Ser Trp Ser Thr Val Ser Ser Glu Ala Ser Thr Glu Asp Val Val
2405 2410 2415

Cys Cys Ser Met Ser Tyr Ser Trp Thr Gly Ala Leu Val Thr Pro
2420 2425 2430

Cys Ala Ala Glu Glu Gln Lys Leu Pro Ile Asn Ala Leu Ser Asn
2435 2440 2445

Ser Leu Leu Arg His His Asn Leu Val Tyr Ser Thr Thr Ser Arg
2450 2455 2460

Ser Ala Cys Gln Arg Gln Lys Lys Val Thr Phe Asp Arg Leu Gln
2465 2470 2475

Val Leu Asp Ser His Tyr Gln Asp Val Leu Lys Glu Val Lys Ala
2480 2485 2490

344

```
Ala Ala Ser Lys Val Lys Ala Asn Leu Leu Ser Val Glu Glu Ala
    2495            2500              2505

Cys Ser Leu Thr Pro Pro His Ser Ala Lys Ser Lys Phe Gly Tyr
    2510            2515              2520

Gly Ala Lys Asp Val Arg Cys His Ala Arg Lys Ala Val Asn His
    2525            2530              2535

Ile Asn Ser Val Trp Lys Asp Leu Leu Glu Asp Ser Val Thr Pro
    2540            2545              2550

Ile Asp Thr Thr Ile Met Ala Lys Asn Glu Val Phe Cys Val Gln
    2555            2560              2565

Pro Glu Lys Gly Gly Arg Lys Pro Ala Arg Leu Ile Val Phe Pro
    2570            2575              2580

Asp Leu Gly Val Arg Val Cys Glu Lys Met Ala Leu Tyr Asp Val
    2585            2590              2595

Val Ser Lys Leu Pro Leu Ala Val Met Gly Ser Ser Tyr Gly Phe
    2600            2605              2610

Gln Tyr Ser Pro Gly Gln Arg Val Glu Phe Leu Val Gln Ala Trp
    2615            2620              2625

Lys Ser Lys Lys Thr Pro Met Gly Phe Ser Tyr Asp Thr Arg Cys
    2630            2635              2640

Phe Asp Ser Thr Val Thr Glu Ser Asp Ile Arg Thr Glu Glu Ala
    2645            2650              2655

Ile Tyr Gln Cys Cys Asp Leu Asp Pro Gln Ala Arg Val Ala Ile
    2660            2665              2670

Lys Ser Leu Thr Glu Arg Leu Tyr Val Gly Gly Pro Leu Thr Asn
    2675            2680              2685

Ser Arg Gly Glu Asn Cys Gly Tyr Arg Arg Cys Arg Ala Ser Gly
    2690            2695              2700

Val Leu Thr Thr Ser Cys Gly Asn Thr Leu Thr Cys Tyr Ile Lys
    2705            2710              2715

Ala Arg Ala Ala Cys Arg Ala Ala Gly Leu Gln Asp Cys Thr Met
    2720            2725              2730

Leu Val Cys Gly Asp Asp Leu Val Val Ile Cys Glu Ser Ala Gly
    2735            2740              2745

Val Gln Glu Asp Ala Ala Ser Leu Arg Ala Phe Thr Glu Ala Met
    2750            2755              2760
```

Thr Arg Tyr Ser Ala Pro Pro Gly Asp Pro Pro Gln Pro Glu Tyr
    2765            2770            2775

Asp Leu Glu Leu Ile Thr Ser Cys Ser Ser Asn Val Ser Val Ala
    2780            2785            2790

His Asp Gly Ala Gly Lys Arg Val Tyr Tyr Leu Thr Arg Asp Pro
    2795            2800            2805

Thr Thr Pro Leu Ala Arg Ala Ala Trp Glu Thr Ala Arg His Thr
    2810            2815            2820

Pro Val Asn Ser Trp Leu Gly Asn Ile Ile Met Phe Ala Pro Thr
    2825            2830            2835

Leu Trp Ala Arg Met Ile Leu Met Thr His Phe Phe Ser Val Leu
    2840            2845            2850

Ile Ala Arg Asp Gln Leu Glu Gln Ala Leu Asp Cys Glu Ile Tyr
    2855            2860            2865

Gly Ala Cys Tyr Ser Ile Glu Pro Leu Asp Leu Pro Pro Ile Ile
    2870            2875            2880

Gln Arg Leu His Gly Leu Ser Ala Phe Ser Leu His Ser Tyr Ser
    2885            2890            2895

Pro Gly Glu Ile Asn Arg Val Ala Ala Cys Leu Arg Lys Leu Gly
    2900            2905            2910

Val Pro Pro Leu Arg Ala Trp Arg His Arg Ala Arg Ser Val Arg
    2915            2920            2925

Ala Arg Leu Leu Ser Arg Gly Gly Arg Ala Ala Ile Cys Gly Lys
    2930            2935            2940

Tyr Leu Phe Asn Trp Ala Val Arg Thr Lys Leu Lys Leu Thr Pro
    2945            2950            2955

Ile Ala Ala Ala Gly Arg Leu Asp Leu Ser Gly Trp Phe Thr Ala
    2960            2965            2970

Gly Tyr Ser Gly Gly Asp Ile Tyr His Ser Val Ser His Ala Arg
    2975            2980            2985

Pro Arg Trp Phe Trp Phe Cys Leu Leu Leu Leu Ala Ala Gly Val
    2990            2995            3000

Gly Ile Tyr Leu Leu Pro Asn Arg
    3005            3010

```
<210>   772
<211>   3020
<212>   PRT
<213>   hepatitis C virus

<400>   772
Met Ser Thr Leu Pro Lys Pro Gln Arg Lys Thr Lys Arg Asn Thr Ile
1           5                   10                  15

Arg Arg Pro Gln Asp Val Lys Phe Pro Gly Gly Gly Gln Ile Val Gly
                20                  25                  30

Gly Val Tyr Val Leu Pro Arg Arg Gly Pro Arg Leu Gly Val Arg Ala
            35                  40                  45

Thr Arg Lys Thr Ser Glu Arg Ser Gln Pro Arg Gly Arg Arg Gln Pro
        50                  55                  60

Ile Pro Lys Ala Arg Arg Ser Glu Gly Arg Ser Trp Ala Gln Pro Gly
65                  70                  75                  80

Tyr Pro Trp Pro Leu Tyr Gly Asn Glu Gly Cys Gly Trp Ala Gly Trp
                85                  90                  95

Leu Leu Ser Pro Arg Gly Ser Arg Pro Ser Trp Gly Pro Asn Asp Pro
                100                 105                 110

Arg Arg Arg Ser Arg Asn Leu Gly Lys Val Ile Asp Thr Leu Thr Cys
            115                 120                 125

Gly Phe Ala Asp Leu Met Gly Tyr Ile Pro Leu Val Gly Ala Pro Val
        130                 135                 140

Gly Gly Val Ala Arg Ala Leu Ala His Gly Val Arg Ala Leu Glu Asp
145                 150                 155                 160

Gly Ile Asn Phe Ala Thr Gly Asn Leu Pro Gly Cys Ser Phe Ser Ile
                165                 170                 175

Phe Leu Leu Ala Leu Phe Ser Cys Leu Ile His Pro Ala Ala Ser Leu
                180                 185                 190

Glu Trp Arg Asn Thr Ser Gly Leu Tyr Val Leu Thr Asn Asp Cys Ser
            195                 200                 205

Asn Ser Ser Ile Val Tyr Glu Ala Asp Asp Val Ile Leu His Thr Pro
        210                 215                 220

Gly Cys Val Pro Cys Val Gln Asp Gly Asn Thr Ser Thr Cys Trp Thr
225                 230                 235                 240
```

EP 1 652 858 A1

```
Pro Val Thr Pro Thr Val Ala Val Arg Tyr Val Gly Ala Thr Thr Ala
                245                 250                 255

Ser Ile Arg Ser His Val Asp Leu Leu Val Gly Ala Ala Thr Met Cys
            260             265                 270

Ser Ala Leu Tyr Val Gly Asp Met Cys Gly Ala Val Phe Leu Val Gly
        275             280                 285

Gln Ala Phe Thr Phe Arg Pro Arg Arg His Gln Thr Val Gln Thr Cys
    290             295                 300

Asn Cys Ser Leu Tyr Pro Gly His Leu Ser Gly His Arg Met Ala Trp
305             310             315                 320

Asp Met Met Met Asn Trp Ser Pro Ala Val Gly Met Val Val Ala His
                325             330                 335

Val Leu Arg Leu Pro Gln Thr Leu Phe Asp Ile Ile Ala Gly Ala His
            340             345                 350

Trp Gly Ile Leu Ala Gly Leu Ala Tyr Tyr Ser Met Gln Gly Asn Trp
        355             360                 365

Ala Lys Val Ala Ile Ile Met Val Met Phe Ser Gly Val Asp Ala Thr
    370             375                 380

Thr Tyr Thr Thr Gly Gly Ser Ala Ala Arg Thr Thr Ser Gly Leu Thr
385             390                 395                 400

Ser Leu Phe Ser Val Gly Pro Gln Gln Lys Leu Gln Leu Val Asn Thr
            405             410                 415

Asn Gly Ser Trp His Ile Asn Ser Thr Ala Leu Asn Cys Asn Glu Ser
            420             425                 430

Ile Asn Thr Gly Phe Ile Ala Gly Leu Phe Tyr Tyr His Lys Phe Asn
        435             440                 445

Ser Thr Gly Cys Pro Gln Arg Leu Ser Ser Cys Lys Pro Ile Thr Phe
    450             455                 460

Phe Ala Gln Gly Trp Gly Pro Leu Thr Asp Ala Asn Ile Thr Gly Pro
465             470                 475                 480

Ser Asp Asp Lys Pro Tyr Cys Trp His Tyr Ala Pro Arg Pro Cys Asp
            485             490                 495

Ile Val Pro Ala Ser Asn Val Cys Gly Pro Val Tyr Cys Phe Thr Pro
            500             505                 510
```

348

Ser Pro Val Val Val Gly Thr Thr Asp Ala Lys Gly Val Pro Thr Tyr
515                      520                      525

Thr Trp Gly Glu Asn Glu Thr Asp Val Phe Leu Leu Glu Ser Leu Arg
530                      535                      540

Pro Pro Ser Gly Arg Trp Phe Gly Cys Thr Trp Met Asn Ser Thr Gly
545                550                      555                560

Phe Thr Lys Thr Cys Gly Ala Pro Pro Cys Asn Ile Tyr Gly Gly Gly
565                      570                      575

Gly Asn Pro Asn Glu Ser Asp Leu Phe Cys Pro Thr Asp Cys Phe Arg
580                      585                      590

Lys His Pro Glu Ala Thr Tyr Ser Arg Cys Gly Ala Gly Pro Trp Leu
595                      600                      605

Thr Pro Arg Cys Met Val Asp Tyr Pro Tyr Arg Leu Trp His Tyr Pro
610                615                      620

Cys Thr Val Asn Phe Thr Leu Phe Lys Val Arg Met Phe Val Gly Gly
625                630                      635                640

Phe Glu His Arg Phe Thr Ala Ala Cys Asn Trp Thr Arg Gly Glu Arg
645                      650                      655

Cys Asp Ile Glu Asp Arg Asp Arg Ser Glu Gln His Pro Leu Leu His
660                      665                      670

Ser Thr Thr Glu Leu Ala Ile Leu Pro Cys Ser Phe Thr Pro Met Pro
675                      680                      685

Ala Leu Ser Thr Gly Leu Ile His Leu His Gln Asn Ile Val Asp Val
690                695                      700

Gln Tyr Leu Tyr Gly Val Gly Ser Gly Met Val Gly Trp Ala Leu Lys
705                710                      715                720

Trp Glu Phe Val Ile Leu Ile Phe Leu Leu Leu Ala Asp Ala Arg Val
725                      730                      735

Cys Val Ala Leu Trp Leu Met Leu Met Ile Ser Gln Ala Glu Ala Ala
740                      745                      750

Leu Glu Asn Leu Val Thr Leu Asn Ala Val Ala Ala Ala Gly Thr His
755                      760                      765

Gly Ile Gly Trp Tyr Leu Val Ala Phe Cys Ala Ala Trp Tyr Val Arg
770                775                      780

Gly Lys Leu Val Pro Leu Val Thr Tyr Ser Leu Thr Gly Leu Trp Ser
785                790                      795                800

```
Leu Ala Leu Leu Val Leu Leu Leu Pro Gln Arg Ala Tyr Ala Trp Ser
            805             810             815

Gly Glu Asp Ser Ala Thr Leu Gly Ala Gly Val Leu Val Leu Phe Gly
            820             825             830

Phe Phe Thr Leu Ser Pro Trp Tyr Lys His Trp Ile Gly Arg Leu Ile
            835             840             845

Trp Trp Asn Gln Tyr Thr Ile Cys Arg Cys Glu Ser Ala Leu Gln Val
    850             855             860

Trp Val Pro Pro Leu Leu Ala Arg Gly Ser Arg Asp Gly Val Ile Leu
865             870             875             880

Leu Thr Ser Leu Leu Tyr Pro Ser Leu Ile Phe Asp Ile Thr Lys Leu
            885             890             895

Leu Ile Ala Val Leu Gly Pro Leu Tyr Leu Ile Gln Ala Thr Ile Thr
            900             905             910

Arg Thr Pro Tyr Phe Val Arg Ala His Val Leu Val Arg Leu Cys Met
            915             920             925

Leu Val Arg Ser Val Met Gly Gly Lys Tyr Phe Gln Met Ile Ile Leu
    930             935             940

Ser Ile Gly Arg Trp Phe Asn Thr Tyr Leu Tyr Asp His Leu Ala Pro
945             950             955             960

Met Gln His Trp Ala Ala Ala Gly Leu Lys Asp Leu Ala Val Ala Thr
            965             970             975

Glu Pro Val Ile Phe Ser Pro Met Glu Ile Lys Val Ile Thr Trp Gly
            980             985             990

Ala Asp Thr Ala Ala Cys Gly Asp Ile Leu Cys Gly Leu Pro Val Ser
            995             1000            1005

Ala Arg Leu Gly Arg Glu Val Leu Leu Gly Pro Ala Asp Asp Tyr
    1010            1015            1020

Arg Glu Met Gly Trp Arg Leu Leu Ala Pro Ile Thr Ala Tyr Ala
    1025            1030            1035

Gln Gln Thr Arg Gly Leu Leu Gly Thr Ile Val Thr Ser Leu Thr
    1040            1045            1050

Gly Arg Asp Lys Asn Val Val Thr Gly Glu Val Gln Val Leu Ser
    1055            1060            1065
```

350

```
Thr Ala  Thr Gln Thr Phe Leu  Gly Thr Thr Val Gly  Gly Val Met
    1070             1075             1080

Trp Thr  Val Tyr His Gly Ala  Gly Ser Arg Thr Leu  Ala Gly Ala
    1085             1090             1095

Lys His  Pro Ala Leu Gln Met  Tyr Thr Asn Val Asp  Gln Asp Leu
    1100             1105             1110

Val Gly  Trp Pro Ala Pro Pro  Gly Ala Lys Ser Leu  Glu Pro Cys
    1115             1120             1125

Ala Cys  Gly Ser Ala Asp Leu  Tyr Leu Val Thr Arg  Asp Ala Asp
    1130             1135             1140

Val Ile  Pro Ala Arg Arg Arg  Gly Asp Ser Thr Ala  Ser Leu Leu
    1145             1150             1155

Ser Pro  Arg Pro Leu Ala Cys  Leu Lys Gly Ser Ser  Gly Gly Pro
    1160             1165             1170

Val Met  Cys Pro Ser Gly His  Val Ala Gly Ile Phe  Arg Ala Ala
    1175             1180             1185

Val Cys  Thr Arg Gly Val Ala  Lys Ala Leu Gln Phe  Ile Pro Val
    1190             1195             1200

Glu Thr  Leu Ser Thr Gln Ala  Arg Ser Pro Ser Phe  Ser Asp Asn
    1205             1210             1215

Ser Thr  Pro Pro Ala Val Pro  Gln Ser Tyr Gln Val  Gly Tyr Leu
    1220             1225             1230

His Ala  Pro Thr Gly Ser Gly  Lys Ser Thr Lys Val  Pro Ala Ala
    1235             1240             1245

Tyr Val  Ala Gln Gly Tyr Asn  Val Leu Val Leu Asn  Pro Ser Val
    1250             1255             1260

Ala Ala  Thr Leu Gly Phe Gly  Ser Phe Met Ser Arg  Ala Tyr Gly
    1265             1270             1275

Ile Asp  Pro Asn Ile Arg Thr  Gly Asn Arg Thr Val  Thr Thr Gly
    1280             1285             1290

Ala Lys  Leu Thr Tyr Ser Thr  Tyr Gly Lys Phe Leu  Ala Asp Gly
    1295             1300             1305

Gly Cys  Ser Gly Gly Ala Tyr  Asp Val Ile Ile Cys  Asp Glu Cys
    1310             1315             1320

His Ala  Gln Asp Ala Thr Ser  Ile Leu Gly Ile Gly  Thr Val Leu
    1325             1330             1335
```

```
Asp Gln Ala Glu Thr Ala Gly Val Arg Leu Thr Val Leu Ala Thr
    1340            1345            1350

Ala Thr Pro Pro Gly Ser Ile Thr Val Pro His Ser Asn Ile Glu
    1355            1360            1365

Glu Val Ala Leu Gly Ser Glu Gly Glu Ile Pro Phe Tyr Gly Lys
    1370            1375            1380

Ala Ile Pro Ile Ala Leu Leu Lys Gly Gly Arg His Leu Ile Phe
    1385            1390            1395

Cys His Ser Lys Lys Lys Cys Asp Glu Ile Ala Ser Lys Leu Arg
    1400            1405            1410

Gly Met Gly Leu Asn Ala Val Ala Tyr Tyr Arg Gly Leu Asp Val
    1415            1420            1425

Ser Val Ile Pro Thr Thr Gly Asp Val Val Val Cys Ala Thr Asp
    1430            1435            1440

Ala Leu Met Thr Gly Phe Thr Gly Asp Phe Asp Ser Val Ile Asp
    1445            1450            1455

Cys Asn Val Ala Val Glu Gln Tyr Val Asp Phe Ser Leu Asp Pro
    1460            1465            1470

Thr Phe Ser Ile Glu Thr Arg Thr Ala Pro Gln Asp Ala Val Ser
    1475            1480            1485

Arg Ser Gln Arg Arg Gly Arg Thr Gly Arg Gly Arg Leu Gly Thr
    1490            1495            1500

Tyr Arg Tyr Val Ala Pro Gly Glu Arg Pro Ser Gly Met Phe Asp
    1505            1510            1515

Ser Val Val Leu Cys Glu Cys Tyr Asp Ala Gly Cys Ser Trp Tyr
    1520            1525            1530

Asp Leu Gln Pro Ala Glu Thr Thr Val Arg Leu Arg Ala Tyr Leu
    1535            1540            1545

Ser Thr Pro Gly Leu Pro Val Cys Gln Asp His Leu Asp Phe Trp
    1550            1555            1560

Glu Ser Val Phe Thr Gly Leu Thr His Ile Asp Ala His Phe Leu
    1565            1570            1575

Ser Gln Thr Lys Gln Gln Gly Leu Asn Phe Ser Tyr Leu Thr Ala
    1580            1585            1590
```

```
Tyr Gln Ala Thr Val Cys Ala Arg Ala Gln Ala Pro Pro Pro Ser
    1595            1600            1605

Trp Asp Glu Met Trp Lys Cys Leu Val Arg Leu Lys Pro Thr Leu
    1610            1615            1620

His Gly Pro Thr Pro Leu Leu Tyr Arg Leu Gly Pro Val Gln Asn
    1625            1630            1635

Glu Val Cys Leu Thr His Pro Ile Thr Lys Tyr Val Met Ala Cys
    1640            1645            1650

Met Ser Ala Asp Leu Glu Val Thr Thr Ser Thr Trp Val Leu Leu
    1655            1660            1665

Gly Gly Val Leu Ala Ala Leu Ala Ala Tyr Cys Leu Ser Val Gly
    1670            1675            1680

Cys Val Val Ile Val Gly His Ile Glu Leu Gly Gly Lys Pro Ala
    1685            1690            1695

Leu Val Pro Asp Lys Glu Val Leu Tyr Gln Gln Tyr Asp Glu Met
    1700            1705            1710

Glu Glu Cys Ser Gln Ala Ala Pro Tyr Ile Glu Gln Ala Gln Gln
    1715            1720            1725

Ile Ala His Gln Phe Lys Glu Lys Val Leu Gly Leu Leu Gln Arg
    1730            1735            1740

Ala Thr Gln Gln Gln Ala Val Ile Glu Pro Ile Val Ala Thr Asn
    1745            1750            1755

Trp Gln Lys Leu Glu Ala Phe Trp His Lys His Met Trp Asn Phe
    1760            1765            1770

Val Ser Gly Ile Gln Tyr Leu Ala Gly Leu Ser Thr Leu Pro Gly
    1775            1780            1785

Asn Pro Ala Val Ala Ser Leu Met Ala Phe Thr Ala Ser Val Thr
    1790            1795            1800

Ser Pro Leu Thr Thr Asn Gln Thr Met Phe Phe Asn Ile Leu Gly
    1805            1810            1815

Gly Trp Val Ala Thr His Leu Ala Gly Pro Gln Ser Ser Ser Ala
    1820            1825            1830

Phe Val Val Ser Gly Leu Ala Gly Ala Ala Ile Gly Gly Ile Gly
    1835            1840            1845

Leu Gly Arg Val Leu Leu Asp Ile Leu Ala Gly Tyr Gly Ala Gly
    1850            1855            1860
```

```
Val Ser Gly Ala Leu Val Ala Phe Lys Ile Met Gly Gly Glu Leu
    1865                1870            1875

Pro Thr Thr Glu Asp Met Val Asn Leu Leu Pro Ala Ile Leu Ser
    1880                1885            1890

Pro Gly Ala Leu Val Val Gly Val Ile Cys Ala Ala Ile Leu Arg
    1895                1900            1905

Arg His Val Gly Pro Gly Glu Gly Ala Val Gln Trp Met Asn Arg
    1910                1915            1920

Leu Ile Ala Phe Ala Ser Arg Gly Asn His Val Ser Pro Thr His
    1925                1930            1935

Tyr Val Pro Glu Ser Asp Ala Ala Ala Arg Val Thr Ala Leu Leu
    1940                1945            1950

Ser Ser Leu Thr Val Thr Ser Leu Leu Arg Arg Leu His Gln Trp
    1955                1960            1965

Ile Asn Glu Asp Tyr Pro Ser Pro Cys Ser Asp Asp Trp Leu Arg
    1970                1975            1980

Ile Ile Trp Asp Trp Val Cys Ser Val Leu Thr Asp Phe Lys Thr
    1985                1990            1995

Trp Leu Ser Ala Lys Ile Met Pro Ala Leu Pro Gly Leu Pro Phe
    2000                2005            2010

Ile Ser Cys Gln Lys Gly Tyr Lys Gly Val Trp Arg Gly Asp Gly
    2015                2020            2025

Val Met Ser Thr Arg Cys Pro Cys Gly Ala Thr Ile Thr Gly His
    2030                2035            2040

Val Lys Asn Gly Ser Met Arg Leu Ala Gly Pro Arg Thr Cys Ala
    2045                2050            2055

Asn Met Trp His Gly Thr Phe Pro Ile Asn Glu Tyr Thr Thr Gly
    2060                2065            2070

Pro Ser Thr Pro Cys Pro Ser Pro Asn Tyr Thr Arg Ala Leu Trp
    2075                2080            2085

Arg Val Ala Ala Asn Ser Tyr Val Glu Val Arg Arg Val Gly Asp
    2090                2095            2100

Phe His Tyr Ile Thr Gly Ala Thr Glu Asp Glu Leu Lys Cys Pro
    2105                2110            2115
```

354

```
Cys Gln Val Pro Ala Ala Glu Phe Phe Thr Glu Val Asp Gly Val
    2120                2125            2130

Arg Leu His Arg Tyr Ala Pro Pro Cys Lys Pro Leu Leu Arg Asp
    2135                2140            2145

Glu Ile Thr Phe Met Val Gly Leu Asn Ser Tyr Leu Ile Gly Ser
    2150                2155            2160

Gln Leu Pro Cys Glu Pro Glu Pro Asp Val Ser Val Leu Thr Ser
    2165                2170            2175

Met Leu Arg Asp Pro Ser His Ile Thr Ala Glu Thr Ala Ala Arg
    2180                2185            2190

Arg Leu Ala Arg Gly Ser Pro Pro Ser Glu Ala Ser Ser Ser Ala
    2195                2200            2205

Ser Gln Leu Ser Ala Pro Ser Leu Lys Ala Thr Cys Gln Thr His
    2210                2215            2220

Arg Pro His Pro Asp Ala Glu Leu Val Asp Ala Asn Leu Leu Trp
    2225                2230            2235

Arg Gln Glu Met Gly Ser Asn Ile Thr Arg Val Glu Ser Glu Thr
    2240                2245            2250

Lys Val Val Ile Leu Asp Ser Phe Glu Pro Leu Arg Ala Glu Thr
    2255                2260            2265

Asp Asp Ala Glu Leu Ser Val Ala Ala Glu Cys Phe Lys Lys Pro
    2270                2275            2280

Pro Lys Tyr Pro Pro Ala Leu Pro Ile Trp Ala Arg Pro Asp Tyr
    2285                2290            2295

Asn Pro Pro Leu Leu Asp Arg Trp Lys Ala Pro Asp Tyr Val Pro
    2300                2305            2310

Pro Thr Val His Gly Cys Ala Leu Pro Pro Arg Gly Ala Pro Pro
    2315                2320            2325

Val Pro Pro Pro Arg Arg Lys Arg Thr Ile Gln Leu Asp Gly Ser
    2330                2335            2340

Asn Val Ser Ala Ala Leu Ala Ala Leu Ala Glu Lys Ser Phe Pro
    2345                2350            2355

Ser Ser Lys Pro Gln Glu Glu Asn Ser Ser Ser Ser Gly Val Asp
    2360                2365            2370

Thr Gln Ser Ser Thr Thr Ser Lys Val Pro Pro Ser Pro Gly Gly
    2375                2380            2385
```

355

Glu Ser Asp Ser Glu Ser Cys Ser Ser Met Pro Pro Leu Glu Gly
    2390            2395              2400

Glu Pro Gly Asp Pro Asp Leu Ser Cys Asp Ser Trp Ser Thr Val
    2405            2410              2415

Ser Asp Ser Glu Glu Gln Ser Val Val Cys Cys Ser Met Ser Tyr
    2420            2425              2430

Ser Trp Thr Gly Ala Leu Ile Thr Pro Cys Ser Ala Glu Glu Glu
    2435            2440              2445

Lys Leu Pro Ile Ser Pro Leu Ser Asn Ser Leu Leu Arg His His
    2450            2455              2460

Asn Leu Val Tyr Ser Thr Ser Ser Arg Ser Ala Ser Gln Arg Gln
    2465            2470              2475

Lys Lys Val Thr Phe Asp Arg Leu Gln Val Leu Asp Asp His Tyr
    2480            2485              2490

Lys Thr Ala Leu Lys Glu Val Lys Glu Arg Ala Ser Arg Val Lys
    2495            2500              2505

Ala Arg Met Leu Thr Ile Glu Glu Ala Cys Ala Leu Val Pro Pro
    2510            2515              2520

His Ser Ala Arg Ser Lys Phe Gly Tyr Ser Ala Lys Asp Val Arg
    2525            2530              2535

Ser Leu Ser Ser Arg Ala Ile Asn Gln Ile Arg Ser Val Trp Glu
    2540            2545              2550

Asp Leu Leu Glu Asp Thr Thr Thr Pro Ile Pro Thr Thr Ile Met
    2555            2560              2565

Ala Lys Asn Glu Val Phe Cys Val Asp Pro Ala Lys Gly Gly Arg
    2570            2575              2580

Lys Pro Ala Arg Leu Ile Val Tyr Pro Asp Leu Gly Val Arg Val
    2585            2590              2595

Cys Glu Lys Arg Ala Leu Tyr Asp Val Ile Gln Lys Leu Ser Ile
    2600            2605              2610

Glu Thr Met Gly Ser Ala Tyr Gly Phe Gln Tyr Ser Pro Gln Gln
    2615            2620              2625

Arg Val Glu Arg Leu Leu Lys Met Trp Thr Ser Lys Lys Thr Pro
    2630            2635              2640

```
Leu Gly Phe Ser Tyr Asp Thr Arg Cys Phe Asp Ser Thr Val Thr
    2645                2650                2655

Glu Gln Asp Ile Arg Val Glu Glu Glu Ile Tyr Gln Cys Cys Asn
    2660                2665                2670

Leu Glu Pro Glu Ala Arg Lys Val Ile Ser Ser Leu Thr Glu Arg
    2675                2680                2685

Leu Tyr Cys Gly Gly Pro Met Phe Asn Ser Lys Gly Ala Gln Cys
    2690                2695                2700

Gly Tyr Arg Arg Cys Arg Ala Ser Gly Val Leu Pro Thr Ser Phe
    2705                2710                2715

Gly Asn Thr Ile Thr Cys Tyr Ile Lys Ala Thr Ala Ala Ala Lys
    2720                2725                2730

Ala Ala Gly Leu Arg Asn Pro Asp Phe Leu Val Cys Gly Asp Asp
    2735                2740                2745

Leu Val Val Val Ala Glu Ser Asp Gly Val Asp Glu Asp Arg Ala
    2750                2755                2760

Ala Leu Arg Ala Phe Thr Glu Ala Met Thr Arg Tyr Ser Ala Pro
    2765                2770                2775

Pro Gly Asp Ala Pro Gln Pro Thr Tyr Asp Leu Glu Leu Ile Thr
    2780                2785                2790

Ser Cys Ser Ser Asn Val Ser Val Ala Arg Asp Asp Lys Gly Lys
    2795                2800                2805

Arg Tyr Tyr Tyr Leu Thr Arg Asp Ala Thr Thr Pro Leu Ala Arg
    2810                2815                2820

Ala Ala Trp Glu Thr Ala Arg His Thr Pro Val Asn Ser Trp Leu
    2825                2830                2835

Gly Asn Ile Ile Met Tyr Ala Pro Thr Ile Trp Val Arg Met Val
    2840                2845                2850

Met Met Thr His Phe Phe Ser Ile Leu Gln Ser Gln Glu Ile Leu
    2855                2860                2865

Asp Arg Pro Leu Asp Phe Glu Met Tyr Gly Ala Thr Tyr Ser Val
    2870                2875                2880

Thr Pro Leu Asp Leu Pro Ala Ile Ile Glu Arg Leu His Gly Leu
    2885                2890                2895

Ser Ala Phe Thr Leu His Ser Tyr Ser Pro Val Glu Leu Asn Arg
    2900                2905                2910
```

Val Ala Gly Thr Leu Arg Lys Leu Gly Cys Pro Pro Leu Arg Ala
    2915                2920            2925

Trp Arg His Arg Ala Arg Ala Val Arg Ala Lys Leu Ile Ala Gln
    2930            2935            2940

Gly Gly Lys Ala Lys Ile Cys Gly Leu Tyr Leu Phe Asn Trp Ala
    2945            2950            2955

Val Arg Thr Lys Thr Lys Leu Thr Pro Leu Pro Ala Ala Gly Gln
    2960            2965            2970

Leu Asp Leu Ser Ser Trp Phe Thr Val Gly Val Gly Gly Asn Asp
    2975            2980            2985

Ile Tyr His Ser Val Ser Arg Ala Arg Thr Arg Tyr Leu Leu Leu
    2990            2995            3000

Cys Leu Leu Leu Leu Thr Val Gly Val Gly Ile Phe Leu Leu Pro
    3005            3010            3015

Ala Arg
    3020

<210>  773

<211>  9

<212>  PRT

<213>  hepatitis C virus


<400>  773

Ala Ala Ala Arg Val Thr Gln Ile Leu
1                 5

<210>  774

<211>  10

<212>  PRT

<213>  hepatitis C virus


<400>  774

Ala Ala Cys Gly Asp Ile Ile Leu Gly Leu
1                 5                 10

<210>  775

<211>  10

```
<212>   PRT
<213>   hepatitis C virus


<400>   775

Ala Ala Lys Leu Gln Asp Cys Thr Met Leu
1               5                   10


<210>   776
<211>   9
<212>   PRT
<213>   hepatitis C virus


<400>   776

Ala Ala Leu Glu Asn Leu Val Val Leu
1               5


<210>   777
<211>   10
<212>   PRT
<213>   hepatitis C virus


<400>   777

Ala Ala Gln Gly Tyr Lys Val Leu Val Leu
1               5                   10


<210>   778
<211>   9
<212>   PRT
<213>   hepatitis C virus


<400>   778

Ala Ala Arg Asn Ser Ser Val Pro Thr
1               5


<210>   779
<211>   10
<212>   PRT
<213>   hepatitis C virus
```

<400> 779

Ala Ala Arg Asn Ser Ser Val Pro Thr Thr
1               5                   10

<210> 780

<211> 9

<212> PRT

<213> hepatitis C virus


<400> 780

Ala Ala Arg Thr Thr Ser Gly Phe Thr
1               5

<210> 781

<211> 10

<212> PRT

<213> hepatitis C virus


<400> 781

Ala Ala Thr Leu Gly Phe Gly Ala Tyr Met
1               5                   10

<210> 782

<211> 9

<212> PRT

<213> hepatitis C virus


<400> 782

Ala Ala Trp Tyr Ile Lys Gly Arg Leu
1               5

<210> 783

<211> 10

<212> PRT

<213> hepatitis C virus


<400> 783

Ala Ala Tyr Ala Ala Gln Gly Tyr Lys Val
1               5                   10

```
<210>  784
<211>  9
<212>  PRT
<213>  hepatitis C virus


<400>  784
Ala Cys Gly Asp Ile Ile Leu Gly Leu
1               5


<210>  785
<211>  10
<212>  PRT
<213>  hepatitis C virus


<400>  785
Ala Cys Tyr Ser Ile Glu Pro Leu Asp Leu
1               5                   10


<210>  786
<211>  9
<212>  PRT
<213>  hepatitis C virus


<400>  786
Ala Asp Leu Glu Val Val Thr Ser Thr
1               5


<210>  787
<211>  9
<212>  PRT
<213>  hepatitis C virus


<400>  787
Ala Glu Ala Ala Ala Arg Arg Leu Ala
1               5


<210>  788
<211>  9
<212>  PRT
```

EP 1 652 858 A1

<213> hepatitis C virus

<400> 788

Ala Glu Ala Ala Leu Glu Lys Leu Val
1               5

<210> 789
<211> 10
<212> PRT
<213> hepatitis C virus

<400> 789

Ala Glu Ala Ala Leu Glu Lys Leu Val Ile
1               5                   10

<210> 790
<211> 9
<212> PRT
<213> hepatitis C virus

<400> 790

Ala Glu Ala Ala Leu Glu Asn Leu Val
1               5

<210> 791
<211> 10
<212> PRT
<213> hepatitis C virus

<400> 791

Ala Glu Ala Ala Leu Glu Asn Leu Val Ile
1               5                   10

<210> 792
<211> 10
<212> PRT
<213> hepatitis C virus

<400> 792

```
Ala Glu Ile Leu Arg Lys Ser Arg Arg Phe
1               5               10

<210>  793

<211>  9

<212>  PRT

<213>  hepatitis C virus


<400>  793

Ala Glu Leu Ala Thr Lys Thr Phe Gly
1               5

<210>  794

<211>  9

<212>  PRT

<213>  hepatitis C virus


<400>  794

Ala Glu Gln Phe Lys Gln Lys Ala Leu
1               5

<210>  795

<211>  10

<212>  PRT

<213>  hepatitis C virus


<400>  795

Ala Glu Gln Phe Lys Gln Lys Ala Leu Gly
1               5               10

<210>  796

<211>  10

<212>  PRT

<213>  hepatitis C virus


<400>  796

Ala Glu Thr Ala Gly Ala Arg Leu Val Val
1               5               10

<210>  797
```

<211> 9

<212> PRT

<213> hepatitis C virus


<400> 797

Ala Glu Thr Ala Gly Val Arg Leu Thr
1               5


<210> 798

<211> 10

<212> PRT

<213> hepatitis C virus


<400> 798

Ala Glu Thr Ala Gly Val Arg Leu Thr Val
1               5                   10


<210> 799

<211> 10

<212> PRT

<213> hepatitis C virus


<400> 799

Ala Glu Thr Ser Val Arg Leu Arg Ala Tyr
1               5                   10


<210> 800

<211> 9

<212> PRT

<213> hepatitis C virus


<400> 800

Ala Glu Thr Thr Val Arg Leu Arg Ala
1               5


<210> 801

<211> 9

<212> PRT

<213> hepatitis C virus

<400> 801

Ala Phe Lys Ile Met Ser Gly Glu Lys
1               5

<210> 802

<211> 10

<212> PRT

<213> hepatitis C virus

<400> 802

Ala Phe Thr Ala Ser Ile Thr Ser Pro Leu
1               5               10

<210> 803

<211> 10

<212> PRT

<213> hepatitis C virus

<400> 803

Ala Phe Trp Ala Lys His Met Trp Asn Phe
1               5               10

<210> 804

<211> 10

<212> PRT

<213> hepatitis C virus

<400> 804

Ala Gly Ala Ala Val Gly Ser Ile Gly Leu
1               5               10

<210> 805

<211> 10

<212> PRT

<213> hepatitis C virus

<400> 805

Ala Gly Ala His Gly Ile Leu Ser Phe Leu
1               5               10

<210> 806

<211> 10

<212> PRT

<213> hepatitis C virus


<400> 806

Ala Gly Pro Lys Gly Pro Ile Thr Gln Met
1               5               10


<210> 807

<211> 10

<212> PRT

<213> hepatitis C virus


<400> 807

Ala Gly Thr Gln Glu Asp Ala Ala Ser Leu
1               5               10


<210> 808

<211> 10

<212> PRT

<213> hepatitis C virus


<400> 808

Ala Gly Val Ala Gly Ala Leu Val Ala Phe
1               5               10


<210> 809

<211> 9

<212> PRT

<213> hepatitis C virus


<400> 809

Ala Gly Tyr Ser Gly Gly Asp Ile Tyr
1               5


<210> 810

<211> 10

<212> PRT

<213> hepatitis C virus


<400> 810

Ala His Asp Ala Ser Gly Lys Arg Val Tyr
1               5                   10


<210> 811

<211> 10

<212> PRT

<213> hepatitis C virus


<400> 811

Ala His Leu Gln Val Trp Val Pro Pro Leu
1               5                   10


<210> 812

<211> 9

<212> PRT

<213> hepatitis C virus


<400> 812

Ala His Trp Gly Val Leu Ala Gly Leu
1               5


<210> 813

<211> 9

<212> PRT

<213> hepatitis C virus


<400> 813

Ala Ile Lys Gly Gly Arg His Leu Ile
1               5


<210> 814

<211> 9

<212> PRT

<213> hepatitis C virus

<400> 814

Ala Ile Lys Trp Glu Tyr Val Leu Leu
1               5

<210> 815

<211> 10

<212> PRT

<213> hepatitis C virus

<400> 815

Ala Ile Lys Trp Glu Tyr Val Leu Leu Leu
1               5                   10

<210> 816

<211> 9

<212> PRT

<213> hepatitis C virus

<400> 816

Ala Ile Leu Gly Pro Leu Met Val Leu
1               5

<210> 817

<211> 10

<212> PRT

<213> hepatitis C virus

<400> 817

Ala Ile Arg Ser Leu Thr Glu Arg Leu Tyr
1               5                   10

<210> 818

<211> 9

<212> PRT

<213> hepatitis C virus

<400> 818

Ala Ile Thr Tyr Ser Thr Tyr Gly Lys
1               5

```
<210> 819
<211> 10
<212> PRT
<213> hepatitis C virus


<400> 819
Ala Lys Leu Gln Asp Cys Thr Met Leu Val
1               5                   10

<210> 820
<211> 9
<212> PRT
<213> hepatitis C virus


<400> 820
Ala Leu Ala Ala Tyr Cys Leu Ser Val
1               5

<210> 821
<211> 9
<212> PRT
<213> hepatitis C virus


<400> 821
Ala Leu Ala Ala Tyr Cys Leu Thr Thr
1               5

<210> 822
<211> 10
<212> PRT
<213> hepatitis C virus


<400> 822
Ala Leu Gly Leu Asn Ala Val Ala Tyr Tyr
1               5                   10

<210> 823
<211> 9
<212> PRT
```

<213> hepatitis C virus

<400> 823

Ala Leu Leu Gly Pro Ala Tyr Leu Leu
1               5

<210> 824

<211> 9

<212> PRT

<213> hepatitis C virus

<400> 824

Ala Leu Gln Val Trp Val Pro Pro Leu
1               5

<210> 825

<211> 9

<212> PRT

<213> hepatitis C virus

<400> 825

Ala Leu Ser Thr Gly Leu Ile His Leu
1               5

<210> 826

<211> 9

<212> PRT

<213> hepatitis C virus

<400> 826

Ala Leu Ser Thr Gly Leu Leu His Leu
1               5

<210> 827

<211> 9

<212> PRT

<213> hepatitis C virus

<400> 827

Ala Leu Val Val Ser Gln Leu Leu Arg
1               5

<210>   828

<211>   9

<212>   PRT

<213>   hepatitis C virus


<400>   828

Ala Leu Tyr Asp Ile Thr Gln Lys Leu
1               5

<210>   829

<211>   9

<212>   PRT

<213>   hepatitis C virus


<400>   829

Ala Leu Tyr Asp Val Ile Gln Lys Leu
1               5

<210>   830

<211>   9

<212>   PRT

<213>   hepatitis C virus


<400>   830

Ala Leu Tyr Gly Val Trp Pro Leu Leu
1               5

<210>   831

<211>   10

<212>   PRT

<213>   hepatitis C virus


<400>   831

Ala Leu Tyr Gly Val Trp Pro Leu Leu Leu
1               5                       10

<210>   832

<211> 9

<212> PRT

<213> hepatitis C virus

<400> 832

Ala Asn Leu Leu Trp Arg Gln Glu Met
1               5

<210> 833

<211> 9

<212> PRT

<213> hepatitis C virus

<400> 833

Ala Pro Ala Cys Lys Pro Leu Leu Arg
1               5

<210> 834

<211> 10

<212> PRT

<213> hepatitis C virus

<400> 834

Ala Pro Ala Cys Lys Pro Leu Leu Arg Glu
1               5                   10

<210> 835

<211> 10

<212> PRT

<213> hepatitis C virus

<400> 835

Ala Pro Ile Thr Ala Tyr Ser Gln Gln Thr
1               5                   10

<210> 836

<211> 10

<212> PRT

<213> hepatitis C virus

```
<400>  836

Ala Pro Leu Gly Gly Ala Ala Arg Ala Leu
1               5                   10


<210>  837

<211>  10

<212>  PRT

<213>  hepatitis C virus


<400>  837

Ala Pro Leu Gly Gly Val Ala Arg Ala Leu
1               5                   10


<210>  838

<211>  9

<212>  PRT

<213>  hepatitis C virus


<400>  838

Ala Pro Asn Tyr Ser Arg Ala Leu Trp
1               5


<210>  839

<211>  9

<212>  PRT

<213>  hepatitis C virus


<400>  839

Ala Pro Pro Gly Ala Arg Ser Leu Thr
1               5


<210>  840

<211>  10

<212>  PRT

<213>  hepatitis C virus


<400>  840

Ala Pro Pro Gly Ala Arg Ser Leu Thr Pro
1               5                   10
```

<210> 841

<211> 9

<212> PRT

<213> hepatitis C virus


<400> 841

Ala Pro Pro Gly Asp Pro Pro Gln Pro
1               5


<210> 842

<211> 10

<212> PRT

<213> hepatitis C virus


<400> 842

Ala Pro Pro Gly Asp Pro Pro Gln Pro Glu
1               5                   10


<210> 843

<211> 9

<212> PRT

<213> hepatitis C virus


<400> 843

Ala Pro Pro Ile Pro Pro Pro Arg Arg
1               5


<210> 844

<211> 9

<212> PRT

<213> hepatitis C virus


<400> 844

Ala Pro Pro Ser Ala Ala Ser Ala Phe
1               5


<210> 845

<211> 10

```
<212>   PRT

<213>   hepatitis C virus


<400>   845

Ala Pro Pro Ser Ala Ala Ser Ala Phe Val
1               5                   10


<210>   846

<211>   9

<212>   PRT

<213>   hepatitis C virus


<400>   846

Ala Pro Arg Pro Cys Gly Ile Val Pro
1               5


<210>   847

<211>   10

<212>   PRT

<213>   hepatitis C virus


<400>   847

Ala Pro Arg Pro Cys Gly Ile Val Pro Ala
1               5                   10


<210>   848

<211>   9

<212>   PRT

<213>   hepatitis C virus


<400>   848

Ala Pro Ser Leu Lys Ala Thr Cys Thr
1               5


<210>   849

<211>   10

<212>   PRT

<213>   hepatitis C virus
```

<400> 849

Ala Pro Ser Leu Lys Ala Thr Cys Thr Thr
1               5                   10

<210> 850

<211> 9

<212> PRT

<213> hepatitis C virus

<400> 850

Ala Pro Thr Ile Trp Val Arg Met Val
1               5

<210> 851

<211> 10

<212> PRT

<213> hepatitis C virus

<400> 851

Ala Pro Thr Ile Trp Val Arg Met Val Leu
1               5                   10

<210> 852

<211> 8

<212> PRT

<213> hepatitis C virus

<400> 852

Ala Pro Thr Leu Trp Ala Arg Met
1               5

<210> 853

<211> 10

<212> PRT

<213> hepatitis C virus

<400> 853

Ala Pro Thr Leu Trp Ala Arg Met Ile Leu
1               5                   10

```
<210>   854
<211>   11
<212>   PRT
<213>   hepatitis C virus


<400>   854
Ala Pro Thr Leu Trp Ala Arg Met Ile Leu Met
1               5                   10

<210>   855
<211>   10
<212>   PRT
<213>   hepatitis C virus


<400>   855
Ala Pro Val Gly Gly Val Ala Arg Ala Leu
1               5                   10

<210>   856
<211>   10
<212>   PRT
<213>   hepatitis C virus


<400>   856
Ala Pro Val Val Glu Ser Lys Trp Arg Ala
1               5                   10

<210>   857
<211>   10
<212>   PRT
<213>   hepatitis C virus


<400>   857
Ala Pro Tyr Ile Glu Gln Ala Gln Ala Ile
1               5                   10

<210>   858
<211>   10
<212>   PRT
```

<213> hepatitis C virus

<400> 858

Ala Gln Ala Glu Ala Ala Leu Glu Asn Leu
1               5                   10

<210> 859

<211> 9

<212> PRT

<213> hepatitis C virus

<400> 859

Ala Gln Gly Leu Ile Arg Ala Cys Met
1               5

<210> 860

<211> 10

<212> PRT

<213> hepatitis C virus

<400> 860

Ala Gln Gly Leu Ile Arg Ala Cys Met Leu
1               5                   10

<210> 861

<211> 10

<212> PRT

<213> hepatitis C virus

<400> 861

Ala Gln Pro Gly Tyr Pro Trp Pro Leu Tyr
1               5                   10

<210> 862

<211> 10

<212> PRT

<213> hepatitis C virus

<400> 862

```
Ala Arg Ala Leu Ala His Gly Val Arg Val
1               5                   10
```

<210> 863

<211> 10

<212> PRT

<213> hepatitis C virus


<400> 863

```
Ala Arg His Thr Pro Val Asn Ser Trp Leu
1               5                   10
```

<210> 864

<211> 10

<212> PRT

<213> hepatitis C virus


<400> 864

```
Ala Arg Met Ile Leu Met Thr His Phe Phe
1               5                   10
```

<210> 865

<211> 9

<212> PRT

<213> hepatitis C virus


<400> 865

```
Ala Arg Arg Gly Arg Glu Ile Leu Leu
1               5
```

<210> 866

<211> 10

<212> PRT

<213> hepatitis C virus


<400> 866

```
Ala Arg Arg Pro Glu Gly Arg Ala Trp Ala
1               5                   10
```

<210> 867

```
<211>    9
<212>    PRT
<213>    hepatitis C virus


<400>    867
Ala Arg Ser Val Arg Ala Lys Leu Leu
1                   5


<210>    868
<211>    10
<212>    PRT
<213>    hepatitis C virus


<400>    868
Ala Arg Val Thr Gln Ile Leu Ser Ser Leu
1                   5                   10


<210>    869
<211>    10
<212>    PRT
<213>    hepatitis C virus


<400>    869
Ala Ser Ala Ala Cys Arg Ala Ala Lys Leu
1                   5                   10


<210>    870
<211>    10
<212>    PRT
<213>    hepatitis C virus


<400>    870
Ala Ser Gly Lys Arg Val Tyr Tyr Leu Thr
1                   5                   10


<210>    871
<211>    10
<212>    PRT
<213>    hepatitis C virus
```

<400> 871

Ala Ser Leu Arg Val Phe Thr Glu Ala Met
1               5                   10

<210> 872

<211> 9

<212> PRT

<213> hepatitis C virus

<400> 872

Ala Ser Gln Leu Ser Ala Pro Ser Leu
1               5

<210> 873

<211> 10

<212> PRT

<213> hepatitis C virus

<400> 873

Ala Ser Gln Leu Ser Ala Pro Ser Leu Lys
1               5                   10

<210> 874

<211> 9

<212> PRT

<213> hepatitis C virus

<400> 874

Ala Ser Ser Val Cys Gly Pro Val Tyr
1               5

<210> 875

<211> 9

<212> PRT

<213> hepatitis C virus

<400> 875

Ala Ser Thr Val Lys Ala Lys Leu Leu
1               5

<210> 876

<211> 10

<212> PRT

<213> hepatitis C virus


<400> 876

Ala Ser Val Ala Gly Ala His Gly Ile Leu
1               5                   10


<210> 877

<211> 10

<212> PRT

<213> hepatitis C virus


<400> 877

Ala Thr Asp Ala Leu Met Thr Gly Tyr Thr
1               5                   10


<210> 878

<211> 10

<212> PRT

<213> hepatitis C virus


<400> 878

Ala Thr Leu Gly Phe Gly Ala Tyr Met Ser
1               5                   10


<210> 879

<211> 9

<212> PRT

<213> hepatitis C virus


<400> 879

Ala Thr Thr Ser Arg Ser Ala Ser Leu
1               5


<210> 880

<211> 10

<212> PRT

<213> hepatitis C virus

<400> 880

Ala Val Ala Tyr Tyr Arg Gly Leu Asp Val
1               5                   10

<210> 881

<211> 9

<212> PRT

<213> hepatitis C virus

<400> 881

Ala Val Cys Ser Arg Gly Val Ala Lys
1               5

<210> 882

<211> 10

<212> PRT

<213> hepatitis C virus

<400> 882

Ala Val Asp Phe Ile Pro Val Glu Ser Met
1               5                   10

<210> 883

<211> 10

<212> PRT

<213> hepatitis C virus

<400> 883

Ala Val Asp Phe Val Pro Val Glu Ser Met
1               5                   10

<210> 884

<211> 9

<212> PRT

<213> hepatitis C virus

<400>  884

Ala Val Asp Leu Tyr Leu Val Thr Arg
1               5


<210>  885

<211>  10

<212>  PRT

<213>  hepatitis C virus


<400>  885

Ala Val Glu Pro Val Val Phe Ser Asp Met
1               5                   10


<210>  886

<211>  9

<212>  PRT

<213>  hepatitis C virus


<400>  886

Ala Val Phe Val Gly Leu Ala Leu Leu
1               5


<210>  887

<211>  9

<212>  PRT

<213>  hepatitis C virus


<400>  887

Ala Val Gly Ile Phe Arg Ala Ala Val
1               5


<210>  888

<211>  9

<212>  PRT

<213>  hepatitis C virus


<400>  888

Ala Val Gly Ser Ile Gly Leu Gly Lys
1               5

<210> 889

<211> 9

<212> PRT

<213> hepatitis C virus


<400> 889

Ala Val Gly Ser Val Gly Leu Gly Lys
1               5


<210> 890

<211> 10

<212> PRT

<213> hepatitis C virus


<400> 890

Ala Val His Pro Glu Leu Ile Phe Asp Ile
1               5                   10


<210> 891

<211> 9

<212> PRT

<213> hepatitis C virus


<400> 891

Ala Val Ile Pro Asp Arg Glu Val Leu
1               5


<210> 892

<211> 9

<212> PRT

<213> hepatitis C virus


<400> 892

Ala Val Leu Gly Pro Leu Tyr Leu Ile
1               5


<210> 893

<211> 9

<212> PRT

<213> hepatitis C virus


<400> 893

Ala Val Gln Trp Met Asn Arg Leu Ile
1                5


<210> 894

<211> 9

<212> PRT

<213> hepatitis C virus


<400> 894

Ala Val Arg Ala Ser Leu Ile Ser Arg
1                5


<210> 895

<211> 9

<212> PRT

<213> hepatitis C virus


<400> 895

Ala Val Arg Thr Lys Leu Lys Leu Thr
1                5


<210> 896

<211> 9

<212> PRT

<213> hepatitis C virus


<400> 896

Ala Trp Ala Lys Val Val Val Ile Leu
1                5


<210> 897

<211> 10

<212> PRT

<213> hepatitis C virus


<400> 897

Ala Trp Glu Thr Ala Arg His Thr Pro Val
1               5                   10

<210>  898

<211>  9

<212>  PRT

<213>  hepatitis C virus


<400>  898

Ala Trp Lys Ser Lys Lys Cys Pro Met
1               5

<210>  899

<211>  10

<212>  PRT

<213>  hepatitis C virus


<400>  899

Ala Trp Lys Ser Lys Lys Cys Pro Met Gly
1               5                   10

<210>  900

<211>  10

<212>  PRT

<213>  hepatitis C virus


<400>  900

Ala Tyr Ala Ala Gln Gly Tyr Lys Val Leu
1               5                   10

<210>  901

<211>  10

<212>  PRT

<213>  hepatitis C virus


<400>  901

Ala Tyr Ala Leu Tyr Gly Val Trp Pro Leu
1               5                   10

<210>  902

<211> 9

<212> PRT

<213> hepatitis C virus


<400> 902

Ala Tyr Phe Ser Met Gln Gly Ala Trp
1               5


<210> 903

<211> 10

<212> PRT

<213> hepatitis C virus


<400> 903

Ala Tyr Leu Asn Thr Pro Gly Leu Pro Val
1               5                   10


<210> 904

<211> 9

<212> PRT

<213> hepatitis C virus


<400> 904

Ala Tyr Met Ser Lys Ala His Gly Ile
1               5


<210> 905

<211> 9

<212> PRT

<213> hepatitis C virus


<400> 905

Ala Tyr Ser Gln Gln Thr Arg Gly Leu
1               5


<210> 906

<211> 10

<212> PRT

<213> hepatitis C virus

<400> 906

Ala Tyr Ser Gln Gln Thr Arg Gly Leu Leu
1               5                   10


<210> 907

<211> 10

<212> PRT

<213> hepatitis C virus


<400> 907

Ala Tyr Tyr Arg Gly Leu Asp Val Ser Val
1               5                   10


<210> 908

<211> 10

<212> PRT

<213> hepatitis C virus


<400> 908

Cys Ala Ala Trp Tyr Ile Lys Gly Arg Leu
1               5                   10


<210> 909

<211> 9

<212> PRT

<213> hepatitis C virus


<400> 909

Cys Ala Cys Leu Trp Met Met Leu Leu
1               5


<210> 910

<211> 10

<212> PRT

<213> hepatitis C virus


<400> 910

Cys Glu Cys Tyr Asp Ala Gly Cys Ala Trp
1               5                   10

```
<210>  911
<211>  9
<212>  PRT
<213>  hepatitis C virus


<400>  911

Cys Glu Lys Met Ala Leu Tyr Asp Ile
1               5


<210>  912
<211>  9
<212>  PRT
<213>  hepatitis C virus


<400>  912

Cys Glu Pro Glu Pro Asp Val Ala Val
1               5


<210>  913
<211>  10
<212>  PRT
<213>  hepatitis C virus


<400>  913

Cys Glu Pro Glu Pro Asp Val Ala Val Leu
1               5                   10


<210>  914
<211>  9
<212>  PRT
<213>  hepatitis C virus


<400>  914

Cys Gly Ala Pro Pro Cys Asn Ile Tyr
1               5


<210>  915
<211>  9
```

```
<212>   PRT
<213>   hepatitis C virus


<400>   915
Cys Gly Gly Ala Val Phe Val Gly Leu
1                   5


<210>   916
<211>   10
<212>   PRT
<213>   hepatitis C virus


<400>   916
Cys Gly Ser Val Phe Leu Val Ser Gln Leu
1                   5                   10


<210>   917
<211>   10
<212>   PRT
<213>   hepatitis C virus


<400>   917
Cys Gly Tyr Arg Arg Cys Arg Ala Ser Gly
1                   5                   10


<210>   918
<211>   10
<212>   PRT
<213>   hepatitis C virus


<400>   918
Cys His Ser Lys Lys Lys Cys Asp Glu Leu
1                   5                   10


<210>   919
<211>   9
<212>   PRT
<213>   hepatitis C virus
```

<400> 919

Cys Ile Asn Gly Val Cys Trp Thr Val
1                   5

<210> 920

<211> 9

<212> PRT

<213> hepatitis C virus

<400> 920

Cys Leu Ile Asp Tyr Pro Tyr Arg Leu
1                   5

<210> 921

<211> 10

<212> PRT

<213> hepatitis C virus

<400> 921

Cys Leu Arg Lys Leu Gly Val Pro Pro Leu
1               5                   10

<210> 922

<211> 9

<212> PRT

<213> hepatitis C virus

<400> 922

Cys Leu Val Asp Tyr Pro Tyr Arg Leu
1               5

<210> 923

<211> 9

<212> PRT

<213> hepatitis C virus

<400> 923

Cys Leu Val His Tyr Pro Tyr Arg Leu
1               5

<210> 924

<211> 9

<212> PRT

<213> hepatitis C virus

<400> 924

Cys Met Val Asp Tyr Pro Tyr Arg Leu
1               5

<210> 925

<211> 10

<212> PRT

<213> hepatitis C virus

<400> 925

Cys Pro Ala Gly His Ala Val Gly Ile Phe
1               5                   10

<210> 926

<211> 9

<212> PRT

<213> hepatitis C virus

<400> 926

Cys Pro Cys Gly Ala Gln Ile Thr Gly
1               5

<210> 927

<211> 10

<212> PRT

<213> hepatitis C virus

<400> 927

Cys Pro Cys Gln Val Pro Ala Pro Glu Phe
1               5                   10

<210> 928

<211> 9

<212> PRT

<213> hepatitis C virus

<400> 928

Cys Pro Leu Pro Pro Thr Lys Ala Pro
1               5

<210> 929

<211> 9

<212> PRT

<213> hepatitis C virus

<400> 929

Cys Pro Arg Gly His Ala Val Gly Ile
1               5

<210> 930

<211> 10

<212> PRT

<213> hepatitis C virus

<400> 930

Cys Pro Arg Gly His Ala Val Gly Ile Phe
1               5                   10

<210> 931

<211> 9

<212> PRT

<213> hepatitis C virus

<400> 931

Cys Pro Ser Gly His Ala Val Gly Ile
1               5

<210> 932

<211> 10

<212> PRT

<213> hepatitis C virus

<400> 932

```
Cys Pro Ser Gly His Ala Val Gly Ile Phe
1               5                   10
```

<210> 933

<211> 9

<212> PRT

<213> hepatitis C virus


<400> 933

```
Cys Pro Ser Gly His Val Val Gly Ile
1               5
```

<210> 934

<211> 9

<212> PRT

<213> hepatitis C virus


<400> 934

```
Cys Pro Thr Asp Cys Phe Arg Lys His
1               5
```

<210> 935

<211> 10

<212> PRT

<213> hepatitis C virus


<400> 935

```
Cys Ser Phe Ser Ile Phe Leu Leu Ala Leu
1               5                   10
```

<210> 936

<211> 9

<212> PRT

<213> hepatitis C virus


<400> 936

```
Cys Ser Phe Thr Thr Leu Pro Ala Leu
1               5
```

<210> 937

<211> 10

<212> PRT

<213> hepatitis C virus


<400> 937

Cys Ser Thr Pro Cys Ser Gly Ser Trp Leu
1               5                   10


<210> 938

<211> 9

<212> PRT

<213> hepatitis C virus


<400> 938

Cys Thr Cys Gly Ala Val Asp Leu Tyr
1               5


<210> 939

<211> 9

<212> PRT

<213> hepatitis C virus


<400> 939

Cys Thr Cys Gly Ser Ala Asp Leu Tyr
1               5


<210> 940

<211> 9

<212> PRT

<213> hepatitis C virus


<400> 940

Cys Thr Cys Gly Ser Ser Asp Leu Tyr
1               5


<210> 941

<211> 10

<212> PRT

<213> hepatitis C virus

<400> 941

Cys Thr Cys Gly Ser Ser Asp Leu Tyr Leu
1               5                   10


<210> 942

<211> 10

<212> PRT

<213> hepatitis C virus


<400> 942

Cys Thr Met Leu Val Asn Gly Asp Asp Leu
1               5                   10


<210> 943

<211> 9

<212> PRT

<213> hepatitis C virus


<400> 943

Cys Thr Pro Ser Pro Ala Pro Asn Tyr
1               5


<210> 944

<211> 9

<212> PRT

<213> hepatitis C virus


<400> 944

Cys Thr Val Asn Phe Ser Ile Phe Lys
1               5


<210> 945

<211> 9

<212> PRT

<213> hepatitis C virus


<400> 945

Cys Thr Val Asn Phe Thr Ile Phe Lys
1               5

```
<210>  946
<211>  9
<212>  PRT
<213>  hepatitis C virus


<400>  946

Cys Thr Val Asn Phe Thr Leu Phe Lys
1               5


<210>  947
<211>  9
<212>  PRT
<213>  hepatitis C virus


<400>  947

Cys Thr Trp Met Asn Ser Thr Gly Tyr
1               5


<210>  948
<211>  10
<212>  PRT
<213>  hepatitis C virus


<400>  948

Cys Val Arg Glu Asn Asn Ser Ser Arg Cys
1               5                   10


<210>  949
<211>  10
<212>  PRT
<213>  hepatitis C virus


<400>  949

Cys Val Thr Gln Thr Val Asp Phe Ser Leu
1               5                   10


<210>  950
<211>  9
```

```
<212>  PRT

<213>  hepatitis C virus


<400>  950

Cys Trp Val Ala Leu Thr Pro Thr Leu
1               5


<210>  951

<211>  9

<212>  PRT

<213>  hepatitis C virus


<400>  951

Cys Tyr Ser Ile Glu Pro Leu Asp Leu
1               5


<210>  952

<211>  10

<212>  PRT

<213>  hepatitis C virus


<400>  952

Asp Ala Ala Ala Arg Val Thr Gln Ile Leu
1               5                   10


<210>  953

<211>  10

<212>  PRT

<213>  hepatitis C virus


<400>  953

Asp Ala Gly Cys Ala Trp Tyr Glu Leu Thr
1               5                   10


<210>  954

<211>  10

<212>  PRT

<213>  hepatitis C virus
```

<400> 954

Asp Ala Arg Val Cys Ala Cys Leu Trp Met
1               5                   10

<210> 955

<211> 10

<212> PRT

<213> hepatitis C virus


<400> 955

Asp Ala Ser Gly Lys Arg Val Tyr Tyr Leu
1               5                   10

<210> 956

<211> 9

<212> PRT

<213> hepatitis C virus


<400> 956

Asp Glu Leu Ala Ala Ala Leu Arg Gly
1               5

<210> 957

<211> 10

<212> PRT

<213> hepatitis C virus


<400> 957

Asp Glu Leu Ala Ala Lys Leu Ser Ala Leu
1               5                   10

<210> 958

<211> 10

<212> PRT

<213> hepatitis C virus


<400> 958

Asp Phe Ser Leu Asp Pro Thr Phe Thr Ile
1               5                   10

<210> 959
<211> 9
<212> PRT
<213> hepatitis C virus

<400> 959

Asp Gly Gly Cys Ser Gly Gly Ala Tyr
1               5

<210> 960
<211> 10
<212> PRT
<213> hepatitis C virus

<400> 960

Asp His Leu Glu Phe Trp Glu Ser Val Phe
1               5                   10

<210> 961
<211> 10
<212> PRT
<213> hepatitis C virus

<400> 961

Asp Ile Thr Lys Leu Leu Leu Ala Ile Leu
1               5                   10

<210> 962
<211> 8
<212> PRT
<213> hepatitis C virus

<400> 962

Asp Leu Cys Gly Ser Val Phe Leu
1               5

<210> 963
<211> 9
<212> PRT

<213> hepatitis C virus

<400> 963

Asp Leu Cys Gly Ser Val Phe Leu Val
1               5

<210> 964

<211> 10

<212> PRT

<213> hepatitis C virus

<400> 964

Asp Leu Glu Asp Arg Asp Arg Ser Glu Leu
1               5                   10

<210> 965

<211> 10

<212> PRT

<213> hepatitis C virus

<400> 965

Asp Leu Met Gly Tyr Ile Pro Leu Val Gly
1               5                   10

<210> 966

<211> 9

<212> PRT

<213> hepatitis C virus

<400> 966

Asp Leu Met Gly Tyr Ile Pro Val Val
1               5

<210> 967

<211> 9

<212> PRT

<213> hepatitis C virus

<400> 967

Asp Leu Pro Gln Ile Ile Glu Arg Leu
1                   5

<210> 968

<211> 9

<212> PRT

<213> hepatitis C virus


<400> 968

Asp Leu Val Asn Leu Leu Pro Ala Ile
1                   5

<210> 969

<211> 10

<212> PRT

<213> hepatitis C virus


<400> 969

Asp Leu Val Asn Leu Leu Pro Ala Ile Leu
1                   5                   10

<210> 970

<211> 9

<212> PRT

<213> hepatitis C virus


<400> 970

Asp Met Arg Pro Tyr Cys Trp His Tyr
1                   5

<210> 971

<211> 9

<212> PRT

<213> hepatitis C virus


<400> 971

Asp Pro Asp Tyr Val Pro Pro Val Val
1                   5

<210> 972

<211> 9

<212> PRT

<213> hepatitis C virus


<400> 972

Asp Pro Asn Ile Arg Thr Gly Val Arg
1               5


<210> 973

<211> 10

<212> PRT

<213> hepatitis C virus


<400> 973

Asp Pro Asn Ile Arg Thr Gly Val Arg Thr
1               5                   10


<210> 974

<211> 8

<212> PRT

<213> hepatitis C virus


<400> 974

Asp Pro Arg Arg Arg Ser Arg Asn
1               5


<210> 975

<211> 10

<212> PRT

<213> hepatitis C virus


<400> 975

Asp Pro Arg Arg Arg Ser Arg Asn Leu Gly
1               5                   10


<210> 976

<211> 9

<212> PRT

<213> hepatitis C virus

<400> 976

Asp Pro Ser His Ile Thr Ala Glu Thr
1               5


<210> 977

<211> 10

<212> PRT

<213> hepatitis C virus


<400> 977

Asp Pro Ser His Ile Thr Ala Glu Thr Ala
1               5                   10


<210> 978

<211> 9

<212> PRT

<213> hepatitis C virus


<400> 978

Asp Pro Thr Phe Thr Ile Glu Thr Thr
1               5


<210> 979

<211> 10

<212> PRT

<213> hepatitis C virus


<400> 979

Asp Pro Thr Phe Thr Ile Glu Thr Thr Thr
1               5                   10


<210> 980

<211> 9

<212> PRT

<213> hepatitis C virus


<400> 980

Asp Pro Thr Thr Pro Leu Ala Arg Ala
1               5

<210> 981

<211> 10

<212> PRT

<213> hepatitis C virus

<400> 981

Asp Pro Thr Thr Pro Leu Ala Arg Ala Ala
1               5                   10

<210> 982

<211> 10

<212> PRT

<213> hepatitis C virus

<400> 982

Asp Gln Met Trp Lys Cys Leu Ile Arg Leu
1               5                   10

<210> 983

<211> 9

<212> PRT

<213> hepatitis C virus

<400> 983

Asp Gln Met Trp Lys Cys Leu Thr Arg
1               5

<210> 984

<211> 9

<212> PRT

<213> hepatitis C virus

<400> 984

Asp Gln Arg Pro Tyr Cys Trp His Tyr
1               5

<210> 985

<211> 9

```
<212>   PRT
<213>   hepatitis C virus


<400>   985
Asp Arg Glu Val Leu Tyr Arg Glu Phe
1                   5


<210>   986
<211>   9
<212>   PRT
<213>   hepatitis C virus


<400>   986
Asp Arg Ser Glu Leu Ser Pro Leu Leu
1                   5


<210>   987
<211>   9
<212>   PRT
<213>   hepatitis C virus


<400>   987
Asp Ser Val Val Leu Cys Glu Cys Tyr
1                   5


<210>   988
<211>   10
<212>   PRT
<213>   hepatitis C virus


<400>   988
Asp Thr Leu Thr Cys Gly Phe Ala Asp Leu
1                   5                   10


<210>   989
<211>   9
<212>   PRT
<213>   hepatitis C virus
```

<400> 989

Asp Val Ala Val Leu Thr Ser Met Leu
1               5

<210> 990

<211> 10

<212> PRT

<213> hepatitis C virus


<400> 990

Asp Val Lys Phe Pro Gly Gly Gly Gln Ile
1               5                   10

<210> 991

<211> 9

<212> PRT

<213> hepatitis C virus


<400> 991

Asp Val Met Trp Lys Cys Leu Thr Arg
1               5

<210> 992

<211> 9

<212> PRT

<213> hepatitis C virus


<400> 992

Asp Val Arg Asn Leu Ser Ser Lys Ala
1               5

<210> 993

<211> 10

<212> PRT

<213> hepatitis C virus


<400> 993

Asp Val Arg Asn Leu Ser Ser Lys Ala Val
1               5                   10

```
<210>  994
<211>  10
<212>  PRT
<213>  hepatitis C virus


<400>  994

Asp Val Val Val Val Ala Thr Asp Ala Leu
1               5                   10


<210>  995
<211>  10
<212>  PRT
<213>  hepatitis C virus


<400>  995

Asp Val Trp Asp Trp Ile Cys Thr Val Leu
1               5                   10


<210>  996
<211>  10
<212>  PRT
<213>  hepatitis C virus


<400>  996

Asp Tyr Asn Pro Pro Leu Leu Glu Thr Trp
1               5                   10


<210>  997
<211>  9
<212>  PRT
<213>  hepatitis C virus


<400>  997

Asp Tyr Pro Tyr Arg Leu Trp His Tyr
1               5


<210>  998
<211>  10
<212>  PRT
```

<213> hepatitis C virus

<400> 998

Glu Ala Ala Leu Glu Asn Leu Val Val Leu
1               5                   10

<210> 999
<211> 10
<212> PRT
<213> hepatitis C virus

<400> 999

Glu Ala Met Thr Arg Tyr Ser Ala Pro Pro
1               5                   10

<210> 1000
<211> 10
<212> PRT
<213> hepatitis C virus

<400> 1000

Glu Ala Asn Leu Leu Trp Arg Gln Glu Met
1               5                   10

<210> 1001
<211> 10
<212> PRT
<213> hepatitis C virus

<400> 1001

Glu Ala Arg Gln Ala Ile Arg Ser Leu Thr
1               5                   10

<210> 1002
<211> 10
<212> PRT
<213> hepatitis C virus

<400> 1002

Glu Cys Tyr Asp Ala Gly Cys Ala Trp Tyr
1               5                   10

<210>   1003

<211>   10

<212>   PRT

<213>   hepatitis C virus


<400>   1003

Glu Glu Ala Arg Thr Ala Ile His Ser Leu
1               5                   10


<210>   1004

<211>   9

<212>   PRT

<213>   hepatitis C virus


<400>   1004

Glu Glu Lys Leu Pro Ile Asn Pro Leu
1               5


<210>   1005

<211>   9

<212>   PRT

<213>   hepatitis C virus


<400>   1005

Glu Glu Lys Leu Pro Ile Ser Pro Leu
1               5


<210>   1006

<211>   10

<212>   PRT

<213>   hepatitis C virus


<400>   1006

Glu Glu Gln Ser Val Val Cys Cys Ser Met
1               5                   10


<210>   1007

<211> 10

<212> PRT

<213> hepatitis C virus


<400> 1007

Glu Glu Ser Ile Tyr Gln Ala Cys Ser Leu
1               5                   10


<210> 1008

<211> 10

<212> PRT

<213> hepatitis C virus


<400> 1008

Glu Glu Ser Ile Tyr Gln Cys Cys Asp Leu
1               5                   10


<210> 1009

<211> 10

<212> PRT

<213> hepatitis C virus


<400> 1009

Glu Glu Ser Lys Leu Pro Ile Asn Ala Leu
1               5                   10


<210> 1010

<211> 10

<212> PRT

<213> hepatitis C virus


<400> 1010

Glu Phe Trp Glu Ser Val Phe Thr Gly Leu
1               5                   10


<210> 1011

<211> 9

<212> PRT

<213> hepatitis C virus

```
<400> 1011

Glu Gly Met Gly Trp Ala Gly Trp Leu
1               5


<210> 1012

<211> 10

<212> PRT

<213> hepatitis C virus


<400> 1012

Glu Gly Met Gly Trp Ala Gly Trp Leu Leu
1               5                   10


<210> 1013

<211> 10

<212> PRT

<213> hepatitis C virus


<400> 1013

Glu Ile Leu Leu Gly Pro Ala Asp Ser Leu
1               5                   10


<210> 1014

<211> 9

<212> PRT

<213> hepatitis C virus


<400> 1014

Glu Ile Asn Arg Val Ala Ser Cys Leu
1               5


<210> 1015

<211> 10

<212> PRT

<213> hepatitis C virus


<400> 1015

Glu Lys Gly Gly Arg Lys Pro Ala Arg Leu
1               5                   10
```

<210> 1016

<211> 9

<212> PRT

<213> hepatitis C virus


<400> 1016

Glu Leu Ala Ala Lys Leu Ser Ala Leu
1               5


<210> 1017

<211> 10

<212> PRT

<213> hepatitis C virus


<400> 1017

Glu Leu Asp Gly Val Arg Leu His Arg Tyr
1               5                   10


<210> 1018

<211> 10

<212> PRT

<213> hepatitis C virus


<400> 1018

Glu Leu Ile Phe Asp Ile Thr Lys Leu Leu
1               5                   10


<210> 1019

<211> 9

<212> PRT

<213> hepatitis C virus


<400> 1019

Glu Met Tyr Gly Ala Thr Tyr Ser Val
1               5


<210> 1020

<211> 9

```
<212>   PRT
<213>   hepatitis C virus


<400>   1020
Glu Met Tyr Gly Ala Val Tyr Ser Val
1               5


<210>   1021
<211>   10
<212>   PRT
<213>   hepatitis C virus


<400>   1021
Glu Asn Lys Val Val Ile Leu Asp Ser Phe
1               5                   10


<210>   1022
<211>   9
<212>   PRT
<213>   hepatitis C virus


<400>   1022
Glu Pro Asp Val Ala Val Leu Thr Ser
1               5


<210>   1023
<211>   10
<212>   PRT
<213>   hepatitis C virus


<400>   1023
Glu Pro Asp Val Ala Val Leu Thr Ser Met
1               5                   10


<210>   1024
<211>   9
<212>   PRT
<213>   hepatitis C virus
```

<400> 1024

Glu Pro Glu Pro Asp Val Ala Val Leu
1               5

<210> 1025

<211> 10

<212> PRT

<213> hepatitis C virus

<400> 1025

Glu Pro Glu Pro Asp Val Ala Val Leu Thr
1               5                   10

<210> 1026

<211> 9

<212> PRT

<213> hepatitis C virus

<400> 1026

Glu Pro Gly Asp Pro Asp Leu Ser Asp
1               5

<210> 1027

<211> 9

<212> PRT

<213> hepatitis C virus

<400> 1027

Glu Pro Leu Asp Leu Pro Gln Ile Ile
1               5

<210> 1028

<211> 10

<212> PRT

<213> hepatitis C virus

<400> 1028

Glu Pro Val Val Phe Ser Asp Met Glu Thr
1               5                   10

<210> 1029

<211> 10

<212> PRT

<213> hepatitis C virus


<400> 1029

Glu Gln Phe Lys Gln Lys Ala Leu Gly Leu
1               5                   10


<210> 1030

<211> 9

<212> PRT

<213> hepatitis C virus


<400> 1030

Glu Ser Glu Asn Lys Val Val Ile Leu
1               5


<210> 1031

<211> 9

<212> PRT

<213> hepatitis C virus


<400> 1031

Glu Ser Lys Leu Pro Ile Asn Ala Leu
1               5


<210> 1032

<211> 10

<212> PRT

<213> hepatitis C virus


<400> 1032

Glu Thr Ala Gly Ala Arg Leu Val Val Leu
1               5                   10


<210> 1033

<211> 10

<212> PRT

EP 1 652 858 A1

<213> hepatitis C virus

<400> 1033

Glu Thr Ser Val Arg Leu Arg Ala Tyr Leu
1               5                   10

<210> 1034

<211> 9

<212> PRT

<213> hepatitis C virus

<400> 1034

Glu Thr Thr Met Arg Ser Pro Val Phe
1               5

<210> 1035

<211> 9

<212> PRT

<213> hepatitis C virus

<400> 1035

Glu Thr Thr Val Arg Leu Arg Ala Tyr
1               5

<210> 1036

<211> 10

<212> PRT

<213> hepatitis C virus

<400> 1036

Glu Val Asp Gly Val Arg Leu His Arg Tyr
1               5                   10

<210> 1037

<211> 10

<212> PRT

<213> hepatitis C virus

<400> 1037

```
Glu Val Asp Gly Val Arg Leu His Arg Tyr
1               5                   10
```

<210> 1038

<211> 10

<212> PRT

<213> hepatitis C virus


<400> 1038

```
Glu Val Phe Cys Val Gln Pro Glu Lys Gly
1               5                   10
```

<210> 1039

<211> 10

<212> PRT

<213> hepatitis C virus


<400> 1039

```
Glu Val Leu Tyr Arg Glu Phe Asp Glu Met
1               5                   10
```

<210> 1040

<211> 9

<212> PRT

<213> hepatitis C virus


<400> 1040

```
Glu Val Ser Val Ala Ala Glu Ile Leu
1               5
```

<210> 1041

<211> 10

<212> PRT

<213> hepatitis C virus


<400> 1041

```
Glu Val Thr Leu Thr His Pro Ile Thr Lys
1               5                   10
```

<210> 1042

```
<211>    9
<212>    PRT
<213>    hepatitis C virus


<400>    1042
Glu Val Val Thr Ser Thr Trp Val Leu
1                   5


<210>    1043
<211>    9
<212>    PRT
<213>    hepatitis C virus


<400>    1043
Glu Trp Ala Ile Leu Pro Cys Ser Tyr
1                   5


<210>    1044
<211>    9
<212>    PRT
<213>    hepatitis C virus


<400>    1044
Glu Trp Val Ile Leu Leu Phe Leu Leu
1                   5


<210>    1045
<211>    9
<212>    PRT
<213>    hepatitis C virus


<400>    1045
Glu Trp Val Val Leu Leu Phe Leu Leu
1                   5


<210>    1046
<211>    10
<212>    PRT
<213>    hepatitis C virus
```

<400> 1046

Glu Tyr Asp Leu Glu Leu Ile Thr Ser Cys
1                 5                 10

<210> 1047

<211> 9

<212> PRT

<213> hepatitis C virus

<400> 1047

Glu Tyr Val Val Leu Leu Phe Leu Leu
1                 5

<210> 1048

<211> 10

<212> PRT

<213> hepatitis C virus

<400> 1048

Phe Ala Asp Leu Met Gly Tyr Ile Pro Leu
1                 5                 10

<210> 1049

<211> 9

<212> PRT

<213> hepatitis C virus

<400> 1049

Phe Ala Ile Lys Trp Glu Tyr Val Leu
1                 5

<210> 1050

<211> 10

<212> PRT

<213> hepatitis C virus

<400> 1050

Phe Ala Ile Lys Trp Glu Tyr Val Leu Leu
1                 5                 10

```
<210>  1051
<211>  10
<212>  PRT
<213>  hepatitis C virus


<400>  1051

Phe Cys His Ser Lys Lys Lys Cys Asp Glu
1               5                   10


<210>  1052
<211>  10
<212>  PRT
<213>  hepatitis C virus


<400>  1052

Phe Cys Ser Ala Met Tyr Val Gly Asp Leu
1               5                   10


<210>  1053
<211>  9
<212>  PRT
<213>  hepatitis C virus


<400>  1053

Phe Asp Ile Thr Lys Leu Leu Leu Ala
1               5


<210>  1054
<211>  10
<212>  PRT
<213>  hepatitis C virus


<400>  1054

Phe Glu Met Tyr Gly Ala Thr Tyr Ser Val
1               5                   10


<210>  1055
<211>  10
```

<212> PRT

<213> hepatitis C virus


<400> 1055

Phe Glu Met Tyr Gly Ala Val Tyr Ser Val
1                   5                   10


<210> 1056

<211> 9

<212> PRT

<213> hepatitis C virus


<400> 1056

Phe Phe Cys Ala Ala Trp Tyr Ile Lys
1                   5


<210> 1057

<211> 9

<212> PRT

<213> hepatitis C virus


<400> 1057

Phe Phe Thr Leu Ser Pro Trp Tyr Lys
1                   5


<210> 1058

<211> 10

<212> PRT

<213> hepatitis C virus


<400> 1058

Phe Ile Ala Gly Leu Phe Tyr Tyr His Lys
1                   5                   10


<210> 1059

<211> 9

<212> PRT

<213> hepatitis C virus

<400> 1059

Phe Ile Pro Val Glu Ser Met Glu Thr
1               5

<210> 1060

<211> 9

<212> PRT

<213> hepatitis C virus

<400> 1060

Phe Ile Ser Gly Ile Gln Tyr Leu Ala
1               5

<210> 1061

<211> 9

<212> PRT

<213> hepatitis C virus

<400> 1061

Phe Ile Thr Arg Ala Glu Ala His Leu
1               5

<210> 1062

<211> 9

<212> PRT

<213> hepatitis C virus

<400> 1062

Phe Lys Gln Lys Ala Leu Gly Leu Leu
1               5

<210> 1063

<211> 9

<212> PRT

<213> hepatitis C virus

<400> 1063

Phe Leu Ala Arg Leu Ile Trp Trp Leu
1               5

<210> 1064

<211> 10

<212> PRT

<213> hepatitis C virus


<400> 1064

Phe Leu Ala Ser Leu Phe Tyr Thr His Lys
1               5                   10


<210> 1065

<211> 9

<212> PRT

<213> hepatitis C virus


<400> 1065

Phe Leu Ala Thr Cys Ile Asn Gly Val
1               5


<210> 1066

<211> 9

<212> PRT

<213> hepatitis C virus


<400> 1066

Phe Leu Gly Thr Ser Ile Ser Gly Val
1               5


<210> 1067

<211> 9

<212> PRT

<213> hepatitis C virus


<400> 1067

Phe Leu Gly Thr Thr Val Gly Gly Val
1               5


<210> 1068

<211> 9

<212> PRT

<213> hepatitis C virus

<400> 1068

Phe Leu Leu Ala Leu Phe Ser Cys Leu
1               5

<210> 1069

<211> 9

<212> PRT

<213> hepatitis C virus

<400> 1069

Phe Leu Leu Ala Leu Leu Ser Cys Ile
1               5

<210> 1070

<211> 10

<212> PRT

<213> hepatitis C virus

<400> 1070

Phe Leu Leu Ala Leu Leu Ser Cys Leu Thr
1               5                   10

<210> 1071

<211> 9

<212> PRT

<213> hepatitis C virus

<400> 1071

Phe Leu Leu Leu Ala Asp Ala Arg Ile
1               5

<210> 1072

<211> 9

<212> PRT

<213> hepatitis C virus

<400> 1072

Phe Leu Leu Leu Ala Asp Ala Arg Val
1                   5

<210>   1073

<211>   9

<212>   PRT

<213>   hepatitis C virus


<400>   1073

Phe Leu Val Cys Gly Asp Asp Leu Val
1                   5

<210>   1074

<211>   10

<212>   PRT

<213>   hepatitis C virus


<400>   1074

Phe Leu Val Cys Gly Asp Asp Leu Val Val
1                   5                   10

<210>   1075

<211>   9

<212>   PRT

<213>   hepatitis C virus


<400>   1075

Phe Met Gly Gly Asp Val Thr Arg Ile
1                   5

<210>   1076

<211>   9

<212>   PRT

<213>   hepatitis C virus


<400>   1076

Phe Asn Trp Ala Val Arg Thr Lys Leu
1                   5


<210>   1077

<211> 10

<212> PRT

<213> hepatitis C virus


<400> 1077

Phe Pro Gly Gly Gly Gln Ile Val Gly Gly
1               5                   10


<210> 1078

<211> 9

<212> PRT

<213> hepatitis C virus


<400> 1078

Phe Pro Pro Ala Leu Pro Ile Trp Ala
1               5


<210> 1079

<211> 10

<212> PRT

<213> hepatitis C virus


<400> 1079

Phe Pro Tyr Leu Val Ala Tyr Gln Ala Thr
1               5                   10


<210> 1080

<211> 10

<212> PRT

<213> hepatitis C virus


<400> 1080

Phe Gln Val Ala His Leu His Ala Pro Thr
1               5                   10


<210> 1081

<211> 10

<212> PRT

<213> hepatitis C virus

<400> 1081

Phe Arg Ala Ala Val Cys Thr Arg Gly Val
1               5                   10


<210> 1082

<211> 9

<212> PRT

<213> hepatitis C virus


<400> 1082

Phe Arg Lys His Pro Glu Ala Thr Tyr
1               5


<210> 1083

<211> 10

<212> PRT

<213> hepatitis C virus


<400> 1083

Phe Ser Ile Leu Leu Ala Gln Glu Gln Leu
1               5                   10


<210> 1084

<211> 9

<212> PRT

<213> hepatitis C virus


<400> 1084

Phe Ser Met Gln Gly Ala Trp Ala Lys
1               5


<210> 1085

<211> 9

<212> PRT

<213> hepatitis C virus


<400> 1085

Phe Thr Ala Ser Ile Thr Ser Pro Leu
1               5

<210> 1086

<211> 10

<212> PRT

<213> hepatitis C virus


<400> 1086

Phe Thr Asp Asn Ser Ser Pro Pro Ala Val
1               5               10


<210> 1087

<211> 10

<212> PRT

<213> hepatitis C virus


<400> 1087

Phe Thr Glu Ala Met Thr Arg Tyr Ser Ala
1               5               10


<210> 1088

<211> 9

<212> PRT

<213> hepatitis C virus


<400> 1088

Phe Thr Ile Phe Lys Ile Arg Met Tyr
1               5


<210> 1089

<211> 9

<212> PRT

<213> hepatitis C virus


<400> 1089

Phe Thr Ile Phe Lys Val Arg Met Tyr
1               5


<210> 1090

<211> 9

<212> PRT

<213> hepatitis C virus

<400> 1090

Phe Val Gly Leu Ala Leu Leu Thr Leu
1               5

<210> 1091

<211> 9

<212> PRT

<213> hepatitis C virus

<400> 1091

Phe Val Arg Ala His Ala Leu Leu Arg
1               5

<210> 1092

<211> 10

<212> PRT

<213> hepatitis C virus

<400> 1092

Phe Val Arg Ala Gln Gly Leu Ile Arg Ala
1               5               10

<210> 1093

<211> 9

<212> PRT

<213> hepatitis C virus

<400> 1093

Phe Val Ser Gly Ile Gln Tyr Leu Ala
1               5

<210> 1094

<211> 9

<212> PRT

<213> hepatitis C virus

<400> 1094

Phe Val Val Ser Gly Leu Ala Gly Ala
1               5


<210> 1095

<211> 9

<212> PRT

<213> hepatitis C virus


<400> 1095

Phe Trp Ala Lys His Met Trp Asn Phe
1               5


<210> 1096

<211> 10

<212> PRT

<213> hepatitis C virus


<400> 1096

Phe Trp Ala Lys His Met Trp Asn Phe Ile
1               5                   10


<210> 1097

<211> 9

<212> PRT

<213> hepatitis C virus


<400> 1097

Phe Trp Ala Asn Asp Met Trp Asn Phe
1               5


<210> 1098

<211> 9

<212> PRT

<213> hepatitis C virus


<400> 1098

Phe Trp Ala Arg His Met Trp Asn Phe
1               5

<210> 1099

<211> 9

<212> PRT

<213> hepatitis C virus


<400> 1099

Phe Trp Glu Ala Val Phe Thr Gly Leu
1               5


<210> 1100

<211> 10

<212> PRT

<213> hepatitis C virus


<400> 1100

Phe Tyr Gly Lys Ala Ile Pro Ile Glu Val
1               5               10


<210> 1101

<211> 9

<212> PRT

<213> hepatitis C virus


<400> 1101

Phe Tyr Pro Gly Val Val Phe Asp Ile
1               5


<210> 1102

<211> 9

<212> PRT

<213> hepatitis C virus


<400> 1102

Gly Ala Ala Val Gly Ser Ile Gly Leu
1               5


<210> 1103

<211> 9

<212> PRT

<213> hepatitis C virus

<400> 1103

Gly Ala Cys Tyr Ser Ile Glu Pro Leu
1               5

<210> 1104
<211> 9
<212> PRT
<213> hepatitis C virus

<400> 1104

Gly Ala His Gly Ile Leu Ser Phe Leu
1               5

<210> 1105
<211> 10
<212> PRT
<213> hepatitis C virus

<400> 1105

Gly Ala His Trp Gly Val Leu Ala Gly Leu
1               5                   10

<210> 1106
<211> 10
<212> PRT
<213> hepatitis C virus

<400> 1106

Gly Ala Arg Leu Val Val Leu Ala Thr Ala
1               5                   10

<210> 1107
<211> 9
<212> PRT
<213> hepatitis C virus

<400> 1107

Gly Ala Val Phe Val Gly Leu Ala Leu
1                   5

<210>   1108

<211>   10

<212>   PRT

<213>   hepatitis C virus


<400>   1108

Gly Ala Val Phe Val Gly Leu Ala Leu Leu
1               5                   10

<210>   1109

<211>   9

<212>   PRT

<213>   hepatitis C virus


<400>   1109

Gly Ala Val Gln Trp Met Asn Arg Leu
1                   5

<210>   1110

<211>   10

<212>   PRT

<213>   hepatitis C virus


<400>   1110

Gly Ala Tyr Met Ser Lys Ala His Gly Val
1               5                   10

<210>   1111

<211>   8

<212>   PRT

<213>   hepatitis C virus


<400>   1111

Gly Cys Ser Phe Ser Ile Phe Leu
1                   5

<210>   1112

<211> 10

<212> PRT

<213> hepatitis C virus


<400> 1112

Gly Asp Asn Phe Pro Tyr Leu Val Ala Tyr
1               5                   10


<210> 1113

<211> 9

<212> PRT

<213> hepatitis C virus


<400> 1113

Gly Glu Asp Val Val Cys Cys Ser Met
1               5


<210> 1114

<211> 10

<212> PRT

<213> hepatitis C virus


<400> 1114

Gly Glu Gly Ala Val Gln Trp Met Asn Arg
1               5                   10


<210> 1115

<211> 10

<212> PRT

<213> hepatitis C virus


<400> 1115

Gly Glu Ile Asn Arg Val Ala Ser Cys Leu
1               5                   10


<210> 1116

<211> 9

<212> PRT

<213> hepatitis C virus

```
<400>  1116

Gly Glu Ile Pro Phe Tyr Gly Lys Ala
1               5


<210>  1117

<211>  10

<212>  PRT

<213>  hepatitis C virus


<400>  1117

Gly Glu Ile Pro Phe Tyr Gly Lys Ala Ile
1               5                       10


<210>  1118

<211>  9

<212>  PRT

<213>  hepatitis C virus


<400>  1118

Gly Glu Ile Pro Phe Tyr Gly Arg Ala
1               5


<210>  1119

<211>  10

<212>  PRT

<213>  hepatitis C virus


<400>  1119

Gly Glu Ile Pro Phe Tyr Gly Arg Ala Ile
1               5                       10


<210>  1120

<211>  9

<212>  PRT

<213>  hepatitis C virus


<400>  1120

Gly Glu Ile Gln Val Leu Ser Thr Val
1               5
```

<210> 1121

<211> 10

<212> PRT

<213> hepatitis C virus


<400> 1121

Gly Glu Ile Gln Val Leu Ser Thr Val Thr
1               5                   10

<210> 1122

<211> 9

<212> PRT

<213> hepatitis C virus


<400> 1122

Gly Glu Asn Phe Ala Tyr Leu Thr Ala
1               5

<210> 1123

<211> 10

<212> PRT

<213> hepatitis C virus


<400> 1123

Gly Glu Asn Phe Ala Tyr Leu Thr Ala Tyr
1               5                   10

<210> 1124

<211> 9

<212> PRT

<213> hepatitis C virus


<400> 1124

Gly Glu Asn Phe Pro Tyr Leu Val Ala
1               5

<210> 1125

<211> 10

<212> PRT

<213> hepatitis C virus


<400> 1125

Gly Glu Asn Phe Pro Tyr Leu Val Ala Tyr
1               5                   10


<210> 1126

<211> 9

<212> PRT

<213> hepatitis C virus


<400> 1126

Gly Glu Asn Leu Pro Tyr Leu Val Ala
1               5


<210> 1127

<211> 10

<212> PRT

<213> hepatitis C virus


<400> 1127

Gly Glu Asn Leu Pro Tyr Leu Val Ala Tyr
1               5                   10


<210> 1128

<211> 9

<212> PRT

<213> hepatitis C virus


<400> 1128

Gly Glu Pro Gly Asp Pro Asp Leu Ser
1               5


<210> 1129

<211> 9

<212> PRT

<213> hepatitis C virus

<400> 1129

Gly Glu Val Gln Val Leu Ser Thr Ala
1               5

<210> 1130

<211> 10

<212> PRT

<213> hepatitis C virus

<400> 1130

Gly Glu Val Gln Val Leu Ser Thr Ala Thr
1               5                   10

<210> 1131

<211> 9

<212> PRT

<213> hepatitis C virus

<400> 1131

Gly Glu Val Gln Val Val Ser Thr Ala
1               5

<210> 1132

<211> 10

<212> PRT

<213> hepatitis C virus

<400> 1132

Gly Phe Ala Asp Leu Met Gly Tyr Ile Pro
1               5                   10

<210> 1133

<211> 10

<212> PRT

<213> hepatitis C virus

<400> 1133

Gly Phe Phe Thr Leu Ser Pro Trp Tyr Lys
1               5                   10

<210> 1134

<211> 9

<212> PRT

<213> hepatitis C virus

<400> 1134

Gly Phe Ile Ala Gly Leu Phe Tyr Tyr
1               5

<210> 1135

<211> 10

<212> PRT

<213> hepatitis C virus

<400> 1135

Gly Gly Ala Val Phe Val Gly Leu Ala Leu
1               5                    10

<210> 1136

<211> 9

<212> PRT

<213> hepatitis C virus

<400> 1136

Gly Gly Gly Gln Ile Val Gly Gly Val
1               5

<210> 1137

<211> 9

<212> PRT

<213> hepatitis C virus

<400> 1137

Gly Gly Gln Ile Val Gly Gly Val Tyr
1               5

<210> 1138

<211> 9

<212> PRT

<213> hepatitis C virus

<400> 1138

Gly Gly Arg Asp Ala Ile Ile Leu Leu
1               5

<210> 1139
<211> 10
<212> PRT
<213> hepatitis C virus

<400> 1139

Gly Gly Arg Lys Pro Ala Arg Leu Ile Val
1               5                   10

<210> 1140
<211> 10
<212> PRT
<213> hepatitis C virus

<400> 1140

Gly Ile Phe Arg Ala Ala Val Cys Ser Arg
1               5                   10

<210> 1141
<211> 10
<212> PRT
<213> hepatitis C virus

<400> 1141

Gly Ile Phe Arg Ala Ala Val Cys Thr Arg
1               5                   10

<210> 1142
<211> 9
<212> PRT
<213> hepatitis C virus

<400> 1142

Gly Ile Leu Ala Gly Leu Ala Tyr Tyr
1               5

<210>   1143

<211>   9

<212>   PRT

<213>   hepatitis C virus


<400>   1143

Gly Ile Asn Ala Val Ala Tyr Tyr Arg
1               5

<210>   1144

<211>   9

<212>   PRT

<213>   hepatitis C virus


<400>   1144

Gly Ile Pro Phe Ile Ser Cys Gln Lys
1               5

<210>   1145

<211>   10

<212>   PRT

<213>   hepatitis C virus


<400>   1145

Gly Ile Ser Gly Ala Leu Val Ala Phe Lys
1               5                   10

<210>   1146

<211>   10

<212>   PRT

<213>   hepatitis C virus


<400>   1146

Gly Lys Ser Thr Lys Val Pro Ala Ala Tyr
1               5                   10

<210>   1147

```
<211>  9
<212>  PRT
<213>  hepatitis C virus


<400>  1147
Gly Leu Asp Ala Phe Ser Leu His Thr
1               5


<210>  1148
<211>  10
<212>  PRT
<213>  hepatitis C virus


<400>  1148
Gly Leu Asp Ala Phe Ser Leu His Thr Tyr
1               5                   10


<210>  1149
<211>  10
<212>  PRT
<213>  hepatitis C virus


<400>  1149
Gly Leu Gly Lys Val Leu Val Asp Ile Leu
1               5                   10


<210>  1150
<211>  9
<212>  PRT
<213>  hepatitis C virus


<400>  1150
Gly Leu Gly Trp Ala Gly Trp Leu Leu
1               5


<210>  1151
<211>  10
<212>  PRT
<213>  hepatitis C virus
```

<400> 1151

Gly Leu Leu Gly Cys Ile Ile Thr Ser Leu
1               5                   10


<210> 1152

<211> 9

<212> PRT

<213> hepatitis C virus


<400> 1152

Gly Leu Pro Phe Ile Ser Cys Gln Lys
1               5


<210> 1153

<211> 10

<212> PRT

<213> hepatitis C virus


<400> 1153

Gly Leu Ser Ala Phe Ser Leu His Ser Tyr
1               5                   10


<210> 1154

<211> 10

<212> PRT

<213> hepatitis C virus


<400> 1154

Gly Leu Ser Ala Phe Ser Leu His Ser Tyr
1               5                   10


<210> 1155

<211> 10

<212> PRT

<213> hepatitis C virus


<400> 1155

Gly Leu Ser Ala Phe Thr Leu His Ser Tyr
1               5                   10

<210> 1156

<211> 9

<212> PRT

<213> hepatitis C virus


<400> 1156

Gly Leu Ser Pro Ala Ile Thr Lys Tyr
1               5


<210> 1157

<211> 9

<212> PRT

<213> hepatitis C virus


<400> 1157

Gly Leu Tyr Leu Phe Asn Trp Ala Val
1               5


<210> 1158

<211> 10

<212> PRT

<213> hepatitis C virus


<400> 1158

Gly Leu Tyr Leu Phe Asn Trp Ala Val Arg
1               5                   10


<210> 1159

<211> 9

<212> PRT

<213> hepatitis C virus


<400> 1159

Gly Met Gly Leu Asn Ala Val Ala Tyr
1               5


<210> 1160

<211> 10

<212> PRT

<213> hepatitis C virus


<400> 1160

Gly Asn Ile Ile Met Tyr Ala Pro Thr Leu
1                   5                   10


<210> 1161

<211> 9

<212> PRT

<213> hepatitis C virus


<400> 1161

Gly Pro Cys Thr Pro Ser Pro Ala Pro
1                   5


<210> 1162

<211> 9

<212> PRT

<213> hepatitis C virus


<400> 1162

Gly Pro Gly Glu Gly Ala Val Gln Trp
1                   5


<210> 1163

<211> 10

<212> PRT

<213> hepatitis C virus


<400> 1163

Gly Pro Gly Glu Gly Ala Val Gln Trp Met
1                   5                   10


<210> 1164

<211> 10

<212> PRT

<213> hepatitis C virus

<400> 1164

Gly Pro Ile Thr Gln Met Tyr Thr Asn Val
1               5                   10

<210> 1165

<211> 9

<212> PRT

<213> hepatitis C virus


<400> 1165

Gly Pro Lys Gly Pro Ile Thr Gln Met
1               5

<210> 1166

<211> 9

<212> PRT

<213> hepatitis C virus


<400> 1166

Gly Pro Lys Gly Pro Val Thr Gln Met
1               5

<210> 1167

<211> 10

<212> PRT

<213> hepatitis C virus


<400> 1167

Gly Pro Leu Leu Cys Pro Ser Gly His Ala
1               5                   10

<210> 1168

<211> 10

<212> PRT

<213> hepatitis C virus


<400> 1168

Gly Pro Leu Met Val Leu Gln Ala Gly Ile
1               5                   10

<210> 1169

<211> 9

<212> PRT

<213> hepatitis C virus


<400> 1169

Gly Pro Pro Cys Asn Ile Gly Gly Val
1               5


<210> 1170

<211> 10

<212> PRT

<213> hepatitis C virus


<400> 1170

Gly Pro Arg Leu Gly Val Arg Ala Thr Arg
1               5                   10


<210> 1171

<211> 9

<212> PRT

<213> hepatitis C virus


<400> 1171

Gly Pro Ser Gln Lys Ile Gln Leu Ile
1               5


<210> 1172

<211> 9

<212> PRT

<213> hepatitis C virus


<400> 1172

Gly Pro Thr Asp Pro Arg Arg Arg Ser
1               5


<210> 1173

<211> 9

<212> PRT

<213> hepatitis C virus

<400> 1173

Gly Pro Trp Leu Thr Pro Arg Cys Leu
1               5

<210> 1174

<211> 10

<212> PRT

<213> hepatitis C virus

<400> 1174

Gly Pro Trp Leu Thr Pro Arg Cys Leu Val
1               5                   10

<210> 1175

<211> 9

<212> PRT

<213> hepatitis C virus

<400> 1175

Gly Pro Trp Leu Thr Pro Arg Cys Met
1               5

<210> 1176

<211> 9

<212> PRT

<213> hepatitis C virus

<400> 1176

Gly Gln Ala Glu Ala Ala Leu Glu Lys
1               5

<210> 1177

<211> 9

<212> PRT

<213> hepatitis C virus

<400> 1177

Gly Gln Ala Phe Thr Phe Arg Pro Arg
1               5

<210>  1178

<211>  10

<212>  PRT

<213>  hepatitis C virus


<400>  1178

Gly Gln Ile Val Gly Gly Val Tyr Leu Leu
1               5                   10


<210>  1179

<211>  9

<212>  PRT

<213>  hepatitis C virus


<400>  1179

Gly Arg Ala Ala Ile Cys Gly Lys Tyr
1               5


<210>  1180

<211>  10

<212>  PRT

<213>  hepatitis C virus


<400>  1180

Gly Arg Ala Trp Ala Gln Pro Gly Tyr Pro
1               5                   10


<210>  1181

<211>  10

<212>  PRT

<213>  hepatitis C virus


<400>  1181

Gly Arg Gly Arg Arg Gly Ile Tyr Arg Phe
1               5                   10


<210>  1182

<211> 10

<212> PRT

<213> hepatitis C virus


<400> 1182

Gly Arg Lys Pro Ala Arg Leu Ile Val Phe
1               5                   10


<210> 1183

<211> 10

<212> PRT

<213> hepatitis C virus


<400> 1183

Gly Arg Arg Gly Ile Tyr Arg Phe Val Thr
1               5                   10


<210> 1184

<211> 10

<212> PRT

<213> hepatitis C virus


<400> 1184

Gly Arg Thr Gly Arg Gly Arg Arg Gly Ile
1               5                   10


<210> 1185

<211> 9

<212> PRT

<213> hepatitis C virus


<400> 1185

Gly Ser Glu Gly Glu Ile Pro Phe Tyr
1               5


<210> 1186

<211> 9

<212> PRT

<213> hepatitis C virus

<400> 1186

Gly Ser Ile Gly Leu Gly Lys Val Leu
1               5

<210> 1187

<211> 10

<212> PRT

<213> hepatitis C virus

<400> 1187

Gly Ser Ile Gly Leu Gly Lys Val Leu Val
1               5                   10

<210> 1188

<211> 9

<212> PRT

<213> hepatitis C virus

<400> 1188

Gly Ser Met Arg Ile Thr Gly Pro Lys
1               5

<210> 1189

<211> 9

<212> PRT

<213> hepatitis C virus

<400> 1189

Gly Ser Val Phe Leu Val Ser Gln Leu
1               5

<210> 1190

<211> 10

<212> PRT

<213> hepatitis C virus

<400> 1190

Gly Ser Trp His Ile Asn Arg Thr Ala Leu
1               5                   10

<210> 1191

<211> 9

<212> PRT

<213> hepatitis C virus


<400> 1191

Gly Val Ala Gly Ala Leu Val Ala Phe
1               5


<210> 1192

<211> 10

<212> PRT

<213> hepatitis C virus


<400> 1192

Gly Val Ala Gly Ala Leu Val Ala Phe Lys
1               5                   10


<210> 1193

<211> 10

<212> PRT

<213> hepatitis C virus


<400> 1193

Gly Val Ala Gly Ala Leu Val Ala Phe Lys
1               5                   10


<210> 1194

<211> 9

<212> PRT

<213> hepatitis C virus


<400> 1194

Gly Val Ile Cys Ala Ala Ile Leu Arg
1               5


<210> 1195

<211> 10

<212> PRT

<213> hepatitis C virus

<400> 1195

Gly Val Ile Cys Ala Ala Ile Leu Arg Arg
1               5                       10

<210> 1196

<211> 9

<212> PRT

<213> hepatitis C virus

<400> 1196

Gly Val Leu Ala Ala Leu Ala Ala Tyr
1               5

<210> 1197

<211> 9

<212> PRT

<213> hepatitis C virus

<400> 1197

Gly Val Leu Ala Gly Leu Ala Tyr Tyr
1               5

<210> 1198

<211> 10

<212> PRT

<213> hepatitis C virus

<400> 1198

Gly Val Met Trp Thr Val Tyr His Gly Ala
1               5                       10

<210> 1199

<211> 9

<212> PRT

<213> hepatitis C virus

<400> 1199

Gly Val Asn Ala Val Ala Tyr Tyr Arg
1               5

<210> 1200

<211> 10

<212> PRT

<213> hepatitis C virus


<400> 1200

Gly Val Arg Ala Thr Arg Lys Thr Ser Glu
1               5                   10

<210> 1201

<211> 10

<212> PRT

<213> hepatitis C virus


<400> 1201

Gly Val Arg Leu His Arg Tyr Ala Pro Ala
1               5                   10

<210> 1202

<211> 9

<212> PRT

<213> hepatitis C virus


<400> 1202

Gly Val Arg Thr Ile Thr Thr Gly Ala
1               5

<210> 1203

<211> 10

<212> PRT

<213> hepatitis C virus


<400> 1203

Gly Val Arg Val Cys Glu Lys Met Ala Leu
1               5                   10

<210> 1204

<211> 10

<212> PRT

<213> hepatitis C virus


<400> 1204

```
Gly Val Ser Gly Ala Leu Val Ala Phe Lys
1               5                   10
```

<210> 1205

<211> 9

<212> PRT

<213> hepatitis C virus


<400> 1205

```
Gly Val Val Cys Ala Ala Ile Leu Arg
1               5
```

<210> 1206

<211> 10

<212> PRT

<213> hepatitis C virus


<400> 1206

```
Gly Val Val Cys Ala Ala Ile Leu Arg Arg
1               5                   10
```

<210> 1207

<211> 9

<212> PRT

<213> hepatitis C virus


<400> 1207

```
Gly Val Trp Pro Leu Leu Leu Leu Leu
1               5
```

<210> 1208

<211> 10

<212> PRT

<213> hepatitis C virus

<400> 1208

Gly Val Trp Pro Leu Leu Leu Leu Leu Leu
1               5                       10

<210> 1209

<211> 9

<212> PRT

<213> hepatitis C virus

<400> 1209

Gly Val Trp Arg Gly Asp Gly Ile Met
1               5

<210> 1210

<211> 10

<212> PRT

<213> hepatitis C virus

<400> 1210

Gly Tyr Gly Ala Gly Val Ala Gly Ala Leu
1               5                       10

<210> 1211

<211> 10

<212> PRT

<213> hepatitis C virus

<400> 1211

Gly Tyr Ile Pro Leu Val Gly Ala Pro Leu
1               5                       10

<210> 1212

<211> 10

<212> PRT

<213> hepatitis C virus

<400> 1212

Gly Tyr Arg Arg Cys Arg Ala Ser Gly Val
1               5                   10

<210> 1213

<211> 10

<212> PRT

<213> hepatitis C virus


<400> 1213

Gly Tyr Thr Gly Asp Phe Asp Ser Val Ile
1               5                   10

<210> 1214

<211> 9

<212> PRT

<213> hepatitis C virus


<400> 1214

Gly Tyr Thr Ser Lys Gly Trp Lys Leu
1               5

<210> 1215

<211> 10

<212> PRT

<213> hepatitis C virus


<400> 1215

His Ala Val Gly Ile Phe Arg Ala Ala Val
1               5                   10

<210> 1216

<211> 10

<212> PRT

<213> hepatitis C virus


<400> 1216

His Asp Ala Ser Gly Lys Arg Val Tyr Tyr
1               5                   10

<210> 1217

<211> 9

<212> PRT

<213> hepatitis C virus

<400> 1217

His Glu Leu Thr Arg Val Ala Ala Ala
1               5

<210> 1218

<211> 10

<212> PRT

<213> hepatitis C virus

<400> 1218

His Glu Leu Thr Arg Val Ala Ala Ala Leu
1               5                   10

<210> 1219

<211> 10

<212> PRT

<213> hepatitis C virus

<400> 1219

His Gly Pro Thr Pro Leu Leu Tyr Arg Leu
1               5                   10

<210> 1220

<211> 9

<212> PRT

<213> hepatitis C virus

<400> 1220

His His Asp Ser Pro Asp Ala Asp Leu
1               5

<210> 1221

<211> 9

<212> PRT

<213> hepatitis C virus

<400> 1221

His Ile Asp Ala His Phe Leu Ser Gln
1               5

<210> 1222

<211> 10

<212> PRT

<213> hepatitis C virus

<400> 1222

His Ile Asp Ala His Phe Leu Ser Gln Thr
1               5                   10

<210> 1223

<211> 9

<212> PRT

<213> hepatitis C virus

<400> 1223

His Ile Arg Ser Val Trp Lys Asp Leu
1               5

<210> 1224

<211> 10

<212> PRT

<213> hepatitis C virus

<400> 1224

His Ile Arg Ser Val Trp Lys Asp Leu Leu
1               5                   10

<210> 1225

<211> 10

<212> PRT

<213> hepatitis C virus

<400> 1225

His Leu Glu Phe Trp Glu Ser Val Phe Thr
1               5                   10

<210> 1226

<211> 10

<212> PRT

<213> hepatitis C virus


<400> 1226

His Leu His Ala Pro Thr Gly Ser Gly Lys
1               5                   10


<210> 1227

<211> 9

<212> PRT

<213> hepatitis C virus


<400> 1227

His Leu His Gln Asn Ile Val Asp Val
1               5


<210> 1228

<211> 10

<212> PRT

<213> hepatitis C virus


<400> 1228

His Leu Ile Phe Cys His Ser Lys Lys Lys
1               5                   10


<210> 1229

<211> 9

<212> PRT

<213> hepatitis C virus


<400> 1229

His Leu Ile Phe Cys His Ser Arg Lys
1               5


<210> 1230

<211> 9

<212> PRT

<213> hepatitis C virus

<400> 1230

His Leu Leu Leu Cys Leu Leu Leu Leu
1               5

<210> 1231

<211> 9

<212> PRT

<213> hepatitis C virus

<400> 1231

His Leu Pro Gly Cys Val Pro Cys Val
1               5

<210> 1232

<211> 9

<212> PRT

<213> hepatitis C virus

<400> 1232

His Leu Gln Val Trp Val Pro Pro Leu
1               5

<210> 1233

<211> 9

<212> PRT

<213> hepatitis C virus

<400> 1233

His Met Trp Asn Phe Ile Ser Gly Ile
1               5

<210> 1234

<211> 9

<212> PRT

<213> hepatitis C virus

<400> 1234

His Met Trp Asn Phe Val Ser Gly Ile
1               5

<210> 1235

<211> 9

<212> PRT

<213> hepatitis C virus


<400> 1235

His Pro Glu Leu Ile Phe Asp Ile Thr
1               5

<210> 1236

<211> 10

<212> PRT

<213> hepatitis C virus


<400> 1236

His Pro Ile Thr Lys Tyr Ile Met Ala Cys
1               5                   10

<210> 1237

<211> 9

<212> PRT

<213> hepatitis C virus


<400> 1237

His Pro Asn Ile Glu Glu Val Ala Leu
1               5

<210> 1238

<211> 9

<212> PRT

<213> hepatitis C virus


<400> 1238

His Pro Val Thr Lys Tyr Ile Met Ala
1               5

<210> 1239

<211> 9

<212> PRT

<213> hepatitis C virus


<400> 1239

His Gln Asn Ile Val Asp Val Gln Tyr
1               5


<210> 1240

<211> 10

<212> PRT

<213> hepatitis C virus


<400> 1240

His Gln Asn Ile Val Asp Val Gln Tyr Leu
1               5                   10


<210> 1241

<211> 9

<212> PRT

<213> hepatitis C virus


<400> 1241

His Ser Ala Lys Ser Lys Phe Gly Tyr
1               5


<210> 1242

<211> 9

<212> PRT

<213> hepatitis C virus


<400> 1242

His Ser Ala Arg Ser Lys Phe Gly Tyr
1               5


<210> 1243

<211> 10

<212> PRT

<213> hepatitis C virus

<400> 1243

His Ser Lys Lys Lys Cys Asp Glu Leu Ala
1               5               10

<210> 1244

<211> 9

<212> PRT

<213> hepatitis C virus

<400> 1244

His Ser Thr Asp Ser Thr Thr Ile Leu
1               5

<210> 1245

<211> 9

<212> PRT

<213> hepatitis C virus

<400> 1245

His Trp Ile Gly Arg Leu Ile Trp Trp
1               5

<210> 1246

<211> 9

<212> PRT

<213> hepatitis C virus

<400> 1246

His Tyr Ala Pro Arg Pro Cys Gly Ile
1               5

<210> 1247

<211> 10

<212> PRT

<213> hepatitis C virus

<400> 1247

His Tyr Pro Cys Thr Val Asn Phe Thr Ile
1               5                   10

<210>  1248

<211>  10

<212>  PRT

<213>  hepatitis C virus


<400>  1248

His Tyr Pro Cys Thr Val Asn Phe Thr Leu
1               5                   10

<210>  1249

<211>  10

<212>  PRT

<213>  hepatitis C virus


<400>  1249

His Tyr Pro Cys Thr Val Asn Tyr Thr Ile
1               5                   10

<210>  1250

<211>  9

<212>  PRT

<213>  hepatitis C virus


<400>  1250

His Tyr Pro Pro Lys Pro Cys Gly Ile
1               5

<210>  1251

<211>  9

<212>  PRT

<213>  hepatitis C virus


<400>  1251

His Tyr Pro Pro Arg Pro Cys Gly Ile
1               5


<210>  1252

```
<211>  9
<212>  PRT
<213>  hepatitis C virus


<400>  1252
His Tyr Pro Tyr Arg Leu Trp His Tyr
1               5


<210>  1253
<211>  9
<212>  PRT
<213>  hepatitis C virus


<400>  1253
His Tyr Arg Asp Val Leu Lys Glu Met
1               5


<210>  1254
<211>  10
<212>  PRT
<213>  hepatitis C virus


<400>  1254
His Tyr Val Pro Glu Ser Asp Ala Ala Ala
1               5                   10


<210>  1255
<211>  10
<212>  PRT
<213>  hepatitis C virus


<400>  1255
Ile Ala Phe Ala Ser Arg Gly Asn His Val
1               5                   10


<210>  1256
<211>  10
<212>  PRT
<213>  hepatitis C virus
```

<400> 1256

Ile Asp Ala His Phe Leu Ser Gln Thr Lys
1               5                   10


<210> 1257

<211> 9

<212> PRT

<213> hepatitis C virus


<400> 1257

Ile Glu Leu Gly Gly Lys Pro Ala Leu
1               5


<210> 1258

<211> 9

<212> PRT

<213> hepatitis C virus


<400> 1258

Ile Glu Pro Leu Asp Leu Pro Gln Ile
1               5


<210> 1259

<211> 9

<212> PRT

<213> hepatitis C virus


<400> 1259

Ile Glu Arg Leu His Gly Leu Asp Ala
1               5


<210> 1260

<211> 10

<212> PRT

<213> hepatitis C virus


<400> 1260

Ile Glu Arg Leu His Gly Leu Asp Ala Phe
1               5                   10

```
<210>   1261
<211>   9
<212>   PRT
<213>   hepatitis C virus


<400>   1261

Ile Glu Arg Leu His Gly Leu Glu Ala
1               5


<210>   1262
<211>   10
<212>   PRT
<213>   hepatitis C virus


<400>   1262

Ile Glu Arg Leu His Gly Leu Glu Ala Phe
1               5                   10


<210>   1263
<211>   9
<212>   PRT
<213>   hepatitis C virus


<400>   1263

Ile Glu Arg Leu His Gly Leu Ser Ala
1               5


<210>   1264
<211>   10
<212>   PRT
<213>   hepatitis C virus


<400>   1264

Ile Glu Arg Leu His Gly Leu Ser Ala Phe
1               5                   10


<210>   1265
<211>   10
```

```
<212>    PRT

<213>    hepatitis C virus


<400>    1265

Ile Glu Val Ile Lys Gly Gly Arg His Leu
1                  5                   10


<210>    1266

<211>    10

<212>    PRT

<213>    hepatitis C virus


<400>    1266

Ile Phe Leu Leu Ala Leu Leu Ser Cys Leu
1                  5                   10


<210>    1267

<211>    10

<212>    PRT

<213>    hepatitis C virus


<400>    1267

Ile Gly Leu Gly Lys Val Leu Val Asp Ile
1                  5                   10


<210>    1268

<211>    10

<212>    .PRT

<213>    hepatitis C virus


<400>    1268

Ile Ile Met Tyr Ala Pro Thr Leu Trp Ala
1                  5                   10


<210>    1269

<211>    9

<212>    PRT

<213>    hepatitis C virus
```

<400> 1269

Ile Leu Ala Gly Tyr Gly Ala Gly Val
1               5


<210> 1270

<211> 10

<212> PRT

<213> hepatitis C virus


<400> 1270

Ile Leu Gly Gly Trp Val Ala Ala Gln Leu
1               5                       10


<210> 1271

<211> 9

<212> PRT

<213> hepatitis C virus


<400> 1271

Ile Leu Leu Gly Pro Ala Asp Ser Leu
1               5


<210> 1272

<211> 10

<212> PRT

<213> hepatitis C virus


<400> 1272

Ile Leu Leu Asn Ile Met Gly Gly Trp Leu
1               5                       10


<210> 1273

<211> 9

<212> PRT

<213> hepatitis C virus


<400> 1273

Ile Leu Met Thr His Phe Phe Ser Ile
1               5

<210> 1274

<211> 10

<212> PRT

<213> hepatitis C virus

<400> 1274

Ile Leu Met Thr His Phe Phe Ser Ile Leu
1               5                   10

<210> 1275

<211> 9

<212> PRT

<213> hepatitis C virus

<400> 1275

Ile Leu Ser Pro Gly Ala Leu Val Val
1               5

<210> 1276

<211> 10

<212> PRT

<213> hepatitis C virus

<400> 1276

Ile Leu Ser Ser Leu Thr Ile Thr Gln Leu
1               5                   10

<210> 1277

<211> 9

<212> PRT

<213> hepatitis C virus

<400> 1277

Ile Leu Thr Leu Ser Pro His Tyr Lys
1               5

<210> 1278

<211> 10

<212> PRT

<213> hepatitis C virus

<400> 1278

Ile Met Ala Lys Asn Glu Val Phe Cys Val
1               5               10

<210> 1279

<211> 10

<212> PRT

<213> hepatitis C virus

<400> 1279

Ile Met Tyr Ala Pro Thr Leu Trp Ala Arg
1               5               10

<210> 1280

<211> 9

<212> PRT

<213> hepatitis C virus

<400> 1280

Ile Pro Ala Ala Ser Gln Leu Asp Leu
1               5

<210> 1281

<211> 10

<212> PRT

<213> hepatitis C virus

<400> 1281

Ile Pro Ala Ser Ala Tyr Glu Val Arg Asn
1               5               10

<210> 1282

<211> 9

<212> PRT

<213> hepatitis C virus

<400> 1282

```
Ile Pro Asp Arg Glu Val Leu Tyr Arg
1               5
```

<210> 1283

<211> 8

<212> PRT

<213> hepatitis C virus


<400> 1283

```
Ile Pro Phe Tyr Gly Lys Ala Ile
1               5
```

<210> 1284

<211> 10

<212> PRT

<213> hepatitis C virus


<400> 1284

```
Ile Pro Phe Tyr Gly Lys Ala Ile Pro Ile
1               5                   10
```

<210> 1285

<211> 10

<212> PRT

<213> hepatitis C virus


<400> 1285

```
Ile Pro Phe Tyr Gly Lys Ala Ile Pro Leu
1               5                   10
```

<210> 1286

<211> 8

<212> PRT

<213> hepatitis C virus


<400> 1286

```
Ile Pro Lys Ala Arg Arg Pro Glu
1               5
```

<210> 1287

<211> 10

<212> PRT

<213> hepatitis C virus

<400> 1287

Ile Pro Lys Ala Arg Arg Pro Glu Gly Arg
1               5                   10

<210> 1288

<211> 8

<212> PRT

<213> hepatitis C virus

<400> 1288

Ile Pro Leu Val Gly Ala Pro Leu
1               5

<210> 1289

<211> 8

<212> PRT

<213> hepatitis C virus

<400> 1289

Ile Pro Leu Val Gly Ala Pro Leu
1               5

<210> 1290

<211> 10

<212> PRT

<213> hepatitis C virus

<400> 1290

Ile Pro Leu Val Gly Ala Pro Leu Gly Gly
1               5                   10

<210> 1291

<211> 9

<212> PRT

<213> hepatitis C virus

```
<400>  1291

Ile Pro Pro Pro Arg Arg Lys Arg Thr
1               5


<210>  1292

<211>  10

<212>  PRT

<213>  hepatitis C virus


<400>  1292

Ile Pro Pro Pro Arg Arg Lys Arg Thr Val
1               5                   10


<210>  1293

<211>  9

<212>  PRT

<213>  hepatitis C virus


<400>  1293

Ile Pro Gln Ala Val Val Asp Met Val
1               5


<210>  1294

<211>  10

<212>  PRT

<213>  hepatitis C virus


<400>  1294

Ile Pro Gln Ala Val Val Asp Met Val Ala
1               5                   10


<210>  1295

<211>  10

<212>  PRT

<213>  hepatitis C virus


<400>  1295

Ile Pro Thr Ser Gly Asp Val Val Val Val
1               5                   10
```

<210> 1296

<211> 10

<212> PRT

<213> hepatitis C virus

<400> 1296

Ile Pro Val Glu Ser Met Glu Thr Thr Met
1               5                   10

<210> 1297

<211> 9

<212> PRT

<213> hepatitis C virus

<400> 1297

Ile Pro Val Arg Arg Arg Gly Asp Ser
1               5

<210> 1298

<211> 10

<212> PRT

<213> hepatitis C virus

<400> 1298

Ile Gln Arg Leu His Gly Leu Ser Ala Phe
1               5                   10

<210> 1299

<211> 9

<212> PRT

<213> hepatitis C virus

<400> 1299

Ile Gln Tyr Ala Pro Thr Ile Trp Val
1               5

<210> 1300

<211> 10

```
<212>  PRT

<213>  hepatitis C virus


<400>  1300

Ile Gln Tyr Ala Pro Thr Ile Trp Val Arg
1               5                   10


<210>  1301

<211>  10

<212>  PRT

<213>  hepatitis C virus


<400>  1301

Ile Arg Ser Leu Thr Glu Arg Leu Tyr Ile
1               5                   10


<210>  1302

<211>  9

<212>  PRT

<213>  hepatitis C virus


<400>  1302

Ile Ser Gly Ala Leu Val Ala Phe Lys
1               5


<210>  1303

<211>  10

<212>  PRT

<213>  hepatitis C virus


<400>  1303

Ile Ser Gly Ile Gln Tyr Leu Ala Gly Leu
1               5                   10


<210>  1304

<211>  9

<212>  PRT

<213>  hepatitis C virus
```

<400> 1304

Ile Ser Gly Val Leu Trp Thr Val Tyr
1               5

<210> 1305

<211> 9

<212> PRT

<213> hepatitis C virus


<400> 1305

Ile Thr Ala Glu Ala Ala Ala Arg Arg
1               5

<210> 1306

<211> 10

<212> PRT

<213> hepatitis C virus


<400> 1306

Ile Thr Ala Glu Thr Ala Lys Arg Arg Leu
1               5                   10

<210> 1307

<211> 9

<212> PRT

<213> hepatitis C virus


<400> 1307

Ile Thr Ala Tyr Ala Gln Gln Thr Arg
1               5

<210> 1308

<211> 9

<212> PRT

<213> hepatitis C virus


<400> 1308

Ile Thr Lys Leu Leu Leu Ala Ile Leu
1               5

<210> 1309

<211> 9

<212> PRT

<213> hepatitis C virus

<400> 1309

Ile Thr Thr Gly Ser Pro Ile Thr Tyr
1                   5

<210> 1310

<211> 10

<212> PRT

<213> hepatitis C virus

<400> 1310

Ile Thr Tyr Ser Thr Tyr Gly Lys Phe Leu
1                   5                   10

<210> 1311

<211> 9

<212> PRT

<213> hepatitis C virus

<400> 1311

Ile Val Asp Val Gln Tyr Leu Tyr Gly
1                   5

<210> 1312

<211> 10

<212> PRT

<213> hepatitis C virus

<400> 1312

Ile Val Phe Pro Asp Leu Gly Val Arg Val
1                   5                   10

<210> 1313

<211> 10

<212> PRT

<213> hepatitis C virus

<400> 1313

Ile Val Tyr Glu Ala Ala Asp Met Ile Met
1               5                   10

<210> 1314

<211> 10

<212> PRT

<213> hepatitis C virus

<400> 1314

Ile Tyr Arg Phe Val Thr Pro Gly Glu Arg
1               5                   10

<210> 1315

<211> 9

<212> PRT

<213> hepatitis C virus

<400> 1315

Lys Ala Arg Arg Pro Glu Gly Arg Ala
1               5

<210> 1316

<211> 9

<212> PRT

<213> hepatitis C virus

<400> 1316

Lys Ala Ser Thr Val Lys Ala Lys Leu
1               5

<210> 1317

<211> 10

<212> PRT

<213> hepatitis C virus

<400> 1317

```
Lys Ala Ser Thr Val Lys Ala Lys Leu Leu
1               5               10
```

<210> 1318

<211> 9

<212> PRT

<213> hepatitis C virus

<400> 1318

```
Lys Cys Asp Glu Leu Ala Ala Lys Leu
1               5
```

<210> 1319

<211> 10

<212> PRT

<213> hepatitis C virus

<400> 1319

```
Lys Cys Leu Ile Arg Leu Lys Pro Thr Leu
1               5               10
```

<210> 1320

<211> 10

<212> PRT

<213> hepatitis C virus

<400> 1320

```
Lys Glu Val Arg Ser Leu Ser Arg Arg Ala
1               5               10
```

<210> 1321

<211> 10

<212> PRT

<213> hepatitis C virus

<400> 1321

```
Lys Phe Pro Gly Gly Gly Gln Ile Val Gly
1               5               10
```

<210> 1322

```
<211>  9
<212>  PRT
<213>  hepatitis C virus


<400>  1322
Lys Phe Pro Pro Ala Leu Pro Ile Trp
1                   5


<210>  1323
<211>  9
<212>  PRT
<213>  hepatitis C virus


<400>  1323
Lys Gly Gly Arg Lys Pro Ala Arg Leu
1                   5


<210>  1324
<211>  10
<212>  PRT
<213>  hepatitis C virus


<400>  1324
Lys Gly Gly Arg Lys Pro Ala Arg Leu Ile
1                   5                   10


<210>  1325
<211>  9
<212>  PRT
<213>  hepatitis C virus


<400>  1325
Lys Gly Ser Ser Gly Gly Pro Leu Leu
1                   5


<210>  1326
<211>  9
<212>  PRT
<213>  hepatitis C virus
```

<400> 1326

Lys Gly Val Trp Arg Gly Asp Gly Ile
1                   5


<210> 1327

<211> 10

<212> PRT

<213> hepatitis C virus


<400> 1327

Lys Lys Cys Asp Glu Leu Ala Ala Lys Leu
1               5                   10

<210> 1328

<211> 9

<212> PRT

<213> hepatitis C virus


<400> 1328

Lys Lys Asp Pro Met Gly Phe Ser Tyr
1               5


<210> 1329

<211> 9

<212> PRT

<213> hepatitis C virus


<400> 1329

Lys Leu Gly Ala Leu Thr Gly Thr Tyr
1               5


<210> 1330

<211> 9

<212> PRT

<213> hepatitis C virus


<400> 1330

Lys Leu Leu Ala Pro Ile Thr Ala Tyr
1               5

```
<210>  1331
<211>  10
<212>  PRT
<213>  hepatitis C virus


<400>  1331

Lys Leu Leu Leu Ala Ile Leu Gly Pro Leu
1               5                   10


<210>  1332
<211>  10
<212>  PRT
<213>  hepatitis C virus


<400>  1332

Lys Leu Leu Leu Ala Val Leu Gly Pro Leu
1               5                   10


<210>  1333
<211>  9
<212>  PRT
<213>  hepatitis C virus


<400>  1333

Lys Leu Gln Asp Cys Thr Met Leu Val
1               5


<210>  1334
<211>  9
<212>  PRT
<213>  hepatitis C virus


<400>  1334

Lys Leu Thr Tyr Ser Thr Tyr Gly Lys
1               5


<210>  1335
<211>  10
```

<212> PRT

<213> hepatitis C virus


<400> 1335

Lys Pro Ala Pro Asn Phe Lys Thr Ala Ile
1               5                       10


<210> 1336

<211> 8

<212> PRT

<213> hepatitis C virus


<400> 1336

Lys Pro Ala Arg Leu Ile Val Phe
1               5


<210> 1337

<211> 9

<212> PRT

<213> hepatitis C virus


<400> 1337

Lys Pro Ala Arg Leu Ile Val Phe Pro
1               5


<210> 1338

<211> 9

<212> PRT

<213> hepatitis C virus


<400> 1338

Lys Pro Leu Leu Arg Glu Glu Val Thr
1               5


<210> 1339

<211> 10

<212> PRT

<213> hepatitis C virus

<400> 1339

Lys Pro Leu Leu Arg Glu Glu Val Thr Phe
1                   5                   10

<210> 1340

<211> 9

<212> PRT

<213> hepatitis C virus

<400> 1340

Lys Pro Gln Arg Lys Thr Lys Arg Asn
1                   5

<210> 1341

<211> 10

<212> PRT

<213> hepatitis C virus

<400> 1341

Lys Pro Gln Arg Lys Thr Lys Arg Asn Thr
1                   5                   10

<210> 1342

<211> 9

<212> PRT

<213> hepatitis C virus

<400> 1342

Lys Pro Thr Leu His Gly Pro Thr Pro
1                   5

<210> 1343

<211> 10

<212> PRT

<213> hepatitis C virus

<400> 1343

Lys Pro Thr Leu His Gly Pro Thr Pro Leu
1                   5                   10

<210> 1344

<211> 10

<212> PRT

<213> hepatitis C virus


<400> 1344

Lys Pro Thr Leu Gln Gly Pro Thr Pro Leu
1               5                   10


<210> 1345

<211> 10

<212> PRT

<213> hepatitis C virus


<400> 1345

Lys Pro Thr Leu Val Gly Pro Thr Pro Leu
1               5                   10


<210> 1346

<211> 10

<212> PRT

<213> hepatitis C virus


<400> 1346

Lys Gln Ala Gly Asp Asn Phe Pro Tyr Leu
1               5                   10


<210> 1347

<211> 9

<212> PRT

<213> hepatitis C virus


<400> 1347

Lys Gln Ser Gly Glu Asn Phe Pro Tyr
1               5


<210> 1348

<211> 9

<212> PRT

<213> hepatitis C virus

<400> 1348

Lys Ser Arg Lys Phe Pro Pro Ala Leu
1               5

<210> 1349

<211> 10

<212> PRT

<213> hepatitis C virus

<400> 1349

Lys Val Ala Gly Gly His Tyr Val Gln Met
1               5                   10

<210> 1350

<211> 10

<212> PRT

<213> hepatitis C virus

<400> 1350

Lys Val Leu Val Asp Ile Leu Ala Gly Tyr
1               5                   10

<210> 1351

<211> 9

<212> PRT

<213> hepatitis C virus

<400> 1351

Lys Val Arg Met Tyr Val Gly Gly Val
1               5

<210> 1352

<211> 10

<212> PRT

<213> hepatitis C virus

<400> 1352

Lys Val Thr Phe Asp Arg Leu Gln Val Leu
1                   5                   10

<210>  1353

<211>  9

<212>  PRT

<213>  hepatitis C virus


<400>  1353

Lys Trp Glu Trp Val Val Leu Leu Phe
1                   5

<210>  1354

<211>  9

<212>  PRT

<213>  hepatitis C virus


<400>  1354

Lys Trp Glu Tyr Val Val Leu Leu Phe
1                   5

<210>  1355

<211>  9

<212>  PRT

<213>  hepatitis C virus


<400>  1355

Lys Tyr Pro Pro Ala Leu Pro Ile Trp
1                   5

<210>  1356

<211>  10

<212>  PRT

<213>  hepatitis C virus


<400>  1356

Leu Ala Ala Lys Leu Ser Ala Leu Gly Leu
1                   5                   10

<210>  1357

<211> 9

<212> PRT

<213> hepatitis C virus


<400> 1357

Leu Ala Ala Leu Ala Ala Tyr Cys Leu
1               5


<210> 1358

<211> 10

<212> PRT

<213> hepatitis C virus


<400> 1358

Leu Ala Asp Ala Arg Val Cys Ala Cys Leu
1               5                   10


<210> 1359

<211> 11

<212> PRT

<213> hepatitis C virus


<400> 1359

Leu Ala Asp Gly Gly Cys Ser Gly Gly Ala Tyr
1               5                   10


<210> 1360

<211> 9

<212> PRT

<213> hepatitis C virus


<400> 1360

Leu Ala Gly Gly Val Leu Ala Ala Val
1               5


<210> 1361

<211> 10

<212> PRT

<213> hepatitis C virus

```
<400> 1361

Leu Ala Ile Leu Gly Pro Leu Met Val Leu
1               5                   10


<210> 1362

<211> 10

<212> PRT

<213> hepatitis C virus


<400> 1362

Leu Ala Gln Glu Gln Leu Glu Lys Ala Leu
1               5                   10


<210> 1363

<211> 9

<212> PRT

<213> hepatitis C virus


<400> 1363

Leu Ala Arg Gly Ser Pro Pro Ser Leu
1               5


<210> 1364

<211> 10

<212> PRT

<213> hepatitis C virus


<400> 1364

Leu Ala Arg Gly Ser Pro Pro Ser Leu Ala
1               5                   10


<210> 1365

<211> 9

<212> PRT

<213> hepatitis C virus


<400> 1365

Leu Ala Ser Ser Ser Ala Ser Gln Leu
1               5
```

<210> 1366

<211> 9

<212> PRT

<213> hepatitis C virus


<400> 1366

Leu Cys Glu Cys Tyr Asp Ala Gly Cys
1               5


<210> 1367

<211> 9

<212> PRT

<213> hepatitis C virus


<400> 1367

Leu Asp Ala Phe Ser Leu His Thr Tyr
1               5


<210> 1368

<211> 9

<212> PRT

<213> hepatitis C virus


<400> 1368

Leu Glu Asp Arg Asp Arg Ser Glu Leu
1               5


<210> 1369

<211> 9

<212> PRT

<213> hepatitis C virus


<400> 1369

Leu Glu Phe Trp Glu Gly Val Phe Thr
1               5


<210> 1370

<211> 10

```
<212>  PRT

<213>  hepatitis C virus


<400>  1370

Leu Glu Phe Trp Glu Gly Val Phe Thr Gly
1               5                   10


<210>  1371

<211>  10

<212>  PRT

<213>  hepatitis C virus


<400>  1371

Leu Glu Phe Trp Glu Ser Val Phe Thr Gly
1               5                   10


<210>  1372

<211>  9

<212>  PRT

<213>  hepatitis C virus


<400>  1372

Leu Glu Leu Ile Thr Ser Cys Ser Ser
1               5


<210>  1373

<211>  9

<212>  PRT

<213>  hepatitis C virus


<400>  1373

Leu Glu Asn Leu Val Ile Leu Asn Ala
1               5


<210>  1374

<211>  10

<212>  PRT

<213>  hepatitis C virus
```

<400> 1374

Leu Glu Gln Phe Trp Ala Lys His Met Trp
1               5                   10


<210> 1375

<211> 9

<212> PRT

<213> hepatitis C virus


<400> 1375

Leu Glu Thr Thr Met Arg Ser Pro Val
1               5


<210> 1376

<211> 10

<212> PRT

<213> hepatitis C virus


<400> 1376

Leu Glu Val Phe Trp Ala Lys His Met Trp
1               5                   10


<210> 1377

<211> 9

<212> PRT

<213> hepatitis C virus


<400> 1377

Leu Glu Val Val Thr Ser Thr Trp Val
1               5


<210> 1378

<211> 10

<212> PRT

<213> hepatitis C virus


<400> 1378

Leu Glu Val Val Thr Ser Thr Trp Val Leu
1               5                   10

<210> 1379

<211> 9

<212> PRT

<213> hepatitis C virus


<400> 1379

Leu Phe Asn Trp Ala Val Lys Thr Lys
1               5


<210> 1380

<211> 9

<212> PRT

<213> hepatitis C virus


<400> 1380

Leu Phe Asn Trp Ala Val Arg Thr Lys
1               5


<210> 1381

<211> 10

<212> PRT

<213> hepatitis C virus


<400> 1381

Leu Phe Asn Trp Ala Val Arg Thr Lys Leu
1               5                   10


<210> 1382

<211> 9

<212> PRT

<213> hepatitis C virus


<400> 1382

Leu Gly Lys Val Leu Val Asp Ile Leu
1               5


<210> 1383

<211> 10

<212> PRT

<213> hepatitis C virus

<400> 1383

Leu Gly Lys Val Leu Val Asp Ile Leu Ala
1               5                   10

<210> 1384

<211> 9

<212> PRT

<213> hepatitis C virus

<400> 1384

Leu Ile Ala Val Leu Gly Pro Leu Tyr
1               5

<210> 1385

<211> 9

<212> PRT

<213> hepatitis C virus

<400> 1385

Leu Ile Phe Cys His Ser Arg Lys Lys
1               5

<210> 1386

<211> 9

<212> PRT

<213> hepatitis C virus

<400> 1386

Leu Ile Phe Asp Ile Thr Lys Leu Leu
1               5

<210> 1387

<211> 10

<212> PRT

<213> hepatitis C virus

<400> 1387

```
Leu Ile Arg Leu Lys Pro Thr Leu His Gly
1               5                   10
```

<210> 1388

<211> 10

<212> PRT

<213> hepatitis C virus


<400> 1388

```
Leu Lys Gly Ser Ser Gly Gly Pro Leu Leu
1               5                   10
```

<210> 1389

<211> 9

<212> PRT

<213> hepatitis C virus


<400> 1389

```
Leu Leu Ala Leu Leu Gly Pro Ala Tyr
1               5
```

<210> 1390

<211> 10

<212> PRT

<213> hepatitis C virus


<400> 1390

```
Leu Leu Ala Leu Leu Ser Cys Leu Thr Val
1               5                   10
```

<210> 1391

<211> 9

<212> PRT

<213> hepatitis C virus


<400> 1391

```
Leu Leu Ala Pro Ile Thr Ala Tyr Ala
1               5
```

<210> 1392

<211> 9

<212> PRT

<213> hepatitis C virus


<400> 1392

Leu Leu Ala Gln Glu Gln Leu Glu Lys
1               5


<210> 1393

<211> 9

<212> PRT

<213> hepatitis C virus


<400> 1393

Leu Leu Cys Leu Leu Leu Leu Thr Val
1               5


<210> 1394

<211> 10

<212> PRT

<213> hepatitis C virus


<400> 1394

Leu Leu Cys Pro Thr Asp Cys Phe Arg Lys
1               5                       10


<210> 1395

<211> 9

<212> PRT

<213> hepatitis C virus


<400> 1395

Leu Leu Phe Leu Leu Leu Ala Asp Ala
1               5


<210> 1396

<211> 10

<212> PRT

<213> hepatitis C virus

<400> 1396

Leu Leu Phe Asn Ile Leu Gly Gly Trp Val
1               5                   10

<210> 1397

<211> 9

<212> PRT

<213> hepatitis C virus

<400> 1397

Leu Leu Gly Cys Ile Ile Thr Ser Leu
1               5

<210> 1398

<211> 9

<212> PRT

<213> hepatitis C virus

<400> 1398

Leu Leu Ile Ala Val Leu Gly Pro Leu
1               5

<210> 1399

<211> 10

<212> PRT

<213> hepatitis C virus

<400> 1399

Leu Leu Leu Ala Asp Ala Arg Val Cys Val
1               5                   10

<210> 1400

<211> 9

<212> PRT

<213> hepatitis C virus

<400> 1400

Leu Leu Leu Ala Ile Phe Gly Pro Leu
1               5

<210> 1401

<211> 9

<212> PRT

<213> hepatitis C virus


<400> 1401

Leu Leu Leu Ala Val Leu Gly Pro Leu
1               5

<210> 1402

<211> 9

<212> PRT

<213> hepatitis C virus


<400> 1402

Leu Leu Leu Cys Leu Leu Leu Leu Thr
1               5

<210> 1403

<211> 10

<212> PRT

<213> hepatitis C virus


<400> 1403

Leu Leu Leu Cys Leu Leu Leu Leu Thr Val
1               5                   10

<210> 1404

<211> 9

<212> PRT

<213> hepatitis C virus


<400> 1404

Leu Leu Leu Leu Gly Leu Leu Leu Leu
1               5

<210> 1405

<211> 10

```
<212>  PRT

<213>  hepatitis C virus


<400>  1405

Leu Leu Leu Leu Thr Val Gly Val Gly Ile
1               5                   10

<210>  1406

<211>  9

<212>  PRT

<213>  hepatitis C virus


<400>  1406

Leu Leu Pro Arg Arg Gly Pro Arg Leu
1               5

<210>  1407

<211>  9

<212>  PRT

<213>  hepatitis C virus


<400>  1407

Leu Leu Arg Ile Pro Tyr Phe Val Arg
1               5

<210>  1408

<211>  9

<212>  PRT

<213>  hepatitis C virus


<400>  1408

Leu Leu Ser Pro Arg Pro Ile Ser Tyr
1               5

<210>  1409

<211>  10

<212>  PRT

<213>  hepatitis C virus
```

<400> 1409

Leu Leu Ser Pro Arg Pro Val Ser Tyr Leu
1               5               10

<210> 1410

<211> 10

<212> PRT

<213> hepatitis C virus


<400> 1410

Leu Leu Ser Val Glu Glu Ala Cys Lys Leu
1               5               10

<210> 1411

<211> 9

<212> PRT

<213> hepatitis C virus


<400> 1411

Leu Leu Ser Val Gly Val Gly Ile Tyr
1               5

<210> 1412

<211> 10

<212> PRT

<213> hepatitis C virus


<400> 1412

Leu Leu Ser Val Gly Val Gly Ile Tyr Leu
1               5               10

<210> 1413

<211> 10

<212> PRT

<213> hepatitis C virus


<400> 1413

Leu Leu Thr Cys Ala Val His Pro Glu Leu
1               5               10

<210> 1414

<211> 9

<212> PRT

<213> hepatitis C virus


<400> 1414

Leu Met Thr His Phe Phe Ser Ile Leu
1               5


<210> 1415

<211> 10

<212> PRT

<213> hepatitis C virus


<400> 1415

Leu Met Thr His Phe Phe Ser Ile Leu Leu
1               5                   10


<210> 1416

<211> 10

<212> PRT

<213> hepatitis C virus


<400> 1416

Leu Asn Ala Val Ala Tyr Tyr Arg Gly Leu
1               5                   10


<210> 1417

<211> 9

<212> PRT

<213> hepatitis C virus


<400> 1417

Leu Pro Ala Ile Leu Ser Pro Gly Ala
1               5


<210> 1418

<211> 10

<212> PRT

<213> hepatitis C virus

<400> 1418

Leu Pro Ala Ile Leu Ser Pro Gly Ala Leu
1               5                   10

<210> 1419

<211> 9

<212> PRT

<213> hepatitis C virus

<400> 1419

Leu Pro Ala Leu Ser Thr Gly Leu Ile
1               5

<210> 1420

<211> 9

<212> PRT

<213> hepatitis C virus

<400> 1420

Leu Pro Ala Leu Ser Thr Gly Leu Leu
1               5

<210> 1421

<211> 9

<212> PRT

<213> hepatitis C virus

<400> 1421

Leu Pro Cys Glu Pro Glu Pro Asp Val
1               5

<210> 1422

<211> 10

<212> PRT

<213> hepatitis C virus

<400> 1422

Leu Pro Cys Glu Pro Glu Pro Asp Val Ala
1               5               10

<210> 1423

<211> 10

<212> PRT

<213> hepatitis C virus


<400> 1423

Leu Pro Cys Ser Phe Ser Asp Leu Pro Ala
1               5               10

<210> 1424

<211> 10

<212> PRT

<213> hepatitis C virus


<400> 1424

Leu Pro Cys Ser Phe Thr Thr Leu Pro Ala
1               5               10

<210> 1425

<211> 8

<212> PRT

<213> hepatitis C virus


<400> 1425

Leu Pro Gly Cys Ser Phe Ser Ile
1               5

<210> 1426

<211> 10

<212> PRT

<213> hepatitis C virus


<400> 1426

Leu Pro Gly Cys Ser Phe Ser Ile Phe Leu
1               5               10

<210> 1427

```
<211>  9
<212>  PRT
<213>  hepatitis C virus


<400>  1427
Leu Pro Gly Asn Pro Ala Ile Ala Ser
1                   5


<210>  1428
<211>  10
<212>  PRT
<213>  hepatitis C virus


<400>  1428
Leu Pro Gly Asn Pro Ala Ile Ala Ser Leu
1               5                   10


<210>  1429
<211>  9
<212>  PRT
<213>  hepatitis C virus


<400>  1429
Leu Pro Gly Val Pro Phe Phe Ser Cys
1               5


<210>  1430
<211>  10
<212>  PRT
<213>  hepatitis C virus


<400>  1430
Leu Pro Ile Asn Ala Leu Ser Asn Ser Leu
1               5                   10


<210>  1431
<211>  9
<212>  PRT
<213>  hepatitis C virus
```

<400> 1431

Leu Pro Ile Trp Ala Arg Pro Asp Tyr
1               5


<210> 1432

<211> 9

<212> PRT

<213> hepatitis C virus


<400> 1432

Leu Pro Lys Ser Arg Phe Pro Pro Ala
1               5


<210> 1433

<211> 10

<212> PRT

<213> hepatitis C virus


<400> 1433

Leu Pro Lys Ser Arg Phe Pro Pro Ala Leu
1               5                   10


<210> 1434

<211> 9

<212> PRT

<213> hepatitis C virus


<400> 1434

Leu Pro Pro Arg Ala Tyr Ala Met Asp
1               5


<210> 1435

<211> 9

<212> PRT

<213> hepatitis C virus


<400> 1435

Leu Pro Pro Thr Lys Ala Pro Pro Ile
1               5

<210> 1436

<211> 9

<212> PRT

<213> hepatitis C virus


<400> 1436

Leu Pro Gln Ala Val Met Gly Ser Ser
1               5


<210> 1437

<211> 10

<212> PRT

<213> hepatitis C virus


<400> 1437

Leu Pro Gln Ala Val Met Gly Ser Ser Tyr
1               5                  10


<210> 1438

<211> 9

<212> PRT

<213> hepatitis C virus


<400> 1438

Leu Pro Gln Ile Ile Glu Arg Leu His
1               5


<210> 1439

<211> 9

<212> PRT

<213> hepatitis C virus


<400> 1439

Leu Pro Arg Leu Pro Gly Val Pro Phe
1               5


<210> 1440

<211> 10

<212> PRT

<213> hepatitis C virus

<400> 1440

Leu Pro Arg Leu Pro Gly Val Pro Phe Phe
1               5                       10

<210> 1441

<211> 8

<212> PRT

<213> hepatitis C virus

<400> 1441

Leu Pro Arg Arg Gly Pro Arg Leu
1               5

<210> 1442

<211> 8

<212> PRT

<213> hepatitis C virus

<400> 1442

Leu Pro Arg Arg Gly Pro Arg Leu
1               5

<210> 1443

<211> 9

<212> PRT

<213> hepatitis C virus

<400> 1443

Leu Pro Thr Ser Phe Gly Asn Thr Ile
1               5

<210> 1444

<211> 10

<212> PRT

<213> hepatitis C virus

<400> 1444

Leu Pro Val Cys Gln Asp His Leu Glu Phe
1               5                   10

<210> 1445

<211> 8

<212> PRT

<213> hepatitis C virus

<400> 1445

Leu Pro Tyr Ile Glu Gln Gly Met
1               5

<210> 1446

<211> 9

<212> PRT

<213> hepatitis C virus

<400> 1446

Leu Pro Tyr Ile Glu Gln Gly Met Gln
1               5

<210> 1447

<211> 10

<212> PRT

<213> hepatitis C virus

<400> 1447

Leu Pro Tyr Ile Glu Gln Gly Met Gln Leu
1               5                   10

<210> 1448

<211> 10

<212> PRT

<213> hepatitis C virus

<400> 1448

Leu Gln Gly Pro Thr Pro Leu Leu Tyr Arg
1               5                   10

```
<210>  1449
<211>  9
<212>  PRT
<213>  hepatitis C virus


<400>  1449
Leu Gln Ser Lys Leu Leu Pro Arg Leu
1               5


<210>  1450
<211>  9
<212>  PRT
<213>  hepatitis C virus


<400>  1450
Leu Gln Thr Gly Phe Leu Ala Ala Leu
1               5


<210>  1451
<211>  9
<212>  PRT
<213>  hepatitis C virus


<400>  1451
Leu Gln Thr Gly Phe Leu Ala Ser Leu
1               5


<210>  1452
<211>  9
<212>  PRT
<213>  hepatitis C virus


<400>  1452
Leu Gln Val Leu Asp Asp His Tyr Lys
1               5


<210>  1453
<211>  9
<212>  PRT
```

<213> hepatitis C virus


<400> 1453

Leu Gln Val Trp Val Pro Pro Leu Leu
1               5


<210> 1454

<211> 10

<212> PRT

<213> hepatitis C virus


<400> 1454

Leu Arg Ala Tyr Leu Asn Thr Pro Gly Leu
1               5                   10


<210> 1455

<211> 9

<212> PRT

<213> hepatitis C virus


<400> 1455

Leu Arg Lys Leu Gly Val Pro Pro Leu
1               5


<210> 1456

<211> 9

<212> PRT

<213> hepatitis C virus


<400> 1456

Leu Ser Ala Phe Ser Leu His Ser Tyr
1               5


<210> 1457

<211> 9

<212> PRT

<213> hepatitis C virus


<400> 1457

```
Leu Ser Ala Phe Thr Leu His Ser Tyr
1               5

<210>  1458

<211>  10

<212>  PRT

<213>  hepatitis C virus


<400>  1458

Leu Ser Cys Leu Thr Ile Pro Ala Ser Ala
1               5                   10

<210>  1459

<211>  10

<212>  PRT

<213>  hepatitis C virus


<400>  1459

Leu Ser Asn Thr Gly Glu Ile Pro Phe Tyr
1               5                   10

<210>  1460

<211>  9

<212>  PRT

<213>  hepatitis C virus


<400>  1460

Leu Ser Pro Arg Pro Val Ser Tyr Leu
1               5

<210>  1461

<211>  9

<212>  PRT

<213>  hepatitis C virus


<400>  1461

Leu Ser Pro Tyr Tyr Lys Val Phe Leu
1               5

<210>  1462
```

<211> 10

<212> PRT

<213> hepatitis C virus

<400> 1462

Leu Ser Arg Ala Arg Pro Arg Trp Phe Met
1               5                   10

<210> 1463

<211> 9

<212> PRT

<213> hepatitis C virus

<400> 1463

Leu Ser Val Gly Val Gly Ile Tyr Leu
1               5

<210> 1464

<211> 9

<212> PRT

<213> hepatitis C virus

<400> 1464

Leu Thr Cys Ala Val His Pro Glu Leu
1               5

<210> 1465

<211> 11

<212> PRT

<213> hepatitis C virus

<400> 1465

Leu Thr Cys Gly Phe Ala Asp Leu Met Gly Tyr
1               5                   10

<210> 1466

<211> 10

<212> PRT

<213> hepatitis C virus

<400> 1466

Leu Thr Cys Tyr Leu Lys Ala Ser Ala Ala
1               5                   10

<210> 1467

<211> 9

<212> PRT

<213> hepatitis C virus

<400> 1467

Leu Thr Asp Pro Ser His Ile Thr Ala
1               5

<210> 1468

<211> 10

<212> PRT

<213> hepatitis C virus

<400> 1468

Leu Thr Asp Pro Ser His Ile Thr Ala Glu
1               5                   10

<210> 1469

<211> 10

<212> PRT

<213> hepatitis C virus

<400> 1469

Leu Thr His Pro Ile Thr Lys Tyr Ile Met
1               5                   10

<210> 1470

<211> 10

<212> PRT

<213> hepatitis C virus

<400> 1470

Leu Thr Ile Thr Gln Leu Leu Lys Arg Leu
1               5                   10

<210> 1471

<211> 10

<212> PRT

<213> hepatitis C virus


<400> 1471

Leu Thr Pro Ala Glu Thr Ser Val Arg Leu
1               5               10


<210> 1472

<211> 10

<212> PRT

<213> hepatitis C virus


<400> 1472

Leu Thr Pro Ile Pro Ala Ala Ser Gln Leu
1               5               10


<210> 1473

<211> 9

<212> PRT

<213> hepatitis C virus


<400> 1473

Leu Thr Pro Arg Cys Leu Ile Asp Tyr
1               5


<210> 1474

<211> 9

<212> PRT

<213> hepatitis C virus


<400> 1474

Leu Thr Pro Arg Cys Leu Val Asp Tyr
1               5


<210> 1475

<211> 9

<212> PRT

<213> hepatitis C virus


<400> 1475

Leu Thr Arg Asp Pro Thr Thr Pro Leu
1               5


<210> 1476

<211> 9

<212> PRT

<213> hepatitis C virus


<400> 1476

Leu Thr Arg Val Pro Tyr Phe Val Arg
1               5


<210> 1477

<211> 9

<212> PRT

<213> hepatitis C virus


<400> 1477

Leu Thr Val Gly Val Gly Ile Phe Leu
1               5


<210> 1478

<211> 10

<212> PRT

<213> hepatitis C virus


<400> 1478

Leu Val Asp Ile Leu Ala Gly Tyr Gly Ala
1               5                   10


<210> 1479

<211> 9

<212> PRT

<213> hepatitis C virus

<400> 1479

Leu Val Gly Gly Val Leu Ala Ala Leu
1               5

<210> 1480

<211> 10

<212> PRT

<213> hepatitis C virus


<400> 1480

Leu Val Asn Gly Asp Asp Leu Val Val Ile
1               5                   10

<210> 1481

<211> 9

<212> PRT

<213> hepatitis C virus


<400> 1481

Leu Val Asn Leu Leu Pro Ala Ile Leu
1               5

<210> 1482

<211> 9

<212> PRT

<213> hepatitis C virus


<400> 1482

Leu Val Pro Gly Ala Ala Tyr Ala Leu
1               5

<210> 1483

<211> 11

<212> PRT

<213> hepatitis C virus


<400> 1483

Leu Trp Ala Arg Met Ile Leu Met Thr His Phe
1               5                   10

<210> 1484

<211> 10

<212> PRT

<213> hepatitis C virus


<400> 1484

Leu Trp His Tyr Pro Cys Thr Val Asn Phe
1               5                   10


<210> 1485

<211> 10

<212> PRT

<213> hepatitis C virus


<400> 1485

Leu Trp Arg Gln Glu Met Gly Gly Asn Ile
1               5                   10


<210> 1486

<211> 10

<212> PRT

<213> hepatitis C virus


<400> 1486

Leu Tyr Asp Val Ile Gln Lys Leu Ser Ile
1               5                   10


<210> 1487

<211> 9

<212> PRT

<213> hepatitis C virus


<400> 1487

Leu Tyr Gly Asn Glu Gly Leu Gly Trp
1               5


<210> 1488

<211> 10

<212> PRT

EP 1 652 858 A1

<213> hepatitis C virus

<400> 1488

Leu Tyr Gly Asn Glu Gly Met Gly Trp Ala
1               5                   10

<210> 1489

<211> 9

<212> PRT

<213> hepatitis C virus

<400> 1489

Leu Tyr Pro Ser Leu Ile Phe Asp Ile
1               5

<210> 1490

<211> 10

<212> PRT

<213> hepatitis C virus

<400> 1490

Met Ala Cys Met Ser Ala Asp Leu Glu Val
1               5                   10

<210> 1491

<211> 9

<212> PRT

<213> hepatitis C virus

<400> 1491

Met Ala Phe Met Lys Leu Ala Ala Leu
1               5

<210> 1492

<211> 10

<212> PRT

<213> hepatitis C virus

<400> 1492

Met Ala Leu Tyr Asp Val Val Ser Thr Leu
1                   5                   10

<210>   1493

<211>   10

<212>   PRT

<213>   hepatitis C virus


<400>   1493

Met Glu Thr Thr Met Arg Ser Pro Val Phe
1                   5                   10

<210>   1494

<211>   10

<212>   PRT

<213>   hepatitis C virus


<400>   1494

Met Gly Phe Ser Tyr Asp Thr Arg Cys Phe
1                   5                   10

<210>   1495

<211>   9

<212>   PRT

<213>   hepatitis C virus


<400>   1495

Met Gly Ser Ala Tyr Gly Phe Gln Tyr
1                   5

<210>   1496

<211>   9

<212>   PRT

<213>   hepatitis C virus


<400>   1496

Met Gly Ser Ser Tyr Gly Phe Gln Tyr
1                   5

<210>   1497

```
<211>  9
<212>  PRT
<213>  hepatitis C virus


<400>  1497
Met Leu Leu Ile Ser Gln Ala Glu Ala
1               5


<210>  1498
<211>  9
<212>  PRT
<213>  hepatitis C virus


<400>  1498
Met Met Met Asn Trp Ser Pro Thr Ala
1               5


<210>  1499
<211>  10
<212>  PRT
<213>  hepatitis C virus


<400>  1499
Met Met Met Asn Trp Ser Pro Thr Ala Ala
1               5                   10


<210>  1500
<211>  9
<212>  PRT
<213>  hepatitis C virus


<400>  1500
Met Met Met Asn Trp Ser Pro Thr Thr
1               5


<210>  1501
<211>  10
<212>  PRT
<213>  hepatitis C virus
```

<400> 1501

Met Met Met Asn Trp Ser Pro Thr Thr Ala
1               5                   10

<210> 1502

<211> 10

<212> PRT

<213> hepatitis C virus

<400> 1502

Met Met Asn Trp Ser Pro Thr Thr Ala Leu
1               5                   10

<210> 1503

<211> 9

<212> PRT

<213> hepatitis C virus

<400> 1503

Met Asn Trp Ser Pro Thr Thr Ala Leu
1               5

<210> 1504

<211> 9

<212> PRT

<213> hepatitis C virus

<400> 1504

Met Pro Ser Thr Glu Asp Leu Val Asn
1               5

<210> 1505

<211> 10

<212> PRT

<213> hepatitis C virus

<400> 1505

Met Pro Ser Thr Glu Asp Leu Val Asn Leu
1               5                   10

<210> 1506

<211> 10

<212> PRT

<213> hepatitis C virus


<400> 1506

Met Gln Leu Ala Glu Gln Phe Lys Gln Lys
1               5                   10


<210> 1507

<211> 10

<212> PRT

<213> hepatitis C virus


<400> 1507

Met Ser Ala Thr Leu Cys Ser Ala Leu Tyr
1               5                   10


<210> 1508

<211> 9

<212> PRT

<213> hepatitis C virus


<400> 1508

Met Thr His Phe Phe Ser Ile Leu Leu
1               5


<210> 1509

<211> 10

<212> PRT

<213> hepatitis C virus


<400> 1509

Met Val Ala Gly Ala His Trp Gly Val Leu
1               5                   10


<210> 1510

<211> 9

<212> PRT

<213> hepatitis C virus


<400> 1510

Met Val Gly Asn Trp Ala Lys Val Leu
1                   5


<210> 1511

<211> 10

<212> PRT

<213> hepatitis C virus


<400> 1511

Met Tyr Ala Pro Thr Leu Trp Ala Arg Met
1                   5                   10


<210> 1512

<211> 9

<212> PRT

<213> hepatitis C virus


<400> 1512

Met Tyr Thr Asn Val Asp Gln Asp Leu
1                   5


<210> 1513

<211> 10

<212> PRT

<213> hepatitis C virus


<400> 1513

Met Tyr Val Gly Gly Val Glu His Arg Leu
1                   5                   10


<210> 1514

<211> 10

<212> PRT

<213> hepatitis C virus

<400> 1514

Asn Ala Trp Lys Ser Lys Lys Cys Pro Met
1               5               10

<210> 1515

<211> 10

<212> PRT

<213> hepatitis C virus

<400> 1515

Asn Asp Ile Arg Val Glu Glu Ser Ile Tyr
1               5               10

<210> 1516

<211> 10

<212> PRT

<213> hepatitis C virus

<400> 1516

Asn Glu Gly Leu Gly Trp Ala Gly Trp Leu
1               5               10

<210> 1517

<211> 10

<212> PRT

<213> hepatitis C virus

<400> 1517

Asn Glu Gly Met Gly Trp Ala Gly Trp Leu
1               5               10

<210> 1518

<211> 9

<212> PRT

<213> hepatitis C virus

<400> 1518

Asn Glu Ile Thr Leu Thr His Pro Val
1               5

<210> 1519

<211> 10

<212> PRT

<213> hepatitis C virus


<400> 1519

Asn Glu Val Thr Leu Thr His Pro Ile Thr
1               5                   10


<210> 1520

<211> 9

<212> PRT

<213> hepatitis C virus


<400> 1520

Asn Glu Val Val Leu Thr His Pro Ile
1               5


<210> 1521

<211> 9

<212> PRT

<213> hepatitis C virus


<400> 1521

Asn Phe Ile Ser Gly Ile Gln Tyr Leu
1               5


<210> 1522

<211> 10

<212> PRT

<213> hepatitis C virus


<400> 1522

Asn Phe Pro Tyr Leu Val Ala Tyr Gln Ala
1               5                   10


<210> 1523

<211> 9

<212> PRT

<213> hepatitis C virus

<400> 1523

Asn Ile Val Asp Val Gln Tyr Leu Tyr
1               5

<210> 1524

<211> 10

<212> PRT

<213> hepatitis C virus

<400> 1524

Asn Leu Phe Met Gly Gly Asp Val Thr Arg
1               5                   10

<210> 1525

<211> 9

<212> PRT

<213> hepatitis C virus

<400> 1525

Asn Met Trp His Gly Thr Phe Pro Ile
1               5

<210> 1526

<211> 10

<212> PRT

<213> hepatitis C virus

<400> 1526

Asn Asn Ser Ser Arg Cys Trp Val Ala Leu
1               5                   10

<210> 1527

<211> 9

<212> PRT

<213> hepatitis C virus

<400> 1527

```
Asn Pro Ala Ile Ala Ser Leu Met Ala
1               5

<210>  1528

<211>  10

<212>  PRT

<213>  hepatitis C virus


<400>  1528

Asn Pro Ala Ile Ala Ser Leu Met Ala Phe
1               5                   10

<210>  1529

<211>  10

<212>  PRT

<213>  hepatitis C virus


<400>  1529

Asn Pro Ala Val Ala Ser Leu Met Ala Phe
1               5                   10

<210>  1530

<211>  10

<212>  PRT

<213>  hepatitis C virus


<400>  1530

Asn Pro Ala Val Ala Ser Met Met Ala Phe
1               5                   10

<210>  1531

<211>  10

<212>  PRT

<213>  hepatitis C virus


<400>  1531

Asn Pro Lys Pro Gln Arg Lys Thr Lys Arg
1               5                   10

<210>  1532
```

<211> 10

<212> PRT

<213> hepatitis C virus


<400> 1532

Asn Pro Ser Val Ala Ala Thr Leu Gly Phe
1                   5                   10


<210> 1533

<211> 9

<212> PRT

<213> hepatitis C virus


<400> 1533

Asn Ser Ser Arg Cys Trp Val Ala Leu
1                   5


<210> 1534

<211> 10

<212> PRT

<213> hepatitis C virus


<400> 1534

Asn Ser Trp Leu Gly Asn Ile Ile Met Tyr
1                   5                   10


<210> 1535

<211> 10

<212> PRT

<213> hepatitis C virus


<400> 1535

Asn Thr Gly Glu Ile Pro Phe Tyr Gly Lys
1                   5                   10


<210> 1536

<211> 9

<212> PRT

<213> hepatitis C virus

<400> 1536

Asn Thr Trp His Gly Thr Phe Pro Ile
1                5


<210> 1537

<211> 9

<212> PRT

<213> hepatitis C virus


<400> 1537

Asn Thr Trp Gln Gly Thr Phe Pro Ile
1                5


<210> 1538

<211> 9

<212> PRT

<213> hepatitis C virus


<400> 1538

Asn Val Arg Gly Gly Arg Asp Ala Ile
1                5


<210> 1539

<211> 10

<212> PRT

<213> hepatitis C virus


<400> 1539

Asn Val Arg Gly Gly Arg Asp Ala Ile Ile
1                5                    10


<210> 1540

<211> 10

<212> PRT

<213> hepatitis C virus


<400> 1540

Asn Trp Ala Val Lys Thr Lys Leu Lys Leu
1                5                    10

```
<210>  1541
<211>  10
<212>  PRT
<213>  hepatitis C virus


<400>  1541

Asn Trp Ala Val Arg Thr Lys Leu Lys Leu
1               5                   10


<210>  1542
<211>  9
<212>  PRT
<213>  hepatitis C virus


<400>  1542

Asn Trp Gln Lys Leu Glu Ala Phe Trp
1               5


<210>  1543
<211>  9
<212>  PRT
<213>  hepatitis C virus


<400>  1543

Asn Tyr Thr Ile Phe Lys Ile Arg Met
1               5


<210>  1544
<211>  9
<212>  PRT
<213>  hepatitis C virus


<400>  1544

Pro Ala Ala Tyr Val Ala Gln Gly Tyr
1               5


<210>  1545
<211>  10
```

```
<212>  PRT
<213>  hepatitis C virus


<400>  1545
Pro Ala Arg Leu Ile Val Phe Pro Asp Leu
1                   5                   10


<210>  1546
<211>  10
<212>  PRT
<213>  hepatitis C virus


<400>  1546
Pro Glu Ala Arg Gln Ala Ile Arg Ser Leu
1                   5                   10


<210>  1547
<211>  10
<212>  PRT
<213>  hepatitis C virus


<400>  1547
Pro Glu Phe Phe Ser Trp Val Asp Gly Val
1                   5                   10


<210>  1548
<211>  10
<212>  PRT
<213>  hepatitis C virus


<400>  1548
Pro Glu Gly Arg Ser Trp Ala Gln Pro Gly
1                   5                   10


<210>  1549
<211>  10
<212>  PRT
<213>  hepatitis C virus
```

<400> 1549

Pro Glu Gly Arg Thr Trp Ala Gln Pro Gly
1               5                   10

<210> 1550

<211> 9

<212> PRT

<213> hepatitis C virus


<400> 1550

Pro Glu Lys Gly Gly Arg Lys Pro Ala
1               5

<210> 1551

<211> 10

<212> PRT

<213> hepatitis C virus


<400> 1551

Pro Glu Ser Asp Ala Ala Ala Arg Val Thr
1               5                   10

<210> 1552

<211> 10

<212> PRT

<213> hepatitis C virus


<400> 1552

Pro Glu Tyr Asp Leu Glu Leu Ile Thr Ser
1               5                   10

<210> 1553

<211> 9

<212> PRT

<213> hepatitis C virus


<400> 1553

Pro Phe Tyr Gly Lys Ala Ile Pro Ile
1               5

<210> 1554

<211> 9

<212> PRT

<213> hepatitis C virus


<400> 1554

Pro Phe Tyr Gly Lys Ala Ile Pro Leu
1               5

<210> 1555

<211> 10

<212> PRT

<213> hepatitis C virus


<400> 1555

Pro His Pro Asn Ile Glu Glu Val Ala Leu
1               5                   10

<210> 1556

<211> 9

<212> PRT

<213> hepatitis C virus


<400> 1556

Pro Ile Thr Tyr Ser Thr Tyr Gly Lys
1               5

<210> 1557

<211> 9

<212> PRT

<213> hepatitis C virus


<400> 1557

Pro Leu Leu Leu Leu Leu Leu Ala Leu
1               5

<210> 1558

<211> 9

<212> PRT

<213> hepatitis C virus

<400> 1558

Pro Leu Leu Tyr Arg Leu Gly Ala Val
1               5

<210> 1559

<211> 10

<212> PRT

<213> hepatitis C virus

<400> 1559

Pro Leu Arg Asp Trp Ala His Ala Gly Leu
1               5                    10

<210> 1560

<211> 9

<212> PRT

<213> hepatitis C virus

<400> 1560

Pro Leu Ser Asn Ser Leu Leu Arg Tyr
1               5

<210> 1561

<211> 9

<212> PRT

<213> hepatitis C virus

<400> 1561

Pro Met Glu Ile Lys Val Ile Thr Trp
1               5

<210> 1562

<211> 9

<212> PRT

<213> hepatitis C virus

<400> 1562

```
Pro Pro Ala Leu Pro Ile Trp Ala Arg
1               5

<210>  1563

<211>  9

<212>  PRT

<213>  hepatitis C virus


<400>  1563

Pro Pro Ala Val Pro Gln Thr Phe Gln
1               5

<210>  1564

<211>  10

<212>  PRT

<213>  hepatitis C virus


<400>  1564

Pro Pro Ala Val Pro Gln Thr Phe Gln Val
1               5                   10

<210>  1565

<211>  9

<212>  PRT

<213>  hepatitis C virus


<400>  1565

Pro Pro Gly Asp Pro Pro Gln Pro Glu
1               5

<210>  1566

<211>  10

<212>  PRT

<213>  hepatitis C virus


<400>  1566

Pro Pro Gly Asp Pro Pro Gln Pro Glu Tyr
1               5                   10

<210>  1567
```

<211> 9

<212> PRT

<213> hepatitis C virus

<400> 1567

Pro Pro Gly Ser Val Thr Val Pro His
1               5

<210> 1568

<211> 9

<212> PRT

<213> hepatitis C virus

<400> 1568

Pro Pro His Ser Ala Lys Ser Lys Phe
1               5

<210> 1569

<211> 9

<212> PRT

<213> hepatitis C virus

<400> 1569

Pro Pro His Ser Ala Arg Ser Lys Phe
1               5

<210> 1570

<211> 9

<212> PRT

<213> hepatitis C virus

<400> 1570

Pro Pro Ile Pro Pro Pro Arg Arg Lys
1               5

<210> 1571

<211> 10

<212> PRT

<213> hepatitis C virus

<400> 1571

Pro Pro Leu Arg Val Trp Arg His Arg Ala
1               5               10


<210> 1572

<211> 9

<212> PRT

<213> hepatitis C virus


<400> 1572

Pro Pro Pro Arg Arg Lys Arg Thr Val
1               5


<210> 1573

<211> 10

<212> PRT

<213> hepatitis C virus


<400> 1573

Pro Pro Pro Arg Arg Lys Arg Thr Val Val
1               5               10


<210> 1574

<211> 10

<212> PRT

<213> hepatitis C virus


<400> 1574

Pro Pro Gln Gly Asn Trp Phe Gly Cys Thr
1               5               10


<210> 1575

<211> 10

<212> PRT

<213> hepatitis C virus


<400> 1575

Pro Pro Gln Pro Glu Tyr Asp Leu Glu Leu
1               5               10

```
<210>    1576
<211>    9
<212>    PRT
<213>    hepatitis C virus


<400>    1576

Pro Pro Arg Ala Tyr Ala Met Asp Arg
1               5


<210>    1577
<211>    9
<212>    PRT
<213>    hepatitis C virus


<400>    1577

Pro Pro Arg Lys Lys Arg Thr Val Val
1               5


<210>    1578
<211>    9
<212>    PRT
<213>    hepatitis C virus


<400>    1578

Pro Pro Arg Arg Lys Arg Thr Val Val
1               5


<210>    1579
<211>    10
<212>    PRT
<213>    hepatitis C virus


<400>    1579

Pro Pro Arg Arg Lys Arg Thr Val Val Leu
1               5                   10


<210>    1580
<211>    9
```

```
<212>   PRT
<213>   hepatitis C virus


<400>   1580
Pro Pro Ser Ala Ala Ser Ala Phe Val
1               5


<210>   1581
<211>   9
<212>   PRT
<213>   hepatitis C virus


<400>   1581
Pro Pro Ser Leu Ala Ser Ser Ser Ala
1               5


<210>   1582
<211>   9
<212>   PRT
<213>   hepatitis C virus


<400>   1582
Pro Pro Val Val His Gly Cys Pro Leu
1               5


<210>   1583
<211>   9
<212>   PRT
<213>   hepatitis C virus


<400>   1583
Pro Arg Arg Lys Arg Thr Val Val Leu
1               5


<210>   1584
<211>   10
<212>   PRT
<213>   hepatitis C virus
```

```
<400>    1584

Pro Ser Gly His Ala Val Gly Ile Phe Arg
1               5                   10


<210>    1585

<211>    10

<212>    PRT

<213>    hepatitis C virus


<400>    1585

Pro Ser Trp Asp Gln Met Trp Lys Cys Leu
1               5                   10


<210>    1586

<211>    10

<212>    PRT

<213>    hepatitis C virus


<400>    1586

Pro Thr Asp Pro Arg Arg Arg Ser Arg Asn
1               5                   10


<210>    1587

<211>    11

<212>    PRT

<213>    hepatitis C virus


<400>    1587

Pro Thr Leu His Gly Pro Thr Pro Leu Leu Tyr
1               5                   10


<210>    1588

<211>    10

<212>    PRT

<213>    hepatitis C virus


<400>    1588

Pro Val Glu Ser Met Glu Thr Thr Met Arg
1               5                   10
```

<210> 1589

<211> 10

<212> PRT

<213> hepatitis C virus


<400> 1589

Pro Val Ser Ala Arg Arg Gly Arg Glu Ile
1               5                   10


<210> 1590

<211> 10

<212> PRT

<213> hepatitis C virus


<400> 1590

Pro Trp Pro Leu Tyr Gly Asn Glu Gly Met
1               5                   10


<210> 1591

<211> 10

<212> PRT

<213> hepatitis C virus


<400> 1591

Pro Tyr Phe Val Arg Ala His Ala Leu Leu
1               5                   10


<210> 1592

<211> 9

<212> PRT

<213> hepatitis C virus


<400> 1592

Pro Tyr Phe Val Arg Ala His Val Leu
1               5


<210> 1593

<211> 9

<212> PRT

<213> hepatitis C virus

<400> 1593

Pro Tyr Ile Glu Gln Ala Gln Ala Ile
1               5

<210> 1594

<211> 10

<212> PRT

<213> hepatitis C virus

<400> 1594

Pro Tyr Leu Val Ala Tyr Gln Ala Thr Val
1               5                   10

<210> 1595

<211> 10

<212> PRT

<213> hepatitis C virus

<400> 1595

Gln Ala Glu Thr Ala Gly Ala Arg Leu Val
1               5                   10

<210> 1596

<211> 10

<212> PRT

<213> hepatitis C virus

<400> 1596

Gln Ala Gly Asp Asn Phe Pro Tyr Leu Val
1               5                   10

<210> 1597

<211> 10

<212> PRT

<213> hepatitis C virus

<400> 1597

```
Gln Ala Ile Arg Ser Leu Thr Glu Arg Leu
1               5                   10
```

<210> 1598

<211> 9

<212> PRT

<213> hepatitis C virus


<400> 1598

```
Gln Ala Pro Pro Gly Ala Arg Ser Leu
1               5
```

<210> 1599

<211> 10

<212> PRT

<213> hepatitis C virus


<400> 1599

```
Gln Ala Val Met Gly Ser Ser Tyr Gly Phe
1               5                   10
```

<210> 1600

<211> 10

<212> PRT

<213> hepatitis C virus


<400> 1600

```
Gln Asp His Leu Glu Phe Trp Glu Ser Val
1               5                   10
```

<210> 1601

<211> 10

<212> PRT

<213> hepatitis C virus


<400> 1601

```
Gln Glu Asp Ala Ala Ser Leu Arg Val Phe
1               5                   10
```

<210> 1602

<211> 9

<212> PRT

<213> hepatitis C virus


<400> 1602

Gln Phe Lys Gln Lys Ala Leu Gly Leu
1               5


<210> 1603

<211> 10

<212> PRT

<213> hepatitis C virus


<400> 1603

Gln Phe Lys Gln Lys Ala Leu Gly Leu Leu
1           5                       10


<210> 1604

<211> 10

<212> PRT

<213> hepatitis C virus


<400> 1604

Gln Phe Trp Ala Lys His Met Trp Asn Phe
1           5                       10


<210> 1605

<211> 10

<212> PRT

<213> hepatitis C virus


<400> 1605

Gln Ile His Arg Phe Ala Pro Thr Pro Lys
1           5                       10


<210> 1606

<211> 10

<212> PRT

<213> hepatitis C virus

<400> 1606

Gln Ile Leu Pro Cys Ser Phe Thr Thr Leu
1               5                   10


<210> 1607

<211> 9

<212> PRT

<213> hepatitis C virus


<400> 1607

Gln Lys Lys Val Thr Phe Asp Arg Leu
1               5


<210> 1608

<211> 9

<212> PRT

<213> hepatitis C virus


<400> 1608

Gln Leu Ala Glu Gln Phe Lys Gln Lys
1               5


<210> 1609

<211> 9

<212> PRT

<213> hepatitis C virus


<400> 1609

Gln Leu Phe Thr Phe Ser Pro Arg Arg
1               5


<210> 1610

<211> 10

<212> PRT

<213> hepatitis C virus


<400> 1610

Gln Met Trp Lys Cys Leu Ile Arg Leu Lys
1               5                   10

EP 1 652 858 A1

<210> 1611
<211> 10
<212> PRT
<213> hepatitis C virus

<400> 1611
Gln Met Tyr Thr Asn Val Asp Gln Asp Leu
1               5               10

<210> 1612
<211> 10
<212> PRT
<213> hepatitis C virus

<400> 1612
Gln Pro Glu Lys Gly Gly Arg Lys Pro Ala
1               5               10

<210> 1613
<211> 9
<212> PRT
<213> hepatitis C virus

<400> 1613
Gln Pro Glu Tyr Asp Leu Glu Leu Ile
1               5

<210> 1614
<211> 10
<212> PRT
<213> hepatitis C virus

<400> 1614
Gln Pro Glu Tyr Asp Leu Glu Leu Ile Thr
1               5               10

<210> 1615
<211> 8

550

```
<212>  PRT
<213>  hepatitis C virus


<400>  1615
Gln Pro Arg Gly Arg Arg Gln Pro
1               5


<210>  1616
<211>  10
<212>  PRT
<213>  hepatitis C virus


<400>  1616
Gln Pro Arg Gly Arg Arg Gln Pro Ile Pro
1               5                   10


<210>  1617
<211>  9
<212>  PRT
<213>  hepatitis C virus


<400>  1617
Gln Pro Arg Arg Arg Arg Gln Pro Ile
1               5


<210>  1618
<211>  10
<212>  PRT
<213>  hepatitis C virus


<400>  1618
Gln Arg Lys Thr Lys Arg Asn Thr Asn Arg
1               5                   10


<210>  1619
<211>  10
<212>  PRT
<213>  hepatitis C virus
```

<400> 1619

Gln Arg Arg Gly Arg Thr Gly Arg Gly Arg
1               5                   10

<210> 1620

<211> 10

<212> PRT

<213> hepatitis C virus


<400> 1620

Gln Thr Gly Phe Leu Ala Ser Leu Phe Tyr
1               5                   10

<210> 1621

<211> 9

<212> PRT

<213> hepatitis C virus


<400> 1621

Gln Thr Arg Gly Leu Leu Gly Cys Ile
1               5

<210> 1622

<211> 10

<212> PRT

<213> hepatitis C virus


<400> 1622

Gln Thr Arg Gly Leu Leu Gly Cys Ile Ile
1               5                   10

<210> 1623

<211> 9

<212> PRT

<213> hepatitis C virus


<400> 1623

Gln Trp Met Asn Arg Leu Ile Ala Phe
1               5

<210> 1624

<211> 10

<212> PRT

<213> hepatitis C virus

<400> 1624

Gln Trp Met Asn Arg Leu Ile Ala Phe Ala
1               5                   10

<210> 1625

<211> 9

<212> PRT

<213> hepatitis C virus

<400> 1625

Gln Tyr Leu Ala Gly Leu Ser Thr Leu
1               5

<210> 1626

<211> 10

<212> PRT

<213> hepatitis C virus

<400> 1626

Gln Tyr Ser Pro Gly Gln Arg Val Glu Phe
1               5                   10

<210> 1627

<211> 9

<212> PRT

<213> hepatitis C virus

<400> 1627

Arg Ala Ala Thr Cys Gly Lys Tyr Leu
1               5

<210> 1628

<211> 9

<212> PRT

<213> hepatitis C virus

<400> 1628
Arg Ala Leu Ala His Gly Val Arg Val
1               5

<210> 1629
<211> 10
<212> PRT
<213> hepatitis C virus

<400> 1629
Arg Ala Leu Ala His Gly Val Arg Val Leu
1               5                   10

<210> 1630
<211> 10
<212> PRT
<213> hepatitis C virus

<400> 1630
Arg Ala Gln Gly Leu Ile Arg Ala Cys Met
1               5                   10

<210> 1631
<211> 9
<212> PRT
<213> hepatitis C virus

<400> 1631
Arg Ala Arg Pro Arg Trp Phe Met Leu
1               5

<210> 1632
<211> 9
<212> PRT
<213> hepatitis C virus

<400> 1632

```
Arg Ala Arg Ser Val Arg Ala Lys Leu
1               5
```

<210> 1633

<211> 10

<212> PRT

<213> hepatitis C virus

<400> 1633

```
Arg Ala Arg Ser Val Arg Ala Lys Leu Leu
1               5                   10
```

<210> 1634

<211> 10

<212> PRT

<213> hepatitis C virus

<400> 1634

```
Arg Ala Trp Ala Gln Pro Gly Tyr Pro Trp
1               5                   10
```

<210> 1635

<211> 10

<212> PRT

<213> hepatitis C virus

<400> 1635

```
Arg Cys Leu Val Asp Tyr Pro Tyr Arg Leu
1               5                   10
```

<210> 1636

<211> 9

<212> PRT

<213> hepatitis C virus

<400> 1636

```
Arg Asp Arg Ser Glu Leu Ser Pro Leu
1               5
```

<210> 1637

```
<211>  10
<212>  PRT
<213>  hepatitis C virus


<400>  1637
Arg Asp Arg Ser Glu Leu Ser Pro Leu Leu
1               5                   10


<210>  1638
<211>  10
<212>  PRT
<213>  hepatitis C virus


<400>  1638
Arg Glu Ile Leu Leu Gly Pro Ala Asp Gly
1               5                   10

<210>  1639
<211>  9
<212>  PRT
<213>  hepatitis C virus


<400>  1639
Arg Glu Ile Ser Val Pro Ala Glu Ile
1               5


<210>  1640
<211>  10
<212>  PRT
<213>  hepatitis C virus


<400>  1640
Arg Glu Ile Ser Val Pro Ala Glu Ile Leu
1               5                   10


<210>  1641
<211>  9
<212>  PRT
<213>  hepatitis C virus
```

<400> 1641

Arg Glu Met Ala Ala Ser Cys Gly Gly
1               5

<210> 1642

<211> 9

<212> PRT

<213> hepatitis C virus

<400> 1642

Arg Glu Pro Ser Ile Pro Ser Glu Tyr
1               5

<210> 1643

<211> 10

<212> PRT

<213> hepatitis C virus

<400> 1643

Arg Glu Pro Ser Ile Pro Ser Glu Tyr Leu
1               5                   10

<210> 1644

<211> 9

<212> PRT

<213> hepatitis C virus

<400> 1644

Arg Glu Val Ser Val Ala Ala Glu Ile
1               5

<210> 1645

<211> 10

<212> PRT

<213> hepatitis C virus

<400> 1645

Arg Glu Val Ser Val Ala Ala Glu Ile Leu
1               5                   10

<210> 1646

<211> 9

<212> PRT

<213> hepatitis C virus


<400> 1646

Arg Glu Val Ser Val Pro Ala Glu Ile
1               5


<210> 1647

<211> 10

<212> PRT

<213> hepatitis C virus


<400> 1647

Arg Glu Val Ser Val Pro Ala Glu Ile Leu
1               5                   10


<210> 1648

<211> 9

<212> PRT

<213> hepatitis C virus


<400> 1648

Arg Gly Asp Ser Arg Gly Ser Leu Leu
1               5


<210> 1649

<211> 10

<212> PRT

<213> hepatitis C virus


<400> 1649

Arg Gly Gly Arg Asp Ala Ile Ile Leu Leu
1               5                   10


<210> 1650

<211> 10

<212> PRT

<213> hepatitis C virus

<400> 1650

Arg Gly Arg Arg Gly Ile Tyr Arg Phe Val
1               5                   10

<210> 1651

<211> 10

<212> PRT

<213> hepatitis C virus

<400> 1651

Arg Gly Arg Arg Gln Pro Ile Pro Lys Ala
1               5                   10

<210> 1652

<211> 10

<212> PRT

<213> hepatitis C virus

<400> 1652

Arg Gly Arg Thr Gly Arg Gly Arg Arg Gly
1               5                   10

<210> 1653

<211> 10

<212> PRT

<213> hepatitis C virus

<400> 1653

Arg Gly Val Ala Lys Ala Val Asp Phe Ile
1               5                   10

<210> 1654

<211> 9

<212> PRT

<213> hepatitis C virus

<400> 1654

Arg Ile Ala Glu Met Leu Lys Ser Lys
1               5

<210> 1655

<211> 10

<212> PRT

<213> hepatitis C virus

<400> 1655

Arg Lys Ser Arg Lys Phe Pro Pro Ala Leu
1               5               10

<210> 1656

<211> 9

<212> PRT

<213> hepatitis C virus

<400> 1656

Arg Leu Gly Ala Val Gln Asn Glu Val
1               5

<210> 1657

<211> 10

<212> PRT

<213> hepatitis C virus

<400> 1657

Arg Leu His Gly Leu Asp Ala Phe Ser Leu
1               5               10

<210> 1658

<211> 10

<212> PRT

<213> hepatitis C virus

<400> 1658

Arg Leu His Gly Leu Glu Ala Phe Ser Leu
1               5               10

<210> 1659

<211> 10

<212> PRT

<213> hepatitis C virus


<400> 1659

Arg Leu His Gly Leu Ser Ala Phe Ser Leu
1               5                   10


<210> 1660

<211> 9

<212> PRT

<213> hepatitis C virus


<400> 1660

Arg Leu His Gly Leu Ser Ala Phe Thr
1               5


<210> 1661

<211> 10

<212> PRT

<213> hepatitis C virus


<400> 1661

Arg Leu Ile Val Phe Pro Asp Leu Gly Val
1               5                   10


<210> 1662

<211> 9

<212> PRT

<213> hepatitis C virus


<400> 1662

Arg Leu Leu Ala Pro Ile Thr Ala Tyr
1               5


<210> 1663

<211> 10

<212> PRT

EP 1 652 858 A1

```
<213>  hepatitis C virus


<400>  1663

Arg Leu Leu Asp Leu Ser Ser Trp Phe Thr
1               5                   10


<210>  1664

<211>  10

<212>  PRT

<213>  hepatitis C virus


<400>  1664

Arg Leu Leu Leu Leu Gly Leu Leu Leu Leu
1               5                   10


<210>  1665

<211>  10

<212>  PRT

<213>  hepatitis C virus


<400>  1665

Arg Leu Gln Val Leu Asp Asp His Tyr Lys
1               5                   10


<210>  1666

<211>  10

<212>  PRT

<213>  hepatitis C virus


<400>  1666

Arg Leu Val Pro Gly Ala Ala Tyr Ala Leu
1               5                   10


<210>  1667

<211>  9

<212>  PRT

<213>  hepatitis C virus


<400>  1667
```

562

Arg Leu Trp His Tyr Pro Cys Thr Ile
1               5

<210>  1668

<211>  9

<212>  PRT

<213>  hepatitis C virus


<400>  1668

Arg Leu Trp His Tyr Pro Cys Thr Leu
1               5

<210>  1669

<211>  9

<212>  PRT

<213>  hepatitis C virus


<400>  1669

Arg Leu Trp His Tyr Pro Cys Thr Val
1               5

<210>  1670

<211>  10

<212>  PRT

<213>  hepatitis C virus


<400>  1670

Arg Leu Tyr Ile Gly Gly Pro Leu Thr Asn
1               5                   10

<210>  1671

<211>  9

<212>  PRT

<213>  hepatitis C virus


<400>  1671

Arg Met Val Leu Met Thr His Phe Phe
1               5

<210>  1672

<211> 10

<212> PRT

<213> hepatitis C virus

<400> 1672

Arg Met Tyr Val Gly Gly Val Glu His Arg
1               5                       10

<210> 1673

<211> 10

<212> PRT

<213> hepatitis C virus

<400> 1673

Arg Asn Leu Gly Lys Val Ile Asp Thr Leu
1               5                       10

<210> 1674

<211> 10

<212> PRT

<213> hepatitis C virus

<400> 1674

Arg Pro Ala Val Ile Pro Asp Arg Glu Val
1               5                       10

<210> 1675

<211> 9

<212> PRT

<213> hepatitis C virus

<400> 1675

Arg Pro Cys Gly Ile Val Pro Ala Leu
1               5

<210> 1676

<211> 9

<212> PRT

<213> hepatitis C virus

<400> 1676

Arg Pro Cys Gly Ile Val Pro Ala Ser
1               5

<210> 1677

<211> 9

<212> PRT

<213> hepatitis C virus

<400> 1677

Arg Pro Asp Tyr Asn Pro Pro Leu Leu
1               5

<210> 1678

<211> 10

<212> PRT

<213> hepatitis C virus

<400> 1678

Arg Pro Glu Gly Arg Ala Trp Ala Gln Pro
1               5                   10

<210> 1679

<211> 9

<212> PRT

<213> hepatitis C virus

<400> 1679

Arg Pro Ile Asp Lys Phe Ala Gln Gly
1               5

<210> 1680

<211> 9

<212> PRT

<213> hepatitis C virus

<400> 1680

Arg Pro Pro Gln Gly Asn Trp Phe Gly
1               5

<210> 1681

<211> 10

<212> PRT

<213> hepatitis C virus


<400> 1681

Arg Pro Gln Asp Val Lys Phe Pro Gly Gly
1               5                   10


<210> 1682

<211> 9

<212> PRT

<213> hepatitis C virus


<400> 1682

Arg Pro Arg Leu Leu Leu Leu Gly Leu
1               5


<210> 1683

<211> 10

<212> PRT

<213> hepatitis C virus


<400> 1683

Arg Pro Arg Leu Leu Leu Leu Gly Leu Leu
1               5                   10


<210> 1684

<211> 9

<212> PRT

<213> hepatitis C virus


<400> 1684

Arg Pro Arg Trp Phe Met Leu Cys Leu
1               5


<210> 1685

<211> 10

<212> PRT

<213> hepatitis C virus


<400> 1685

Arg Pro Arg Trp Phe Met Leu Cys Leu Leu
1               5               10


<210> 1686

<211> 9

<212> PRT

<213> hepatitis C virus


<400> 1686

Arg Pro Ser Gly Met Phe Asp Ser Ser
1               5


<210> 1687

<211> 10

<212> PRT

<213> hepatitis C virus


<400> 1687

Arg Pro Ser Gly Met Phe Asp Ser Ser Val
1               5               10


<210> 1688

<211> 9

<212> PRT

<213> hepatitis C virus


<400> 1688

Arg Pro Ser Gly Met Phe Asp Ser Val
1               5


<210> 1689

<211> 10

<212> PRT

<213> hepatitis C virus

<400> 1689

Arg Pro Ser Gly Met Phe Asp Ser Val Val
1               5               10

<210> 1690

<211> 10

<212> PRT

<213> hepatitis C virus


<400> 1690

Arg Pro Ser Trp Gly Pro Thr Asp Pro Arg
1               5               10

<210> 1691

<211> 9

<212> PRT

<213> hepatitis C virus


<400> 1691

Arg Pro Tyr Cys Trp His Tyr Ala Pro
1               5

<210> 1692

<211> 10

<212> PRT

<213> hepatitis C virus


<400> 1692

Arg Gln Glu Met Gly Gly Asn Ile Thr Arg
1               5               10

<210> 1693

<211> 10

<212> PRT

<213> hepatitis C virus


<400> 1693

Arg Gln Glu Met Gly Ser Asn Ile Thr Arg
1               5               10

<210> 1694

<211> 9

<212> PRT

<213> hepatitis C virus


<400> 1694

Arg Gln Lys Lys Val Thr Phe Asp Arg
1               5


<210> 1695

<211> 10

<212> PRT

<213> hepatitis C virus


<400> 1695

Arg Gln Lys Lys Val Thr Phe Asp Arg Leu
1               5                   10


<210> 1696

<211> 10

<212> PRT

<213> hepatitis C virus


<400> 1696

Arg Arg Pro Gln Asp Val Lys Phe Pro Gly
1               5                   10


<210> 1697

<211> 10

<212> PRT

<213> hepatitis C virus


<400> 1697

Arg Arg Arg Gly Asp Ser Arg Gly Ser Leu
1               5                   10


<210> 1698

<211> 10

<212> PRT

<213> hepatitis C virus

<400> 1698

Arg Arg Arg Ser Arg Asn Leu Gly Lys Val
1               5                   10

<210> 1699

<211> 10

<212> PRT

<213> hepatitis C virus

<400> 1699

Arg Arg Ser Arg Asn Leu Gly Lys Val Ile
1               5                   10

<210> 1700

<211> 9

<212> PRT

<213> hepatitis C virus

<400> 1700

Arg Ser Glu Leu Ser Pro Leu Leu Leu
1               5

<210> 1701

<211> 10

<212> PRT

<213> hepatitis C virus

<400> 1701

Arg Ser Glu Leu Ser Pro Leu Leu Leu Ser
1               5                   10

<210> 1702

<211> 10

<212> PRT

<213> hepatitis C virus

<400> 1702

```
Arg Ser Leu Thr Glu Arg Leu Tyr Ile Gly
1               5               10
```

<210> 1703

<211> 10

<212> PRT

<213> hepatitis C virus


<400> 1703

```
Arg Thr Ala Leu Asn Cys Asn Asp Ser Leu
1               5               10
```

<210> 1704

<211> 10

<212> PRT

<213> hepatitis C virus


<400> 1704

```
Arg Thr Gly Arg Gly Arg Arg Gly Ile Tyr
1               5               10
```

<210> 1705

<211> 9

<212> PRT

<213> hepatitis C virus


<400> 1705

```
Arg Thr Lys Leu Lys Leu Thr Pro Ile
1               5
```

<210> 1706

<211> 9

<212> PRT

<213> hepatitis C virus


<400> 1706

```
Arg Thr Thr Ser Gly Phe Thr Ser Leu
1               5
```

<210> 1707

<211> 9

<212> PRT

<213> hepatitis C virus


<400> 1707

Arg Val Ala Ser Cys Leu Arg Lys Leu
1               5


<210> 1708

<211> 9

<212> PRT

<213> hepatitis C virus


<400> 1708

Arg Val Cys Ala Cys Leu Trp Met Met
1               5


<210> 1709

<211> 10

<212> PRT

<213> hepatitis C virus


<400> 1709

Arg Val Cys Ala Cys Leu Trp Met Met Leu
1               5                   10


<210> 1710

<211> 9

<212> PRT

<213> hepatitis C virus


<400> 1710

Arg Val Glu Phe Leu Val Asn Ala Trp
1               5


<210> 1711

<211> 10

<212> PRT

<213> hepatitis C virus

<400> 1711

Arg Val Glu Phe Leu Val Asn Ala Trp Lys
1               5                   10

<210> 1712

<211> 10

<212> PRT

<213> hepatitis C virus

<400> 1712

Arg Val Phe Thr Glu Ala Met Thr Arg Tyr
1               5                   10

<210> 1713

<211> 9

<212> PRT

<213> hepatitis C virus

<400> 1713

Arg Val Leu Glu Asp Gly Ile Asn Tyr
1               5

<210> 1714

<211> 9

<212> PRT

<213> hepatitis C virus

<400> 1714

Arg Val Thr Gln Ile Leu Ser Ser Leu
1               5

<210> 1715

<211> 9

<212> PRT

<213> hepatitis C virus

<400> 1715

Arg Tyr Ala Pro Pro Cys Lys Pro Leu
1               5

<210> 1716

<211> 10

<212> PRT

<213> hepatitis C virus


<400> 1716

Arg Tyr Ala Pro Pro Cys Lys Pro Leu Leu
1               5               10


<210> 1717

<211> 10

<212> PRT

<213> hepatitis C virus


<400> 1717

Ser Ala Leu Gly Leu Asn Ala Val Ala Tyr
1               5               10


<210> 1718

<211> 9

<212> PRT

<213> hepatitis C virus


<400> 1718

Ser Ala Arg Arg Gly Arg Glu Ile Leu
1               5


<210> 1719

<211> 10

<212> PRT

<213> hepatitis C virus


<400> 1719

Ser Ala Arg Arg Gly Arg Glu Ile Leu Leu
1               5               10


<210> 1720

<211> 10

<212> PRT

<213> hepatitis C virus

<400> 1720

Ser Ala Ser Gln Leu Ser Ala Pro Ser Leu
1               5               10

<210> 1721

<211> 10

<212> PRT

<213> hepatitis C virus

<400> 1721

Ser Cys Gly Gly Ala Val Phe Val Gly Leu
1               5               10

<210> 1722

<211> 9

<212> PRT

<213> hepatitis C virus

<400> 1722

Ser Cys Gly Asn Thr Leu Thr Cys Tyr
1               5

<210> 1723

<211> 10

<212> PRT

<213> hepatitis C virus

<400> 1723

Ser Glu Ala Ser Ser Ser Ala Ser Gln Leu
1               5               10

<210> 1724

<211> 9

<212> PRT

<213> hepatitis C virus

<400> 1724

Ser Glu Asp Val Val Cys Cys Ser Met
1               5

<210> 1725

<211> 9

<212> PRT

<213> hepatitis C virus


<400> 1725

Ser Glu Leu Ser Pro Leu Leu Leu Ser
1               5

<210> 1726

<211> 10

<212> PRT

<213> hepatitis C virus


<400> 1726

Ser Glu Leu Ser Pro Leu Leu Leu Ser Thr
1               5                   10

<210> 1727

<211> 10

<212> PRT

<213> hepatitis C virus


<400> 1727

Ser Glu Tyr Leu Leu Pro Lys Ser Arg Phe
1               5                   10

<210> 1728

<211> 10

<212> PRT

<213> hepatitis C virus


<400> 1728

Ser Phe Leu Val Phe Phe Cys Ala Ala Trp
1               5                   10

```
<210>   1729
<211>   10
<212>   PRT
<213>   hepatitis C virus


<400>   1729

Ser Phe Ser Ile Phe Leu Leu Ala Leu Phe
1               5                   10


<210>   1730
<211>   10
<212>   PRT
<213>   hepatitis C virus


<400>   1730

Ser Phe Ser Ile Phe Leu Leu Ala Leu Leu
1               5                   10


<210>   1731
<211>   9
<212>   PRT
<213>   hepatitis C virus


<400>   1731

Ser Gly Gly Asp Ile Tyr His Ser Leu
1               5


<210>   1732
<211>   9
<212>   PRT
<213>   hepatitis C virus


<400>   1732

Ser Gly Ile Gln Tyr Leu Ala Gly Leu
1               5


<210>   1733
<211>   10
<212>   PRT
```

<213> hepatitis C virus

<400> 1733

Ser Gly Lys Arg Val Tyr Tyr Leu Thr Arg
1               5               10

<210> 1734

<211> 10

<212> PRT

<213> hepatitis C virus

<400> 1734

Ser Gly Lys Ser Thr Lys Val Pro Ala Ala
1               5               10

<210> 1735

<211> 10

<212> PRT

<213> hepatitis C virus

<400> 1735

Ser Gly Pro Trp Leu Thr Pro Arg Cys Leu
1               5               10

<210> 1736

<211> 10

<212> PRT

<213> hepatitis C virus

<400> 1736

Ser Ile Pro Ser Glu Tyr Leu Leu Pro Lys
1               5               10

<210> 1737

<211> 9

<212> PRT

<213> hepatitis C virus

<400> 1737

Ser Ile Ser Gly Val Leu Trp Thr Val
1               5

<210>  1738

<211>  9

<212>  PRT

<213>  hepatitis C virus


<400>  1738

Ser Ile Thr Tyr Ser Thr Tyr Gly Lys
1               5

<210>  1739

<211>  10

<212>  PRT

<213>  hepatitis C virus


<400>  1739

Ser Lys Lys Cys Pro Met Gly Phe Ser Tyr
1               5               10

<210>  1740

<211>  10

<212>  PRT

<213>  hepatitis C virus


<400>  1740

Ser Leu Ala Ser Ser Ser Ala Ser Gln Leu
1               5               10

<210>  1741

<211>  10

<212>  PRT

<213>  hepatitis C virus


<400>  1741

Ser Leu Asp Pro Thr Phe Thr Ile Glu Thr
1               5               10

<210>  1742

<211> 10

<212> PRT

<213> hepatitis C virus


<400> 1742

Ser Leu Gly Leu Asn Ala Val Ala Tyr Tyr
1               5                   10


<210> 1743

<211> 10

<212> PRT

<213> hepatitis C virus


<400> 1743

Ser Leu Leu Arg His His Asn Met Val Tyr
1               5                   10


<210> 1744

<211> 9

<212> PRT

<213> hepatitis C virus


<400> 1744

Ser Leu Leu Arg Ile Pro Tyr Phe Val
1               5


<210> 1745

<211> 10

<212> PRT

<213> hepatitis C virus


<400> 1745

Ser Leu Leu Arg Ile Pro Tyr Phe Val Arg
1               5                   10


<210> 1746

<211> 9

<212> PRT

<213> hepatitis C virus

```
<400>  1746

Ser Leu Met Ala Phe Thr Ala Ser Val
1               5


<210>  1747

<211>  9

<212>  PRT

<213>  hepatitis C virus


<400>  1747

Ser Leu Thr Ile Thr Ser Leu Leu Arg
1               5


<210>  1748

<211>  9

<212>  PRT

<213>  hepatitis C virus


<400>  1748

Ser Leu Thr Val Thr Gln Leu Leu Arg
1               5


<210>  1749

<211>  9

<212>  PRT

<213>  hepatitis C virus


<400>  1749

Ser Leu Thr Val Thr Ser Leu Leu Arg
1               5


<210>  1750

<211>  10

<212>  PRT

<213>  hepatitis C virus


<400>  1750

Ser Met Glu Thr Thr Met Arg Ser Pro Val
1               5                   10
```

<210> 1751

<211> 9

<212> PRT

<213> hepatitis C virus


<400> 1751

Ser Met Met Ala Phe Ser Ala Ala Leu
1               5


<210> 1752

<211> 9

<212> PRT

<213> hepatitis C virus


<400> 1752

Ser Met Gln Gly Ala Trp Ala Lys Val
1               5


<210> 1753

<211> 9

<212> PRT

<213> hepatitis C virus


<400> 1753

Ser Met Val Gly Asn Trp Ala Lys Val
1               5


<210> 1754

<211> 10

<212> PRT

<213> hepatitis C virus


<400> 1754

Ser Met Val Gly Asn Trp Ala Lys Val Leu
1               5                    10


<210> 1755

<211> 9

<212> PRT

<213> hepatitis C virus

<400> 1755

Ser Pro Ala Pro Asn Tyr Ser Arg Ala
1               5

<210> 1756

<211> 10

<212> PRT

<213> hepatitis C virus

<400> 1756

Ser Pro Ala Pro Asn Tyr Ser Arg Ala Leu
1               5                   10

<210> 1757

<211> 9

<212> PRT

<213> hepatitis C virus

<400> 1757

Ser Pro Ala Gln Arg Val Glu Phe Leu
1               5

<210> 1758

<211> 9

<212> PRT

<213> hepatitis C virus

<400> 1758

Ser Pro Asp Ala Asp Leu Ile Glu Ala
1               5

<210> 1759

<211> 9

<212> PRT

<213> hepatitis C virus

<400> 1759

Ser Pro Gly Ala Leu Val Val Gly Val
1               5

<210> 1760

<211> 10

<212> PRT

<213> hepatitis C virus

<400> 1760

Ser Pro Gly Ala Leu Val Val Gly Val Val
1               5                   10

<210> 1761

<211> 9

<212> PRT

<213> hepatitis C virus

<400> 1761

Ser Pro Gly Glu Ile Asn Arg Val Ala
1               5

<210> 1762

<211> 10

<212> PRT

<213> hepatitis C virus

<400> 1762

Ser Pro Gly Glu Ile Asn Arg Val Ala Ser
1               5                   10

<210> 1763

<211> 9

<212> PRT

<213> hepatitis C virus

<400> 1763

Ser Pro Gly Pro Ser Gln Lys Ile Gln
1               5

```
<210>  1764
<211>  10
<212>  PRT
<213>  hepatitis C virus


<400>  1764

Ser Pro Gly Pro Ser Gln Lys Ile Gln Leu
1               5                   10


<210>  1765
<211>  10
<212>  PRT
<213>  hepatitis C virus


<400>  1765

Ser Pro Gly Gln Arg Val Glu Phe Leu Val
1               5                   10


<210>  1766
<211>  9
<212>  PRT
<213>  hepatitis C virus


<400>  1766

Ser Pro Leu Thr Thr Asn Gln Thr Met
1               5


<210>  1767
<211>  9
<212>  PRT
<213>  hepatitis C virus


<400>  1767

Ser Pro Leu Thr Thr Gln His Thr Leu
1               5


<210>  1768
<211>  10
<212>  PRT
```

<213> hepatitis C virus

<400> 1768

Ser Pro Leu Thr Thr Gln His Thr Leu Leu
1               5                   10

<210> 1769

<211> 9

<212> PRT

<213> hepatitis C virus

<400> 1769

Ser Pro Met Glu Lys Lys Val Ile Val
1               5

<210> 1770

<211> 9

<212> PRT

<213> hepatitis C virus

<400> 1770

Ser Pro Pro Ala Val Pro Gln Thr Phe
1               5

<210> 1771

<211> 10

<212> PRT

<213> hepatitis C virus

<400> 1771

Ser Pro Pro Ser Leu Ala Ser Ser Ser Ala
1               5                   10

<210> 1772

<211> 9

<212> PRT

<213> hepatitis C virus

<400> 1772

.

```
Ser Pro Arg Gly Ser Arg Pro Asn Trp
1               5

<210>   1773

<211>   10

<212>   PRT

<213>   hepatitis C virus


<400>   1773

Ser Pro Arg Gly Ser Arg Pro Ser Trp Gly
1               5                   10

<210>   1774

<211>   9

<212>   PRT

<213>   hepatitis C virus


<400>   1774

Ser Pro Arg Gly Ser Arg Pro Thr Trp
1               5

<210>   1775

<211>   9

<212>   PRT

<213>   hepatitis C virus


<400>   1775

Ser Pro Arg Pro Val Ser Tyr Leu Lys
1               5

<210>   1776

<211>   10

<212>   PRT

<213>   hepatitis C virus


<400>   1776

Ser Pro Arg Pro Val Ser Tyr Leu Lys Gly
1               5                   10

<210>   1777
```

```
<211>  9
<212>  PRT
<213>  hepatitis C virus


<400>  1777
Ser Pro Arg Arg His Glu Thr Val Gln
1                   5


<210>  1778
<211>  10
<212>  PRT
<213>  hepatitis C virus


<400>  1778
Ser Pro Arg Arg His Glu Thr Val Gln Asp
1                   5                   10


<210>  1779
<211>  9
<212>  PRT
<213>  hepatitis C virus


<400>  1779
Ser Pro Thr His Tyr Val Pro Glu Ser
1                   5


<210>  1780
<211>  9
<212>  PRT
<213>  hepatitis C virus


<400>  1780
Ser Pro Tyr Tyr Lys Val Phe Leu Ala
1                   5


<210>  1781
<211>  9
<212>  PRT
<213>  hepatitis C virus
```

<400> 1781

Ser Gln Leu Ser Ala Pro Ser Leu Lys
1               5

<210> 1782

<211> 9

<212> PRT

<213> hepatitis C virus

<400> 1782

Ser Gln Leu Ser Ala Pro Ser Leu Arg
1               5

<210> 1783

<211> 10

<212> PRT

<213> hepatitis C virus

<400> 1783

Ser Gln Arg Arg Gly Arg Thr Gly Arg Gly
1               5                   10

<210> 1784

<211> 10

<212> PRT

<213> hepatitis C virus

<400> 1784

Ser Ser Ala Leu Ala Glu Leu Ala Thr Lys
1               5                   10

<210> 1785

<211> 9

<212> PRT

<213> hepatitis C virus

<400> 1785

Ser Ser Leu Thr Ile Thr Gln Leu Leu
1               5

<210> 1786

<211> 10

<212> PRT

<213> hepatitis C virus


<400> 1786

Ser Thr Ala Leu Ala Glu Leu Ala Ala Lys
1               5               10


<210> 1787

<211> 9

<212> PRT

<213> hepatitis C virus


<400> 1787

Ser Thr Glu Asp Leu Val Asn Leu Leu
1               5


<210> 1788

<211> 10

<212> PRT

<213> hepatitis C virus


<400> 1788

Ser Thr Glu Asp Leu Val Asn Leu Leu Pro
1               5               10


<210> 1789

<211> 9

<212> PRT

<213> hepatitis C virus


<400> 1789

Ser Thr Ile Pro Lys Pro Gln Arg Lys
1               5


<210> 1790

<211> 10

<212> PRT

<213> hepatitis C virus


<400> 1790

Ser Thr Lys Val Pro Ala Ala Tyr Ala Ala
1               5                   10


<210> 1791

<211> 9

<212> PRT

<213> hepatitis C virus


<400> 1791

Ser Thr Thr Gly Glu Ile Pro Phe Tyr
1               5


<210> 1792

<211> 10

<212> PRT

<213> hepatitis C virus


<400> 1792

Ser Thr Val Ser Ser Ala Leu Ala Glu Leu
1               5                   10


<210> 1793

<211> 9

<212> PRT

<213> hepatitis C virus


<400> 1793

Ser Thr Trp Val Leu Val Gly Gly Val
1               5


<210> 1794

<211> 10

<212> PRT

<213> hepatitis C virus

<400> 1794

Ser Thr Trp Val Leu Val Gly Gly Val Leu
1               5                   10

<210> 1795

<211> 10

<212> PRT

<213> hepatitis C virus

<400> 1795

Ser Thr Tyr Gly Lys Phe Leu Ala Asp Gly
1               5                   10

<210> 1796

<211> 9

<212> PRT

<213> hepatitis C virus

<400> 1796

Ser Val Ala Gly Ala His Gly Ile Leu
1               5

<210> 1797

<211> 10

<212> PRT

<213> hepatitis C virus

<400> 1797

Ser Val Ala His Asp Ala Ser Gly Lys Arg
1               5                   10

<210> 1798

<211> 10

<212> PRT

<213> hepatitis C virus

<400> 1798

Ser Val Ala Leu Asp Pro Arg Gly Arg Arg
1               5                   10

```
<210>  1799
<211>  9
<212>  PRT
<213>  hepatitis C virus


<400>  1799
Ser Val Gly Val Gly Ile Tyr Leu Leu
1               5


<210>  1800
<211>  9
<212>  PRT
<213>  hepatitis C virus


<400>  1800
Ser Val Pro Ala Glu Ile Leu Arg Lys
1               5


<210>  1801
<211>  9
<212>  PRT
<213>  hepatitis C virus


<400>  1801
Ser Val Arg Ala Lys Leu Leu Ser Arg
1               5


<210>  1802
<211>  10
<212>  PRT
<213>  hepatitis C virus


<400>  1802
Ser Val Arg Leu Arg Ala Tyr Leu Asn Thr
1               5                   10


<210>  1803
<211>  10
<212>  PRT
```

```
<213>  hepatitis C virus


<400>  1803

Ser Val Val Ile Val Gly Arg Ile Ile Leu
1               5                   10


<210>  1804

<211>  10

<212>  PRT

<213>  hepatitis C virus


<400>  1804

Ser Trp Asp Gln Met Trp Lys Cys Leu Ile
1               5                   10


<210>  1805

<211>  10

<212>  PRT

<213>  hepatitis C virus


<400>  1805

Ser Trp Asp Val Met Trp Lys Cys Leu Ile
1               5                   10


<210>  1806

<211>  10

<212>  PRT

<213>  hepatitis C virus


<400>  1806

Ser Trp Leu Gly Asn Ile Ile Met Tyr Ala
1               5                   10


<210>  1807

<211>  9

<212>  PRT

<213>  hepatitis C virus


<400>  1807
```

Ser Trp Leu Arg Asp Val Trp Asp Trp
1               5

<210>   1808

<211>   10

<212>   PRT

<213>   hepatitis C virus


<400>   1808

Ser Tyr Asp Thr Arg Cys Phe Asp Ser Thr
1               5                       10

<210>   1809

<211>   9

<212>   PRT

<213>   hepatitis C virus


<400>   1809

Ser Tyr Ser Trp Thr Gly Ala Leu Ile
1               5

<210>   1810

<211>   9

<212>   PRT

<213>   hepatitis C virus


<400>   1810

Ser Tyr Thr Trp Thr Gly Ala Leu Ile
1               5

<210>   1811

<211>   9

<212>   PRT

<213>   hepatitis C virus


<400>   1811

Thr Ala Ala Cys Gly Asp Ile Ile Leu
1               5

<210>   1812

```
<211>  9
<212>  PRT
<213>  hepatitis C virus


<400>  1812
Thr Ala Leu Asn Cys Asn Asp Ser Leu
1               5


<210>  1813
<211>  9
<212>  PRT
<213>  hepatitis C virus


<400>  1813
Thr Ala Leu Val Val Ser Gln Leu Leu
1               5


<210>  1814
<211>  10
<212>  PRT
<213>  hepatitis C virus


<400>  1814
Thr Ala Tyr Ser Gln Gln Thr Arg Gly Leu
1               5                   10


<210>  1815
<211>  10
<212>  PRT
<213>  hepatitis C virus


<400>  1815
Thr Cys Gly Phe Ala Asp Leu Met Gly Tyr
1               5                   10


<210>  1816
<211>  9
<212>  PRT
<213>  hepatitis C virus
```

<400> 1816

Thr Cys Gly Ser Ser Asp Leu Tyr Leu
1               5


<210> 1817

<211> 10

<212> PRT

<213> hepatitis C virus


<400> 1817

Thr Asp Pro Arg Arg Arg Ser Arg Asn Leu
1               5                   10


<210> 1818

<211> 10

<212> PRT

<213> hepatitis C virus


<400> 1818

Thr Glu Asp Leu Val Asn Leu Leu Pro Ala
1               5                   10


<210> 1819

<211> 9

<212> PRT

<213> hepatitis C virus


<400> 1819

Thr Glu Pro Val Ile Phe Ser Pro Met
1               5


<210> 1820

<211> 10

<212> PRT

<213> hepatitis C virus


<400> 1820

Thr Glu Arg Leu Tyr Ile Gly Gly Pro Leu
1               5                   10

```
<210>  1821
<211>  9
<212>  PRT
<213>  hepatitis C virus


<400>  1821
Thr Glu Val Leu Ala Ser Met Leu Thr
1               5


<210>  1822
<211>  10
<212>  PRT
<213>  hepatitis C virus


<400>  1822
Thr Glu Trp Ala Ile Leu Pro Cys Ser Tyr
1               5                   10


<210>  1823
<211>  9
<212>  PRT
<213>  hepatitis C virus


<400>  1823
Thr Phe Thr Ile Glu Thr Thr Thr Leu
1               5


<210>  1824
<211>  10
<212>  PRT
<213>  hepatitis C virus


<400>  1824
Thr Phe Trp Ala Lys His Met Trp Asn Phe
1               5                   10


<210>  1825
<211>  10
```

```
<212>  PRT

<213>  hepatitis C virus


<400>  1825

Thr Gly Arg Gly Arg Arg Gly Ile Tyr Arg
1               5                   10


<210>  1826

<211>  9

<212>  PRT

<213>  hepatitis C virus


<400>  1826

Thr Ile Leu Gly Ile Gly Thr Val Leu
1               5


<210>  1827

<211>  9

<212>  PRT

<213>  hepatitis C virus


<400>  1827

Thr Ile Met Ala Lys Asn Glu Val Phe
1               5


<210>  1828

<211>  9

<212>  PRT

<213>  hepatitis C virus


<400>  1828

Thr Ile Arg Arg His Val Asp Leu Leu
1               5


<210>  1829

<211>  10

<212>  PRT

<213>  hepatitis C virus
```

<400> 1829

Thr Ile Thr Thr Gly Ala Pro Ile Thr Tyr
1               5                   10

<210> 1830

<211> 9

<212> PRT

<213> hepatitis C virus

<400> 1830

Thr Leu Phe Lys Val Arg Met Tyr Val
1               5

<210> 1831

<211> 10

<212> PRT

<213> hepatitis C virus

<400> 1831

Thr Leu Gly Phe Gly Ala Tyr Met Ser Lys
1               5                   10

<210> 1832

<211> 10

<212> PRT

<213> hepatitis C virus

<400> 1832

Thr Leu Gly Phe Gly Thr Tyr Met Ser Lys
1               5                   10

<210> 1833

<211> 10

<212> PRT

<213> hepatitis C virus

<400> 1833

Thr Leu His Gly Pro Thr Pro Leu Leu Tyr
1               5                   10

<210> 1834

<211> 10

<212> PRT

<213> hepatitis C virus


<400> 1834

Thr Leu Leu Cys Pro Thr Asp Cys Phe Arg
1               5                   10


<210> 1835

<211> 9

<212> PRT

<213> hepatitis C virus


<400> 1835

Thr Leu Pro Ala Leu Ser Thr Gly Leu
1               5


<210> 1836

<211> 10

<212> PRT

<213> hepatitis C virus


<400> 1836

Thr Leu Ser Pro Tyr Tyr Lys Val Phe Leu
1               5                   10


<210> 1837

<211> 10

<212> PRT

<213> hepatitis C virus


<400> 1837

Thr Leu Trp Ala Arg Met Ile Leu Met Thr
1               5                   10


<210> 1838

<211> 10

<212> PRT

<213> hepatitis C virus

<400> 1838

Thr Asn Arg Arg Pro Gln Asp Val Lys Phe
1               5                   10

<210> 1839

<211> 9

<212> PRT

<213> hepatitis C virus

<400> 1839

Thr Pro Cys Ala Ala Glu Glu Ser Lys
1               5

<210> 1840

<211> 10

<212> PRT

<213> hepatitis C virus

<400> 1840

Thr Pro Cys Ala Ala Glu Glu Ser Lys Leu
1               5                   10

<210> 1841

<211> 9

<212> PRT

<213> hepatitis C virus

<400> 1841

Thr Pro Cys Ser Gly Ser Trp Leu Arg
1               5

<210> 1842

<211> 9

<212> PRT

<213> hepatitis C virus

<400> 1842

Thr Pro Cys Thr Cys Gly Ser Ser Asp
1                   5

<210>   1843

<211>   10

<212>   PRT

<213>   hepatitis C virus


<400>   1843

Thr Pro Cys Thr Cys Gly Ser Ser Asp Leu
1                   5                   10

<210>   1844

<211>   9

<212>   PRT

<213>   hepatitis C virus


<400>   1844

Thr Pro Gly Cys Val Pro Cys Val Arg
1                   5

<210>   1845

<211>   10

<212>   PRT

<213>   hepatitis C virus


<400>   1845

Thr Pro Gly Glu Arg Pro Ser Gly Met Phe
1                   5                   10

<210>   1846

<211>   9

<212>   PRT

<213>   hepatitis C virus


<400>   1846

Thr Pro Gly Leu Pro Val Cys Gln Asp
1                   5

<210>   1847

603

<211>  9

<212>  PRT

<213>  hepatitis C virus


<400>  1847

Thr Pro Ile Asp Thr Thr Ile Met Ala
1               5


<210>  1848

<211>  9

<212>  PRT

<213>  hepatitis C virus


<400>  1848

Thr Pro Ile Pro Ala Ala Ser Gln Leu
1               5


<210>  1849

<211>  9

<212>  PRT

<213>  hepatitis C virus


<400>  1849

Thr Pro Leu Ala Arg Ala Ala Trp Glu
1               5


<210>  1850

<211>  10

<212>  PRT

<213>  hepatitis C virus


<400>  1850

Thr Pro Leu Ala Arg Ala Ala Trp Glu Thr
1               5                   10


<210>  1851

<211>  10

<212>  PRT

<213>  hepatitis C virus

<400> 1851

Thr Pro Leu Leu Tyr Arg Leu Gly Ala Val
1               5                   10

<210> 1852

<211> 9

<212> PRT

<213> hepatitis C virus

<400> 1852

Thr Pro Leu Arg Asp Trp Ala His Ala
1               5

<210> 1853

<211> 9

<212> PRT

<213> hepatitis C virus

<400> 1853

Thr Pro Pro Gly Ser Val Thr Val Pro
1               5

<210> 1854

<211> 10

<212> PRT

<213> hepatitis C virus

<400> 1854

Thr Pro Pro Gly Ser Val Thr Val Pro His
1               5                   10

<210> 1855

<211> 9

<212> PRT

<213> hepatitis C virus

<400> 1855

Thr Pro Pro His Ser Ala Lys Ser Lys
1               5

<210> 1856

<211> 10

<212> PRT

<213> hepatitis C virus


<400> 1856

Thr Pro Pro His Ser Ala Lys Ser Lys Phe
1               5               10


<210> 1857

<211> 10

<212> PRT

<213> hepatitis C virus


<400> 1857

Thr Pro Arg Cys Leu Val Asp Tyr Pro Tyr
1               5               10


<210> 1858

<211> 10

<212> PRT

<213> hepatitis C virus


<400> 1858

Thr Pro Arg Cys Met Val Asp Tyr Pro Tyr
1               5               10


<210> 1859

<211> 9

<212> PRT

<213> hepatitis C virus


<400> 1859

Thr Pro Ser Pro Ala Pro Asn Tyr Ser
1               5


<210> 1860

<211> 9

<212> PRT

<213> hepatitis C virus

<400> 1860

Thr Pro Ser Pro Val Val Val Gly Thr
1               5

<210> 1861

<211> 10

<212> PRT

<213> hepatitis C virus

<400> 1861

Thr Pro Ser Pro Val Val Val Gly Thr Thr
1               5                   10

<210> 1862

<211> 10

<212> PRT

<213> hepatitis C virus

<400> 1862

Thr Pro Val Asn Ser Trp Leu Gly Asn Ile
1               5                   10

<210> 1863

<211> 10

<212> PRT

<213> hepatitis C virus

<400> 1863

Thr Pro Tyr Phe Val Arg Ala His Val Leu
1               5                   10

<210> 1864

<211> 9

<212> PRT

<213> hepatitis C virus

```
<400>  1864

Thr Gln Asp Cys Asn Cys Ser Ile Tyr
1               5


<210>  1865

<211>  10

<212>  PRT

<213>  hepatitis C virus


<400>  1865

Thr Arg Asp Pro Thr Thr Pro Leu Ala Arg
1               5                   10


<210>  1866

<211>  10

<212>  PRT

<213>  hepatitis C virus


<400>  1866

Thr Arg Gly Val Ala Lys Ala Val Asp Phe
1               5                   10


<210>  1867

<211>  10

<212>  PRT

<213>  hepatitis C virus


<400>  1867

Thr Ser Cys Gly Asn Thr Leu Thr Cys Tyr
1               5                   10


<210>  1868

<211>  10

<212>  PRT

<213>  hepatitis C virus


<400>  1868

Thr Ser Phe Gly Asn Thr Ile Thr Cys Tyr
1               5                   10
```

<210> 1869

<211> 10

<212> PRT

<213> hepatitis C virus


<400> 1869

Thr Ser Pro Leu Thr Thr Gln His Thr Leu
1               5                   10


<210> 1870

<211> 9

<212> PRT

<213> hepatitis C virus


<400> 1870

Thr Ser Val Arg Leu Arg Ala Tyr Leu
1               5


<210> 1871

<211> 10

<212> PRT

<213> hepatitis C virus


<400> 1871

Thr Thr Ala Leu Val Val Ser Gln Leu Leu
1               5                   10


<210> 1872

<211> 10

<212> PRT

<213> hepatitis C virus


<400> 1872

Thr Thr Asp Arg Ser Gly Ala Pro Thr Tyr
1               5                   10


<210> 1873

<211> 10

<212> PRT

<213> hepatitis C virus

<400> 1873

Thr Thr Gly Glu Ile Pro Phe Tyr Gly Lys
1               5                   10

<210> 1874

<211> 9

<212> PRT

<213> hepatitis C virus

<400> 1874

Thr Thr Ile Arg Arg His Val Asp Leu
1               5

<210> 1875

<211> 10

<212> PRT

<213> hepatitis C virus

<400> 1875

Thr Thr Ile Arg Arg His Val Asp Leu Leu
1               5                   10

<210> 1876

<211> 10

<212> PRT

<213> hepatitis C virus

<400> 1876

Thr Thr Leu Pro Ala Leu Ser Thr Gly Leu
1               5                   10

<210> 1877

<211> 10

<212> PRT

<213> hepatitis C virus

<400> 1877

Thr Thr Gln Asp Cys Asn Cys Ser Ile Tyr
1                   5                   10

<210> 1878

<211> 9

<212> PRT

<213> hepatitis C virus


<400> 1878

Thr Thr Ser Arg Ser Ala Ser Leu Arg
1               5

<210> 1879

<211> 9

<212> PRT

<213> hepatitis C virus


<400> 1879

Thr Thr Ser Arg Ser Ala Ser Gln Arg
1               5

<210> 1880

<211> 10

<212> PRT

<213> hepatitis C virus


<400> 1880

Thr Val Leu Thr Asp Phe Lys Thr Trp Leu
1                   5                   10

<210> 1881

<211> 9

<212> PRT

<213> hepatitis C virus


<400> 1881

Thr Val Ser Ser Ala Leu Ala Glu Leu
1               5


<210> 1882

<211> 9

<212> PRT

<213> hepatitis C virus

<400> 1882

Thr Val Tyr His Gly Ala Gly Asn Lys
1               5

<210> 1883

<211> 9

<212> PRT

<213> hepatitis C virus

<400> 1883

Thr Trp Ala Gln Pro Gly Tyr Pro Trp
1               5

<210> 1884

<211> 10

<212> PRT

<213> hepatitis C virus

<400> 1884

Thr Tyr Ile Tyr Asn His Leu Thr Pro Leu
1               5                   10

<210> 1885

<211> 8

<212> PRT

<213> hepatitis C virus

<400> 1885

Thr Tyr Ser Thr Tyr Gly Lys Phe
1               5

<210> 1886

<211> 10

<212> PRT

<213> hepatitis C virus

<400> 1886

Thr Tyr Ser Thr Tyr Gly Lys Phe Leu Ala
1                5                    10


<210> 1887

<211> 10

<212> PRT

<213> hepatitis C virus


<400> 1887

Val Ala Ala Thr Leu Gly Phe Gly Ala Tyr
1                5                    10


<210> 1888

<211> 10

<212> PRT

<213> hepatitis C virus


<400> 1888

Val Ala Phe Lys Val Met Ser Gly Glu Met
1                5                    10


<210> 1889

<211> 9

<212> PRT

<213> hepatitis C virus


<400> 1889

Val Ala Gly Ala His Trp Gly Val Leu
1                5


<210> 1890

<211> 9

<212> PRT

<213> hepatitis C virus


<400> 1890

Val Ala Gly Ala Leu Val Ala Phe Lys
1                5

```
<210>  1891
<211>  10
<212>  PRT
<213>  hepatitis C virus


<400>  1891
Val Ala Gly Ala Leu Val Ala Phe Lys Val
1               5                   10

<210>  1892
<211>  10
<212>  PRT
<213>  hepatitis C virus


<400>  1892
Val Ala His Asp Ala Ser Gly Lys Arg Val
1               5                   10

<210>  1893
<211>  10
<212>  PRT
<213>  hepatitis C virus


<400>  1893
Val Ala Lys Ala Val Asp Phe Ile Pro Val
1               5                   10

<210>  1894
<211>  10
<212>  PRT
<213>  hepatitis C virus


<400>  1894
Val Ala Thr Asp Ala Leu Met Thr Gly Tyr
1               5                   10

<210>  1895
<211>  10
```

<210> PRT

<213> hepatitis C virus


<400> 1895

Val Cys Ala Ala Ile Leu Arg Arg His Val
1               5                   10


<210> 1896

<211> 10

<212> PRT

<213> hepatitis C virus


<400> 1896

Val Cys Ala Cys Leu Trp Met Met Leu Leu
1               5                   10


<210> 1897

<211> 10

<212> PRT

<213> hepatitis C virus


<400> 1897

Val Cys Glu Lys Met Ala Leu Tyr Asp Val
1               5                   10


<210> 1898

<211> 10

<212> PRT

<213> hepatitis C virus


<400> 1898

Val Glu Asp Val Val Asn Leu Leu Pro Ala
1               5                   10


<210> 1899

<211> 10

<212> PRT

<213> hepatitis C virus

<400> 1899

Val Glu Glu Ser Ile Tyr Gln Cys Cys Asp
1               5                   10

<210> 1900

<211> 10

<212> PRT

<213> hepatitis C virus

<400> 1900

Val Glu Phe Leu Val Asn Ala Trp Lys Ser
1               5                   10

<210> 1901

<211> 9

<212> PRT

<213> hepatitis C virus

<400> 1901

Val Glu Ser Glu Asn Lys Val Val Ile
1               5

<210> 1902

<211> 10

<212> PRT

<213> hepatitis C virus

<400> 1902

Val Glu Ser Glu Asn Lys Val Val Ile Leu
1               5                   10

<210> 1903

<211> 9

<212> PRT

<213> hepatitis C virus

<400> 1903

Val Glu Ser Glu Asn Lys Val Val Val
1               5

<210> 1904

<211> 10

<212> PRT

<213> hepatitis C virus


<400> 1904

Val Glu Ser Glu Asn Lys Val Val Val Leu
1               5               10


<210> 1905

<211> 10

<212> PRT

<213> hepatitis C virus


<400> 1905

Val Glu Ser Glu Thr Lys Val Val Ile Leu
1               5               10


<210> 1906

<211> 9

<212> PRT

<213> hepatitis C virus


<400> 1906

Val Phe Asp Ile Thr Lys Trp Leu Leu
1               5


<210> 1907

<211> 9

<212> PRT

<213> hepatitis C virus


<400> 1907

Val Phe Phe Cys Ala Ala Trp Tyr Ile
1               5


<210> 1908

<211> 10

<212> PRT

<213> hepatitis C virus


<400> 1908

Val Phe Phe Cys Ala Ala Trp Tyr Ile Lys
1                   5                   10


<210> 1909

<211> 10

<212> PRT

<213> hepatitis C virus


<400> 1909

Val Phe Trp Ala Lys His Met Trp Asn Phe
1                   5                   10


<210> 1910

<211> 10

<212> PRT

<213> hepatitis C virus


<400> 1910

Val Gly Asp Leu Cys Gly Ser Val Phe Leu
1                   5                   10


<210> 1911

<211> 10

<212> PRT

<213> hepatitis C virus


<400> 1911

Val Gly Ser Ile Gly Leu Gly Lys Val Leu
1                   5                   10


<210> 1912

<211> 9

<212> PRT

<213> hepatitis C virus


<400> 1912

```
Val Gly Val Val Cys Ala Ala Ile Leu
1               5
```

<210> 1913

<211> 9

<212> PRT

<213> hepatitis C virus


<400> 1913

```
Val Ile Cys Ala Ala Ile Leu Arg Arg
1               5
```

<210> 1914

<211> 10

<212> PRT

<213> hepatitis C virus


<400> 1914

```
Val Ile Asp Thr Leu Thr Cys Gly Phe Ala
1               5                   10
```

<210> 1915

<211> 9

<212> PRT

<213> hepatitis C virus


<400> 1915

```
Val Ile Phe Ser Pro Met Glu Ile Lys
1               5
```

<210> 1916

<211> 9

<212> PRT

<213> hepatitis C virus


<400> 1916

```
Val Ile Lys Gly Gly Arg His Leu Ile
1               5
```

<210> 1917

<211> 10

<212> PRT

<213> hepatitis C virus


<400> 1917

Val Ile Lys Gly Gly Arg His Leu Ile Phe
1               5                   10


<210> 1918

<211> 9

<212> PRT

<213> hepatitis C virus


<400> 1918

Val Ile Leu Asp Ser Phe Asp Pro Leu
1               5


<210> 1919

<211> 9

<212> PRT

<213> hepatitis C virus


<400> 1919

Val Ile Leu Leu Phe Leu Leu Leu Ala
1               5


<210> 1920

<211> 10

<212> PRT

<213> hepatitis C virus


<400> 1920

Val Lys Phe Pro Gly Gly Gly Gln Ile Val
1               5                   10


<210> 1921

<211> 10

<212> PRT

<213> hepatitis C virus

<400> 1921

Val Leu Ala Ala Leu Ala Ala Tyr Cys Leu
1               5                   10


<210> 1922

<211> 9

<212> PRT

<213> hepatitis C virus


<400> 1922

Val Leu Ala Gly Gly Val Leu Ala Ala
1               5


<210> 1923

<211> 10

<212> PRT

<213> hepatitis C virus


<400> 1923

Val Leu Ala Gly Gly Val Leu Ala Ala Val
1               5                   10


<210> 1924

<211> 9

<212> PRT

<213> hepatitis C virus


<400> 1924

Val Leu Ala Gly Tyr Gly Ala Gly Ile
1               5


<210> 1925

<211> 9

<212> PRT

<213> hepatitis C virus


<400> 1925

Val Leu Ala Gly Tyr Gly Ala Gly Val
1               5

```
<210>  1926
<211>  9
<212>  PRT
<213>  hepatitis C virus


<400>  1926

Val Leu Ala Leu Pro Gln Gln Ala Tyr
1               5


<210>  1927
<211>  10
<212>  PRT
<213>  hepatitis C virus


<400>  1927

Val Leu Glu Asp Gly Val Asn Tyr Ala Thr
1               5                   10


<210>  1928
<211>  9
<212>  PRT
<213>  hepatitis C virus


<400>  1928

Val Leu Met Thr His Phe Phe Ser Ile
1               5


<210>  1929
<211>  10
<212>  PRT
<213>  hepatitis C virus


<400>  1929

Val Leu Met Thr His Phe Phe Ser Ile Leu
1               5                   10


<210>  1930
<211>  10
```

```
<212>  PRT
<213>  hepatitis C virus


<400>  1930
Val Leu Thr Thr Ser Cys Gly Asn Thr Leu
 1               5                   10


<210>  1931
<211>  9
<212>  PRT
<213>  hepatitis C virus


<400>  1931
Val Leu Val Asp Ile Leu Ala Gly Tyr
1               5


<210>  1932
<211>  10
<212>  PRT
<213>  hepatitis C virus


<400>  1932
Val Leu Val Asp Ile Leu Ala Gly Tyr Gly
1               5                   10

<210>  1933
<211>  9
<212>  PRT
<213>  hepatitis C virus


<400>  1933
Val Leu Val Gly Gly Val Leu Ala Ala
1               5


<210>  1934
<211>  10
<212>  PRT
<213>  hepatitis C virus
```

<400> 1934

Val Leu Val Gly Gly Val Leu Ala Ala Leu
1               5                       10

<210> 1935

<211> 9

<212> PRT

<213> hepatitis C virus

<400> 1935

Val Leu Trp Thr Val Tyr His Gly Ala
1               5

<210> 1936

<211> 9

<212> PRT

<213> hepatitis C virus

<400> 1936

Val Met Ala Cys Met Ser Ala Asp Leu
1               5

<210> 1937

<211> 10

<212> PRT

<213> hepatitis C virus

<400> 1937

Val Met Phe Gly Leu Ala Tyr Phe Ser Met
1               5                       10

<210> 1938

<211> 8

<212> PRT

<213> hepatitis C virus

<400> 1938

Val Met Gly Ser Ser Tyr Gly Phe
1               5

<210> 1939

<211> 10

<212> PRT

<213> hepatitis C virus

<400> 1939

Val Met Gly Ser Ser Tyr Gly Phe Gln Tyr
1               5                   10

<210> 1940

<211> 9

<212> PRT

<213> hepatitis C virus

<400> 1940

Val Met Trp Lys Cys Leu Ile Arg Leu
1               5

<210> 1941

<211> 9

<212> PRT

<213> hepatitis C virus

<400> 1941

Val Met Trp Lys Cys Leu Thr Arg Leu
1               5

<210> 1942

<211> 9

<212> PRT

<213> hepatitis C virus

<400> 1942

Val Met Trp Thr Val Tyr His Gly Ala
1               5

<210> 1943

<211> 10

<212> PRT

<213> hepatitis C virus

<400> 1943

Val Pro Ala Ala Tyr Ala Ala Gln Gly Tyr
1               5                   10

<210> 1944
<211> 10
<212> PRT
<213> hepatitis C virus

<400> 1944

Val Pro Ala Pro Glu Phe Phe Thr Glu Val
1               5                   10

<210> 1945
<211> 10
<212> PRT
<213> hepatitis C virus

<400> 1945

Val Pro Ala Arg Ser Val Cys Gly Pro Val
1               5                   10

<210> 1946
<211> 10
<212> PRT
<213> hepatitis C virus

<400> 1946

Val Pro Ala Ser Gln Val Cys Gly Pro Val
1               5                   10

<210> 1947
<211> 10
<212> PRT
<213> hepatitis C virus

<400> 1947

```
Val Pro Ala Ser Ser Val Cys Gly Pro Val
1               5                   10


<210>   1948

<211>   10

<212>   PRT

<213>   hepatitis C virus


<400>   1948

Val Pro Glu Ser Asp Ala Ala Ala Arg Val
1               5                   10


<210>   1949

<211>   9

<212>   PRT

<213>   hepatitis C virus


<400>   1949

Val Pro Gly Ala Ala Tyr Ala Leu Tyr
1               5


<210>   1950

<211>   9

<212>   PRT

<213>   hepatitis C virus


<400>   1950

Val Pro His Pro Asn Ile Glu Glu Val
1               5


<210>   1951

<211>   10

<212>   PRT

<213>   hepatitis C virus


<400>   1951

Val Pro His Pro Asn Ile Glu Glu Val Ala
1               5                   10


<210>   1952
```

<211> 9

<212> PRT

<213> hepatitis C virus


<400> 1952

Val Pro Pro Val Val His Gly Cys Pro
1               5


<210> 1953

<211> 10

<212> PRT

<213> hepatitis C virus


<400> 1953

Val Pro Pro Val Val His Gly Cys Pro Leu
1               5               10

<210> 1954

<211> 10

<212> PRT

<213> hepatitis C virus


<400> 1954

Val Pro Gln Thr Phe Gln Val Ala His Leu
1               5               10

<210> 1955

<211> 9

<212> PRT

<213> hepatitis C virus


<400> 1955

Val Pro Thr Thr Thr Ile Arg Arg His
1               5


<210> 1956

<211> 10

<212> PRT

<213> hepatitis C virus

```
<400>  1956

Val Pro Thr Thr Thr Ile Arg Arg His Val
1               5                   10


<210>  1957

<211>  9

<212>  PRT

<213>  hepatitis C virus


<400>  1957

Val Pro Val Glu Ser Met Glu Thr Thr
1               5


<210>  1958

<211>  10

<212>  PRT

<213>  hepatitis C virus


<400>  1958

Val Pro Val Glu Ser Met Glu Thr Thr Met
1               5                   10


<210>  1959

<211>  10

<212>  PRT

<213>  hepatitis C virus


<400>  1959

Val Pro Tyr Phe Val Arg Ala His Ala Leu
1               5                   10


<210>  1960

<211>  10

<212>  PRT

<213>  hepatitis C virus


<400>  1960

Val Pro Tyr Phe Val Arg Ala Gln Gly Leu
1               5                   10
```

```
<210>   1961
<211>   9
<212>   PRT
<213>   hepatitis C virus


<400>   1961
Val Gln Asp Cys Asn Cys Ser Ile Tyr
1                   5


<210>   1962
<211>   9
<212>   PRT
<213>   hepatitis C virus


<400>   1962
Val Gln Glu Cys Asn Cys Ser Ile Tyr
1                   5


<210>   1963
<211>   10
<212>   PRT
<213>   hepatitis C virus


<400>   1963
Val Gln Trp Met Asn Arg Leu Ile Ala Phe
1                   5                   10


<210>   1964
<211>   10
<212>   PRT
<213>   hepatitis C virus


<400>   1964
Val Arg Ala Thr Arg Lys Thr Ser Glu Arg
1                   5                   10


<210>   1965
<211>   9
```

```
<212>   PRT

<213>   hepatitis C virus


<400>   1965

Val Arg Gly Gly Arg Asp Ala Ile Ile
1               5


<210>   1966

<211>   10

<212>   PRT

<213>   hepatitis C virus


<400>   1966

Val Arg Gly Gly Arg Asp Ala Ile Ile Leu
1               5                   10


<210>   1967

<211>   10

<212>   PRT

<213>   hepatitis C virus


<400>   1967

Val Arg Val Cys Glu Lys Met Ala Leu Tyr
1               5                   10


<210>   1968

<211>   10

<212>   PRT

<213>   hepatitis C virus


<400>   1968

Val Arg Val Leu Glu Asp Gly Val Asn Tyr
1               5                   10


<210>   1969

<211>   9

<212>   PRT

<213>   hepatitis C virus
```

<400> 1969

Val Ser Ala Arg Arg Gly Arg Glu Ile
1               5

<210> 1970

<211> 10

<212> PRT

<213> hepatitis C virus


<400> 1970

Val Ser Ala Arg Arg Gly Arg Glu Ile Leu
1               5                   10

<210> 1971

<211> 9

<212> PRT

<213> hepatitis C virus


<400> 1971

Val Ser Gly Ala Leu Val Ala Phe Lys
1               5

<210> 1972

<211> 10

<212> PRT

<213> hepatitis C virus


<400> 1972

Val Ser Arg Ser Gln Arg Arg Gly Arg Thr
1               5                   10

<210> 1973

<211> 9

<212> PRT

<213> hepatitis C virus


<400> 1973

Val Ser Thr Ala Thr Gln Ser Phe Leu
1               5

<210> 1974

<211> 10

<212> PRT

<213> hepatitis C virus


<400> 1974

Val Ser Val Pro Ala Glu Ile Leu Arg Lys
1               5                   10


<210> 1975

<211> 9

<212> PRT

<213> hepatitis C virus


<400> 1975

Val Thr Phe Asp Arg Leu Gln Val Leu
1               5


<210> 1976

<211> 10

<212> PRT

<213> hepatitis C virus


<400> 1976

Val Thr Leu Thr His Pro Ile Thr Lys Tyr
1               5                   10


<210> 1977

<211> 10

<212> PRT

<213> hepatitis C virus


<400> 1977

Val Thr Gln Ile Leu Ser Ser Leu Thr Ile
1               5                   10


<210> 1978

<211> 9

<212> PRT

<213> hepatitis C virus

<400> 1978

Val Val Cys Ala Ala Ile Leu Arg Arg
1               5

<210> 1979

<211> 9

<212> PRT

<213> hepatitis C virus

<400> 1979

Val Val Glu Ser Lys Trp Arg Ala Leu
1               5

<210> 1980

<211> 10

<212> PRT

<213> hepatitis C virus

<400> 1980

Val Val Gly Val Val Cys Ala Ala Ile Leu
1               5                   10

<210> 1981

<211> 10

<212> PRT

<213> hepatitis C virus

<400> 1981

Val Val Ile Leu Asp Ser Phe Asp Pro Leu
1               5                   10

<210> 1982

<211> 9

<212> PRT

<213> hepatitis C virus

<400> 1982

```
Val Val Ile Val Gly Arg Ile Ile Leu
1               5

<210>  1983

<211>  9

<212>  PRT

<213>  hepatitis C virus


<400>  1983

Val Val Leu Leu Phe Leu Leu Leu Ala
1               5

<210>  1984

<211>  9

<212>  PRT

<213>  hepatitis C virus


<400>  1984

Val Val Leu Thr His Pro Ile Thr Lys
1               5

<210>  1985

<211>  10

<212>  PRT

<213>  hepatitis C virus


<400>  1985

Val Val Ser Thr Ala Thr Gln Ser Phe Leu
1               5                    10

<210>  1986

<211>  10

<212>  PRT

<213>  hepatitis C virus


<400>  1986

Val Val Ser Thr Leu Pro Gln Ala Val Met
1               5                    10

<210>  1987
```

<211>  10

<212>  PRT

<213>  hepatitis C virus


<400>  1987

Val Val Val Ala Thr Asp Ala Leu Met Thr
1                   5                   10


<210>  1988

<211>  9

<212>  PRT

<213>  hepatitis C virus


<400>  1988

Val Val Val Val Ala Thr Asp Ala Leu
1                   5


<210>  1989

<211>  10

<212>  PRT

<213>  hepatitis C virus


<400>  1989

Val Val Val Val Ala Thr Asp Ala Leu Met
1                   5                   10


<210>  1990

<211>  10

<212>  PRT

<213>  hepatitis C virus


<400>  1990

Val Tyr Leu Leu Pro Arg Arg Gly Pro Arg
1                   5                   10


<210>  1991

<211>  10

<212>  PRT

<213>  hepatitis C virus

<400> 1991

Val Tyr Tyr Leu Thr Arg Asp Pro Thr Thr
1               5                   10

<210> 1992

<211> 9

<212> PRT

<213> hepatitis C virus

<400> 1992

Trp Ala His Ala Gly Leu Arg Asp Leu
1               5

<210> 1993

<211> 9

<212> PRT

<213> hepatitis C virus

<400> 1993

Trp Ala Lys His Met Trp Asn Phe Ile
1               5

<210> 1994

<211> 9

<212> PRT

<213> hepatitis C virus

<400> 1994

Trp Ala Lys Val Leu Ile Val Met Leu
1               5

<210> 1995

<211> 10

<212> PRT

<213> hepatitis C virus

<400> 1995

Trp Ala Lys Val Leu Ile Val Met Leu Leu
1               5                   10

<210> 1996

<211> 10

<212> PRT

<213> hepatitis C virus

<400> 1996

Trp Ala Gln Pro Gly Tyr Pro Trp Pro Leu
1               5                   10

<210> 1997

<211> 10

<212> PRT

<213> hepatitis C virus

<400> 1997

Trp Ala Arg Met Ile Leu Met Thr His Phe
1               5                   10

<210> 1998

<211> 10

<212> PRT

<213> hepatitis C virus

<400> 1998

Trp Ala Arg Pro Asp Tyr Asn Pro Pro Leu
1               5                   10

<210> 1999

<211> 9

<212> PRT

<213> hepatitis C virus

<400> 1999

Trp Ala Val Arg Thr Lys Leu Lys Leu
1               5

<210> 2000

<211> 9

<212> PRT

<213> hepatitis C virus

<400> 2000

Trp Glu Ala Val Phe Thr Gly Leu Thr
1               5

<210> 2001

<211> 9

<212> PRT

<213> hepatitis C virus

<400> 2001

Trp Glu Gly Val Phe Thr Gly Leu Thr
1               5

<210> 2002

<211> 10

<212> PRT

<213> hepatitis C virus

<400> 2002

Trp Glu Ser Val Phe Thr Gly Leu Thr His
1               5                    10

<210> 2003

<211> 9

<212> PRT

<213> hepatitis C virus

<400> 2003

Trp Glu Thr Ala Arg His Thr Pro Ile
1               5

<210> 2004

<211> 10

<212> PRT

<213> hepatitis C virus

```
<400>  2004

Trp Glu Thr Ala Arg His Thr Pro Val Asn
1               5                   10


<210>  2005

<211>  9

<212>  PRT

<213>  hepatitis C virus


<400>  2005

Trp Glu Thr Val Arg His Ser Pro Val
1               5


<210>  2006

<211>  9

<212>  PRT

<213>  hepatitis C virus


<400>  2006

Trp Glu Thr Val Arg His Thr Pro Val
1               5


<210>  2007

<211>  9

<212>  PRT

<213>  hepatitis C virus


<400>  2007

Trp Glu Trp Val Ile Leu Leu Phe Leu
1               5


<210>  2008

<211>  10

<212>  PRT

<213>  hepatitis C virus


<400>  2008

Trp Glu Trp Val Ile Leu Leu Phe Leu Leu
1               5                   10
```

<210> 2009

<211> 9

<212> PRT

<213> hepatitis C virus


<400> 2009

Trp Glu Trp Val Val Leu Leu Phe Leu
1               5


<210> 2010

<211> 10

<212> PRT

<213> hepatitis C virus


<400> 2010

Trp Glu Trp Val Val Leu Leu Phe Leu Leu
1               5                   10


<210> 2011

<211> 9

<212> PRT

<213> hepatitis C virus


<400> 2011

Trp Glu Tyr Val Val Leu Leu Phe Leu
1               5


<210> 2012

<211> 10

<212> PRT

<213> hepatitis C virus


<400> 2012

Trp Glu Tyr Val Val Leu Leu Phe Leu Leu
1               5                   10


<210> 2013

<211> 10

<212> PRT

<213> hepatitis C virus


<400> 2013

Trp Lys Ser Lys Lys Cys Pro Met Gly Phe
1               5                    10


<210> 2014

<211> 10

<212> PRT

<213> hepatitis C virus


<400> 2014

Trp Leu Gln Ser Lys Leu Leu Pro Arg Leu
1               5                    10


<210> 2015

<211> 9

<212> PRT

<213> hepatitis C virus


<400> 2015

Trp Met Asn Arg Leu Ile Ala Phe Ala
1               5


<210> 2016

<211> 9

<212> PRT

<213> hepatitis C virus


<400> 2016

Trp Met Asn Ser Thr Gly Phe Thr Lys
1               5


<210> 2017

<211> 9

<212> PRT

<213> hepatitis C virus


<400> 2017

Trp Pro Leu Leu Leu Leu Leu Leu Ala
1               5

<210>  2018

<211>  10

<212>  PRT

<213>  hepatitis C virus


<400>  2018

Trp Pro Leu Leu Leu Leu Leu Leu Ala Leu
1               5                   10

<210>  2019

<211>  9

<212>  PRT

<213>  hepatitis C virus


<400>  2019

Trp Pro Leu Tyr Gly Asn Glu Gly Met
1               5

<210>  2020

<211>  10

<212>  PRT

<213>  hepatitis C virus


<400>  2020

Trp Pro Leu Tyr Gly Asn Glu Gly Met Gly
1               5                   10

<210>  2021

<211>  10

<212>  PRT

<213>  hepatitis C virus


<400>  2021

Trp Gln Ala Pro Pro Gly Ala Arg Ser Leu
1               5                   10

<210>  2022

<211> 9
<212> PRT
<213> hepatitis C virus

<400> 2022
Trp Thr Arg Gly Glu Arg Cys Asp Leu
1               5

<210> 2023
<211> 10
<212> PRT
<213> hepatitis C virus

<400> 2023
Trp Tyr Leu Val Ala Phe Cys Ala Ala Trp
1               5                   10

<210> 2024
<211> 10
<212> PRT
<213> hepatitis C virus

<400> 2024
Tyr Ala Ala Gln Gly Tyr Lys Val Leu Val
1               5                   10

<210> 2025
<211> 9
<212> PRT
<213> hepatitis C virus

<400> 2025
Tyr Ala Leu Tyr Gly Val Trp Pro Leu
1               5

<210> 2026
<211> 10
<212> PRT
<213> hepatitis C virus

<400> 2026

Tyr Ala Leu Tyr Gly Val Trp Pro Leu Leu
1               5                   10

<210> 2027

<211> 9

<212> PRT

<213> hepatitis C virus

<400> 2027

Tyr Ala Pro Ala Cys Lys Pro Leu Leu
1               5

<210> 2028

<211> 10

<212> PRT

<213> hepatitis C virus

<400> 2028

Tyr Ala Pro Thr Leu Trp Ala Arg Met Ile
1               5                   10

<210> 2029

<211> 10

<212> PRT

<213> hepatitis C virus

<400> 2029

Tyr Ala Thr Thr Ser Arg Ser Ala Ser Leu
1               5                   10

<210> 2030

<211> 10

<212> PRT

<213> hepatitis C virus

<400> 2030

Tyr Asp Ala Gly Cys Ala Trp Tyr Glu Leu
1               5                   10

<210> 2031

<211> 9

<212> PRT

<213> hepatitis C virus


<400> 2031

Tyr Glu Leu Thr Pro Ala Glu Thr Thr
1               5


<210> 2032

<211> 9

<212> PRT

<213> hepatitis C virus


<400> 2032

Tyr Phe Val Arg Ala His Ala Leu Leu
1               5


<210> 2033

<211> 10

<212> PRT

<213> hepatitis C virus


<400> 2033

Tyr Gly Ala Cys Tyr Ser Ile Glu Pro Leu
1               5                   10


<210> 2034

<211> 9

<212> PRT

<213> hepatitis C virus


<400> 2034

Tyr Gly Lys Ala Ile Pro Ile Glu Val
1               5


<210> 2035

<211> 10

```
<212>   PRT
<213>   hepatitis C virus


<400>   2035
Tyr Gly Lys Ala Ile Pro Ile Glu Val Ile
1               5                   10


<210>   2036
<211>   9
<212>   PRT
<213>   hepatitis C virus


<400>   2036
Tyr Gly Val Trp Pro Leu Leu Leu Leu
1               5


<210>   2037
<211>   10
<212>   PRT
<213>   hepatitis C virus


<400>   2037
Tyr Ile Met Ala Cys Met Ser Ala Asp Leu
1               5                   10


<210>   2038
<211>   9
<212>   PRT
<213>   hepatitis C virus


<400>   2038
Tyr Ile Pro Leu Val Gly Ala Pro Leu
1               5


<210>   2039
<211>   9
<212>   PRT
<213>   hepatitis C virus
```

```
<400>  2039
Tyr Ile Tyr Asp His Leu Thr Pro Met
1               5


<210>  2040
<211>  9
<212>  PRT
<213>  hepatitis C virus


<400>  2040
Tyr Ile Tyr Asn His Leu Thr Pro Leu
1               5


<210>  2041
<211>  10
<212>  PRT
<213>  hepatitis C virus


<400>  2041
Tyr Lys Val Leu Val Leu Asn Pro Ser Val
1               5                   10


<210>  2042
<211>  10
<212>  PRT
<213>  hepatitis C virus


<400>  2042
Tyr Leu Phe Asn Trp Ala Val Lys Thr Lys
1               5                   10


<210>  2043
<211>  9
<212>  PRT
<213>  hepatitis C virus


<400>  2043
Tyr Leu Phe Asn Trp Ala Val Arg Thr
1               5
```

<210> 2044

<211> 10

<212> PRT

<213> hepatitis C virus


<400> 2044

Tyr Leu Phe Asn Trp Ala Val Arg Thr Lys
1               5                   10


<210> 2045

<211> 10

<212> PRT

<213> hepatitis C virus


<400> 2045

Tyr Leu Lys Ala Ser Ala Ala Cys Arg Ala
1               5                   10


<210> 2046

<211> 10

<212> PRT

<213> hepatitis C virus


<400> 2046

Tyr Leu Lys Gly Ser Ser Gly Gly Pro Leu
1               5                   10


<210> 2047

<211> 10

<212> PRT

<213> hepatitis C virus


<400> 2047

Tyr Leu Leu Pro Arg Arg Gly Pro Arg Leu
1               5                   10


<210> 2048

<211> 9

<212> PRT

<213> hepatitis C virus

<400> 2048

Tyr Leu Asn Thr Pro Gly Leu Pro Val
1               5

<210> 2049

<211> 9

<212> PRT

<213> hepatitis C virus

<400> 2049

Tyr Leu Ser Thr Pro Gly Leu Pro Val
1               5

<210> 2050

<211> 9

<212> PRT

<213> hepatitis C virus

<400> 2050

Tyr Leu Thr Ala Tyr Gln Ala Thr Val
1               5

<210> 2051

<211> 10

<212> PRT

<213> hepatitis C virus

<400> 2051

Tyr Leu Thr Arg Asp Pro Thr Thr Pro Leu
1               5                   10

<210> 2052

<211> 9

<212> PRT

<213> hepatitis C virus

<400> 2052

Tyr Leu Val Ala Tyr Gln Ala Thr Val
1               5

<210> 2053

<211> 9

<212> PRT

<213> hepatitis C virus


<400> 2053

Tyr Leu Val Thr Arg His Ala Asp Val
1               5

<210> 2054

<211> 10

<212> PRT

<213> hepatitis C virus


<400> 2054

Tyr Leu Val Thr Arg His Ala Asp Val Ile
1               5                   10

<210> 2055

<211> 9

<212> PRT

<213> hepatitis C virus


<400> 2055

Tyr Leu Val Thr Arg Asn Ala Asp Val
1               5

<210> 2056

<211> 9

<212> PRT

<213> hepatitis C virus


<400> 2056

Tyr Leu Tyr Gly Val Gly Ser Ala Val
1               5

<210> 2057

<211> 10

<212> PRT

<213> hepatitis C virus


<400> 2057

Tyr Leu Tyr Gly Val Gly Ser Ala Val Val
1               5                   10


<210> 2058

<211> 9

<212> PRT

<213> hepatitis C virus


<400> 2058

Tyr Pro Cys Thr Val Asn Phe Ser Ile
1               5


<210> 2059

<211> 9

<212> PRT

<213> hepatitis C virus


<400> 2059

Tyr Pro Cys Thr Val Asn Phe Thr Ile
1               5


<210> 2060

<211> 10

<212> PRT

<213> hepatitis C virus


<400> 2060

Tyr Pro Cys Thr Val Asn Phe Thr Ile Phe
1               5                   10


<210> 2061

<211> 9

<212> PRT

<213> hepatitis C virus

<400> 2061

Tyr Pro Cys Thr Val Asn Phe Thr Leu
1               5

<210> 2062

<211> 9

<212> PRT

<213> hepatitis C virus

<400> 2062

Tyr Pro Gly His Val Ser Gly His Arg
1               5

<210> 2063

<211> 10

<212> PRT

<213> hepatitis C virus

<400> 2063

Tyr Pro Gly His Val Ser Gly His Arg Met
1               5               10

<210> 2064

<211> 10

<212> PRT

<213> hepatitis C virus

<400> 2064

Tyr Pro Trp Pro Leu Tyr Gly Asn Glu Gly
1               5               10

<210> 2065

<211> 10

<212> PRT

<213> hepatitis C virus

<400> 2065

Tyr Arg Leu Gly Ala Val Gln Asn Glu Val
1               5               10

<210> 2066

<211> 10

<212> PRT

<213> hepatitis C virus

<400> 2066

Tyr Arg Arg Cys Arg Ala Ser Gly Val Leu
1               5                   10

<210> 2067

<211> 10

<212> PRT

<213> hepatitis C virus

<400> 2067

Tyr Ser Gly Gly Asp Ile Tyr His Ser Leu
1               5                   10

<210> 2068

<211> 10

<212> PRT

<213> hepatitis C virus

<400> 2068

Tyr Ser Pro Gly Gln Arg Val Glu Phe Leu
1               5                   10

<210> 2069

<211> 9

<212> PRT

<213> hepatitis C virus

<400> 2069

Tyr Ser Gln Gln Thr Arg Gly Leu Leu
1               5

<210> 2070

<211> 9

<212> PRT

<213> hepatitis C virus


<400> 2070

Tyr Thr Ile Phe Lys Ile Arg Met Tyr
1               5


<210> 2071

<211> 10

<212> PRT

<213> hepatitis C virus


<400> 2071

Tyr Thr Lys Cys Gly Ser Gly Pro Trp Leu
1               5                   10


<210> 2072

<211> 9

<212> PRT

<213> hepatitis C virus


<400> 2072

Tyr Val Gly Gly Val Glu His Arg Leu
1               5


<210> 2073

<211> 9

<212> PRT

<213> hepatitis C virus


<400> 2073

Tyr Val Leu Leu Leu Phe Leu Leu Leu
1               5


<210> 2074

<211> 9

<212> PRT

<213> hepatitis C virus

<400> 2074

Tyr Val Gln Met Ala Phe Met Lys Leu
1                   5

<210> 2075

<211> 9

<212> PRT

<213> hepatitis C virus

<400> 2075

Tyr Val Val Leu Leu Phe Leu Leu Leu
1                   5

<210> 2076

<211> 10

<212> PRT

<213> hepatitis C virus

<400> 2076

Tyr Val Val Leu Leu Phe Leu Leu Leu Ala
1                   5                   10

<210> 2077

<211> 9

<212> PRT

<213> hepatitis C virus

<400> 2077

Tyr Val Tyr Asp His Leu Thr Pro Leu
1                   5

<210> 2078

<211> 10

<212> PRT

<213> hepatitis C virus

<400> 2078

Tyr Val Tyr Asp His Leu Thr Pro Leu Arg
1                   5                   10

```
<210>  2079
<211>  9
<212>  PRT
<213>  hepatitis C virus


<400>  2079

Tyr Val Tyr Asn His Leu Thr Pro Leu
1               5


<210>  2080
<211>  10
<212>  PRT
<213>  hepatitis C virus


<400>  2080

Tyr Val Tyr Asn His Leu Thr Pro Leu Arg
1               5                   10


<210>  2081
<211>  10
<212>  PRT
<213>  hepatitis C virus


<400>  2081

Tyr Tyr Leu Thr Arg Asp Pro Thr Thr Pro
1               5                   10


<210>  2082
<211>  9
<212>  PRT
<213>  hepatitis C virus


<400>  2082

Tyr Tyr Arg Gly Leu Asp Val Ser Ile
1               5


<210>  2083
<211>  10
<212>  PRT
```

<213> hepatitis C virus


<400> 2083

Tyr Tyr Arg Gly Leu Asp Val Ser Val Ile
1               5                   10


<210> 2084

<211> 9

<212> PRT

<213> hepatitis C virus


<400> 2084

Gly Glu Met Pro Ser Thr Glu Asp Leu
1               5


<210> 2085

<211> 9

<212> PRT

<213> hepatitis C virus


<400> 2085

Ala Asp Leu Met Gly Tyr Ile Pro Leu
1               5


<210> 2086

<211> 9

<212> PRT

<213> hepatitis C virus


<400> 2086

Gly Ala Leu Val Ala Phe Lys Val Met
1               5


<210> 2087

<211> 9

<212> PRT

<213> hepatitis C virus


<400> 2087

Thr Arg Val Pro Tyr Phe Val Arg Ala
1               5

<210>   2088

<211>   9

<212>   PRT

<213>   hepatitis C virus


<400>   2088

Ser Asp Val Glu Ser Tyr Ser Ser Met
1               5

<210>   2089

<211>   9

<212>   PRT

<213>   hepatitis C virus


<400>   2089

Glu Ser Tyr Ser Ser Met Pro Pro Leu
1               5

<210>   2090

<211>   9

<212>   PRT

<213>   hepatitis C virus


<400>   2090

Val Leu Tyr Arg Glu Phe Asp Glu Met
1               5

<210>   2091

<211>   9

<212>   PRT

<213>   hepatitis C virus


<400>   2091

Gln Ile Ile Glu Arg Leu His Gly Leu
1               5

<210>   2092

<211> 9

<212> PRT

<213> hepatitis C virus


<400> 2092

Leu Ser Val Glu Glu Ala Cys Lys Leu
1               5


<210> 2093

<211> 9

<212> PRT

<213> hepatitis C virus


<400> 2093

Trp His Tyr Pro Cys Thr Val Asn Phe
1               5


<210> 2094

<211> 9

<212> PRT

<213> hepatitis C virus


<400> 2094

Cys Gln Val Pro Ala Pro Glu Phe Phe
1               5


<210> 2095

<211> 9

<212> PRT

<213> hepatitis C virus


<400> 2095

His Gly Ile Leu Ser Phe Leu Val Phe
1               5


<210> 2096

<211> 9

<212> PRT

<213> hepatitis C virus

<400> 2096

Ser Thr Leu Pro Gly Asn Pro Ala Ile
1               5


<210> 2097

<211> 9

<212> PRT

<213> hepatitis C virus


<400> 2097

Ala Lys Val Leu Ile Val Met Leu Leu
1               5


<210> 2098

<211> 9

<212> PRT

<213> hepatitis C virus


<400> 2098

Gln Asn Ile Val Asp Val Gln Tyr Leu
1               5


<210> 2099

<211> 9

<212> PRT

<213> hepatitis C virus


<400> 2099

Asn Gln Tyr Leu Val Gly Ser Gln Leu
1               5


<210> 2100

<211> 9

<212> PRT

<213> hepatitis C virus


<400> 2100

Phe Gln Val Gly Leu Asn Gln Tyr Leu
1               5

<210> 2101

<211> 9

<212> PRT

<213> hepatitis C virus


<400> 2101

Thr Met Leu Val Asn Gly Asp Asp Leu
1               5


<210> 2102

<211> 15

<212> PRT

<213> hepatitis C virus


<400> 2102

Ala Ala Gln Leu Ala Pro Pro Ser Ala Ala Ser Ala Phe Val Gly
1               5                   10                  15


<210> 2103

<211> 15

<212> PRT

<213> hepatitis C virus


<400> 2103

Ala Cys Lys Leu Thr Pro Pro His Ser Ala Lys Ser Lys Phe Gly
1               5                   10                  15


<210> 2104

<211> 15

<212> PRT

<213> hepatitis C virus


<400> 2104

Ala Asp Leu Ile Glu Ala Asn Leu Leu Trp Arg Gln Glu Met Gly
1               5                   10                  15


<210> 2105

<211> 15

```
<212>   PRT

<213>   hepatitis C virus


<400>   2105

Ala Asp Leu Met Gly Tyr Ile Pro Leu Val Gly Ala Pro Leu Gly
1               5                   10                  15


<210>   2106

<211>   15

<212>   PRT

<213>   hepatitis C virus


<400>   2106

Ala Pro Thr Leu Trp Ala Arg Met Ile Leu Met Thr His Phe Phe
1               5                   10                  15


<210>   2107

<211>   15

<212>   PRT

<213>   hepatitis C virus


<400>   2107

Ala Gln Gly Tyr Lys Val Leu Val Leu Asn Pro Ser Val Ala Ala
1               5                   10                  15


<210>   2108

<211>   15

<212>   PRT

<213>   hepatitis C virus


<400>   2108

Ala Arg Ala Ala Trp Glu Thr Ala Arg His Thr Pro Val Asn Ser
1               5                   10                  15


<210>   2109

<211>   15

<212>   PRT

<213>   hepatitis C virus
```

```
<400>    2109

Ala Arg Leu Val Val Leu Ala Thr Ala Thr Pro Pro Gly Ser Val
1               5               10              15


<210>    2110

<211>    15

<212>    PRT

<213>    hepatitis C virus



<400>    2110

Ala Ser Cys Leu Arg Lys Leu Gly Val Pro Pro Leu Arg Val Trp
1               5               10              15


<210>    2111

<211>    15

<212>    PRT

<213>    hepatitis C virus



<400>    2111

Ala Ser Gln Leu Ser Ala Pro Ser Leu Lys Ala Thr Cys Thr Thr
1               5               10              15


<210>    2112

<211>    15

<212>    PRT

<213>    hepatitis C virus



<400>    2112

Ala Val Gly Ile Phe Arg Ala Ala Val Cys Thr Arg Gly Val Ala
1               5               10              15


<210>    2113

<211>    15

<212>    PRT

<213>    hepatitis C virus



<400>    2113

Ala Val Gln Trp Met Asn Arg Leu Ile Ala Phe Ala Ser Arg Gly
1               5               10              15
```

<210> 2114

<211> 15

<212> PRT

<213> hepatitis C virus

<400> 2114

Ala Trp Asp Met Met Met Asn Trp Ser Pro Thr Thr Ala Leu Val
1               5                   10                  15

<210> 2115

<211> 15

<212> PRT

<213> hepatitis C virus

<400> 2115

Ala Tyr Cys Leu Thr Thr Gly Ser Val Val Ile Val Gly Arg Ile
1               5                   10                  15

<210> 2116

<211> 15

<212> PRT

<213> hepatitis C virus

<400> 2116

Cys Gln Ile Tyr Gly Ala Cys Tyr Ser Ile Glu Pro Leu Asp Leu
1               5                   10                  15

<210> 2117

<211> 15

<212> PRT

<213> hepatitis C virus

<400> 2117

Asp Ala Asp Leu Ile Glu Ala Asn Leu Leu Trp Arg Gln Glu Met
1               5                   10                  15

<210> 2118

<211> 15

<212> PRT

<213> hepatitis C virus

<400> 2118

Asp Ala His Phe Leu Ser Gln Thr Lys Gln Ala Gly Asp Asn Phe
1               5                   10                  15

<210> 2119

<211> 15

<212> PRT

<213> hepatitis C virus

<400> 2119

Asp Ile Ile Ile Cys Asp Glu Cys His Ser Thr Asp Ser Thr Thr
1               5                   10                  15

<210> 2120

<211> 15

<212> PRT

<213> hepatitis C virus

<400> 2120

Asp Leu Ala Val Ala Val Glu Pro Val Val Phe Ser Asp Met Glu
1               5                   10                  15

<210> 2121

<211> 14

<212> PRT

<213> hepatitis C virus

<400> 2121

Asp Leu Val Asn Leu Leu Pro Ala Ile Leu Ser Pro Gly Ala
1               5                   10

<210> 2122

<211> 15

<212> PRT

<213> hepatitis C virus

<400> 2122

```
Asp Pro Thr Phe Thr Ile Glu Thr Thr Thr Val Pro Gln Asp Ala
1               5               10              15
```

<210> 2123

<211> 15

<212> PRT

<213> hepatitis C virus

<400> 2123

```
Asp Ser Ser Val Leu Cys Glu Cys Tyr Asp Ala Gly Cys Ala Trp
1               5               10              15
```

<210> 2124

<211> 15

<212> PRT

<213> hepatitis C virus

<400> 2124

```
Asp Val Val Val Val Ala Thr Asp Ala Leu Met Thr Gly Phe Thr
1               5               10              15
```

<210> 2125

<211> 15

<212> PRT

<213> hepatitis C virus

<400> 2125

```
Asp Val Val Val Val Ala Thr Asp Ala Leu Met Thr Gly Tyr Thr
1               5               10              15
```

<210> 2126

<211> 14

<212> PRT

<213> hepatitis C virus

<400> 2126

```
Glu Asp Leu Val Asn Leu Leu Pro Ala Ile Leu Ser Pro Gly
1               5               10
```

<210> 2127

<211> 15

<212> PRT

<213> hepatitis C virus


<400> 2127

Glu Pro Asp Val Ala Val Leu Thr Ser Met Leu Thr Asp Pro Ser
1               5               10              15


<210> 2128

<211> 15

<212> PRT

<213> hepatitis C virus


<400> 2128

Phe Asn Ile Leu Gly Gly Trp Val Ala Ala Gln Leu Ala Pro Pro
1               5               10              15


<210> 2129

<211> 15

<212> PRT

<213> hepatitis C virus


<400> 2129

Phe Pro Tyr Leu Val Ala Tyr Gln Ala Thr Val Cys Ala Arg Ala
1               5               10              15


<210> 2130

<211> 15

<212> PRT

<213> hepatitis C virus


<400> 2130

Phe Ser Ile Phe Leu Leu Ala Leu Leu Ser Cys Leu Thr Ile Pro
1               5               10              15


<210> 2131

<211> 15

<212> PRT

<213> hepatitis C virus

<400> 2131

Phe Ser Ile Phe Leu Leu Ala Leu Leu Ser Cys Leu Thr Val Pro
1               5                   10                  15

<210> 2132

<211> 15

<212> PRT

<213> hepatitis C virus

<400> 2132

Phe Thr Thr Leu Pro Ala Leu Ser Thr Gly Leu Ile His Leu His
1               5                   10                  15

<210> 2133

<211> 15

<212> PRT

<213> hepatitis C virus

<400> 2133

Gly Ala Cys Tyr Ser Ile Glu Pro Leu Asp Leu Pro Gln Ile Ile
1               5                   10                  15

<210> 2134

<211> 15

<212> PRT

<213> hepatitis C virus

<400> 2134

Gly Ala Gly Val Ala Gly Ala Leu Val Ala Phe Lys Ile Met Ser
1               5                   10                  15

<210> 2135

<211> 15

<212> PRT

<213> hepatitis C virus

<400> 2135

Gly Ala Gly Val Ala Gly Ala Leu Val Ala Phe Lys Val Met Ser
1               5                   10                  15

<210> 2136

<211> 15

<212> PRT

<213> hepatitis C virus


<400> 2136

Gly Ala Leu Val Val Gly Val Val Cys Ala Ala Ile Leu Arg Arg
1               5               10                  15

<210> 2137

<211> 15

<212> PRT

<213> hepatitis C virus


<400> 2137

Gly Ala Arg Leu Val Val Leu Ala Thr Ala Thr Pro Pro Gly Ser
1               5               10                  15

<210> 2138

<211> 15

<212> PRT

<213> hepatitis C virus


<400> 2138

Gly Cys Gly Trp Ala Gly Trp Leu Leu Ser Pro Arg Gly Ser Arg
1               5               10                  15

<210> 2139

<211> 15

<212> PRT

<213> hepatitis C virus


<400> 2139

Gly Cys Ser Phe Ser Ile Phe Leu Leu Ala Leu Leu Ser Cys Leu
1               5               10                  15

<210> 2140

<211> 15

<212> PRT

<213> hepatitis C virus


<400> 2140

Gly Asp Asn Phe Pro Tyr Leu Val Ala Tyr Gln Ala Thr Val Cys
1               5               10              15


<210> 2141

<211> 15

<212> PRT

<213> hepatitis C virus


<400> 2141

Gly Gly Val Leu Ala Ala Leu Ala Ala Tyr Cys Leu Thr Thr Gly
1               5               10              15


<210> 2142

<211> 15

<212> PRT

<213> hepatitis C virus


<400> 2142

Gly His Arg Met Ala Trp Asp Met Met Met Asn Trp Ser Pro Thr
1               5               10              15


<210> 2143

<211> 15

<212> PRT

<213> hepatitis C virus


<400> 2143

Gly Ile Gln Tyr Leu Ala Gly Leu Ser Thr Leu Pro Gly Asn Pro
1               5               10              15


<210> 2144

<211> 15

<212> PRT

<213> hepatitis C virus

<400> 2144

Gly Lys Tyr Leu Phe Asn Trp Ala Val Arg Thr Lys Leu Lys Leu
1               5               10              15

<210> 2145

<211> 15

<212> PRT

<213> hepatitis C virus


<400> 2145

Gly Leu Gly Trp Ala Gly Trp Leu Leu Ser Pro Arg Gly Ser Arg
1               5               10              15

<210> 2146

<211> 15

<212> PRT

<213> hepatitis C virus


<400> 2146

Gly Leu Pro Val Cys Gln Asp His Leu Glu Phe Trp Glu Ser Val
1               5               10              15

<210> 2147

<211> 15

<212> PRT

<213> hepatitis C virus


<400> 2147

Gly Met Gly Trp Ala Gly Trp Leu Leu Ser Pro Arg Gly Ser Arg
1               5               10              15

<210> 2148

<211> 15

<212> PRT

<213> hepatitis C virus


<400> 2148

Gly Met Gln Leu Ala Glu Gln Phe Lys Gln Lys Ala Leu Gly Leu
1               5               10              15

<210> 2149

<211> 15

<212> PRT

<213> hepatitis C virus


<400> 2149

Gly Pro Arg Leu Gly Val Arg Ala Thr Arg Lys Thr Ser Glu Arg
1               5                   10                  15

<210> 2150

<211> 15

<212> PRT

<213> hepatitis C virus


<400> 2150

Gly Gln Gly Trp Arg Leu Leu Ala Pro Ile Thr Ala Tyr Ser Gln
1               5                   10                  15

<210> 2151

<211> 15

<212> PRT

<213> hepatitis C virus


<400> 2151

Gly Gln Ile Val Gly Gly Val Tyr Leu Leu Pro Arg Arg Gly Pro
1               5                   10                  15

<210> 2152

<211> 15

<212> PRT

<213> hepatitis C virus


<400> 2152

Gly Ser Gln Leu Pro Cys Glu Pro Glu Pro Asp Val Ala Val Leu
1               5                   10                  15

<210> 2153

<211> 15

<212> PRT

<213> hepatitis C virus

<400> 2153

Gly Ser Ser Tyr Gly Phe Gln Tyr Ser Pro Gly Gln Arg Val Glu
1               5                   10                  15

<210> 2154

<211> 15

<212> PRT

<213> hepatitis C virus

<400> 2154

Gly Thr Val Leu Asp Gln Ala Glu Thr Ala Gly Ala Arg Leu Val
1               5                   10                  15

<210> 2155

<211> 15

<212> PRT

<213> hepatitis C virus

<400> 2155

Gly Val Asn Tyr Ala Thr Gly Asn Leu Pro Gly Cys Ser Phe Ser
1               5                   10                  15

<210> 2156

<211> 15

<212> PRT

<213> hepatitis C virus

<400> 2156

Gly Val Arg Val Leu Glu Asp Gly Val Asn Tyr Ala Thr Gly Asn
1               5                   10                  15

<210> 2157

<211> 15

<212> PRT

<213> hepatitis C virus

<400> 2157

```
Gly Tyr Lys Val Leu Val Leu Asn Pro Ser Val Ala Ala Thr Leu
1               5               10                  15
```

<210> 2158

<211> 15

<212> PRT

<213> hepatitis C virus


<400> 2158

```
His Leu Ile Phe Cys His Ser Lys Lys Lys Cys Asp Glu Leu Ala
1               5               10                  15
```

<210> 2159

<211> 15

<212> PRT

<213> hepatitis C virus


<400> 2159

```
His Gln Trp Ile Asn Glu Asp Cys Ser Thr Pro Cys Ser Gly Ser
1               5               10                  15
```

<210> 2160

<211> 15

<212> PRT

<213> hepatitis C virus


<400> 2160

```
Ile Glu Arg Leu His Gly Leu Ser Ala Phe Ser Leu His Ser Tyr
1               5               10                  15
```

<210> 2161

<211> 15

<212> PRT

<213> hepatitis C virus


<400> 2161

```
Ile Gln Arg Leu His Gly Leu Ser Ala Phe Ser Leu His Ser Tyr
1               5               10                  15
```

<210> 2162

```
<211>  15
<212>  PRT
<213>  hepatitis C virus


<400>  2162

Ile Gln Tyr Leu Ala Gly Leu Ser Thr Leu Pro Gly Asn Pro Ala
1               5                   10                  15


<210>  2163
<211>  15
<212>  PRT
<213>  hepatitis C virus


<400>  2163

Ile Thr Arg Val Glu Ser Glu Asn Lys Val Val Ile Leu Asp Ser
1               5                   10                  15


<210>  2164
<211>  15
<212>  PRT
<213>  hepatitis C virus


<400>  2164

Lys Pro Thr Leu His Gly Pro Thr Pro Leu Leu Tyr Arg Leu Gly
1               5                   10                  15


<210>  2165
<211>  15
<212>  PRT
<213>  hepatitis C virus


<400>  2165

Lys Val Leu Val Leu Asn Pro Ser Val Ala Ala Thr Leu Gly Phe
1               5                   10                  15


<210>  2166
<211>  15
<212>  PRT
<213>  hepatitis C virus
```

<400> 2166

Leu Ala Gly Tyr Gly Ala Gly Val Ala Gly Ala Leu Val Ala Phe
1               5               10              15

<210> 2167

<211> 15

<212> PRT

<213> hepatitis C virus


<400> 2167

Leu Phe Leu Leu Leu Ala Asp Ala Arg Val Cys Ala Cys Leu Trp
1               5               10              15

<210> 2168

<211> 15

<212> PRT

<213> hepatitis C virus


<400> 2168

Leu Phe Asn Ile Leu Gly Gly Trp Val Ala Ala Gln Leu Ala Pro
1               5               10              15

<210> 2169

<211> 15

<212> PRT

<213> hepatitis C virus


<400> 2169

Leu Gly Lys Val Leu Val Asp Ile Leu Ala Gly Tyr Gly Ala Gly
1               5               10              15

<210> 2170

<211> 15

<212> PRT

<213> hepatitis C virus


<400> 2170

Leu His Ser Tyr Ser Pro Gly Glu Ile Asn Arg Val Ala Ser Cys
1               5               10              15

```
<210>   2171
<211>   15
<212>   PRT
<213>   hepatitis C virus


<400>   2171
Leu Ile Arg Leu Lys Pro Thr Leu His Gly Pro Thr Pro Leu Leu
1               5                   10                  15


<210>   2172
<211>   15
<212>   PRT
<213>   hepatitis C virus


<400>   2172
Leu Pro Ala Ile Leu Ser Pro Gly Ala Leu Val Val Gly Val Val
1               5                   10                  15


<210>   2173
<211>   15
<212>   PRT
<213>   hepatitis C virus


<400>   2173
Leu Pro Ala Leu Ser Thr Gly Leu Ile His Leu His Gln Asn Ile
1               5                   10                  15


<210>   2174
<211>   15
<212>   PRT
<213>   hepatitis C virus


<400>   2174
Leu Ser Thr Leu Pro Gly Asn Pro Ala Ile Ala Ser Leu Met Ala
1               5                   10                  15


<210>   2175
<211>   15
```

<212> PRT

<213> hepatitis C virus


<400> 2175

Leu Thr His Ile Asp Ala His Phe Leu Ser Gln Thr Lys Gln Ala
1               5               10              15


<210> 2176

<211> 15

<212> PRT

<213> hepatitis C virus


<400> 2176

Leu Thr His Ile Asp Ala His Phe Leu Ser Gln Thr Lys Gln Ser
1               5               10              15


<210> 2177

<211> 15

<212> PRT

<213> hepatitis C virus


<400> 2177

Leu Thr Ser Met Leu Thr Asp Pro Ser His Ile Thr Ala Glu Thr
1               5               10              15


<210> 2178

<211> 15

<212> PRT

<213> hepatitis C virus


<400> 2178

Leu Val Ala Tyr Gln Ala Thr Val Cys Ala Arg Ala Gln Ala Pro
1               5               10              15


<210> 2179

<211> 15

<212> PRT

<213> hepatitis C virus

```
<400> 2179

Leu Val Asn Leu Leu Pro Ala Ile Leu Ser Pro Gly Ala Leu Val
1               5               10              15


<210> 2180

<211> 15

<212> PRT

<213> hepatitis C virus


<400> 2180

Leu Val Val Leu Ala Thr Ala Thr Pro Pro Gly Ser Val Thr Val
1               5               10              15


<210> 2181

<211> 15

<212> PRT

<213> hepatitis C virus


<400> 2181

Met Ala Cys Met Ser Ala Asp Leu Glu Val Val Thr Ser Thr Trp
1               5               10              15


<210> 2182

<211> 15

<212> PRT

<213> hepatitis C virus


<400> 2182

Met Asn Arg Leu Ile Ala Phe Ala Ser Arg Gly Asn His Val Ser
1               5               10              15


<210> 2183

<211> 13

<212> PRT

<213> hepatitis C virus


<400> 2183

Met Pro Pro Leu Glu Gly Glu Pro Gly Asp Pro Asp Leu
1               5               10
```

<210> 2184

<211> 12

<212> PRT

<213> hepatitis C virus

<400> 2184

Met Ser Thr Asn Pro Lys Pro Gln Arg Lys Thr Lys
1               5               10

<210> 2185

<211> 15

<212> PRT

<213> hepatitis C virus

<400> 2185

Met Thr Gly Phe Thr Gly Asp Phe Asp Ser Val Ile Asp Cys Asn
1               5               10              15

<210> 2186

<211> 15

<212> PRT

<213> hepatitis C virus

<400> 2186

Asn Pro Ala Ile Ala Ser Leu Met Ala Phe Thr Ala Ser Ile Thr
1               5               10              15

<210> 2187

<211> 15

<212> PRT

<213> hepatitis C virus

<400> 2187

Asn Ser Trp Leu Gly Asn Ile Ile Met Tyr Ala Pro Thr Leu Trp
1               5               10              15

<210> 2188

<211> 15

<212> PRT

<213> hepatitis C virus

<400> 2188

Asn Val Ser Val Ala His Asp Ala Ser Gly Lys Arg Val Tyr Tyr
1               5                   10                  15

<210> 2189

<211> 15

<212> PRT

<213> hepatitis C virus

<400> 2189

Pro Cys Ser Phe Thr Thr Leu Pro Ala Leu Ser Thr Gly Leu Ile
1               5                   10                  15

<210> 2190

<211> 15

<212> PRT

<213> hepatitis C virus

<400> 2190

Pro Gly Ala Leu Val Val Gly Val Val Cys Ala Ala Ile Leu Arg
1               5                   10                  15

<210> 2191

<211> 15

<212> PRT

<213> hepatitis C virus

<400> 2191

Pro Met Gly Phe Ser Tyr Asp Thr Arg Cys Phe Asp Ser Thr Val
1               5                   10                  15

<210> 2192

<211> 15

<212> PRT

<213> hepatitis C virus

<400> 2192

```
Pro Gln Thr Phe Gln Val Ala His Leu His Ala Pro Thr Gly Ser
1               5                   10                  15
```

<210> 2193

<211> 15

<212> PRT

<213> hepatitis C virus


<400> 2193

```
Pro Thr His Tyr Val Pro Glu Ser Asp Ala Ala Ala Arg Val Thr
1               5                   10                  15
```

<210> 2194

<211> 15

<212> PRT

<213> hepatitis C virus


<400> 2194

```
Pro Thr Leu Trp Ala Arg Met Ile Leu Met Thr His Phe Phe Ser
1               5                   10                  15
```

<210> 2195

<211> 15

<212> PRT

<213> hepatitis C virus


<400> 2195

```
Pro Tyr Leu Val Ala Tyr Gln Ala Thr Val Cys Ala Arg Ala Gln
1               5                   10                  15
```

<210> 2196

<211> 15

<212> PRT

<213> hepatitis C virus


<400> 2196

```
Gln Asp Ala Val Ser Arg Ser Gln Arg Arg Gly Arg Thr Gly Arg
1               5                   10                  15
```

<210> 2197

<211> 15

<212> PRT

<213> hepatitis C virus

<400> 2197

Gln Lys Lys Val Thr Phe Asp Arg Leu Gln Val Leu Asp Asp His
1               5                   10                  15

<210> 2198

<211> 15

<212> PRT

<213> hepatitis C virus

<400> 2198

Gln Pro Glu Tyr Asp Leu Glu Leu Ile Thr Ser Cys Ser Ser Asn
1               5                   10                  15

<210> 2199

<211> 15

<212> PRT

<213> hepatitis C virus

<400> 2199

Arg Ala Ala Val Cys Thr Arg Gly Val Ala Lys Ala Val Asp Phe
1               5                   10                  15

<210> 2200

<211> 15

<212> PRT

<213> hepatitis C virus

<400> 2200

Arg Gly Leu Leu Gly Cys Ile Ile Thr Ser Leu Thr Gly Arg Asp
1               5                   10                  15

<210> 2201

<211> 15

<212> PRT

<213> hepatitis C virus

```
<400>  2201

Arg Leu Gly Val Arg Ala Thr Arg Lys Thr Ser Glu Arg Ser Gln
1               5                   10                  15


<210>  2202

<211>  15

<212>  PRT

<213>  hepatitis C virus


<400>  2202

Arg Leu Ile Val Phe Pro Asp Leu Gly Val Arg Val Cys Glu Lys
1               5                   10                  15


<210>  2203

<211>  15

<212>  PRT

<213>  hepatitis C virus


<400>  2203

Arg Leu Val Val Leu Ala Thr Ala Thr Pro Pro Gly Ser Val Thr
1               5                   10                  15


<210>  2204

<211>  15

<212>  PRT

<213>  hepatitis C virus


<400>  2204

Arg Pro Glu Tyr Asp Leu Glu Leu Ile Thr Ser Cys Ser Ser Asn
1               5                   10                  15


<210>  2205

<211>  15

<212>  PRT

<213>  hepatitis C virus


<400>  2205

Arg Gln Glu Met Gly Gly Asn Ile Thr Arg Val Glu Ser Glu Asn
1               5                   10                  15
```

<210> 2206

<211> 15

<212> PRT

<213> hepatitis C virus


<400> 2206

Arg Ser Glu Leu Ser Pro Leu Leu Leu Ser Thr Thr Glu Trp Gln
1               5                   10                  15


<210> 2207

<211> 15

<212> PRT

<213> hepatitis C virus


<400> 2207

Arg Ser Pro Val Phe Thr Asp Asn Ser Ser Pro Pro Ala Val Pro
1               5                   10                  15


<210> 2208

<211> 15

<212> PRT

<213> hepatitis C virus


<400> 2208

Ser Ala Met Tyr Val Gly Asp Leu Cys Gly Ser Val Phe Leu Val
1               5                   10                  15


<210> 2209

<211> 15

<212> PRT

<213> hepatitis C virus


<400> 2209

Ser Asp Leu Tyr Leu Val Thr Arg His Ala Asp Val Ile Pro Val
1               5                   10                  15


<210> 2210

<211> 15

<212> PRT

<213> hepatitis C virus

<400> 2210

Ser Phe Ser Ile Phe Leu Leu Ala Leu Leu Ser Cys Leu Thr Ile
1               5                   10                  15

<210> 2211

<211> 15

<212> PRT

<213> hepatitis C virus

<400> 2211

Ser Phe Ser Ile Phe Leu Leu Ala Leu Leu Ser Cys Leu Thr Val
1               5                   10                  15

<210> 2212

<211> 15

<212> PRT

<213> hepatitis C virus

<400> 2212

Ser Ile Phe Leu Leu Ala Leu Leu Ser Cys Leu Thr Ile Pro Ala
1               5                   10                  15

<210> 2213

<211> 15

<212> PRT

<213> hepatitis C virus

<400> 2213

Ser Lys Gly Trp Arg Leu Leu Ala Pro Ile Thr Ala Tyr Ala Gln
1               5                   10                  15

<210> 2214

<211> 15

<212> PRT

<213> hepatitis C virus

<400> 2214

Ser Leu Arg Val Phe Thr Glu Ala Met Thr Arg Tyr Ser Ala Pro
1               5               10              15


<210> 2215

<211> 15

<212> PRT

<213> hepatitis C virus


<400> 2215

Ser Arg Cys Trp Val Ala Leu Thr Pro Thr Leu Ala Ala Arg Asn
1               5               10              15


<210> 2216

<211> 15

<212> PRT

<213> hepatitis C virus


<400> 2216

Ser Thr Lys Val Pro Ala Ala Tyr Ala Ala Gln Gly Tyr Lys Val
1               5               10              15


<210> 2217

<211> 15

<212> PRT

<213> hepatitis C virus


<400> 2217

Ser Thr Thr Ile Leu Gly Ile Gly Thr Val Leu Asp Gln Ala Glu
1               5               10              15


<210> 2218

<211> 15

<212> PRT

<213> hepatitis C virus


<400> 2218

Ser Thr Trp Val Leu Val Gly Gly Val Leu Ala Ala Leu Ala Ala
1               5               10              15

<210> 2219

<211> 15

<212> PRT

<213> hepatitis C virus


<400> 2219

Ser Tyr Thr Trp Thr Gly Ala Leu Ile Thr Pro Cys Ala Ala Glu
1               5                   10                  15


<210> 2220

<211> 15

<212> PRT

<213> hepatitis C virus


<400> 2220

Thr Phe Gln Val Ala His Leu His Ala Pro Thr Gly Ser Gly Lys
1               5                   10                  15


<210> 2221

<211> 15

<212> PRT

<213> hepatitis C virus


<400> 2221

Thr Met Leu Val Cys Gly Asp Asp Leu Val Val Ile Cys Glu Ser
1               5                   10                  15


<210> 2222

<211> 15

<212> PRT

<213> hepatitis C virus


<400> 2222

Thr Pro Cys Ala Ala Glu Glu Ser Lys Leu Pro Ile Asn Ala Leu
1               5                   10                  15


<210> 2223

<211> 15

<212> PRT

<213> hepatitis C virus

<400> 2223

Thr Pro Cys Ala Ala Glu Glu Ser Lys Leu Pro Ile Asn Pro Leu
1               5               10              15

<210> 2224

<211> 15

<212> PRT

<213> hepatitis C virus

<400> 2224

Thr Pro Leu Leu Tyr Arg Leu Gly Ala Val Gln Asn Glu Val Thr
1               5               10              15

<210> 2225

<211> 15

<212> PRT

<213> hepatitis C virus

<400> 2225

Thr Arg Gly Leu Leu Gly Cys Ile Ile Thr Ser Leu Thr Gly Arg
1               5               10              15

<210> 2226

<211> 15

<212> PRT

<213> hepatitis C virus

<400> 2226

Thr Thr Ile Met Ala Lys Asn Glu Val Phe Cys Val Gln Pro Glu
1               5               10              15

<210> 2227

<211> 15

<212> PRT

<213> hepatitis C virus

<400> 2227

```
Thr Thr Thr Val Pro Gln Asp Ala Val Ser Arg Ser Gln Arg Arg
1               5                   10                  15
```

<210> 2228

<211> 15

<212> PRT

<213> hepatitis C virus


<400> 2228

```
Thr Val Asp Phe Ser Leu Asp Pro Thr Phe Thr Ile Glu Thr Thr
1               5                   10                  15
```

<210> 2229

<211> 15

<212> PRT

<213> hepatitis C virus


<400> 2229

```
Thr Trp Val Leu Val Gly Gly Val Leu Ala Ala Leu Ala Ala Tyr
1               5                   10                  15
```

<210> 2230

<211> 15

<212> PRT

<213> hepatitis C virus


<400> 2230

```
Val Asp Ile Leu Ala Gly Tyr Gly Ala Gly Val Ala Gly Ala Leu
1               5                   10                  15
```

<210> 2231

<211> 15

<212> PRT

<213> hepatitis C virus


<400> 2231

```
Val Glu Ser Met Glu Thr Thr Met Arg Ser Pro Val Phe Thr Asp
1               5                   10                  15
```

<210> 2232

```
<211>  15
<212>  PRT
<213>  hepatitis C virus


<400>  2232
Val Phe Cys Val Gln Pro Glu Lys Gly Gly Arg Lys Pro Ala Arg
1               5                   10                  15


<210>  2233
<211>  15
<212>  PRT
<213>  hepatitis C virus


<400>  2233
Val Gly Gly Val Leu Ala Ala Leu Ala Ala Tyr Cys Leu Thr Thr
1               5                   10                  15


<210>  2234
<211>  15
<212>  PRT
<213>  hepatitis C virus


<400>  2234
Val Gly Ile Phe Arg Ala Ala Val Cys Thr Arg Gly Val Ala Lys
1               5                   10                  15


<210>  2235
<211>  15
<212>  PRT
<213>  hepatitis C virus


<400>  2235
Val Leu Val Leu Asn Pro Ser Val Ala Ala Thr Leu Gly Phe Gly
1               5                   10                  15


<210>  2236
<211>  15
<212>  PRT
<213>  hepatitis C virus
```

```
<400>  2236

Val Asn Leu Leu Pro Ala Ile Leu Ser Pro Gly Ala Leu Val Val
1               5                   10                  15


<210>  2237

<211>  15

<212>  PRT

<213>  hepatitis C virus


<400>  2237

Val Asn Ser Trp Leu Gly Asn Ile Ile Met Tyr Ala Pro Thr Leu
1               5                   10                  15


<210>  2238

<211>  15

<212>  PRT

<213>  hepatitis C virus


<400>  2238

Val Gln Trp Met Asn Arg Leu Ile Ala Phe Ala Ser Arg Gly Asn
1               5                   10                  15


<210>  2239

<211>  15

<212>  PRT

<213>  hepatitis C virus


<400>  2239

Val Val Gly Val Val Cys Ala Ala Ile Leu Arg Arg His Val Gly
1               5                   10                  15


<210>  2240

<211>  15

<212>  PRT

<213>  hepatitis C virus


<400>  2240

Val Val Val Val Ala Thr Asp Ala Leu Met Thr Gly Phe Thr Gly
1               5                   10                  15
```

```
<210>  2241
<211>  15
<212>  PRT
<213>  hepatitis C virus


<400>  2241
Val Val Val Val Ala Thr Asp Ala Leu Met Thr Gly Tyr Thr Gly
1               5                   10                  15


<210>  2242
<211>  15
<212>  PRT
<213>  hepatitis C virus


<400>  2242
Val Trp Pro Leu Leu Leu Leu Leu Leu Ala Leu Pro Pro Arg Ala
1               5                   10                  15


<210>  2243
<211>  15
<212>  PRT
<213>  hepatitis C virus


<400>  2243
Val Tyr Tyr Leu Thr Arg Asp Pro Thr Thr Pro Leu Ala Arg Ala
1               5                   10                  15


<210>  2244
<211>  15
<212>  PRT
<213>  hepatitis C virus


<400>  2244
Trp Asp Gln Met Trp Lys Cys Leu Ile Arg Leu Lys Pro Thr Leu
1               5                   10                  15


<210>  2245
<211>  15
```

<212> PRT

<213> hepatitis C virus


<400> 2245

Trp Glu Ser Val Phe Thr Gly Leu Thr His Ile Asp Ala His Phe
1               5                   10                  15


<210> 2246

<211> 15

<212> PRT

<213> hepatitis C virus


<400> 2246

Trp Lys Cys Leu Ile Arg Leu Lys Pro Thr Leu His Gly Pro Thr
1               5                   10                  15


<210> 2247

<211> 15

<212> PRT

<213> hepatitis C virus


<400> 2247

Trp Val Leu Val Gly Gly Val Leu Ala Ala Leu Ala Ala Tyr Cys
1               5                   10                  15


<210> 2248

<211> 15

<212> PRT

<213> hepatitis C virus


<400> 2248

Tyr Asp Ile Ile Ile Cys Asp Glu Cys His Ser Thr Asp Ser Thr
1               5                   10                  15


<210> 2249

<211> 15

<212> PRT

<213> hepatitis C virus

<400> 2249

Tyr Gly Lys Phe Leu Ala Asp Gly Gly Cys Ser Gly Gly Ala Tyr
1               5               10                  15

<210> 2250

<211> 15

<212> PRT

<213> hepatitis C virus

<400> 2250

Tyr Gly Val Trp Pro Leu Leu Leu Leu Leu Ala Leu Pro Pro
1               5               10                  15

<210> 2251

<211> 15

<212> PRT

<213> hepatitis C virus

<400> 2251

Tyr Ile Pro Leu Val Gly Ala Pro Leu Gly Gly Ala Ala Arg Ala
1               5               10                  15

<210> 2252

<211> 15

<212> PRT

<213> Hepatitis C virus

<400> 2252

Tyr Lys Val Leu Val Leu Asn Pro Ser Val Ala Ala Thr Leu Gly
1               5               10                  15

<210> 2253

<211> 15

<212> PRT

<213> Hepatitis C virus

<400> 2253

Tyr Tyr Ser Met Val Gly Asn Trp Ala Lys Val Leu Ile Val Met
1               5               10                  15